(19) Europäisches Patentamt / European Patent Office / Office européen des brevets

(11) **EP 4 324 922 A1**

(12) **EUROPEAN PATENT APPLICATION**
published in accordance with Art. 153(4) EPC

(43) Date of publication:
**21.02.2024 Bulletin 2024/08**

(21) Application number: **22788161.2**

(22) Date of filing: **12.04.2022**

(51) International Patent Classification (IPC):
**C12N 15/00** (2006.01)

(52) Cooperative Patent Classification (CPC):
**C12N 15/00**

(86) International application number:
**PCT/JP2022/017576**

(87) International publication number:
**WO 2022/220236 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.04.2021 US 202163174500 P**

(71) Applicant: **Kyoto University**
**Kyoto-shi, Kyoto 606-8501 (JP)**

(72) Inventors:
• **INOUE Haruhisa**
**Kyoto-shi, Kyoto 606-8501 (JP)**

• **KONDO Takayuki**
**Kyoto-shi, Kyoto 606-8501 (JP)**
• **IKEUCHI Takeshi**
**Niigata-shi, Niigata 950-2181 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **INFORMATION PROCESSING METHOD, INFORMATION PROCESSING DEVICE, AND PROGRAM**

(57) An information processing method includes: Step 1 of detecting a first SNP which is a mutation of an Alzheimer's disease-associated gene in a genomic DNA sample derived from a subject; and Step 2 of determining whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

EP 4 324 922 A1

**Description**

[Technical Field]

The present invention relates to an information processing method, an information processing device, and a program.
Priority is claimed on United States Patent No. 63/174,500, provisionally filed in the United States April 13, 2021, the content of which is incorporated herein by reference.

[Background Art]

Alzheimer's disease (AD) has the largest number of patients among neurodegenerative diseases that cause dementia, and it is thought that there are 30 million patients worldwide as of 2010. With the advent of a super-aged society, the number of AD patients continues to increase, and is estimated to reach 60 million in 2030 and exceed 100 million in 2050 if there is no fundamental treatment.
With the development of biological and genetic studies in recent years, molecular biological understanding of the pathology of dementia, especially AD, has progressed. Specifically, a genome-wide association study (GWAS) has investigated the genetic background associated with various human traits, and identified 50 or more gene loci as AD-associated genes (for example, refer to Non-Patent Document 1). However, there is currently limited symptomatic treatment for AD. Against this background, AD is thought to develop due to a combined action of multiple genes (polygenes), and especially for sporadic AD with no family history, which accounts for 95% of AD patients, there is no effective approach to explore the genetic cause of the pathology.

[Citation List]

[Non-Patent Document]

[Non-Patent Document 1] Sims R et al., "The multiplex model of the genetics of Alzheimer's disease," Nature Neuroscience, Vol. 23, pp. 311-322, 2020

[Summary of Invention]

[Technical Problem]

The present invention has been made from the viewpoint of the above-described circumstances, and provides an information processing method, an information processing device, and a program capable of predicting a risk of developing AD in subjects.

[Solution to Problem]

The present inventors have conducted extensive studies to solve the above-described problem, and as a result, have produced cerebral cortical neurons using an iPS cohort consisting of iPS cells established from sporadic AD patients. Next, cell GWAS (GWAS) using the cerebral cortical neurons is performed using amyloid-$\beta$ (A$\beta$) 42/40 ratio (A$\beta$42/40 ratio), which is one of pathological indicators of AD, as a phenotype to search for gene loci associated with the A$\beta$42/40 ratio. Furthermore, the present inventors have found that the risk of developing AD in subjects can be predicted using the identified gene loci associated with the A$\beta$42/40 ratio as a polygene dataset, thus leading to realization of the present invention.
In the present specification, the above-described method, that is, new technology that produces cerebral cortical neurons from iPS cells, decomposes the complex AD pathology into phenotypes (pathological traits) for each cell type and pathology, and reconstructs the actual pathology of AD from the background genetic data is referred to as "cellular dissection of polygenicity (abbreviated as CDiP)" technology.
That is, the present invention includes the following aspects.

(1) An information processing method including: Step 1 of detecting a first SNP which is a mutation of an Alzheimer's disease-associated gene in a genomic DNA sample derived from a subject; and Step 2 of determining whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

(2) The information processing method according to (1), in which the machine learning model is Random Forest including a plurality of classifiers, and each classifier trains using a specific training dataset selected from among the plurality of training datasets based on principal component information in attribute information and genetic information of the patient who has developed Alzheimer's disease.

(3) The information processing method according to (1) or (2), in which the plurality of training datasets contain a dataset labeled with information on the onset of Alzheimer's disease for a genotype belonging to the second SNP inferred from the second SNP through genotype imputation.

(4) The information processing method according to any one of (1) to (3), in which the mutations are one or more mutations shown in Tables 1-1 to 1-77.

[Table 1-1]

| SNP ID | chr | position | gene locus | SNP location in gene locus |
|---|---|---|---|---|
| rs11260631 | 1 | 5565535 | NA | |
| rs12027231 | 1 | 5566294 | NA | |
| rs140532989 | 1 | 11054703 | NA | |
| rs145588311 | 1 | 11170657 | MTOR | intron_variant |
| rs17875920 | 1 | 11801023 | AGTRAP | intron_variant |
| rs7418059 | 1 | 11803080 | AGTRAP | intron_variant |
| rs34791043 | 1 | 11803485 | NA | NA |
| rs17875934 | 1 | 11804288 | AGTRAP | intron_variant |
| rs17875948 | 1 | 11807970 | AGTRAP | intron variant |
| rs17875954 | 1 | 11809161 | AGTRAP | intron_variant |
| rs17875967 | 1 | 11811273 | AGTRAP | intron_variant |
| rs17875970 | 1 | 11811886 | AGTRAP | intron_variant |
| rs116001018 | 1 | 11816860 | NA | |
| rs147306013 | 1 | 11817560 | NA | |
| rs72641053 | 1 | 12128435 | TNFRSF8 | intron_variant |
| rs79459531 | 1 | 17605062 | PADI3 | intron_variant |
| rs79724306 | 1 | 17607570 | PADI3 | intron_variant |
| rs187218036 | 1 | 17627131 | NA | |
| rs147239745 | 1 | 17631035 | NA | |
| rs10917055 | 1 | 19495467 | UBR4 | intron_variant |
| rs57482167 | 1 | 24495578 | NA | NA |
| rs4649197 | 1 | 24495722 | IFNLR1 | intron_variant |
| rs11249017 | 1 | 24496662 | IFNLR1 | intron_variant |
| rs12128905 | 1 | 24497000 | IFNLR1 | intron_variant |
| rs11249018 | 1 | 24498130 | IFNLR1 | intron_variant |
| rs3932667 | 1 | 24498696 | IFNLR1 | intron_variant |
| 1:24499617:C:CA | 1 | 24499617 | NA | NA |
| rs34212240 | 1 | 24500325 | IFNLR1 | intron_variant |
| rs3897438 | 1 | 24506247 | IFNLR1 | intron_variant |
| rs3897439 | 1 | 24506510 | IFNLR1 | intron_variant |

(continued)

| SNP ID | chr | position | gene locus | SNP location in gene locus |
|---|---|---|---|---|
| rs4081342 | 1 | 24506522 | IFNLR1 | intron_variant |
| 1:24506786:CA:C | 1 | 24506786 | NA | NA |
| rs3897440 | 1 | 24506861 | IFNLR1 | intron_variant |
| rs6680101 | 1 | 24507433 | IFNLR1 | intron_variant |
| rs6665649 | 1 | 24507437 | IFNLR1 | intron_variant |
| rs6683433 | 1 | 24507774 | IFNLR1 | intron_variant |

[Table 1-2]

| SNP ID | chr | position | gene locus | SNP location in gene locus |
|---|---|---|---|---|
| rs10903046 | 1 | 24507855 | IFNLR1 | intron_variant |
| rs79806176 | 1 | 34231799 | CSMD2 | NMD_transcript_variant |
| rs74658700 | 1 | 34234656 | CSMD2 | NMD_transcript_variant |
| rs76405888 | 1 | 34238146 | CSMD2 | NMD_transcript_variant |
| rs74224650 | 1 | 34244450 | CSMD2 | NMD_transcript_variant |
| rs58862022 | 1 | 34244587 | CSMD2 | NMD_transcript_variant |
| rs34278452 | 1 | 40588662 | NA | |
| rs12760525 | 1 | 40589754 | NA | |
| rs11800679 | 1 | 40603234 | NA | |
| rs35156697 | 1 | 40617030 | NA | |
| rs10493090 | 1 | 40626918 | NA | |
| rs11584362 | 1 | 40631460 | RLF | intron_variant |
| rs5028951 | 1 | 40632581 | RLF | intron_variant |
| rs12724048 | 1 | 40632954 | RLF | intron_variant |
| rs34795058 | 1 | 40636231 | RLF | intron_variant |
| rs12744808 | 1 | 40644934 | RLF | intron_variant |
| rs61780442 | 1 | 40649468 | RLF | intron_variant |
| rs11808248 | 1 | 40662090 | RLF | intron_variant |
| rs17878476 | 1 | 40668727 | RLF | intron_variant |
| 1:40682172:AAGAG:A | 1 | 40682172 | NA | NA |
| rs61778546 | 1 | 40687368 | RLF | intron_variant |
| rs16827064 | 1 | 40688132 | RLF | intron_variant |
| rs16827106 | 1 | 40735472 | ZMPSTE24 | intron_variant |
| rs16827109 | 1 | 40735817 | ZMPSTE24 | intron_variant |
| rs12757549 | 1 | 40742366 | ZMPSTE24 | intron_variant |
| rs71060364 | 1 | 40753372 | NA | NA |
| rs12725815 | 1 | 40753766 | ZMPSTE24 | intron_variant |
| rs76609695 | 1 | 41315101 | NA | |

(continued)

| SNP ID | chr | position | gene locus | SNP location in gene locus |
|---|---|---|---|---|
| rs61773507 | 1 | 48411428 | TRABD2B | intron_variant |
| rs115906838 | 1 | 58708368 | DAB1 | intron_variant |
| rs117567026 | 1 | 58715824 | DAB1 | intron_variant |
| rs12039506 | 1 | 58721695 | DAB1 | intron_variant |
| rs12035886 | 1 | 58721704 | DAB1 | intron_variant |
| rs140969406 | 1 | 58721802 | NA | NA |
| rs17117229 | 1 | 58733538 | DAB1 | intron_variant |
| rs151064393 | 1 | 58734680 | DAB1 | intron_variant |

[Table 1-3]

| SNP ID | chr | position | gene locus | SNP location in gene locus |
|---|---|---|---|---|
| rs117388414 | 1 | 58745146 | DAB1 | intron_variant |
| rs17117259 | 1 | 58753770 | DAB1 | intron_variant |
| rs17117277 | 1 | 58763802 | DAB1 | intron_variant |
| rs17117283 | 1 | 58764638 | DAB1 | intron_variant |
| rs142567959 | 1 | 58766083 | DAB1 | intron_variant |
| rs11542fi189 | 1 | 64539974 | R0R1 | intron_variant |
| rs138138409 | 1 | 64554364 | R0R1 | intron_variant |
| rs78457326 | 1 | 76867147 | ST6GALNAC3 | intron_variant |
| rs140199001 | 1 | 81198118 | NA | |
| rs192853907 | 1 | 81226954 | NA | |
| rs114618747 | 1 | 81240468 | NA | |
| rs117721292 | 1 | 81251534 | NA | |
| rs144408429 | 1 | 81252438 | NA | |
| rs145068891 | 1 | 81303947 | NA | |
| rs77325472 | 1 | 81308364 | NA | |
| rs142985266 | 1 | 81326827 | NA | |
| rs75819420 | 1 | 81328646 | NA | |
| rs76876118 | 1 | 81329832 | NA | |
| rs80094910 | 1 | 81330079 | NA | |
| rs10493678 | 1 | 81333502 | NA | |
| rs10493679 | 1 | 81334027 | NA | |
| rs61767086 | 1 | 89066582 | NA | |
| rs1988189 | 1 | 89082365 | NA | |
| rs4596856 | 1 | 89082375 | NA | |
| rs4259618 | 1 | 89082380 | NA | |
| rs12033271 | 1 | 89084247 | NA | |

(continued)

| SNP ID | chr | position | gene locus | SNP location in gene locus |
|---|---|---|---|---|
| 1:89085206:TAAA:T | 1 | 89085206 | NA | NA |
| 1:89085227:A:G | 1 | 89085227 | NA | NA |
| rs75316566 | 1 | 91967640 | CDC7 | intron_variant |
| 1:92031771:C:CA | 1 | 92031771 | NA | NA |
| rs61209856 | 1 | 94589551 | NA | |
| rs56931023 | 1 | 94590379 | NA | |
| rs78142006 | 1 | 94590533 | NA | |
| rs72962326 | 1 | 94595330 | NA | |
| rs117428698 | 1 | 95399858 | NA | |
| rs141570401 | 1 | 95424962 | NA | |

[Table 1-4]

| SNP ID | chr | position | gene locus | SNP location in gene locus |
|---|---|---|---|---|
| 1:102574438:C:T | 1 | 102574438 | NA | NA |
| rs117439108 | 1 | 104894981 | NA | |
| rs189348849 | 1 | 104978328 | NA | |
| rs11799443 | 1 | 107989087 | NTNG1 | intron_variant |
| rs142243476 | 1 | 107991761 | NTNG1 | intron_variant |
| rs111451024 | 1 | 114832660 | NA | |
| rs76185230 | 1 | 114833460 | NA | |
| rs79125386 | 1 | 114834247 | NA | |
| rs6660468 | 1 | 114835204 | NA | |
| rs116493739 | 1 | 114836116 | NA | |
| rs77470545 | 1 | 114836560 | NA | |
| rs7522214 | 1 | 114840898 | NA | |
| rs75376490 | 1 | 114841671 | NA | |
| rs7534361 | 1 | 114844072 | NA | |
| rs148371870 | 1 | 114845387 | NA | |
| rs142305684 | 1 | 114848595 | NA | NA |
| rs199724897 | 1 | 114849666 | NA | |
| 1:114850653:T:C | 1 | 114850653 | NA | NA |
| rs142658681 | 1 | 114850730 | NA | |
| rs7540256 | 1 | 114851811 | NA | |
| rs116039729 | 1 | 114852370 | NA | |
| rs145900544 | 1 | 114852385 | NA | |
| rs142737501 | 1 | 114852517 | NA | |
| 1:114855828:T:A | 1 | 114855828 | NA | NA |
| rs41470845 | 1 | 114856929 | NA | |

(continued)

| SNP ID | chr | position | gene locus | SNP location in gene locus |
|---|---|---|---|---|
| rs74580704 | 1 | 114862402 | NA | |
| rs76169339 | 1 | 114865513 | NA | |
| rs6669141 | 1 | 114865863 | NA | |
| rs12132759 | 1 | 115628467 | TSPAN2 | intron_variant |
| rs6701221 | 1 | 152778558 | LCE1C | intron_variant |
| 1:158992906:AT:A | 1 | 158992906 | NA | NA |
| rs4646881 | 1 | 165667980 | ALDH9A1 | 5_prime_UTR_variant |
| rs79887819 | 1 | 167633592 | RCSD1 | intron variant |
| rs78645780 | 1 | 170132537 | METTL11B | intron_variant |
| rs115223117 | 1 | 170139597 | NA | |
| rs115433886 | 1 | 170140312 | NA | NA |

[Table 1-5]

| SNP ID | chr | position | gene locus | SNP location in gene locus |
|---|---|---|---|---|
| rs7537272 | 1 | 170142133 | NA | |
| rs10564453 | 1 | 170142729 | NA | |
| rs143134448 | 1 | 170144306 | NA | |
| rs77373691 | 1 | 170146623 | NA | |
| rs6689201 | 1 | 170146938 | NA | |
| rs6671761 | 1 | 170158174 | NA | |
| rs74822235 | 1 | 170159553 | NA | |
| rs77849060 | 1 | 170161011 | NA | |
| rs17345810 | 1 | 170166066 | NA | |
| rs17349942 | 1 | 170167702 | NA | |
| rs78968813 | 1 | 170167937 | NA | |
| rs140761847 | 1 | 170169900 | NA | |
| 1:170171514:T:A | 1 | 170171514 | NA | NA |
| rs10919327 | 1 | 170171514 | NA | |
| rs17345893 | 1 | 170171874 | NA | |
| rs6662495 | 1 | 170172742 | NA | |
| rs6687139 | 1 | 170173262 | NA | |
| rs17345907 | 1 | 170174046 | NA | |
| rs12144655 | 1 | 170174298 | NA | |
| rs12142060 | 1 | 170176678 | NA | |
| rs77199503 | 1 | 170179140 | NA | |
| rs17345935 | 1 | 170179341 | NA | |
| rs17350032 | 1 | 170181363 | NA | |
| rs17350053 | 1 | 170184112 | NA | |

(continued)

| SNP ID | chr | position | gene locus | SNP location in gene locus |
|---|---|---|---|---|
| rs74981093 | 1 | 170193063 | NA | |
| rs12562203 | 1 | 175145938 | KIAA0040 | intron_variant |
| rs139718675 | 1 | 177096274 | ASTN1 | intron_variant |
| rs57530364 | 1 | 177096284 | ASTN1 | intron_variant |
| rs10798498 | 1 | 177096980 | ASTN1 | intron_variant |
| rs10753143 | 1 | 177097241 | ASTN1 | intron_variant |
| rs10798499 | 1 | 177097442 | ASTN1 | intron_variant |
| 1:177126430:TTTG:T | 1 | 177126430 | NA | NA |
| rs60826838 | 1 | 183545685 | NCF2 | intron_variant |
| rs117250867 | 1 | 184582654 | C1orf21 | intron_variant |
| rs12096795 | 1 | 184590346 | C1orf21 | 3_prime_UTR_variant |
| rs12023933 | 1 | 184621712 | NA | |

[Table 1-6]

| SNP ID | chr | position | gene locus | SNP location in gene locus |
|---|---|---|---|---|
| rs16823401 | 1 | 184666214 | EDEM3 | intron_variant |
| rs116897291 | 1 | 184814667 | NIBAN1 | intron_variant |
| rs148184379 | 1 | 184886968 | NIBAN1 | intron_variant |
| rs117517283 | 1 | 184921127 | NIBAN1 | intron_variant |
| rs74607511 | 1 | 192513026 | NA | |
| rs77505887 | 1 | 192515584 | NA | |
| rs74508649 | 1 | 192526317 | NA | |
| rs74419088 | 1 | 192529469 | NA | |
| rs77258347 | 1 | 192534699 | NA | |
| rs74367555 | 1 | 192538724 | NA | |
| rs76623154 | 1 | 192538746 | NA | |
| rs74130685 | 1 | 192538948 | NA | |
| rs115743395 | 1 | 192538965 | NA | |
| rs74130686 | 1 | 192539013 | NA | |
| rs147211012 | 1 | 199309033 | NA | |
| rs4915476 | 1 | 201047062 | CACNA1S | synonymous_variant |
| rs4915213 | 1 | 201047774 | CACNA1S | intron_variant |
| rs6427877 | 1 | 201048265 | CACNA1S | intron_variant |
| rs3767511 | 1 | 201052199 | CACNA1S | intron_variant |
| rs75488105 | 1 | 204065966 | SOX13 | intron_variant |
| rs113144751 | 1 | 204066542 | SOX13 | intron_variant |
| rs145279852 | 1 | 204066674 | SOX13 | intron_variant |
| rs147225273 | 1 | 204066680 | SOX13 | intron_variant |

(continued)

| SNP ID | chr | position | gene locus | SNP location in gene locus |
|---|---|---|---|---|
| rs17188797 | 1 | 211187783 | AL590132.1 | intron_variant |
| rs2378009 | 1 | 218947264 | NA | |
| rs6681600 | 1 | 218948258 | NA | |
| rs6673437 | 1 | 218948649 | NA | |
| rs6541208 | 1 | 218952920 | NA | |
| rs11452039 | 1 | 218954472 | NA | |
| rs6541210 | 1 | 218955436 | NA | |
| rs12032085 | 1 | 223641019 | NA | |
| rs117913581 | 1 | 232777842 | NA | |
| rs138567986 | 1 | 241325817 | RGS7 | intron_variant |
| 1:244577487:T:TAATA | 1 | 244577487 | NA | NA |
| rs3127454 | 1 | 244578852 | ADSS2 | intron_variant |
| rs3127462 | 1 | 244597892 | ADSS2 | intron_variant |

[Table 1-7]

| SNP ID | chr | position | gene locus | SNP location in gene locus |
|---|---|---|---|---|
| rs3127463 | 1 | 244598196 | ADSS2 | intron_variant |
| rs3127464 | 1 | 244599858 | ADSS2 | intron_variant |
| 1:244601900:C:CAT | 1 | 244601900 | NA | NA |
| rs3003213 | 1 | 244608181 | ADSS2 | intron_variant |
| 1:244608950:T:TA | 1 | 244608950 | NA | NA |
| 1:244608950TTAA | 1 | 244608950 | NA | NA |
| rs3006004 | 1 | 244609092 | ADSS2 | intron_variant |
| rs3003212 | 1 | 244612252 | ADSS2 | intron_variant |
| rs3127466 | 1 | 244614497 | ADSS2 | intron_variant |
| rs3003210 | 1 | 244617509 | NA | |
| rs141944966 | 1 | 245746729 | KIF26B | intron_variant |
| rs145014144 | 1 | 245759684 | KIF26B | intron_variant |
| rs78194184 | 1 | 247365152 | AL390728.4 | intron_variant |
| rs61272272 | 2 | 6215734 | NA | |
| rs12621265 | 2 | 6220141 | NA | |
| rs2609122 | 2 | 6220654 | NA | |
| rs76756243 | 2 | 6224392 | NA | |
| rs12052388 | 2 | 6224960 | NA | |
| rs60904524 | 2 | 6225735 | NA | |
| rs73150377 | 2 | 6227222 | NA | |
| rs73150378 | 2 | 6227726 | NA | |

(continued)

| SNP ID | chr | position | gene locus | SNP location in gene locus |
|---|---|---|---|---|
| rs77944704 | 2 | 6228902 | NA | |
| rs76144832 | 2 | 6231193 | NA | |
| rs74706208 | 2 | 6233421 | NA | |
| rs16864697 | 2 | 6233460 | NA | |
| rs12052438 | 2 | 6236379 | NA | |
| rs77918648 | 2 | 6270044 | NA | |
| rs76480233 | 2 | 6270267 | NA | |
| rs16864772 | 2 | 6271097 | NA | |
| rs16864773 | 2 | 6271288 | NA | |
| rs16864776 | 2 | 6271533 | NA | |
| rs16864778 | 2 | 6271646 | NA | |
| rs78933180 | 2 | 6274257 | NA | |
| rs75637712 | 2 | 6278287 | NA | |
| rs7557935 | 2 | 6281053 | NA | |
| rs7567221 | 2 | 6281198 | NA | |

[Table 1-8]

| SNP ID | chr | position | gene locus | SNP location in gene locus |
|---|---|---|---|---|
| rs11897984 | 2 | 7538773 | NA | |
| rs1346810 | 2 | 7539421 | NA | |
| rs10179128 | 2 | 7539984 | NA | |
| rs6709430 | 2 | 7544818 | NA | |
| rs1546026 | 2 | 10832167 | NA | |
| rs77715032 | 2 | 14959674 | NA | |
| 2:16523989:AT:A | 2 | 16523989 | NA | NA |
| rs201150095 | 2 | 16523991 | NA | NA |
| rs55641030 | 2 | 16982622 | NA | |
| rs74671599 | 2 | 16983140 | NA | |
| rs114875341 | 2 | 18374830 | KCNS3 | intron_variant |
| rs115436106 | 2 | 20365483 | NA | NA |
| rs2090033 | 2 | 20372497 | NA | |
| rs4233758 | 2 | 20376022 | NA | |
| rs75019476 | 2 | 31155554 | GALNT14 | intron_variant |
| rs75182639 | 2 | 31244662 | GALNT14 | intron_variant |
| rs59717368 | 2 | 34269641 | NA | |
| rs117140225 | 2 | 41848143 | NA | |
| rs117076730 | 2 | 41855067 | NA | |
| rs75983203 | 2 | 41860583 | NA | |

(continued)

| SNP ID | chr | position | gene locus | SNP location in gene locus |
|---|---|---|---|---|
| 2:47055014:A:T | 2 | 47055014 | NA | NA |
| rs72806356 | 2 | 52982063 | NA | |
| rs199881113 | 2 | 52987966 | NA | |
| rs115366500 | 2 | 52996352 | NA | |
| rs62127009 | 2 | 52998723 | NA | |
| rs72908943 | 2 | 53689606 | NA | |
| rs6745622 | 2 | 53704053 | NA | |
| rs145963514 | 2 | 66470046 | NA | |
| rs76686125 | 2 | 111908163 | BCL2L11 | intron_variant |
| rs113351584 | 2 | 114643053 | NA | |
| rs142890162 | 2 | 114728805 | NA | |
| rs111252481 | 2 | 114734142 | NA | |
| rs143348587 | 2 | 114753287 | NA | |
| rs10207532 | 2 | 138096983 | THSD7B | intron_variant |
| rs10196367 | 2 | 138210725 | THSD7B | intron_variant |
| rs28415678 | 2 | 138297618 | THSD7B | intron_variant |

[Table 1-9]

| SNP ID | chr | position | gene locus | SNP location in gene locus |
|---|---|---|---|---|
| rs72048484 | 2 | 142051977 | LRP1B | intron_variant |
| rs2679451 | 2 | 142053453 | LRP1B | intron_variant |
| rs2679455 | 2 | 142060374 | LRP1B | intron_variant |
| rs2714170 | 2 | 142068173 | LRP1B | intron_variant |
| rs118164915 | 2 | 142489626 | LRP1B | intron_variant |
| rs80027625 | 2 | 142563101 | LRP1B | intron_variant |
| rs7563677 | 2 | 161356717 | NA | |
| 2:161357267:AAAAG:A | 2 | 161357267 | NA | NA |
| rs141879065 | 2 | 161358374 | NA | |
| rs201036025 | 2 | 161358504 | NA | |
| rs72970060 | 2 | 161360952 | NA | |
| rs72970066 | 2 | 161362938 | NA | |
| rs72970074 | 2 | 161365114 | NA | |
| rs6736111 | 2 | 161368211 | NA | |
| rs187697067 | 2 | 161384015 | NA | NA |
| rs192262735 | 2 | 172895083 | METAP1D | intron_variant |
| rs79029608 | 2 | 172907025 | METAP1D | intron_variant |
| rs60153685 | 2 | 172920131 | METAP1D | intron_variant |

(continued)

| SNP ID | chr | position | gene locus | SNP location in gene locus |
|---|---|---|---|---|
| rs148393698 | 2 | 173138493 | NA | |
| 2:173790761:CGGAG:C | 2 | 173790761 | NA | NA |
| rs11695398 | 2 | 173791196 | RAPGEF4 | intron_variant |
| 2:173792363:T:TA | 2 | 173792363 | NA | NA |
| rs143907126 | 2 | 193371023 | NA | |
| rs12185569 | 2 | 202875552 | NA | |
| rs12185583 | 2 | 202875768 | NA | |
| rs10931984 | 2 | 202876281 | NA | |
| rs10931985 | 2 | 202876761 | NA | |
| rs13420220 | 2 | 202876981 | NA | |
| rs10490083 | 2 | 202877944 | NA | |
| rs10182797 | 2 | 202878867 | NA | |
| rs11682513 | 2 | 202879218 | NA | |
| rs17386240 | 2 | 202879624 | NA | |
| rs12328354 | 2 | 202882815 | NA | |
| rs12328774 | 2 | 202882897 | NA | |
| rs62184862 | 2 | 204683751 | NA | |
| rs62184863 | 2 | 204683797 | NA | |

[Table 1-10]

| SNP ID | chr | position | gene locus | SNP location in gene locus |
|---|---|---|---|---|
| 2:204695698:T:TA | 2 | 204695698 | NA | NA |
| rs58738696 | 2 | 204712127 | NA | |
| rs57248923 | 2 | 204712398 | NA | |
| rs62182593 | 2 | 204713735 | NA | |
| rs75622501 | 2 | 204714054 | NA | |
| rs6741283 | 2 | 204714810 | NA | |
| rs61602779 | 2 | 204716370 | NA | |
| rs13016652 | 2 | 211927188 | NA | |
| rs10932366 | 2 | 211954880 | NA | |
| rs188502110 | 2 | 216164766 | NA | |
| rs140760330 | 2 | 216165186 | NA | |
| rs111485414 | 2 | 228498025 | C2orf83 | non_coding_transcript_exon_variant |
| rs2048812 | 2 | 228518634 | NA | |
| rs2048813 | 2 | 228518649 | NA | |
| rs2048814 | 2 | 228518704 | NA | |
| rs7424668 | 2 | 228521539 | NA | |
| rs62189839 | 2 | 228521691 | NA | |

(continued)

| SNP ID | chr | position | gene locus | SNP location in gene locus |
|--------|-----|----------|------------|----------------------------|
| rs140104950 | 2 | 228523377 | NA | |
| rs112530470 | 2 | 228523536 | NA | |
| rs146239501 | 2 | 235171239 | NA | NA |
| rs1878963 | 2 | 235171335 | NA | |
| 2:235172453:T:C | 2 | 235172453 | NA | NA |
| rs6711496 | 2 | 235172514 | NA | |
| 2:235172825: ATATAGTTGGAG:A | 2 | 235172825 | NA | NA |
| rs4663367 | 2 | 235172880 | NA | |
| rs10865081 | 2 | 239876256 | NA | |
| rs149564461 | 2 | 239881016 | NA | |
| rs4643560 | 2 | 239881730 | NA | |
| rs4544463 | 2 | 239883557 | NA | |
| rs4852058 | 2 | 239884849 | NA | |
| rs4852059 | 2 | 239884850 | NA | |
| rs13000373 | 2 | 239886775 | NA | |
| rs6543539 | 2 | 239888052 | NA | |
| rs7579583 | 2 | 239888571 | NA | |
| rs7586010 | 2 | 239888597 | NA | |

[Table 1-11]

| SNP ID | chr | position | gene locus | SNP location in gene locus |
|--------|-----|----------|------------|----------------------------|
| rs6721090 | 2 | 239888880 | NA | |
| rs11387730 | 2 | 239888961 | NA | |
| rs11124201 | 2 | 239889428 | NA | |
| rs11124202 | 2 | 239889603 | NA | |
| rs4452172 | 2 | 239890163 | NA | |
| rs4852064 | 2 | 239890370 | NA | |
| rs4852065 | 2 | 239890581 | NA | |
| rs4852066 | 2 | 239890696 | NA | |
| rs4852067 | 2 | 239890850 | NA | |
| 2:239890901:G:GCCA | 2 | 239890901 | NA | NA |
| rs6754831 | 2 | 239890949 | NA | |
| rs4851977 | 2 | 239891296 | NA | |
| rs6543542 | 2 | 239891644 | NA | |
| rs7580803 | 2 | 239892335 | NA | |
| rs4241232 | 2 | 239893010 | NA | |
| rs145427397 | 2 | 239902414 | NA | |
| rs181670597 | 2 | 242151208 | AN07 | intron_variant |

(continued)

| SNP ID | chr | position | gene locus | SNP location in gene locus |
|---|---|---|---|---|
| 3:1749235:A:ATAT | 3 | 1749235 | NA | NA |
| rs139850749 | 3 | 1917654 | NA | |
| rs138485991 | 3 | 1934976 | NA | |
| rs7617928 | 3 | 5679782 | NA | |
| rs73808617 | 3 | 7086525 | GRM7 | NMD_transcript_variant |
| rs17046693 | 3 | 7088276 | GRM7 | NMD_transcript_variant |
| rs17046715 | 3 | 7090793 | GRM7 | NMD_transcript variant |
| rs74285835 | 3 | 7100399 | GRM7 | NMD_transcript_variant |
| rs74285838 | 3 | 7111851 | GRM7 | NMD_transcript_variant |
| rs6767094 | 3 | 7118997 | GRM7 | NMD_transcript_variant |
| rs75442424 | 3 | 7121831 | GRM7 | NMD_transcript_variant |
| rs76240495 | 3 | 7122393 | GRM7 | NMD_transcript_variant |
| 3:7122411:CA:GA | 3 | 7122411 | NA | NA |
| 3:7122411:CA:C | 3 | 7122411 | NA | NA |
| rs77578494 | 3 | 11980791 | NA | |
| rs75863593 | 3 | 12814936 | NA | |
| rs73028847 | 3 | 14272393 | NA | |
| rs28547889 | 3 | 14273762 | NA | |
| rs55961609 | 3 | 14274855 | NA | |

[Table 1-12]

| SNP ID | chr | position | gene locus | SNP location in gene locus |
|---|---|---|---|---|
| rs6762079 | 3 | 14275457 | NA | |
| rs7615075 | 3 | 14282434 | NA | |
| rs9867815 | 3 | 14282631 | NA | |
| rs17305345 | 3 | 14285488 | NA | |
| rs9855896 | 3 | 14287150 | NA | |
| rs9839148 | 3 | 14287726 | NA | |
| rs6764810 | 3 | 14289062 | NA | |
| rs9882488 | 3 | 14290607 | NA | |
| rs7640375 | 3 | 14290987 | NA | |
| rs7626720 | 3 | 14291503 | NA | |
| rs9817301 | 3 | 14292389 | NA | |
| rs9823520 | 3 | 14293832 | NA | |
| rs9860771 | 3 | 14293838 | NA | |
| 3:14296073:G: GCCTTGGGTGCACCTGGCCCT | 3 | 14296073 | NA | NA |
| rs11920518 | 3 | 14296545 | NA | |

(continued)

| SNP ID | chr | position | gene locus | SNP location in gene locus |
|---|---|---|---|---|
| rs2341654 | 3 | 14298116 | NA | |
| rs2128162 | 3 | 14298264 | NA | |
| rs1038298 | 3 | 14302455 | NA | |
| rs74710259 | 3 | 21874157 | ZNF385D | NMD_transcript_variant |
| rs79680055 | 3 | 21874582 | ZNF385D | NMD_transcript_variant |
| rs75670791 | 3 | 21914171 | ZNF385D | NMD_transcript variant |
| rs74903313 | 3 | 28653345 | AC098650. 1 | NMD_transcript variant |
| rs143877686 | 3 | 28653565 | AC098650. 1 | NMD_transcript_variant |
| rs78649247 | 3 | 28660023 | AC098650. 1 | NMD_transcript_variant |
| rs75624012 | 3 | 28762528 | AC098650. 1 | NMD_transcript variant |
| rs12491791 | 3 | 28769252 | AC098650. 1 | NMD_transcript variant |
| rs144410553 | 3 | 28796544 | AC098650. 1 | NMD_transcript_variant |
| rs114769913 | 3 | 28799686 | AC098650. 1 | NMD_transcript_variant |
| rs62241678 | 3 | 29333361 | AC098650. 1 | NMD_transcript_variant |
| rs150022738 | 3 | 32753524 | CN0T10 | intron_variant |
| rs62250842 | 3 | 32758483 | CN0T10 | intron_variant |
| rs62250843 | 3 | 32760921 | CN0T10 | intron_variant |
| rs6793550 | 3 | 32765663 | CN0T10 | intron_variant |
| 3:32774098:G:GA | 3 | 32774098 | NA | NA |
| rs145862784 | 3 | 32780082 | CN0T10 | intron_variant |

[Table 1-13]

| SNP ID | chr | position | gene locus | SNP location in gene locus |
|---|---|---|---|---|
| rs7612883 | 3 | 32889638 | TRIM71 | intron_variant |
| rs13060342 | 3 | 32891423 | TRIM71 | intron_variant |
| rs6800867 | 3 | 32981043 | NA | |
| rs6803409 | 3 | 32981316 | NA | |
| rs113716980 | 3 | 32982985 | NA | |
| 3:32983158:CT: C | 3 | 32983158 | NA | NA |
| 3:32987068:C:CT | 3 | 32987068 | NA | NA |
| rs4074331 | 3 | 32989009 | NA | |
| rs4074330 | 3 | 32989010 | NA | |
| rs73055186 | 3 | 32991856 | NA | |
| rs62250867 | 3 | 32991963 | NA | |
| rs7632357 | 3 | 32992203 | NA | |
| rs3774527 | 3 | 53705003 | CACNA1D | intron_variant |
| rs74426676 | 3 | 54509829 | CACNA2D3 | intron_variant |

(continued)

| SNP ID | chr | position | gene locus | SNP location in gene locus |
|---|---|---|---|---|
| 3:60608100:A:AT | 3 | 60608100 | NA | NA |
| rs142745963 | 3 | 65616074 | MAGI1 | intron variant |
| rs61555164 | 3 | 65741871 | MAGI1 | intron_variant |
| rs9841082 | 3 | 65743597 | MAGI1 | intron_variant |
| rs35219449 | 3 | 65872186 | MAGI1 | intron_variant |
| rs71306785 | 3 | 65872578 | MAGI1 | intron_variant |
| rs71306786 | 3 | 65873461 | MAGI1 | intron_variant |
| rs58687721 | 3 | 65873820 | MAGI1 | intron_variant |
| rs35168985 | 3 | 65874420 | MAGI1 | intron_variant |
| rs2372397 | 3 | 65875834 | MAGI1 | intron_variant |
| rs117501871 | 3 | 65876113 | MAGI1 | intron_variant |
| rs181795375 | 3 | 65877601 | MAGI1 | intron_variant |
| rs71306788 | 3 | 65877703 | MAGI1 | intron_variant |
| rs71306789 | 3 | 65877793 | MAGI1 | intron_variant |
| rs34548503 | 3 | 65878656 | MAGI1 | intron_variant |
| 3:65878951:G:GA | 3 | 65878951 | NA | NA |
| rs924021 | 3 | 65879837 | MAGI1 | intron_variant |
| rs35482956 | 3 | 65880339 | MAGI1 | intron_variant |
| rs74795753 | 3 | 65926053 | MAGI1 | intron_variant |
| rs117740205 | 3 | 65931724 | MAGI1 | intron_variant |
| rs114019667 | 3 | 66710033 | NA | |
| rs13319937 | 3 | 68599941 | NA | |

[Table 1-14]

| SNP ID | chr | position | gene locus | SNP location in gene locus |
|---|---|---|---|---|
| rs1858373 | 3 | 68601038 | NA | |
| rs1858372 | 3 | 68601193 | NA | |
| rs55744776 | 3 | 68601734 | NA | |
| rs7623705 | 3 | 68601948 | NA | |
| rs72106943 | 3 | 68605001 | NA | |
| rs10576290 | 3 | 68605535 | NA | |
| rs7616164 | 3 | 68606548 | NA | |
| rs9837106 | 3 | 68606690 | NA | |
| rs9866302 | 3 | 68609673 | NA | |
| rs13321458 | 3 | 68610030 | NA | |
| rs9835386 | 3 | 68610847 | NA | |
| rs67172613 | 3 | 77035984 | R0B02 | intron_variant |

(continued)

| SNP ID | chr | position | gene locus | SNP location in gene locus |
|---|---|---|---|---|
| rs72902045 | 3 | 77036238 | R0B02 | intron_variant |
| rs7427534 | 3 | 77041173 | R0B02 | intron_variant |
| rs6795635 | 3 | 77045304 | R0B02 | intron_variant |
| rs6806644 | 3 | 77045353 | R0B02 | intron_variant |
| rs6548472 | 3 | 77045731 | R0B02 | intron_variant |
| rs11374746 | 3 | 77045771 | R0B02 | intron_variant |
| rs6786589 | 3 | 77046740 | R0B02 | intron_variant |
| rs9875709 | 3 | 77047311 | R0B02 | intron_variant |
| rs6774797 | 3 | 77048488 | R0B02 | intron_variant |
| rs11918007 | 3 | 77051881 | R0B02 | intron_variant |
| 3:84969212:T:A | 3 | 84969212 | NA | NA |
| rs144035609 | 3 | 84972857 | NA | |
| rs150062308 | 3 | 85019042 | CADM2 | intron_variant |
| rs148678484 | 3 | 85042238 | CADM2 | intron_variant |
| rs143096570 | 3 | 85051020 | CADM2 | intron_variant |
| rs2875508 | 3 | 86021016 | CADM2 | intron_variant |
| rs76958889 | 3 | 86022860 | CADM2 | intron_variant |
| 3:107339790:T:G | 3 | 107339790 | NA | NA |
| 3:107502792:C:CA | 3 | 107502792 | NA | NA |
| 3:112698726:AATGG:AATGGATGG | 3 | 112698726 | NA | NA |
| 3:112698726:AATGG:A | 3 | 112698726 | NA | NA |
| rs2613959 | 3 | 112808299 | NA | |
| rs73229010 | 3 | 112899616 | NA | |
| rs73229016 | 3 | 112901722 | NA | |

[Table 1-15]

| SNP ID | chr | position | gene locus | SNP location in gene locus |
|---|---|---|---|---|
| rs79608790 | 3 | 112958412 | B0C | intron_variant |
| 3:112958412:T:G | 3 | 112958412 | NA | NA |
| rs58481194 | 3 | 172947355 | NA | |
| rs6443469 | 3 | 177286455 | NA | |
| rs7627379 | 3 | 177286946 | NA | |
| rs937509 | 3 | 177287701 | NA | |
| rs6768291 | 3 | 177290183 | NA | |
| rs2055366 | 3 | 177290345 | NA | |
| rs1970860 | 3 | 177290953 | NA | |
| rs1970861 | 3 | 177291213 | NA | |

(continued)

| SNP ID | chr | position | gene locus | SNP location in gene locus |
|---|---|---|---|---|
| rs4241483 | 3 | 177291318 | NA | |
| rs5854759 | 3 | 177292429 | NA | |
| rs6806363 | 3 | 177294081 | NA | |
| rs13083115 | 3 | 177294781 | NA | |
| rs1973469 | 3 | 177295093 | NA | |
| rs2863011 | 3 | 177295520 | NA | |
| rs2133596 | 3 | 177295608 | NA | |
| rs11713121 | 3 | 177295661 | NA | |
| rs60882310 | 3 | 177295932 | NA | |
| rs35403806 | 3 | 177296263 | NA | |
| rs36018104 | 3 | 177296267 | NA | |
| rs57592389 | 3 | 177296362 | NA | |
| rs58786527 | 3 | 177296363 | NA | |
| rs13062339 | 3 | 177296394 | NA | |
| rs13084960 | 3 | 177296448 | NA | |
| rs34858531 | 3 | 177296554 | NA | |
| rs4857705 | 3 | 177296885 | NA | |
| rs48577Q6 | 3 | 177296956 | NA | |
| rs4857707 | 3 | 177297074 | NA | |
| rs4857708 | 3 | 177297173 | NA | |
| 3:177297644:G:GTT | 3 | 177297644 | NA | NA |
| rs6801659 | 3 | 177297792 | NA | |
| rs6801849 | 3 | 177297824 | NA | |
| rs1463859 | 3 | 177298071 | NA | |
| rs1463858 | 3 | 177298127 | NA | |
| rs1463857 | 3 | 177298327 | NA | |

[Table 1-16]

| SNP ID | chr | position | gene locus | SNP location in gene locus |
|---|---|---|---|---|
| rs4555562 | 3 | 177298563 | NA | |
| rs4257602 | 3 | 177298695 | NA | |
| rs4273409 | 3 | 177298785 | NA | |
| rs4290846 | 3 | 177298934 | NA | |
| rs35168340 | 3 | 177299052 | NA | |
| rs34332266 | 3 | 177299061 | NA | |
| rs34316712 | 3 | 177299065 | NA | |
| rs36102700 | 3 | 177299109 | NA | |

(continued)

| SNP ID | chr | position | gene locus | SNP location in gene locus |
|---|---|---|---|---|
| rs35921520 | 3 | 177299232 | NA | |
| rs6805429 | 3 | 177299269 | NA | |
| rs6781232 | 3 | 177299379 | NA | |
| 3:177299739:A:ATTT | 3 | 177299739 | NA | NA |
| rs6808260 | 3 | 177299832 | NA | |
| rs7630147 | 3 | 177300391 | NA | |
| rs7610690 | 3 | 177300717 | NA | |
| rs7610926 | 3 | 177300753 | NA | |
| rs4857709 | 3 | 177300955 | NA | |
| rs4857710 | 3 | 177300965 | NA | |
| rs4857711 | 3 | 177300980 | NA | |
| rs4857712 | 3 | 177301016 | NA | |
| rs6763782 | 3 | 177301280 | NA | |
| 3:177301483: AGGACTTAAGCAGAT:A | 3 | 177301483 | NA | NA |
| rs2863019 | 3 | 177301836 | NA | |
| rs2863018 | 3 | 177301911 | NA | |
| rs2133592 | 3 | 177301939 | NA | |
| rs2133593 | 3 | 177301992 | NA | |
| rs2863017 | 3 | 177302033 | NA | |
| rs2173608 | 3 | 177302338 | NA | |
| rs6783033 | 3 | 177302751 | NA | |
| rs6770216 | 3 | 177302927 | NA | |
| rs2863016 | 3 | 177302980 | NA | |
| rs2863015 | 3 | 177303179 | NA | |
| rs2863014 | 3 | 177303192 | NA | |
| rs2133595 | 3 | 177303610 | NA | |
| rs1875095 | 3 | 177303723 | NA | |

[Table 1-17]

| SNP ID | chr | position | gene locus | SNP location in gene locus |
|---|---|---|---|---|
| rs1875096 | 3 | 177303864 | NA | |
| rs1875097 | 3 | 177304032 | NA | |
| rs7620560 | 3 | 177304176 | NA | |
| rs7620503 | 3 | 177304298 | NA | |
| rs7634809 | 3 | 177304668 | NA | |
| rs7645281 | 3 | 177304836 | NA | |
| rs7623309 | 3 | 177304851 | NA | |

(continued)

| SNP ID | chr | position | gene locus | SNP location in gene locus |
|---|---|---|---|---|
| rs6443474 | 3 | 177305124 | NA | |
| rs6777561 | 3 | 177305198 | NA | |
| rs13077994 | 3 | 177305705 | NA | |
| rs58453015 | 3 | 177305847 | NA | NA |
| rs4857713 | 3 | 177306442 | NA | |
| rs4857612 | 3 | 177306621 | NA | |
| rs11350527 | 3 | 177306757 | NA | |
| rs7637965 | 3 | 177307642 | NA | |
| rs6443476 | 3 | 177307695 | NA | |
| rs6443477 | 3 | 177307933 | NA | |
| rs6769466 | 3 | 177308014 | NA | |
| rs7616679 | 3 | 177308486 | NA | |
| rs11708469 | 3 | 177308718 | NA | |
| rs6797636 | 3 | 177309442 | NA | |
| rs13079175 | 3 | 177309533 | NA | |
| 3:186444809:A:G | 3 | 186444809 | NA | NA |
| rs117064584 | 3 | 189164430 | NA | |
| rs117987191 | 3 | 189523891 | TP63 | intron_variant |
| rs72613184 | 4 | 5235040 | STK32B | intron_variant |
| rs149515956 | 4 | 6341633 | PPP2R2C | intron_variant |
| rs147293851 | 4 | 13966004 | NA | |
| rs142792029 | 4 | 14017337 | NA | |
| rs62294496 | 4 | 17260883 | NA | |
| rs118004921 | 4 | 17304792 | NA | |
| rs11933789 | 4 | 17825159 | NCAPG | intron_variant |
| rs62295154 | 4 | 19769915 | NA | |
| rs13435165 | 4 | 19772721 | NA | |
| rs79235820 | 4 | 22171802 | NA | |
| rs116460686 | 4 | 31892334 | NA | |

[Table 1-18]

| SNP ID | chr | position | gene locus | SNP location in gene locus |
|---|---|---|---|---|
| rs115446883 | 4 | 31896317 | NA | |
| rs146966940 | 4 | 41153294 | APBB2 | intron_variant |
| 4:41784275:A:C | 4 | 41784275 | NA | NA |
| rs11942428 | 4 | 44580439 | NA | |
| rs148742662 | 4 | 54567050 | NA | NA |
| rs144162078 | 4 | 54580834 | AC058822. 1 | intron_variant |

(continued)

| SNP ID | chr | position | gene locus | SNP location in gene locus |
|---|---|---|---|---|
| 4:54582351:CA:C | 4 | 54582351 | NA | NA |
| rs143711414 | 4 | 54604589 | AC058822. 1 | intron_variant |
| 4:61494990:A:AT | 4 | 61494990 | NA | NA |
| rs117340277 | 4 | 63017478 | NA | |
| rs76314468 | 4 | 64039941 | NA | |
| rs79892130 | 4 | 64040056 | NA | |
| rs75655453 | 4 | 64040607 | NA | |
| rs77849378 | 4 | 64041936 | NA | |
| rs74461070 | 4 | 64042807 | NA | |
| rs77802168 | 4 | 64042829 | NA | |
| rs6816831 | 4 | 64044660 | NA | |
| rs6848438 | 4 | 64044675 | NA | |
| rs143395876 | 4 | 64046194 | NA | |
| rs4502710 | 4 | 64047829 | NA | |
| rs143437614 | 4 | 64048178 | NA | |
| rs4035516 | 4 | 64049319 | NA | |
| rs138747488 | 4 | 64049566 | NA | |
| rs79890012 | 4 | 64050856 | NA | |
| rs139008026 | 4 | 64051485 | NA | |
| rs116239045 | 4 | 64051678 | NA | |
| rs139446359 | 4 | 64052143 | NA | |
| rs144091716 | 4 | 64052292 | NA | |
| rs114269773 | 4 | 64053241 | NA | |
| rs148230700 | 4 | 64054216 | NA | |
| rs114403067 | 4 | 64054570 | NA | |
| rs115936064 | 4 | 64055253 | NA | |
| rs142760460 | 4 | 64055706 | NA | |
| rs147936120 | 4 | 64057031 | NA | |
| rs115409842 | 4 | 64057046 | NA | |
| rs115633947 | 4 | 64058695 | NA | |

[Table 1-19]

| SNP ID | chr | position | gene locus | SNP location in gene locus |
|---|---|---|---|---|
| rs148720007 | 4 | 64058783 | NA | |
| rs140223857 | 4 | 64058820 | NA | |
| rs7680092 | 4 | 64061134 | NA | |
| 4:64061283:AT:ATT | 4 | 64061283 | NA | NA |

(continued)

| SNP ID | chr | position | gene locus | SNP location in gene locus |
|---|---|---|---|---|
| 4:64061283:AT:A | 4 | 64061283 | NA | NA |
| rs80287150 | 4 | 64061543 | NA | |
| rs115458156 | 4 | 64061728 | NA | |
| 4:64062006:C:CA | 4 | 64062006 | NA | NA |
| 4:64062006:C:CAA | 4 | 64062006 | NA | NA |
| rs74340891 | 4 | 64062350 | NA | |
| rs116187324 | 4 | 64064808 | NA | |
| rs74796023 | 4 | 64066685 | NA | |
| rs74864007 | 4 | 64066878 | NA | |
| rs115897647 | 4 | 64066892 | NA | |
| rs201614722 | 4 | 64067415 | NA | |
| rs201701309 | 4 | 64067892 | NA | |
| rs201649360 | 4 | 64068147 | NA | |
| rs184640169 | 4 | 64069492 | NA | |
| rs77460761 | 4 | 64069972 | NA | |
| rs182226229 | 4 | 64070081 | NA | |
| rs183837605 | 4 | 64071489 | NA | |
| rs146847342 | 4 | 64072931 | NA | |
| rs76764827 | 4 | 64072957 | NA | |
| rs74392895 | 4 | 64074311 | NA | |
| rs79387056 | 4 | 64074978 | NA | |
| rs75876899 | 4 | 64076116 | NA | |
| rs116177742 | 4 | 64076886 | NA | |
| rs114946744 | 4 | 64076904 | NA | |
| rs80315791 | 4 | 64078203 | NA | |
| rs80199562 | 4 | 64079037 | NA | |
| rs76630438 | 4 | 64079127 | NA | |
| rs78126082 | 4 | 64079408 | NA | |
| rs2881626 | 4 | 64080016 | NA | |
| rs75900830 | 4 | 64080483 | NA | |
| rs77305257 | 4 | 64080642 | NA | |
| rs77019672 | 4 | 64081403 | NA | |

[Table 1-20]

| SNP ID | chr | position | gene locus | SNP location in gene locus |
|---|---|---|---|---|
| rs115178258 | 4 | 64082381 | NA | |
| rs79113257 | 4 | 64086632 | NA | |

(continued)

| SNP ID | chr | position | gene locus | SNP location in gene locus |
|---|---|---|---|---|
| rs147278503 | 4 | 93215159 | NA | |
| rs141054368 | 4 | 93217241 | NA | |
| rs7658734 | 4 | 96509012 | NA | |
| 4:96513678:C:T | 4 | 96513678 | NA | NA |
| rs62306715 | 4 | 96513884 | NA | |
| 4:96514526:T:C | 4 | 96514526 | NA | NA |
| rs113843935 | 4 | 96514739 | NA | |
| rs67930114 | 4 | 96515437 | NA | |
| rs57799366 | 4 | 96517154 | NA | |
| rs62306720 | 4 | 96517458 | NA | |
| 4:96518109:G:GA | 4 | 96518109 | NA | NA |
| rs2123367 | 4 | 109711528 | NA | |
| rs77922013 | 4 | 109712306 | NA | |
| rs76550713 | 4 | 109712541 | NA | |
| rs150990204 | 4 | 109712817 | NA | |
| rs17039415 | 4 | 109722415 | NA | |
| rs146480894 | 4 | 113229907 | ALPK1 | non_coding_transcript_variant |
| rs150718823 | 4 | 113232846 | ALPK1 | non_coding_transcript_variant |
| rs115122068 | 4 | 117719540 | NA | |
| rs150939893 | 4 | 117744934 | NA | |
| rs190696080 | 4 | 117750582 | NA | |
| rs113295558 | 4 | 117755974 | NA | |
| rs191714458 | 4 | 117758243 | NA | |
| rs186618613 | 4 | 117762073 | NA | |
| rs190966932 | 4 | 117762377 | NA | |
| rs192343018 | 4 | 117767150 | NA | |
| rs181001803 | 4 | 117773288 | NA | |
| rs7684310 | 4 | 117773712 | NA | |
| rs189192694 | 4 | 117774888 | NA | |
| rs191512269 | 4 | 117777305 | NA | |
| 4:117780739:G:T | 4 | 117780739 | NA | NA |
| rs182361185 | 4 | 117787075 | NA | |
| 4:117793406:A:T | 4 | 117793406 | NA | NA |
| rs74657487 | 4 | 122236579 | NA | |

[Table 1-21]

| SNP ID | chr | position | gene locus | SNP location in gene locus |
|---|---|---|---|---|
| rs80300492 | 4 | 122239822 | NA | |
| rs75622168 | 4 | 122240327 | NA | |
| rs79585077 | 4 | 122241796 | NA | |
| rs76926525 | 4 | 122242129 | NA | |
| rs77958862 | 4 | 122244530 | NA | |
| rs77294448 | 4 | 122246900 | NA | |
| rs6821123 | 4 | 122249973 | QRFPR | 3_prime_UTR_variant |
| rs113334103 | 4 | 122252388 | QRFPR | intron_variant |
| rs55975435 | 4 | 122254014 | QRFPR | 3_prime_UTR_variant |
| rs55776058 | 4 | 122254284 | QRFPR | intron_variant |
| rs2302308 | 4 | 122258149 | QRFPR | intron_variant |
| rs74398478 | 4 | 122259584 | QRFPR | intron_variant |
| rs17051338 | 4 | 122260042 | QRFPR | intron_variant |
| rs77438622 | 4 | 122264692 | QRFPR | intron_variant |
| rs76536576 | 4 | 122268524 | QRFPR | intron_variant |
| rs182992688 | 4 | 122270068 | QRFPR | intron_variant |
| rs187168138 | 4 | 122270076 | QRFPR | intron_variant |
| rs77006299 | 4 | 122270900 | QRFPR | intron_variant |
| rs10518388 | 4 | 122280335 | QRFPR | intron_variant |
| rs17051344 | 4 | 122285263 | QRFPR | intron_variant |
| rs66511907 | 4 | 122286927 | QRFPR | intron_variant |
| rs76503879 | 4 | 122287794 | QRFPR | intron_variant |
| 4:133557517:A:T | 4 | 133557517 | NA | NA |
| rs201392439 | 4 | 134152521 | NA | |
| 4:136278810:T:G | 4 | 136278810 | NA | NA |
| rs79757000 | 4 | 136309269 | NA | |
| rs10005391 | 4 | 136318357 | NA | |
| rs10017268 | 4 | 136318429 | NA | |
| rs10005518 | 4 | 136318641 | NA | |
| rs10008741 | 4 | 136319479 | NA | |
| rs10008854 | 4 | 136319621 | NA | |
| 4:136320586G:GTT | 4 | 136320586 | NA | NA |
| 4:136320587:C:A | 4 | 136320587 | NA | NA |
| rs150556358 | 4 | 136320899 | NA | NA |
| rs28701471 | 4 | 136321575 | NA | |
| 4:136321714:CAA:CA | 4 | 136321714 | NA | NA |

[Table 1-22]

| SNP ID | chr | position | gene locus | SNP location in gene locus |
|---|---|---|---|---|
| 4:136321714:CAA:C | 4 | 136321714 | NA | NA |
| rs33960846 | 4 | 136321824 | NA | |
| rs28481887 | 4 | 136322868 | NA | |
| rs28673025 | 4 | 136322980 | NA | |
| rs28709698 | 4 | 136323895 | NA | |
| rs76261505 | 4 | 136324955 | NA | |
| rs10028807 | 4 | 136327412 | NA | |
| rs145137955 | 4 | 136327637 | NA | |
| 4:136327754:CT:C | 4 | 136327754 | NA | NA |
| rs28872626 | 4 | 136327960 | NA | |
| rs28790884 | 4 | 136327980 | NA | |
| rs28838886 | 4 | 136327990 | NA | |
| rs28857199 | 4 | 136328206 | NA | |
| 4:136328885:T:A | 4 | 136328885 | NA | NA |
| 4:136328886:G:T | 4 | 136328886 | NA | NA |
| rs6810998 | 4 | 140693268 | MAML3 | intron_variant |
| rs78180498 | 4 | 141207904 | SCOC | intron_variant |
| rs78619534 | 4 | 141219174 | SCOC | intron_variant |
| rs17021272 | 4 | 147156085 | NA | |
| rs72948673 | 4 | 147174693 | NA | |
| rs60367087 | 4 | 147199809 | SLC10A7 | intron_variant |
| rs72950642 | 4 | 147238329 | SLC10A7 | intron_variant |
| rs72950644 | 4 | 147239235 | SLC10A7 | intron_variant |
| 4:147245033:T:C | 4 | 147245033 | NA | NA |
| rs112713961 | 4 | 147245957 | SLC10A7 | intron_variant |
| rs72950652 | 4 | 147246540 | SLC10A7 | intron_variant |
| rs72950654 | 4 | 147248032 | SLC10A7 | intron_variant |
| rs113969284 | 4 | 147248604 | SLC10A7 | intron_variant |
| rs72950658 | 4 | 147253902 | SLC10A7 | intron_variant |
| rs72950664 | 4 | 147255021 | SLC10A7 | intron_variant |
| rs72950667 | 4 | 147255582 | SLC10A7 | intron_variant |
| rs142662018 | 4 | 147255649 | SLC10A7 | intron_variant |
| rs72950672 | 4 | 147260169 | SLC10A7 | intron_variant |
| rs72950677 | 4 | 147261151 | SLC10A7 | intron_variant |
| rs72950679 | 4 | 147262990 | SLC10A7 | intron_variant |
| rs72950681 | 4 | 147264589 | SLC10A7 | intron_variant |

[Table 1-23]

| SNP ID | chr | position | gene locus | SNP location in gene locus |
|---|---|---|---|---|
| rs72950682 | 4 | 147264764 | SLC10A7 | intron_variant |
| rs78199829 | 4 | 147267044 | SLC10A7 | intron_variant |
| rs72950684 | 4 | 147267419 | SLC10A7 | intron_variant |
| rs72950690 | 4 | 147269478 | SLC10A7 | intron_variant |
| rs58672301 | 4 | 147269836 | SLC10A7 | intron_variant |
| rs111963516 | 4 | 147271022 | SLC10A7 | intron_variant |
| rs76206578 | 4 | 147271027 | SLC10A7 | intron_variant |
| 4:147271544:A:G | 4 | 147271544 | NA | NA |
| 4:147272211:A:T | 4 | 147272211 | NA | NA |
| 4:147272243:A:G | 4 | 147272243 | NA | NA |
| 4:147272464:C:T | 4 | 147272464 | NA | NA |
| rs72950697 | 4 | 147277445 | SLC10A7 | intron_variant |
| rs58494546 | 4 | 147278079 | SLC10A7 | intron_var iant |
| rs72952504 | 4 | 147278240 | SLC10A7 | intron_variant |
| rs72952506 | 4 | 147278447 | SLC10A7 | intron_variant |
| rs72952508 | 4 | 147278472 | SLC10A7 | intron_variant |
| rs11455281 | 4 | 147279083 | SLC10A7 | intron_variant |
| rs111635640 | 4 | 147279100 | SLC10A7 | intron_variant |
| rs6816058 | 4 | 147279394 | SLC10A7 | intron_variant |
| rs72952510 | 4 | 147280039 | SLC10A7 | intron_variant |
| rs111476662 | 4 | 147280504 | SLC10A7 | intron_variant |
| rs72952511 | 4 | 147280820 | SLC10A7 | intron_variant |
| rs72952513 | 4 | 147281127 | SLC10A7 | intron_variant |
| rs72952515 | 4 | 147281316 | SLC10A7 | intron_variant |
| 4:147281756:C:CT | 4 | 147281756 | NA | NA |
| rs60404214 | 4 | 147282897 | SLC10A7 | intron_variant |
| rs2630284 | 4 | 147284129 | SLC10A7 | intron_variant |
| rs4027057 | 4 | 147284502 | SLC10A7 | intron_variant |
| rs2679154 | 4 | 147285031 | SLC10A7 | intron_variant |
| rs2679153 | 4 | 147286796 | SLC10A7 | intron_variant |
| rs2255242 | 4 | 147286963 | SLC10A7 | intron_variant |
| rs2679151 | 4 | 147287007 | SLC10A7 | intron_variant |
| rs2244316 | 4 | 147289414 | SLC10A7 | intron_variant |
| rs2244317 | 4 | 147289424 | SLC10A7 | intron_variant |
| rs2244318 | 4 | 147289431 | SLC10A7 | intron_variant |
| rs2244321 | 4 | 147289522 | SLC10A7 | intron_variant |

[Table 1-24]

| SNP ID | chr | position | gene locus | SNP location in gene locus |
|---|---|---|---|---|
| rs2679149 | 4 | 147290788 | SLC10A7 | intron_variant |
| rs2630278 | 4 | 147292164 | SLC10A7 | intron_variant |
| rs2630277 | 4 | 147292832 | SLC10A7 | intron_variant |
| rs2244840 | 4 | 147293347 | SLC10A7 | intron_variant |
| rs2244850 | 4 | 147293653 | SLC10A7 | intron_variant |
| rs2679145 | 4 | 147294299 | SLC10A7 | intron_variant |
| 4:147295793:C:G | 4 | 147295793 | NA | NA |
| rs2679121 | 4 | 147296826 | SLC10A7 | intron_variant |
| rs2245274 | 4 | 147297184 | SLC10A7 | intron_variant |
| rs2245282 | 4 | 147297453 | SLC10A7 | intron_var iant |
| rs2262884 | 4 | 147297927 | SLC10A7 | intron_variant |
| rs111925113 | 4 | 147298186 | SLC10A7 | intron_variant |
| 4:147300051:A :AAAGCAGATAATCTGCT | 4 | 147300051 | NA | NA |
| rs2248347 | 4 | 147300898 | SLC10A7 | intron_variant |
| rs2248348 | 4 | 147300931 | SLC10A7 | intron_variant |
| rs2248826 | 4 | 147305421 | SLC10A7 | intron_variant |
| rs2630297 | 4 | 147306646 | SLC10A7 | intron_variant |
| rs2460033 | 4 | 147307479 | SLC10A7 | intron_variant |
| rs138801798 | 4 | 157046897 | NA | |
| rs6834759 | 4 | 158779650 | NA | |
| rs150752209 | 4 | 158783439 | NA | |
| rs17036774 | 4 | 158783852 | NA | |
| rs150456849 | 4 | 158784767 | NA | |
| rs6836401 | 4 | 158786983 | NA | |
| rs6813626 | 4 | 158787102 | NA | |
| rs55817402 | 4 | 174427221 | NA | |
| rs116894613 | 4 | 185063146 | ENPP6 | intron_variant |
| rs140512564 | 4 | 185116348 | ENPP6 | intron_variant |
| rs76233088 | 4 | 185808870 | NA | |
| rs35834666 | 4 | 187138175 | KLKB1 | intron_variant |
| 4:187138442:T:TTTG | 4 | 187138442 | NA | NA |
| rs34852777 | 4 | 187139062 | KLKB1 | intron_variant |
| rs13102931 | 4 | 187145626 | KLKB1 | intron_variant |
| rs13102788 | 4 | 187145738 | KLKB1 | intron_variant |
| rs138695518 | 4 | 187146418 | KLKB1 | intron_variant |

[Table 1-25]

| SNP ID | chr | position | gene locus | SNP location in gene locus |
|---|---|---|---|---|
| rs35669195 | 4 | 187146559 | KLKB1 | intron_variant |
| rs4253241 | 4 | 187149233 | KLKB1 | intron_variant |
| rs4253246 | 4 | 187154424 | KLKB1 | intron_variant |
| rs184644242 | 4 | 187157181 | KLKB1 | intron_variant |
| rs145549717 | 4 | 187157232 | KLKB1 | intron_variant |
| 5:2020945:T:A | 5 | 2020945 | NA | NA |
| rs142127390 | 5 | 11734734 | CTNND2 | intron_variant |
| rs10076098 | 5 | 11737241 | CTNND2 | intron_variant |
| rs57612673 | 5 | 13733453 | DNAH5 | intron_variant |
| 5:14710594:C:G | 5 | 14710594 | NA | NA |
| 5:14722584:T:C | 5 | 14722584 | NA | NA |
| 5:14740327:C:T | 5 | 14740327 | NA | NA |
| rs77818967 | 5 | 19736762 | CDH18 | intron_variant |
| rs12657194 | 5 | 19762895 | CDH18 | intron_variant |
| rs141488363 | 5 | 19785302 | CDH18 | intron_variant |
| rs140741351 | 5 | 19808374 | CDH18 | intron_variant |
| rs34847415 | 5 | 19909533 | CDH18 | intron_variant |
| rs72740870 | 5 | 19910167 | CDH18 | intron_variant |
| rs79509881 | 5 | 24693838 | NA | |
| rs62357273 | 5 | 25683630 | NA | |
| rs111941500 | 5 | 25688951 | NA | |
| rs113538604 | 5 | 25690607 | NA | |
| rs13159231 | 5 | 25691118 | NA | |
| rs148804803 | 5 | 25696038 | NA | |
| rs138213578 | 5 | 25705703 | NA | |
| rs200325656 | 5 | 25706012 | NA | |
| rs35750564 | 5 | 28029472 | NA | |
| rs139552645 | 5 | 28069123 | NA | |
| rs147157201 | 5 | 37104905 | NA | |
| rs301904 | 5 | 37111565 | CPLANE1 | intron_variant |
| 5:37111995:T:TA | 5 | 37111995 | NA | NA |
| rs301894 | 5 | 37127778 | CPLANE1 | intron_variant |
| rs301895 | 5 | 37128315 | CPLANE1 | intron_variant |
| rs12110069 | 5 | 37160914 | CPLANE1 | intron_variant |
| rs10056250 | 5 | 37171884 | CPLANE1 | intron_variant |
| rs75589774 | 5 | 37182902 | CPLANE1 | missense_variant |

[Table 1-26]

| SNP ID | chr | position | gene locus | SNP location in gene locus |
|---|---|---|---|---|
| rs79298055 | 5 | 37202084 | CPLANE1 | intron_variant |
| rs76607706 | 5 | 37892303 | NA | |
| rs111622158 | 5 | 37928055 | NA | |
| rs75661047 | 5 | 41337919 | PLCXD3 | intron_variant |
| rs189501062 | 5 | 41421703 | PLCXD3 | intron_variant |
| rs318065 | 5 | 41442044 | PLCXD3 | intron_variant |
| rs78362058 | 5 | 41448308 | PLCXD3 | intron_variant |
| rs78872736 | 5 | 41449972 | PLCXD3 | intron_variant |
| rs79993711 | 5 | 41451447 | PLCXD3 | intron_variant |
| rs3883955 | 5 | 41455054 | PLCXD3 | intron_variant |
| rs191995881 | 5 | 41456607 | PLCXD3 | intron_variant |
| rs77129467 | 5 | 41459002 | PLCXD3 | intron_variant |
| rs117577517 | 5 | 41460797 | PLCXD3 | intron_variant |
| rs149176711 | 5 | 41460944 | PLCXD3 | intron_variant |
| rs145608729 | 5 | 41462819 | PLCXD3 | intron_variant |
| rs142790831 | 5 | 41462961 | PLCXD3 | intron_variant |
| rs80187604 | 5 | 41464479 | PLCXD3 | intron_variant |
| rs118037167 | 5 | 41465566 | PLCXD3 | intron_variant |
| rs116809573 | 5 | 41469306 | PLCXD3 | intron_variant |
| rs117880348 | 5 | 41471050 | PLCXD3 | intron_variant |
| rs74850462 | 5 | 41473868 | PLCXD3 | intron_variant |
| rs75235442 | 5 | 41474166 | PLCXD3 | intron_variant |
| rs117907903 | 5 | 41474535 | PLCXD3 | intron_variant |
| rs117365372 | 5 | 41474536 | PLCXD3 | intron_variant |
| rs318043 | 5 | 41474553 | PLCXD3 | intron_variant |
| rs318042 | 5 | 41474575 | PLCXD3 | intron_variant |
| rs139821901 | 5 | 41475221 | PLCXD3 | intron_variant |
| rs185789756 | 5 | 41475660 | PLCXD3 | intron_variant |
| rs189018681 | 5 | 41475661 | PLCXD3 | intron_variant |
| rs149824778 | 5 | 41475846 | PLCXD3 | intron_variant |
| rs6451577 | 5 | 41476751 | PLCXD3 | intron_variant |
| rs148818052 | 5 | 41476865 | PLCXD3 | intron_variant |
| rs117592865 | 5 | 41479099 | PLCXD3 | intron_variant |
| rs78415819 | 5 | 41480451 | PLCXD3 | intron_variant |
| rs185514305 | 5 | 41480794 | PLCXD3 | intron_variant |
| rs149877177 | 5 | 41480973 | PLCXD3 | intron_variant |

[Table 1-27]

| SNP ID | chr | position | gene locus | SNP location in gene locus |
|---|---|---|---|---|
| rs144898fi61 | 5 | 41481008 | PLCXD3 | intron_variant |
| 5:41481231:A:G | 5 | 41481231 | NA | NA |
| rs592607 | 5 | 41482461 | PLCXD3 | intron_variant |
| rs76025981 | 5 | 41484106 | PLCXD3 | intron_variant |
| rs147796140 | 5 | 41485734 | PLCXD3 | intron_var iant |
| rs115209137 | 5 | 41485860 | PLCXD3 | intron_variant |
| rs150658476 | 5 | 41487649 | PLCXD3 | intron_variant |
| 5:41492084:G:A | 5 | 41492084 | NA | NA |
| rs318047 | 5 | 41492737 | PLCXD3 | intron variant |
| rs193139535 | 5 | 41494163 | PLCXD3 | intron_variant |
| 5:41496974:CTT:C | 5 | 41496974 | NA | NA |
| 5:41499344:T:TA | 5 | 41499344 | NA | NA |
| rs79200111 | 5 | 41503974 | PLCXD3 | intron_variant |
| rs143005632 | 5 | 41505112 | PLCXD3 | intron_variant |
| rs80129576 | 5 | 41507151 | PLCXD3 | intron_variant |
| rs149750323 | 5 | 41508080 | PLCXD3 | intron_variant |
| rs145378048 | 5 | 41508511 | PLCXD3 | intron_variant |
| rs142480844 | 5 | 41509735 | PLCXD3 | intron_variant |
| 5:41510809:C:T | 5 | 41510809 | NA | NA |
| rs74732406 | 5 | 41511954 | NA | |
| rs77991880 | 5 | 41515898 | NA | |
| rs3863151 | 5 | 41518789 | NA | |
| rs140198935 | 5 | 41521169 | NA | |
| rs140097421 | 5 | 41525867 | NA | |
| rs78523327 | 5 | 41525908 | NA | |
| rs78310877 | 5 | 41528894 | NA | |
| rs79027993 | 5 | 41529340 | NA | |
| rs149621750 | 5 | 41530150 | NA | |
| rs148809865 | 5 | 41530798 | NA | |
| rs142486112 | 5 | 41530854 | NA | |
| 5:41535859:G:A | 5 | 41535859 | NA | NA |
| rs79544594 | 5 | 41538064 | NA | |
| rs78787781 | 5 | 41538772 | NA | |
| rs76663349 | 5 | 41539637 | NA | |
| rs111541941 | 5 | 41541437 | NA | |
| rs77020012 | 5 | 41541725 | NA | |

[Table 1-28]

| SNP ID | chr | position | gene locus | SNP location in gene locus |
|---|---|---|---|---|
| rs79117365 | 5 | 41542625 | NA | |
| 5:41548979:G:A | 5 | 41548979 | NA | NA |
| 5:41566271:G:A | 5 | 41566271 | NA | NA |
| rs142949110 | 5 | 41572513 | NA | |
| 5:41597151:T:C | 5 | 41597151 | NA | NA |
| rs74919030 | 5 | 41611365 | NA | |
| rs137930663 | 5 | 41692962 | NA | |
| 5:41715811:C:T | 5 | 41715811 | NA | NA |
| rs139531587 | 5 | 42148442 | NA | |
| rs79035719 | 5 | 42217448 | NA | |
| rs185613690 | 5 | 42243590 | NA | |
| rs184276051 | 5 | 42257845 | NA | |
| 5:42354014:C:T | 5 | 42354014 | NA | NA |
| rs142622848 | 5 | 42379421 | NA | |
| rs12522729 | 5 | 60541282 | NA | |
| rs74948200 | 5 | 60601182 | NA | |
| rs12522321 | 5 | 60723105 | ZSWIM6 | intron_variant |
| rs79053743 | 5 | 60829488 | ZSWIM6 | intron_variant |
| rs1827387 | 5 | 81930239 | NA | |
| rs74366119 | 5 | 88156454 | MEF2C | intron_variant |
| 5:88795078:G:GAA | 5 | 88795078 | NA | NA |
| rs80106148 | 5 | 92325356 | NA | |
| rs75089614 | 5 | 100358025 | NA | |
| rs28641111 | 5 | 100366992 | NA | |
| rs10072145 | 5 | 100395409 | NA | |
| rs10057008 | 5 | 100395973 | NA | |
| rs10055504 | 5 | 100397154 | NA | |
| rs115735320 | 5 | 100399760 | NA | |
| rs1423681 | 5 | 100404890 | NA | |
| 5:100432544:C:CAT | 5 | 100432544 | NA | NA |
| rs13356491 | 5 | 112378789 | MCC | intron_variant |
| rs6865320 | 5 | 112388791 | MCC | intron_variant |
| 5:124492243:T:C | 5 | 124492243 | NA | NA |
| rs78855508 | 5 | 124557431 | NA | |
| rs73326798 | 5 | 125390012 | NA | |
| rs4373275 | 5 | 125393103 | NA | |

[Table 1-29]

| SNP ID | chr | position | gene locus | SNP location in gene locus |
|---|---|---|---|---|
| 5:125393103:T:G | 5 | 125393103 | NA | NA |
| rs138975964 | 5 | 125393851 | NA | |
| rs60924028 | 5 | 125399302 | NA | |
| rs57885244 | 5 | 125399376 | NA | |
| rs60069077 | 5 | 125399447 | NA | |
| rs57317805 | 5 | 125399676 | NA | |
| rs57908529 | 5 | 125399679 | NA | |
| rs60950409 | 5 | 125400706 | NA | |
| rs73328735 | 5 | 125401914 | NA | |
| rs111944066 | 5 | 125402054 | NA | |
| rs73328738 | 5 | 125402365 | NA | |
| rs58002426 | 5 | 125402709 | NA | |
| rs61572014 | 5 | 125402753 | NA | |
| rs61376002 | 5 | 125402760 | NA | |
| rs57762059 | 5 | 125403219 | NA | |
| rs57282782 | 5 | 125403419 | NA | |
| rs60443581 | 5 | 125403795 | NA | |
| rs73328747 | 5 | 125404393 | NA | |
| rs73328752 | 5 | 125404704 | NA | |
| rs73328754 | 5 | 125405005 | NA | |
| rs73328756 | 5 | 125405014 | NA | |
| rs73328762 | 5 | 125406249 | NA | |
| rs28867125 | 5 | 125408077 | NA | |
| rs141339888 | 5 | 125408665 | NA | |
| rs73328767 | 5 | 125408969 | NA | |
| rs74968976 | 5 | 125410229 | NA | |
| rs6869320 | 5 | 125410380 | NA | |
| rs6870176 | 5 | 125410856 | NA | |
| rs77250321 | 5 | 125412256 | NA | |
| rs73328774 | 5 | 125412457 | NA | |
| 5:143430342:GTTGT:G | 5 | 143430342 | NA | NA |
| rs76612072 | 5 | 147183847 | NA | |
| rs11954595 | 5 | 147194146 | NA | |
| rs78681108 | 5 | 147232262 | NA | |
| rs116969221 | 5 | 151269893 | GLRA1 | intron_variant |
| 5:153395900:GA:G | 5 | 153395900 | NA | NA |

32

[Table 1-30]

| SNP ID | chr | position | gene locus | SNP location in gene locus |
|---|---|---|---|---|
| 5:153395903:A:G | 5 | 153395903 | NA | NA |
| rs141072734 | 5 | 163003513 | NA | |
| rs79560759 | 5 | 163021709 | NA | |
| rs60283376 | 5 | 166904236 | TENM2 | intron_variant |
| rs77246061 | 5 | 166906243 | TENM2 | intron_variant |
| rs79999983 | 5 | 166910600 | TENM2 | intron_variant |
| rs79072342 | 5 | 166910635 | TENM2 | intron_variant |
| rs117190309 | 5 | 166910664 | TENM2 | intron_variant |
| rs140266055 | 5 | 166910826 | TENM2 | intron_variant |
| rs79374298 | 5 | 166911354 | TENM2 | intron_variant |
| rs117911019 | 5 | 166913316 | TENM2 | intron_var iant |
| rs59932109 | 5 | 166919170 | TENM2 | intron_variant |
| rs1445795 | 5 | 172839988 | NA | |
| rs10463029 | 5 | 172842872 | NA | |
| rs11134795 | 5 | 172843707 | NA | |
| rs11134796 | 5 | 172844177 | NA | |
| rs13357412 | 5 | 172844784 | NA | |
| rs4867711 | 5 | 172847035 | NA | |
| rs115721274 | 5 | 172858622 | NA | |
| rs190979351 | 5 | 173034050 | NA | |
| rs75505838 | 5 | 173111885 | NA | |
| rs117041609 | 5 | 173234139 | NA | |
| rs75159910 | 5 | 173282893 | NA | |
| rs149874863 | 5 | 173855117 | NA | |
| rs72811273 | 5 | 176226113 | NA | |
| rs7720553 | 5 | 179623637 | RASGEF1C | intron_variant |
| rs60316563 | 6 | 6368046 | NA | |
| rs57686849 | 6 | 6368154 | NA | |
| rs141864440 | 6 | 8237754 | NA | |
| rs9349929 | 6 | 14046145 | NA | |
| rs6915882 | 6 | 14046411 | NA | |
| rs2840257 | 6 | 14048680 | NA | |
| rs909709 | 6 | 14049882 | NA | |
| 6:14051468:TAC:T | 6 | 14051468 | NA | NA |
| 6:14051468:TACAC:T | 6 | 14051468 | NA | NA |
| rs1928638 | 6 | 14057157 | NA | |

[Table 1-31]

| SNP ID | chr | position | gene locus | SNP location in gene locus |
|---|---|---|---|---|
| rs1928639 | 6 | 14057161 | NA | |
| rs57463781 | 6 | 14057622 | NA | |
| rs12208529 | 6 | 14063790 | NA | |
| rs9476476 | 6 | 14064273 | NA | |
| rs72836088 | 6 | 19842560 | NA | |
| rs115079901 | 6 | 29558364 | NA | NA |
| rs200911534 | 6 | 30741569 | NA | |
| 6:30744985:G:T | 6 | 30744985 | NA | NA |
| rs191987001 | 6 | 30775579 | NA | NA |
| rs2499716 | 6 | 34073338 | GRM4 | intron_variant |
| rs1873248 | 6 | 34073344 | GRM4 | intron_variant |
| rs139096142 | 6 | 44147011 | CAPN11 | intron_variant |
| rs78401606 | 6 | 70051582 | ADGRB3 | intron_variant |
| rs144698880 | 6 | 70207289 | NA | |
| 6:70210202:G:A | 6 | 70210202 | NA | NA |
| rs11757257 | 6 | 70217452 | NA | |
| rs13205898 | 6 | 70271095 | NA | |
| rs79873641 | 6 | 71389721 | SMAP1 | intron_variant |
| rs145666404 | 6 | 71513933 | SMAP1 | intron_variant |
| rs78049725 | 6 | 75836785 | COL12A1 | intron_variant |
| rs77854307 | 6 | 82472695 | NA | |
| rs79069596 | 6 | 82806731 | NA | |
| rs77125903 | 6 | 82807806 | NA | |
| rs11962fi48 | 6 | 82824374 | NA | |
| rs6914737 | 6 | 82824575 | NA | |
| rs201137924 | 6 | 82824635 | NA | |
| rs77793849 | 6 | 82825638 | NA | |
| rs75421360 | 6 | 82826545 | NA | |
| rs74572694 | 6 | 82831444 | NA | |
| rs11965072 | 6 | 82832842 | NA | |
| rs11755112 | 6 | 82833728 | NA | |
| rs36156078 | 6 | 82838594 | NA | |
| rs2152842 | 6 | 82841134 | NA | |
| rs11967918 | 6 | 82842822 | NA | |
| rs10943858 | 6 | 82845197 | NA | |
| rs79009267 | 6 | 82846023 | NA | |

[Table 1-32]

| SNP ID | chr | position | gene locus | SNP location in gene locus |
|---|---|---|---|---|
| rs11961436 | 6 | 82847140 | NA | |
| rs11752161 | 6 | 82848130 | NA | |
| rs11752130 | 6 | 82848159 | NA | |
| rs77937699 | 6 | 82880897 | IBTK | 3_prime_UTR_variant |
| rs201520516 | 6 | 82965363 | NA | |
| rs76325080 | 6 | 84248518 | NA | |
| rs78628697 | 6 | 86590415 | NA | |
| rs58725789 | 6 | 86600143 | NA | |
| 6:91762389:C:A | 6 | 91762389 | NA | NA |
| rs112761794 | 6 | 105688813 | NA | |
| rs141339608 | 6 | 105688876 | NA | NA |
| rs72936201 | 6 | 105691598 | NA | |
| rs111427560 | 6 | 105692447 | NA | |
| rs183560286 | 6 | 105692807 | NA | NA |
| rs1429383Q3 | 6 | 105695489 | NA | |
| 6:105697055:G:T | 6 | 105697055 | NA | NA |
| rs35695447 | 6 | 105699862 | NA | |
| rs111612241 | 6 | 105703217 | NA | |
| rs17065682 | 6 | 105704149 | NA | |
| rs17065694 | 6 | 105704658 | NA | |
| rs10499042 | 6 | 105706581 | NA | |
| rs111857724 | 6 | 105707260 | NA | |
| rs113487540 | 6 | 105707498 | NA | |
| rs72938014 | 6 | 105707927 | NA | |
| rs17054938 | 6 | 105709113 | NA | |
| rs72938025 | 6 | 105713764 | NA | |
| rs75718226 | 6 | 105715441 | NA | |
| rs72938030 | 6 | 105716807 | NA | |
| rs72938031 | 6 | 105716902 | NA | |
| rs72938040 | 6 | 105721926 | PREP | 3_prime_UTR_variant |
| rs17054949 | 6 | 105721969 | PREP | 3_prime_UTR_variant |
| rs111772455 | 6 | 105722560 | PREP | 3_prime_UTR_variant |
| rs17467544 | 6 | 105722943 | PREP | 3_prime_UTR_variant |
| rs41285466 | 6 | 105726874 | PREP | intron_variant |
| rs72944139 | 6 | 105727873 | PREP | intron_variant |
| rs72944142 | 6 | 105728191 | PREP | intron_variant |

[Table 1-33]

| SNP ID | chr | position | gene locus | SNP location in gene locus |
|---|---|---|---|---|
| rs77676511 | 6 | 105729054 | PREP | intron_variant |
| rs2236285 | 6 | 105730619 | PREP | intron_variant |
| rs3800001 | 6 | 105735808 | PREP | intron_variant |
| rs17546638 | 6 | 105738261 | PREP | intron_variant |
| rs17546735 | 6 | 105741586 | PREP | intron_variant |
| rs17468237 | 6 | 105742660 | PREP | intron_variant |
| rs17068024 | 6 | 107484367 | PDSS2 | intron_variant |
| rs145412027 | 6 | 107493416 | PDSS2 | intron_variant |
| rs75692260 | 6 | 107498607 | PDSS2 | intron_variant |
| rs147212820 | 6 | 107504762 | PDSS2 | intron_variant |
| rs79713062 | 6 | 110875473 | NA | |
| rs12195889 | 6 | 129623880 | LAMA2 | intron_variant |
| rs2451682 | 6 | 129625432 | LAMA2 | intron_variant |
| rs12200836 | 6 | 129640745 | LAMA2 | intron_variant |
| rs12200538 | 6 | 129646004 | LAMA2 | intron_variant |
| rs2451679 | 6 | 129660222 | LAMA2 | i ntron variant |
| rs77026545 | 6 | 137559310 | NA | |
| rs117451378 | 6 | 137570062 | NA | |
| rs57516822 | 6 | 137739295 | NA | |
| rs59391960 | 6 | 137739389 | NA | |
| rs77091814 | 6 | 137742839 | NA | |
| rs73566001 | 6 | 137744363 | NA | |
| rs7769446 | 6 | 137745965 | NA | |
| rs71811814 | 6 | 137746238 | NA | NA |
| rs6917588 | 6 | 137746860 | NA | |
| rs1335302 | 6 | 141637219 | NA | |
| rs1335308 | 6 | 141645736 | NA | |
| rs4270804 | 6 | 141652711 | NA | |
| rs9376607 | 6 | 141661460 | NA | |
| rs9403288 | 6 | 141672901 | NA | |
| rs9399363 | 6 | 141682404 | NA | |
| rs9373305 | 6 | 141683547 | NA | |
| rs11760135 | 6 | 141685630 | NA | |
| 6:141689730:AT:A | 6 | 141689730 | NA | NA |
| rs9376611 | 6 | 141692633 | NA | |
| rs11754010 | 6 | 141700708 | NA | |

[Table 1-34]

| SNP ID | chr | position | gene locus | SNP location in gene locus |
|---|---|---|---|---|
| rs55968168 | 6 | 141703453 | NA | |
| rs9403300 | 6 | 141706902 | NA | |
| 6:141712104:G:A | 6 | 141712104 | NA | NA |
| rs73779399 | 6 | 145196065 | NA | |
| rs55645496 | 6 | 145197134 | NA | |
| rs73779401 | 6 | 145199490 | NA | |
| rs73781203 | 6 | 145200275 | NA | |
| rs73781205 | 6 | 145201088 | NA | |
| rs73781206 | 6 | 145201247 | NA | |
| rs6940222 | 6 | 145203608 | NA | |
| rs73781208 | 6 | 145205435 | NA | |
| rs57049867 | 6 | 145205680 | NA | |
| rs73781214 | 6 | 145210203 | NA | |
| rs62436288 | 6 | 147952729 | SAMD5 | intron_variant |
| rs2064333 | 6 | 147953574 | SAMD5 | intron_variant |
| rs17388532 | 6 | 147956771 | SAMD5 | intron_variant |
| rs7753842 | 6 | 148553550 | NA | |
| rs1871921 | 6 | 149221294 | UST | intron_variant |
| rs17665064 | 6 | 149226781 | UST | intron_variant |
| rs7760563 | 6 | 149232496 | UST | intron_variant |
| rs72994230 | 6 | 149236366 | UST | intron_variant |
| rs112183061 | 6 | 149236666 | UST | intron_variant |
| 6:149269058:G:GA | 6 | 149269058 | NA | NA |
| 6:149269058:G:GAA | 6 | 149269058 | NA | NA |
| rs117816679 | 6 | 150790648 | NA | |
| rs147175280 | 6 | 150793218 | NA | |
| rs146914832 | 6 | 150795155 | NA | |
| 6:150796988:G:T | 6 | 150796988 | NA | NA |
| rs148080540 | 6 | 150806519 | NA | |
| rs117959775 | 6 | 150809802 | NA | |
| rs111474682 | 6 | 150811773 | NA | |
| rs77886049 | 6 | 156030889 | NA | |
| rs9397273 | 6 | 156248029 | NA | |
| rs4370375 | 6 | 156250340 | NA | |
| rs7756499 | 6 | 156250986 | NA | |
| rs9384398 | 6 | 156252277 | NA | |

[Table 1-35]

| SNP ID | chr | position | gene locus | SNP location in gene locus |
|---|---|---|---|---|
| rs9397274 | 6 | 156253640 | NA | |
| rs7740419 | 6 | 156255416 | NA | |
| rs17086886 | 6 | 156261635 | NA | |
| rs74781715 | 6 | 156271293 | NA | |
| rs9397864 | 6 | 156271539 | NA | |
| rs4259259 | 6 | 156272383 | NA | |
| rs9397865 | 6 | 156272671 | NA | |
| rs9397866 | 6 | 156272681 | NA | |
| rs12111075 | 6 | 162304205 | PRKN | intron_variant |
| rs12110765 | 6 | 162304245 | PRKN | intron_variant |
| rs58436306 | 6 | 170758245 | NA | |
| rs7750857 | 6 | 170760133 | NA | |
| rs140838906 | 6 | 170761583 | NA | |
| 7:4230856:G:A | 7 | 4230856 | NA | NA |
| rs201457651 | 7 | 9092066 | NA | |
| rs148328240 | 7 | 9093559 | NA | |
| rs10265621 | 7 | 9099399 | NA | |
| rs76355504 | 7 | 9313494 | NA | |
| rs58749480 | 7 | 13292034 | NA | |
| rs139024590 | 7 | 17010996 | NA | NA |
| rs11980801 | 7 | 22290160 | RAPGEF5 | intron_variant |
| rs2686469 | 7 | 22299059 | RAPGEF5 | intron_variant |
| 7:22888700:CAA:CTTAA | 7 | 22888700 | NA | NA |
| rs182887339 | 7 | 22895543 | NA | |
| rs80009912 | 7 | 22920084 | NA | |
| rs147497510 | 7 | 28699881 | CREB5 | intron_variant |
| rs73685962 | 7 | 30292368 | NA | |
| rs6958640 | 7 | 30293612 | NA | |
| rs114684317 | 7 | 30294944 | NA | |
| rs149057499 | 7 | 30296625 | NA | |
| rs6970146 | 7 | 30297969 | NA | |
| rs77413551 | 7 | 30301817 | NA | |
| rs201581869 | 7 | 30303728 | NA | |
| 7:30303731:GCACTCCTCCC:G | 7 | 30303731 | NA | NA |
| 7:30303742:GC:G | 7 | 30303742 | NA | NA |

[Table 1-36]

| SNP ID | chr | position | gene locus | SNP location in gene locus |
|---|---|---|---|---|
| rs149622189 | 7 | 30307465 | NA | |
| rs200550125 | 7 | 30311765 | NA | |
| 7:30319553:TA:T | 7 | 30319553 | NA | NA |
| rs114645450 | 7 | 30321650 | NA | |
| rs79699598 | 7 | 30322482 | NA | |
| 7:30324192:C:CG | 7 | 30324192 | NA | NA |
| rs143478611 | 7 | 30327096 | ZNRF2 | intron_variant |
| rs6970041 | 7 | 30332914 | ZNRF2 | intron_variant |
| rs17158839 | 7 | 30335403 | ZNRF2 | intron_variant |
| rs148707872 | 7 | 30336156 | ZNRF2 | intron_variant |
| rs115884980 | 7 | 30336503 | ZNRF2 | intron_variant |
| rs140607084 | 7 | 30336572 | ZNRF2 | intron_variant |
| rs147812016 | 7 | 30336672 | ZNRF2 | intron_variant |
| rs116610215 | 7 | 30338896 | ZNRF2 | intron_variant |
| rs74501594 | 7 | 30339220 | ZNRF2 | intron_variant |
| rs6968097 | 7 | 30344956 | ZNRF2 | intron_variant |
| 7:30346948:T:G | 7 | 30346948 | NA | NA |
| rs8180762 | 7 | 30348387 | ZNRF2 | intron_variant |
| rs73685995 | 7 | 30349511 | ZNRF2 | intron_variant |
| rs9639614 | 7 | 30350328 | ZNRF2 | intron_variant |
| rs7804225 | 7 | 30350755 | ZNRF2 | intron_variant |
| rs34458739 | 7 | 30351379 | ZNRF2 | intron_variant |
| 7:30355351:G:C | 7 | 30355351 | NA | NA |
| rs112527213 | 7 | 30358471 | ZNRF2 | intron_variant |
| rs6959139 | 7 | 30360832 | ZNRF2 | intron_variant |
| rs78301492 | 7 | 30362276 | ZNRF2 | intron_variant |
| rs140099650 | 7 | 30362853 | ZNRF2 | intron_variant |
| rs114690145 | 7 | 30364225 | ZNRF2 | intron_variant |
| rs6943601 | 7 | 30365310 | ZNRF2 | intron_variant |
| rs77946931 | 7 | 30365824 | ZNRF2 | intron_variant |
| rs115112447 | 7 | 30365833 | ZNRF2 | intron_variant |
| rs12056300 | 7 | 30367221 | ZNRF2 | intron_variant |
| rs17158851 | 7 | 30368265 | ZNRF2 | intron_variant |
| rs56694867 | 7 | 30368384 | ZNRF2 | intron_variant |
| rs12056131 | 7 | 30369299 | ZNRF2 | intron_variant |
| rs6979671 | 7 | 30370051 | ZNRF2 | intron_variant |

[Table 1-37]

| SNP ID | chr | position | gene locus | SNP location in gene locus |
|---|---|---|---|---|
| rs11974360 | 7 | 30370786 | ZNRF2 | intron_variant |
| rs76302490 | 7 | 30373774 | ZNRF2 | intron_variant |
| rs80280885 | 7 | 30373940 | ZNRF2 | intron_variant |
| rs145282100 | 7 | 30375239 | ZNRF2 | intron_variant |
| rs9691527 | 7 | 30376086 | ZNRF2 | intron_variant |
| rs11979006 | 7 | 30381303 | ZNRF2 | intron_variant |
| 7:30383851:CT:C | 7 | 30383851 | NA | NA |
| rs111250775 | 7 | 30384822 | ZNRF2 | intron_variant |
| rs80107665 | 7 | 30387141 | ZNRF2 | intron_variant |
| rs146758046 | 7 | 30387248 | ZNRF2 | intron_variant |
| rs17158885 | 7 | 30389865 | ZNRF2 | intron_variant |
| rs17158888 | 7 | 30391066 | ZNRF2 | intron_variant |
| rs11531494 | 7 | 30392422 | ZNRF2 | intron_variant |
| rs42580 | 7 | 30397028 | ZNRF2 | intron_variant |
| rs42589 | 7 | 30402571 | AC006978. 2 | intron_variant |
| rs73695251 | 7 | 39331345 | POU6F2 | NMD_transcript_variant |
| rs73695253 | 7 | 39333216 | POU6F2 | NMD_transcript_variant |
| rs117797113 | 7 | 42239771 | GLI3 | intron_variant |
| rs1525251 | 7 | 46738745 | NA | |
| 7:46949185:A:ACCCCC | 7 | 46949185 | NA | NA |
| rs12673320 | 7 | 46955408 | NA | |
| rs58346043 | 7 | 46957249 | NA | |
| rs55968856 | 7 | 46963997 | NA | |
| 7:46964575:GT:G | 7 | 46964575 | NA | NA |
| rs115849624 | 7 | 46964743 | NA | NA |
| rs13224225 | 7 | 46966997 | NA | |
| rs10951882 | 7 | 46967335 | NA | |
| rs57132170 | 7 | 46968669 | NA | |
| rs16881424 | 7 | 46973649 | NA | |
| rs117604188 | 7 | 52054590 | NA | |
| rs146024011 | 7 | 52061279 | NA | |
| rs114085512 | 7 | 52062875 | NA | |
| rs117563572 | 7 | 52067629 | NA | |
| rs138791169 | 7 | 52068597 | NA | |
| rs139032318 | 7 | 52071591 | NA | |
| rs78326066 | 7 | 52146162 | NA | |

[Table 1-38]

| SNP ID | chr | position | gene locus | SNP location in gene locus |
|---|---|---|---|---|
| rs77343772 | 7 | 73408830 | NA | |
| rs6972672 | 7 | 73409861 | NA | |
| rs6973358 | 7 | 73410290 | NA | |
| rs6973541 | 7 | 73410408 | NA | |
| 7:75438562:T:TTTTTTA | 7 | 75438562 | NA | NA |
| rs76364545 | 7 | 75440752 | NA | |
| rs11465296 | 7 | 75442294 | CCL24 | intron_variant |
| rs11465293 | 7 | 75442723 | CCL24 | missense_variant |
| rs11465286 | 7 | 75443961 | CCL24 | intron variant |
| rs79404066 | 7 | 75444215 | CCL24 | intron_variant |
| rs117679282 | 7 | 75446052 | CCL24 | intron_variant |
| rs11514980 | 7 | 75459923 | NA | |
| rs11521067 | 7 | 75459954 | NA | |
| rs147465974 | 7 | 75468367 | NA | |
| 7:76910430:C:CA | 7 | 76910430 | NA | NA |
| 7:101289699:G:A | 7 | 101289699 | NA | NA |
| rs145263975 | 7 | 128449126 | CCDC136 | intron variant |
| rs189900162 | 7 | 128453455 | CCDC136 | intron variant |
| rs149344851 | 7 | 128455033 | CCDC136 | intron_variant |
| rs143205471 | 7 | 128464165 | NA | |
| rs147658468 | 7 | 128465079 | NA | |
| rs141683491 | 7 | 128469854 | NA | |
| rs199842696 | 7 | 128470680 | FLNC | 5_prime_UTR_variant |
| rs151072488 | 7 | 128473938 | FLNC | intron_variant |
| rs138755199 | 7 | 128475236 | FLNC | intron_variant |
| rs144736830 | 7 | 128479080 | FLNC | intron_variant |
| rs60691847 | 7 | 128499388 | NA | |
| rs73459264 | 7 | 128505510 | KCP | intron_variant |
| rs183994302 | 7 | 128513368 | KCP | intron_variant |
| rs112749538 | 7 | 128513415 | KCP | intron variant |
| rs182514276 | 7 | 128514838 | KCP | intron_variant |
| 7:128522809:AAAAC:A | 7 | 128522809 | NA | NA |
| 7:128522809:AAAAGAAACAAAC :AAAAC | 7 | 128522809 | NA | NA |
| rs143202507 | 7 | 128523082 | KCP | intron_variant |
| rs111838117 | 7 | 128524190 | KCP | intron_variant |

[Table 1-39]

| SNP ID | chr | position | gene locus | SNP location in gene locus |
|---|---|---|---|---|
| rs144312682 | 7 | 128526221 | KCP | intron_variant |
| 7:131372591:A:G | 7 | 131372591 | NA | NA |
| rs137956592 | 7 | 138063857 | NA | |
| rs186944015 | 7 | 141792526 | MGAM | intron variant |
| 7:148274173:T:C | 7 | 148274173 | NA | NA |
| rs140478856 | 7 | 148374761 | NA | |
| rs77070865 | 7 | 148384615 | NA | |
| rs35116518 | 7 | 148386436 | NA | |
| rs114140823 | 7 | 148386467 | NA | |
| rs34369394 | 7 | 148388377 | NA | |
| rs10235843 | 7 | 148400466 | CUL1 | intron_variant |
| 7:148422315:A:G | 7 | 148422315 | NA | NA |
| rs11974639 | 7 | 148438804 | CUL1 | intron_variant |
| rs147617254 | 7 | 148458807 | CUL1 | intron_variant |
| rs17626232 | 7 | 148459675 | CUL1 | intron_variant |
| rs10246773 | 7 | 148460536 | CUL1 | intron_variant |
| rs3807446 | 7 | 148467447 | CUL1 | intron_variant |
| rs758880 | 7 | 148477589 | CUL1 | intron_variant |
| rs113817468 | 7 | 148478201 | CUL1 | intron_variant |
| rs10245290 | 7 | 148530294 | EZH2 | intron_variant |
| rs73158265 | 7 | 148537086 | EZH2 | intron_variant |
| rs56248471 | 7 | 148553810 | EZH2 | intron_variant |
| rs10952780 | 7 | 148554335 | EZH2 | intron_variant |
| rs113910590 | 7 | 148554794 | EZH2 | intron_variant |
| 7:148558320:CT:C | 7 | 148558320 | NA | NA |
| rs10952783 | 7 | 148559071 | EZH2 | intron_variant |
| 7:148560015:G:GTTTTGT | 7 | 148560015 | NA | NA |
| rs28706086 | 7 | 148560068 | EZH2 | intron_variant |
| rs10259102 | 7 | 148561029 | EZH2 | intron_variant |
| rs10249392 | 7 | 148561324 | EZH2 | intron_variant |
| rs11976154 | 7 | 148561586 | EZH2 | intron_variant |
| rs28617605 | 7 | 148562161 | EZH2 | intron_variant |
| 7:148562217:CAAA:C | 7 | 148562217 | NA | NA |
| rs1996996 | 7 | 148562543 | EZH2 | intron_variant |
| rs73158280 | 7 | 148563393 | EZH2 | intron_variant |

[Table 1-40]

| SNP ID | chr | position | gene locus | SNP location in gene locus |
|---|---|---|---|---|
| rs142648914 | 7 | 148563610 | EZH2 | intron_variant |
| rs142248416 | 7 | 148563611 | EZH2 | intron_variant |
| rs111880657 | 7 | 148564058 | EZH2 | intron_variant |
| rs113161209 | 7 | 148564367 | EZH2 | intron_variant |
| rs73158281 | 7 | 148565649 | EZH2 | intron_variant |
| rs149022366 | 7 | 148566024 | EZH2 | intron_variant |
| rs1917057 | 7 | 148567957 | EZH2 | intron_variant |
| rs2373415 | 7 | 148570280 | EZH2 | intron_variant |
| rs2373416 | 7 | 148570344 | EZH2 | intron_variant |
| rs2373417 | 7 | 148570382 | EZH2 | intron_variant |
| rs2373418 | 7 | 148570447 | EZH2 | intron_variant |
| rs7458365 | 7 | 148570551 | EZH2 | intron variant |
| rs28455679 | 7 | 148570741 | EZH2 | intron_variant |
| rs6972079 | 7 | 148571196 | EZH2 | intron_variant |
| rs6963116 | 7 | 148571393 | EZH2 | intron_variant |
| rs28814083 | 7 | 148573027 | EZH2 | intron_variant |
| rs28844638 | 7 | 148573061 | EZH2 | intron_variant |
| rs12531383 | 7 | 148573311 | AC073140.1 | non_coding_transcript_exon_variant |
| 7:148573384:G:GA | 7 | 148573384 | NA | NA |
| rs149343167 | 7 | 148573692 | NA | NA |
| rs7783459 | 7 | 148573898 | EZH2 | intron_variant |
| rs7795158 | 7 | 148576193 | EZH2 | intron_variant |
| rs1880357 | 7 | 148577610 | EZH2 | intron_variant |
| rs2177567 | 7 | 148578753 | EZH2 | intron_variant |
| rs80052686 | 7 | 148580809 | EZH2 | intron_variant |
| rs11984309 | 7 | 148589869 | NA | |
| rs67648693 | 7 | 148591911 | NA | |
| 7:148595511:C:CA | 7 | 148595511 | NA | NA |
| 7:148596986:C:CT | 7 | 148596986 | NA | NA |
| rs202221022 | 7 | 148597557 | NA | NA |
| rs7790642 | 7 | 148600310 | NA | |
| rs7777746 | 7 | 148602583 | NA | |
| rs62505409 | 7 | 148602689 | NA | |
| rs58371016 | 7 | 148604818 | NA | |
| rs6558814 | 8 | 3589281 | CSMD1 | intron_variant |
| rs79572000 | 8 | 3594099 | CSMD1 | intron_variant |

[Table 1-41]

| SNP ID | chr | position | gene locus | SNP location in gene locus |
|---|---|---|---|---|
| rs79727403 | 8 | 3595476 | CSMD1 | intron_variant |
| rs78364798 | 8 | 3596451 | CSMD1 | intron_variant |
| rs140360037 | 8 | 3596652 | CSMD1 | intron_variant |
| rs17067299 | 8 | 3599207 | CSMD1 | intron_variant |
| rs74706909 | 8 | 3600132 | CSMD1 | intron_variant |
| rs74985428 | 8 | 3600451 | CSMD1 | intron_variant |
| rs12549613 | 8 | 3601137 | CSMD1 | intron_variant |
| rs79210493 | 8 | 3602245 | CSMD1 | intron_variant |
| rs4639541 | 8 | 3604375 | CSMD1 | intron_variant |
| rs4424283 | 8 | 3605823 | CSMD1 | intron_variant |
| rs78615686 | 8 | 4795722 | CSMD1 | intron_variant |
| rs75778595 | 8 | 4801168 | CSMD1 | intron_variant |
| rs6558944 | 8 | 4802163 | CSMD1 | intron_variant |
| rs75096153 | 8 | 4807509 | CSMD1 | intron_variant |
| rs80229469 | 8 | 4823427 | CSMD1 | intron_variant |
| rs75338858 | 8 | 4825118 | CSMD1 | intron_variant |
| rs76891224 | 8 | 4827394 | CSMD1 | intron_variant |
| rs3911307 | 8 | 4839914 | CSMD1 | intron_variant |
| rs77772200 | 8 | 4840914 | CSMD1 | intron_variant |
| rs73497791 | 8 | 4842344 | CSMD1 | intron_variant |
| rs74350143 | 8 | 4842467 | CSMD1 | intron_variant |
| rs17071982 | 8 | 4842970 | CSMD1 | intron_variant |
| rs6999675 | 8 | 4844827 | CSMD1 | intron_variant |
| rs7013059 | 8 | 4845003 | CSMD1 | intron_variant |
| rs145241498 | 8 | 4854469 | NA | |
| rs78561947 | 8 | 4856358 | NA | |
| rs79998369 | 8 | 4858392 | NA | |
| rs75281977 | 8 | 4863064 | NA | |
| rs74639771 | 8 | 4870975 | NA | |
| rs77504238 | 8 | 5030143 | NA | |
| rs73219702 | 8 | 13552293 | NA | |
| rs79671431 | 8 | 15284873 | TUSC3 | non_coding_transcript_variant |
| rs12155706 | 8 | 16684041 | NA | |
| rs12155708 | 8 | 16684111 | NA | |
| rs56340335 | 8 | 16685007 | NA | |
| rs73201722 | 8 | 16687313 | NA | |

[Table 1-42]

| SNP ID | chr | position | gene locus | SNP location in gene locus |
|---|---|---|---|---|
| rs73201728 | 8 | 16688532 | NA | |
| rs73201732 | 8 | 16689879 | NA | |
| rs73201733 | 8 | 16689886 | NA | |
| rs4922106 | 8 | 19691250 | INTS10 | intron_variant |
| 8:19691517:C:CA | 8 | 19691517 | NA | NA |
| rs4922107 | 8 | 19691603 | INTS10 | intron_variant |
| rs140015982 | 8 | 26335752 | BNIP3L | intron_variant |
| rs112411185 | 8 | 53110658 | ST18 | intron_variant |
| rs62509183 | 8 | 64209694 | NA | |
| rs147990281 | 8 | 65206514 | NA | |
| 8:65811756:TC:T | 8 | 65811756 | NA | NA |
| rs147627421 | 8 | 72783939 | NA | |
| rs147840558 | 8 | 72968161 | TRPA1 | intron_variant |
| rs142352715 | 8 | 72968759 | TRPA1 | intron_variant |
| rs141156682 | 8 | 72970760 | TRPA1 | intron_variant |
| rs2587560 | 8 | 72990136 | NA | |
| rs2587563 | 8 | 72991997 | NA | |
| rs2587564 | 8 | 72992052 | NA | |
| rs2587565 | 8 | 72992580 | NA | |
| rs1443951 | 8 | 72993388 | NA | |
| rs2587568 | 8 | 72994349 | NA | |
| rs2587569 | 8 | 72994434 | NA | |
| rs2701457 | 8 | 72995307 | NA | |
| rs2587572 | 8 | 72995403 | NA | |
| rs1838286 | 8 | 72995797 | NA | |
| rs2587574 | 8 | 72996845 | NA | |
| rs2587575 | 8 | 72997364 | NA | |
| rs1899762 | 8 | 72997899 | NA | |
| rs1899761 | 8 | 72998278 | NA | |
| rs2701455 | 8 | 72998638 | NA | |
| rs13274010 | 8 | 73000500 | NA | |
| rs59359973 | 8 | 73001147 | NA | |
| rs1443950 | 8 | 73001565 | NA | |
| rs1443949 | 8 | 73001624 | NA | |
| rs10504528 | 8 | 73001662 | NA | |
| rs10504529 | 8 | 73001833 | NA | |

[Table 1-43]

| SNP ID | chr | position | gene locus | SNP location in gene locus |
|---|---|---|---|---|
| rs11995530 | 8 | 73002151 | NA | |
| rs1443947 | 8 | 73002691 | NA | |
| rs34722660 | 8 | 73003389 | NA | |
| rs16938000 | 8 | 73003527 | NA | |
| rs34257316 | 8 | 73003708 | NA | |
| rs34109030 | 8 | 73003720 | NA | |
| rs2383847 | 8 | 73004294 | NA | |
| rs2383848 | 8 | 73004297 | NA | |
| rs2383850 | 8 | 73004347 | NA | |
| rs2383851 | 8 | 73004372 | NA | |
| rs11444287 | 8 | 73004443 | NA | |
| rs2120468 | 8 | 73005062 | NA | |
| rs2028707 | 8 | 73005371 | NA | |
| rs35398772 | 8 | 73016364 | NA | |
| rs13276665 | 8 | 73016825 | NA | |
| rs17815050 | 8 | 73017580 | NA | |
| rs13278765 | 8 | 73018900 | NA | |
| rs4601363 | 8 | 73024793 | NA | |
| rs35873044 | 8 | 73025944 | NA | |
| rs13267160 | 8 | 73030409 | NA | |
| rs28429254 | 8 | 76012207 | NA | |
| rs17370342 | 8 | 76012812 | NA | |
| rs10504590 | 8 | 76013464 | NA | |
| rs28450580 | 8 | 76014941 | NA | |
| rs142904843 | 8 | 89944036 | NA | |
| rs76655252 | 8 | 89952367 | NA | |
| rs114639281 | 8 | 90009741 | NA | |
| 8:101583921:A:C | 8 | 101583921 | NA | NA |
| rs184447468 | 8 | 101800848 | NA | |
| rs143939515 | 8 | 103149542 | NA | |
| rs6993195 | 8 | 103164396 | NA | |
| rs142025872 | 8 | 103169327 | NA | |
| rs56191596 | 8 | 103177900 | NA | |
| rs33954588 | 8 | 106008738 | ZFPM2 | non_coding_transcript_variant |
| rs1879688 | 8 | 106010200 | ZFPM2 | non_coding_transcript_variant |
| 8:106025397:TAAA:T | 8 | 106025397 | NA | NA |

[Table 1-44]

| SNP ID | chr | position | gene locus | SNP location in gene locus |
|---|---|---|---|---|
| rs6984262 | 8 | 106030373 | ZFPM2 | non_coding_transcript_variant |
| rs73304184 | 8 | 106537180 | ZFPM2 | intron_variant |
| rs76868435 | 8 | 106537986 | ZFPM2 | intron_variant |
| rs79001083 | 8 | 106538183 | ZFPM2 | intron_variant |
| rs73304192 | 8 | 106540300 | ZFPM2 | intron_variant |
| rs74395542 | 8 | 106544183 | ZFPM2 | intron_variant |
| 8:106545091:GTT:GT | 8 | 106545091 | NA | NA |
| 8:106545091:GTT:G | 8 | 106545091 | NA | NA |
| rs73306213 | 8 | 106550015 | ZFPM2 | intron_variant |
| rs73306228 | 8 | 106556176 | ZFPM2 | intron_variant |
| rs73306230 | 8 | 106558263 | ZFPM2 | intron_variant |
| rs73306231 | 8 | 106559352 | ZFPM2 | intron_variant |
| rs7844467 | 8 | 106562252 | ZFPM2 | intron_variant |
| rs34823616 | 8 | 106566606 | ZFPM2 | intron_variant |
| 8:108811752:T:C | 8 | 108811752 | NA | NA |
| 8:108849027:T: A | 8 | 108849027 | NA | NA |
| 8:108850008:G:A | 8 | 108850008 | NA | NA |
| 8:108850585:G:A | 8 | 108850585 | NA | NA |
| rs115268316 | 8 | 108863551 | NA | |
| rs57284855 | 8 | 108865154 | NA | |
| 8:108870725:G:A | 8 | 108870725 | NA | NA |
| rs149805601 | 8 | 108872570 | NA | |
| rs13269427 | 8 | 108873161 | NA | |
| rs186061014 | 8 | 108889120 | NA | |
| 8:108896855:G:A | 8 | 108896855 | NA | NA |
| rs146645406 | 8 | 108899766 | NA | |
| rs150156788 | 8 | 108900989 | NA | |
| 8:108901410:A:G | 8 | 108901410 | NA | NA |
| 8:108906538:C:T | 8 | 108906538 | NA | NA |
| 8:108907254:G:T | 8 | 108907254 | NA | NA |
| 8:108907257:C:G | 8 | 108907257 | NA | NA |
| rs191118779 | 8 | 108907979 | NA | NA |
| 8:108908022:G:A | 8 | 108908022 | NA | NA |
| rs191097915 | 8 | 108915170 | RSP02 | intron_variant |
| rs114143791 | 8 | 108916220 | RSP02 | intron_variant |
| 8:108921884:GT:G | 8 | 108921884 | NA | NA |

[Table 1-45]

| SNP ID | chr | position | gene locus | SNP location in gene locus |
|---|---|---|---|---|
| rs138179792 | 8 | 108923715 | RSP02 | intron_variant |
| rs140554138 | 8 | 108925826 | RSP02 | intron_var iant |
| rs144255075 | 8 | 108925827 | RSP02 | intron_var iant |
| 8:108927282:A:C | 8 | 108927282 | NA | NA |
| 8:108928843:G:A | 8 | 108928843 | NA | NA |
| rs140670079 | 8 | 108929408 | RSP02 | intron_var iant |
| rs190110401 | 8 | 108930742 | RSP02 | intron_variant |
| rs192028485 | 8 | 116822572 | NA | |
| 8:116832799:T:C | 8 | 116832799 | NA | NA |
| rs7836570 | 8 | 116840302 | NA | |
| rs16887807 | 8 | 116843619 | NA | |
| 8:116846927:A:C | 8 | 116846927 | NA | NA |
| rs28366597 | 8 | 116851543 | NA | |
| 8:116855719:T:A | 8 | 116855719 | NA | NA |
| 8:126647803:C:A | 8 | 126647803 | NA | NA |
| 8:142514003:AT:A | 8 | 142514003 | NA | NA |
| rs34896467 | 9 | 2213511 | NA | |
| rs12350147 | 9 | 2217178 | NA | |
| rs7024532 | 9 | 2220348 | NA | |
| rs71504697 | 9 | 2222951 | NA | |
| rs71504698 | 9 | 2223409 | NA | |
| rs17390013 | 9 | 2226756 | NA | |
| rs10491702 | 9 | 2227837 | NA | |
| rs10491704 | 9 | 2228755 | NA | |
| rs12344786 | 9 | 2229313 | NA | |
| rs74987881 | 9 | 2229455 | NA | |
| rs1105379 | 9 | 16319287 | NA | |
| 9:16325344:G:A | 9 | 16325344 | NA | NA |
| rs72714927 | 9 | 16325344 | NA | |
| rs67783457 | 9 | 16325568 | NA | |
| rs12553053 | 9 | 16330357 | NA | |
| rs56190435 | 9 | 16331743 | NA | |
| rs12686134 | 9 | 19605230 | SLC24A2 | intron_variant |
| rs35444308 | 9 | 19605274 | SLC24A2 | intron_variant |
| rs141589871 | 9 | 19609951 | SLC24A2 | intron_variant |
| rs752539 | 9 | 19640344 | SLC24A2 | intron_variant |

[Table 1-46]

| SNP ID | chr | position | gene locus | SNP location in gene locus |
|---|---|---|---|---|
| rs12683123 | 9 | 19642100 | SLC24A2 | intron_variant |
| rs16937677 | 9 | 19642563 | SLC24A2 | intron_variant |
| rs75209268 | 9 | 19653017 | SLC24A2 | intron_variant |
| rs12686530 | 9 | 19655641 | SLC24A2 | intron_variant |
| rs78907538 | 9 | 19655825 | SLC24A2 | intron_variant |
| rs75642022 | 9 | 19657307 | SLC24A2 | intron_variant |
| rs72501450 | 9 | 19658168 | SLC24A2 | intron_variant |
| rs79412153 | 9 | 19658668 | SLC24A2 | intron_variant |
| rs114378135 | 9 | 25102230 | NA | |
| rs10966802 | 9 | 25226973 | NA | |
| 9:25264525:G:T | 9 | 25264525 | NA | NA |
| rs143890288 | 9 | 25267126 | NA | |
| 9:25269360:G:GAC | 9 | 25269360 | NA | NA |
| rs138481346 | 9 | 27860943 | NA | |
| rs117835027 | 9 | 28108997 | LING02 | intron_variant |
| rs7034549 | 9 | 28109195 | LING02 | intron_variant |
| rs7034769 | 9 | 28109318 | LING02 | intron_variant |
| rs148234325 | 9 | 28109777 | NA | NA |
| rs9722119 | 9 | 28111224 | LING02 | intron_variant |
| rs189664295 | 9 | 28111978 | LING02 | intron_variant |
| rs147490749 | 9 | 28112009 | LING02 | intron_variant |
| 9:28117021:T:G | 9 | 28117021 | NA | NA |
| rs112776037 | 9 | 37038910 | NA | |
| rs76697541 | 9 | 37039286 | NA | |
| rs118107060 | 9 | 73107527 | NA | |
| rs11137993 | 9 | 81625189 | NA | |
| rs148742339 | 9 | 82896313 | NA | |
| rs148593924 | 9 | 85082296 | AL162726. 3 | non_coding_transcript_variant |
| rs111776923 | 9 | 87283234 | NA | |
| rs111867627 | 9 | 87336518 | NTRK2 | intron_variant |
| rs113711779 | 9 | 87352036 | NTRK2 | intron_variant |
| rs11141920 | 9 | 90262011 | DAPK1 | intron_variant |
| rs2274607 | 9 | 90262393 | DAPK1 | intron_variant |
| rs145382400 | 9 | 90796382 | NA | |
| rs10991738 | 9 | 93757561 | NA | |

[Table 1-47]

| SNP ID | chr | position | gene locus | SNP location in gene locus |
|---|---|---|---|---|
| 9:93759544:GGTGT:GGTGTGT | 9 | 93759544 | NA | NA |
| rs71509677 | 9 | 93763449 | NA | |
| rs35931712 | 9 | 93769334 | NA | |
| rs35794334 | 9 | 93777994 | NA | |
| rs35246326 | 9 | 93789668 | NA | |
| rs72739474 | 9 | 93838589 | NA | |
| rs296636 | 9 | 93843942 | NA | |
| rs35748257 | 9 | 93848258 | NA | |
| rs55687842 | 9 | 93856705 | NA | |
| rs72739496 | 9 | 93857087 | NA | |
| rs11312435 | 9 | 93858207 | NA | |
| rs79287236 | 9 | 108886577 | NA | |
| rs116925051 | 9 | 110829415 | NA | |
| rs12337098 | 9 | 113513534 | MUSK | intron_variant |
| rs7043625 | 9 | 113516179 | MUSK | intron_variant |
| rs77542599 | 9 | 115702427 | NA | |
| rs11413824 | 9 | 118344670 | NA | |
| rs72765920 | 9 | 124702107 | TTLL11 | NMD_transcript_variant |
| rs11999389 | 9 | 126872652 | NA | |
| rs58848387 | 9 | 128211958 | MAPKAP1 | intron_variant |
| 9:128723505:T:C | 9 | 128723505 | NA | NA |
| rs73582691 | 9 | 128752360 | NA | |
| rs118085053 | 9 | 139614734 | DIPK1B | intron_variant |
| rs17158762 | 10 | 2701823 | NA | |
| 10:5725303:C:T | 10 | 5725303 | NA | NA |
| rs4749996 | 10 | 10878872 | CELF2 | intron_variant |
| rs138294262 | 10 | 10891440 | CELF2 | intron_variant |
| 10:13274942:TA:T | 10 | 13274942 | NA | NA |
| rs41291315 | 10 | 13275437 | UCMA | intron_variant |
| rs116961903 | 10 | 14008637 | FRMD4A | intron_variant |
| rs139902130 | 10 | 14009189 | FRMD4A | intron_variant |
| 10:14017770:T:C | 10 | 14017770 | NA | NA |
| 10:14017770:T:TGC | 10 | 14017770 | NA | NA |
| rs1609477 | 10 | 14021383 | FRMD4A | intron_variant |
| rs11258773 | 10 | 14025006 | FRMD4A | intron_variant |

[Table 1-48]

| SNP ID | chr | position | gene locus | SNP location in gene locus |
|---|---|---|---|---|
| rs12572939 | 10 | 14030842 | FRMD4A | intron_variant |
| rs7096979 | 10 | 17539678 | NA | |
| 10:28077198:AT:A | 10 | 28077198 | NA | NA |
| rs351803 | 10 | 55317094 | NA | |
| rs351802 | 10 | 55317221 | NA | |
| rs351801 | 10 | 55317903 | NA | |
| rs351800 | 10 | 55317928 | NA | |
| rs351799 | 10 | 55317976 | NA | |
| rs2992011 | 10 | 55318012 | NA | |
| rs2931565 | 10 | 55318016 | NA | |
| rs6481015 | 10 | 55318082 | NA | |
| rs6481016 | 10 | 55318158 | NA | |
| rs6415918 | 10 | 55318166 | NA | |
| rs145658941 | 10 | 55318826 | NA | NA |
| rs689581 | 10 | 55318856 | NA | |
| rs35010388 | 10 | 55319128 | NA | |
| rs145725620 | 10 | 55319312 | NA | NA |
| rs394770 | 10 | 55319395 | NA | |
| rs149503551 | 10 | 55319419 | NA | NA |
| rs413137 | 10 | 55319542 | NA | |
| rs443931 | 10 | 55319592 | NA | |
| rs420314 | 10 | 55319617 | NA | |
| rs428563 | 10 | 55319634 | NA | |
| rs450516 | 10 | 55319865 | NA | |
| rs426290 | 10 | 55320087 | NA | |
| rs444728 | 10 | 55320115 | NA | |
| rs430495 | 10 | 55320281 | NA | |
| rs423146 | 10 | 55320430 | NA | |
| rs453158 | 10 | 55320437 | NA | |
| rs2484209 | 10 | 55320471 | NA | |
| rs668434 | 10 | 55320596 | NA | |
| rs668014 | 10 | 55320664 | NA | |
| rs451485 | 10 | 55320992 | NA | |
| rs396374 | 10 | 55321338 | NA | |
| rs423384 | 10 | 55321415 | NA | |
| rs409001 | 10 | 55321543 | NA | |

[Table 1-49]

| SNP ID | chr | position | gene locus | SNP location in gene locus |
|---|---|---|---|---|
| rs399606 | 10 | 55321588 | NA | |
| rs371543 | 10 | 55321684 | NA | |
| rs382870 | 10 | 55321728 | NA | |
| rs428467 | 10 | 55321781 | NA | |
| rs446990 | 10 | 55321931 | NA | |
| rs379026 | 10 | 55321948 | NA | |
| rs379033 | 10 | 55321950 | NA | |
| rs407646 | 10 | 55322061 | NA | |
| rs407337 | 10 | 55322216 | NA | |
| rs445488 | 10 | 55322557 | NA | |
| rs394942 | 10 | 55322731 | NA | |
| rs423806 | 10 | 55322780 | NA | |
| rs389244 | 10 | 55322918 | NA | |
| rs451607 | 10 | 55323157 | NA | |
| rs425467 | 10 | 55323180 | NA | |
| rs395825 | 10 | 55323468 | NA | |
| rs437390 | 10 | 55323572 | NA | |
| rs2484208 | 10 | 55323779 | NA | |
| rs2450530 | 10 | 55323807 | NA | |
| rs440743 | 10 | 55323832 | NA | |
| rs427506 | 10 | 55324048 | NA | |
| rs11527477 | 10 | 55324102 | NA | |
| rs11527478 | 10 | 55324117 | NA | |
| 10:55324128:A:G | 10 | 55324128 | NA | NA |
| 10:55324134:T:G | 10 | 55324134 | NA | NA |
| rs7075007 | 10 | 55324148 | NA | |
| rs11527272 | 10 | 55324150 | NA | |
| rs610251 | 10 | 55324303 | NA | |
| rs61859905 | 10 | 55324317 | NA | |
| rs609908 | 10 | 55324342 | NA | |
| rs663624 | 10 | 55324413 | NA | |
| rs455078 | 10 | 55324459 | NA | |
| rs374891 | 10 | 55324475 | NA | |
| 10:55324510:AC:A | 10 | 55324510 | NA | NA |
| 10:55324513:C:T | 10 | 55324513 | NA | NA |
| 10:55324514:A:G | 10 | 55324514 | NA | NA |

[Table 1-50]

| SNP ID | chr | position | gene locus | SNP location in gene locus |
|---|---|---|---|---|
| rs398169 | 10 | 55324751 | NA | |
| rs416275 | 10 | 55324754 | NA | |
| rs665850 | 10 | 55324915 | NA | |
| rs665881 | 10 | 55324942 | NA | |
| rs607276 | 10 | 55324943 | NA | |
| rs607228 | 10 | 55324976 | NA | |
| rs596456 | 10 | 55325084 | NA | |
| rs4456174 | 10 | 55325288 | NA | |
| rs678445 | 10 | 55325383 | NA | |
| rs678459 | 10 | 55325386 | NA | |
| rs399032 | 10 | 55325442 | NA | |
| 10:55325458:G:C | 10 | 55325458 | NA | NA |
| 10:55325459:G:T | 10 | 55325459 | NA | NA |
| 10:55325460:A:G | 10 | 55325460 | NA | NA |
| rs57606495 | 10 | 55325466 | NA | NA |
| rs34789289 | 10 | 55325562 | NA | |
| rs392668 | 10 | 55325629 | NA | |
| 10:55325657:T:TAA | 10 | 55325657 | NA | NA |
| rs451452 | 10 | 55325763 | NA | |
| rs401333 | 10 | 55325803 | NA | |
| rs381979 | 10 | 55325986 | NA | |
| rs364694 | 10 | 55326079 | NA | |
| rs428127 | 10 | 55326235 | NA | |
| rs376278 | 10 | 55326251 | NA | |
| rs417712 | 10 | 55326306 | NA | |
| rs59540423 | 10 | 55326321 | NA | NA |
| rs11003681 | 10 | 55326332 | NA | |
| rs11003682 | 10 | 55326343 | NA | |
| rs66668165 | 10 | 55326413 | NA | |
| rs61859906 | 10 | 55326428 | NA | |
| rs61859907 | 10 | 55326465 | NA | |
| rs61859908 | 10 | 55326470 | NA | |
| 10:553265544:TGA | 10 | 55326554 | NA | NA |
| rs34095257 | 10 | 55326663 | NA | |
| rs35891588 | 10 | 55326698 | NA | |
| rs34632242 | 10 | 55326715 | NA | |

[Table 1-51]

| SNP ID | chr | position | gene locus | SNP location in gene locus |
|---|---|---|---|---|
| rs35424889 | 10 | 55326737 | NA | |
| rs66678299 | 10 | 55326829 | NA | |
| 10:55326858 :GCAAAGAGTTCATGAC:G | 10 | 55326858 | NA | NA |
| 10:55326914:AGAG:A | 10 | 55326914 | NA | NA |
| rs7898607 | 10 | 55326930 | NA | |
| rs7898708 | 10 | 55326954 | NA | |
| rs598529 | 10 | 55327033 | NA | |
| rs598536 | 10 | 55327042 | NA | |
| 10:55327064:A:G | 10 | 55327064 | NA | NA |
| 10:55327072:GAA:G | 10 | 55327072 | NA | NA |
| rs674159 | 10 | 55327158 | NA | |
| rs409369 | 10 | 55327195 | NA | |
| rs55653225 | 10 | 55327405 | NA | |
| rs55712224 | 10 | 55327472 | NA | |
| rs418047 | 10 | 55327526 | NA | |
| 10:55327577:G:A | 10 | 55327577 | NA | NA |
| rs401407 | 10 | 55327799 | NA | |
| rs2632535 | 10 | 55327835 | NA | |
| rs637714 | 10 | 55327902 | NA | |
| rs1762109 | 10 | 55327985 | NA | |
| rs142344752 | 10 | 55328020 | NA | |
| rs659421 | 10 | 55328188 | NA | |
| rs636340 | 10 | 55328236 | NA | |
| rs659014 | 10 | 55328271 | NA | |
| 10:55328288:T:A | 10 | 55328288 | NA | NA |
| rs658983 | 10 | 55328294 | NA | |
| rs4628595 | 10 | 55328376 | NA | |
| rs392293 | 10 | 55328466 | NA | |
| rs454270 | 10 | 55328737 | NA | |
| rs414849 | 10 | 55328752 | NA | |
| rs365966 | 10 | 55328774 | NA | |
| rs404593 | 10 | 55328985 | NA | |
| rs409814 | 10 | 55328990 | NA | |
| 10:55329057:AC:A | 10 | 55329057 | NA | NA |
| rs368703 | 10 | 55329112 | NA | |

[Table 1-52]

| SNP ID | chr | position | gene locus | SNP location in gene locus |
|--------|-----|----------|------------|----------------------------|
| rs447549 | 10 | 55329255 | NA | |
| rs368110 | 10 | 55329416 | NA | |
| rs374009 | 10 | 55329487 | NA | |
| rs436403 | 10 | 55329513 | NA | |
| rs452019 | 10 | 55329523 | NA | |
| rs427201 | 10 | 55329632 | NA | |
| rs433582 | 10 | 55329954 | NA | |
| rs433574 | 10 | 55329961 | NA | |
| rs441336 | 10 | 55329964 | NA | |
| rs427372 | 10 | 55330380 | NA | |
| rs380989 | 10 | 55330434 | NA | |
| rs17695283 | 10 | 55332545 | NA | |
| rs444186 | 10 | 55334166 | NA | |
| rs10996833 | 10 | 67784976 | CTNNA3 | intron_variant |
| rs34539937 | 10 | 67787534 | CTNNA3 | intron_variant |
| rs35716649 | 10 | 67788443 | CTNNA3 | intron_variant |
| rs12571522 | 10 | 67790052 | CTNNA3 | intron_variant |
| rs35744226 | 10 | 67803006 | CTNNA3 | intron_variant |
| rs12778483 | 10 | 67805050 | CTNNA3 | intron_variant |
| rs17243463 | 10 | 67805413 | CTNNA3 | intron_variant |
| rs75400168 | 10 | 67834004 | CTNNA3 | intron_variant |
| rs71493960 | 10 | 67835457 | CTNNA3 | intron_variant |
| rs35053158 | 10 | 67841299 | CTNNA3 | intron_variant |
| rs35376201 | 10 | 67842582 | CTNNA3 | intron_variant |
| rs67242129 | 10 | 67842799 | NA | NA |
| rs1247787 | 10 | 78830256 | KCNMA1 | intron_variant |
| rs1247779 | 10 | 78839034 | KCNMA1 | intron_variant |
| rs2258870 | 10 | 78840266 | KCNMA1 | intron_variant |
| rs10762747 | 10 | 78842860 | KCNMA1 | intron_variant |
| rs2574789 | 10 | 78851342 | KCNMA1 | intron_variant |
| rs74462085 | 10 | 78856298 | KCNMA1 | intron_variant |
| rs80058374 | 10 | 78859025 | KCNMA1 | intron_variant |
| rs79411761 | 10 | 78861868 | KCNMA1 | intron_variant |
| rs2574791 | 10 | 78864094 | KCNMA1 | intron_variant |
| rs2574787 | 10 | 78883564 | KCNMA1 | intron_variant |
| rs4980128 | 10 | 78886557 | KCNMA1 | intron_variant |

[Table 1-53]

| SNP ID | chr | position | gene locus | SNP location in gene locus |
|---|---|---|---|---|
| rs61574126 | 10 | 80740616 | NA | |
| rs60394423 | 10 | 80742406 | NA | |
| rs77451861 | 10 | 80743419 | NA | |
| rs116906077 | 10 | 80755918 | NA | |
| rs78685078 | 10 | 80755928 | NA | |
| rs117213013 | 10 | 81685744 | NA | |
| rs7089837 | 10 | 85433827 | NA | |
| 10:85434294:T:A | 10 | 85434294 | NA | NA |
| rs115787429 | 10 | 85436477 | NA | |
| rs17102402 | 10 | 85436920 | NA | |
| rs149846651 | 10 | 85437351 | NA | |
| rs74773350 | 10 | 99321878 | UBTD1 | intron_variant |
| 10:112855651:T:C | 10 | 112855651 | NA | NA |
| rs75665088 | 10 | 116038167 | VWA2 | intron_variant |
| rs10736246 | 10 | 117982524 | GFRA1 | intron_variant |
| rs7094346 | 10 | 117986207 | GFRA1 | intron_variant |
| rs4628599 | 10 | 117986457 | GFRA1 | intron_variant |
| rs141923679 | 10 | 118354939 | PNLIPRP1 | intron_variant |
| rs59679890 | 10 | 118636461 | EN04 | intron_variant |
| rs72631124 | 10 | 125679317 | CPXM2 | intron_variant |
| rs72631125 | 10 | 125679694 | CPXM2 | intron_variant |
| rs72631126 | 10 | 125679714 | CPXM2 | intron_variant |
| rs72631127 | 10 | 125680048 | CPXM2 | intron_variant |
| rs72631128 | 10 | 125680075 | CPXM2 | intron_variant |
| rs182795662 | 10 | 125680804 | CPXM2 | intron_variant |
| rs186918206 | 10 | 125680805 | CPXM2 | intron_variant |
| rs72631129 | 10 | 125680924 | CPXM2 | intron_variant |
| rs72631130 | 10 | 125681159 | CPXM2 | intron_variant |
| 10:125681455:T:CC | 10 | 125681455 | NA | NA |
| rs115841965 | 10 | 125681633 | CPXM2 | intron_variant |
| rs72631132 | 10 | 125682219 | CPXM2 | intron_variant |
| rs72631134 | 10 | 125682486 | CPXM2 | intron_variant |
| rs142639355 | 10 | 125682701 | CPXM2 | intron_variant |
| rs115903046 | 10 | 125682823 | CPXM2 | intron_variant |
| rs140301692 | 10 | 125682862 | CPXM2 | intron_variant |
| rs149963239 | 10 | 125727519 | NA | |

[Table 1-54]

| SNP ID | chr | position | gene locus | SNP location in gene locus |
|---|---|---|---|---|
| rs143323962 | 10 | 125728098 | NA | |
| rs11594441 | 10 | 130456079 | NA | |
| rs3847456 | 10 | 130457648 | NA | |
| rs3847457 | 10 | 130457732 | NA | |
| rs72847142 | 10 | 130458683 | NA | |
| rs72280102 | 10 | 130460388 | NA | |
| rs11598828 | 10 | 130463073 | NA | |
| rs11598671 | 10 | 130466623 | NA | |
| rs1475391 | 10 | 130474188 | NA | |
| rs112036204 | 10 | 130475046 | NA | |
| rs113908408 | 11 | 7845074 | NA | |
| rs112933444 | 11 | 7845341 | NA | |
| rs112043976 | 11 | 7845400 | NA | |
| rs73408430 | 11 | 7855420 | NA | |
| rs73408442 | 11 | 7857655 | NA | |
| rs73408450 | 11 | 7858446 | NA | |
| rs73408454 | 11 | 7859334 | NA | |
| rs79725644 | 11 | 7861967 | NA | |
| rs58454329 | 11 | 7864544 | NA | |
| rs369143 | 11 | 7865713 | NA | |
| rs57721290 | 11 | 7867024 | NA | |
| 11:7868688:AT:A | 11 | 7868688 | NA | NA |
| rs11606178 | 11 | 7872593 | NA | |
| rs77771703 | 11 | 7879250 | NA | |
| rs79195468 | 11 | 7897753 | NA | |
| 11:8797713:A:G | 11 | 8797713 | NA | NA |
| rs11042070 | 11 | 8819367 | DENND2B | intron_variant |
| rs142465660 | 11 | 8821504 | NA | NA |
| rs4929915 | 11 | 8824418 | DENND2B | intron_variant |
| rs17337866 | 11 | 8827750 | DENND2B | intron_variant |
| rs3915868 | 11 | 8833800 | DENND2B | intron_variant |
| rs34023999 | 11 | 8839379 | DENND2B | intron_variant |
| rs36094920 | 11 | 8845266 | DENND2B | intron_variant |
| rs7127197 | 11 | 8845607 | DENND2B | intron variant |
| rs35503692 | 11 | 8848785 | DENND2B | intron_variant |
| rs34033747 | 11 | 8853774 | DENND2B | intron_variant |

[Table 1-55]

| SNP ID | chr | position | gene locus | SNP location in gene locus |
|---|---|---|---|---|
| 11:8854132:A:ACTGCG | 11 | 8854132 | NA | NA |
| rs34418236 | 11 | 8859656 | DENND2B | intron_variant |
| rs77784346 | 11 | 8998278 | NA | |
| rs149624805 | 11 | 12188071 | MICAL2 | intron_variant |
| rs118093648 | 11 | 18162065 | NA | |
| rs7120135 | 11 | 24831117 | LUZP2 | intron_variant |
| rs61877058 | 11 | 26600213 | AN03 | intron_variant |
| rs61877059 | 11 | 26604143 | AN03 | intron_variant |
| 11:26605036:C:T | 11 | 26605036 | NA | NA |
| rs17243461 | 11 | 26607661 | AN03 | intron_variant |
| rs192211136 | 11 | 26608276 | AN03 | intron_variant |
| rs17243468 | 11 | 26608776 | AN03 | intron_variant |
| 11:26610935:G:GA | 11 | 26610935 | NA | NA |
| rs61878565 | 11 | 26611476 | AN03 | intron_variant |
| rs61878566 | 11 | 26612275 | AN03 | intron_variant |
| rs61878567 | 11 | 26613230 | AN03 | intron_variant |
| rs61878568 | 11 | 26613870 | AN03 | intron_variant |
| rs61878572 | 11 | 26616967 | AN03 | intron_variant |
| rs61878588 | 11 | 26618785 | AN03 | intron_variant |
| rs61878589 | 11 | 26618960 | AN03 | intron_variant |
| rs200761473 | 11 | 26625591 | AN03 | intron_variant |
| rs61878592 | 11 | 26625601 | AN03 | intron_variant |
| rs36063975 | 11 | 35574891 | NA | |
| rs12798613 | 11 | 35574908 | NA | |
| rs10836415 | 11 | 35575254 | NA | |
| rs59100957 | 11 | 35575495 | NA | |
| rs12420268 | 11 | 35576512 | NA | |
| rs144733009 | 11 | 44566820 | NA | |
| rs2045018 | 11 | 44843041 | TSPAN18 | intron_variant |
| rs11230471 | 11 | 60539384 | MS4A15 | intron_variant |
| rs142518783 | 11 | 62237951 | AHNAK | intron_variant |
| rs12281345 | 11 | 62773213 | SLC22A8 | intron_variant |
| rs 12808517 | 11 | 72601276 | FCHSD2 | intron_variant |
| rs150783164 | 11 | 74983240 | ARRB1 | intron_variant |
| rs78089535 | 11 | 79215837 | NA | |
| rs112798455 | 11 | 79216359 | NA | |

[Table 1-56]

| SNP ID | chr | position | gene locus | SNP location in gene locus |
|---|---|---|---|---|
| 11:79243641:AT:A | 11 | 79243641 | NA | NA |
| rs74659625 | 11 | 79250136 | NA | |
| rs76280090 | 11 | 79250154 | NA | |
| rs147247218 | 11 | 84483667 | DLG2 | intron_variant |
| rs139305706 | 11 | 89858108 | NA | |
| rs149012812 | 11 | 90175103 | NA | |
| rs17860938 | 11 | 102652744 | WTAPP1 | non_coding_transcript_variant |
| rs72977553 | 11 | 102653834 | WTAPP1 | non_coding_transcript_variant |
| rs7112080 | 11 | 102655053 | WTAPP1 | noncodingtranscriptvariant |
| rs7933558 | 11 | 102655137 | WTAPP1 | non_coding_transcript_variant |
| rs72977568 | 11 | 102657657 | WTAPP1 | non_coding_transcript_variant |
| rs187059006 | 11 | 102658035 | WTAPP1 | non_coding_transcript_variant |
| rs11390663 | 11 | 102660054 | WTAPP1 | non_coding_transcript_variant |
| rs7945189 | 11 | 102660564 | WTAPP1 | non_coding_transcript_variant |
| rs17878905 | 11 | 102661757 | MMP1 | intron_variant |
| rs17882844 | 11 | 102661813 | MMP1 | intron_variant |
| rs146555295 | 11 | 105747176 | NA | NA |
| rs12419614 | 11 | 105840184 | GRIA4 | intron_variant |
| rs111988538 | 11 | 107103114 | NA | |
| rs78496161 | 11 | 107148735 | NA | |
| 11:107151031:A:AT | 11 | 107151031 | NA | NA |
| rs79062526 | 11 | 107153318 | NA | |
| rs76404176 | 11 | 107157966 | NA | |
| rs17106711 | 11 | 107158740 | NA | |
| rs7943234 | 11 | 107165710 | NA | |
| rs78568149 | 11 | 107222059 | CWF19L2 | intron_variant |
| rs74346769 | 11 | 107226643 | CWF19L2 | intron_variant |
| rs118053355 | 11 | 107328818 | NA | |
| rs17107015 | 11 | 107353313 | NA | |
| rs7937106 | 11 | 107354312 | NA | |
| rs80050705 | 11 | 107354858 | NA | |
| rs77739772 | 11 | 107356982 | NA | |
| rs201645325 | 11 | 107357860 | NA | NA |
| rs78482726 | 11 | 107362733 | NA | |
| rs149277905 | 11 | 107364050 | NA | |
| rs12417562 | 11 | 107364413 | NA | |

[Table 1-57]

| SNP ID | chr | position | gene locus | SNP location in gene locus |
|---|---|---|---|---|
| rs115051850 | 11 | 117280512 | CEP164 | synonymous_variant |
| rs61743854 | 11 | 117280517 | CEP164 | missense_variant |
| rs111910740 | 11 | 119625360 | NA | |
| rs140081101 | 11 | 119625819 | NA | |
| rs148275870 | 11 | 119630952 | NA | |
| rs115832414 | 11 | 119656930 | NA | |
| rs146686876 | 11 | 119659763 | NA | |
| rs117150747 | 11 | 126994608 | NA | |
| rs78730731 | 11 | 127031263 | NA | |
| rs1944828 | 11 | 127048359 | NA | |
| rs2513442 | 11 | 127053597 | NA | |
| 12:7859133:G:A | 12 | 7859133 | NA | NA |
| rs17834986 | 12 | 15261437 | RERG | 3_prime_UTR_variant |
| rs111641368 | 12 | 26800096 | ITPR2 | intron_variant |
| rs140725931 | 12 | 26802734 | ITPR2 | intron_variant |
| rs112815221 | 12 | 26829320 | ITPR2 | intron_variant |
| rs74849908 | 12 | 26836556 | ITPR2 | intron_variant |
| rs76630621 | 12 | 26841931 | ITPR2 | intron_variant |
| 12:28124654:A:G | 12 | 28124654 | NA | NA |
| rs61918684 | 12 | 29635132 | 0VCH1 | intron_variant |
| rs61922198 | 12 | 29779895 | TMTC1 | non_coding_transcript_variant |
| rs11050326 | 12 | 29782241 | TMTC1 | non_coding_transcript_variant |
| rs12300501 | 12 | 29782247 | TMTC1 | non_coding_transcript_variant |
| rs11050329 | 12 | 29786413 | TMTC1 | non_coding_transcript_variant |
| 12:29788125:C:CA | 12 | 29788125 | NA | NA |
| rs10771557 | 12 | 29788245 | TMTC1 | non_coding_transcript_variant |
| rs11050333 | 12 | 29789329 | TMTC1 | non_coding_transcript_variant |
| rs10843455 | 12 | 29789750 | TMTC1 | non_coding_transcript_variant |
| rs10843457 | 12 | 29790399 | TMTC1 | non_codingtranscriptvariant |
| rs11050334 | 12 | 29790982 | TMTC1 | non_coding_transcript_variant |
| rs12827042 | 12 | 29791219 | TMTC1 | non_coding_transcript_variant |
| rs12826106 | 12 | 29791229 | TMTC1 | non_coding_transcript_variant |
| rs1972681 | 12 | 29791455 | TMTC1 | non_coding_transcript_variant |
| rs1972682 | 12 | 29791633 | TMTC1 | non_coding_transcript_variant |
| rs1549408 | 12 | 29791646 | TMTC1 | non_coding_transcript_variant |
| rs1549409 | 12 | 29791672 | TMTC1 | non_coding_transcript_variant |

[Table 1-58]

| SNP ID | chr | position | gene locus | SNP location in gene locus |
|---|---|---|---|---|
| rs1549410 | 12 | 29791697 | TMTC1 | non_coding_transcript_variant |
| rs10843458 | 12 | 29791936 | TMTC1 | non_codingtranscriptvariant |
| rs7955768 | 12 | 29792069 | TMTC1 | non_coding_transcript_variant |
| rs10771558 | 12 | 29792233 | TMTC1 | non_coding_transcript_variant |
| rs34265384 | 12 | 29792497 | TMTC1 | non_coding_transcript_variant |
| rs1549411 | 12 | 29793140 | TMTC1 | non_coding_transcript_variant |
| rs113386809 | 12 | 29794462 | TMTC1 | non_coding_transcript_variant |
| rs113094413 | 12 | 29795584 | TMTC1 | non_coding_transcript_variant |
| rs113767075 | 12 | 29795807 | TMTC1 | non_coding_transcript_variant |
| rs12371181 | 12 | 31193873 | NA | |
| rs150392011 | 12 | 31198640 | NA | |
| rs11051218 | 12 | 31201717 | NA | |
| rs11051219 | 12 | 31201922 | NA | |
| rs11051221 | 12 | 31203590 | NA | |
| rs11051222 | 12 | 31203920 | NA | |
| rs4244855 | 12 | 31213686 | NA | |
| rs4931415 | 12 | 31216523 | NA | |
| rs4931416 | 12 | 31219161 | NA | |
| rs145929359 | 12 | 31222582 | NA | |
| 12:31225561:G :GTTAGTGAGA | 12 | 31225561 | NA | NA |
| rs2302846 | 12 | 31226930 | DDX11 | 5_primeUTR_variant |
| rs12579271 | 12 | 31230735 | DDX11 | intron_variant |
| rs11832720 | 12 | 57257244 | AC121758.2 | intron_variant |
| rs75465620 | 12 | 57259691 | AC121758.2 | intron_variant |
| rs144935800 | 12 | 57263384 | NA | NA |
| rs117991841 | 12 | 57263617 | AC121758.2 | intron_variant |
| rs74995587 | 12 | 57264426 | AC121758.2 | intron_variant |
| rs4403833 | 12 | 57265921 | AC121758.2 | non_coding_transcript_exon_variant |
| rs12810024 | 12 | 57331444 | NA | |
| rs200182685 | 12 | 57333170 | NA | |
| 12:57335432:A:ATTTA | 12 | 57335432 | NA | NA |
| 12:57335432:A:ATTTT | 12 | 57335432 | NA | NA |
| rs143723418 | 12 | 57339182 | NA | |
| rs1072670 | 12 | 57342100 | NA | |
| rs11836517 | 12 | 57343892 | NA | |

[Table 1-59]

| SNP ID | chr | position | gene locus | SNP location in gene locus |
|---|---|---|---|---|
| rs901068 | 12 | 57346805 | RDH16 | intron_variant |
| rs746049 | 12 | 57349039 | RDH16 | intron_variant |
| rs67771054 | 12 | 57349813 | RDH16 | intron_variant |
| rs12815538 | 12 | 57352871 | RDH16 | non coding transcript exon_variant |
| rs144844809 | 12 | 57353192 | NA | NA |
| rs138278919 | 12 | 57353274 | NA | |
| rs1481543fi4 | 12 | 57356239 | NA | NA |
| rs143470873 | 12 | 57356240 | NA | NA |
| rs34253098 | 12 | 57356325 | NA | |
| 12: 57356830: A: G | 12 | 57356830 | NA | NA |
| rs12820005 | 12 | 57356885 | NA | |
| rs11837093 | 12 | 57356893 | NA | |
| rs79861271 | 12 | 57357106 | NA | |
| 12: 57357322: A: G | 12 | 57357322 | NA | NA |
| rs18105fi553 | 12 | 57357512 | NA | |
| rs77781354 | 12 | 57357721 | NA | |
| 12:57357983: G: A | 12 | 57357983 | NA | NA |
| 12:57358181:C:G | 12 | 57358181 | NA | NA |
| rs703818 | 12 | 57359073 | NA | |
| 12:57359261:C:CA | 12 | 57359261 | NA | NA |
| rs2888139 | 12 | 57359436 | NA | |
| rs12812499 | 12 | 57359643 | NA | |
| rs144223544 | 12 | 57360141 | NA | |
| rs60380217 | 12 | 57360851 | NA | |
| rs1843311 | 12 | 57361414 | NA | |
| rs1843312 | 12 | 57361562 | NA | |
| rs1843313 | 12 | 57361594 | NA | |
| rs2167306 | 12 | 57362345 | NA | |
| rs7969312 | 12 | 57362968 | NA | |
| rs7956166 | 12 | 57363128 | NA | |
| rs7487948 | 12 | 57363907 | NA | |
| rs149233794 | 12 | 57364288 | NA | NA |
| rs12827286 | 12 | 57365564 | NA | |
| 12:57365771:A:AAC | 12 | 57365771 | NA | NA |
| rs145602952 | 12 | 59622709 | NA | |
| rs142940655 | 12 | 59629178 | NA | |

[Table 1-60]

| SNP ID | chr | position | gene locus | SNP location in gene locus |
|---|---|---|---|---|
| rs76598008 | 12 | 59709510 | NA | |
| rs75811180 | 12 | 59825922 | NA | |
| rs144524094 | 12 | 64412328 | SRGAP1 | intron_variant |
| rs75779275 | 12 | 66668726 | NA | |
| rs1117613fi | 12 | 66700104 | HELB | intron_variant |
| rs7976871 | 12 | 69125513 | NUP107 | intron_variant |
| rs7976876 | 12 | 69125514 | NUP107 | intron_variant |
| rs7976687 | 12 | 69125578 | NUP107 | intron_variant |
| rs10878865 | 12 | 69149478 | SLC35E3 | NMD_transcript_variant |
| rs3730603 | 12 | 69222379 | MDM2 | intron_variant |
| rs146370968 | 12 | 69223034 | MDM2 | intron_variant |
| rs3730617 | 12 | 69224892 | MDM2 | intron_variant |
| rs71454208 | 12 | 69226396 | MDM2 | intron_variant |
| rs3730635 | 12 | 69229123 | MDM2 | intron_variant |
| rs3730652 | 12 | 69232793 | MDM2 | intron_variant |
| rs769412 | 12 | 69233215 | MDM2 | synonymous_variant |
| rs11296638 | 12 | 69238437 | MDM2 | 3_prime_UTR_variant |
| rs7956669 | 12 | 69238507 | MDM2 | 3 prime UTR variant |
| rs78497497 | 12 | 94965816 | TMCC3 | intron_variant |
| rs142944124 | 12 | 99162625 | ANKS1B | intron_variant |
| rs143208630 | 12 | 99174638 | ANKS1B | intron_variant |
| rs10507253 | 12 | 115218522 | NA | |
| rs10850384 | 12 | 115218755 | NA | |
| rs11608897 | 12 | 115219079 | NA | |
| rs144088350 | 12 | 115219743 | NA | |
| rs118030982 | 12 | 118766151 | TAOK3 | intron_variant |
| 12:118770780:A:AAC | 12 | 118770780 | NA | NA |
| 12:118770780:A :AACACACACACACAC | 12 | 118770780 | NA | NA |
| 12:125161441:T:C | 12 | 125161441 | NA | NA |
| rs11058227 | 12 | 126018404 | TMEM132B | intron_variant |
| rs146061449 | 13 | 31045629 | HMGB1 | intron_variant |
| rs9541338 | 13 | 35177997 | NA | |
| rs9599308 | 13 | 35178625 | NA | |
| rs9592551 | 13 | 35179080 | NA | |
| rs7400628 | 13 | 38475425 | NA | |

[Table 1-61]

| SNP ID | chr | position | gene locus | SNP location in gene locus |
|---|---|---|---|---|
| rs4941879 | 13 | 38482388 | NA | |
| rs9603278 | 13 | 38484455 | NA | |
| rs77525380 | 13 | 38486498 | NA | |
| rs11620108 | 13 | 38523618 | NA | |
| rs75300170 | 13 | 38527975 | NA | |
| rs10161861 | 13 | 38533329 | NA | |
| rs140158226 | 13 | 38545248 | NA | |
| rs11619292 | 13 | 38545843 | NA | |
| rs113982010 | 13 | 38582994 | NA | |
| rs9603302 | 13 | 38585865 | NA | |
| rs7999781 | 13 | 38593133 | NA | |
| rs139272125 | 13 | 61294761 | NA | |
| rs140760839 | 13 | 61350501 | NA | |
| rs79016205 | 13 | 65322102 | NA | |
| rs9317907 | 13 | 70792954 | NA | |
| rs118058117 | 13 | 71328142 | NA | |
| rs9599962 | 13 | 73002308 | NA | |
| rs184865348 | 13 | 73049971 | NA | |
| rs117639698 | 13 | 73077947 | NA | |
| rs35985160 | 13 | 75124959 | NA | |
| rs76467960 | 13 | 78194746 | SCEL | intron_variant |
| rs75814545 | 13 | 78196291 | SCEL | intron_variant |
| 13:78211558:T:C | 13 | 78211558 | NA | NA |
| rs9593249 | 13 | 78229617 | NA | |
| rs9544568 | 13 | 78242133 | NA | |
| rs186924328 | 13 | 88085503 | NA | |
| rs184461986 | 13 | 88122173 | NA | |
| rs184036706 | 13 | 88124358 | NA | |
| rs143514766 | 13 | 88150782 | NA | |
| rs145616607 | 13 | 88175773 | NA | |
| rs146077205 | 13 | 88183851 | NA | |
| rs115011805 | 13 | 88183871 | NA | |
| rs149371095 | 13 | 88186758 | NA | |
| rs141046761 | 13 | 88188257 | NA | |
| rs7990951 | 13 | 95592144 | NA | |
| rs4773831 | 13 | 95595122 | NA | |

[Table 1-62]

| SNP ID | chr | position | gene locus SNP | location in gene locus |
|---|---|---|---|---|
| rs7991523 | 13 | 95602158 | NA | |
| rs8002051 | 13 | 95606804 | NA | |
| rs9524705 | 13 | 95609326 | NA | |
| rs7998757 | 13 | 95610131 | NA | |
| rs192331957 | 13 | 101175688 | PCCA | intron_variant |
| rs1408046 | 13 | 103484653 | BIVM-ERCC5 | intron_variant |
| rs76029744 | 13 | 103486018 | BIVM-ERCC5 | intron_variant |
| rs7327832 | 13 | 103499900 | BIVM-ERCC5 | intron_variant |
| rs6491714 | 13 | 103500458 | BIVM-ERCC5 | intron_variant |
| rs75982504 | 13 | 103501928 | BIVM-ERCC5 | intron_variant |
| rs7328951 | 13 | 103502204 | BIVM-ERCC5 | intron_variant |
| rs7333684 | 13 | 103502332 | BIVM-ERCC5 | intron_variant |
| rs2104301 | 13 | 103502553 | BIVM-ERCC5 | intron_variant |
| rs4150263 | 13 | 103504344 | BIVM-ERCC5 | intron_variant |
| rs4150278 | 13 | 103507753 | BIVM-ERCC5 | intron_variant |
| rs4150281 | 13 | 103508095 | BIVM-ERCC5 | intron variant |
| rs4150287 | 13 | 103509132 | BIVM-ERCC5 | intron_variant |
| rs12854564 | 13 | 103976233 | NA | |
| rs75174938 | 13 | 108015726 | FAM155A | intron_variant |
| rs9520931 | 13 | 109193709 | NA | |
| rs3825469 | 13 | 110881627 | COL4A1 | intron_variant |
| 13:113504421:A:G | 13 | 113504421 | NA | NA |
| 13:113505107:A:G | 13 | 113505107 | NA | NA |
| rs12877285 | 13 | 113519561 | ATP11A | intron_variant |
| 13:113679279:G:A | 13 | 113679279 | NA | NA |
| 13:113687158:C:T | 13 | 113687158 | NA | NA |
| rs117419951 | 13 | 114211421 | NA | |
| rs182523160 | 13 | 114214047 | NA | |
| rs78785829 | 14 | 29770720 | NA | |
| rs79415577 | 14 | 29823747 | NA | |
| rs9788541 | 14 | 29841230 | NA | |
| rs10141779 | 14 | 46320098 | NA | |
| rs10130975 | 14 | 46320102 | NA | |
| rs10142107 | 14 | 46320162 | NA | |
| rs17116552 | 14 | 46322340 | NA | |
| rs145926281 | 14 | 47920866 | MDGA2 | intron_variant |

[Table 1-63]

| SNP ID | chr | position | gene locus | SNP location in gene locus |
|---|---|---|---|---|
| rs74506457 | 14 | 48138790 | MDGA2 | intron_variant |
| rs140552479 | 14 | 48452990 | NA | |
| rs4483778 | 14 | 62669733 | NA | |
| rs74055659 | 14 | 62672299 | NA | |
| rs74055661 | 14 | 62674106 | NA | |
| rs76079973 | 14 | 62674412 | NA | |
| rs60686625 | 14 | 62675734 | NA | |
| rs4902111 | 14 | 62679573 | NA | |
| rs72476707 | 14 | 62679925 | NA | |
| rs72476708 | 14 | 62679941 | NA | |
| rs74055665 | 14 | 62683062 | NA | |
| rs74055666 | 14 | 62683158 | NA | |
| rs11622218 | 14 | 62684550 | NA | |
| rs74055670 | 14 | 62695028 | NA | |
| rs200453336 | 14 | 62696350 | NA | |
| rs28793052 | 14 | 62697293 | NA | |
| rs2090184 | 14 | 62698275 | NA | |
| rs2365677 | 14 | 78681121 | NRXN3 | intron_variant |
| rs142079680 | 14 | 84269688 | NA | |
| rs77476904 | 14 | 85191547 | NA | |
| rs74344396 | 14 | 85195062 | NA | |
| rs12435636 | 14 | 85195464 | NA | |
| rs1076961 | 14 | 85203036 | NA | |
| rs74890854 | 14 | 88800193 | NA | |
| rs78906101 | 14 | 89689421 | FOXN3 | intron_variant |
| rs113893721 | 14 | 92365125 | FBLN5 | intron_variant |
| rs1861086 | 14 | 92371328 | FBLN5 | intron_variant |
| rs72702992 | 14 | 101655820 | NA | |
| rs61994508 | 14 | 101656254 | NA | |
| rs72702993 | 14 | 101656819 | NA | |
| rs72702996 | 14 | 101657080 | NA | |
| rs56907901 | 14 | 101657647 | NA | |
| rs72702997 | 14 | 101659182 | NA | |
| rs7147137 | 14 | 101678763 | NA | |
| rs35480234 | 15 | 31093529 | NA | |
| rs7163291 | 15 | 31093881 | NA | |

[Table 1-64]

| SNP ID | chr | position | gene locus | SNP location in gene locus |
|---|---|---|---|---|
| rs35579443 | 15 | 31095933 | NA | |
| rs35622207 | 15 | 31097419 | NA | |
| rs11853322 | 15 | 31097795 | NA | |
| rs6493297 | 15 | 31099054 | NA | |
| rs71399735 | 15 | 31099728 | NA | |
| rs57386153 | 15 | 31101060 | NA | |
| rs3933429 | 15 | 31101915 | NA | |
| rs28584365 | 15 | 31103814 | NA | |
| rs10557815 | 15 | 31244352 | MTMR10 | intron variant |
| rs147616718 | 15 | 31253523 | MTMR10 | intron_variant |
| rs76467407 | 15 | 31255220 | MTMR10 | intron_variant |
| rs12898290 | 15 | 31294343 | TRPM1 | missense_variant |
| rs3784589 | 15 | 31294714 | TRPM1 | stop_gained |
| rs10162919 | 15 | 31295788 | TRPM1 | intron_variant |
| rs7182547 | 15 | 31297672 | TRPM1 | intron_variant |
| rs7161796 | 15 | 31298145 | TRPM1 | intron_variant |
| rs7167074 | 15 | 31298352 | TRPM1 | intron_variant |
| rs7182571 | 15 | 31298442 | TRPM1 | intron_variant |
| rs7166214 | 15 | 31298483 | TRPM1 | intron_variant |
| rs35616506 | 15 | 31298737 | TRPM1 | intron_variant |
| rs143969953 | 15 | 31299528 | TRPM1 | intron_variant |
| 15:31300031:C:CATTT | 15 | 31300031 | NA | NA |
| rs61997145 | 15 | 31301159 | TRPM1 | intron_variant |
| rs12913087 | 15 | 31304551 | TRPM1 | intron_variant |
| rs201396966 | 15 | 31305173 | TRPM1 | intron_variant |
| rs35891405 | 15 | 31307063 | TRPM1 | intron_variant |
| 15:31309197:A:C | 15 | 31309197 | NA | NA |
| rs12911930 | 15 | 31309225 | TRPM1 | intron_variant |
| rs12898815 | 15 | 31309468 | TRPM1 | intron_variant |
| rs35208656 | 15 | 31310534 | TRPM1 | intron_variant |
| rs71420543 | 15 | 31312193 | NA | NA |
| rs12915504 | 15 | 31312619 | TRPM1 | intron_variant |
| rs12902573 | 15 | 31314317 | TRPM1 | intron_variant |
| rs151309116 | 15 | 33681236 | RYR3 | intron_variant |
| rs148846874 | 15 | 33899434 | RYR3 | intron_variant |
| rs12905712 | 15 | 37264445 | MEIS2 | intron_variant |

[Table 1-65]

| SNP ID | chr | position | gene locus | SNP location in gene locus |
|---|---|---|---|---|
| rs1194770 | 15 | 37268081 | MEIS2 | intron_variant |
| rs80351865 | 15 | 40161155 | GPR176 | intron_variant |
| rs138955007 | 15 | 55865286 | PYG01 | intron_variant |
| rs77212634 | 15 | 55875096 | PYG01 | intron variant |
| rs72738039 | 15 | 57687411 | CGNL1 | non_coding_transcript_variant |
| rs73414637 | 15 | 62288216 | VPS13C | intron_variant |
| rs78465010 | 15 | 62313069 | VPS13C | intron_variant |
| rs67566652 | 15 | 64125992 | HERC1 | intron_variant |
| rs9972484 | 15 | 64127038 | NA | |
| rs9972527 | 15 | 64127531 | NA | |
| rs7169537 | 15 | 64129914 | NA | |
| rs12148623 | 15 | 64131474 | NA | |
| rs2120252 | 15 | 64136472 | NA | |
| rs116914345 | 15 | 66971210 | NA | |
| rs190172459 | 15 | 78702652 | NA | |
| rs144928882 | 15 | 78702663 | NA | |
| rs181427460 | 15 | 78702665 | NA | |
| rs77156427 | 15 | 78703944 | NA | |
| rs76061647 | 15 | 78704580 | NA | |
| rs76765649 | 15 | 78705102 | NA | |
| rs11637161 | 15 | 78706391 | NA | |
| rs79961164 | 15 | 78707978 | NA | |
| rs80225953 | 15 | 78708302 | NA | |
| rs77826529 | 15 | 78708555 | NA | |
| rs72738490 | 15 | 80424475 | ZFAND6 | intron_variant |
| rs12905402 | 15 | 80516383 | CTXND1 | intron_variant |
| rs35971717 | 15 | 80517610 | CTXND1 | intron_variant |
| 15:80517776:T:TC | 15 | 80517776 | NA | NA |
| rs71399403 | 15 | 80517919 | CTXND1 | intron_variant |
| rs34765237 | 15 | 80520275 | CTXND1 | intron_variant |
| rs80244168 | 15 | 80520961 | CTXND1 | intron_variant |
| rs28621634 | 15 | 80521264 | CTXND1 | intron_variant |
| rs142665219 | 15 | 82201101 | NA | |
| rs138527956 | 15 | 82208029 | NA | |
| rs146297278 | 15 | 82224258 | NA | |
| rs74028454 | 15 | 82275400 | NA | |

[Table 1-66]

| SNP ID | chr | position | gene locus | SNP location in gene locus |
|---|---|---|---|---|
| rs141857089 | 15 | 82275840 | NA | |
| 15:82290701:T:A | 15 | 82290701 | NA | NA |
| rs74028477 | 15 | 82298644 | NA | |
| rs72746755 | 15 | 85851875 | ADAMTS7P4 | non_coding_transcript_variant |
| rs4284612 | 15 | 85880568 | NA | |
| rs74039987 | 15 | 101405738 | NA | |
| rs141389573 | 15 | 101715258 | NA | |
| rs76076521 | 16 | 11530047 | AC099489. 1 | intron_variant |
| rs113344842 | 16 | 11697890 | LITAF | intron_variant |
| 1629011862:C:T | 16 | 29011862 | NA | NA |
| rs147914226 | 16 | 29114347 | AC009093. 9 | non_coding_transcript_variant |
| rs117889226 | 16 | 29115569 | NA | NA |
| rs117272179 | 16 | 29222277 | AC009093. 8 | non_coding_transcript_variant |
| rs147323464 | 16 | 29294541 | NA | |
| rs143769600 | 16 | 29332310 | AC025279. 2 | non_coding_transcript_exon_variant |
| 16:55795832:C:T | 16 | 55795832 | NA | NA |
| rs79304792 | 16 | 57532336 | NA | |
| rs8052995 | 16 | 60372574 | NA | |
| rs935754 | 16 | 60377491 | NA | |
| 16:71153772:G:A | 16 | 71153772 | NA | NA |
| rs4888922 | 16 | 79121475 | WW0X | intron_variant |
| rs57113334 | 16 | 80150523 | NA | |
| rs114992408 | 16 | 82564106 | NA | |
| rs111336258 | 16 | 82565313 | NA | |
| rs12448746 | 16 | 84495825 | ATP2C2 | intron_variant |
| rs12149961 | 16 | 85133324 | FAM92B | intron_variant |
| rs113597486 | 16 | 85135378 | FAM92B | intron_variant |
| rs75760369 | 16 | 85136066 | FAM92B | intron_variant |
| rs150351869 | 16 | 85136199 | FAM92B | intron_variant |
| rs77654959 | 16 | 85136253 | FAM92B | intron_variant |
| 16:85185004:G:A | 16 | 85185004 | NA | NA |
| rs142376288 | 16 | 85194913 | NA | |
| rs143768726 | 17 | 6245605 | NA | |
| rs116884024 | 17 | 7444424 | NA | |
| rs11655029 | 17 | 17649172 | RAI1 | intron_variant |
| rs9900803 | 17 | 17650978 | RAI1 | intron_variant |

[Table 1-67]

| SNP ID | chr | position | gene locus | SNP location in gene locus |
|---|---|---|---|---|
| rs11656775 | 17 | 17654319 | RAI1 | intron_variant |
| rs941448 | 17 | 17654542 | RAI1 | intron_variant |
| rs4925109 | 17 | 17661802 | RAI1 | intron_variant |
| 17:17666365:G:GA | 17 | 17666365 | NA | NA |
| 17:17678848:T :TAACACACATTTTGTA | 17 | 17678848 | NA | NA |
| rs8072510 | 17 | 33772658 | SLFN13 | stop_gained |
| rs11652363 | 17 | 33774675 | SLFN13 | intron_variant |
| rs145674942 | 17 | 33775604 | SLFN13 | intron_variant |
| rs75361910 | 17 | 33793208 | SLFN12L | intron_variant |
| rs78856173 | 17 | 33805956 | SLFN12L | intron_variant |
| 17:64650539:C:CAG | 17 | 64650539 | NA | NA |
| 17:64650539:C:CAT | 17 | 64650539 | NA | NA |
| rs138440170 | 17 | 71704366 | NA | |
| rs78274099 | 17 | 77275723 | RBF0X3 | intron_variant |
| rs9675480 | 18 | 1444697 | NA | |
| rs73365235 | 18 | 1450563 | NA | |
| rs2160960 | 18 | 1453341 | NA | |
| rs143955284 | 18 | 1453688 | NA | NA |
| rs57847079 | 18 | 1453700 | NA | |
| rs78812196 | 18 | 1459687 | NA | |
| rs75905563 | 18 | 1460889 | NA | |
| 18:1460930:C:G | 18 | 1460930 | NA | NA |
| rs16940518 | 18 | 1460930 | NA | |
| rs73365261 | 18 | 1463910 | NA | |
| rs72634385 | 18 | 3225679 | NA | |
| rs55762828 | 18 | 3242809 | NA | |
| rs1662349 | 18 | 3246289 | NA | |
| rs7235642 | 18 | 4454027 | DLGAP1 | intron_variant |
| rs7236361 | 18 | 4454161 | DLGAP1 | intron_variant |
| rs151109374 | 18 | 4575611 | NA | |
| rs56830227 | 18 | 9699877 | NA | |
| 18:11624174:G:C | 18 | 11624174 | NA | NA |
| 18:19415747:T:A | 18 | 19415747 | NA | NA |
| rs1944288 | 18 | 25749095 | CDH2 | intron_variant |
| rs2191636 | 18 | 25749888 | CDH2 | intron_variant |

[Table 1-68]

| SNP ID | chr | position | gene locus | SNP location in gene locus |
|---|---|---|---|---|
| rs11661487 | 18 | 25750950 | CDH2 | intron_variant |
| rs11874478 | 18 | 25751637 | CDH2 | intron_variant |
| rs75095140 | 18 | 28690670 | NA | |
| rs77485878 | 18 | 28713843 | DSC1 | intron_variant |
| rs117854132 | 18 | 32857590 | ZSCAN30 | intron_variant |
| rs75504094 | 18 | 32926349 | NA | |
| rs72893124 | 18 | 35136494 | CELF4 | intron_variant |
| rs72893132 | 18 | 35138693 | CELF4 | intron_variant |
| rs150664883 | 18 | 35146206 | NA | |
| rs55996300 | 18 | 35158843 | NA | |
| rs72893187 | 18 | 35172187 | NA | |
| rs72895006 | 18 | 35189638 | NA | |
| rs55778135 | 18 | 35192829 | NA | |
| rs111869452 | 18 | 37930904 | NA | NA |
| rs111697888 | 18 | 49777775 | NA | |
| rs113445640 | 18 | 49787377 | NA | |
| rs112197694 | 18 | 49792926 | NA | |
| rs62081521 | 18 | 49793572 | NA | |
| rs73445368 | 18 | 49794194 | NA | |
| rs62081522 | 18 | 49796696 | NA | |
| rs73445370 | 18 | 49796944 | NA | |
| rs62081545 | 18 | 49803581 | NA | |
| rs62081546 | 18 | 49805925 | NA | |
| rs145744015 | 18 | 49808004 | NA | NA |
| rs62081550 | 18 | 49810293 | NA | |
| rs7240258 | 18 | 49812967 | NA | |
| rs62081552 | 18 | 49813508 | NA | |
| rs62081553 | 18 | 49816209 | NA | |
| rs62081554 | 18 | 49816348 | NA | |
| rs8097353 | 18 | 49816961 | NA | |
| rs62081555 | 18 | 49817639 | NA | |
| rs28592006 | 18 | 50295649 | DCC | intron_variant |
| rs149621133 | 18 | 50299840 | DCC | intron_variant |
| rs114489043 | 18 | 50301355 | DCC | intron_variant |
| rs74671490 | 18 | 50301411 | DCC | intron_variant |
| rs74600925 | 18 | 50301425 | DCC | intron_variant |

[Table 1-69]

| SNP ID | chr | position | gene locus | SNP location in gene locus |
|---|---|---|---|---|
| rs78939599 | 18 | 50301601 | DCC | intron_variant |
| rs75368394 | 18 | 50301737 | DCC | intron_variant |
| rs113902371 | 18 | 50301765 | DCC | intron_variant |
| rs9954223 | 18 | 50301788 | DCC | intron_variant |
| rs9954366 | 18 | 50301832 | DCC | intron_variant |
| rs9954387 | 18 | 50301888 | DCC | intron_variant |
| rs9956954 | 18 | 50302147 | DCC | intron_variant |
| rs9946240 | 18 | 50302221 | DCC | intron_variant |
| rs9957208 | 18 | 50302225 | DCC | intron_variant |
| rs9957066 | 18 | 50302280 | DCC | intron_variant |
| rs28720976 | 18 | 50302448 | DCC | intron_variant |
| rs28710315 | 18 | 50302476 | DCC | intron_variant |
| rs199781562 | 18 | 50302657 | DCC | intron_variant |
| 18:50302661:AG:A | 18 | 50302661 | NA | NA |
| rs75255207 | 18 | 50302726 | DCC | intron_variant |
| rs146773466 | 18 | 50302743 | DCC | intron_variant |
| rs112096055 | 18 | 50302828 | DCC | intron_variant |
| rs77911615 | 18 | 50302866 | DCC | intron_variant |
| rs77161102 | 18 | 50303025 | DCC | intron_variant |
| rs9960305 | 18 | 50303080 | DCC | intron_variant |
| rs76926243 | 18 | 50303305 | DCC | intron_variant |
| rs9949728 | 18 | 50303409 | DCC | intron_variant |
| rs72079451 | 18 | 50303416 | DCC | intron_variant |
| rs140753418 | 18 | 50303838 | NA | NA |
| rs28418704 | 18 | 50303935 | DCC | intron_variant |
| rs1984342 | 18 | 50303991 | DCC | intron_variant |
| 18:50304137:A:AGGGGG | 18 | 50304137 | NA | NA |
| rs75177472 | 18 | 50304283 | DCC | intron_variant |
| rs28662803 | 18 | 50304359 | DCC | intron_variant |
| rs79241149 | 18 | 50304605 | DCC | intron_variant |
| rs9963981 | 18 | 50304678 | DCC | intron_variant |
| rs74601030 | 18 | 50304685 | DCC | intron_variant |
| rs9966586 | 18 | 50304959 | DCC | intron_variant |
| rs9955760 | 18 | 50305011 | DCC | intronvariant |
| rs9955775 | 18 | 50305063 | DCC | intron_variant |
| rs76362387 | 18 | 50305186 | DCC | intron_variant |

[Table 1-70]

| SNP ID | chr | position | gene locus | SNP location in gene locus |
|---|---|---|---|---|
| rs76980476 | 18 | 50305329 | DCC | intron_variant |
| rs140215942 | 18 | 50305430 | DCC | intron_variant |
| rs79518038 | 18 | 50305494 | DCC | intron_variant |
| rs201972311 | 18 | 50305531 | DCC | intron_variant |
| rs116940662 | 18 | 50305545 | DCC | intron_variant |
| rs9967152 | 18 | 50305573 | DCC | intron_variant |
| rs9956376 | 18 | 50305628 | DCC | intron_variant |
| rs9967408 | 18 | 50305708 | DCC | intron_variant |
| rs9946762 | 18 | 50305989 | DCC | intron_variant |
| rs4638688 | 18 | 50306239 | DCC | intron_variant |
| rs9947241 | 18 | 50306289 | DCC | intron_variant |
| rs9959370 | 18 | 50306321 | DCC | intron_variant |
| 18:50306418:C:CTTCATTCA | 18 | 50306418 | NA | NA |
| rs78062210 | 18 | 50306489 | DCC | intron_variant |
| rs78835166 | 18 | 50306510 | DCC | intron_variant |
| rs183630099 | 18 | 50306529 | DCC | intron_variant |
| rs188676378 | 18 | 50306530 | DCC | intron_variant |
| rs74542236 | 18 | 50306601 | DCC | intron_variant |
| rs74406804 | 18 | 50306611 | DCC | intron_variant |
| rs76093543 | 18 | 50306638 | DCC | intron_variant |
| rs74641489 | 18 | 50306652 | DCC | intron_variant |
| rs79335626 | 18 | 50306756 | DCC | intron_variant |
| rs79579873 | 18 | 50306952 | DCC | intron_variant |
| rs9962392 | 18 | 50307080 | DCC | intron_variant |
| rs9950339 | 18 | 50307252 | DCC | intron_variant |
| rs9950448 | 18 | 50307351 | DCC | intron_variant |
| rs9950558 | 18 | 50307440 | DCC | intron_variant |
| rs9950856 | 18 | 50307670 | DCC | intron_variant |
| rs9951083 | 18 | 50307728 | DCC | intron_variant |
| rs9950936 | 18 | 50307750 | DCC | intron_variant |
| rs143651192 | 18 | 50308170 | NA | NA |
| 18:50308240:G:GTT | 18 | 50308240 | NA | NA |
| rs80118519 | 18 | 50308340 | DCC | intron_variant |
| rs80289134 | 18 | 50308699 | DCC | intron_variant |
| rs77481124 | 18 | 50308701 | DCC | intron_variant |

[Table 1-71]

| SNP ID | chr | position | gene locus | SNP location in gene locus |
|---|---|---|---|---|
| rs113720878 | 18 | 50308740 | DCC | intron_variant |
| rs112252157 | 18 | 50308746 | DCC | intron_variant |
| rs28407519 | 18 | 50308840 | DCC | intron_variant |
| rs28485029 | 18 | 50308845 | DCC | intron_variant |
| rs28610143 | 18 | 50308878 | DCC | intron_variant |
| rs142971261 | 18 | 50308896 | DCC | intron_variant |
| rs111627202 | 18 | 50308902 | DCC | intron_variant |
| rs28398323 | 18 | 50309063 | DCC | intron_variant |
| rs28659932 | 18 | 50309081 | DCC | intronvariant |
| rs28524721 | 18 | 50309291 | DCC | intron_variant |
| rs77721880 | 18 | 50309453 | DCC | intron_variant |
| rs78434669 | 18 | 50309461 | DCC | intron_variant |
| rs137865031 | 18 | 50309499 | DCC | intron_variant |
| rs114083506 | 18 | 50309615 | DCC | intron_variant |
| rs79875724 | 18 | 50309626 | DCC | intron_variant |
| rs150063286 | 18 | 50309652 | DCC | intron_variant |
| rs9959936 | 18 | 50309944 | DCC | intron_variant |
| rs111664411 | 18 | 50309956 | DCC | intron_variant |
| rs139769416 | 18 | 50310074 | DCC | intron_variant |
| rs75906728 | 18 | 50310166 | DCC | intron_variant |
| rs74650785 | 18 | 50310219 | DCC | intron_variant |
| rs9949295 | 18 | 50310263 | DCC | intron_variant |
| rs76434772 | 18 | 50310291 | DCC | intron_variant |
| rs75112069 | 18 | 50310392 | DCC | intron_variant |
| rs111554653 | 18 | 50310597 | DCC | intron_variant |
| 18:50310625:C:CTTTCT | 18 | 50310625 | NA | NA |
| rs78714353 | 18 | 50310649 | DCC | intron_variant |
| rs75493591 | 18 | 50310680 | DCC | intron_variant |
| rs77393461 | 18 | 50310710 | DCC | intron_variant |
| rs9960581 | 18 | 50310772 | DCC | intron_variant |
| rs77105189 | 18 | 50310911 | DCC | intron_variant |
| rs115627213 | 18 | 50310920 | DCC | intron_variant |
| rs113795849 | 18 | 50310929 | DCC | intron_variant |
| rs76212234 | 18 | 50311037 | DCC | intron_variant |
| rs75762275 | 18 | 50311039 | DCC | intron_variant |
| rs78544169 | 18 | 50311097 | DCC | intron_variant |

[Table 1-72]

| SNP ID | chr | position | gene locus | SNP location in gene locus |
|---|---|---|---|---|
| rs28762705 | 18 | 50311168 | DCC | intron_variant |
| rs28820319 | 18 | 50311273 | DCC | intron_variant |
| 18:50311304:G:GGAGA | 18 | 50311304 | NA | NA |
| rs9953235 | 18 | 50311467 | DCC | intron_variant |
| rs9953246 | 18 | 50311486 | DCC | intron_variant |
| rs28624269 | 18 | 50311586 | DCC | intron_variant |
| rs28416202 | 18 | 50311587 | DCC | intron_variant |
| rs28587032 | 18 | 50311625 | DCC | intron_variant |
| rs9953616 | 18 | 50311854 | DCC | intron_variant |
| rs9956105 | 18 | 50311914 | DCC | intron_variant |
| rs9956125 | 18 | 50311962 | DCC | intron_variant |
| rs9945323 | 18 | 50312029 | DCC | intron_variant |
| rs9956060 | 18 | 50312136 | DCC | intron_variant |
| rs9945509 | 18 | 50312217 | DCC | intron_variant |
| rs9956657 | 18 | 50312399 | DCC | intron_variant |
| rs9956597 | 18 | 50312504 | DCC | intron_variant |
| rs111891224 | 18 | 50312516 | DCC | intron_variant |
| rs9941424 | 18 | 50312580 | DCC | intron_variant |
| rs143959738 | 18 | 50312634 | DCC | intron_variant |
| rs9941425 | 18 | 50312809 | DCC | intron_variant |
| 18:50312832:T:TA | 18 | 50312832 | NA | NA |
| rs9941430 | 18 | 50313014 | DCC | intron_variant |
| rs9941431 | 18 | 50313046 | DCC | intron_variant |
| rs78139846 | 18 | 50313451 | DCC | intron_variant |
| rs75846297 | 18 | 50313646 | DCC | intron_variant |
| rs28748057 | 18 | 50313692 | DCC | intron_variant |
| rs199624929 | 18 | 50313803 | DCC | intron_variant |
| 18:50313941:T:TAAATA | 18 | 50313941 | NA | NA |
| rs28770857 | 18 | 50314106 | DCC | intron_variant |
| rs28872917 | 18 | 50314112 | DCC | intron_variant |
| rs9941438 | 18 | 50314590 | DCC | intron_variant |
| rs9941411 | 18 | 50314697 | DCC | intron_variant |
| rs9941419 | 18 | 50314952 | DCC | intron_variant |
| rs9941444 | 18 | 50315007 | DCC | intron_variant |
| rs138603450 | 18 | 50315409 | DCC | intron_variant |
| rs9955427 | 18 | 50315451 | DCC | intron_variant |
| rs9966590 | 18 | 50315572 | DCC | intron_variant |
| rs9955595 | 18 | 50315604 | DCC | intron_variant |

(continued)

| SNP ID | chr | position | gene locus | SNP location in gene locus |
|---|---|---|---|---|
| rs9966461 | 18 | 50315677 | DCC | intron_variant |

[Table 1-73]

| SNP ID | chr | position | gene locus | SNP location in gene locus |
|---|---|---|---|---|
| rs77259281 | 18 | 50316233 | DCC | intron_variant |
| rs79494668 | 18 | 50316355 | DCC | intron_variant |
| rs9946558 | 18 | 50316362 | DCC | intron_variant |
| rs9958738 | 18 | 50316480 | DCC | intron_variant |
| rs9946735 | 18 | 50316499 | DCC | intron_variant |
| rs9958825 | 18 | 50316550 | DCC | intron_variant |
| rs144978667 | 18 | 56289433 | ALPK2 | intron_variant |
| rs28757575 | 18 | 61639238 | HMSD | i ntron variant |
| rs28374149 | 18 | 61639311 | HMSD | intron_variant |
| rs117230606 | 18 | 64368841 | NA | |
| rs77930714 | 18 | 64381429 | NA | |
| rs75773722 | 18 | 64426972 | NA | |
| rs73967432 | 18 | 65688400 | NA | |
| rs113960301 | 18 | 65707948 | NA | |
| rs183621604 | 18 | 74404956 | NA | |
| rs80055446 | 18 | 75608998 | NA | |
| rs74686099 | 18 | 75861241 | NA | |
| rs77331644 | 18 | 75861619 | NA | |
| rs12458593 | 18 | 75862469 | NA | |
| rs113119443 | 18 | 76384089 | NA | |
| 18:76393189:G:A | 18 | 76393189 | NA | NA |
| rs11672916 | 19 | 1036631 | CNN2 | intron_variant |
| rs116δ7191 | 19 | 1036718 | CNN2 | intron_variant |
| rs11672975 | 19 | 1036793 | CNN2 | intron_variant |
| rs11670441 | 19 | 1036913 | CNN2 | intron_variant |
| rs11665845 | 19 | 1037170 | CNN2 | intron_variant |
| rs113353568 | 19 | 1037551 | CNN2 | intron_variant |
| rs111525696 | 19 | 1037563 | CNN2 | intron_variant |
| rs72973561 | 19 | 1037584 | CNN2 | intron_variant |
| rs2232775 | 19 | 8373152 | CD320 | missense_variant |
| rs7249111 | 19 | 8378870 | AC010323. 1 | intron_variant |
| rs8109861 | 19 | 8379894 | AC010323.1 | intron_variant |
| rs8110376 | 19 | 8380149 | AC010323.1 | intron_variant |

[Table 1-74]

| SNP ID | chr | position | gene locus | SNP location in gene locus |
|---|---|---|---|---|
| rs11669423 | 19 | 21091222 | NA | |
| rs11667242 | 19 | 21092697 | NA | |
| rs2288896 | 19 | 30067812 | NA | |
| 19:44183972:G:C | 19 | 44183972 | NA | NA |
| rs145903192 | 19 | 44185599 | NA | |
| rs112639353 | 19 | 44187225 | NA | |
| rs79273520 | 19 | 44187737 | NA | |
| rs75691094 | 19 | 44195271 | AC005622.1 | intron_variant |
| rs140415511 | 19 | 44195583 | AC005622.1 | intron_variant |
| rs201608105 | 19 | 44198053 | AC005622.1 | intron_variant |
| rs59447107 | 19 | 44199652 | AC005622.1 | intron_variant |
| rs148383153 | 19 | 44200274 | AC005622.1 | intron_variant |
| 19:56565924A:G | 19 | 56565924 | NA | NA |
| rs117472015 | 19 | 56566789 | NLRP5 | intron_variant |
| rs199933435 | 19 | 56574059 | NA | |
| rs79145402 | 19 | 56576351 | NA | |
| rs56070738 | 20 | 6655570 | NA | |
| rs62194864 | 20 | 6655626 | NA | |
| rs62194867 | 20 | 6663199 | NA | |
| rs8121041 | 20 | 6664256 | NA | |
| rs7272805 | 20 | 6666442 | NA | |
| rs118144766 | 20 | 11371362 | NA | |
| rs147182367 | 20 | 11374504 | NA | |
| rs3067207 | 20 | 11516851 | NA | |
| rs6111325 | 20 | 16858631 | NA | |
| rs73247070 | 20 | 16872792 | NA | |
| rs6105671 | 20 | 16874257 | NA | |
| rs6105672 | 20 | 16874319 | NA | |
| rs75411817 | 20 | 17437518 | PCSK2 | intron_variant |
| rs2284912 | 20 | 17438213 | PCSK2 | intron_variant |
| rs199610440 | 20 | 17444153 | PCSK2 | intron_variant |
| rs73900815 | 20 | 17445166 | PCSK2 | intron_variant |
| rs2269028 | 20 | 17446328 | PCSK2 | intron_variant |
| rs2269029 | 20 | 17446362 | PCSK2 | intron_variant |
| rs2269035 | 20 | 17450530 | PCSK2 | intron_variant |
| rs117870212 | 20 | 17796971 | NA | |

[Table 1-75]

| SNP ID | chr | position | gene locus | SNP location in gene locus |
|---|---|---|---|---|
| rs149160109 | 20 | 19787051 | RIN2 | intron_variant |
| 20:19957482:G:GTC | 20 | 19957482 | NA | NA |
| rs60381469 | 20 | 19958065 | RIN2 | intron_variant |
| rs143672948 | 20 | 42432890 | NA | |
| rs17728712 | 20 | 50064221 | NFATC2 | intron_variant |
| rs6096436 | 20 | 50064556 | NFATC2 | intron_variant |
| rs79332570 | 20 | 55465963 | NA | |
| rs12624457 | 20 | 55642740 | NA | |
| rs16980692 | 20 | 55643296 | NA | |
| rs6014915 | 20 | 55643568 | NA | |
| rs1923179 | 20 | 55644273 | NA | |
| rs78441963 | 20 | 55657024 | NA | |
| rs201245644 | 20 | 55681308 | NA | |
| rs8115658 | 20 | 55686994 | NA | |
| rs149841438 | 20 | 55704642 | NA | |
| rs142759888 | 20 | 55708318 | NA | |
| rs76064436 | 20 | 55750436 | BMP7 | intron_variant |
| rs6128207 | 20 | 56436532 | NA | |
| rs117535594 | 20 | 57263529 | STX16-NPEPL1 | intron_variant |
| rs242$13 | 20 | 58649794 | NA | |
| 20:61493128:C:T | 20 | 61493128 | NA | NA |
| 20:61493129:GCC:G | 20 | 61493129 | NA | NA |
| 20:61493132:G:T | 20 | 61493132 | NA | NA |
| 20:61493133:GCCACA:G | 20 | 61493133 | NA | NA |
| rs144044846 | 21 | 19485388 | CH0DL | intron_variant |
| rs139603158 | 21 | 32201681 | KRTAP7-1 | 3_prime_UTR_variant |
| rs66481055 | 21 | 40382795 | NA | NA |
| rs450808 | 21 | 43706944 | ABCG1 | intron_variant |
| rs77905583 | 21 | 44278878 | WDR4 | intron_variant |
| rs77931345 | 21 | 44282099 | WDR4 | intron_variant |
| rs112237505 | 21 | 44283785 | WDR4 | intron_variant |
| rs5748948 | 22 | 17694856 | ADA2 | intron_variant |
| rs117100146 | 22 | 17700318 | ADA2 | 5_prime_UTR_variant |
| rs200828580 | 22 | 19162510 | NA | |
| rs5746673 | 22 | 19163047 | NA | |
| rs190264011 | 22 | 19166705 | NA | |

[Table 1-76]

| SNP ID | chr | position | gene locus | SNP location in gene locus |
|---|---|---|---|---|
| rs5748024 | 22 | 19168288 | CLTCL1 | missense_variant |
| rs193038051 | 22 | 19169259 | CLTCL1 | intron_variant |
| rs5748025 | 22 | 19169793 | CLTCL1 | intron_variant |
| rs139626 | 22 | 25224556 | SGSM1 | intron_variant |
| rs139629 | 22 | 25225119 | SGSM1 | intron_variant |
| rs139633 | 22 | 25225879 | SGSM1 | intron_variant |
| rs139637 | 22 | 25228375 | SGSM1 | intron_variant |
| rs139644 | 22 | 25232065 | SGSM1 | intron_variant |
| rs139648 | 22 | 25233260 | SGSM1 | intron_variant |
| rs139649 | 22 | 25233565 | SGSM1 | intron_variant |
| rs132548 | 22 | 29317559 | ZNRF3 | intron_variant |
| rs5997861 | 22 | 31464163 | SMTN | intron_variant |
| 22:31466683:G:T | 22 | 31466683 | NA | NA |
| rs7287041 | 22 | 31469691 | SMTN | intron_variant |
| rs9621179 | 22 | 31472299 | SMTN | intron_variant |
| rs35749963 | 22 | 39244118 | NA | |
| rs9611038 | 22 | 39247482 | NA | |
| rs9607583 | 22 | 39248667 | NA | |
| rs5764095 | 22 | 44530469 | PARVB | intron_variant |
| rs118092933 | 22 | 44547847 | PARVB | intron_var i ant |
| rs8137152 | 22 | 44750797 | NA | |
| 22:47168035:T:C | 22 | 47168035 | NA | NA |
| 22:47168059:T:C | 22 | 47168059 | NA | NA |
| rs184367871 | 22 | 47177009 | TBC1D22A | intron_variant |
| rs112425324 | 22 | 48826561 | NA | |
| rs140475504 | 22 | 49593633 | NA | |
| 22:49780452:C:T | 22 | 49780452 | NA | NA |
| rs5934808 | 23 | 10164106 | CLCN4 | intron_variant |
| rs5979274 | 23 | 10164535 | CLCN4 | intron_variant |
| rs2238891 | 23 | 10165403 | CLCN4 | intron_variant |
| rs72626456 | 23 | 42378072 | NA | |
| rs5909566 | 23 | 118006329 | NA | |
| rs59778322 | 23 | 118053423 | NA | |
| rs35026453 | 23 | 118058304 | NA | |
| rs200499538 | 23 | 118062390 | NA | NA |
| X: 118069424: CTCTT: C | 23 | 118069424 | NA | NA |

[Table 1-77]

| SNP ID | chr | position | gene locus | SNP location in gene locus |
|---|---|---|---|---|
| rs5957082 | 23 | 118072203 | NA | |
| rs200504262 | 23 | 118073507 | NA | |
| rs4825392 | 23 | 118076725 | NA | |
| rs5910471 | 23 | 118120492 | L0NRF3 | intron_variant |
| rs112219900 | 23 | 118820044 | SEPTIN6 | intron_variant |
| rs6634729 | 23 | 128444668 | NA | |
| rs72614106 | 23 | 128449159 | NA | |
| rs6637592 | 23 | 128543945 | NA | |
| rs6637593 | 23 | 128551239 | NA | |
| rs6637596 | 23 | 128559316 | NA | |
| rs185542983 | 23 | 143920769 | NA | |
| rs137885389 | 23 | 151775134 | NA | NA |
| rs4400524 | 23 | 151777266 | NA | |
| rs58154948 | 23 | 151793551 | NA | |

(5) The information processing method according to (4), in which the mutations further include one or more mutations shown in Tables 2-1 to 2-9.

[Table 2-1]

| SNP ID | chr | position | gene locus |
|---|---|---|---|
| rs1209461 | 1 | 58408470 | DAB1 |
| rs1202850 | 1 | 58408855 | DAB1 |
| rs1202851 | 1 | 58409755 | DAB1 |
| rs1202788 | 1 | 58414715 | DAB1 |
| rs1202790 | 1 | 58417348 | DAB1 |
| rs969719 | 2 | 50874681 | CTNNA3 |
| rs79725983 | 2 | 50876601 | CTNNA3 |
| rs9309188 | 2 | 50916658 | NA |
| rs9309190 | 2 | 50918967 | NA |
| rs7606024 | 2 | 56616595 | SYT9 |
| rs2566747 | 2 | 82304139 | MAML2 |
| rs2116045 | 2 | 82315179 | MAML2 |
| rs7601451 | 2 | 82322288 | NA |
| rs10202137 | 2 | 82322613 | NA |
| rs12998884 | 2 | 82323175 | NA |
| rs7605223 | 2 | 82323314 | NA |
| rs5832525 | 2 | 82324709 | NA |
| rs1346859 | 2 | 82324973 | NA |
| rs10173814 | 2 | 82326240 | NA |

80

(continued)

| SNP ID | chr | position | gene locus |
|---|---|---|---|
| rs10197852 | 2 | 82326536 | NA |
| rs1346860 | 2 | 82327886 | NA |
| rs10658639 | 2 | 82335834 | NA |
| rs113286166 | 2 | 82335943 | NA |
| rs4852212 | 2 | 82337967 | NA |
| rs1430708 | 2 | 82339149 | NA |
| 2:82340763:C:T | 2 | 82340763 | NA |
| 2:82340764:A:C | 2 | 82340764 | NA |
| 2:82340765:C:A | 2 | 82340765 | NRXN3 |
| rs10206958 | 2 | 82342060 | NA |
| rs7583816 | 2 | 82344289 | PDXDC1 |
| rs1521665 | 2 | 82373290 | PDXDC1 |
| rs1367437 | 2 | 82379605 | PDXDC1 |
| rs1367438 | 2 | 82380031 | PDXDC1 |
| rs10204952 | 2 | 82383092 | PDXDC1 |
| 2:82383337:AT:A | 2 | 82383337 | PDXDC1 |
| rs6704771 | 2 | 82384630 | PDXDC1 |
| rs2052957 | 2 | 82388160 | PDXDC1 |
| rs934308 | 2 | 82391200 | NA |
| rs10432699 | 2 | 82391773 | NTAN1 |
| rs934309 | 2 | 82393511 | NTAN1 |
| rs10190586 | 2 | 82394929 | NTAN1 |
| rs4852215 | 2 | 82401288 | NA |
| rs11676296 | 2 | 82402793 | NTAN1 |

[Table 2-2]

| SNP ID | chr | position | gene locus |
|---|---|---|---|
| rs1367442 | 2 | 82403420 | NTAN1 |
| rs1430723 | 2 | 82405835 | NTAN1 |
| rs4516453 | 2 | 82405937 | NTAN1 |
| rs2902367 | 2 | 82407091 | RBF0X1 |
| rs1430725 | 2 | 82410156 | NA |
| rs7561758 | 2 | 82410827 | GAS7 |
| rs11894492 | 2 | 82413470 | GAS7 |
| rs12471807 | 2 | 82413980 | GAS7 |
| rs4571075 | 2 | 82433870 | NA |
| rs7599433 | 2 | 82436635 | NA |
| rs4852682 | 2 | 82440268 | NA |

(continued)

| SNP ID | chr | position | gene locus |
|---|---|---|---|
| rs11690894 | 2 | 82452553 | NA |
| rs16856198 | 2 | 131609539 | NA |
| rs12464519 | 2 | 131622215 | NA |
| rs7573895 | 2 | 131625295 | NA |
| rs34469492 | 2 | 131628354 | ARHGEF4 |
| 2:131629169:TAC:T | 2 | 131629169 | ARHGEF4 |
| rs3072355 | 2 | 131631093 | ARHGEF4 |
| rs12693750 | 2 | 154396396 | ARHGEF4 |
| rs1595456 | 2 | 154396518 | NA |
| rs6747679 | 2 | 154397389 | ARHGEF4 |
| rs6736497 | 2 | 154397886 | NA |
| rs7605995 | 2 | 154398711 | NA |
| rs7603189 | 2 | 154398824 | NA |
| 2:154398862:C:A | 2 | 154398862 | NA |
| 2:154398866:C:A | 2 | 154398866 | NA |
| 2:154398870:C:A | 2 | 154398870 | NA |
| 2:154411738:T:C | 2 | 154411738 | NA |
| 2:154411747:A:G | 2 | 154411747 | NA |
| rs77398198 | 2 | 173160555 | NA |
| rs55687168 | 2 | 212203087 | NA |
| rs16845916 | 2 | 212203146 | NA |
| rs74342940 | 2 | 238791983 | NA |
| rs4685035 | 3 | 13847602 | NA |
| rs4685036 | 3 | 13848830 | NA |
| rs6777825 | 3 | 13850933 | RAMP1 |
| rs9812834 | 3 | 76104023 | NRXN1 |
| rs66550319 | 3 | 77725567 | NRXN1 |
| rs6444327 | 3 | 167158977 | NRXN1 |
| rs9874384 | 3 | 167161621 | NRXN1 |
| rs12487400 | 3 | 167162076 | NA |
| rs9859743 | 3 | 167162439 | NA |
| rs13085856 | 3 | 167164661 | NA |

[Table 2-3]

| SNP ID | chr | position | gene locus |
|---|---|---|---|
| rs13081388 | 3 | 167164968 | NA |
| rs9853197 | 3 | 167166760 | NA |
| rs11373865 | 3 | 167167493 | NA |

(continued)

| SNP ID | chr | position | gene locus |
|---|---|---|---|
| rs1104570 | 3 | 167167997 | NA |
| rs9863185 | 3 | 167168148 | NA |
| 3:167200293: T:TA | 3 | 167200293 | NA |
| rs74715659 | 3 | 187690581 | NA |
| rs78575164 | 3 | 187691168 | NA |
| rs77292072 | 3 | 187692633 | NA |
| rs78313080 | 3 | 187693634 | NA |
| rs17745214 | 3 | 187695429 | NA |
| rs17807098 | 3 | 187696291 | NA |
| rs60889349 | 3 | 187696727 | NA |
| rs56398386 | 4 | 4890108 | NA |
| rs62291849 | 4 | 4910704 | NA |
| rs6446700 | 4 | 4911642 | NA |
| rs10029683 | 4 | 4926097 | NA |
| rs10866392 | 4 | 32100576 | NA |
| rs7434982 | 4 | 32102920 | NA |
| rs7700063 | 4 | 32108077 | NA |
| rs4331815 | 4 | 32109975 | NA |
| rs4501264 | 4 | 32113715 | NA |
| 4:32135307:A:T | 4 | 32135307 | NA |
| rs12502887 | 4 | 36986503 | NA |
| rs10034215 | 4 | 37006612 | NA |
| rs10023279 | 4 | 37006976 | NA |
| rs113390631 | 4 | 37007446 | NA |
| rs7686039 | 4 | 37008671 | NA |
| rs36022714 | 4 | 37009144 | NA |
| rs13111377 | 4 | 37013371 | NA |
| rs9995577 | 4 | 37014190 | NA |
| rs10028774 | 4 | 37014223 | NA |
| rs13127134 | 4 | 37019361 | NA |
| rs13111292 | 4 | 37020558 | NA |
| rs9306943 | 4 | 37024228 | NA |
| rs1885879 | 4 | 37029967 | NA |
| rs13145678 | 4 | 37030696 | NA |
| rs34180164 | 4 | 37030722 | NA |
| rs77850243 | 4 | 37031126 | NA |
| rs145655984 | 4 | 37031740 | NA |
| r s 11417369 | 4 | 37032249 | NA |

(continued)

| SNP ID | chr | position | gene locus |
|---|---|---|---|
| rs1885880 | 4 | 37034278 | NA |
| rs17289939 | 4 | 37036317 | NA |

[Table 2-4]

| SNP ID | chr | position | gene locus |
|---|---|---|---|
| rs34767825 | 4 | 37038306 | NA |
| rs61540608 | 4 | 37040276 | JPH2 |
| rs17289946 | 4 | 37042065 | NA |
| rs13127946 | 4 | 37042611 | NA |
| rs34800491 | 4 | 37044502 | NA |
| 4:37044634:AG:A | 4 | 37044634 | NA |
| rs7655238 | 4 | 37045620 | NA |
| rs11096857 | 4 | 37046140 | NA |
| rs2608817 | 4 | 39427201 | SERPINI2 |
| rs200342845 | 4 | 39428057 | SERPINI2 |
| rs1979283 | 4 | 39430969 | SERPINI2 |
| rs2687978 | 4 | 39431576 | SERPINI2 |
| 4.39432027:T:C | 4 | 39432027 | SERPINI2 |
| rs1542423 | 4 | 39432132 | SERPINI2 |
| rs2926042 | 4 | 39432747 | SERPINI2 |
| rs3107672 | 4 | 39432966 | SERPINI2 |
| rs12643330 | 4 | 74729606 | SERPINI2 |
| rs28391070 | 4 | 166476773 | NA |
| rs28487969 | 4 | 166478246 | NA |
| rs7658683 | 4 | 166478405 | NA |
| rs7659327 | 4 | 166478477 | NA |
| rs1914836 | 4 | 166482946 | NA |
| 4:166484748:CT:C | 4 | 166484748 | NA |
| rs79544601 | 4 | 166484791 | NA |
| rs17046745 | 4 | 166485142 | NA |
| rs1914835 | 4 | 166485729 | R0B02 |
| rs1914834 | 4 | 166485893 | NA |
| rs6831798 | 4 | 166487806 | NA |
| rs6855033 | 4 | 166488036 | NA |
| rs10017297 | 4 | 166488615 | NA |
| rs150507584 | 4 | 166488986 | NA |
| rs6819473 | 4 | 166489595 | NA |

(continued)

| SNP ID | chr | position | gene locus |
|---|---|---|---|
| rs17046756 | 4 | 166491564 | NA |
| rs7694281 | 4 | 166492825 | NA |
| rs7694612 | 4 | 166492946 | NA |
| rs28583482 | 4 | 166494345 | NA |
| rs10517996 | 4 | 169425778 | NA |
| rs2218255 | 4 | 180035723 | NA |
| rs4410622 | 5 | 4672392 | NA |
| rs1496366 | 5 | 7146388 | NA |
| rs1006152 | 5 | 7151032 | NA |
| rs1494558 | 5 | 35861068 | NA |
| rs6888116 | 5 | 118659579 | NA |

[Table 2-5]

| SNP ID | chr | position | gene locus |
|---|---|---|---|
| rs1876736 | 5 | 118665393 | NA |
| rs250307 | 5 | 118667748 | NA |
| rs250306 | 5 | 118669094 | NA |
| rs1895202 | 5 | 118669851 | PALLD |
| rs250305 | 5 | 118670120 | NA |
| rs1355123 | 5 | 118675585 | NA |
| rs2652696 | 5 | 132139308 | NA |
| rs708460 | 5 | 132144898 | NA |
| rs254289 | 5 | 132167542 | NA |
| rs254287 | 5 | 132172048 | NA |
| rs254293 | 5 | 132178571 | NA |
| rs809140 | 5 | 132193555 | NA |
| rs254285 | 5 | 132198287 | NA |
| rs2431138 | 5 | 174833410 | NA |
| rs265984 | 5 | 174836280 | NA |
| 6:32579277:C:T | 6 | 32579277 | NA |
| rs116953562 | 6 | 32676388 | NA |
| rs114931041 | 6 | 32677158 | NA |
| rs116823632 | 6 | 32677235 | NA |
| rs112057165 | 6 | 32677822 | NA |
| rs114912316 | 6 | 32677960 | NA |
| rs114134393 | 6 | 32678064 | LINC02616 |
| rs116915647 | 6 | 32679726 | NA |
| rs114788646 | 6 | 32679762 | NA |

(continued)

| SNP ID | chr | position | gene locus |
|---|---|---|---|
| rs116115695 | 6 | 32680012 | NA |
| rs114245356 | 6 | 32680153 | NA |
| rs115025251 | 6 | 32680267 | NA |
| rs115719910 | 6 | 32680352 | NA |
| rs115942220 | 6 | 32680448 | NA |
| rs114095721 | 6 | 32680867 | NA |
| rs115938991 | 6 | 32681079 | NA |
| rs115036360 | 6 | 32681308 | NA |
| rs2254737 | 7 | 17811797 | NA |
| rs2110015 | 7 | 17812739 | NA |
| rs2254594 | 7 | 17813614 | NA |
| rs2723521 | 7 | 17813651 | NA |
| rs2723520 | 7 | 17813677 | NA |
| rs2723519 | 7 | 17814005 | NA |
| rs1404419 | 7 | 17814029 | NA |
| rs2723518 | 7 | 17814046 | KLB |
| rs2537592 | 7 | 17814051 | KLB |
| rs1404418 | 7 | 17814085 | KLB |
| rs1404417 | 7 | 17814165 | KLB |

[Table 2-6]

| SNP ID | chr | position | gene locus |
|---|---|---|---|
| rs2723517 | 7 | 17814202 | NA |
| rs1404416 | 7 | 17814272 | KLB |
| rs2723516 | 7 | 17814284 | KLB |
| rs2537593 | 7 | 17814531 | KLB |
| rs2537594 | 7 | 17814536 | NA |
| rs2723514 | 7 | 17814548 | NA |
| rs2068172 | 7 | 17814607 | NA |
| 7:17814644:AT:A | 7 | 17814644 | NA |
| rs2723513 | 7 | 17814888 | NA |
| rs2254361 | 7 | 17815625 | TNFAIP8 |
| rs2691 617 | 7 | 17822328 | TNFAIP8 |
| rs2691621 | 7 | 17822450 | TNFAIP8 |
| rs146211890 | 7 | 17823353 | TNFAIP8 |
| rs145940129 | 7 | 17823499 | TNFAIP8 |
| rs2691625 | 7 | 17823624 | TNFAIP8 |
| rs72079142 | 7 | 17824200 | TNFAIP8 |

(continued)

| SNP ID | chr | position | gene locus |
|---|---|---|---|
| rs2691627 | 7 | 17824251 | SEPTIN8 |
| rs2691628 | 7 | 17824356 | NA |
| rs2691629 | 7 | 17824631 | NA |
| rs2723500 | 7 | 17824735 | NA |
| rs2691630 | 7 | 17824787 | NA |
| rs2723499 | 7 | 17824885 | NA |
| rs2723498 | 7 | 17824986 | GDF9 |
| rs2254407 | 7 | 17825281 | NA |
| rs998342 | 7 | 17825589 | NA |
| rs2254176 | 7 | 17825605 | IL7R |
| rs144839998 | 7 | 17825719 | NA |
| rs2723497 | 7 | 17825828 | NA |
| rs2723496 | 7 | 17825991 | NA |
| rs2691553 | 7 | 17826282 | NA |
| rs1852010 | 7 | 17826368 | NA |
| rs1852011 | 7 | 17826628 | NA |
| rs1404420 | 7 | 17831018 | NA |
| rs2691583 | 7 | 17831806 | NA |
| rs2293768 | 7 | 100348384 | NA |
| rs78389571 | 7 | 100368389 | NA |
| rs2293766 | 7 | 100371358 | NA |
| rs17147741 | 7 | 100371583 | NA |
| 7:100372539:GTTTTTTGT:G | 7 | 100372539 | NA |
| rs56872215 | 7 | 100376414 | NA |
| rs75119362 | 7 | 100376679 | NA |
| rs77017835 | 7 | 100377364 | NA |
| rs113223392 | 7 | 100378274 | NA |

[Table 2-7]

| SNP ID | chr | position | gene locus |
|---|---|---|---|
| 7:100382845:CTT:C | 7 | 100382845 | NA |
| rs2622261 | 7 | 153522341 | NA |
| rs2622262 | 7 | 153522368 | NA |
| rs2622263 | 7 | 153522433 | ZAN |
| rs113278579 | 7 | 153522636 | ZAN |
| rs2538419 | 7 | 153522987 | ZAN |
| rs2538418 | 7 | 153523926 | ZAN |
| rs10687210 | 7 | 153524089 | NA |

(continued)

| SNP ID | chr | position | gene locus |
| --- | --- | --- | --- |
| rs1544613 | 7 | 153524357 | ZAN |
| rs1544612 | 7 | 153524514 | ZAN |
| rs1544611 | 7 | 153525542 | ZAN |
| rs2622241 | 7 | 153526331 | ZAN |
| rs2622242 | 7 | 153526464 | NA |
| rs2070494 | 8 | 37688733 | NA |
| rs10957210 | 8 | 62969408 | NA |
| rs4871749 | 8 | 127897460 | NA |
| rs1863563 | 8 | 129020784 | NA |
| rs12550891 | 9 | 26569055 | NA |
| rs1033988 | 9 | 26571951 | NA |
| rs10819940 | 9 | 98927435 | NA |
| rs10819941 | 9 | 98927481 | NA |
| rs4743609 | 9 | 98930736 | NA |
| rs4743611 | 9 | 98932408 | NA |
| rs10819949 | 9 | 98934331 | NA |
| rs4742873 | 9 | 98950164 | NA |
| rs4742876 | 9 | 98955876 | NA |
| rs7020106 | 9 | 98974141 | NA |
| rs7047022 | 9 | 98974144 | NA |
| rs7035399 | 9 | 101776487 | NA |
| rs77405629 | 9 | 112867809 | NA |
| rs17806439 | 9 | 112886940 | NA |
| rs13297551 | 9 | 120961083 | NA |
| rs71895773 | 9 | 120961500 | NA |
| rs79652695 | 10 | 6976748 | NA |
| rs75366909 | 10 | 68602734 | NA |
| rs17231504 | 10 | 68650441 | NA |
| rs1320042 | 11 | 7444027 | NA |
| rs4753718 | 11 | 95830567 | NA |
| rs7944701 | 11 | 95831023 | NA |
| rs7934906 | 11 | 119488133 | NA |
| rs1855286 | 13 | 47746652 | NA |
| rs1537457 | 13 | 47752877 | NA |
| rs4142743 | 13 | 47756210 | NA |

[Table 2-8]

| SNP ID | chr | position | gene locus |
|---|---|---|---|
| rs4142742 | 13 | 47756308 | NA |
| rs2406095 | 13 | 47762205 | NA |
| rs1930826 | 13 | 47767039 | NA |
| rs7332153 | 13 | 47767339 | NA |
| rs6561358 | 13 | 47770535 | NA |
| rs7333999 | 13 | 47771258 | NA |
| rs10140617 | 14 | 51843558 | NA |
| rs2356901 | 14 | 51845948 | NA |
| rs7153577 | 14 | 78790792 | NA |
| rs58546183 | 14 | 101716329 | NA |
| rs78489963 | 14 | 101719276 | NA |
| rs75949493 | 14 | 101719529 | NA |
| rs12587451 | 14 | 101719796 | NA |
| 15:87561879:AT:ATT | 15 | 87561879 | NA |
| rs2034597 | 16 | 6144047 | NA |
| rs4985124 | 16 | 15125441 | NA |
| rs72789541 | 16 | 15127534 | NA |
| rs72789542 | 16 | 15127535 | NA |
| rs7200543 | 16 | 15129970 | NA |
| rs1741 | 16 | 15130351 | NA |
| rs6498540 | 16 | 15130594 | NA |
| rs1121 | 16 | 15131076 | NA |
| rs4985154 | 16 | 15131642 | NA |
| 16:15131654:C:CA | 16 | 15131654 | NA |
| rs1135999 | 16 | 15131962 | NA |
| rs1136001 | 16 | 15131974 | SNX13 |
| rs2740 | 16 | 15132108 | SNX13 |
| 16:15132211:A:AAAAGTTG | 16 | 15132211 | NA |
| rs34614532 | 16 | 15132908 | NA |
| rs14347 | 16 | 15133889 | ADGRA2 |
| rs4500751 | 16 | 15140211 | NA |
| rs6498541 | 16 | 15140657 | C0L15A1 |
| rs7192753 | 16 | 80098249 | PALM2AKAP2 |
| rs1024370 | 17 | 10054798 | PALM2AKAP2 |
| rs1024369 | 17 | 10054889 | NA |
| rs1468086 | 17 | 10055514 | NA |
| rs4794500 | 17 | 52577372 | NA |
| rs60809192 | 17 | 52578429 | NA |

(continued)

| SNP ID | chr | position | gene locus |
|---|---|---|---|
| rs113532194 | 17 | 52581343 | NA |
| rs56347481 | 17 | 52582030 | NA |
| rs11871596 | 17 | 52582282 | NA |
| rs7226207 | 17 | 52583485 | NA |
| rs753719 | 19 | 411849 | NA |

[Table 2-9]

| SNP ID | chr | position | gene locus |
|---|---|---|---|
| rs6028721 | 20 | 38550395 | NA |
| rs761207 | 20 | 42758834 | NA |
| 20:60359090:G:A | 20 | 60359090 | NA |
| rs6001258 | 22 | 39245583 | NA |

(6) An information processing method including: a detection unit configured to detect a first SNP which is a mutation of an Alzheimer's disease-associated gene in a genomic DNA sample derived from a subject; and a determination unit configured to determine whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

(7) A program causing a computer to execute: Step 1 of detecting a first SNP which is a mutation of an Alzheimer's disease-associated gene in a genomic DNA sample derived from a subject; and Step 2 of determining whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

(8) A method for predicting a risk of developing Alzheimer's disease using the information processing method according to any one of (1) to (5).

[Advantageous Effects of Invention]

According to the information processing method, information processing device, and program of the above-described aspect, it is possible to predict a risk of developing AD in subjects. The prediction of the onset risk can contribute to improvement in AD prevention or therapeutic effects. Furthermore, new therapeutic targets for AD can be provided.

[Brief Description of Drawings]

FIG. 1 is a view showing an example of a configuration of an information processing device 100 according to a first embodiment.
FIG. 2A is a flowchart showing a flow of a series of runtime processes of a processing unit 120 according to the first embodiment.
FIG. 2B is a view showing an example of a prediction model MDL according to the first embodiment.
FIG. 2C is a flowchart showing a flow of a series of training processes of the processing unit 120 according to the first embodiment.
FIG. 3A is a graph showing the amount of Aβ40 corresponding to apolipoprotein E (APOE) ε4 genotypes in Example 1, the number of patients with an APOE 3/3 genotype being n=44, the number of patients with an APOE 3/4 genotype being n=44, the number of patients with an APOE 4/4 genotype being n=14, and "N.S." indicating no significant difference (P>0.05).

FIG. 3B is a graph showing the amount of Aβ42 corresponding to the APOE-ε4 genotypes in Example 1, the number of patients with an APOE 3/3 genotype being n=44, the number of patients with an APOE 3/4 genotype being n=44, the number of patients with an APOE 4/4 genotype being n=14, and "N.S." indicating no significant difference (P>0.05).

FIG. 3C is a graph showing Aβ42/40 ratios corresponding to the APOE-ε4 genotypes in Example 1, the number of patients with an APOE 3/3 genotype being n=44, the number of patients with an APOE 3/4 genotype being n=44, and the number of patients with an APOE 4/4 genotype being n=14.

FIG. 3D is a graph showing protein concentrations of iPS cell-derived neurons corresponding to the APOE-ε4 genotypes in Example 1, the number of patients with an APOE 3/3 genotype being n=44, the number of patients with an APOE 3/4 genotype being n=44, the number of patients with an APOE 4/4 genotype being n=14, and "N.S." indicating no significant difference (P>0.05).

FIG. 4A is a scatter diagram (N=102) showing the amount of Aβ40 corresponding to ages at onset of cognitive dysfunction in Example 1.

FIG. 4B is a scatter diagram (N=102) showing the amount of Aβ42 corresponding to ages at onset of cognitive dysfunction in Example 1.

FIG. 4C is a scatter diagram (N=102) showing Aβ42/40 ratios corresponding to ages at onset of cognitive dysfunction in Example 1.

FIG. 4D is a plot showing the amount of Aβ40 in culture supernatants of iPS cell-derived neurons, corresponding to sex in Example 1, the number of male patients being n=36, and the number of female patients being n=66.

FIG. 4E is a plot showing the amount of Aβ42 in the culture supernatants of the iPS cell-derived neurons, corresponding to sex in Example 1, the number of male patients being n=36 and the number of female patients being n=66.

FIG. 4F is a plot showing Aβ42/40 ratios in the culture supernatants of the iPS cell-derived neurons, corresponding to sex in Example 1, the number of male patients being n=36 and the number of female patients being n=66.

FIG. 5A is a Manhattan plot of a genome-wide association study of cellular dissection of polygenicity (CDiP) to identify gene loci associated with the Aβ42/40 ratios without considering the APOE-ε4 genotypes in Example 1. The x-axis shows chromosomes, and y-axis shows -log10 (p-value) of all SNPs tested. The upper line indicates the Bonferroni-corrected significance threshold value ($p < 5 \times 10^{-8}$).

FIG. 5B is a Manhattan plot of a genome-wide association study of CDiP to identify gene loci associated with the Aβ42/40 ratios considering the APOE-ε4 genotypes in Example 1. The x-axis indicates chromosomes, and y-axis indicates -log10 (p-value) of all SNPs tested. The upper line indicates the Bonferroni-corrected significance threshold value ($p < 5 \times 10^{-8}$).

FIG. 5C is a graph showing results of pathway analysis of 24 genes identified through CDiP using the AP42/40 ratios in Example 1. The horizontal axis indicates p-values.

FIG. 6A is a plot showing phosphorylated tau (p231-tau)/total tau ratios corresponding to the APOE-ε4 genotypes in Example 1, the number of patients with an APOE 3/3 genotype being n=44, the number of patients with an APOE 3/4 genotype being n=44, the number of patients with an APOE 4/4 genotype being n=14, and "N.S." indicating no significant difference (P>0.05).

FIG. 6B is a plot showing p231-tau/total tau ratios in the culture supernatants of the iPS cell-derived neurons, corresponding to sex in Example 1, the number of male patients being n=36 and the number of female patients being n=66.

FIG. 6C is a scatter diagram (N=102) showing p231-tau/total tau ratios corresponding to ages at onset of cognitive dysfunction in Example 1.

FIG. 6D is a Manhattan plot of a genome-wide association study of CDiP to identify gene loci associated with the p231-tau/total tau ratios considering the APOE-ε4 genotypes in Example 1. The x-axis indicates chromosomes, and y-axis indicates - log10 (p-value) of all SNPs tested. The upper line indicates the Bonferroni-corrected significance threshold value ($p < 5 \times 10^{-8}$).

FIG. 6E is a Manhattan plot of a genome-wide association study of CDiP to identify gene loci associated with the p231-tau/total tau ratios without considering the APOE-ε4 genotypes in Example 1. The x-axis indicates chromosomes, and y-axis indicates -log10 (p-value) of all SNPs tested. The upper line indicates the Bonferroni-corrected significance threshold value ($p < 5 \times 10^{-8}$).

FIG. 7A is a graph showing alteration in Aβ42/40 ratios due to knockdown of the identified genes in Example 1. The x-axis indicates the level of alteration in Aβ42/40 ratios compared to a non-siRNA-treated control. The values are shown as mean±standard deviation. * is p<0.05, ** is p and 0.01, *** is p<0.005, and **** is p<0.001.

FIG. 7B is a graph showing alteration in amount of Aβ40 due to knockdown of the identified genes in Example 1. The x-axis indicates the level of alteration in amount of Aβ40 compared to a non-siRNA-treated control. The values are shown as mean±standard deviation. * is p<0.05, ** is p and 0.01, *** is p<0.005, and **** is p<0.001.

FIG. 7C is a graph showing alteration in amount of Aβ42 due to knockdown of the identified genes in Example 1.

The x-axis indicates the level of alteration in amount of Aβ42 compared to a non-siRNA-treated control. The values are shown as mean±standard deviation. * is p<0.05, ** is p and 0.01, *** is p<0.005, and **** is p<0.001.

FIG. 7D is a graph showing alteration in protein concentration due to knockdown of the identified genes in Example 1. The x-axis indicates the level of alteration in protein concentration compared to a non-siRNA-treated control. The values are shown as mean±standard deviation. * is p<0.05, ** is p and 0.01, *** is p<0.005, and **** is p<0.001.

FIG. 7E is a graph comparing expression levels in neurons of genes in which siRNA altered the Aβ42/40 ratios between Alzheimer's disease brains and non-dementia control brains in Example 1.

FIG. 7F is a graph comparing expression levels in neurons of genes in which siRNA reduced the amount of Aβ42 between the Alzheimer's disease brains and the non-dementia control brains in Example 1.

FIG. 7G is a view showing genes which are identified using single cell-based transcriptomic data of 6 AD brains and 6 control brains providing transcriptomic data for individual cell types in Example 1, show higher expression in the AD brains, and can be therapeutic targets.

FIG. 8A is a box plot showing a relationship between the ages at onset and Aβ-positive and Aβ-negative patients in Example 2, the number of Aβ-positive patients being 15 and the number of Aβ-negative patients being 4.

FIG. 8B is a plot showing a relationship between the amount of Aβ40 in culture supernatants of cerebral cortical neurons induced from human iPS cells and the Aβ-positive and Aβ-negative patients in Example 2, the number of Aβ-positive patients being n=15 and the number of Aβ-negative patients being n=4.

FIG. 8C is a plot showing a relationship between the amount of Aβ42 in the culture supernatants of the cerebral cortical neurons induced from the human iPS cells and the Aβ-positive and Aβ-negative patients in Example 2, the number of Aβ-positive patients being n=15 and the number of Aβ-negative patients being n=4.

FIG. 8D is a plot showing a relationship between Aβ42/40 ratios in the culture supernatants of the cerebral cortical neurons induced from the human iPS cells and the Aβ-positive and Aβ-negative patients in Example 2, the number of Aβ-positive patients being n=15 and the number of Aβ-negative patients being n=4.

FIG. 9A shows graphs illustrating prediction results of Aβ deposition of brains in which covariates (age, sex, and APOE-ε4 allele genotypes) (left graph) in Example 2 or covariates and genotype sets identified through CDiP (right graph) were used.

FIG. 9B shows graphs illustrating prediction results of the amount of Aβ (1-42) in cerebrospinal fluid (CSF) in which covariates (age, sex, and APOE-ε4 allele genotypes) (left graph) in Example 2 or covariates and genotype sets identified through CDiP (right graph) were used.

FIG. 9C shows graphs illustrating prediction results of total tau (t-tau) levels in CSF in which covariates (age, sex, and APOE-ε4 allele genotypes) (left graph) in Example 2 or covariates and genotype sets identified through CDiP (right graph) were used.

FIG. 9D shows graphs illustrating prediction results of phosphorylated tau (p-tau) levels in CSF in which covariates (age, sex, and APOE-ε4 allele genotypes) (left graph) in Example 2 or covariates and genotype sets identified through CDiP (right graph) were used.

[Description of Embodiments]

Embodiments of the present invention will be described. The following embodiments are merely examples for explaining the present invention, and are not intended to limit the present invention to these embodiments. The present invention can be implemented in various forms without departing from the gist thereof.

<Mutation of Alzheimer's disease (AD)-associated gene>

The present inventors have used cerebral cortical neurons induced from iPS cells established from sporadic AD patients to perform cell GWAS (GWAS) using an Aβ42/40 ratio which is one of the pathological indicators of AD as a phenotype, and they have found mutations shown in Tables 1-1 to 1-77 above as mutations associated with the Aβ42/40 ratio among AD-associated genes as shown in examples described below. In addition, as shown in the examples described below, when comparing a case where AD-associated gene mutations including one or more mutations shown in Tables 1-1 to 1-77 above and information such as age, sex, and APOE4 genotypes considered to be involved in intracerebral accumulation of Aβ are analyzed with a case where only the information such as age, sex, and APOE4 genotypes considered to be involved in intracerebral accumulation of Aβ are analyzed without analyzing the AD-associated gene mutations including one or more mutations shown in Tables 1-1 to 1-77 above, a higher AUC score which is one of the indicators of prediction accuracy can be obtained in the case where AD-associated gene mutations including one or more mutations shown in Tables 1-1 to 1-77 above and the information such as age, sex, and APOE4 genotypes considered to be involved in intracerebral accumulation of Aβ are analyzed. Specifically, it is possible to predict whether intracerebral accumulation of Aβ would occur with an accuracy of AUC=0.76±0.050 using the SNP information of the sporadic AD patients, and it is possible to predict whether abnormal test values for Aβ in cerebrospinal fluid would occur with an accuracy of AUC=0.73±0.059 using the SNP information of the sporadic AD patients. Such intracerebral accumulation of Aβ and abnormal test values for Aβ in cerebrospinal fluid are almost consistent with a clinical diagnosis of AD. Accordingly, it is possible to predict the risk of developing AD using the information processing device and the information processing method of the present embodiment with an accuracy of AUC of about 0.7 (more specifically, about 0.73 to 0.76). For sporadic AD, rather than familial AD (hereditary AD), no method to date has been able to accurately predict the risk with AUC in the above-described numerical ranges. In contrast, in the information processing device and the information processing method of the present embodiment, it is possible to provide a method for determining the risk of AD with high accuracy or high predictability by determining the risk by analyzing the SNP set including one or more mutations shown in Tables 1-1 to 1-77 above. That is, the information processing device and the information processing method of the present embodiment can be called an AD onset risk prediction device and method. In addition, according to the information processing device and the information processing method of the present embodiment, it is possible to predict a risk of developing AD in subjects including subjects suspected of having sporadic AD without a family history.

Furthermore, in a disease, such as AD, caused by a plurality of genes, stratification of patients into subtypes based on common characteristics is of great significance in the prevention and treatment of the disease. This is because effective means for prevention of treatment may differ depending on the subtypes. As shown in the examples described below, the information processing device and the information processing method of the present embodiment can also contribute to AD stratification. This can also contribute to precision medicine.

The term "risk of Alzheimer's disease (AD)" in the present specification refers to the probability of suffering from Alzheimer's disease, such as susceptibility to or resistance to AD. The expression "determining the risk" includes, for example, outputting the probability of suffering from AD at present or in the future by dividing it into several levels and outputting it as a numerical value. Determining the risk of AD involves assessing genetic factors or genetic susceptibility to a disease, such as whether a patient has susceptibility to or resistance to AD.

In determining the risk of AD, it does not matter whether or not a subject undergoing the AD risk assessment actually has (has developed) AD at the time of the AD risk assessment.

One or more of the mutations shown in Tables 1-1 to 1-77 above can be used, and the mutations shown in Tables 1-1 to 1-77 above are SNPs that have not previously been found to be associated with AD. That is, AD is thought to develop due to a combined action of polygenes, and the risk of AD can be determined with higher accuracy by analyzing two or more mutations shown in Tables 1-1 to 1-77 as an integrated set of SNPs rather than by individually analyzing the mutations shown in Tables 1-1 to 1-77 above. Accordingly, it is preferable to use an SNP set including all mutations shown in Table 4 described below (between the mutations shown in Tables 1-1 to 1-77 above, mutations particularly highly associated with AD from the viewpoint of Aβ42/40 ratios), it is more preferable to use an SNP set including mutations having a p-value of less than $5\times10^{-8}$ between mutations shown in Tables 3-1 to 3-77 described below, and it is still more preferable to use an SNP set including all the mutations shown in Tables 3-1 to 3-77.

In addition, as shown in the examples described below, the present inventors have found, between AD-associated gene mutations, mutations shown in Tables 2-1 to 2-9 above as mutations associated with a phosphorylated tau/total tau ratio. Accordingly, one or more mutations shown in Tables 2-1 to 2-9 above can be further included. However, since Ad is thought to be developed by a combined action of polygenes, it is preferable to use an SNP set further including mutations shown in Table 6 described below (between the mutations shown in Tables 2-1 to 2-9 above, mutations particularly highly associated with AD from the viewpoint of the phosphorylated tau/total tau ratio) among the mutations associated with the phosphorylated tau/total tau ratio in addition to the above-described mutations associated with the Aβ42/40 ratio, and it is more preferable to use an SNP set further including all the mutations shown in Tables 2-1 to 2-9 above.

For the SNPs described in each table of the present specification, rs numbers, chromosome numbers where each SNP is present (indicated by X or Y in a case of a sex chromosome), and the position of each SNP on the chromosome are listed. The information such as base sequences or diseases relating to each SNP can be obtained, for example, by searching the NCBI SNP Database based on the rs numbers. Such information can be referred to the database and is incorporated herein by reference. The position of each SNP on the chromosome corresponds to the assembly genome version GRCh37.

As shown in each table, each SNP can be identified by referring to a base sequence identified by an rs number. In a case where an rs number described in the present specification is merged with another rs number and a new rs number is given, the corresponding rs number in the present specification includes the merged rs number and the other rs number to be merged. In addition, in a case where an rs number described in the present specification is a number assigned by merging a plurality of rs numbers, the corresponding rs number in the present specification includes other original rs numbers.

In addition, a base sequence indicated by each rs number relating to an SNP is indicated as a specific base sequence by referring to a database such as the NCBI SNP Database, but a base sequence in a part of the base sequence other than the corresponding SNP may be changed due to differences in race and the like.

In the information processing method, the information processing device, and the program of the present embodiment, the race and sex of a subject are not limited.

Hereinafter, the information processing method, the information processing device, and the program to which the present invention is applied will be described with reference to the drawings.

<First Embodiment>

[Overall structure]

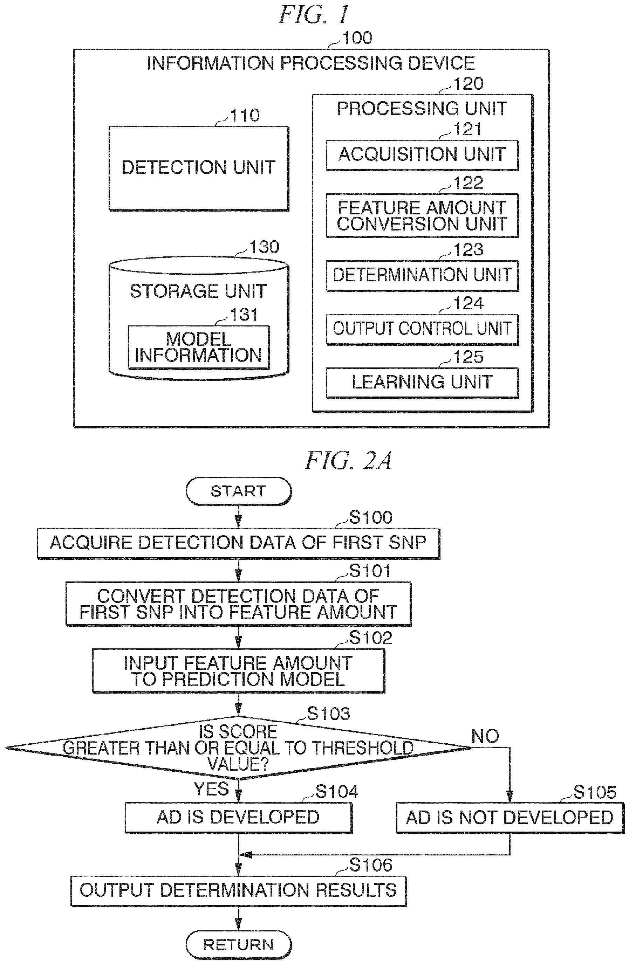

FIG. 1 is a view showing an example of a configuration of an information processing device 100 of a first embodiment. As shown in FIG. 1, the information processing device 100 includes, for example, a detection unit 110, a processing unit 120, and a storage unit 130.

(Detection unit)

In the detection unit 110, an SNP (hereinafter referred to as a first SNP) which is a mutation of an Alzheimer's disease (AD)-associated gene in a genomic DNA sample derived from a subject is detected (Step 1).

Cells or tissues collected from a living body of a subject can be used as a genomic DNA sample derived from a subject. Samples are not particularly limited as long as they contain nucleated cells, and examples thereof include blood, cerebrospinal fluid, lymphatic fluid, and hair. Among these, blood can be preferably used because of its low invasiveness, and examples of blood-derived nucleated cells include peripheral blood mononuclear cells. For detection of SNPs, genomic DNA isolated from these samples through a usual method may be directly used, or the isolated genomic DNA may be amplified and the amplified genomic DNA may be used.

A method for extracting genomic DNA is not particularly limited, and a well-known method can be used for extraction. Examples thereof include a phenolchloroform method and a cetyltrimethylammonium bromide (CTAB) method. A commercially available kit may be used for DNA extraction. Examples of kits include Wizard Genomic DNA Purification Kit (manufactured by Promega Corporation).

The detection unit 110 is composed of a device used for normal genetic polymorphism analysis. Examples of such devices include: a DNA microarray; a conventional sequencer or a next generation sequencer (NGS); and a nucleic acid amplifier such as a polymerase chain reaction (PCR) device.

SNPs can be detected through a well-known SNP detection method using the above exemplified devices, and examples of the methods to be used include a direct sequencing method, a PCR method, a restriction fragment length polymorphism (RFLP) method, a hybridization method, a TaqMan (registered trademark) PCR method (hereinafter, the description of "registered trademark" will not be repeated), and mass spectrometry.

The direct sequencing method involves cloning a region containing an SNP into a vector or amplifying the region through PCR, and determining a base sequence of the region. As a cloning method, the region can be cloned by performing screening from cDNA library using an appropriate probe. In addition, the region can be amplified through a PCR reaction using an appropriate primer and linked to an appropriate vector to clone the region. Furthermore, the region can be subcloned into another vector, but the present invention is not limited thereto. As vectors, for example, commercially available plasmid vectors such as pBlue-Script SK (+) (manufactured by Stratagene), pGEM-T (manufactured by Promega Corporation), pAmp (manufactured by Gibco-BRL), p-Direct (manufactured by Clontech), and pCR2.1-TOPO (manufactured by Invitrogene), virus vectors, artificial chromosome vectors, and cosmid vectors can be used. A well-known method can be used for determining a base sequence. Examples thereof include a manual sequencing method using a radioactive marker nucleotide and an automatic sequencing method using a dye terminator, but are not limited thereto. Whether or not a sample has an SNP is determined based on a base sequence obtained in this manner.

The PCR method is performed using an oligonucleotide primer (hereinafter sometimes referred to as an "SNP detection primer") that hybridizes only with a sequence having an SNP. Since there are a plurality of SNPs, as an SNP detection primer, a primer capable of detecting all the SNPs may be used alone, or a combination of two or more primers capable of detecting each SNP may be used. These primers are used to amplify DNA of a specimen. In a case where a SNP detection primer produces a PCR product, the sample is considered to have an SNP. In a case where no PCR product is produced, this indicates that the sample is free of SNPs.

In the RFLP method, first, a region containing an SNP in a sample is amplified through PCR. Subsequently, this PCR product is cleaved with a restriction enzyme appropriate for the region containing an SNP. The restriction enzyme-digested PCR product is separated through gel electrophoresis and visualized through ethidium bromide staining. The presence of an SNP in a sample can be detected by comparing the length of the fragment with, for example, a molecular weight marker and, as a control, the above-described PCR product which has not been treated with a restriction enzyme.

The hybridization method is a method for determining the presence or absence of an SNP in a sample based on the property of DNA in the sample to hybridize with a complementary DNA molecule (for example, an oligonucleotide probe). Such a hybridization method can be performed using a variety of techniques for detection and hybridization such as well-known hybridization such as colony hybridization, plaque hybridization, and Southern blotting. For a detailed procedure of the hybridization method, "Molecular Cloning, A Laboratory Manual 3rd ed." (Cold Spring Harbor Press (2001); especially Sections 6-7), "Current Protocols in Molecular Biology" (John Wiley&Sons (1987-1997); especially Sections 6.3-6.4), "DNA Cloning 1: Core Techniques, A Practical Approach 2nd ed." (Oxford University (1995); especially Section 2.10 for hybridization conditions), and the like can be referred to. Furthermore, hybridization can also be detected using a DNA chip. In this method, an SNP-specific oligonucleotide probe is designed and attached to a solid phase support. Then, DNA in a sample is brought into contact with the DNA chip to detect hybridization.

The TaqMan PCR method is a method of performing SNP detection and amplification of a region containing an SNP simultaneously and in parallel using Taq polymerase and a TaqMan probe specific to an SNP. A TaqMan probe is an oligonucleotide of about 20 bases labeled with a fluorescent substance at the 5' end and a quencher at the 3' end, and is designed to hybridize with an SNP site of interest. Taq polymerase has 5'→ 3' nuclease activity. When a region containing a target SNP site is amplified using a PCR primer designed to amplify the region containing the SNP site in the presence of such Taq polymerase and TaqMan probe, the TaqMan probe is hybridized with the target SNP site of template DNA in parallel with the amplification. When the extension reaction from the forward primer side reaches the TaqMan probe hybridized with the template, the 5'→3' nuclease activity of the Taq polymerase cleaves the fluorescent substance which has been bound to the 5' end of the TaqMan probe. As a result, the released fluorescent substance is no longer affected by the quencher and emits fluorescence. SNPs can be detected through measurement of the fluorescence intensity.

As a method using mass spectrometry, a method combined with the primer extension method can also be exemplified as SNP-typing method to which a MALDI-TOF/MS method is applied. This method enables high-throughput analysis which is performed by steps of 1) PCR, 2) purification of PCR products, 3) primer extension reaction, 4) purification of extension products, 5) mass spectrometry, and 6) determination of genotypes. First, a region containing a target SNP site is amplified from genomic DNA through PCR. A PCR primer is designed so as not to overlap with an SNP site base. Then, purification is performed through an enzymatic removal method using an exonuclease or shrimp alkaline phosphatase, or through an ethanol precipitation method. Next, a primer extension reaction is performed using a genotyping primer designed so that the 3' end is directly adjacent to the SNP site. The PCR product is denatured at high temperature, and an excess amount of genotyping primer is added thereto for annealing. When ddNTP and a DNA polymerase are added to the reaction system and subjected to a thermal cycle reaction, an oligomer one base longer than the genotyping primer is produced. The oligomer one-base-long oligomer produced through this extension reaction differs according to alleles due to the above-described design of the genotyping primer. The purified extension reaction product is subjected to mass spectrometry and analyzed from a mass spectrum.

As another detection method, a method to which single-molecule fluorescence spectrometry is applied is exemplified as an SNP-typing method enabling high throughput. For example, MF20/10S (manufactured by Olympus Corporation) is a system that employs this method. Specifically, the single-molecule-level translational diffusion time of a fluorescent-labeled primer amplified through a PCR method using complementary and non-complementary primers in a ultrafine region of about 1 femtoliter (1/1000 trillion liter) is measured and analyzed using a confocal laser optical system and a high-sensitivity photodetector.

In addition, a method using a DNA chip is also one of the typing enabling high throughput. A DNA chip has many types of DNA probes arrayed and immobilized on a substrate, and a labeled DNA sample is hybridized on the chip to detect fluorescent signals from the probes.

An example of the SNP-typing method using a gene amplification method other than the PCR method is a Snipper method. The method is an SNP-typing method to which a rolling circle amplification (RCA) method which is a DNA amplification method in which complementary strand DNA is synthesized while a DNA polymerase moves on circular single-stranded DNA as a template is applied. Each probe is oligo-DNA with a length of 80 bases to 90 bases, contains a sequence of 10 bases to 20 bases in length complementary to the vicinity of the 5' end and the 3' end of a target SNP site at both ends, and is designed to be annealed to target DNA and become circular. In addition, the 3' end of the probe is designed to have a sequence complementary to the target SNP site. If the 3' end of the probe is perfectly complementary to the target SNP site, the probe will be circularized, but if the 3' end of the probe is mismatched, the probe will not be circularized. In addition, the probe has a backbone sequence of 40 to 50 bases in length and contains sequences complementary to two types of RCA amplification primers.

Other examples of SNP-typing methods using a gene amplification method other than the PCR method include a typing method using a UCAN method or a LAMP method.

The UCAN method is a method to which an ICAN method which is an isothermal gene amplification method developed by Takara Bio is applied. In the UCAN method, a DNA-RNA-DNA (DRD) chimeric oligonucleotide is used as a primer precursor. In this DRD primer precursor, DNA at the 3' end is modified so that replication of template DNA due to a DNA polymerase does not occur, and the DRD primer precursor is designed such that the RNA site binds to an SNP site. When this DRD primer precursor is incubated with the template, the coexisting RNase H cleaves the RNA portion of the paired DRD primer only when the DRD primer and the template are perfectly matched. As a result, the modified DNA is removed from the 3' end of the primer to form a new one, so that an extension reaction due to a DNA polymerase proceeds and the template DNA is amplified. On the other hand, when the DRD primer and the template DNA do not match, RNase H does not cleave the DRD primer and DNA amplification does not occur. The amplification reaction after the perfectly matched DRD primer precursor is cleaved by RNase H proceeds through an ICAN reaction mechanism.

The LAMP method is an isothermal gene amplification method developed by Liken Chemical Co., Ltd., in which six regions (F3c, F2c, and F1c from the 3' end side, and B3, B2, and B1 from the 5' end side) of a target gene are defined, and four types of primers (an FIP primer, an F3 primer, a BIP primer, and a B3 primer) for the six regions are used for amplification. For the purpose of typing, only the target SNP site (1 base) is sufficient between F1 and B1, and the FIP primer and the BIP primer are designed so that the 1 base of the SNP is at the 5' end. In a case where there is no SNP, a DNA synthetic reaction occurs from a dumbbell structure which is an origin structure of the LAMP method, and the amplification reaction proceeds continuously. In a case where there is an SNP, no DNA synthetic reaction occurs from the dumbbell structure, and the amplification reaction does not proceed.

An invader method is a method that uses two types of non-fluorescence-labeled probes (allele probe and an invader probe), one type of fluorescence-labeled probe (a FRET probe), and Cleavase which is an endonuclease without using a nucleic acid amplification method. The allele probe has a sequence complementary to the template DNA on the 3' end side from the SNP site, and a sequence unrelated to the template DNA called a flap on the 5' end side of the probe. The invader probe has a complementary sequence on the 5' end side from the SNP site of the template DNA, and the portion corresponding to the SNP site has any base. The FRET probe has a sequence complementary to the flap sequence on the 3' end side. One 5' end side is labeled with a fluorescent dye and a quencher, but the FRET probe is designed to form a double strand intramolecularly and is usually quenched. When these are reacted with the template DNA, the 3' end (arbitrary base portion) of the invader probe invades the SNP site when the allele probe forms a double strand with the template DNA. Cleavase recognizes the structure invaded by the base and cleaves the flap portion of the allele probe. When this released flap then binds to the complementary sequence of the FRET probe, the 3' end of the flap invades the intramolecular double-stranded portion of the FRET probe. Similarly to the cases of the above-described allele probe and invader probe, Cleavase recognizes the structure in which the base of the flap has invaded the FRET probe, and cleaves the fluorescent dye of the FRET probe. Since the fluorescent dye moves away from the quencher, fluorescence is generated. In a case where the allele probe does not match the template DNA, the above-described specific structure recognized by Cleavase is not formed, and therefore, the flap is not cleaved.

In a case where primers are used for detecting SNPs, they are designed to be suitable for a region to be amplified and a typing method. For example, it is preferable to be able to fully amplify the above-described region, and sequences can be designed based on sequences near both ends of the above-described region. Techniques for designing primers are well known in the technical field, and primers that can be used in the method of the present embodiment are designed to satisfy conditions that allow specific annealing, for example, to have a length and a base composition (melting temperature) that allow specific annealing. The length of the region to be amplified is not limited as long as it does not interfere with typing, and may be increased or decreased depending on the detection method. In addition, although a part of the region to be amplified contains an SNP site, there is no restriction on the position of the site in the region to be amplified, and the site may be arranged at an appropriate position according to the detection method (typing method). For this reason, when designing a primer, the positional relationship between the primer and an SNP site can be freely designed according to the detection method, and the primer can be designed while taking into account the characteristics of the typing method as long as it is hybridized with the region containing an SNP to be detected (for example, consecutive 50 to 500 bases in length). The length that exhibits the function as a primer is preferably 10 to 100 bases, more preferably 15 to 50 bases, and still more preferably 15 to 30 bases. In addition, in designing, it is preferable to confirm the melting temperature (Tm) of the primer which is a temperature at which 50% of an arbitrary nucleic acid strand forms a hybrid with its complementary strand. For a template DNA and a primer to form a double strand and anneal, it is necessary for the annealing temperature to be optimized. On the other hand, if the temperature is too low, nonspecific reactions may occur, which is not preferable. Well-known software for designing a primer can be used to confirm Tm.

In a case where probes are used to detect SNPs, they are designed to recognize SNP sites. In designing a probe, an SNP site may be recognized anywhere in a probe according to the typing method, and may be recognized at an end of a probe depending on the typing method. In a case where an SNP detection polynucleotide is used as a probe, the length of a base sequence complementary to a genomic DNA is usually 15 bases to 200 bases, preferably 15 bases to 100 bases, and more preferably 15 bases to 50 bases. However, it may be longer or shorter depending on the typing method.

(Processing unit)

In the processing unit 120, it is determined whether or not the subject will develop AD from the SNP (that is the "first SNP") detected by the detection unit 110 using a machine learning model trained on a plurality of training datasets labeled with information on the onset of AD for an SNP, which is a mutation of the AD-associated gene detected in a genomic DNA sample derived from a patient who has developed AD (Step 2). Hereinafter, the SNP contained in the training datasets will be referred to as a "second SNP."

The processing unit 120 includes, for example, an acquisition unit 121, a feature amount conversion unit 122, a determination unit 123, an output control unit 124, and a machine learning unit 125.

The constituent elements of the processing unit 120 are realized by, for example, a processor, such as a central processing unit (CPU) or a graphics processing unit (GPU), executing a program stored in the storage unit 130. In addition, some or all of the constituent elements of the processing unit 120 may be executed by hardware (circuitry) such as large-scale integration (LSI), an application-specific integrated circuit (ASIC), or a field-programmable gate array (FPGA), or may be realized by cooperation of software and hardware.

(Storage unit)

The storage unit 130 is realized by a storage device such as a hard disc drive (HDD), flash memory, electrically erasable programmable read-only memory (EEPROM), read-only memory (ROM), or random-access memory (RAM). The storage unit 130 stores model information 131 in addition to various programs such as application programs and firmware. The model information 131 will be described below.

[Runtime processing flow]

Hereinafter, a flow of a series of runtime processes of a processing unit 120 according to the first embodiment will be described based on a flowchart. Runtime is a state of using an already trained prediction model MDL. FIG. 2A is a flowchart showing a flow of a series of runtime processes of the processing unit 120 according to the first embodiment. The processing of this flowchart may be performed repeatedly at a predetermined cycle, for example.

First, the acquisition unit 121 acquires detection data of the first SNP which is a mutation of an Alzheimer's disease-associated gene in a genomic DNA sample derived from a subject from the detection unit 110 (Step S100). The acquired detection data of the first SNP can also be said to be genotype data of the AD-associated gene possessed by the subject (hereinafter sometimes referred to as a "first SNP set" or "subject's genotype data").

Next, the feature amount conversion unit 122 converts the data of the first SNP acquired by the acquisition unit 121 into a feature amount that can be input to the model (Step S101). The feature amount referred to herein is, for example, a parameter indicating whether the genotype data of the subject is any of a homozygous type (AA), homozygous type (BB), and a heterozygous type (AB) for each SNP. In general, the genotype is indicated by nucleotides such as "GG" indicating that both SNPs on homologous chromosomes are guanine (G), or "AG" indicating that one is guanine (G) and the other is adenine (A). Therefore, the genotype data of the subject is converted into a parameter that can be input to a model in which the second SNP (the genotype data of the AD-associated gene possessed by the AD patient (hereinafter sometimes referred to as a "second SNP set" or "AD patient's genotype data")) which is a mutation of the AD-associated gene detected in the genomic DNA sample derived from the patient who has developed AD. However, in a case where the model does not require such conversion into a parameter, the above-described conversion is not required.

The conversion of the genotype data of the subject into a feature amount can be performed, for example, by assigning a value to the genotype data of the subject for each SNP contained in the second SNP set. For example, for each SNP, a value (for example, 0, 1, or 2) is assigned to each SNP depending on whether the genotype data of the subject corresponds to any of a homozygous type (AA), homozygous type (BB), and a heterozygous type (AB). Accordingly, the genotype data of the subject can be converted into a feature amount. A case where the value corresponding to each SNP is set to 0, 1, or 2 will be described below as an example, but the value corresponding to each SNP is not limited to the three values of 0, 1, and 2.

The values corresponding to the junction types can be determined for each SNP. For example, for an SNP, a value of 2 may be assigned in a case where the genotype data of the subject is a homozygous type (AA), a value of 1 may be assigned in a case where the genotype data is a heterozygous type (AB), and a value of 0 may be assigned in a case where the genotype data is a homozygous type (BB), and for the other SNP, a value of 2 may be assigned in a case where the genotype data of the subject is a heterozygous type (AB), a value of 1 may be assigned in a case where the genotype data is a homozygous type (AA), and a value of 0 may be assigned in a case where the genotype data is a homozygous type (BB). In addition, a value of 2 may be assigned in a case where the genotype data of the subject is a homozygous type (BB), a value of 1 may be assigned in a case where the genotype data is a heterozygous type (AB), and a value of 0 may be assigned in a case where the genotype data is a homozygous type (AA).

As described above, the genotype data of the subject can be converted into a feature amount. A value used for the assignment in the conversion into this feature amount can be arbitrarily determined. For example, a value of 2 can be assigned to a genotype with high relevance to AD for each SNP, and a value of 1 or 0 can be assigned to a genotype with low relevance to AD for each SNP.

Next, the determination unit 123 inputs the feature amount converted from the first SNP data by the feature amount conversion unit 122 to a prediction model MDL indicated by the model information 131 (Step S102).

The model information 131 is information (a program or a data structure) that defines the prediction model MDL for determining a risk of AD from the genotype data of the subject.

The prediction model MDL is realized by a logistic regression model, neural networks such as a multilayer perceptron, a convolutional neural network (CNN), and a recurrent neural network (RNN), a support vector machine using an arbitrary kernel function such as the Gaussian kernel, Random Forest modeled as a regression tree, multiple regression analysis, models using a hidden Markov model and the like, and other various models such as a statistical model or a probabilistic model. In addition, it is also possible to employ a model that combines various models to perform a comprehensive determination. For example, the prediction model MDL may be Random Forest including a plurality of classifiers. Hereinafter, an example in which the prediction model MDL is Random Forest will be described.

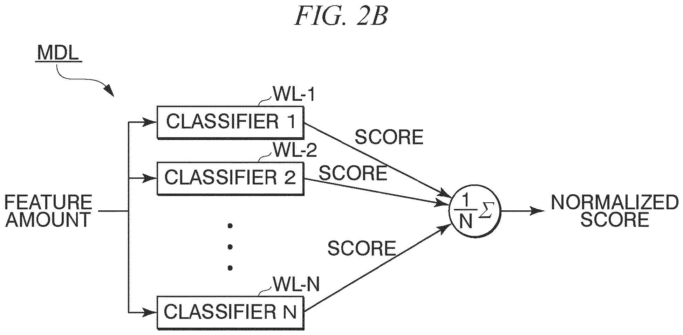

FIG. 2B is a view showing an example of a prediction model MDL according to the first embodiment. The prediction model MDL includes, for example, N classifiers WL-1 to WL-N. Each classifier WL is a weak learner that is preliminarily trained to output a score indicating the likelihood or probability that a subject will develop AD, as a likelihood or probability, when a feature amount converted from data of a first SNP is input. The classifiers WL are in parallel with each other. The technique of generating one machine learning model by combining a plurality of weak learners in this manner is called ensemble learning.

For example, the prediction model MDL normalizes the score of each classifier WL, which is a weak learner, and outputs the normalized score. The normalization of the score is shown in Equation (1).

[Equation 1]

$$S = \frac{1}{N} \sum_{i=1}^{N} s_i \qquad \cdots (1)$$

S in the equation represents a normalized score, and $s_i$ represents a score of the i-th classifier WL. The scores $S_i$ and S are, for example, two-dimensional vectors (=[P1, P2]) whose elements are probability P1 of developing AD and probability P2 of not developing AD. As shown in Equation (1), the prediction model MDL may normalize the scores by dividing the sum of the scores of all classifiers WL by N which is the sum of the classifiers WL. By using ensemble learning in this manner, it is possible to improve the prediction accuracy of the onset of AD for unknown (untrained) data that has not been used in training described later.

The prediction model MDL is a combination of N classifiers WL as shown in FIG. 2B, but is not limited thereto. For example, the prediction model MDL may be one classifier WL.

The description will return to the flowchart of FIG. 2A. Next, the determination unit 123 determines whether or not the score (normalized score) output by the prediction model MDL is greater than or equal to a threshold value (Step S103).

The determination unit 123 determines that there is a high probability that a subject will develop AD in a case where the score is greater than or equal to the threshold value (Step S104), and determines that there is a low probability that a subject will develop AD in a case where the score is less than the threshold value (Step S105).

Next, the output control unit 124 outputs the determination results (for example, information indicating the probability of the onset of Alzheimer's disease) by the determination unit 123 (Step S106). For example, the output control unit 124 may transmit the determination results to an external terminal device (not shown in the drawing) via a communication interface. The communication interface is, for example, a wireless communication module or a network card such as a network interface card (NIC). In addition, in a case where the information processing device 100 includes a display (not shown in the drawing), the output control unit 124 may display the determination results on the display.

[Training processing flow]

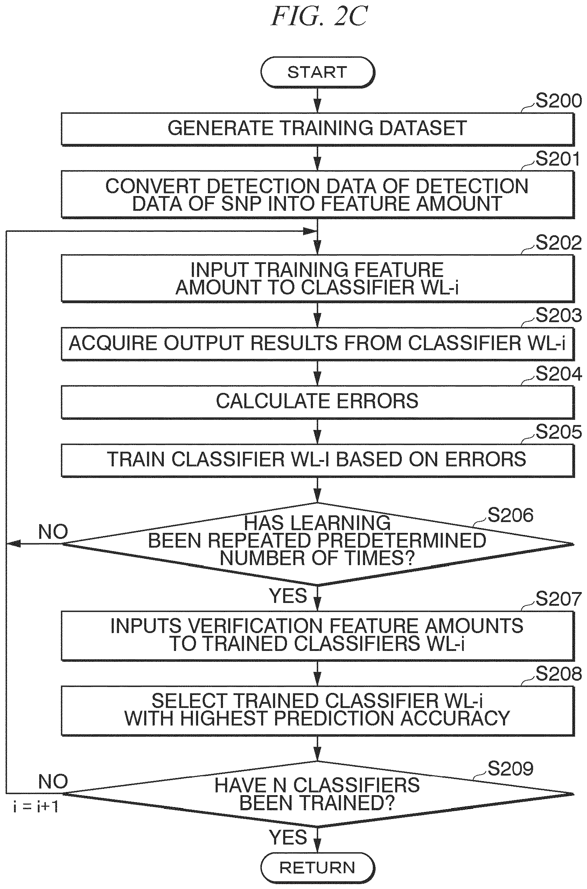

Hereinafter, a flow of a series of training processes of the processing unit 120 according to the first embodiment will be described based on a flowchart. Training is a state in which the prediction model MDL used at runtime is trained. FIG. 2C is a flowchart showing a flow of a series of training processes of the processing unit 120 according to the first embodiment.

First, the machine learning unit 125 generates a training dataset for training the prediction model MDL (Step S200). For example, the training dataset is a dataset in which genotype data of AD-associated genes possessed by healthy persons or AD patients are labeled with information on the onset of AD of the subjects (for example, positive or negative for the onset of AD). In a case where the second SNP set contains some unknown SNPs, an attributed genotype inferred through genotype imputation can be used.

For example, genotype data of AD-associated genes possessed by healthy persons or AD patients are acquired. Genotype data of AD-associated genes possessed by healthy persons are labeled with information (for example, a score of 0.0) indicating that they have not developed AD, and genotype data of AD-associated genes possessed by AD patients are labeled with information (for example, a score of 1.0) indicating that they have developed AD.

In this manner, when genotype data of the plurality of labeled AD-associated genes are generated as a training dataset, the feature amount conversion unit 122 converts the genotype data of the AD-associated genes contained in the training dataset into feature amounts (Step S201).

Next, the machine learning unit 125 divides the plurality of feature amounts converted from the genotype data of the AD-associated genes in the training dataset by the feature amount conversion unit 122 into training feature amounts (training samples) and verification feature amounts (test samples), and the training feature amounts are input to the i-th classifier WL-i out of the N classifiers WL included in the prediction model MDL as weak learners (Step S202).

The machine learning unit 125 may use main component analysis when dividing the plurality of feature amounts converted from the genotype data of the AD-associated genes in the training dataset, that is, the feature amounts of the population, into training feature amounts (training samples) and verification feature amounts (test samples). For example, the machine learning unit may select the training feature amounts (training samples) from the feature amounts of the population based on information that is a main component between genetic information and attribute information of the healthy persons or the AD patients who are the providers of the AD-associated genes. The attribute information of the healthy persons or the AD patients may contain, for example, information such as age and sex. The genetic information of the healthy persons and the AD patients may contain, for example, information on whether or not they have APOE-$\varepsilon$4 genotypes, and other information. The training feature amounts (training samples) selected based on the main component are an example of a "specific training dataset."

Next, the machine learning unit 125 acquires output results, that is, scores $s_i$ from the i-th classifier WL-i to which the training feature amounts are input (Step S203).

Next, the machine learning unit 125 calculates errors (also referred to as loss) between the scores si acquired from the i-th classifier WL-i and the scores labeled with the training feature amounts (Step S204).

Next, the machine learning unit 125 determines a parameter of the i-th classifier WL-i to reduce the errors (Step S205).

Next, the machine learning unit 125 determines whether training of the i-th classifier WL-i has been repeated a predetermined number of times E (Step S206). In a case where the training has not reached the predetermined number of times E, the process returns to S202, and training of the i-th classifier WL-i is repeated by inputting the same feature amounts as the training feature amounts used for the training in the previous process to the i-th classifier WL-i. At this time, the machine learning unit 125 stores the parameter updated by training in the storage unit 130 and inputs the training feature amounts to the i-th classifier WL-i whose parameter is initialized. As a result, E classifiers WL-i with different parameters are generated until the training of the i-th classifier WL-i reaches a predetermined number of times E.

On the other hand, in a case where the training of the i-th classifier WL-i reaches a predetermined number of times E, the machine learning unit 125 inputs the verification feature amounts to the E i-th classifiers WL-i (Step S207).

Next, the machine learning unit 125 selects a classifier WL-i with the highest prediction accuracy among the E i-th classifiers WL-i (Step S208). For example, the machine learning unit 125 selects a classifier WL-i with the smallest errors between the scores of the training data and the scores si obtained when the verification feature amounts are input among the E i-th classifiers WL-i.

Next, the machine learning unit 125 determines whether or not all the N classifiers WL included in the prediction model MDL as weak learners have been trained (Step S209). In a case where the N classifiers WL have not yet been trained, the process returns to S202, and an (i+1)th classifier WL-(i+1) is trained on the training feature amounts.

On the other hand, in a case where all the N classifiers WL have been trained, the machine learning unit 125 terminates the process of this flowchart.

According to the first embodiment described above, the information processing device 100 inputs a feature amount converted from genotype data of an AD-associated gene possessed by a subject to a prediction model MDL trained on a training dataset in which the feature amount converted from the genotype data of the AD-associated gene is labeled with information indicating whether the onset of AD is positive or negative, and predicts that the subject will develop AD based on output results of the prediction model MDL to which the feature amounts are input. Therefore, the information processing device can accurately predict whether or not the subject will develop AD in the future.

In general, it is difficult to predict the risk of AD in subjects who have not developed AD, such as infants and young adults. On the other hand, in the present embodiment, since a prediction model MDL including a plurality of classifiers WL realized by a machine learning model is used, it is possible to expect that a weighting that indicates that a specific SNP is positively correlated with the risk of AD in genotype data of an AD-associated gene can be calculated as a score. As a result, it is possible to predict the risk of AD in subjects who have not developed AD, such as infants and young adults at an early stage.

In addition, according to the above-described first embodiment, since ensemble learning is performed on a plurality of classifiers WL included as weak learners in the prediction model MDL, the prediction model MDL with high prediction accuracy can be generated.

<Modification example of first embodiment>

Hereinafter, a modification example of the first embodiment will be described. In the first embodiment described above, the training dataset has been described as data in which genotype data of AD-associated genes possessed by healthy persons or AD patients are labeled with a score indicating whether or not the healthy persons or AD patients will develop AD, but is not limited thereto. For example, the training dataset may be data in which genotype data of AD-associated genes possessed by healthy persons or AD patients are labeled with age at onset of AD in addition to the above-described score. When the genotype data of the AD-associated genes are input using such a training dataset, the machine learning unit 125 trains the prediction model MDL to output a three-dimensional vector (=[P1, P2, t]) whose elements are probability P1 of developing AD, probability P2 of not developing AD, and age t at onset of AD. The determination unit 123 predicts the age at which a subject will develop AD based on the element of t of the vector output by the prediction model MDL.

In addition, the label is not limited to the score indicating the presence or absence of the onset of AD or the age at onset of AD, and may contain attributes of the subjects who are the providers of the genotype data of the AD-associated genes. The attributes may contain, for example, various pieces of information such as sex, weight, height, lifestyle habits, presence or absence of illness, and family medical history. In addition, the attributes may contain genetic information of well-known AD-associated genotypes such as APOEε4 genotypes. By training the prediction model MDL using the genotype data of AD-associated genes assigned to such labels, the prediction model MDL with even higher prediction accuracy can be generated. As a result, by inputting the subject's attributes into the prediction model MDL in addition to the genotype data of the AD-associated genes at runtime, it is possible to more accurately predict the subject's future risk of AD.

The determination results obtained as described above are also used as aid when an AD specialist diagnoses AD. That is, the information processing device and the information processing method of the present embodiment can also be called an AD diagnosis support device and method.

(Another Embodiment)

In one embodiment, the present invention provides a processor configured to execute instructions described in the above-described information processing method, specifically: detecting a first SNP which is a mutation of an Alzheimer's disease-associated gene in a genomic DNA sample derived from a subject; and determining whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

[Examples]

Hereinafter, the present invention will be further described in more detail using examples and comparative examples, but is not limited to these examples and the like.

<Method>

[Establishment of iPS cohort from peripheral blood cells of sporadic AD patients]

This test was approved by the Ethics Committee of the Center for iPS Cell Research and Application, Kyoto University (approval numbers: CiRA19-05 and CiRA20-14). For the establishment of iPS cells from human peripheral blood mononuclear cells (PBMCs), PBMCs of Alzheimer's disease (AD) patients were collected according to a study project approved by the Ethics Committee of Kyoto University Graduate School of Medicine (approval numbers: R0091, G259, and G0722). Written informed consent was obtained from all participants in this test. Human cDNA of a reprogramming factor was introduced into human PBMCs using episomal vectors (SOX2, KLF4, OCT4, L-MYC, LIN28, and dominant-negative p53). Several days after transduction, the PBMCs were collected and re-seeded in a plate coated with laminin 511-E8 fragment (iMatrix 511, manufactured by Nippi Inc.). On the next day, the medium was replaced with StemFit AK03. Thereafter, the medium was replaced every two days. After 20 days of the transduction, iPS cell colonies were picked up. The iPS cells established from the PBMCs were subjected to expansion culture for neural differentiation.

[Cerebral cortical neurons induced from human iPS cells]

A robust and rapid differentiation induction method was established using direct conversion technology. Human neurogenin 2 (NGN2) cDNA under a tetracyclineinducible promoter (tetO) was transformed into iPS cells using a piggyBac transposon system and Lipofectamine LTX (manufactured by Thermo Fisher Scientific Inc.). A vector containing tetO::NGN2 was used. After antibiotic selection with G418 disulfate (manufactured by Nacalai Tesque, Inc.), colonies were selected, and subclones that could efficiently differentiate into neurons by inducing temporal expression of NGN2 were selected with MAP2/DAPI purity more than 96%.

[Karyotype analysis and genotyping]

Karyotype analysis was performed by this laboratory or LSI Medience. Genotyping of a single nucleotide polymorphism was performed by PCR amplification of genomic DNA and direct sequencing was performed (3100 Genetic Analyzer; manufactured by Thermo Fisher Scientific Inc.). APOE gene was amplified through PCR (forward primer TCCAAGGAGCTGCAGGCGGCGCA (SEQ ID NO: 1); and reverse primer ACAGAATTCGCCCCGGCCTGGTACACTG (SEQ ID NO: 2)). After the PCR products were digested with Hhal at 37°C for 2 hours, electrophoresis was performed to analyze the band size.

[Immunocytochemical staining]

Cells were fixed with 4 v/v% paraformaldehyde (pH 7.4) at room temperature (RT, about 25°C) and permeabilized with PBST containing 0.2 v/v% triton X-100. To suppress nonspecific binding, blocking treatment was performed at RT for 60 minutes with BlockingONE histo (manufactured by Nacalai Tesque, Inc.). The cells were incubated with a primary antibody overnight at 4°C and then labeled with a fluorescently tagged secondary antibody. Nuclei were labeled using DAPI (Thermo Fisher Scientific Inc.).
Cell images were obtained using a high-content confocal microscope IN Cell Analyzer 6000 (manufactured by GE Healthcare). The following primary antibodies were used for immunocytochemical staining: NANOG (1:100 dilution; manufactured by Abeam, ab80892), TRA1-60 (1:400 dilution; CST#4746, Danvers, MA), MAP2 (1:4,000 dilution; manufactured by Abcam, ab5392), SATB2 (1:400 dilution; manufactured by Abeam EPNCIR130A ab92446), an Alexa488-conjugated antibody (1:400 dilution; A11029 manufactured by Thermo Fisher Scientific Inc.), an Alexa488-conjugated antibody (1:400 dilution; A11039 manufactured by Thermo Fisher Scientific Inc.), an Alexa594-conjugated antibody (1:400 dilution; A21207 manufactured by Thermo Fisher Scientific Inc.).

[Quantification of protein concentration]

On day 10, a RIPA-soluble fraction of total protein was extracted from differentiated neurons, cultured in a 96-well plate with 30 $\mu$L of RIPA buffer, and centrifuged at 12,000 g for 30 minutes to collect a supernatant. The protein concentration of the supernatant was measured using Pierce BCA Protein Assay Kit (Thermo Fisher Scientific Inc.) according to the kit manual.

[Pathway analysis of identified genes]

Pathway analysis of 230 identified genes ($p < 5 \times 10^{-5}$) was performed using commercially available Ingenuity Pathway Analysis (IPA, manufactured by QIAGEN, https://www.qiagenbioinformatics.com/) software to analyze top networks.

[Electrochemiluminescence assay of amyloid $\beta$ (A$\beta$)]

All medium was replaced with 100 $\mu$L of a fresh medium on day 8. The acclimation medium was collected for further analysis on day 10. A$\beta$ species in the medium were measured for extracellular human A$\beta$ using Human (6E10) A$\beta$3-Plex Kit (manufactured by Meso Scale Discovery). In the case of the A$\beta$ species, the 6E10 antibody was used in this assay to capture A$\beta$ peptides, and a C-terminal-specific anti-A$\beta$ antibody having different SULFO-TAG labels for detection through electrochemiluminescence using Sector Imager 2400 (manufactured by Meso Scale Discovery) was used. Quantified Aβ values (N=2 wells per clone) were adjusted using total neuronal protein concentration to compare conditions to minimize noise due to alteration in the number of cells.

[Electrochemiluminescence assay of tau proteins]

Tau species in RIPA lysate extracted from iPS cell-derived neurons were measured by Phospho (Thr231)/Total Tau Kit (manufactured by Meso Scale Discovery) according to the kit manual. Quantified tau values (N=2 wells per clone) were adjusted using total neuronal protein concentration to compare conditions to minimize noise due to altered cell numbers.

[Genome-wide association study (GWAS) for SNP genotyping and cellular dissection of polygenicity of AD patients]

Genotypes of all 102 AD patient samples were determined with the Infinium OmniExpress Exome-8v1.4 Bead-Chip according to the kit manual (manufactured by Illumina, Inc.). To separate algorithmic issues from data format issues, all genotype data were standardized to the forward strand GRCh37.p13 direction generated by variant calls from WGS data. After GenomeStudio (manufactured by Illumina, Inc.) and quality control (Hardy-Weinberg equilibrium: $p>1.0\times10^{-6}$; minor allele frequency$\geq$0.01; linkage disequilibrium-based variant pruning r2<0.8, window size: 100 kb, step size: 5) were used to determine genotypes, the genotypes were attributed by minimac4 using 1,000 Genome Project Phase 3 as a reference panel. 7,349,481 SNPs exceeded the quality threshold value after substitution (r2$\geq$0.3, minor allele frequency$\geq$0.01). A linear association between SNPs and an accumulation rate of Aβ42/40 ratios of the iPS cell-derived neurons was analyzed with PLINK 1.9, and the age at onset, sex, and genotypes of the APOE-ε4 allele were included as covariates in the linear regression model. $p<5\times10^{-5}$ was set as a suggestive level and $p<5\times10^{-8}$ was set as a significance level for the association analysis. No statistical methods were used to predetermine the sample size, but the sample size is similar to that reported in previous publications.

[Prediction of clinical data from ADNI dataset]

Aβ42/40 accumulation rate results in cerebral cortical neurons were processed through LD-based aggregation (r2>0.2, window size=1 Mb) using PLINK 1.9. Among independent SNPs, 496 SNPs exceeded the recommended threshold value level ($p<5\times10^{-5}$) for genome-wide analysis and were used as variables in the prediction model. An SNP genotype matrix of the selected 102 AD patient samples, originally composed of 0, 1, or 2, was normalized and analyzed through principal component analysis (PCA).

Sample genotypes from Alzheimer's Disease Neuroimaging Initiative (ADNI) 1/GO/2 dataset were collected (manufactured by Illumina, Inc.; Omni 2.5M BeadChip). Quality control and imputation were performed on the genotype data under the same conditions. Attributed genotypes of 10,121,962 SNPs were filtered by 496 SNPs obtained from the genome-wide analysis. Genotypes of SNPs that were listed in a list of cellular dissection of polygenicity (CDiP) but not in the ADNI dataset were complemented with the mean genotypes of AD patients. Then, phenotypes of ADNI samples were predicted from the genotypes. It was predicted whether the samples belonged to a valid status for AD (positive) or not (negative). The samples were independently classified as positive and negative according to four criteria based on results reported in the ADNI database. First, standardized uptake value ratio (SUVR, reference: cerebellar reference region) from AV45 PET data (>1.1, threshold value for positivity); second, Aβ(1-42) in CSF (<977 pg/mL, threshold value for positivity); third, t-tau/Aβ(1-42) in CSF (>0.27, threshold value for positivity); and fourth, p-tau/Aβ(1-42) in CSF (>0.025, threshold value for positivity). All the reported results were obtained from ADNIMERGE dataset of a baseline. Samples containing both genotype data and phenotype data were included in the study (SUVR AV45: n=512; CSFAβ(1-42), t-t-tau/Aβ(1-42), p-tau/ Aβ(1-42): n=581). Genotype vectors of the ADNI samples were mapped to the main component space derived from the genotype matrix of in-hospital AD patients. 10-Fold cross-validation was performed.

The ADNI samples were divided into training samples and test samples. Random Forest classifiers (estimated to 100 pieces) were trained on the training samples and the target variables (positive/negative AD-like conditions) were predicted from the genotype matrix and the top three PCs of the covariates (age, sex, and APOE-ε4 genotypes). Prediction performance was assessed by the area under the curve (AUC) of a receiver operating characteristic (ROC) curve obtained from the prediction of the test samples. The prediction performance was compared to a case where the target variables were predicted only from the covariates. The significance of AUC improvement was tested with the Wilcoxon signed-rank test (significance threshold value: p<0.05). The target variables correspond to the "information on the onset of Alzheimer's disease" described above.

[Knockdown of target gene]

Cells were seeded on day 5 in a 6-well plate at an initial density of 3,000,000 cells per well. 24 Hours after seeding (on day 6), the medium was replaced with Neurobasal medium containing 1 $\mu$M Accell SMARTpool siRNA (manufactured by Horizon Discovery Ltd.). To maximize the effect of Acell siRNA, iPS cell-derived neurons were cultured from day 6 to day 9 for 72 hours. 72 hours after the addition of siRNA (on day 9), the culture medium was replaced with fresh Neurobasal medium containing 1 $\mu$M Accell SMARTpool siRNA or 1 $\mu$M JNJ-40418677 (manufactured by Sigma-Aldrich), and the replaced medium was collected on day 11 and subjected to A$\beta$ phenotype analysis.

[Analysis of rare variants associated with onset of AD]

Whole-exome sequencing was performed on 407 blood-derived genomic DNA samples obtained from 255 AD patients and 152 cognitively normal controls participating in the Japanese ADNI project. An exon sequence was concentrated by hybridization using SureSelect Human All Exon kit (V6) manufactured by Agilent Technologies, Inc., and sequenced on HiSeq 4000 manufactured by Illumina, Inc. using paired-end read chemistry. Short read sequences of a target region were mapped to a human reference genome (hg38) using BWA-MEM version 0.7.15-r1140 with default settings. Subsequent analysis (reading processing, variant calling and variant filtering) was performed according to GATK4 best practice recommendations, followed by variant annotation using snpEff version 4.3t. Among all variants identified by whole-exome sequencing, the focus was on non-synonymous, nonsense, splice sites, and insertion or deletion variants. Furthermore, this was narrowed down to variants with MAF<0.05 in the public databases using public databases: ExAC release 0.3 (http://exac.broadinstitute.org/), gnomAD release 2.1.1 for exome and r.3.0 for genome (https://gnomad.broadinstitute.org/), HGVD version 2.3 (http://www.hgvd.genome.med.kyoto-u.ac.jp/), and TfoMMo version 8.3KJPN (https://jmorp.megabank.tohoku.ac.jp). Using J-ADNI (n=407) and ADNI (n=479) exome data, gene-based association analysis of variants was performed using Burdentest with R package seqMeta version 1.6.7.

[Data availability]

Data used in this test were obtained from the Alzheimer's Disease Neuroimaging Initiative (ADNI) database (adni.loni.usc.edu). ADNI was launched in 2003 as a public-private partnership led by principal investigator Michael W. Weiner, MD. ADNI's primary goal is to combine serial magnetic resonance imaging (MRI), positron emission tomography (PET), and other biological markers, as well as clinical and neuropsychological assessments and to test whether it is possible to measure progression of mild cognitive impairment (MCI) and early Alzheimer's disease (AD). SNP array data are available at the National Bioscience Database Center (NBDC) (https://humandbs.biosciencedbc.jp/en/, Study ID: hum0314.v1).

[Code availability]

All codes for data management and analysis are archived online at GitHub (https://github.com/HaruhisaInoue/iSNPs4ADNIpred). All other codes are publicly available on the inventors' site.

[Statistics and reproducibility]

Statistical analysis was performed as follows, except for the prediction of clinical data from the ADNI dataset and the analysis of rare variants associated with the onset of AD. All data are mean $\pm$ S. D. Experimental duplication was performed 2 to 3 times to confirm reproducibility. Data distribution was assumed to be normal, but this has not been formally tested. Comparison of averages among 3 or more groups was performed by one-way analysis of variance (ANOVA) followed by Tukey's multiple comparison test or Uncorrected Fisher's LSD (post-hoc test using GraphPad Prism 7.0 software (manufactured by GraphPad Software)). A p-value less than 0.05 was considered significant.

[Example 1]

In this test, genome-wide analysis was performed using A$\beta$ released from cerebral cortical neurons derived from iPS cells of an AD cohort as a pathological feature. CDiP was then performed to reveal complex pathological mechanisms in a neuron-specific manner.

1. Establishment of iPS cells and analysis of A$\beta$ phenotypes in iPS cell-derived neurons

First, to analyze neuronal AD pathology, iPS cells exhibiting a normal karyotype were established from patients in the sporadic AD (SAD) cohort (N=102). The established iPS cells showed an ability to generate all three germ layers in vitro and X-inactive-specific transcripts (XIST) similar to human ESCs.

All iPS cell clones were directly differentiated into cerebral cortical neurons through forced expression of human NGN2 gene. In this differentiation protocol, exogenous NGN2 was fully suppressed after day 8, and Aβ phenotypes remained constant from day 8 to day 14. The complex AD pathology is made up of various types of molecules or biological events such as Aβ and tau that are potential candidates for GWAS characteristics. Aβ was selected as a pathological feature of cerebral cortical neurons because Aβ is the triggering event in initiation of a long-term pathological cascade of AD and causes dementia. Aβ40 and Aβ42 were respectively quantified as protective and toxic Aβ, and the Aβ42/40 ratio was quantified in a culture supernatant of neurons in the SAD cerebral cortex.

APP and PSEN1 genes, which play a central role in an Aβ production pathway, are known to affect neurodevelopment and neuronal differentiation tendency from human iPS cells. Accordingly, in a case of assessing Aβ among iPS cells from different patients, it is important to maintain uniform purity of differentiation into neurons and normalize variations in the number of neurons per well. The direct differentiation method used in this test yielded uniform and highly pure cerebral cortical neurons, but caused variations in neuronal density between patients due to stress of direct conversion from day 0 to day 5, and this variability affected the amount of Aβ. The total protein concentration extracted from neurons across wells was used to normalize variations in number of neurons per well. This is because alteration in protein concentration linearly reflect the number of neurons per well of patients or different independent neurons.

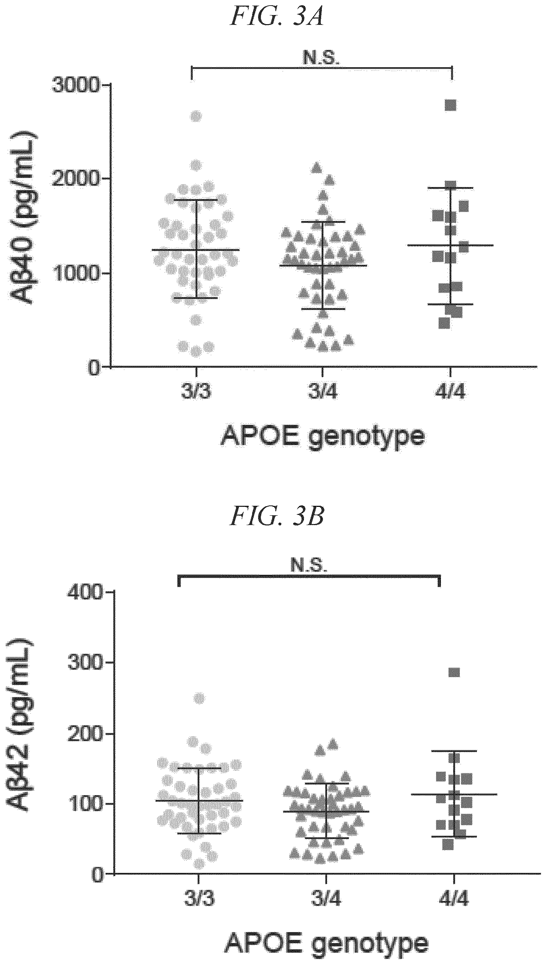

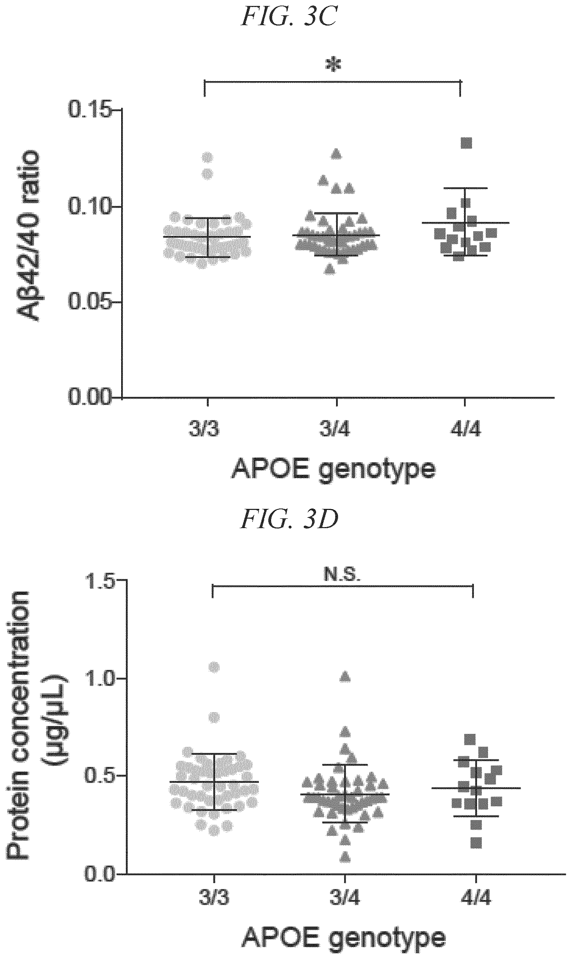

To confirm the correlation with genomic information, the correlation between the Aβ species and the APOE genotypes, which were the strongest genetic risks for AD, was analyzed. The APOE-ε4 genotypes correlated moderately with the Aβ42/40 ratio (FIG. 3C) but not with the amount of Aβ (FIGS. 3A and 3B) or the protein concentration (FIG. 3D), as evidenced by other modalities.

Previous reports using gene recombination techniques have also shown that APOE4 allele affects Aβ phenotypes of iPS cell-derived neurons with the same genetic background. However, alteration in Aβ phenotypes in different SAD populations of APOE 3/3 vs. 4/4 (1.09-fold alteration in this test) was smaller than the alteration due to genomic correction (approximately 1.2-fold or 2-fold alteration in previous reports) (FIG. 3C).

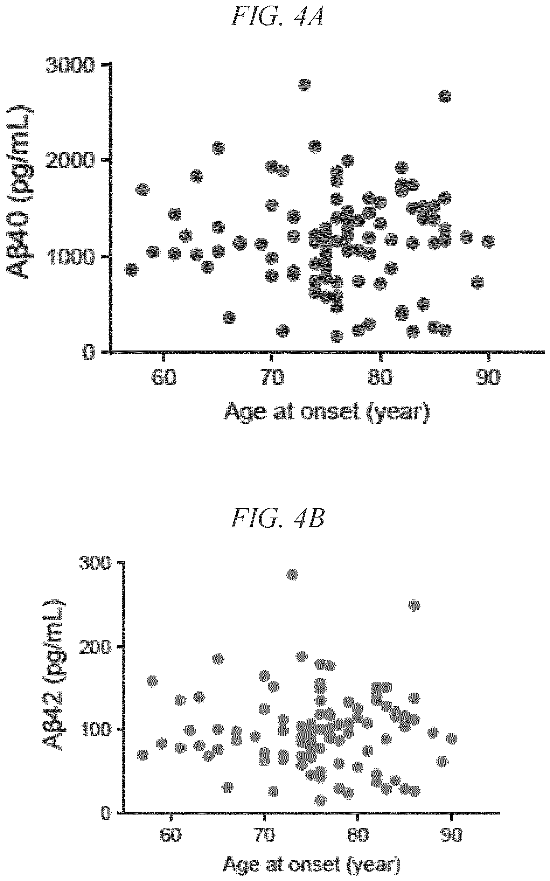

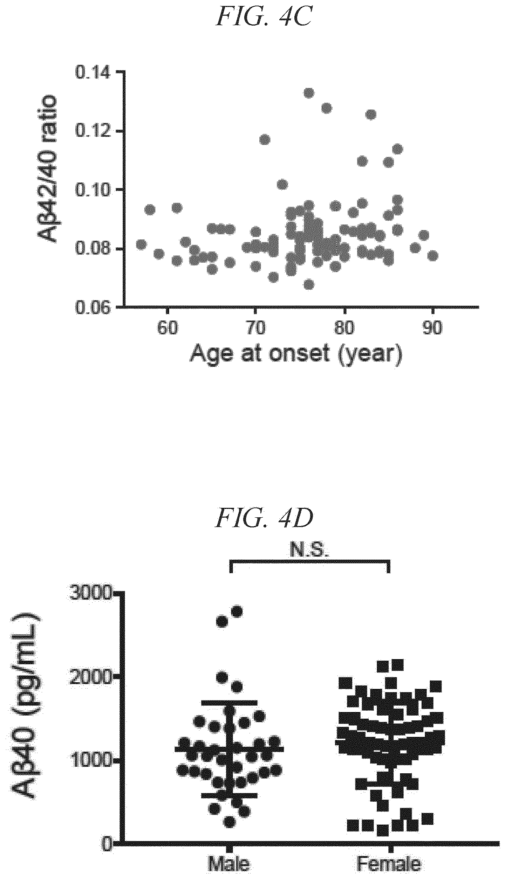

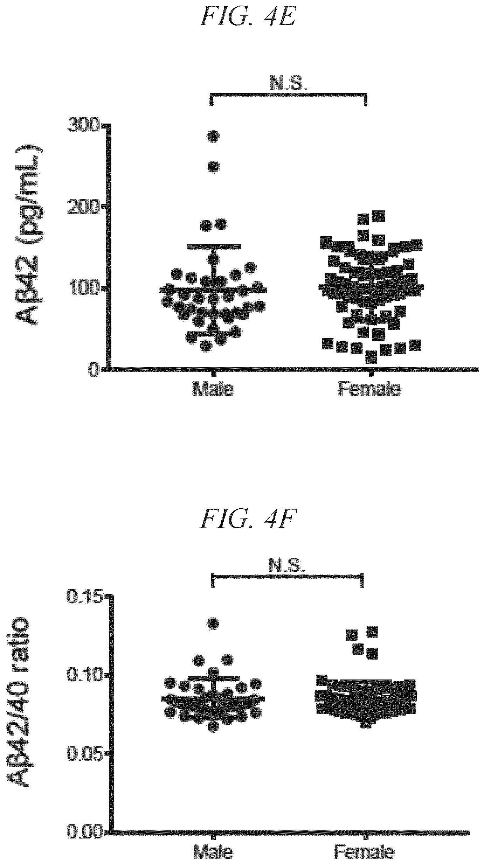

In addition, the correlation between the quantified Aβ phenotypes in cerebral cortical neurons and clinical conditions such as age at onset and sex was analyzed. The amount and proportion of Aβ species did not correlate with the age at onset (FIGS. 4A, 4B, and 4C) and sex (FIGS. 4D, 4E, and 4F).

These results indicated that the Aβ phenotypes of SAD were affected by the diverse polygenic architecture of SAD. Accordingly, genome-wide analysis was performed using Aβ of cerebral cortical neurons of SAD for the pathological feature of AD.

2. Genome-wide analysis

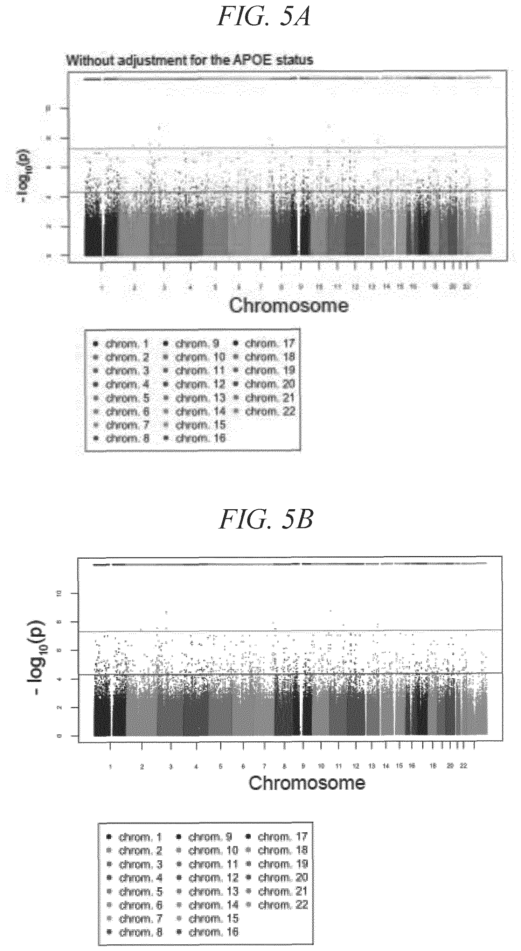

To understand the polygenicity of Aβ, genome-wide analysis was performed using the Aβ42/40 ratio of cerebral cortical neurons as a pathological feature. Statistical analysis was adjusted according to the APOE status and a mis-detection rate of multiple testing was applied. Overall results showed no significant deviations from what was expected by chance ($\lambda$=0.9659), meaning that there was no evidence of test statistic bias or inflation due to population structuring. To estimate the effect of APOE genotypes, CDiP was initially performed without adjustment of the APOE genotypes (FIG. 5A). As a result, the p-value of rs429358 (T/C, APOE-ε4 gene locus) was 0.794, which was not statistically significant. Although APOE-ε4 carries a high risk of clinical AD, CDiP showed that the Aβ42/40 ratio in single cell-type culture of iPS cell-derived neurons was primarily affected by APOE-ε4 as well as other complex gene sets.

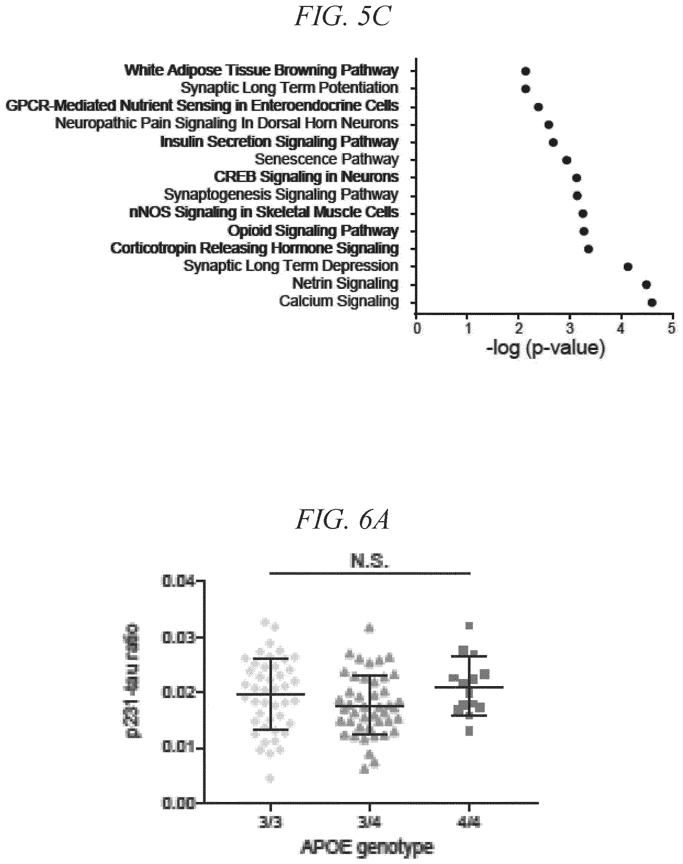

Accordingly, CDiP was performed by adjusting the APOE genotypes (FIG. 5B), and 24 SNP genotypes and associated gene loci ("p-value<$5 \times 10^{-8}$" or "gene loci containing more than 10 SNPs with p-value<$5 \times 10^{-5}$") which were associated with the altered Aβ42/40 ratio were identified (FIG. 5 and Tables 3-1 to 3-77). In Tables 3-1 to 3-77, "chr" means a chromosome, "BETA" means partial regression coefficient, and "SE" means a standard error. The same meaning applies to the subsequent tables.

[Table 3-1]

| SNP ID | chr | position | gene locus | p-value | SNP location in gene locus | BETA | SE |
|---|---|---|---|---|---|---|---|
| rs11260631 | 1 | 5565535 | NA | 2. 17E-05 | | 0. 005099 | 0. 001142 |
| rs12027231 | 1 | 5566294 | NA | 2. 17E-05 | | 0. 005099 | 0. 001142 |
| rs140532989 | 1 | 11054703 | NA | 2. 22E-07 | | 0.0185 | 0.003319 |
| rs145588311 | 1 | 11170657 | MTOR | 8. 52E-06 | intron_variant | 0. 02781 | 0. 005915 |
| rs17875920 | 1 | 11801023 | AGTRAP | 9. 79E-06 | intron_variant | 0. 01261 | 0. 002701 |

(continued)

| SNP ID | chr | position | gene locus | p-value | SNP location in gene locus | BETA | SE |
|---|---|---|---|---|---|---|---|
| rs7418059 | 1 | 11803080 | AGTRAP | 9. 79E-06 | intron_variant | 0.01261 | 0. 002701 |
| rs34791043 | 1 | 11803485 | NA | 9. 79E-06 | NA | 0.01261 | 0. 002701 |
| rs17875934 | 1 | 11804288 | AGTRAP | 9. 79E-06 | intron_variant | 0. 01261 | 0. 002701 |
| rs17875948 | 1 | 11807970 | AGTRAP | 9. 79E-06 | intron_variant | 0. 01261 | 0. 002701 |
| rs17875954 | 1 | 11809161 | AGTRAP | 9. 79E-06 | intron_variant | 0.01261 | 0. 002701 |
| rs17875967 | 1 | 11811273 | AGTRAP | 9. 79E-06 | intron_variant | 0.01261 | 0. 002701 |
| rs17875970 | 1 | 11811886 | AGTRAP | 9. 79E-06 | intron_variant | 0. 01261 | 0. 002701 |
| rs116001018 | 1 | 11816860 | NA | 9. 79E-06 | | 0.01261 | 0. 002701 |
| rs147306013 | 1 | 11817560 | NA | 9. 79E-06 | | 0.01261 | 0. 002701 |
| rs72641053 | 1 | 12128435 | TNFRSF8 | 3. 08E-05 | intron_variant | 0.01044 | 0. 002388 |
| rs79459531 | 1 | 17605062 | PADI3 | 1. 07E-05 | intron_variant | 0. 02722 | 0. 005861 |
| rs79724306 | 1 | 17607570 | PADI3 | 1. 07E-05 | intron_variant | 0. 02722 | 0. 005861 |
| rs187218036 | 1 | 17627131 | NA | 1. 07E-05 | | 0. 02722 | 0. 005861 |
| rs147239745 | 1 | 17631035 | NA | 1. 07E-05 | | 0. 02722 | 0. 005861 |
| rs10917055 | 1 | 19495467 | UBR4 | 1. 27E-05 | intron_variant | 0. 01294 | 0. 002812 |
| rs57482167 | 1 | 24495578 | NA | 4. 76E-06 | NA | 0. 009858 | 0. 002034 |
| rs4649197 | 1 | 24495722 | IFNLR1 | 4. 76E-06 | intron_variant | 0.009858 | 0. 002034 |
| rs11249017 | 1 | 24496662 | IFNLR1 | 8. 75E-06 | intron_variant | 0. 00949 | 0. 002021 |
| rs12128905 | 1 | 24497000 | IFNLR1 | 4. 76E-06 | intron_variant | 0. 009858 | 0. 002034 |
| rs11249018 | 1 | 24498130 | IFNLR1 | 4. 76E-06 | intron_variant | 0.009858 | 0. 002034 |
| rs3932667 | 1 | 24498696 | IFNLR1 | 4. 76E-06 | intron_variant | 0 . 009858 | 0. 002034 |
| 1:24499617:C:CA | 1 | 24499617 | NA | 4. 08E-07 | NA | 0.01002 | 0.001844 |
| rs34212240 | 1 | 24500325 | IFNLR1 | 4. 76E-06 | intron_variant | 0. 009858 | 0. 002034 |
| rs3897438 | 1 | 24506247 | IFNLR1 | 2. 07E-05 | intron_variant | 0.008779 | 0. 001961 |
| rs3897439 | 1 | 24506510 | IFNLR1 | 2. 07E-05 | intron_variant | 0. 008779 | 0. 001961 |
| rs4081342 | 1 | 24506522 | IFNLR1 | 2. 07E-05 | intron_variant | 0. 008779 | 0. 001961 |
| 1:24506786:CA:C | 1 | 24506786 | NA | 2. 07E-05 | NA | 0.008779 | 0.001961 |
| rs3897440 | 1 | 24506861 | IFNLR1 | 2. 07E-05 | intron_variant | 0 . 008779 | 0. 001961 |
| rs6680101 | 1 | 24507433 | IFNLR1 | 2. 07E-05 | intron_variant | 0. 008779 | 0. 001961 |
| rs6665649 | 1 | 24507437 | IFNLR1 | 2. 07E-05 | intron_variant | 0. 008779 | 0. 001961 |
| rs6683433 | 1 | 24507774 | IFNLR1 | 3. 09E-05 | intron_variant | 0 . 007511 | 0. 001718 |

[Table 3-2]

| SNP ID | chr | position | gene locus | p-value | SNP location in gene locus | BETA | SE |
|---|---|---|---|---|---|---|---|
| rs10903046 | 1 | 24507855 | IFNLR1 | 2. 54E-05 | intron_variant | 0. 008056 | 0.001821 |

105

(continued)

| SNP ID | chr | position | gene locus | p-value | SNP location in gene locus | BETA | SE |
|---|---|---|---|---|---|---|---|
| rs79806176 | 1 | 34231799 | CSMD2 | 2. 43E-05 | NMD_transcript_variant | 0.01293 | 0. 002916 |
| rs74658700 | 1 | 34234656 | CSMD2 | 2. 43E-05 | NMD transcript_variant | 0.01293 | 0.002916 |
| rs76405888 | 1 | 34238146 | CSMD2 | 3. 44E-06 | NMD transcript_variant | 0.01871 | 0. 003798 |
| rs74224650 | 1 | 34244450 | CSMD2 | 3. 44E-06 | NMD_transcript_variant | 0.01871 | 0. 003798 |
| rs58862022 | 1 | 34244587 | CSMD2 | 2. 83E-06 | NMD transcript_variant | 0.01726 | 0. 00347 |
| rs34278452 | 1 | 40588662 | NA | 7. 60E-06 | | 0.01014 | 0. 002143 |
| rs12760525 | 1 | 40589754 | NA | 2.90E-05 | | 0.01101 | 0. 002509 |
| rs11800679 | 1 | 40603234 | NA | 3. 88E-05 | | 0. 007743 | 0. 001795 |
| rs35156697 | 1 | 40617030 | NA | 4. 67E-07 | | 0.01471 | 0. 002723 |
| rs10493090 | 1 | 40626918 | NA | 4. 67E-07 | | 0.01471 | 0. 002723 |
| rs11584362 | 1 | 40631460 | RLF | 4. 67E-07 | intron_variant | 0.01471 | 0. 002723 |
| rs5028951 | 1 | 40632581 | RLF | 4. 67E-07 | intron_variant | 0.01471 | 0. 002723 |
| rs12724048 | 1 | 40632954 | RLF | 4. 67E-07 | intron_variant | 0.01471 | 0. 002723 |
| rs34795058 | 1 | 40636231 | RLF | 4. 67E-07 | intron_variant | 0.01471 | 0. 002723 |
| rs12744808 | 1 | 40644934 | RLF | 1.60E-06 | intron_variant | 0.01738 | 0. 003399 |
| rs61780442 | 1 | 40649468 | RLF | 1.60E-06 | intron_variant | 0.01738 | 0. 003399 |
| rs11808248 | 1 | 40662090 | RLF | 4. 67E-07 | intron_variant | 0.01471 | 0. 002723 |
| rs17878476 | 1 | 40668727 | RLF | 4. 67E-07 | intron_variant | 0.01471 | 0. 002723 |
| 1:40682172:AAGAG:A | 1 | 40682172 | NA | 1.60E-06 | NA | 0.01738 | 0. 003399 |
| rs61778546 | 1 | 40687368 | RLF | 4. 67E-07 | intron_variant | 0.01471 | 0. 002723 |
| rs16827064 | 1 | 40688132 | RLF | 4. 67E-07 | intron_variant | 0.01471 | 0. 002723 |
| rs16827106 | 1 | 40735472 | ZMPSTE24 | 3. 92E-05 | intron_variant | 0.01126 | 0. 002612 |
| rs16827109 | 1 | 40735817 | ZMPSTE24 | 3. 92E-05 | intron_variant | 0.01126 | 0. 002612 |

(continued)

| SNP ID | chr | position | gene locus | p-value | SNP location in gene locus | BETA | SE |
|---|---|---|---|---|---|---|---|
| rs12757549 | 1 | 40742366 | ZMPSTE24 | 3. 92E-05 | intron_variant | 0.01126 | 0. 002612 |
| rs71060364 | 1 | 40753372 | NA | 1.29E-06 | NA | 0.01349 | 0. 002613 |
| rs12725815 | 1 | 40753766 | ZMPSTE24 | 3. 92E-05 | intron_variant | 0.01126 | 0. 002612 |
| rs76609695 | 1 | 41315101 | NA | 2. 57E-05 | | 0.0156 | 0. 003529 |
| rs61773507 | 1 | 48411428 | TRABD2B | 2.07E-05 | intron variant | 0.01741 | 0. 003889 |
| rs115906838 | 1 | 58708368 | DAB1 | 2. 72E-05 | intron_variant | 0.01062 | 0.002411 |
| rs117567026 | 1 | 58715824 | DAB1 | 6. 69E-06 | intron_variant | 0.01523 | 0. 003197 |
| rs12039506 | 1 | 58721695 | DAB1 | 6. 69E-06 | intron_variant | 0.01523 | 0.003197 |
| rs12035886 | 1 | 58721704 | DAB1 | 6. 69E-06 | intron_variant | 0.01523 | 0.003197 |
| rs140969406 | 1 | 58721802 | NA | 6. 69E-06 | NA | 0.01523 | 0.003197 |
| rs17117229 | 1 | 58733538 | DAB1 | 6. 69E-06 | intron_variant | 0.01523 | 0.003197 |
| rs151064393 | 1 | 58734680 | DAB1 | 6. 69E-06 | intron_variant | 0.01523 | 0.003197 |

[Table 3-3]

| SNP ID | chr | position | gene locus | p-value | SNP location in gene locus | BETA | SE |
|---|---|---|---|---|---|---|---|
| rs117388414 | 1 | 58745146 | DAB1 | 6. 69E-06 | intron_variant | 0.01523 | 0.003197 |
| rs17117259 | 1 | 58753770 | DAB1 | 6. 69E-06 | intron_variant | 0. 01523 | 0. 003197 |
| rs17117277 | 1 | 58763802 | DAB1 | 6. 69E-06 | intron_variant | 0. 01523 | 0. 003197 |
| rs17117283 | 1 | 58764638 | DAB1 | 6. 69E-06 | intron_variant | 0.01523 | 0.003197 |
| rs142567959 | 1 | 58766083 | DAB1 | 6. 69E-06 | intron_variant | 0. 01523 | 0. 003197 |
| rs115426189 | 1 | 64539974 | ROR1 | 2.50E-05 | intron_variant | 0. 02626 | 0. 005931 |
| rs138138409 | 1 | 64554364 | ROR1 | 2.50E-05 | intron_variant | 0. 02626 | 0. 005931 |
| rs78457326 | 1 | 76867147 | ST6GALNAC3 | 5. 59E-06 | intron_variant | 0. 02836 | 0. 005899 |

(continued)

| SNP ID | chr | position | gene locus | p-value | SNP location in gene locus | BETA | SE |
|---|---|---|---|---|---|---|---|
| rs140199001 | 1 | 81198118 | NA | 1. 04E-07 | | 0. 03229 | 0.005617 |
| rs192853907 | 1 | 81226954 | NA | 1.04E-07 | | 0. 03229 | 0.005617 |
| rs114618747 | 1 | 81240468 | NA | 1. 04E-07 | | 0. 03229 | 0.005617 |
| rs117721292 | 1 | 81251534 | NA | 1. 04E-07 | | 0. 03229 | 0.005617 |
| rs144408429 | 1 | 81252438 | NA | 1. 04E-07 | | 0. 03229 | 0.005617 |
| rs145068891 | 1 | 81303947 | NA | 1. 04E-07 | | 0. 03229 | 0. 005617 |
| rs77325472 | 1 | 81308364 | NA | 1.17E-05 | | 0. 02238 | 0. 004842 |
| rs142985266 | 1 | 81326827 | NA | 1.17E-05 | | 0. 02238 | 0. 004842 |
| rs75819420 | 1 | 81328646 | NA | 1.17E-05 | | 0. 02238 | 0. 004842 |
| rs76876118 | 1 | 81329832 | NA | 1.17E-05 | | 0. 02238 | 0. 004842 |
| rs80094910 | 1 | 81330079 | NA | 1.17E-05 | | 0. 02238 | 0. 004842 |
| rs10493678 | 1 | 81333502 | NA | 1.17E-05 | | 0. 02238 | 0. 004842 |
| rs10493679 | 1 | 81334027 | NA | 1.17E-05 | | 0. 02238 | 0. 004842 |
| rs61767086 | 1 | 89066582 | NA | 1. 56E-05 | | 0. 01054 | 0. 002318 |
| rs1988189 | 1 | 89082365 | NA | 4.17E-05 | | 0.01254 | 0. 002921 |
| rs4596856 | 1 | 89082375 | NA | 4.17E-05 | | 0.01254 | 0. 002921 |
| rs4259618 | 1 | 89082380 | NA | 4.17E-05 | | 0.01254 | 0. 002921 |
| rs12033271 | 1 | 89084247 | NA | 4.17E-05 | | 0.01254 | 0. 002921 |
| 1:89085206: TAAA:T | 1 | 89085206 | NA | 4.17E-05 | NA | 0.01254 | 0. 002921 |
| 1:89085227 :A:G | 1 | 89085227 | NA | 4.17E-05 | NA | 0.01254 | 0. 002921 |
| rs75316566 | 1 | 91967640 | CDC7 | 7. 85E-06 | intron_ variant | 0.01798 | 0. 003807 |
| 1:92031771 :C:CA | 1 | 92031771 | NA | 1.86E-05 | NA | 0.005199 | 0.001154 |
| rs61209856 | 1 | 94589551 | NA | 2.60E-05 | | 0.0103 | 0. 002333 |
| rs56931023 | 1 | 94590379 | NA | 2.60E-05 | | 0.0103 | 0. 002333 |
| rs78142006 | 1 | 94590533 | NA | 2.60E-05 | | 0.0103 | 0. 002333 |
| rs72962326 | 1 | 94595330 | NA | 2.60E-05 | | 0.0103 | 0. 002333 |
| rs117428698 | 1 | 95399858 | NA | 1. 84E-05 | | 0.01577 | 0. 0035 |
| rs141570401 | 1 | 95424962 | NA | 1. 84E-05 | | 0.01577 | 0.0035 |

[Table 3-4]

| SNP ID | ch r | position | gene locus | p-value | SNP location in gene locus | BETA | SE |
|---|---|---|---|---|---|---|---|
| 1:102574438 :C:T | 1 | 102574438 | NA | 5.59E-06 | NA | 0.02836 | 0.005899 |
| rs117439108 | 1 | 104894981 | NA | 1.04E-07 | | 0.03229 | 0.005617 |
| rs189348849 | 1 | 104978328 | NA | 1.04E-07 | | 0.03229 | 0.005617 |
| rs11799443 | 1 | 107989087 | NTNGI | 1.72E-05 | intron_variant | 0.02707 | 0.005982 |

(continued)

| SNP ID | ch r | position | gene locus | p-value | SNP location in gene locus | BETA | SE |
|---|---|---|---|---|---|---|---|
| rs142243476 | 1 | 107991761 | NTNG1 | 1.72E-05 | intron_variant | 0.02707 | 0.005982 |
| rs111451024 | 1 | 114832660 | NA | 1.07E-05 | | 0.02722 | 0.005861 |
| rs76185230 | 1 | 114833460 | NA | 1.07E-05 | | 0.02722 | 0.005861 |
| rs79125386 | 1 | 114834247 | NA | 1.07E-05 | | 0.02722 | 0.005861 |
| rs6660468 | 1 | 114835204 | NA | 1.07E-05 | | 0.02722 | 0.005861 |
| rs116493739 | 1 | 114836116 | NA | 1.07E-05 | | 0.02722 | 0.005861 |
| rs77470545 | 1 | 114836560 | NA | 1.07E-05 | | 0.02722 | 0.005861 |
| rs7522214 | 1 | 114840898 | NA | 1.07E-05 | | 0.02722 | 0.005861 |
| rs75376490 | 1 | 114841671 | NA | 1.07E-05 | | 0.02722 | 0.005861 |
| rs7534361 | 1 | 114844072 | NA | 1.07E-05 | | 0.02722 | 0.005861 |
| rs148371870 | 1 | 114845387 | NA | 1.07E-05 | | 0.02722 | 0.005861 |
| rs142305684 | 1 | 114848595 | NA | 1.07E-05 | NA | 0.02722 | 0.005861 |
| rs199724897 | 1 | 114849666 | NA | 1.07E-05 | | 0.02722 | 0.005861 |
| 1:114850653 :T:C | 1 | 114850653 | NA | 1.07E-05 | NA | 0.02722 | 0.005861 |
| rs142658681 | 1 | 114850730 | NA | 1.07E-05 | | 0.02722 | 0.005861 |
| rs7540256 | 1 | 114851811 | NA | 1.07E-05 | | 0.02722 | 0.005861 |
| rs116039729 | 1 | 114852370 | NA | 1.07E-05 | | 0.02722 | 0.005861 |
| rs145900544 | 1 | 114852385 | NA | 1.07E-05 | | 0.02722 | 0.005861 |
| rs142737501 | 1 | 114852517 | NA | 1.07E-05 | | 0.02722 | 0.005861 |
| 1:114855828 :T:A | 1 | 114855828 | NA | 1.07E-05 | NA | 0.02722 | 0.005861 |
| rs41470845 | 1 | 114856929 | NA | 1.07E-05 | | 0.02722 | 0.005861 |
| rs74580704 | 1 | 114862402 | NA | 1.07E-05 | | 0.02722 | 0.005861 |
| rs76169339 | 1 | 114865513 | NA | 1.07E-05 | | 0.02722 | 0.005861 |
| rs6669141 | 1 | 114865863 | NA | 1.07E-05 | | 0.02722 | 0.005861 |
| rs12132759 | 1 | 115628467 | TSPAN2 | 1.60E-05 | intron_variant | 0.01704 | 0.003751 |
| rs6701221 | 1 | 152778558 | LCE1C | 1.55E-05 | intron_variant | 0.01154 | 0.002535 |
| 1:158992906 :AT:A | 1 | 158992906 | NA | 2.76E-06 | NA | 0.02389 | 0.004797 |
| rs4646881 | 1 | 165667980 | ALDH9A1 | 1.52E-07 | 5_prime_UTR_variant | 0.02647 | 0.004674 |
| rs79887819 | 1 | 167633592 | RCSD1 | 5.59E-06 | intron_variant | 0.02836 | 0.005899 |
| rs78645780 | 1 | 170132537 | METTL11 B | 1.28E-06 | intron_variant | 0.02126 | 0.004115 |
| rs115223117 | 1 | 170139597 | NA | 1.28E-06 | | 0.02126 | 0.004115 |
| rs115433886 | 1 | 170140312 | NA | 1.28E-06 | NA | 0.02126 | 0.004115 |

[Table 3-5]

| SNP ID | ch r | position | gene locus | p-value | SNP location in gene locus | BETA | SE |
|---|---|---|---|---|---|---|---|
| rs7537272 | 1 | 170142133 | NA | 1.28E-06 | | 0.02126 | 0.004115 |
| rs10564453 | 1 | 170142729 | NA | 1.28E-06 | | 0.02126 | 0.004115 |
| rs143134448 | 1 | 170144306 | NA | 1.28E-06 | | 0.02126 | 0.004115 |
| rs77373691 | 1 | 170146623 | NA | 1.28E-06 | | 0.02126 | 0.004115 |
| rs6689201 | 1 | 170146938 | NA | 1.28E-06 | | 0.02126 | 0.004115 |
| rs6671761 | 1 | 170158174 | NA | 1.28E-06 | | 0.02126 | 0.004115 |
| rs74822235 | 1 | 170159553 | NA | 1.28E-06 | | 0.02126 | 0.004115 |
| rs77849060 | 1 | 170161011 | NA | 1.28E-06 | | 0.02126 | 0.004115 |
| rs17345810 | 1 | 170166066 | NA | 1.28E-06 | | 0.02126 | 0.004115 |
| rs17349942 | 1 | 170167702 | NA | 1.28E-06 | | 0.02126 | 0.004115 |
| rs78968813 | 1 | 170167937 | NA | 1.28E-06 | | 0.02126 | 0.004115 |
| rs140761847 | 1 | 170169900 | NA | 1.28E-06 | | 0.02126 | 0.004115 |
| 1:170171514 :T:A | 1 | 170171514 | NA | 2.82E-05 | NA | 0.01677 | 0.003814 |
| rs10919327 | 1 | 170171514 | NA | NA | | NA | NA |
| rs17345893 | 1 | 170171874 | NA | 2.82E-05 | | 0.01677 | 0.003814 |
| rs6662495 | 1 | 170172742 | NA | 2.82E-05 | | 0.01677 | 0.003814 |
| rs6687139 | 1 | 170173262 | NA | 2.82E-05 | | 0.01677 | 0.003814 |
| rs17345907 | 1 | 170174046 | NA | 2.82E-05 | | 0.01677 | 0.003814 |
| rs12144655 | 1 | 170174298 | NA | 2.82E-05 | | 0.01677 | 0.003814 |
| rs12142060 | 1 | 170176678 | NA | 2.82E-05 | | 0.01677 | 0.003814 |
| rs77199503 | 1 | 170179140 | NA | 2.82E-05 | | 0.01677 | 0.003814 |
| rs17345935 | 1 | 170179341 | NA | 2.82E-05 | | 0.01677 | 0.003814 |
| rs17350032 | 1 | 170181363 | NA | 2.82E-05 | | 0.01677 | 0.003814 |
| rs17350053 | 1 | 170184112 | NA | 2.82E-05 | | 0.01677 | 0.003814 |
| rs74981093 | 1 | 170193063 | NA | 2.82E-05 | | 0.01677 | 0.003814 |
| rs12562203 | 1 | 175145938 | KIAA0 040 | 3.51E-05 | intron_variant | 0.02589 | 0.005965 |
| rs139718675 | 1 | 177096274 | ASTN1 | 1.27E-05 | intron_variant | 0.006061 | 0.001317 |
| rs57530364 | 1 | 177096284 | ASTN1 | 1.56E-05 | intron_variant | 0.005845 | 0.001285 |
| rs10798498 | 1 | 177096980 | ASTN1 | 6.37E-06 | intron_variant | 0.005701 | 0.001194 |
| rs10753143 | 1 | 177097241 | ASTN1 | 6.37E-06 | intron_variant | 0.005701 | 0.001194 |
| rs10798499 | 1 | 177097442 | ASTN1 | 6.37E-06 | intron_variant | 0.005701 | 0.001194 |
| 1:177126430 TTTG:T | 1 | 177126430 | NA | 1.95E-06 | NA | 0.01417 | 0.002798 |

(continued)

| SNP ID | ch r | position | gene locus | p-value | SNP location in gene locus | BETA | SE |
|---|---|---|---|---|---|---|---|
| rs60826838 | 1 | 183545685 | NCF2 | 2.50E-05 | intron_ variant | 0.02626 | 0.005931 |
| rs117250867 | 1 | 184582654 | C1orf 21 | 1.07E-05 | intron_ variant | 0.02722 | 0.005861 |
| rs12096795 | 1 | 184590346 | C1orf 21 | 1.07E-05 | 3_prime_ UTR_ variant | 0.02722 | 0.005861 |
| rs12023933 | 1 | 184621712 | NA | 1.07E-05 | | 0.02722 | 0.005861 |

[Table 3-6]

| SNP ID | ch r | position | gene locus | p-value | SNP location in gene locus | BETA | SE |
|---|---|---|---|---|---|---|---|
| rs16823401 | 1 | 184666214 | EDEM3 | 1. 07E-05 | intron_variant | 0. 02722 | 0. 005861 |
| rs116897291 | 1 | 184814667 | NIBAN1 | 1. 07E-05 | intron_variant | 0. 02722 | 0. 005861 |
| rs148184379 | 1 | 184886968 | NIBAN1 | 1. 07E-05 | intron_variant | 0. 02722 | 0. 005861 |
| rs117517283 | 1 | 184921127 | NIBAN1 | 1. 07E-05 | intron_variant | 0. 02722 | 0. 005861 |
| rs74607511 | 1 | 192513026 | NA | 3. 26E-07 | | 0. 02048 | 0. 003733 |
| rs77505887 | 1 | 192515584 | NA | 9. 66E-06 | | 0. 01987 | 0. 004254 |
| rs74508649 | 1 | 192526317 | NA | 1. 42E-06 | | 0. 01799 | 0. 0035 |
| rs74419088 | 1 | 192529469 | NA | 1. 42E-06 | | 0. 01799 | 0. 0035 |
| rs77258347 | 1 | 192534699 | NA | 1. 42E-06 | | 0. 01799 | 0. 0035 |
| rs74367555 | 1 | 192538724 | NA | 8.13E-07 | | 0.01715 | 0. 003252 |
| rs76623154 | 1 | 192538746 | NA | 8.13E-07 | | 0.01715 | 0.003252 |
| rs74130685 | 1 | 192538948 | NA | 8.13E-07 | | 0.01715 | 0. 003252 |
| rs115743395 | 1 | 192538965 | NA | 1. 42E-06 | | 0. 01799 | 0. 0035 |
| rs74130686 | 1 | 192539013 | NA | 8.13E-07 | | 0.01715 | 0. 003252 |
| rs147211012 | 1 | 199309033 | NA | 4.18E-05 | | 0. 01627 | 0.00379 |

(continued)

| SNP ID | ch r | position | gene locus | p-value | SNP location in gene locus | BETA | SE |
|---|---|---|---|---|---|---|---|
| rs4915476 | 1 | 201047062 | CACNA1S | 3. 22E-05 | synonymous_variant | 0. 02682 | 0.00615 |
| rs4915213 | 1 | 201047774 | CACNA1S | 3. 22E-05 | intron_variant | 0. 02682 | 0.00615 |
| rs6427877 | 1 | 201048265 | CACNA1S | 3. 22E-05 | intron variant | 0. 02682 | 0.00615 |
| rs3767511 | 1 | 201052199 | CACNAL S | 3. 22E-05 | intron_variant | 0. 02682 | 0.00615 |
| rs75488105 | 1 | 204065966 | SOX13 | 1. 07E-05 | intron_variant | 0. 02722 | 0. 005861 |
| rs113144751 | 1 | 204066542 | SOX13 | 1. 07E-05 | intron_variant | 0. 02722 | 0. 005861 |
| rs145279852 | 1 | 204066674 | SOX13 | 1. 07E-05 | intron_variant | 0. 02722 | 0. 005861 |
| rs147225273 | 1 | 204066680 | SOX13 | 1. 07E-05 | intron_variant | 0. 02722 | 0. 005861 |
| rs17188797 | 1 | 211187783 | AL590132.1 | 8. 52E-06 | intron_variant | 0. 02781 | 0.005915 |
| rs2378009 | 1 | 218947264 | NA | 4. 74E-05 | | 0. 005621 | 0.00132 |
| rs6681600 | 1 | 218948258 | NA | 4. 74E-05 | | 0. 005621 | 0.00132 |
| rs6673437 | 1 | 218948649 | NA | 4. 74E-05 | | 0. 005621 | 0.00132 |
| rs6541208 | 1 | 218952920 | NA | 5. 22E-06 | | 0. 006264 | 0.001298 |
| rs11452039 | 1 | 218954472 | NA | 1. 85E-06 | | 0. 008352 | 0.001645 |
| rs6541210 | 1 | 218955436 | NA | 5. 22E-06 | | 0. 006264 | 0.001298 |
| rs12032085 | 1 | 223641019 | NA | 3. 64E-05 | | 0.01087 | 0.00251 |
| rs117913581 | 1 | 232777842 | NA | 3. 22E-05 | | 0. 02682 | 0.00615 |
| rs138567986 | 1 | 241325817 | RGS7 | 2. 19E-06 | intron_variant | 0. 02908 | 0. 005774 |
| 1:244577487 : T:TAATA | 1 | 244577487 | NA | 2. 58E-05 | NA | 0. 007157 | 0.00162 |
| rs3127454 | 1 | 244578852 | ADSS2 | 2. 58E-05 | intron_variant | 0. 007157 | 0.00162 |
| rs3127462 | 1 | 244597892 | ADSS2 | 9. 82E-06 | intron_variant | 0. 00926 | 0.001984 |

[Table 3-7]

| SNP ID | chr | position | gene locus | p-value | SNP location in gene locus | BETA | SE |
|---|---|---|---|---|---|---|---|
| rs3127463 | 1 | 244598196 | ADSS2 | 2. 48E-07 | intron_variant | 0.01023 | 0.001843 |
| rs3127464 | 1 | 244599858 | ADSS2 | 2. 48E-07 | intron_variant | 0.01023 | 0.001843 |
| 1:244601900 :C:CAT | 1 | 244601900 | NA | 6. 59E-06 | NA | 0.008394 | 0.001761 |
| rs3003213 | 1 | 244608181 | ADSS2 | 2. 48E-07 | intron_variant | 0.01023 | 0.001843 |
| 1:244608950 :T:TA | 1 | 244608950 | NA | 3.41E-06 | NA | 0.009674 | 0.001963 |
| 1:244608950 :T:TAA | 1 | 244608950 | NA | NA | NA | NA | NA |
| rs3006004 | 1 | 244609092 | ADSS2 | 2. 48E-07 | intron_variant | 0.01023 | 0.001843 |
| rs3003212 | 1 | 244612252 | ADSS2 | 2. 48E-07 | intron_variant | 0.01023 | 0.001843 |
| rs3127466 | 1 | 244614497 | ADSS2 | 2. 48E-07 | intron_variant | 0.01023 | 0.001843 |
| rs3003210 | 1 | 244617509 | NA | 2. 48E-07 | | 0.01023 | 0.001843 |
| rs141944966 | 1 | 245746729 | KIF26B | 3. 69E-06 | intron_variant | 0. 02364 | 0. 004815 |
| rs145014144 | 1 | 245759684 | KIF26B | 8. 52E-06 | intron_variant | 0.02781 | 0.005915 |
| rs78194184 | 1 | 247365152 | AL390728.4 | 1. 07E-05 | intron_variant | 0.02722 | 0.005861 |
| rs61272272 | 2 | 6215734 | NA | 3. 77E-06 | | 0.006521 | 0.00133 |
| rs12621265 | 2 | 6220141 | NA | 3. 77E-06 | | 0. 006521 | 0. 00133 |
| rs2609122 | 2 | 6220654 | NA | 3. 77E-06 | | 0.006521 | 0. 00133 |
| rs76756243 | 2 | 6224392 | NA | 3. 77E-06 | | 0. 006521 | 0.00133 |
| rs12052388 | 2 | 6224960 | NA | 3. 77E-06 | | 0. 006521 | 0.00133 |
| rs60904524 | 2 | 6225735 | NA | 3. 77E-06 | | 0.006521 | 0.00133 |
| rs73150377 | 2 | 6227222 | NA | 3. 77E-06 | | 0.006521 | 0.00133 |
| rs73150378 | 2 | 6227726 | NA | 3. 77E-06 | | 0. 006521 | 0. 00133 |
| rs77944704 | 2 | 6228902 | NA | 9. 69E-06 | | 0.006195 | 0.001327 |
| rs76144832 | 2 | 6231193 | NA | 1. 50E-05 | | 0.005724 | 0.001255 |
| rs74706208 | 2 | 6233421 | NA | 1. 98E-05 | | 0.005627 | 0.001254 |
| rs16864697 | 2 | 6233460 | NA | 1. 50E-05 | | 0. 005724 | 0.001255 |
| rs12052438 | 2 | 6236379 | NA | 9.71E-06 | | 0. 005844 | 0.001251 |
| rs77918648 | 2 | 6270044 | NA | 1. 77E-05 | | 0. 006383 | 0.001413 |
| rs76480233 | 2 | 6270267 | NA | 1. 77E-05 | | 0.006383 | 0.001413 |
| rs16864772 | 2 | 6271097 | NA | 1. 77E-05 | | 0.006383 | 0.001413 |
| rs16864773 | 2 | 6271288 | NA | 1. 77E-05 | | 0.006383 | 0.001413 |

(continued)

| SNP ID | chr | position | gene locus | p-value | SNP location in gene locus | BETA | SE |
|---|---|---|---|---|---|---|---|
| rs168fi4776 | 2 | 6271533 | NA | 1. 77E-05 | | 0. 006383 | 0.001413 |
| rs16864778 | 2 | 6271646 | NA | 1. 77E-05 | | 0. 006383 | 0.001413 |
| rs78933180 | 2 | 6274257 | NA | 3.03E-05 | | 0.006171 | 0.00141 |
| rs75637712 | 2 | 6278287 | NA | 3. 03E-05 | | 0.006171 | 0.00141 |
| rs7557935 | 2 | 6281053 | NA | 2. 42E-05 | | 0.00624 | 0.001407 |
| rs7567221 | 2 | 6281198 | NA | 2. 42E-05 | | 0.00624 | 0.001407 |

[Table 3-8]

| SNR ID | chr | position | gene locus | p-value | SNP location in gene locus | BETA | SE |
|---|---|---|---|---|---|---|---|
| rs11897984 | 2 | 7538773 | NA | 1. 49E-05 | | 0. 006423 | 0.001408 |
| rs1346810 | 2 | 7539421 | NA | 1. 49E-05 | | 0.006423 | 0.001408 |
| rs10179128 | 2 | 7539984 | NA | 1. 49E-05 | | 0. 006423 | 0. 001408 |
| rs6709430 | 2 | 7544818 | NA | 2. 23E-05 | | 0. 006309 | 0.001416 |
| rs1546026 | 2 | 10832167 | NA | 3.10E-05 | | 0.009931 | 0. 002272 |
| rs77715032 | 2 | 14959674 | NA | 4.71E-07 | | 0. 03074 | 0. 00569 |
| 2:16523989 :AT:A | 2 | 16523989 | NA | 6. 00E-06 | NA | 0.01033 | 0. 002157 |
| rs201150095 | 2 | 16523991 | NA | 6. 00E-06 | NA | 0.01033 | 0. 002157 |
| rs55641030 | 2 | 16982622 | NA | 1. 58E-05 | | 0. 02214 | 0. 004871 |
| rs74671599 | 2 | 16983140 | NA | 1. 58E-05 | | 0 . 02214 | 0 . 004871 |
| rs114875341 | 2 | 18374830 | KCNS3 | 3. 22E-05 | intron_variant | 0.02682 | 0. 00615 |
| rs115436106 | 2 | 20365483 | NA | 8.81E-06 | NA | 0.014 | 0. 002983 |
| rs2090033 | 2 | 20372497 | NA | 8.81E-06 | | 0.014 | 0. 002983 |
| rs4233758 | 2 | 20376022 | NA | 8.81E-06 | | 0.014 | 0. 002983 |
| rs75019476 | 2 | 31155554 | GALNT14 | 5. 89E-06 | intron_variant | 0. 02323 | 0. 004846 |
| rs75182639 | 2 | 31244662 | GALNT14 | 4.81E-05 | intron_variant | 0.01498 | 0. 003521 |
| rs59717368 | 2 | 34269641 | NA | 2. 53E-05 | | 0 . 01881 | 0 . 004252 |
| rs117140225 | 2 | 41848143 | NA | 4.71E-07 | | 0. 03074 | 0. 00569 |
| rs117076730 | 2 | 41855067 | NA | 4.71E-07 | | 0 . 03074 | 0. 00569 |
| rs75983203 | 2 | 41860583 | NA | 4.71E-07 | | 0. 03074 | 0. 00569 |
| 2:47055014 :A:T | 2 | 47055014 | NA | 2. 84E-05 | NA | 0.02129 | 0. 004845 |
| rs72806356 | 2 | 52982063 | NA | 5. 59E-06 | | 0. 02836 | 0. 005899 |
| rs199881113 | 2 | 52987966 | NA | 5. 59E-06 | | 0. 02836 | 0. 005899 |
| rs115366500 | 2 | 52996352 | NA | 5. 89E-06 | | 0. 02323 | 0. 004846 |
| rs62127009 | 2 | 52998723 | NA | 5. 89E-06 | | 0 . 02323 | 0 . 004846 |
| rs72908943 | 2 | 53689606 | NA | 1. 07E-05 | | 0. 02722 | 0. 005861 |

(continued)

| SNR ID | chr | position | gene locus | p-value | SNP location in gene locus | BETA | SE |
|---|---|---|---|---|---|---|---|
| rs6745622 | 2 | 53704053 | NA | 1. 07E-05 | | 0 . 02722 | 0 . 005861 |
| rs145963514 | 2 | 66470046 | NA | 1.21E-05 | | 0.02218 | 0 . 004807 |
| rs76686125 | 2 | 111908163 | BCL2L11 | 3.00E-05 | intron_variant | 0.0141 | 0 . 003219 |
| rs113351584 | 2 | 114643053 | NA | 3. 70E-08 | | 0. 02736 | 0. 004575 |
| rs142890162 | 2 | 114728805 | NA | 3. 70E-08 | | 0. 02736 | 0. 004575 |
| rs111252481 | 2 | 114734142 | NA | 3. 70E-08 | | 0. 02736 | 0. 004575 |
| rs143348587 | 2 | 114753287 | NA | 3. 70E-08 | | 0. 02736 | 0. 004575 |
| rs10207532 | 2 | 138096983 | THSD7B | 2.50E-05 | intron_variant | 0. 02626 | 0. 005931 |
| rs10196367 | 2 | 138210725 | THSD7B | 2.50E-05 | intron_variant | 0.02626 | 0. 005931 |
| rs28415678 | 2 | 138297618 | THSD7B | 2. 50E-05 | intron_variant | 0.02626 | 0. 005931 |

[Table 3-9]

| SNP ID | chr | position | gene locus | p-value | SNP location in gene locus | BETA | SE |
|---|---|---|---|---|---|---|---|
| rs72048484 | 2 | 142051977 | LRP1B | 4. 42E-05 | intron_variant | 0.009119 | 0.002131 |
| rs2679451 | 2 | 142053453 | LRP1B | 4. 42E-05 | intron_variant | 0.009119 | 0.002131 |
| rs2679455 | 2 | 142060374 | LRP1B | 4. 42E-05 | intron_variant | 0.009119 | 0.002131 |
| rs2714170 | 2 | 142068173 | LRP1B | 4. 42E-05 | intron_variant | 0.009119 | 0.002131 |
| rs118164915 | 2 | 142489626 | LRP1B | 2. 19E-06 | intron_variant | 0.02908 | 0. 005774 |
| rs80027625 | 2 | 142563101 | LRP1B | 2. 19E-06 | intron_variant | 0. 02908 | 0.005774 |
| rs7563677 | 2 | 161356717 | NA | 2. 93E-06 | | 0.02385 | 0. 004803 |
| 2:161357267 : AAAAG:A | 2 | 161357267 | NA | 2. 93E-06 | NA | 0. 02385 | 0. 004803 |
| rs141879065 | 2 | 161358374 | NA | 2. 93E-06 | | 0. 02385 | 0. 004803 |
| rs201036025 | 2 | 161358504 | NA | 2. 93E-06 | | 0. 02385 | 0. 004803 |
| rs72970060 | 2 | 161360952 | NA | 2. 93E-06 | | 0. 02385 | 0. 004803 |
| rs72970066 | 2 | 161362938 | NA | 2. 93E-06 | | 0. 02385 | 0. 004803 |
| rs72970074 | 2 | 161365114 | NA | 2. 93E-06 | | 0. 02385 | 0. 004803 |

(continued)

| SNP ID | chr | position | gene locus | p-value | SNP location in gene locus | BETA | SE |
|--------|-----|----------|------------|---------|----------------------------|------|-----|
| rs6736111 | 2 | 161368211 | NA | 2. 93E-06 |  | 0. 02385 | 0. 004803 |
| rs187697067 | 2 | 161384015 | NA | 2. 93E-06 | NA | 0. 02385 | 0. 004803 |
| rsl92262735 | 2 | 172895083 | METAP1D | 3. 22E-05 | intron_variant | 0.01661 | 0. 003807 |
| rs79029608 | 2 | 172907025 | METAP1D | 3. 22E-05 | intron_variant | 0.01661 | 0. 003807 |
| rs60153685 | 2 | 172920131 | METAP1D | 2. 53E-05 | intron_variant | 0.01887 | 0. 004265 |
| rs148393698 | 2 | 173138493 | NA | 1. 86E-05 |  | 0.01573 | 0. 003492 |
| 2:173790761 : CGGAG:C | 2 | 173790761 | NA | 4.61E-05 | NA | 0.005897 | 0.001382 |
| rs11695398 | 2 | 173791196 | RAPGEF4 | 4.61E-05 | intron_variant | 0. 005897 | 0.001382 |
| 2:173792363 : T:TA | 2 | 173792363 | NA | 2.01E-05 | NA | 0. 006274 | 0.001399 |
| rs143907126 | 2 | 193371023 | NA | 6. 26E-06 |  | 0. 01378 | 0. 002882 |
| rs12185569 | 2 | 202875552 | NA | 5.61E-06 |  | 0. 01534 | 0.003192 |
| rs12185583 | 2 | 202875768 | NA | 5.61E-06 |  | 0. 01534 | 0.003192 |
| rs10931984 | 2 | 202876281 | NA | 5.61E-06 |  | 0.01534 | 0.003192 |
| rs10931985 | 2 | 202876761 | NA | 5.61E-06 |  | 0.01534 | 0.003192 |
| rs13420220 | 2 | 202876981 | NA | 5.61E-06 |  | 0.01534 | 0. 003192 |
| rs10490083 | 2 | 202877944 | NA | 5.61E-06 |  | 0.01534 | 0.003192 |
| rs10182797 | 2 | 202878867 | NA | 5.61E-06 |  | 0.01534 | 0.003192 |
| rs11682513 | 2 | 202879218 | NA | 5.61E-06 |  | 0.01534 | 0.003192 |
| rs17386240 | 2 | 202879624 | NA | 5.61E-06 |  | 0.01534 | 0.003192 |
| rs12328354 | 2 | 202882815 | NA | 5.61E-06 |  | 0.01534 | 0.003192 |
| rs12328774 | 2 | 202882897 | NA | 5.61E-06 |  | 0.01534 | 0.003192 |
| rs62184862 | 2 | 204683751 | NA | 1. 07E-05 |  | 0. 02722 | 0. 005861 |

(continued)

| SNP ID | chr | position | gene locus | p-value | SNP location in gene locus | BETA | SE |
|---|---|---|---|---|---|---|---|
| rs62184863 | 2 | 204683797 | NA | 1. 07E-05 | | 0. 02722 | 0. 005861 |

[Table 3-10]

| SNP ID | chr | position | gene locus | p-value | SNP location in gene locus | BETA | SE |
|---|---|---|---|---|---|---|---|
| 2:204695698 :T:TA | 2 | 204695698 | NA | 1.07E-05 | NA | 0.02722 | 0.005861 |
| rs58738696 | 2 | 204712127 | NA | 1.07E-05 | | 0.02722 | 0.005861 |
| rs57248923 | 2 | 204712398 | NA | 1.07E-05 | | 0.02722 | 0.005861 |
| rs62182593 | 2 | 204713735 | NA | 1.07E-05 | | 0.02722 | 0.005861 |
| rs75622501 | 2 | 204714054 | NA | 1.07E-05 | | 0.02722 | 0.005861 |
| rs6741283 | 2 | 204714810 | NA | 1.07E-05 | | 0.02722 | 0.005861 |
| rs61602779 | 2 | 204716370 | NA | 1.07E-05 | | 0.02722 | 0.005861 |
| rs13016652 | 2 | 211927188 | NA | 4.95E-05 | | 0.006507 | 0.001532 |
| rs10932366 | 2 | 211954880 | NA | 3.62E-05 | | 0.006165 | 0.001423 |
| rs188502110 | 2 | 216164766 | NA | 1.07E-05 | | 0.02722 | 0.005861 |
| rs140760330 | 2 | 216165186 | NA | 1.07E-05 | | 0.02722 | 0.005861 |
| rs111485414 | 2 | 228498025 | C2orf83 | 3.11E-05 | non_coding_ transcript_ exon_ variant | 0.02143 | 0.004902 |
| rs2048812 | 2 | 228518634 | NA | 5.11E-07 | | 0.02201 | 0.004089 |
| rs2048813 | 2 | 228518649 | NA | 4.37E-05 | | 0.01323 | 0.003089 |
| rs2048814 | 2 | 228518704 | NA | 6.79E-06 | | 0.01533 | 0.003221 |
| rs7424668 | 2 | 228521539 | NA | 9.26E-06 | | 0.0176 | 0.00376 |
| rs62189839 | 2 | 228521691 | NA | 5.11E-07 | | 0.02201 | 0.004089 |

(continued)

| SNP ID | chr | position | gene locus | p-value | SNP location in gene locus | BETA | SE |
|---|---|---|---|---|---|---|---|
| rs140104950 | 2 | 228523377 | NA | 3.11E-05 | | 0.02143 | 0.004902 |
| rs112530470 | 2 | 228523536 | NA | 3.11E-05 | | 0.02143 | 0.004902 |
| rs146239501 | 2 | 235171239 | NA | 1.21E-05 | NA | 0.006359 | 0.001378 |
| rs1878963 | 2 | 235171335 | NA | 1.21E-05 | | 0.006359 | 0.001378 |
| 2:235172453 :T:C | 2 | 235172453 | NA | 2.38E-05 | NA | 0.007996 | 0.001801 |
| rs6711496 | 2 | 235172514 | NA | 1.21E-05 | | 0.006359 | 0.001378 |
| 2:235172825 : ATATAGTTGGA G:A | 2 | 235172825 | NA | 1.21E-05 | NA | 0.006359 | 0.001378 |
| rs4663367 | 2 | 235172880 | NA | 1.21E-05 | | 0.006359 | 0.001378 |
| rs10865081 | 2 | 239876256 | NA | 1.68E-05 | | 0.01736 | 0.003833 |
| rs149564461 | 2 | 239881016 | NA | 1.85E-05 | | 0.01462 | 0.003245 |
| rs4643560 | 2 | 239881730 | NA | 1.85E-05 | | 0.01462 | 0.003245 |
| rs4544463 | 2 | 239883557 | NA | 3.49E-05 | | 0.01274 | 0.002936 |
| rs4852058 | 2 | 239884849 | NA | 2.20E-05 | | 0.01369 | 0.00307 |
| rs4852059 | 2 | 239884850 | NA | 2.20E-05 | | 0.01369 | 0.00307 |
| rs13000373 | 2 | 239886775 | NA | 4.42E-06 | | 0.01471 | 0.003024 |
| rs6543539 | 2 | 239888052 | NA | 8.89E-08 | | 0.02085 | 0.003604 |
| rs7579583 | 2 | 239888571 | NA | 8.89E-08 | | 0.02085 | 0.003604 |
| rs7586010 | 2 | 239888597 | NA | 1.30E-05 | | 0.01335 | 0.002905 |

[Table 3-11]

| SNP ID | chr | position | gene locus | p-value | SNP location in gene locus | BETA | SE |
|---|---|---|---|---|---|---|---|
| rs6721090 | 2 | 239888880 | NA | 8.89E-08 | | 0.02085 | 0.003604 |
| rs11387730 | 2 | 239888961 | NA | 8.89E-08 | | 0.02085 | 0.003604 |

(continued)

| SNP ID | chr | position | gene locus | p-value | SNP location in gene locus | BETA | SE |
|---|---|---|---|---|---|---|---|
| rs11124201 | 2 | 239889428 | NA | 8.89E-08 | | 0.02085 | 0.003604 |
| rs11124202 | 2 | 239889603 | NA | 8.89E-08 | | 0.02085 | 0.003604 |
| rs4452172 | 2 | 239890163 | NA | 8.89E-08 | | 0.02085 | 0.003604 |
| rs4852064 | 2 | 239890370 | NA | 8.89E-08 | | 0.02085 | 0.003604 |
| rs4852065 | 2 | 239890581 | NA | 8.89E-08 | | 0.02085 | 0.003604 |
| rs4852066 | 2 | 239890696 | NA | 8.89E-08 | | 0.02085 | 0.003604 |
| rs4852067 | 2 | 239890850 | NA | 8.89E-08 | | 0.02085 | 0.003604 |
| 2:239890901:G: GCCA | 2 | 239890901 | NA | 2.81E-08 | NA | 0.01987 | 0.003289 |
| rs6754831 | 2 | 239890949 | NA | 2.81E-08 | | 0.01987 | 0.003289 |
| rs4851977 | 2 | 239891296 | NA | 2.81E-08 | | 0.01987 | 0.003289 |
| rs6543542 | 2 | 239891644 | NA | 8.89E-08 | | 0.02085 | 0.003604 |
| rs7580803 | 2 | 239892335 | NA | 3.13E-05 | | 0.01121 | 0.002565 |
| rs4241232 | 2 | 239893010 | NA | 2.81E-08 | | 0.01987 | 0.003289 |
| rs145427397 | 2 | 239902414 | NA | 3.44E-06 | | 0.01695 | 0.00344 |
| rs181670597 | 2 | 242151208 | AN07 | 5.59E-06 | intron_variant | 0.02836 | 0.005899 |
| 3:1749235:A: ATAT | 3 | 1749235 | NA | 4.09E-05 | NA | 0.02119 | 0.00493 |
| rs139850749 | 3 | 1917654 | NA | 1.07E-05 | | 0.02722 | 0.005861 |
| rs138485991 | 3 | 1934976 | NA | 1.07E-05 | | 0.02722 | 0.005861 |
| rs7617928 | 3 | 5679782 | NA | 3.12E-05 | | 0.009989 | 0.002286 |
| rs73808617 | 3 | 7086525 | GRM7 | 1.52E-05 | NMD_transcript _variant | 0.01767 | 0.003878 |
| rs17046693 | 3 | 7088276 | GRM7 | 1.52E-05 | NMD_transcript _variant | 0.01767 | 0.003878 |
| rs17046715 | 3 | 7090793 | GRM7 | 1.80E-05 | NMD_transcript _variant | 0.02205 | 0.004888 |
| rs74285835 | 3 | 7100399 | GRM7 | 1.80E-05 | NMD_transcript _variant | 0.02205 | 0.004888 |
| rs74285838 | 3 | 7111851 | GRM7 | 1.52E-05 | NMD_transcript _variant | 0.01767 | 0.003878 |
| rs6767094 | 3 | 7118997 | GRM7 | 1.32E-05 | NMD_transcript _variant | 0.01558 | 0.003392 |
| rs75442424 | 3 | 7121831 | GRM7 | 2.34E-06 | NMD_transcript _variant | 0.01928 | 0.003839 |
| rs76240495 | 3 | 7122393 | GRM7 | 2.22E-07 | NMD_transcript _variant | 0.02081 | 0.003732 |
| 3:7122411:CA: GA | 3 | 7122411 | NA | NA | NA | NA | NA |
| 3:7122411:CA:C | 3 | 7122411 | NA | 2.53E-06 | NA | 0.02131 | 0.004261 |

(continued)

| SNP ID | chr | position | gene locus | p-value | SNP location in gene locus | BETA | SE |
|---|---|---|---|---|---|---|---|
| rs77578494 | 3 | 11980791 | NA | 5.59E-06 | | 0.02836 | 0.005899 |
| rs75863593 | 3 | 12814936 | NA | 2.26E-05 | | 0.02188 | 0.004912 |
| rs73028847 | 3 | 14272393 | NA | 2.50E-05 | | 0.02626 | 0.005931 |
| rs28547889 | 3 | 14273762 | NA | 2.50E-05 | | 0.02626 | 0.005931 |
| rs55961609 | 3 | 14274855 | NA | 2.50E-05 | | 0.02626 | 0.005931 |

[Table 3-12]

| SNP ID | chr | position | gene locus | p-value | SNP location in gene locus | BETA | SE |
|---|---|---|---|---|---|---|---|
| rs6762079 | 3 | 14275457 | NA | 2.50E-05 | | 0.02626 | 0.005931 |
| rs7615075 | 3 | 14282434 | NA | 2.50E-05 | | 0.02626 | 0.005931 |
| rs9867815 | 3 | 14282631 | NA | 2.50E-05 | | 0.02626 | 0.005931 |
| rs17305345 | 3 | 14285488 | NA | 2.50E-05 | | 0.02626 | 0.005931 |
| rs9855896 | 3 | 14287150 | NA | 2.50E-05 | | 0.02626 | 0.005931 |
| rs9839148 | 3 | 14287726 | NA | 2.50E-05 | | 0.02626 | 0.005931 |
| rs6764810 | 3 | 14289062 | NA | 6.90E-07 | | 0.01997 | 0.003759 |
| rs9882488 | 3 | 14290607 | NA | 6.90E-07 | | 0.01997 | 0.003759 |
| rs7640375 | 3 | 14290987 | NA | 6.90E-07 | | 0.01997 | 0.003759 |
| rs7626720 | 3 | 14291503 | NA | 6.90E-07 | | 0.01997 | 0.003759 |
| rs9817301 | 3 | 14292389 | NA | 6.90E-07 | | 0.01997 | 0.003759 |
| rs9823520 | 3 | 14293832 | NA | 6.90E-07 | | 0.01997 | 0.003759 |
| rs9860771 | 3 | 14293838 | NA | 6.90E-07 | | 0.01997 | 0.003759 |
| 3:14296073:G: GCCTTGGGTGCACCT GGCCCT | 3 | 14296073 | NA | 5.65E-07 | NA | 0.01631 | 0.003044 |
| rs11920518 | 3 | 14296545 | NA | 5.65E-07 | | 0.01631 | 0.003044 |
| rs2341654 | 3 | 14298116 | NA | 2.79E-06 | | 0.01484 | 0.002982 |

(continued)

| SNP ID | chr | position | gene locus | p-value | SNP location in gene locus | BETA | SE |
|---|---|---|---|---|---|---|---|
| rs2128162 | 3 | 14298264 | NA | 5. 65E-07 | | 0.01631 | 0. 003044 |
| rs1038298 | 3 | 14302455 | NA | 6. 90E-07 | | 0. 01997 | 0.003759 |
| rs74710259 | 3 | 21874157 | ZNF385D | 7. 06E-06 | NMD_transcript_variant | 0. 01753 | 0. 003691 |
| rs79680055 | 3 | 21874582 | ZNF385D | 7. 06E-06 | NMD_transcript_variant | 0. 01753 | 0. 003691 |
| rs75670791 | 3 | 21914171 | ZNF385D | 9. 49E-07 | NMD_transcript_variant | 0.01761 | 0. 003363 |
| rs74903313 | 3 | 28653345 | AC098650.1 | 1. 07E-06 | NMD_transcript_variant | 0.02165 | 0.004157 |
| rs143877686 | 3 | 28653565 | AC098650.1 | 2.81E-06 | NMD_transcr i pt_variant | 0. 01888 | 0. 003793 |
| rs78649247 | 3 | 28660023 | AC098650.1 | 1. 07E-06 | NMD_transcript_variant | 0.02165 | 0. 004157 |
| rs75624012 | 3 | 28762528 | AC098650.1 | 5. 17E-07 | NMD_transcript_variant | 0. 02554 | 0. 004747 |
| rs12491791 | 3 | 28769252 | AC098650.1 | 5. 17E-07 | NMD_transcript_variant | 0. 02554 | 0. 004747 |
| rs144410553 | 3 | 28796544 | AC098650.1 | 5. 17E-07 | NMD_transcript_variant | 0. 02554 | 0. 004747 |
| rs114769913 | 3 | 28799686 | AC098650.1 | 5. 17E-07 | NMD_transcript_variant | 0. 02554 | 0. 004747 |
| rs62241678 | 3 | 29333361 | AC098650.1 | 7. 21E-06 | NMD_transcript_variant | 0. 01296 | 0. 002733 |
| rs150022738 | 3 | 32753524 | CN0T10 | 1. 07E-05 | intron_variant | 0. 02722 | 0. 005861 |
| rs62250842 | 3 | 32758483 | CNOT10 | 1. 07E-05 | intron_variant | 0. 02722 | 0. 005861 |
| rs62250843 | 3 | 32760921 | CNOT10 | 1. 07E-05 | intron_variant | 0. 02722 | 0. 005861 |
| rs6793550 | 3 | 32765663 | CNOT10 | 1. 07E-05 | intron_variant | 0. 02722 | 0. 005861 |

(continued)

| SNP ID | chr | position | gene locus | p-value | SNP location in gene locus | BETA | SE |
|---|---|---|---|---|---|---|---|
| 3: 32774098: G:GA | 3 | 32774098 | NA | 1. 07E-05 | NA | 0. 02722 | 0. 005861 |
| rs145862784 | 3 | 32780082 | CNOT10 | 1. 07E-05 | intron_ variant | 0. 02722 | 0. 005861 |

[Table 3-13]

| SNP ID | chr | position | gene locus | p-value | SNP location in gene locus | BETA | SE |
|---|---|---|---|---|---|---|---|
| rs7612883 | 3 | 32889638 | TRIM71 | 3. 67E-05 | intron_variant | 0.01653 | 0.003819 |
| rs13060342 | 3 | 32891423 | TRIM71 | 3. 67E-05 | intron_variant | 0.01653 | 0.003819 |
| rs6800867 | 3 | 32981043 | NA | 3. 36E-07 | | 0. 0159 | 0. 002901 |
| rs6803409 | 3 | 32981316 | NA | 3. 36E-07 | | 0.0159 | 0. 002901 |
| rs113716980 | 3 | 32982985 | NA | 3. 36E-07 | | 0. 0159 | 0. 002901 |
| 3:32983158 :CT:C | 3 | 32983158 | NA | 2. 36E-05 | NA | 0.01284 | 0.00289 |
| 3:32987068 :C:CT | 3 | 32987068 | NA | 2. 68E-06 | NA | 0. 01386 | 0. 002779 |
| rs4074331 | 3 | 32989009 | NA | 2. 68E-06 | | 0. 01386 | 0. 002779 |
| rs4074330 | 3 | 32989010 | NA | 2. 68E-06 | | 0. 01386 | 0. 002779 |
| rs73055186 | 3 | 32991856 | NA | 8. 81E-06 | | 0. 01617 | 0. 003444 |
| rs62250867 | 3 | 32991963 | NA | 8. 81E-06 | | 0. 01617 | 0. 003444 |
| rs7632357 | 3 | 32992203 | NA | 8. 81E-06 | | 0. 01617 | 0. 003444 |
| rs3774527 | 3 | 53705003 | CACNA1D | 4. 89E-06 | intron_variant | 0. 02045 | 0. 004224 |
| rs74426676 | 3 | 54509829 | CACNA2D3 | 1. 41E-05 | intron_variant | 0. 009442 | 0. 002064 |
| 3:60608100 :A:AT | 3 | 60608100 | NA | 3. 22E-05 | NA | 0. 02682 | 0.00615 |
| rs142745963 | 3 | 65616074 | MAGI1 | 2. 50E-05 | intron_variant | 0. 02626 | 0. 005931 |
| rs61555164 | 3 | 65741871 | MAGI1 | 8. 52E-06 | intron_variant | 0. 02781 | 0. 005915 |
| rs9841082 | 3 | 65743597 | MAGI1 | 8. 52E-06 | intron_variant | 0. 02781 | 0. 005915 |
| rs35219449 | 3 | 65872186 | MAGI 1 | 3. 04E-08 | intron_variant | 0.01686 | 0. 002798 |
| rs71306785 | 3 | 65872578 | MAGI1 | 3. 04E-08 | intron_variant | 0.01686 | 0. 002798 |
| rs71306786 | 3 | 65873461 | MAGI1 | 3. 04E-08 | intron_variant | 0.01686 | 0. 002798 |
| rs58687721 | 3 | 65873820 | MAGI1 | 1. 97E-09 | intron_variant | 0.01916 | 0. 002893 |
| rs35168985 | 3 | 65874420 | MAGI1 | 3. 04E-08 | intron_variant | 0.01686 | 0. 002798 |
| rs2372397 | 3 | 65875834 | MAGI1 | 3. 04E-08 | intron_variant | 0.01686 | 0. 002798 |
| rs117501871 | 3 | 65876113 | MAGI1 | 3. 04E-08 | intron_variant | 0.01686 | 0. 002798 |
| rs181795375 | 3 | 65877601 | MAGI 1 | 1. 97E-09 | intron_variant | 0.01916 | 0. 002893 |
| rs71306788 | 3 | 65877703 | MAGI1 | 3. 04E-08 | intron_variant | 0.01686 | 0. 002798 |
| rs71306789 | 3 | 65877793 | MAGI1 | 3. 04E-08 | intron_variant | 0.01686 | 0. 002798 |
| rs34548503 | 3 | 65878656 | MAGI1 | 3. 04E-08 | intron_variant | 0.01686 | 0. 002798 |

(continued)

| SNP ID | chr | position | gene locus | p-value | SNP location in gene locus | BETA | SE |
|---|---|---|---|---|---|---|---|
| 3:65878951 :G:GA | 3 | 65878951 | NA | 3. 04E-08 | NA | 0.01686 | 0. 002798 |
| rs924021 | 3 | 65879837 | MAGI1 | 2. 54E-09 | intron_variant | 0.01622 | 0. 002469 |
| rs35482956 | 3 | 65880339 | MAGI1 | 3. 48E-07 | intron_variant | 0.01802 | 0. 003293 |
| rs74795753 | 3 | 65926053 | MAGI1 | 2. 72E-07 | intron_variant | 0.03139 | 0. 005677 |
| rs117740205 | 3 | 65931724 | MAGI1 | 4. 31E-05 | intron_variant | 0. 02113 | 0. 004931 |
| rs114019667 | 3 | 66710033 | NA | 4. 02E-05 | | 0.01038 | 0.002413 |
| rs13319937 | 3 | 68599941 | NA | 2. 26E-05 | | 0.02188 | 0.004912 |

[Table 3-14]

| SNP ID | chr | position | gene locus | p-value | SNP location in gene locus | BETA | SE |
|---|---|---|---|---|---|---|---|
| rs1858373 | 3 | 68601038 | NA | 2.26E-05 | | 0.02188 | 0.004912 |
| rs1858372 | 3 | 68601193 | NA | 2.26E-05 | | 0.02188 | 0.004912 |
| rs55744776 | 3 | 68601734 | NA | 2.26E-05 | | 0.02188 | 0.004912 |
| rs7623705 | 3 | 68601948 | NA | 2.26E-05 | | 0.02188 | 0.004912 |
| rs72106943 | 3 | 68605001 | NA | 2.26E-05 | | 0.02188 | 0.004912 |
| rs10576290 | 3 | 68605535 | NA | 2.26E-05 | | 0.02188 | 0.004912 |
| rs7616164 | 3 | 68606548 | NA | 2.26E-05 | | 0.02188 | 0.004912 |
| rs9837106 | 3 | 68606690 | NA | 2.26E-05 | | 0.02188 | 0.004912 |
| rs9866302 | 3 | 68609673 | NA | 2.26E-05 | | 0.02188 | 0.004912 |
| rs13321458 | 3 | 68610030 | NA | 2.26E-05 | | 0.02188 | 0.004912 |
| rs9835386 | 3 | 68610847 | NA | 2.26E-05 | | 0.02188 | 0.004912 |
| rs67172613 | 3 | 77035984 | ROBO2 | 1.60E-05 | intron_variant | 0.01728 | 0.003804 |
| rs72902045 | 3 | 77036238 | ROBO2 | 3.35E-05 | intron_variant | 0.01888 | 0.004339 |
| rs7427534 | 3 | 77041173 | ROBO2 | 4.52E-05 | intron_variant | 0.006758 | 0.001582 |
| rs6795635 | 3 | 77045304 | ROBO2 | 4.52E-05 | intron_variant | 0.006758 | 0.001582 |

(continued)

| SNP ID | chr | position | gene locus | p-value | SNP location in gene locus | BETA | SE |
|---|---|---|---|---|---|---|---|
| rs6806644 | 3 | 77045353 | ROBO2 | 4.52E-05 | intron_variant | 0.006758 | 0.001582 |
| rs6548472 | 3 | 77045731 | ROBO2 | 4.52E-05 | intron_variant | 0.006758 | 0.001582 |
| rs11374746 | 3 | 77045771 | ROBO2 | 4.52E-05 | intron_variant | 0.006758 | 0.001582 |
| rs6786589 | 3 | 77046740 | ROBO2 | 4.52E-05 | intron_variant | 0.006758 | 0.001582 |
| rs9875709 | 3 | 77047311 | ROBO2 | 4.52E-05 | intron_variant | 0.006758 | 0.001582 |
| rs6774797 | 3 | 77048488 | ROBO2 | 4.52E-05 | intron_variant | 0.006758 | 0.001582 |
| rs11918007 | 3 | 77051881 | ROBO2 | 4.94E-05 | intron_variant | 0.006751 | 0.001589 |
| 3:84969212:T:A | 3 | 84969212 | NA | 3.95E-05 | NA | 0.01841 | 0.004273 |
| rs144035609 | 3 | 84972857 | NA | 3.95E-05 | | 0.01841 | 0.004273 |
| rs150062308 | 3 | 85019042 | CADM2 | 3.95E-05 | intron_variant | 0.01841 | 0.004273 |
| rs148678484 | 3 | 85042238 | CADM2 | 3.95E-05 | intron_variant | 0.01841 | 0.004273 |
| rs143096570 | 3 | 85051020 | CADM2 | 3.95E-05 | intron_variant | 0.01841 | 0.004273 |
| rs2875508 | 3 | 86021016 | CADM2 | 7.84E-06 | intron_variant | 0.01354 | 0.002867 |
| rs76958889 | 3 | 86022860 | CADM2 | 7.84E-06 | intron_variant | 0.01354 | 0.002867 |
| 3:107339790:T:G | 3 | 107339790 | NA | 1.28E-05 | NA | 0.008583 | 0.001866 |
| 3:107502792 :C:CA | 3 | 107502792 | NA | 3.51E-05 | NA | 0.02589 | 0.005965 |
| 3:112698726 : AATGG : AATGGATGG | 3 | 112698726 | NA | NA | NA | NA | NA |
| 3:112698726 : AATGG:A | 3 | 112698726 | NA | 2.73E-06 | NA | 0.01259 | 0.002528 |
| rs2613959 | 3 | 112808299 | NA | 2.13E-07 | | 0.02042 | 0.003656 |
| rs73229010 | 3 | 112899616 | NA | 1.84E-05 | | 0.01357 | 0.003012 |
| rs73229016 | 3 | 112901722 | NA | 2.06E-05 | | 0.01451 | 0.00324 |

[Table 3-15]

| SNP ID | chr | position | gene locus | p-value | SNP location in gene locus | BETA | SE |
|---|---|---|---|---|---|---|---|
| rs79608790 | 3 | 112958412 | BOC | 3. 41E-05 | intron_variant | 0. 01657 | 0. 003812 |
| 3:112958412 :T:G | 3 | 112958412 | NA | NA | NA | NA | NA |
| rs58481194 | 3 | 172947355 | NA | 3.51E-05 | | 0.02589 | 0.005965 |
| rs6443469 | 3 | 177286455 | NA | 3.23E-05 | | -0.005338 | 0.001224 |
| rs7627379 | 3 | 177286946 | NA | 3.23E-05 | | -0.005338 | 0.001224 |
| rs937509 | 3 | 177287701 | NA | 3.23E-05 | | -0.005338 | 0.001224 |
| rs6768291 | 3 | 177290183 | NA | 3.23E-05 | | -0.005338 | 0.001224 |
| rs2055366 | 3 | 177290345 | NA | 3.23E-05 | | -0.005338 | 0.001224 |
| rs1970860 | 3 | 177290953 | NA | 3.23E-05 | | -0.005338 | 0.001224 |
| rs1970861 | 3 | 177291213 | NA | 3.23E-05 | | -0.005338 | 0.001224 |
| rs4241483 | 3 | 177291318 | NA | 3.23E-05 | | -0.005338 | 0.001224 |
| rs5854759 | 3 | 177292429 | NA | 3.23E-05 | | -0.005338 | 0.001224 |
| rs6806363 | 3 | 177294081 | NA | 3.23E-05 | | -0.005338 | 0.001224 |
| rs13083115 | 3 | 177294781 | NA | 3.23E-05 | | -0.005338 | 0.001224 |
| rs1973469 | 3 | 177295093 | NA | 3.23E-05 | | -0.005338 | 0.001224 |
| rs2863011 | 3 | 177295520 | NA | 3.23E-05 | | -0.005338 | 0.001224 |
| rs2133596 | 3 | 177295608 | NA | 3.23E-05 | | -0.005338 | 0.001224 |
| rs11713121 | 3 | 177295661 | NA | 3.23E-05 | | -0.005338 | 0.001224 |
| rs60882310 | 3 | 177295932 | NA | 3.23E-05 | | -0.005338 | 0.001224 |
| rs35403806 | 3 | 177296263 | NA | 3.23E-05 | | -0.005338 | 0.001224 |
| rs36018104 | 3 | 177296267 | NA | 3.23E-05 | | -0.005338 | 0.001224 |
| rs57592389 | 3 | 177296362 | NA | 3.23E-05 | | -0.005338 | 0.001224 |

(continued)

| SNP ID | chr | position | gene locus | p-value | SNP location in gene locus | BETA | SE |
|---|---|---|---|---|---|---|---|
| rs58786527 | 3 | 177296363 | NA | 3.23E-05 | | -0.005338 | 0.001224 |
| rs13062339 | 3 | 177296394 | NA | 3.23E-05 | | -0.005338 | 0.001224 |
| rs13084960 | 3 | 177296448 | NA | 3.23E-05 | | -0.005338 | 0.001224 |
| rs34858531 | 3 | 177296554 | NA | 3.23E-05 | | -0.005338 | 0.001224 |
| rs4857705 | 3 | 177296885 | NA | 3.23E-05 | | -0.005338 | 0.001224 |
| rs4857706 | 3 | 177296956 | NA | 3.23E-05 | | -0.005338 | 0.001224 |
| rs4857707 | 3 | 177297074 | NA | 3.23E-05 | | -0.005338 | 0.001224 |
| rs4857708 | 3 | 177297173 | NA | 3.23E-05 | | -0.005338 | 0.001224 |
| 3:177297644 :G: GTT | 3 | 177297644 | NA | 3.23E-05 | NA | -0.005338 | 0.001224 |
| rs6801659 | 3 | 177297792 | NA | 3.23E-05 | | -0.005338 | 0.001224 |
| rs6801849 | 3 | 177297824 | NA | 3.23E-05 | | -0.005338 | 0.001224 |
| rs1463859 | 3 | 177298071 | NA | 3.23E-05 | | -0.005338 | 0.001224 |
| rs1463858 | 3 | 177298127 | NA | 3.23E-05 | | -0.005338 | 0.001224 |
| rs1463857 | 3 | 177298327 | NA | 3.23E-05 | | -0.005338 | 0.001224 |

[Table 3-16]

| SNP ID | chr | position | gene locus | p-value | SNP location in gene locus | BETA | SE |
|---|---|---|---|---|---|---|---|
| rs4555562 | 3 | 177298563 | NA | 3.23E-05 | | -0.005338 | 0.001224 |
| rs4257602 | 3 | 177298695 | NA | 3.23E-05 | | -0.005338 | 0.001224 |
| rs4273409 | 3 | 177298785 | NA | 3.23E-05 | | -0.005338 | 0.001224 |
| rs4290846 | 3 | 177298934 | NA | 3.23E-05 | | -0.005338 | 0.001224 |
| rs35168340 | 3 | 177299052 | NA | 3.23E-05 | | -0.005338 | 0.001224 |

(continued)

| SNP ID | chr | position | gene locus | p-value | SNP location in gene locus | BETA | SE |
|---|---|---|---|---|---|---|---|
| rs34332266 | 3 | 177299061 | NA | 3.23E-05 | | -0.005338 | 0.001224 |
| rs34316712 | 3 | 177299065 | NA | 3.23E-05 | | -0.005338 | 0.001224 |
| rs36102700 | 3 | 177299109 | NA | 3.23E-05 | | -0.005338 | 0.001224 |
| rs35921520 | 3 | 177299232 | NA | 3.23E-05 | | -0.006338 | 0.001224 |
| rs6805429 | 3 | 177299269 | NA | 3.23E-05 | | -0.005338 | 0.001224 |
| rs6781232 | 3 | 177299379 | NA | 3.23E-05 | | -0.005338 | 0.001224 |
| 3:177299739 :A:ATTT | 3 | 177299739 | NA | 3.23E-05 | NA | -0.005338 | 0.001224 |
| rs6808260 | 3 | 177299832 | NA | 3.23E-05 | | -0.005338 | 0.001224 |
| rs7630147 | 3 | 177300391 | NA | 3.23E-05 | | -0.005338 | 0.001224 |
| rs7610690 | 3 | 177300717 | NA | 3.23E-05 | | -0.005338 | 0.001224 |
| rs7610926 | 3 | 177300753 | NA | 3.23E-05 | | -0.005338 | 0.001224 |
| rs4857709 | 3 | 177300955 | NA | 3.23E-05 | | -0.005338 | 0.001224 |
| rs4857710 | 3 | 177300965 | NA | 3.23E-05 | | -0.005338 | 0.001224 |
| rs4857711 | 3 | 177300980 | NA | 3.23E-05 | | -0.005338 | 0.001224 |
| rs4857712 | 3 | 177301016 | NA | 3.23E-05 | | -0.005338 | 0.001224 |
| rs6763782 | 3 | 177301280 | NA | 3.23E-05 | | -0.005338 | 0.001224 |
| 3:177301483 : AGGACTTAAGCAGAT:A | 3 | 177301483 | NA | 3.23E-05 | NA | -0.005338 | 0.001224 |
| rs2863019 | 3 | 177301836 | NA | 3.23E-05 | | -0.005338 | 0.001224 |
| rs2863018 | 3 | 177301911 | NA | 3.23E-05 | | -0.005338 | 0.001224 |
| rs2133592 | 3 | 177301939 | NA | 3.23E-05 | | -0.005338 | 0.001224 |
| rs2133593 | 3 | 177301992 | NA | 3.23E-05 | | -0.005338 | 0.001224 |
| rs2863017 | 3 | 177302033 | NA | 3.23E-05 | | -0.005338 | 0.001224 |

(continued)

| SNP ID | chr | position | gene locus | p-value | SNP location in gene locus | BETA | SE |
|---|---|---|---|---|---|---|---|
| rs2173608 | 3 | 177302338 | **NA** | 3.23E-05 | | -0.005338 | 0.001224 |
| rs6783033 | 3 | 177302751 | **NA** | 3.23E-05 | | -0.005338 | 0.001224 |
| rs6770216 | 3 | 177302927 | **NA** | 3.23E-05 | | -0.005338 | 0.001224 |
| rs2863016 | 3 | 177302980 | **NA** | 3.23E-05 | | -0.005338 | 0.001224 |
| rs2863015 | 3 | 177303179 | **NA** | 3.23E-05 | | -0.005338 | 0.001224 |
| rs2863014 | 3 | 177303192 | **NA** | 3.23E-05 | | -0.005338 | 0.001224 |
| rs2133595 | 3 | 177303610 | **NA** | 3.23E-05 | | -0.005338 | 0.001224 |
| rs1875095 | 3 | 177303723 | **NA** | 3.23E-05 | | -0.005338 | 0.001224 |

[Table 3-17]

| SNP ID | chr | position | gene locus | p-value | SNP location in gene locus | BETA | SE |
|---|---|---|---|---|---|---|---|
| rs1875096 | 3 | 177303864 | NA | 3.23E-05 | | -0.005338 | 0.001224 |
| rs1875097 | 3 | 177304032 | NA | 3.23E-05 | | -0.005338 | 0.001224 |
| rs7620560 | 3 | 177304176 | NA | 3.23E-05 | | -0.005338 | 0.001224 |
| rs7620503 | 3 | 177304298 | NA | 3.23E-05 | | -0.005338 | 0.001224 |
| rs7634809 | 3 | 177304668 | NA | 3.23E-05 | | -0.005338 | 0.001224 |
| rs7645281 | 3 | 177304836 | NA | 3.23E-05 | | -0.005338 | 0.001224 |
| rs7623309 | 3 | 177304851 | NA | 3.23E-05 | | -0.005338 | 0.001224 |
| rs6443474 | 3 | 177305124 | NA | 3.23E-05 | | -0.005338 | 0.001224 |
| rs6777561 | 3 | 177305198 | NA | 3.23E-05 | | -0.005338 | 0.001224 |
| rs13077994 | 3 | 177305705 | NA | 3.23E-05 | | -0.005338 | 0.001224 |
| rs58453015 | 3 | 177305847 | NA | 3.23E-05 | NA | -0.005338 | 0.001224 |
| rs4857713 | 3 | 177306442 | NA | 3.23E-05 | | -0.005338 | 0.001224 |
| rs4857612 | 3 | 177306621 | NA | 3.23E-05 | | -0.005338 | 0.001224 |
| rs11350527 | 3 | 177306757 | NA | 3.23E-05 | | -0.005338 | 0.001224 |
| rs7637965 | 3 | 177307642 | NA | 3.23E-05 | | -0.005338 | 0.001224 |
| rs6443476 | 3 | 177307695 | NA | 3.23E-05 | | -0.005338 | 0.001224 |
| rs6443477 | 3 | 177307933 | NA | 3.23E-05 | | -0.005338 | 0.001224 |
| rs6769466 | 3 | 177308014 | NA | 3.23E-05 | | -0.005338 | 0.001224 |
| rs7616679 | 3 | 177308486 | NA | 3.23E-05 | | -0.005338 | 0.001224 |
| rs11708469 | 3 | 177308718 | NA | 3.23E-05 | | -0.005338 | 0.001224 |

(continued)

| SNP ID | chr | position | gene locus | p-value | SNP location in gene locus | BETA | SE |
|---|---|---|---|---|---|---|---|
| rs6797636 | 3 | 177309442 | NA | 3.23E-05 | | -0.005338 | 0.001224 |
| rs13079175 | 3 | 177309533 | NA | 3.23E-05 | | -0.005338 | 0.001224 |
| 3:186444809 :A:G | 3 | 186444809 | NA | 2.84E-05 | NA | 0.01758 | 0.004 |
| rs117064584 | 3 | 189164430 | NA | 1.73E-06 | | 0.01443 | 0.002834 |
| rs117987791 | 3 | 189523891 | TPfi3 | 7.80E-06 | intronvariant | 0.01998 | 0.004228 |
| rs72613184 | 4 | 5235040 | STK32B | 2.42E-05 | intron_variant | 0.0107 | 0.002412 |
| rs149515956 | 4 | 6341633 | PPP2R2C | 2.19E-06 | intron_variant | 0.02908 | 0.005774 |
| rs147293851 | 4 | 13966004 | NA | 3.22E-05 | | 0.02682 | 0.00615 |
| rs142792029 | 4 | 14017337 | NA | 3.22E-05 | | 0.02682 | 0.00615 |
| rs62294496 | 4 | 17260883 | NA | 3.51E-05 | | 0.02589 | 0.005965 |
| rs118004921 | 4 | 17304792 | NA | 2.19E-06 | | 0.02908 | 0.005774 |
| rs11933789 | 4 | 17825159 | NCAPG | 2.86E-O5 | intron_variant | 0.01861 | 0.004237 |
| rs62295154 | 4 | 19769915 | NA | 2.33E-05 | | 0.01694 | 0.003811 |
| rs13435165 | 4 | 19772721 | NA | 2.33E-05 | | 0.01694 | 0.003811 |
| rs79235820 | 4 | 22171802 | NA | 2.93E-06 | | 0.02385 | 0.004803 |
| rs116460686 | 4 | 31892334 | NA | 1.07E-05 | | 0.02722 | 0.005861 |

[Table 3-18]

| SNP ID | chr | position | gene locus | p-value | SNP location in gene locus | BETA | SE |
|---|---|---|---|---|---|---|---|
| rs115446883 | 4 | 31896317 | NA | 1.07E-05 | | 0.02722 | 0.005861 |
| rsl46966940 | 4 | 41153294 | APBB2 | 8.52E-06 | intron_variant | 0.02781 | 0.005915 |
| 4:41784275 :A:C | 4 | 41784275 | NA | 3.51E-05 | NA | 0.02589 | 0.005965 |
| rs11942428 | 4 | 44580439 | NA | 3.40E-05 | | 0.008618 | 0.001982 |
| rs148742662 | 4 | 54567050 | NA | 3.18E-07 | NA | 0.02247 | 0.00409 |
| rs144162078 | 4 | 54580834 | AC058822.1 | 1.04E-07 | intron_variant | 0.03229 | 0.005617 |
| 4:5458235 1:CA:C | 4 | 54582351 | NA | 3.49E-05 | NA | 0.009926 | 0.002287 |
| rs143711414 | 4 | 54604589 | AC058822.1 | 1.04E-07 | intron_variant | 0.03229 | 0.005617 |
| 4:61494990 :A:AT | 4 | 61494990 | NA | 4.09E-06 | NA | 0.01556 | 0.003186 |
| rs117340277 | 4 | 63017478 | NA | 1.07E-05 | | 0.02722 | 0.005861 |
| rs76314468 | 4 | 64039941 | NA | 2.72E-07 | | 0.01569 | 0.002839 |
| rs79892130 | 4 | 64040056 | NA | 2.72E-07 | | 0.01569 | 0.002839 |
| rs75655453 | 4 | 64040607 | NA | 2.72E-07 | | 0.01569 | 0.002839 |
| rs77849378 | 4 | 64041936 | NA | 2.72E-07 | | 0.01569 | 0.002839 |
| rs74461070 | 4 | 64042807 | NA | 2.72E-07 | | 0.01569 | 0.002839 |
| rs77802168 | 4 | 64042829 | NA | 2.72E-07 | | 0.01569 | 0.002839 |
| rs6816831 | 4 | 64044660 | NA | 2.72E-07 | | 0.01569 | 0.002839 |

(continued)

| SNP ID | chr | position | gene locus | p-value | SNP location in gene locus | BETA | SE |
|---|---|---|---|---|---|---|---|
| rs6848438 | 4 | 64044675 | NA | 2.72E-07 | | 0.01569 | 0.002839 |
| rs143395876 | 4 | 64046194 | NA | 2.72E-07 | | 0.01569 | 0.002839 |
| rs4502710 | 4 | 64047829 | NA | 2.72E-07 | | 0.01569 | 0.002839 |
| rs143437614 | 4 | 64048178 | NA | 2.72E-07 | | 0.01569 | 0.002839 |
| rs4035516 | 4 | 64049319 | NA | 2.72E-07 | | 0.01569 | 0.002839 |
| rs138747488 | 4 | 64049566 | NA | 2.72E-07 | | 0.01569 | 0.002839 |
| rs79890012 | 4 | 64050856 | NA | 2.72E-07 | | 0.01569 | 0.002839 |
| rs139003026 | 4 | 64051485 | NA | 2.72E-07 | | 0.01569 | 0.002839 |
| rs116239045 | 4 | 64051678 | NA | 2.72E-07 | | 0.01569 | 0.002839 |
| rs139446359 | 4 | 64052143 | NA | 2.72E-07 | | 0.01569 | 0.002839 |
| rs144091716 | 4 | 64052292 | NA | 2.72E-07 | | 0.01569 | 0.002839 |
| rs114269773 | 4 | 64053241 | NA | 2.72E-07 | | 0.01569 | 0.002839 |
| rs148230700 | 4 | 64054216 | NA | 2.72E-07 | | 0.01569 | 0.002839 |
| rs114403067 | 4 | 64054570 | NA | 2.72E-07 | | 0.01569 | 0.002839 |
| rs115936064 | 4 | 64055253 | NA | 2.72E-07 | | 0.01569 | 0.002839 |
| rs142760460 | 4 | 64055706 | NA | 2.72E-07 | | 0.01569 | 0.002839 |
| rs147936120 | 4 | 64057031 | NA | 2.72E-07 | | 0.01569 | 0.002839 |
| rs115409842 | 4 | 64057046 | NA | 2.72E-07 | | 0.01569 | 0.002839 |
| rs115633947 | 4 | 64058695 | NA | 2.72E-07 | | 0.01569 | 0.002839 |

[Table 3-19]

| SNP ID | chr | position | gene locus | p-value | SNP location in gene locus | BETA | SE |
|---|---|---|---|---|---|---|---|
| rs148720007 | 4 | 64058783 | NA | 2.72E-07 | | 0.01569 | 0.002839 |
| rs140223857 | 4 | 64058820 | NA | 2.72E-07 | | 0.01569 | 0.002839 |
| rs7680092 | 4 | 64061134 | NA | 2.72E-07 | | 0.01569 | 0.002839 |
| 4:64061283:AT: ATT | 4 | 64061283 | NA | NA | NA | NA | NA |
| 4:64061283:AT:A | 4 | 64061283 | NA | 2.72E-07 | NA | 0.01569 | 0.002839 |
| rs80287150 | 4 | 64061543 | NA | 4.41E-06 | | 0.01799 | 0.003696 |
| rs115458156 | 4 | 64061728 | NA | 2.72E-07 | | 0.01569 | 0.002839 |
| 4:64062006:C: CA | 4 | 64062006 | NA | NA | NA | NA | NA |
| 4:64062006:C: CAA | 4 | 64062006 | NA | 2.72E-07 | NA | 0.01569 | 0.002839 |
| rs74340891 | 4 | 64062350 | NA | 2.72E-07 | | 0.01569 | 0.002839 |
| rs116187324 | 4 | 64064808 | NA | 2.72E-07 | | 0.01569 | 0.002839 |
| rs74796023 | 4 | 64066685 | NA | 2.72E-07 | | 0.01569 | 0.002839 |

(continued)

| SNP ID | chr | position | gene locus | p-value | SNP location in gene locus | BETA | SE |
|---|---|---|---|---|---|---|---|
| rs74864007 | 4 | 64066878 | NA | 2.72E-07 | | 0.01569 | 0.002839 |
| rs115897647 | 4 | 64066892 | NA | 2.72E-07 | | 0.01569 | 0.002839 |
| rs201614722 | 4 | 64067415 | NA | 2.72E-07 | | 0.01569 | 0.002839 |
| rs201701309 | 4 | 64067892 | NA | 2.72E-07 | | 0.01569 | 0.002839 |
| rs201649360 | 4 | 64068147 | NA | 2.72E-07 | | 0.01569 | 0.002839 |
| rs184640169 | 4 | 64069492 | NA | 4.41E-06 | | 0.01799 | 0.003696 |
| rs77460761 | 4 | 64069972 | NA | 4.41E-06 | | 0.01799 | 0.003696 |
| rs182226229 | 4 | 64070081 | NA | 4.18E-07 | | 0.01335 | 0.002459 |
| rs183837605 | 4 | 64071489 | NA | 4.18E-07 | | 0.01335 | 0.002459 |
| rs146847342 | 4 | 64072931 | NA | 4.18E-07 | | 0.01335 | 0.002459 |
| rs76764827 | 4 | 64072957 | NA | 4.18E-07 | | 0.01335 | 0.002459 |
| rs74392895 | 4 | 64074311 | NA | 4.18E-07 | | 0.01335 | 0.002459 |
| rs79387056 | 4 | 64074978 | NA | 4.18E-07 | | 0.01335 | 0.002459 |
| rs75876899 | 4 | 64076116 | NA | 4.18E-07 | | 0.01335 | 0.002459 |
| rs116177742 | 4 | 64076886 | NA | 4.18E-07 | | 0.01335 | 0.002459 |
| rs114946744 | 4 | 64076904 | NA | 4.18E-07 | | 0.01335 | 0.002459 |
| rs80315791 | 4 | 64078203 | NA | 4.18E-07 | | 0.01335 | 0.002459 |
| rs80199562 | 4 | 64079037 | NA | 4.18E-07 | | 0.01335 | 0.002459 |
| rs76630438 | 4 | 64079127 | NA | 4.18E-07 | | 0.01335 | 0.002459 |
| rs78126082 | 4 | 64079408 | NA | 4.18E-07 | | 0.01335 | 0.002459 |
| rs2881626 | 4 | 64080016 | NA | 4.18E-07 | | 0.01335 | 0.002459 |
| rs75900830 | 4 | 64080483 | NA | 4.18E-07 | | 0.01335 | 0.002459 |
| rs77305257 | 4 | 64080642 | NA | 4.18E-07 | | 0.01335 | 0.002459 |
| rs77019672 | 4 | 64081403 | NA | 4.18E-07 | | 0.01335 | 0.002459 |

[Table 3-20]

| SNP ID | ch r | position | gene locus | p-value | SNP location in gene locus | BETA | SE |
|---|---|---|---|---|---|---|---|
| rs115778258 | 4 | 64082381 | NA | 4.18E-07 | | 0.01335 | 0.002459 |
| rs79113257 | 4 | 64086632 | NA | 4.18E-07 | | 0.01335 | 0.002459 |
| rs147278503 | 4 | 93215159 | NA | 1.03E-05 | | 0.01484 | 0.003188 |
| rs141054368 | 4 | 93217241 | NA | 1.03E-05 | | 0.01484 | 0.003188 |
| rs7658734 | 4 | 96509012 | NA | 3.14E-05 | | 0.01867 | 0.004274 |
| 4:96513678 :C:T | 4 | 96513678 | NA | 3.14E-05 | NA | 0,01867 | 0.004274 |
| rs62306715 | 4 | 96513884 | NA | 3.14E-05 | | 0.01867 | 0.004274 |
| 4:96514526 :T:C | 4 | 96514526 | NA | 3.14E-05 | NA | 0.01867 | 0.004274 |
| rs113843935 | 4 | 96514739 | NA | 3.14E-05 | | 0.01867 | 0.004274 |

(continued)

| SNP ID | ch r | position | gene locus | p-value | SNP location in gene locus | BETA | SE |
|---|---|---|---|---|---|---|---|
| rs67930114 | 4 | 96515437 | NA | 3.14E-05 | | 0.01867 | 0.004274 |
| rs57799366 | 4 | 96517154 | NA | 3.14E-05 | | 0.01867 | 0.004274 |
| rs62306720 | 4 | 96517458 | NA | 3.14E-05 | | 0.01867 | 0.004274 |
| 4:96518109 :G:GA | 4 | 96518109 | NA | 3.14E-05 | NA | 0.01867 | 0.004274 |
| rs2123367 | 4 | 109711528 | NA | 2.50E-05 | | 0.02626 | 0.005931 |
| rs77922013 | 4 | 109712306 | NA | 2.50E-05 | | 0.02626 | 0.005931 |
| rs76550713 | 4 | 109712541 | NA | 2.50E-05 | | 0.02626 | 0.005931 |
| rs150990204 | 4 | 109712817 | NA | 2.50E-05 | | 0.02626 | 0.005931 |
| rs17039415 | 4 | 109722415 | NA | 2.50E-05 | | 0.02626 | 0.005931 |
| rs146480894 | 4 | 113229907 | ALPK1 | 5.59E-06 | non_coding_tra nscript_ variant | 0.02836 | 0.005899 |
| rs150718823 | 4 | 113232846 | ALPK1 | 5.59E-06 | non_coding_tra nscript_ variant | 0.02836 | 0.005899 |
| rs115122068 | 4 | 117719540 | NA | 8.52E-06 | | 0.02781 | 0.005915 |
| rs150939893 | 4 | 117744934 | NA | 8.52E-06 | | 0.02781 | 0.005915 |
| rs190696080 | 4 | 117750582 | NA | 8.52E-O6 | | 0.02781 | 0.005915 |
| rs113295558 | 4 | 117755974 | NA | 8.52E-06 | | 0.02781 | 0.005915 |
| rs191714458 | 4 | 117758243 | NA | 8.52E-06 | | 0.02781 | 0.005915 |
| rs186618613 | 4 | 117762073 | NA | 8.52E-06 | | 0.02781 | 0.005915 |
| rs190966932 | 4 | 117762377 | NA | 8.52E-06 | | 0.02781 | 0.005915 |
| rs192343018 | 4 | 117767150 | NA | 8.52E-06 | | 0.02781 | 0.005915 |
| rs181001803 | 4 | 117773288 | NA | 8.52E-06 | | 0.02781 | 0.005915 |
| rs7684310 | 4 | 117773712 | NA | 8.52E-06 | | 0.02781 | 0.005915 |
| rs189192694 | 4 | 117774888 | NA | 8.52E-06 | | 0.02781 | 0.005915 |
| rs191512269 | 4 | 117777305 | NA | 8.52E-06 | | 0.02781 | 0.005915 |
| 4:117780739:G:T | 4 | 117780739 | NA | 8.52E-06 | NA | 0.02781 | 0.005915 |
| rs182361185 | 4 | 117787075 | NA | 8.52E-06 | | 0.02781 | 0.005915 |
| 4:117793406:A:T | 4 | 117793406 | NA | 8.52E-06 | NA | 0.02781 | 0.005915 |
| rs74657487 | 4 | 122236579 | NA | 3.98E-07 | | 0.02549 | 0.004685 |

[Table 3-21]

| SNP ID | chr | position | gene locus | p-value | SNP location in gene locus | BETA | SE |
|---|---|---|---|---|---|---|---|
| rs80300492 | 4 | 122239822 | NA | 3.98E-07 | | 0.02549 | 0.004685 |
| rs75622168 | 4 | 122240327 | NA | 3.98E-07 | | 0.02549 | 0.004685 |
| rs79585077 | 4 | 122241796 | NA | 3.98E-07 | | 0.02549 | 0.004685 |

(continued)

| SNP ID | chr | position | gene locus | p-value | SNP location in gene locus | BETA | SE |
|---|---|---|---|---|---|---|---|
| rs76926525 | 4 | 122242129 | NA | 3.98E-07 | | 0.02549 | 0.004685 |
| rs77958862 | 4 | 122244530 | NA | 3.98E-07 | | 0.02549 | 0.004685 |
| rs77294448 | 4 | 122246900 | NA | 3.98E-07 | | 0.02549 | 0.004685 |
| rs6821123 | 4 | 122249973 | QRFPR | 3.98E-07 | 3_prime_UTR_variant | 0.02549 | 0.004685 |
| rs113334103 | 4 | 122252388 | QRFPR | 3.98E-07 | intron_variant | 0.02549 | 0.004685 |
| rs55975435 | 4 | 122254014 | QRFPR | 3.98E-07 | 3_primeUTRvariant | 0.02549 | 0.004685 |
| rs55776058 | 4 | 122254284 | QRFPR | 3.98E-07 | intron_variant | 0.02549 | 0.004685 |
| rs2302308 | 4 | 122258149 | QRFPR | 3.98E-07 | intron_variant | 0.02549 | 0.004685 |
| rs74398478 | 4 | 122259584 | QRFPR | 3.98E-07 | intron_variant | 0.02549 | 0.004685 |
| rs17051338 | 4 | 122260042 | QRFPR | 3.98E-07 | intron_variant | 0.02549 | 0.004685 |
| rs77438622 | 4 | 122264692 | QRFPR | 3.98E-07 | intron_variant | 0.02549 | 0.004685 |
| rs76536576 | 4 | 122268524 | QRFPR | 2.50E-05 | intron_variant | 0.02626 | 0.005931 |
| rs182992688 | 4 | 122270068 | QRFPR | 2.50E-05 | intron_variant | 0.02626 | 0.005931 |
| rs187168138 | 4 | 122270076 | QRFPR | 2.50E-05 | intron_variant | 0.02626 | 0.005931 |
| rs77006299 | 4 | 122270900 | QRFPR | 3.98E-07 | intron_variant | 0.02549 | 0.004685 |
| rs10518388 | 4 | 122280335 | QRFPR | 3.98E-07 | intron_variant | 0.02549 | 0.004685 |
| rs17051344 | 4 | 122285263 | QRFPR | 3.98E-07 | intron_variant | 0.02549 | 0.004685 |
| rs66511907 | 4 | 122286927 | QRFPR | 3.98E-07 | intron_variant | 0.02549 | 0.004685 |
| rs76503879 | 4 | 122287794 | QRFPR | 3.98E-07 | intron_variant | 0.02549 | 0.004685 |
| 4:133557517:A:T | 4 | 133557517 | NA | 2.40E-05 | NA | 0.01518 | 0.00342 |
| rs201392439 | 4 | 134152521 | NA | 2.43E-05 | | 0.01737 | 0.003917 |

(continued)

| SNP ID | chr | position | gene locus | p-value | SNP location in gene locus | BETA | SE |
|---|---|---|---|---|---|---|---|
| 4: 136278810 : T:G | 4 | 136278810 | NA | 4.71E-07 | NA | 0.03074 | 0.00569 |
| rs79757000 | 4 | 136309269 | NA | 4.71E-07 | | 0.03074 | 0.00569 |
| rs10005391 | 4 | 136318357 | NA | 4.71E-07 | | 0.03074 | 0.00569 |
| rs10017268 | 4 | 136318429 | NA | 4.71E-07 | | 0.03074 | 0.00569 |
| rs10005518 | 4 | 136318641 | NA | 4.71E-07 | | 0.03074 | 0.00569 |
| rs10008741 | 4 | 136319479 | NA | 4.71E-07 | | 0.03074 | 0.00569 |
| rs10008854 | 4 | 136319621 | NA | 4.71E-07 | | 0.03074 | 0.00569 |
| 4:136320586 G:GTT | 4 | 136320586 | NA | 4.71E-07 | NA | 0.03074 | 0.00569 |
| 4: 136320587 : C:A | 4 | 136320587 | NA | 4.71E-07 | NA | 0.03074 | 0.00569 |
| rs150556358 | 4 | 136320899 | NA | 4.71E-07 | NA | 0.03074 | 0.00569 |
| rs28701471 | 4 | 136321575 | NA | 4.71E-07 | | 0.03074 | 0.00569 |
| 4: 136321714 : CAA:CA | 4 | 136321714 | NA | NA | NA | NA | NA |

[Table 3-22]

| SNP ID | chr | position | gene locus | p-value | SNP location in gene locus | BETA | SE |
|---|---|---|---|---|---|---|---|
| 4:136321714 :CAA:C | 4 | 136321714 | NA | 4.83E-05 | NA | 0.02074 | 0.004874 |
| rs33960846 | 4 | 136321824 | NA | 4.71E-07 | | 0.03074 | 0.00569 |
| rs28481887 | 4 | 136322868 | NA | 4.71E-07 | | 0.03074 | 0.00569 |
| rs28673025 | 4 | 136322980 | NA | 4.71E-07 | | 0.03074 | 0.00569 |
| rs28709698 | 4 | 136323895 | NA | 4.71E-07 | | 0.03074 | 0.00569 |
| rs76261505 | 4 | 136324955 | NA | 4.71E-07 | | 0.03074 | 0.00569 |
| rs10028807 | 4 | 136327412 | NA | 4.71E-07 | | 0.03074 | 0.00569 |
| rs145137955 | 4 | 136327637 | NA | 4.71E-07 | | 0.03074 | 0.00569 |
| 4:136327754 :CT:C | 4 | 136327754 | NA | 4.71E-07 | NA | 0.03074 | 0.00569 |
| rs28872626 | 4 | 136327960 | NA | 4.71E-07 | | 0.03074 | 0.00569 |

(continued)

| SNP ID | chr | position | gene locus | p-value | SNP location in gene locus | BETA | SE |
|---|---|---|---|---|---|---|---|
| rs28790884 | 4 | 136327980 | NA | 4.71E-07 | | 0.03074 | 0.00569 |
| rs28838886 | 4 | 136327990 | NA | 4.71E-07 | | 0.03074 | 0.00569 |
| rs28857199 | 4 | 136328206 | NA | 4.71E-07 | | 0.03074 | 0.00569 |
| 4:136328885 :T:A | 4 | 136328885 | NA | 4.71E-07 | NA | 0.03074 | 0.00569 |
| 4:136328886 :G:T | 4 | 136328886 | NA | 4.71E-07 | NA | 0.03074 | 0.00569 |
| rs6810998 | 4 | 140693268 | MAML3 | 1.85E-05 | intron_variant | 0.02211 | 0.004908 |
| rs78180498 | 4 | 141207904 | SCOC | 2.59E-05 | intron_variant | 0.008905 | 0.002015 |
| rs78619534 | 4 | 141219174 | SCOC | 2.34E-05 | intron_variant | 0.0098 | 0.002205 |
| rs17021272 | 4 | 147156085 | NA | 3.15E-05 | | 0.02128 | 0.004872 |
| rs72948673 | 4 | 147174693 | NA | 3.15E-05 | | 0.02128 | 0.004872 |
| rs60367087 | 4 | 147199809 | SLC10A7 | 3.15E-05 | intron_variant | 0.02128 | 0.004872 |
| rs72950642 | 4 | 147238329 | SLC10A7 | 3.15E-05 | intron_variant | 0.02128 | 0.004872 |
| rs72950644 | 4 | 147239235 | SLC10A7 | 3.15E-05 | intron_variant | 0.02128 | 0.004872 |
| 4:147245033 :T:C | 4 | 147245033 | NA | 3.15E-05 | NA | 0.02128 | 0.004872 |
| rs112713961 | 4 | 147245957 | SLC10A7 | 3.15E-05 | intron_variant | 0.02128 | 0.004872 |
| rs72950652 | 4 | 147246540 | SLC10A7 | 3.15E-05 | intron_variant | 0.02128 | 0.004872 |
| rs72950654 | 4 | 147248032 | SLC10A7 | 3.15E-05 | intron_variant | 0.02128 | 0.004872 |
| rs113969284 | 4 | 147248604 | SLC10A7 | 3.15E-05 | intron_variant | 0.02128 | 0.004872 |
| rs72950658 | 4 | 147253902 | SLC10A7 | 3.15E-05 | intron_variant | 0.02128 | 0.004872 |
| rs72950664 | 4 | 147255021 | SLC10A7 | 3.15E-05 | intron_variant | 0.02128 | 0.004872 |
| rs72950667 | 4 | 147255582 | SLC10A7 | 3.15E-05 | intron_variant | 0.02128 | 0.004872 |
| rs142662018 | 4 | 147255649 | SLC10A7 | 3.15E-05 | intron_variant | 0.02128 | 0.004872 |
| rs72950672 | 4 | 147260169 | SLC10A7 | 3.15E-05 | intron_variant | 0.02128 | 0.004872 |
| rs72950677 | 4 | 147261151 | SLC10A7 | 3.15E-05 | intron_variant | 0.02128 | 0.004872 |
| rs72950679 | 4 | 147262990 | SLC10A7 | 3.15E-05 | intron_variant | 0.02128 | 0.004872 |

(continued)

| SNP ID | chr | position | gene locus | p-value | SNP location in gene locus | BETA | SE |
|---|---|---|---|---|---|---|---|
| rs72950681 | 4 | 147264589 | SLC10A7 | 3.15E-05 | intron_variant | 0.02128 | 0.004872 |

[Table 3-23]

| SNP ID | chr | position | gene locus | p-value | SNP location in gene locus | BETA | SE |
|---|---|---|---|---|---|---|---|
| rs72950682 | 4 | 147264764 | SLC10A7 | 3.15E-05 | intron_variant | 0.02128 | 0.004872 |
| rs78199829 | 4 | 147267044 | SLC10A7 | 3.15E-05 | intron_variant | 0.02128 | 0.004872 |
| rs72950684 | 4 | 147267419 | SLC10A7 | 3.15E-05 | intron_variant | 0.02128 | 0.004872 |
| rs72950690 | 4 | 147269478 | SLC10A7 | 3.15E-05 | intron_variant | 0.02128 | 0.004872 |
| rs58672301 | 4 | 147269836 | SLC10A7 | 3.15E-05 | intron_variant | 0.02128 | 0.004872 |
| rs111963516 | 4 | 147271022 | SLC10A7 | 3.15E-05 | intron_variant | 0.02128 | 0.004872 |
| rs76206578 | 4 | 147271027 | SLC10A7 | 3.15E-05 | intron_variant | 0.02128 | 0.004872 |
| 4:147271544 :A:G | 4 | 147271544 | NA | 3.15E-05 | NA | 0.02128 | 0.004872 |
| 4:147272211 :A:T | 4 | 147272211 | NA | 3.15E-05 | NA | 0.02128 | 0.004872 |
| 4:147272243 :A:G | 4 | 147272243 | NA | 3.15E-05 | NA | 0.02128 | 0.004872 |
| 4:147272464 :C:T | 4 | 147272464 | NA | 3.15E-05 | NA | 0.02128 | 0.004872 |
| rs72950697 | 4 | 147277445 | SLC10A7 | 3.15E-05 | intron_variant | 0.02128 | 0.004872 |
| rs58494546 | 4 | 147278079 | SLC10A7 | 3.15E-05 | intron_variant | 0.02128 | 0.004872 |
| rs72952504 | 4 | 147278240 | SLC10A7 | 3.15E-05 | intron_variant | 0.02128 | 0.004872 |
| rs72952506 | 4 | 147278447 | SLC10A7 | 3.15E-05 | intron_variant | 0.02128 | 0.004872 |
| rs72952508 | 4 | 147278472 | SLC10A7 | 3.15E-05 | intron_variant | 0.02128 | 0.004872 |
| rs11455281 | 4 | 147279083 | SLC10A7 | 3.15E-05 | intron_variant | 0.02128 | 0.004872 |
| rs111635640 | 4 | 147279100 | SLC10A7 | 3.15E-05 | intronvariant | 0.02128 | 0.004872 |
| rs6816058 | 4 | 147279394 | SLC10A7 | 3.15E-05 | intron_variant | 0.02128 | 0.004872 |
| rs72952510 | 4 | 147280039 | SLC10A7 | 3.15E-05 | intron_variant | 0.02128 | 0.004872 |
| rs111476662 | 4 | 147280504 | SLC10A7 | 3.15E-05 | intron_variant | 0.02128 | 0.004872 |
| rs72952511 | 4 | 147280820 | SLC10A7 | 3.15E-05 | intron_variant | 0.02128 | 0.004872 |
| rs72952513 | 4 | 147281127 | SLC10A7 | 3.15E-05 | intron_variant | 0.02128 | 0.004872 |
| rs72952515 | 4 | 147281316 | SLC10A7 | 3.15E-05 | intron_variant | 0.02128 | 0.004872 |
| 4:147281756 :C:CT | 4 | 147281756 | NA | 3.15E-05 | NA | 0.02128 | 0.004872 |
| rs60404214 | 4 | 147282897 | SLC10A7 | 3.15E-05 | intron_variant | 0.02128 | 0.004872 |
| rs2630284 | 4 | 147284129 | SLC10A7 | 3.15E-05 | intron_variant | 0.02128 | 0.004872 |
| rs4027057 | 4 | 147284502 | SLC10A7 | 3.15E-05 | intron_variant | 0.02128 | 0.004872 |
| rs2679154 | 4 | 147285031 | SLC10A7 | 3.15E-05 | intron_variant | 0.02128 | 0.004872 |
| rs2679153 | 4 | 147286796 | SLC10A7 | 3.15E-05 | intron_variant | 0.02128 | 0.004872 |
| rs2255242 | 4 | 147286963 | SLC10A7 | 3.15E-05 | intron_variant | 0.02128 | 0.004872 |

(continued)

| SNP ID | chr | position | gene locus | p-value | SNP location in gene locus | BETA | SE |
|--------|-----|----------|------------|---------|----------------------------|------|-----|
| rs2679151 | 4 | 147287007 | SLC10A7 | 3.15E-05 | intron_variant | 0.02128 | 0.004872 |
| rs2244316 | 4 | 147289414 | SLC10A7 | 3.15E-05 | intron_variant | 0.02128 | 0.004872 |
| rs2244317 | 4 | 147289424 | SLC10A7 | 3.15E-05 | intron_variant | 0.02128 | 0.004872 |
| rs2244318 | 4 | 147289431 | SLC10A7 | 3.15E-05 | intron_variant | 0.02128 | 0.004872 |
| rs2244321 | 4 | 147289522 | SLC10A7 | 3.15E-05 | intron_variant | 0.02128 | 0.004872 |

[Table 3-24]

| SNP ID | chr | position | gene locus | p-value | SNP location in gene locus | BETA | SE |
|---|---|---|---|---|---|---|---|
| rs2679149 | 4 | 147290788 | SLC10A7 | 3.15E-05 | intron_variant | 0.02128 | 0.004872 |
| rs2630278 | 4 | 147292164 | SLC10A7 | 3.15E-05 | intron_variant | 0.02128 | 0.004872 |
| rs2630277 | 4 | 147292832 | SLC1OA7 | 3.15E-05 | intron_variant | 0.02128 | 0.004872 |
| rs2244840 | 4 | 147293347 | SLC10A7 | 3.15E-05 | intron_variant | 0.02128 | 0.004872 |
| rs2244850 | 4 | 147293653 | SLC10A7 | 3.15E-05 | intron_variant | 0.02128 | 0.004872 |
| rs2679145 | 4 | 147294299 | SLC10A7 | 3.15E-05 | intron_variant | 0.02128 | 0.004872 |
| 4:147295793 :C:G | 4 | 147295793 | NA | 3.15E-05 | NA | 0.02128 | 0.004872 |
| rs2679121 | 4 | 147296826 | SLC10A7 | 3.15E-05 | intron_variant | 0.02128 | 0.004872 |
| rs2245274 | 4 | 147297184 | SLC10A7 | 3.15E-05 | intron_variant | 0.02128 | 0.004872 |
| rs2245282 | 4 | 147297453 | SLC10A7 | 3.15E-05 | intron_variant | 0.02128 | 0.004872 |
| rs2262884 | 4 | 147297927 | SLC10A7 | 3.15E-05 | intron_variant | 0.02128 | 0.004872 |
| rs111925113 | 4 | 147298186 | SLC10A7 | 3.15E-05 | intron_variant | 0.02128 | 0.004872 |
| 4:147300051:A :AAAGCAGATAATCTGCT | 4 | 147300051 | NA | 3.15E-05 | NA | 0.02128 | 0.004872 |
| rs2248347 | 4 | 147300898 | SLC10A7 | 3.15E-05 | intron_variant | 0.02128 | 0.004872 |
| rs2248348 | 4 | 147300931 | SLC10A7 | 3.15E-05 | intron_variant | 0.02128 | 0.004872 |
| rs2248826 | 4 | 147305421 | SLC1OA7 | 3.15E-05 | intron_variant | 0.02128 | 0.004872 |
| rs2630297 | 4 | 147306646 | SLC10A7 | 3.15E-05 | intron_variant | 0.02128 | 0.004872 |
| rs2460033 | 4 | 147307479 | SLC10A7 | 3.15E-05 | intron_variant | 0.02128 | 0.004872 |
| rs138801798 | 4 | 157046897 | NA | 2.50E-05 | | 0.02626 | 0.005931 |
| rs6834759 | 4 | 158779650 | NA | 5.81E-06 | | 0.01656 | 0.003451 |
| rs150752209 | 4 | 158783439 | NA | 5.81E-06 | | 0.01656 | 0.003451 |
| rs17036774 | 4 | 158783852 | NA | 2.50E-05 | | 0.02626 | 0.005931 |
| rs150456849 | 4 | 158784767 | NA | 2.50E-05 | | 0.02626 | 0.005931 |
| rs6836401 | 4 | 158786983 | NA | 2.50E-05 | | 0.02626 | 0.005931 |
| rs6813626 | 4 | 158787102 | NA | 2.50E-05 | | 0.02626 | 0.005931 |

| SNP ID | chr | position | gene locus | p-value | SNP location in gene locus | BETA | SE |
|---|---|---|---|---|---|---|---|
| rs55817402 | 4 | 174427221 | NA | 1.40E-05 | | 0.02227 | 0.004867 |
| rs116894δ13 | 4 | 185063146 | ENPP6 | 8.84E-06 | intron_variant | 0.02259 | 0.004813 |
| rs140512564 | 4 | 185116348 | ENPP6 | 1.72E-05 | intron_variant | 0.02707 | 0.005982 |
| rs76233088 | 4 | 185808870 | NA | 1.44E-05 | | 0.01747 | 0.003824 |
| rs35834666 | 4 | 187138175 | KLKB1 | 1.45E-07 | intron_variant | 0.02636 | 0.004646 |
| 4:187138442 :T:TTTG | 4 | 187138442 | NA | 1.45E-07 | NA | 0.02636 | 0.004646 |
| rs34852777 | 4 | 187139062 | KLKB1 | 1.45E-07 | intron_variant | 0.02636 | 0.004646 |
| rs13102931 | 4 | 187145626 | KLKB1 | 1.45E-07 | intron_variant | 0.02636 | 0.004646 |
| rs13102788 | 4 | 187145738 | KLKB1 | 1.45E-07 | intron_variant | 0.02636 | 0.004646 |
| rs138695518 | 4 | 187146418 | KLKB1 | 1.45E-07 | intronvariant | 0.02636 | 0.004646 |

[Table 3-25]

| SNP ID | chr | position | gene locus | p-value | SNP location in gene locus | BETA | SE |
|---|---|---|---|---|---|---|---|
| rs35669195 | 4 | 187146559 | KLKB1 | 1.45E-07 | intron_variant | 0.02636 | 0.004646 |
| rs4253241 | 4 | 187149233 | KLKB1 | 1.45E-07 | intron_variant | 0.02636 | 0.004646 |
| rs4253246 | 4 | 187154424 | KLKB1 | 1.45E-07 | intron_variant | 0.02636 | 0.004646 |
| rs184644242 | 4 | 187157181 | KLKB1 | 1.45E-07 | intron_variant | 0.02636 | 0.004646 |
| rs145549717 | 4 | 187157232 | KLKB1 | 1.45E-07 | intron_variant | 0.02636 | 0.004646 |
| 5:2020945 :T:A | 5 | 2020945 | NA | 3.60E-05 | NA | 0.01313 | 0.00303 |
| rs142127390 | 5 | 11734734 | CTNND2 | 1.04E-07 | intron_variant | 0.03229 | 0.005617 |
| rs10076098 | 5 | 11737241 | CTNND2 | 1.04E-07 | intron_variant | 0.03229 | 0.005617 |
| rs57612673 | 5 | 13733453 | DNAH5 | 8.52E-06 | intron_variant | 0.02781 | 0.005915 |
| 5:14710594 :C:G | 5 | 14710594 | NA | 3.22E-05 | NA | 0.02682 | 0.00615 |
| 5:14722584 :T:C | 5 | 14722584 | NA | 3.22E-05 | NA | 0.02682 | 0.00615 |
| 5:14740327 :C:T | 5 | 14740327 | NA | 3.22E-05 | NA | 0.02682 | 0.00615 |
| rs77818967 | 5 | 19736762 | CDH18 | 4.80E-05 | intron_variant | 0.0162 | 0.003807 |
| rs12657194 | 5 | 19762895 | CDH18 | 2.32E-05 | intron_variant | 0.008049 | 0.00181 |
| rs141488363 | 5 | 19785302 | CDH18 | 4.80E-05 | intron_variant | 0.0162 | 0.003807 |
| rs140741351 | 5 | 19808374 | CDH18 | 4.80E-05 | intron_variant | 0.0162 | 0.003807 |
| rs34847415 | 5 | 19909533 | CDH18 | 3.54E-05 | intron_variant | 0.007588 | 0.00175 |
| rs72740870 | 5 | 19910167 | CDH18 | 2.17E-05 | intron_variant | 0.00813 | 0.001821 |
| rs79509881 | 5 | 24693838 | NA | 3.46E-05 | | 0.01143 | 0.002631 |
| rs62357273 | 5 | 25683630 | NA | 1.94E-05 | | 0.01721 | 0.003831 |
| rs111941500 | 5 | 25688951 | NA | 3.39E-06 | | 0.02369 | 0.004804 |
| rs113538604 | 5 | 25690607 | NA | 3.39E-06 | | 0.02369 | 0.004804 |
| rs13159231 | 5 | 25691118 | NA | 1.94E-05 | | 0.01721 | 0.003831 |
| rs148804803 | 5 | 25696038 | NA | 3.39E-06 | | 0.02369 | 0.004804 |
| rs138213578 | 5 | 25705703 | NA | 3.39E-06 | | 0.02369 | 0.004804 |
| rs200325656 | 5 | 25706012 | NA | 3.39E-06 | | 0.02369 | 0.004804 |
| rs35750564 | 5 | 28029472 | NA | 2.38E-05 | | 0.01346 | 0.003031 |
| rs139552645 | 5 | 28069123 | NA | 4.41E-06 | | 0.01799 | 0.003696 |
| rs147157201 | 5 | 37104905 | NA | 1.07E-05 | | 0.02722 | 0.005861 |
| rs301904 | 5 | 37111565 | CPLANE1 | 1.07E-05 | intron_variant | 0.02722 | 0.005861 |
| 5:37111995 :T:TA | 5 | 37111995 | NA | 1.07E-05 | NA | 0.02722 | 0.005861 |
| rs301894 | 5 | 37127778 | CPLANE1 | 1.07E-05 | intron_variant | 0.02722 | 0.005861 |
| rs301895 | 5 | 37128315 | CPLANE1 | 1.07E-05 | intron_variant | 0.02722 | 0.005861 |
| rs12110069 | 5 | 37160914 | CPLANE1 | 1.07E-05 | intron_variant | 0.02722 | 0.005861 |
| rs10056250 | 5 | 37171884 | CPLANE1 | 1.07E-05 | intron_variant | 0.02722 | 0.005861 |
| rs75589774 | 5 | 37182902 | CPLANE1 | 1.07E-05 | missense_variant | 0.02722 | 0.005861 |

[Table 3-26]

| SNP ID | chr | position | gene locus | p-value | SNP location in gene locus | BETA | SE |
|---|---|---|---|---|---|---|---|
| rs79298055 | 5 | 37202084 | CPLANE1 | 1.07E-05 | intron_variant | 0.02722 | 0.005861 |
| rs76607706 | 5 | 37892303 | NA | 8.52E-06 | | 0.02781 | 0.005915 |
| rs111622158 | 5 | 37928055 | NA | 2.89E-05 | | 0.0213 | 0.004851 |
| rs75661047 | 5 | 41337919 | PLCXD3 | 7.33E-06 | intron_variant | 0.02 | 0.00422 |
| rs189501062 | 5 | 41421703 | PLCXD3 | 7.33E-06 | intronvariant | 0.02 | 0.00422 |
| rs318065 | 5 | 41442044 | PLCXD3 | 1.80E-07 | intron_variant | 0.01662 | 0.002955 |
| rs78362058 | 5 | 41448308 | PLCXD3 | 1.80E-07 | intron_variant | 0.01662 | 0.002955 |
| rs78872736 | 5 | 41449972 | PLCXD3 | 1.80E-07 | intron_variant | 0.01662 | 0.002955 |
| rs79993711 | 5 | 41451447 | PLCXD3 | 1.80E-07 | intron_variant | 0.01662 | 0.002955 |
| rs3883955 | 5 | 41455054 | PLCXD3 | 1.80E-07 | intron_variant | 0.01662 | 0.002955 |
| rs191995881 | 5 | 41456607 | PLCXD3 | 1.80E-07 | intron_variant | 0.01662 | 0.002955 |
| rs77129467 | 5 | 41459002 | PLCXD3 | 1.80E-07 | intron_variant | 0.01662 | 0.002955 |
| rs117577517 | 5 | 41460797 | PLCXD3 | 1.80E-07 | intron_variant | 0.01662 | 0.002955 |
| rs149176711 | 5 | 41460944 | PLCXD3 | 1.80E-07 | intron_variant | 0.01662 | 0.002955 |
| rs145608729 | 5 | 41462819 | PLCXD3 | 1.80E-07 | intron_variant | 0.01662 | 0.002955 |
| rs142790831 | 5 | 41462961 | PLCXD3 | 1.80E-07 | intron_variant | 0.01662 | 0.002955 |
| rs80187604 | 5 | 41464479 | PLCXD3 | 1.80E-07 | intron_variant | 0.01662 | 0.002955 |
| rs118037167 | 5 | 41465566 | PLCXD3 | 1.80E-07 | intron_variant | 0.01662 | 0.002955 |
| rs116809573 | 5 | 41469306 | PLCXD3 | 1.80E-07 | intron_variant | 0.01662 | 0.002955 |
| rs117880348 | 5 | 41471050 | PLCXD3 | 1.80E-07 | intron_variant | 0.01662 | 0.002955 |
| rs74850462 | 5 | 41473868 | PLCXD3 | 1.80E-07 | intron_variant | 0.01662 | 0.002955 |
| rs75235442 | 5 | 41474166 | PLCXD3 | 1.80E-07 | intron_variant | 0.01662 | 0.002955 |
| rs117907903 | 5 | 41474535 | PLCXD3 | 1.80E-07 | intron_variant | 0.01662 | 0.002955 |
| rs117365372 | 5 | 41474536 | PLCXD3 | 1.80E-07 | intron_variant | 0.01662 | 0.002955 |
| rs318043 | 5 | 41474553 | PLCXD3 | 1.80E-07 | intron_variant | 0.01662 | 0.002955 |
| rs318042 | 5 | 41474575 | PLCXD3 | 1.80E-07 | intron_variant | 0.01662 | 0.002955 |
| rs139821901 | 5 | 41475221 | PLCXD3 | 1.80E-07 | intron_variant | 0.01662 | 0.002955 |
| rs185789756 | 5 | 41475660 | PLCXD3 | 1.80E-07 | intron_variant | 0.01662 | 0.002955 |
| rs189078681 | 5 | 41475661 | PLCXD3 | 1.80E-07 | intron_variant | 0.01662 | 0.002955 |
| rs149824778 | 5 | 41475846 | PLCXD3 | 1.80E-07 | intronvariant | 0.01662 | 0.002955 |
| rs6451577 | 5 | 41476751 | PLCXD3 | 1.80E-07 | intron_variant | 0.01662 | 0.002955 |
| rs148818052 | 5 | 41476865 | PLCXD3 | 1.80E-07 | intron_variant | 0.01662 | 0.002955 |
| rs117592865 | 5 | 41479099 | PLCXD3 | 4.05E-07 | intron_variant | 0.02041 | 0.003754 |
| rs78415819 | 5 | 41480451 | PLCXD3 | 4.05E-07 | intron_variant | 0.02041 | 0.003754 |
| rs185514305 | 5 | 41480194 | PLCXD3 | 4.05E-07 | intron_variant | 0.02041 | 0.003754 |
| rs149877177 | 5 | 41480973 | PLCXD3 | 4.05E-07 | intron_variant | 0.02041 | 0.003754 |

EP 4 324 922 A1

[Table 3-27]

| SNP ID | chr | position | gene locus | p-value | SNP location in gene locus | BETA | SE |
|---|---|---|---|---|---|---|---|
| rs144898661 | 5 | 41481008 | PLCXD3 | 4. 05E-07 | intron_variant | 0.02041 | 0. 003754 |
| 5:41481231:A:G | 5 | 41481231 | NA | 4.05E-07 | NA | 0.02041 | 0.003754 |
| rs592607 | 5 | 41482461 | PLCXD3 | 4.05E-07 | intron_variant | 0.02041 | 0.003754 |
| rs76025981 | 5 | 41484106 | PLCXD3 | 4.05E-07 | intron_variant | 0.02041 | 0.003754 |
| rs147796140 | 5 | 41485734 | PLCXD3 | 4.05E-07 | intron_variant | 0.02041 | 0.003754 |
| rs115209137 | 5 | 41485860 | PLCXD3 | 4.05E-07 | intron_variant | 0.02041 | 0.003754 |
| rs150658476 | 5 | 41487649 | PLCXD3 | 4.05E-07 | intron_variant | 0.02041 | 0.003754 |
| 5:41492084:G:A | 5 | 41492084 | NA | 4.05E-07 | NA | 0.02041 | 0.003754 |
| rs318047 | 5 | 41492737 | PLCXD3 | 4.05E-07 | intron_variant | 0.02041 | 0.003754 |
| rs193139535 | 5 | 41494163 | PLCXD3 | 4.05E-07 | intron_variant | 0.02041 | 0.003754 |
| 5:41496974:CTT:C | 5 | 41496974 | NA | 4.05E-07 | NA | 0.02041 | 0.003754 |
| 5:41499344:T:TA | 5 | 41499344 | NA | 4.05E-07 | NA | 0.02041 | 0.003754 |
| rs79200111 | 5 | 41503974 | PLCXD3 | 4.05E-07 | intron_variant | 0.02041 | 0.003754 |
| rs143005632 | 5 | 41505112 | PLCXD3 | 4.05E-07 | intron_variant | 0.02041 | 0.003754 |
| rs80129576 | 5 | 41507151 | PLCXD3 | 4.05E-07 | intron_variant | 0.02041 | 0.003754 |
| rs149750323 | 5 | 41508080 | PLCXD3 | 4.05E-07 | intron_variant | 0.02041 | 0.003754 |
| rs145378048 | 5 | 41508511 | PLCXD3 | 4.05E-07 | intron_variant | 0.02041 | 0.003754 |
| rs142480844 | 5 | 41509735 | PLCXD3 | 4.05E-07 | intron_variant | 0.02041 | 0.003754 |
| 5:41510809:C:T | 5 | 41510809 | NA | 4.05E-07 | NA | 0.02041 | 0.003754 |
| rs74732406 | 5 | 41511954 | NA | 4.05E-07 | | 0.02041 | 0.003754 |
| rs77991880 | 5 | 41515898 | NA | 4.05E-07 | | 0.02041 | 0.003754 |
| rs3863151 | 5 | 41518789 | NA | 7.33E-06 | | 0.02 | 0.00422 |
| rs140198935 | 5 | 41521169 | NA | 7.33E-06 | | 0.02 | 0.00422 |
| rs140097421 | 5 | 41525867 | NA | 7.33E-06 | | 0.02 | 0.00422 |
| rs78523327 | 5 | 41525908 | NA | 7.33E-06 | | 0.02 | 0.00422 |
| rs78310877 | 5 | 41528894 | NA | 7.33E-06 | | 0.02 | 0.00422 |
| rs79027993 | 5 | 41529340 | NA | 7.33E-D6 | | 0.02 | 0.00422 |
| rs149621750 | 5 | 41530150 | NA | 7.33E-06 | | 0.02 | 0.00422 |
| rs148809865 | 5 | 41530798 | NA | 7.33E-06 | | 0.02 | 0.00422 |
| rs142486112 | 5 | 41530854 | NA | 7.33E-06 | | 0.02 | 0.00422 |
| 5:41535859:G:A | 5 | 41535859 | NA | 7.33E-06 | NA | 0.02 | 0.00422 |
| rs79544594 | 5 | 41538064 | NA | 7.33E-06 | | 0.02 | 0.00422 |
| rs78787781 | 5 | 41538772 | NA | 7.33E-06 | | 0.02 | 0.00422 |
| rs76663349 | 5 | 41539637 | NA | 7.33E-06 | | 0.02 | 0.00422 |
| rs111541941 | 5 | 41541437 | NA | 7.33E-06 | | 0.02 | 0.00422 |
| rs77020012 | 5 | 41541725 | NA | 7.33E-D6 | | 0.02 | 0.00422 |

142

[Table 3-28]

| SNP ID | chr | position | gene locus | p-value | SNP location in gene locus | BETA | SE |
|---|---|---|---|---|---|---|---|
| rs79117365 | 5 | 41542625 | NA | 7.33E-D6 | | 0.02 | 0.00422 |
| 5:41548979 :G:A | 5 | 41548979 | NA | 7.33E-06 | NA | 0.02 | 0.00422 |
| 5:41566271 :G:A | 5 | 41566271 | NA | 7.33E-06 | NA | 0.02 | 0.00422 |
| rs142949110 | 5 | 41572513 | NA | 7.33E-06 | | 0.02 | 0.00422 |
| 5:41597151 :T:C | 5 | 41597151 | NA | 7.33E-06 | NA | 0.02 | 0.00422 |
| rs74919030 | 5 | 41611365 | NA | 7.33E-06 | | 0.02 | 0.00422 |
| rs137930663 | 5 | 41692962 | NA | 7.33E-06 | | 0.02 | 0.00422 |
| 5:41715811 :C:T | 5 | 41715811 | NA | 1.05E-07 | NA | 0.0269 | 0.00468 |
| rs139531587 | 5 | 42148442 | NA | 7.33E-06 | | 0.02 | 0.00422 |
| rs79035719 | 5 | 42217448 | NA | 7.33E-06 | | 0.02 | 0.00422 |
| rs185613690 | 5 | 42243590 | NA | 7.33E-06 | | 0.02 | 0.00422 |
| rs184276051 | 5 | 42257845 | NA | 7.33E-06 | | 0.02 | 0.00422 |
| 5:42354014:C:T | 5 | 42354014 | NA | 7.33E-06 | NA | 0.02 | 0.00422 |
| rs142622848 | 5 | 42379421 | NA | 7.33E-06 | | 0.02 | 0.00422 |
| rs12522729 | 5 | 60541282 | NA | 3.07E-05 | | 0.01523 | 0.003482 |
| rs74948200 | 5 | 60601182 | NA | 1.20E-07 | | 0.02295 | 0.004016 |
| rs12522321 | 5 | 60723105 | ZSWIM6 | 1.20E-07 | intron_variant | 0.02295 | 0.004016 |
| rs79053743 | 5 | 60829488 | ZSW1M6 | 1.12E-05 | intron_variant | 0.01743 | 0.003762 |
| rs1827387 | 5 | 81930239 | NA | 2.90E-05 | | 0.01554 | 0.003541 |
| rs74366119 | 5 | 88156454 | MEF2C | 1.60E-05 | intron_variant | 0.01704 | 0.003751 |
| 5:88795078 :G:GAA | 5 | 88795078 | NA | 4.13E-06 | NA | 0.01442 | 0.002953 |
| rs80106148 | 5 | 92325356 | NA | 1.91E-05 | | 0.01298 | 0.002885 |
| rs75089614 | 5 | 100358025 | NA | 1.07E-05 | | 0.02722 | 0.005861 |
| rs28641111 | 5 | 100366992 | NA | 1.07E-05 | | 0.02722 | 0.005861 |
| rs10072145 | 5 | 100395409 | NA | 1.07E-05 | | 0.02722 | 0.005861 |
| rs10057008 | 5 | 100395973 | NA | 1.07E-05 | | 0.02722 | 0.005861 |
| rs10055504 | 5 | 100397154 | NA | 1.07E-05 | | 0.02722 | 0.005861 |
| rs115735320 | 5 | 100399760 | NA | 1.07E-05 | | 0.02722 | 0.005861 |
| rs1423681 | 5 | 100404890 | NA | 1.07E-05 | | 0.02722 | 0.005861 |
| 5:100432544 :C:CAT | 5 | 100432544 | NA | 1.07E-05 | NA | 0.02722 | 0.005861 |
| rs13356491 | 5 | 112378789 | MCC | 4.75E-05 | intron_variant | 0.008252 | 0.001937 |
| rs6865320 | 5 | 112388791 | MCC | 2.43E-05 | intron_variant | 0.007226 | 0.001629 |
| 5:124492243:T:C | 5 | 124492243 | NA | 1.04E-07 | NA | 0.03229 | 0.005617 |

(continued)

| SNP ID | chr | position | gene locus | p-value | SNP location in gene locus | BETA | SE |
|---|---|---|---|---|---|---|---|
| rs78855508 | 5 | 124557431 | NA | 1.04E-07 | | 0.03229 | 0.005617 |
| rs73326798 | 5 | 125390012 | NA | 5.93E-07 | | 0.01463 | 0.002736 |
| rs4373275 | 5 | 125393103 | NA | NA | | NA | NA |

[Table 3-29]

| SNP ID | chr | position | gene locus | p-value | SNP location in gene locus | BETA | SE |
|---|---|---|---|---|---|---|---|
| 5:125393103 :T:G | 5 | 125393103 | NA | 2.39E-05 | NA | 0.01102 | 0.002482 |
| rs138975964 | 5 | 125393851 | NA | 9.54E-07 | | 0.01368 | 0.002614 |
| rs60924028 | 5 | 125399302 | NA | 9.54E-07 | | 0.01368 | 0.002614 |
| rs57885244 | 5 | 125399376 | NA | 9.54E-07 | | 0.01368 | 0.002614 |
| rs60069077 | 5 | 125399447 | NA | 9.54E-07 | | 0.01368 | 0.002614 |
| rs57317805 | 5 | 125399676 | NA | 9.54E-07 | | 0.01368 | 0.002614 |
| rs57908529 | 5 | 125399679 | NA | 9.54E-07 | | 0.01368 | 0.002614 |
| rs60950409 | 5 | 125400706 | NA | 9.54E-07 | | 0.01368 | 0.002614 |
| rs73328735 | 5 | 125401914 | NA | 9.54E-07 | | 0.01368 | 0.002614 |
| rs111944066 | 5 | 125402054 | NA | 9.54E-07 | | 0.01368 | 0.002614 |
| rs73328738 | 5 | 125402365 | NA | 9.54E-07 | | 0.01368 | 0.002614 |
| rs58002426 | 5 | 125402709 | NA | 9.54E-07 | | 0.01368 | 0.002614 |
| rs61572014 | 5 | 125402753 | NA | 9.54E-07 | | 0.01368 | 0.002614 |
| rs61376002 | 5 | 125402760 | NA | 9.54E-07 | | 0.01368 | 0.002614 |
| rs57762059 | 5 | 125403219 | NA | 9.54E-07 | | 0.01368 | 0.002614 |
| rs57282782 | 5 | 125403419 | NA | 9.54E-07 | | 0.01368 | 0.002614 |
| rs60443581 | 5 | 125403795 | NA | 9.54E-07 | | 0.01368 | 0.002614 |
| rs73328747 | 5 | 125404393 | NA | 9.54E-07 | | 0.01368 | 0.002614 |
| rs73328752 | 5 | 125404704 | NA | 9.54E-07 | | 0.01368 | 0.002614 |
| rs73328754 | 5 | 125405005 | NA | 9.54E-07 | | 0.01368 | 0.002614 |
| rs73328756 | 5 | 125405014 | NA | 9.54E-07 | | 0.01368 | 0.002614 |
| rs73328762 | 5 | 125406249 | NA | 9.54E-07 | | 0.01368 | 0.002614 |
| rs28867125 | 5 | 125408077 | NA | 9.54E-07 | | 0.01368 | 0.002614 |
| rs141339888 | 5 | 125408665 | NA | 9.54E-07 | | 0.01368 | 0.002614 |
| rs73328767 | 5 | 125408969 | NA | 9.54E-07 | | 0.01368 | 0.002614 |
| rs74968976 | 5 | 125410229 | NA | 9.54E-07 | | 0.01368 | 0.002614 |
| rs6869320 | 5 | 125410380 | NA | 9.54E-07 | | 0.01368 | 0.002614 |
| rs6870176 | 5 | 125410856 | NA | 9.54E-07 | | 0.01368 | 0.002614 |
| rs77250321 | 5 | 125412256 | NA | 9.54E-07 | | 0.01368 | 0.002614 |

(continued)

| SNP ID | chr | position | gene locus | p-value | SNP location in gene locus | BETA | SE |
|---|---|---|---|---|---|---|---|
| rs73328774 | 5 | 125412457 | NA | 9.54E-07 | | 0.01368 | 0.002614 |
| 5:143430342 : GTTGT:G | 5 | 143430342 | NA | 5.59E-06 | NA | 0.02836 | 0.005899 |
| rs76612072 | 5 | 147183847 | NA | 9.20E-06 | | 0.0227 | 0.004847 |
| rs11954595 | 5 | 147194146 | NA | 1.11E-05 | | 0.01771 | 0.003821 |
| rs78681108 | 5 | 147232262 | NA | 3.32E-05 | | 0.01862 | 0.004277 |
| rs116969221 | 5 | 151269893 | GLRA1 | 3.22E-05 | intron_variant | 0.02682 | 0.00615 |
| 5:153395900 : GA:G | 5 | 153395900 | NA | 2.86E-05 | NA | 0.01908 | 0.004345 |

[Table 3-30]

| SNP ID | chr | position | gene locus | p-value | SNP location in gene locus | BETA | SE |
|---|---|---|---|---|---|---|---|
| 5:153395903 : A:G | 5 | 153395903 | NA | 2.86E-05 | NA | 0.01908 | 0.004345 |
| rs141072734 | 5 | 163003513 | NA | 2.50E-05 | | 0.02626 | 0.005931 |
| rs79560759 | 5 | 163021709 | NA | 2.50E-05 | | 0.02626 | 0.005931 |
| rs60283376 | 5 | 166904236 | TENM2 | 2.19E-06 | intron_variant | 0.02908 | 0.005774 |
| rs77246061 | 5 | 166906243 | TENM2 | 2.19E-06 | intron_variant | 0.02908 | 0.005774 |
| rs79999983 | 5 | 166910600 | TENM2 | 2.19E-06 | intron_variant | 0.02908 | 0.005774 |
| rs79072342 | 5 | 166910635 | TENM2 | 2.19E-06 | intron_variant | 0.02908 | 0.005774 |
| rs117190309 | 5 | 166910664 | TENM2 | 2.19E-06 | intron_variant | 0.02908 | 0.005774 |
| rs140266055 | 5 | 166910826 | TENM2 | 2.19E-06 | intron_variant | 0.02908 | 0.005774 |
| rs79374298 | 5 | 166911354 | TENM2 | 2.19E-06 | intron_variant | 0.02908 | 0.005774 |
| rs117911019 | 5 | 166913316 | TENM2 | 2.19E-06 | intron_variant | 0.02908 | 0.005774 |
| rs59932109 | 5 | 166919170 | TENM2 | 2.19E-06 | intron_variant | 0.02908 | 0.005774 |
| rs1445795 | 5 | 172839988 | NA | 3.59E-05 | | 0.004849 | 0.001119 |
| rs10463029 | 5 | 172842872 | NA | 2.37E-05 | | 0.004894 | 0.001102 |
| rs11134795 | 5 | 172843707 | NA | 1.99E-05 | | 0.004887 | 0.001089 |

(continued)

| SNP ID | chr | position | gene locus | p-value | SNP location in gene locus | BETA | SE |
|---|---|---|---|---|---|---|---|
| rs11134796 | 5 | 172844177 | NA | 2.18E-05 | | 0.00489 | 0.001096 |
| rs13357412 | 5 | 172844784 | NA | 2.18E-05 | | 0.00489 | 0.001096 |
| rs4867711 | 5 | 172847035 | NA | 2.18E-05 | | 0.00489 | 0.001096 |
| rs115721274 | 5 | 172858622 | NA | 2.72E-07 | | 0.03139 | 0.005677 |
| rs190979351 | 5 | 173034050 | NA | 3.51E-05 | | 0.02589 | 0.005965 |
| rs75505838 | 5 | 173111885 | NA | 3.51E-Q5 | | 0.02589 | 0.005965 |
| rs117041609 | 5 | 173234139 | NA | 1.72E-05 | | 0.02707 | 0.005982 |
| rs75159910 | 5 | 173282893 | NA | 1.72E-05 | | 0.02707 | 0.005982 |
| rs149874863 | 5 | 173855117 | NA | 1.09E-05 | | 0.01949 | 0.0042 |
| rs72811273 | 5 | 176225113 | **NA** | 4.53E-05 | | 0.01095 | 0.002562 |
| rs7720553 | 5 | 179623637 | RASGEF1C | 2.50E-05 | intron_variant | 0.02626 | 0.005931 |
| rs60316563 | 6 | 6368046 | NA | 2.50E-05 | | 0.02626 | 0.005931 |
| rs57686849 | 6 | 6368154 | NA | 2.50E-05 | | 0.02626 | 0.005931 |
| rs1418fi4440 | 6 | 8237754 | NA | 2.64E-05 | | 0.01442 | 0.003266 |
| rs9349929 | 6 | 14046145 | NA | 2.53E-05 | | 0.006224 | 0.001407 |
| rs6915882 | 6 | 14046411 | NA | 2.53E-05 | | 0.006224 | 0.001407 |
| rs2840257 | 6 | 14048680 | NA | 2.88E-06 | | 0.007227 | 0.001454 |
| rs909709 | 6 | 14049882 | NA | 9.73E-06 | | 0.006845 | 0.001466 |
| 6:14051468 : TAC:T | 6 | 14051468 | NA | 9.73E-06 | NA | 0.006845 | 0.001466 |
| 6:14051468 : TACAC:T | 6 | 14051468 | NA | NA | NA | NA | NA |
| rs1928638 | 6 | 14057157 | NA | 2.88E-06 | | 0.007227 | 0.001454 |

[Table 3-31]

| SNP ID | chr | position | gene locus | p-value | SNP location in gene locus | BETA | SE |
|---|---|---|---|---|---|---|---|
| rs1928fi39 | 6 | 14057161 | NA | 2.88E-06 | | 0.007227 | 0.001454 |
| rs57463781 | 6 | 14057622 | NA | 2.88E-06 | | 0.007227 | 0.001454 |
| rs12208529 | 6 | 14063790 | NA | 9.73E-06 | | 0.006845 | 0.001466 |
| rs9476476 | 6 | 14064273 | NA | 6.75E-06 | | 0.007049 | 0.001481 |
| rs72836088 | 6 | 19842560 | NA | 3.55E-05 | | 0.01669 | 0.00385 |
| rs115079901 | 6 | 29558364 | NA | 3.22E-05 | NA | 0.02682 | 0.00615 |
| rs200911534 | 6 | 30741569 | NA | 4.71E-07 | | 0.03074 | 0.00569 |
| 6:30744985:G:T | 6 | 30744985 | NA | 4.71E-07 | NA | 0.03074 | 0.00569 |
| rs191987001 | 6 | 30775579 | NA | 3.61E-05 | NA | 0.02121 | 0.004897 |
| rs2499716 | 6 | 34073338 | GRM4 | 4.71E-07 | intron_variant | 0.03074 | 0.00569 |
| rs1873248 | 6 | 34073344 | GRM4 | 4.71E-07 | intron_variant | 0.03074 | 0.00569 |
| rs139096142 | 6 | 44147011 | CAPN11 | 2.50E-05 | intron_variant | 0.02626 | 0.005931 |
| rs78401606 | 6 | 70051582 | ADGRB3 | 2.72E-07 | intron_variant | 0.03139 | 0.005677 |
| rs144698880 | 6 | 70207289 | NA | 2.46E-05 | | 0.01413 | 0.003188 |
| 6:70210202:G:A | 6 | 70210202 | NA | 2.46E-05 | NA | 0.01413 | 0.003188 |
| rs11757257 | 6 | 70217452 | NA | 2.46E-05 | | 0.01413 | 0.003188 |
| rs13205898 | 6 | 70271095 | NA | 2.46E-05 | | 0.01413 | 0.003188 |
| rs79873641 | 6 | 71389721 | SMAP1 | 4.44E-05 | intron_variant | 0.01086 | 0.002539 |
| rs145666404 | 6 | 71513933 | SMAP1 | 4.82E-05 | intron_variant | 0.01053 | 0.002474 |
| rs78049725 | 6 | 75836785 | COL12A1 | 1.07E-05 | intron_variant | 0.02722 | 0.005861 |
| rs77854307 | 6 | 82472695 | NA | 2.72E-07 | | 0.03139 | 0.005677 |
| rs79069596 | 6 | 82806731 | NA | 3.24E-05 | | 0.01162 | 0.002666 |
| rs77125903 | 6 | 82807806 | NA | 3.24E-05 | | 0.01162 | 0.002666 |
| rs11962648 | 6 | 82824374 | NA | 1.07E-05 | | 0.01756 | 0.00378 |
| rs6914737 | 6 | 82824575 | NA | 4.10E-06 | | 0.01682 | 0.003444 |
| rs201137924 | 6 | 82824635 | NA | 1.07E-05 | | 0.01756 | 0.00378 |
| rs77793849 | 6 | 82825638 | NA | 4.10E-06 | | 0.01682 | 0.003444 |
| rs75421360 | 6 | 82826545 | NA | 4.10E-06 | | 0.01682 | 0.003444 |
| rs74572694 | 6 | 82831444 | NA | 1.07E-05 | | 0.01756 | 0.00378 |
| rs11965072 | 6 | 82832842 | NA | 1.07E-05 | | 0.01756 | 0.00378 |
| rs11755112 | 6 | 82833728 | NA | 1.07E-05 | | 0.01756 | 0.00378 |
| rs36156078 | 6 | 82838594 | NA | 4.10E-06 | | 0.01682 | 0.003444 |
| rs2152842 | 6 | 82841134 | NA | 4.10E-06 | | 0.01682 | 0.003444 |
| rs11967918 | 6 | 82842822 | NA | 4.10E-06 | | 0.01682 | 0.003444 |
| rs10943858 | 6 | 82845197 | NA | 1.07E-05 | | 0.01756 | 0.00378 |
| rs79009267 | 6 | 82846023 | NA | 1.07E-05 | | 0.01756 | 0.00378 |

[Table 3-32]

| SNP ID | chr | position | gene locus | p-value | SNP location in gene locus | BETA | SE |
|---|---|---|---|---|---|---|---|
| rs11961436 | 6 | 82847140 | NA | 1.07E-05 | | 0.01756 | 0.00378 |
| rs11752161 | 6 | 82848130 | NA | 4.10E-06 | | 0.01682 | 0.003444 |
| rs11752130 | 6 | 82848159 | NA | 4.10E-06 | | 0.01682 | 0.003444 |
| rs77937699 | 6 | 82880897 | IBTK | 1.35E-05 | 3_prime_UTR_variant | 0.02259 | 0.004924 |
| rs201520516 | 6 | 82965363 | NA | 1.04E-07 | | 0.03229 | 0.005617 |
| rs76325080 | 6 | 84248518 | NA | 2.19E-06 | | 0.02908 | 0.005774 |
| rs78628697 | 6 | 86590415 | NA | 3.13E-D6 | | 0.01456 | 0.002942 |
| rs58725789 | 6 | 86600143 | NA | 3.13E-D6 | | 0.01456 | 0.002942 |
| 6:91762389 :C:A | 6 | 91762389 | NA | 2.72E-07 | NA | 0.03139 | 0.005677 |
| rs112761794 | 6 | 105688813 | NA | 1.66E-06 | | 0.02426 | 0.004753 |
| rs141339608 | 6 | 105688876 | NA | 1.66E-06 | NA | 0.02426 | 0.004753 |
| rs72936201 | 6 | 105691598 | NA | 1.66E-06 | | 0.02426 | 0.004753 |
| rs111427560 | 6 | 105692447 | NA | 1.66E-06 | | 0.02426 | 0.004753 |
| rs183560286 | 6 | 105692807 | NA | 1.66E-06 | NA | 0.02426 | 0.004753 |
| rs142938303 | 6 | 105695489 | NA | 1.66E-06 | | 0.02426 | 0.004753 |
| 6:105697055 :G:T | 6 | 105697055 | NA | 1.66E-06 | NA | 0.02426 | 0.004753 |
| rs35695447 | 6 | 105699862 | NA | 1.66E-06 | | 0.02426 | 0.004753 |
| rs111612241 | 6 | 105703217 | NA | 1.66E-06 | | 0.02426 | 0.004753 |
| rs17065682 | 6 | 105704149 | NA | 1.66E-06 | | 0.02426 | 0.004753 |
| rs17065694 | 6 | 105704658 | NA | 1.66E-06 | | 0.02426 | 0.004753 |
| rs10499042 | 6 | 105706581 | NA | 1.66E-06 | | 0.02426 | 0.004753 |
| rs111857724 | 6 | 105707260 | NA | 1.66E-06 | | b.02426 | 0.004753 |
| rs113487540 | 6 | 105707498 | NA | 1.66E-06 | | 0.02426 | 0.004753 |
| rs72938014 | 6 | 105707927 | NA | 1.66E-06 | | 0.02426 | 0.004753 |
| rs17054938 | 6 | 105709113 | NA | 1.66E-06 | | 0.02426 | 0.004753 |
| rs72938025 | 6 | 105713764 | NA | 1.66E-06 | | 0.02426 | 0.004753 |
| rs75718226 | 6 | 105715441 | NA | 1.66E-06 | | 0.02426 | 0.004753 |
| rs72938030 | 6 | 105716807 | NA | 1.66E-06 | | 0.02426 | 0.004753 |
| rs72938031 | 6 | 105716902 | NA | 1.66E-06 | | 0.02426 | 0.004753 |
| rs72938040 | 6 | 105721926 | PREP | 1.66E-06 | 3_prime_UTR_variant | 0.02426 | 0.004753 |
| rs17054949 | 6 | 105721969 | PREP | 1.66E-06 | 3_prime_UTR_variant | 0.02426 | 0.004753 |
| rs111772455 | 6 | 105722560 | PREP | 1.07E-05 | 3_prime_UTR_variant | 0.02722 | 0.005861 |
| rs17467544 | 6 | 105722943 | PREP | 1.66E-06 | 3_prime_UTR_variant | 0.02426 | 0.004753 |
| rs41285466 | 6 | 105726874 | PREP | 1.66E-06 | intron_variant | 0.02426 | 0.004753 |
| rs72944139 | 6 | 105727873 | PREP | 1.66E-06 | intron_variant | 0.02426 | 0.004753 |
| rs12944142 | 6 | 105728191 | PREP | 1.66E-06 | intron_variant | 0.02426 | 0.004753 |

[Table 3-33]

| SNP ID | chr | position | gene locus | p-value | SNP location in gene locus | BETA | SE |
|---|---|---|---|---|---|---|---|
| rs77676511 | 6 | 105729054 | PREP | 1.66E-06 | intron_variant | 0.02426 | 0.004753 |
| rs2236285 | 6 | 105730619 | PREP | 1.66E-06 | intron_variant | 0.02426 | 0.004753 |
| rs3800001 | 6 | 105735808 | PREP | 1.66E-06 | intron_variant | 0.02426 | 0.004753 |
| rs17546638 | 6 | 105738261 | PREP | 1.66E-06 | intron_variant | 0.02426 | 0.004753 |
| rs17546735 | 6 | 105741586 | PREP | 1.66E-06 | intron_variant | 0.02426 | 0.004753 |
| rs17468237 | 6 | 105742660 | PREP | 1.66E-06 | intron_variant | 0.02426 | 0.004753 |
| rs17068024 | 6 | 107484367 | PDSS2 | 3.49E-06 | intron_variant | 0.01268 | 0.002575 |
| rs145412027 | 6 | 107493416 | PDSS2 | 3.49E-06 | intron_variant | 0.01268 | 0.002575 |
| rs75692260 | 6 | 107498607 | PDSS2 | 3.49E-06 | intron_variant | 0.01268 | 0.002575 |
| rs147212820 | 6 | 107504762 | PDSS2 | 3.49E-06 | intron_variant | 0.01268 | 0.002575 |
| rs79713062 | 6 | 110875473 | NA | 6.77E-06 | | 0.02023 | 0.00425 |
| rs12195889 | 6 | 129623880 | LAMA2 | 2.80E-05 | intron_variant | 0.01535 | 0.003491 |
| rs2451682 | 6 | 129625432 | LAMA2 | 3.41E-06 | intron_variant | 0.01832 | 0.003717 |
| rs12200836 | 6 | 129640745 | LAMA2 | 3.41E-06 | intron_variant | 0.01832 | 0.003717 |
| rs12200538 | 6 | 129646004 | LAMA2 | 3.41E-06 | intronvariant | 0.01832 | 0.003717 |
| rs2451679 | 6 | 129660222 | LAMA2 | 2.80E-05 | intron_variant | 0.01535 | 0.003491 |
| rs77026545 | 6 | 137559310 | NA | 1.04E-07 | | 0.03229 | 0.005617 |
| rs117451378 | 6 | 137570062 | NA | 1.04E-07 | | 0.03229 | 0.005617 |
| rs57516822 | 6 | 137739295 | NA | 1.90E-06 | | 0.02435 | 0.004803 |
| rs59391960 | 6 | 137739389 | NA | 1.90E-06 | | 0.02435 | 0.004803 |
| rs77091814 | 6 | 137742839 | NA | 3.51E-05 | | 0.02589 | 0.005965 |
| rs73566001 | 6 | 137744363 | NA | 1.90E-06 | | 0.02435 | 0.004803 |
| rs7769446 | 6 | 137745965 | NA | 1.90E-06 | | 0.02435 | 0.004803 |
| rs71811814 | 6 | 137746238 | NA | 1.90E-06 | NA | 0.02435 | 0.004803 |
| rs6917588 | 6 | 137746860 | NA | 1.90E-06 | | 0.02435 | 0.004803 |
| rs1335302 | 6 | 141637219 | NA | 4.43E-05 | | 0.008615 | 0.002014 |
| rs1335308 | 6 | 141645736 | NA | 2.16E-05 | | 0.008966 | 0.002008 |
| rs4270804 | 6 | 141652711 | NA | 1.58E-05 | | 0.007999 | 0.00176 |
| rs9376607 | 6 | 141661460 | NA | 1.50E-06 | | 0.009565 | 0.001865 |
| rs9403288 | 6 | 141672901 | NA | 5.47E-07 | | 0.01016 | 0.001894 |
| rs9399363 | 6 | 141682404 | NA | 5.47E-07 | | 0.01016 | 0.001894 |
| rs9373305 | 6 | 141683547 | NA | 1.53E-05 | | 0.008393 | 0.001843 |
| rs11760135 | 6 | 141685630 | NA | 1.53E-05 | | 0.008393 | 0.001843 |
| 6:141689730:AT:A | 6 | 141689730 | NA | 1.41E-05 | NA | 0.009813 | 0.002145 |
| rs9376611 | 6 | 141692633 | NA | 1.41E-05 | | 0.009813 | 0.002145 |
| rs11754010 | 6 | 141700708 | NA | 1.41E-05 | | 0.009813 | 0.002145 |

[Table 3-34]

| SNP ID | chr | position | gene locus | p-value | SNP location in gene locus | BETA | SE |
|---|---|---|---|---|---|---|---|
| rs55968168 | 6 | 141703453 | NA | 7.83E-06 | | 0.01037 | 0.002196 |
| rs9403300 | 6 | 141706902 | NA | 7.83E-06 | | 0.01037 | 0.002196 |
| 6:141712104 : G:A | 6 | 141712104 | NA | 7.83E-06 | NA | 0.01037 | 0.002196 |
| rs73779399 | 6 | 145196065 | NA | 1.07E-05 | | 0.02722 | 0.005861 |
| rs55645496 | 6 | 145197134 | NA | 1.07E-05 | | 0.02722 | 0.005861 |
| rs73779401 | 6 | 145199490 | NA | 1.07E-05 | | 0.02722 | 0.005861 |
| rs73781203 | 6 | 145200275 | NA | 1.07E-05 | | 0.02722 | 0.005861 |
| rs73781205 | 6 | 145201088 | NA | 1.07E-05 | | 0.02722 | 0.005861 |
| rs73781206 | 6 | 145201247 | NA | 1.07E-05 | | 0.02722 | 0.005861 |
| rs6940222 | 6 | 145203608 | NA | 1.07E-05 | | 0.02722 | 0.005861 |
| rs73781208 | 6 | 145205435 | NA | 1.07E-05 | | 0.02722 | 0.005861 |
| rs57049867 | 6 | 145205680 | NA | 1.07E-05 | | 0.02722 | 0.005861 |
| rs73781214 | 6 | 145210203 | NA | 1.07E-05 | | 0.02722 | 0.005861 |
| rs62436288 | 6 | 147952729 | SAMD5 | 3.40E-05 | intron_variant | 0.02137 | 0.004916 |
| rs2064333 | 6 | 147953574 | SAMD5 | 3.40E-05 | intron_variant | 0.02137 | 0.004916 |
| rs17388532 | 6 | 147956771 | SAMD5 | 3.40E-05 | intron_variant | 0.02137 | 0.004916 |
| rs7753842 | 6 | 148553550 | NA | 2.66E-05 | | 0.008976 | 0.002035 |
| rs1871921 | 6 | 149221294 | UST | 2.91E-05 | intron_variant | 0.008947 | 0.002039 |
| rs17665064 | 6 | 149226781 | UST | 2.91E-05 | intron_variant | 0.008947 | 0.002039 |
| rs7760563 | 6 | 149232496 | UST | 3.17E-05 | intron_variant | 0.006095 | 0.001396 |
| rs72994230 | 6 | 149236366 | UST | 4.57E-05 | intronvariant | 0.007373 | 0.001727 |
| rs112183061 | 6 | 149236666 | UST | 4.57E-05 | intron_variant | 0.007373 | 0.001727 |
| 6:149269058 : G:GA | 6 | 149269058 | NA | 2.94E-05 | NA | 0.007023 | 0.001601 |
| 6:149269058 : G:GAA | 6 | 149269058 | NA | NA | NA | NA | NA |
| rs117816679 | 6 | 150790648 | NA | 8.52E-06 | | 0.02781 | 0.005915 |
| rs147175280 | 6 | 150793218 | NA | 8.52E-06 | | 0.02781 | 0.005915 |
| rs146914832 | 6 | 150795155 | NA | 8.52E-06 | | 0.02781 | 0.005915 |
| 6:150796988: G:T | 6 | 150796988 | NA | 8.52E-06 | NA | 0.02781 | 0.005915 |
| rs148080540 | 6 | 150806519 | NA | 8.52E-06 | | 0.02781 | 0.005915 |
| rs117959775 | 6 | 150809802 | NA | 8.52E-06 | | 0.02781 | 0.005915 |
| rs111474682 | 6 | 150811773 | NA | 8.52E-06 | | 0.02781 | 0.005915 |
| rs77886049 | 6 | 156030889 | NA | 3.36E-05 | | 0.01174 | 0.002699 |
| rs9397273 | 6 | 156248029 | NA | 3.62E-05 | | 0.005812 | 0.001342 |
| rs4370375 | 6 | 156250340 | NA | 2.79E-05 | | 0.005911 | 0.001344 |

(continued)

| SNP ID | chr | position | gene locus | p-value | SNP location in gene locus | BETA | SE |
|--------|-----|----------|------------|---------|---------------------------|------|-----|
| rs7756499 | 6 | 156250986 | NA | 2.79E-05 | | 0.005911 | 0.001344 |
| rs9384398 | 6 | 156252277 | NA | 3.62E-05 | | 0.005812 | 0.001342 |

[Table 3-35]

| SNP ID | chr | position | gene locus | p-value | SNP location in gene locus | BETA | SE |
|--------|-----|----------|------------|---------|---------------------------|------|-----|
| rs9397274 | 6 | 156253640 | NA | 2.20E-05 | | 0.006002 | 0.001346 |
| rs7740419 | 6 | 156255416 | NA | 2.20E-05 | | 0.006002 | 0.001346 |
| rs17086886 | 6 | 156261635 | NA | 3.62E-05 | | 0.005812 | 0.001342 |
| rs74781715 | 6 | 156271293 | NA | 2.87E-05 | | 0.005903 | 0.001344 |
| rs9397864 | 6 | 156271539 | NA | 2.87E-05 | | 0.005903 | 0.001344 |
| rs4259259 | 6 | 156272383 | NA | 3.62E-05 | | 0.005812 | 0.001342 |
| rs9397865 | 6 | 156272671 | NA | 3.62E-05 | | 0.005812 | 0.001342 |
| rs9397866 | 6 | 156272681 | NA | 3.62E-05 | | 0.005812 | 0.001342 |
| rs12111075 | 6 | 162304205 | PRKN | 7.76E-06 | intron_variant | -0.007184 | 0.00152 |
| rs12110765 | 6 | 162304245 | PRKN | 1.67E-05 | intron_variant | -0.006751 | 0.00149 |
| rs58436306 | 6 | 170758245 | NA | 2.19E-06 | | 0.02908 | 0.005774 |
| rs7750857 | 6 | 170760133 | NA | 2.19E-06 | | 0.02908 | 0.005774 |
| rs140838906 | 6 | 170761583 | NA | 2.19E-06 | | 0.02908 | 0.005774 |
| 7:4230856 :G:A | 7 | 4230856 | NA | 1.72E-05 | NA | 0.02707 | 0.005982 |
| rs201457651 | 7 | 9092066 | NA | 3.08E-05 | | 0.01858 | 0.004248 |
| rs148328240 | 7 | 9093559 | NA | 3.08E-05 | | 0.01858 | 0.004248 |
| rs10265621 | 7 | 9099399 | NA | 3.08E-05 | | 0.01858 | 0.004248 |
| rs76355504 | 7 | 9313494 | NA | 3.75E-05 | | 0.01302 | 0.003012 |

(continued)

| SNP ID | chr | position | gene locus | p-value | SNP location in gene locus | BETA | SE |
|---|---|---|---|---|---|---|---|
| rs58749480 | 7 | 13292034 | NA | 4.71E-07 | | 0.03074 | 0.00569 |
| rs139024590 | 7 | 17010996 | NA | 2.19E-06 | NA | 0.02908 | 0.005774 |
| rs11980801 | 7 | 22290160 | RAPGEF5 | 2.68E-05 | intron_variant | 0.01056 | 0.002396 |
| rs2686469 | 7 | 22299059 | RAPGEF5 | 1.87E-05 | intron_variant | 0.01111 | 0.002468 |
| 7:22888700 :CAA: CTTAA | 7 | 22888700 | NA | 1.72E-05 | NA | 0.02707 | 0.005982 |
| rs182887339 | 7 | 22895543 | NA | 1.72E-05 | | 0.02707 | 0.005982 |
| rs80009912 | 7 | 22920084 | NA | 1.72E-05 | | 0.02707 | 0.005982 |
| rs147497510 | 7 | 28699881 | CREB5 | 1.72E-05 | intron_variant | 0.02707 | 0.005982 |
| rs73685962 | 7 | 30292368 | NA | 1.76E-05 | | 0.01132 | 0.002506 |
| rs6958640 | 7 | 30293612 | NA | 1.76E-05 | | 0.01132 | 0.002506 |
| rs114684317 | 7 | 30294944 | NA | 1.76E-05 | | 0.01132 | 0.002506 |
| rs149057499 | 7 | 30296625 | NA | 1.76E-05 | | 0.01132 | 0.002506 |
| rs6970146 | 7 | 30297969 | NA | 1.76E-05 | | 0.01132 | 0.002506 |
| rs77413551 | 7 | 30301817 | NA | 1.76E-05 | | 0.01132 | 0.002506 |
| rs201581869 | 7 | 30303728 | NA | 1.76E-05 | | 0.01132 | 0.002506 |
| 7:30303731 : GCACTCCTCCC:G | 7 | 30303731 | NA | 1.76E-05 | NA | 0.01132 | 0.002506 |
| 7:30303742 :GC:G | 7 | 30303742 | NA | 1.76E-05 | NA | 0.01132 | 0.002506 |

[Table 3-36]

| SNP ID | chr | position | gene locus | p-value | SNP location in gene locus | BETA | SE |
|---|---|---|---|---|---|---|---|
| rs149622189 | 7 | 30307465 | NA | 1.76E-05 | | 0.01132 | 0.002506 |
| rs200550125 | 7 | 30311765 | NA | 1.76E-05 | | 0.01132 | 0.002506 |
| 7:30319553:TA:T | 7 | 30319553 | NA | 1.76E-05 | NA | 0.01132 | 0.002506 |
| rs114645450 | 7 | 30321650 | NA | 1.76E-05 | | 0.01132 | 0.002506 |
| rs79699598 | 7 | 30322482 | NA | 1.76E-05 | | 0.01132 | 0.002506 |

(continued)

| SNP ID | chr | position | gene locus | p-value | SNP location in gene locus | BETA | SE |
|---|---|---|---|---|---|---|---|
| 7:30324192:C:CG | 7 | 30324192 | NA | 1.76E-05 | NA | 0.01132 | 0.002506 |
| rs143478611 | 7 | 30327096 | ZNRF2 | 1.76E-05 | intron_variant | 0.01132 | 0.002506 |
| rs6970041 | 7 | 30332914 | ZNRF2 | 1.76E-05 | intron_variant | 0.01132 | 0.002506 |
| rs17158839 | 7 | 30335403 | ZNRF2 | 1.76E-05 | intron_variant | 0.01132 | 0.002506 |
| rs148707872 | 7 | 30336156 | ZNRF2 | 1.76E-05 | intron_variant | 0.01132 | 0.002506 |
| rs115884980 | 7 | 30336503 | ZNRF2 | 1.76E-05 | intron_variant | 0.01132 | 0.002506 |
| rs140607084 | 7 | 30336572 | ZNRF2 | 1.76E-05 | intron_variant | 0.01132 | 0.002506 |
| rs147812016 | 7 | 30336672 | ZNRF2 | 1.76E-05 | intron_variant | 0.01132 | 0.002506 |
| rs116610215 | 7 | 30338896 | ZNRF2 | 1.76E-05 | intron_variant | 0.01132 | 0.002506 |
| rs74501594 | 7 | 30339220 | ZNRF2 | 1.76E-05 | intron_variant | 0.01132 | 0.002506 |
| rs6968097 | 7 | 30344956 | ZNRF2 | 4.00E-05 | intron_variant | 0.01048 | 0.002435 |
| 7:30346948:T:G | 7 | 30346948 | NA | 1.76E-05 | NA | 0.01132 | 0.002506 |
| rs8180762 | 7 | 30348387 | ZNRF2 | 1.76E-05 | intron_variant | 0.01132 | 0.002506 |
| rs73685995 | 7 | 30349511 | ZNRF2 | 4.00E-05 | intron_variant | 0.01048 | 0.002435 |
| rs9639614 | 7 | 30350328 | ZNRF2 | 1.76E-05 | intron_variant | 0.01132 | 0.002506 |
| rs7804225 | 7 | 30350755 | ZNRF2 | 1.76E-05 | intron variant | 0.01132 | 0.002506 |
| rs34458739 | 7 | 30351379 | ZNRF2 | 4.00E-05 | intron_variant | 0.01048 | 0.002435 |
| 7:30355351 :G:C | 7 | 30355351 | NA | 1.76E-05 | NA | 0.01132 | 0. 002506 |
| rs112527213 | 7 | 30358471 | ZNRF2 | 1.76E-05 | intron_variant | 0.01132 | 0.002506 |
| rs6959139 | 7 | 30360832 | ZNRF2 | 1.76E-05 | intron variant | 0 01132 | 0.002506 |
| rs78301492 | 7 | 30362276 | ZNRF2 | 1.76E-05 | intron_variant | 0.01132 | 0.002506 |
| rs140099650 | 7 | 30362853 | ZNRF2 | 1.76E-05 | intron_variant | 0.01132 | 0.002506 |
| rs114690145 | 7 | 30364225 | ZNRF2 | 1.76E-05 | intron_variant | 0.01132 | 0.002506 |
| rs6943601 | 7 | 30365310 | ZNRF2 | 1.76E-05 | intron_variant | 0.01132 | 0.002506 |
| rs77946931 | 7 | 30365824 | ZNRF2 | 1.76E-05 | intron_variant | 0.01132 | 0.002506 |
| rs115112447 | 7 | 30365833 | ZNRF2 | 1.76E-05 | intron_variant | 0.01132 | 0.002506 |
| rs12056300 | 7 | 30367221 | ZNRF2 | 1.76E-05 | intron_variant | 0.01132 | 0.002506 |
| rs17158851 | 7 | 30368265 | ZNRF2 | 1.76E-05 | intron_variant | 0.01132 | 0.002506 |
| rs56694867 | 7 | 30368384 | ZNRF2 | 4.00E-05 | intron_variant | 0.01048 | 0.002435 |
| rs12056131 | 7 | 30369299 | ZNRF2 | 1.76E-05 | intron_variant | 0.01132 | 0.002506 |
| rs6979671 | 7 | 30370051 | ZNRF2 | 4.00E-05 | intron_variant | 0.01048 | 0.002435 |

[Table 3-37]

| SNP ID | chr | position | gene locus | p-value | SNP location in gene locus | BETA | SE |
|---|---|---|---|---|---|---|---|
| rs11974360 | 7 | 30370786 | ZNRF2 | 5.12E-06 | intron_variant | 0.01079 | 0.002234 |
| rs76302490 | 7 | 30373774 | ZNRF2 | 1.76E-05 | intron_variant | 0.01132 | 0.002506 |
| rs80280885 | 7 | 30373940 | ZNRF2 | 1.76E-05 | intronvariant | 0.01132 | 0.002506 |

(continued)

| SNP ID | c h r | position | gene locus | p-value | SNP location in gene locus | BETA | SE |
|---|---|---|---|---|---|---|---|
| rs145282100 | 7 | 30375239 | ZNRF2 | 1.76E-05 | intron_variant | 0.01132 | 0.002506 |
| rs9691527 | 7 | 30376086 | ZNRF2 | 4.00E-05 | intron_variant | 0.01048 | 0.002435 |
| rs11979006 | 7 | 30381303 | ZNRF2 | 5.12E-06 | intron_variant | 0.01079 | 0.002234 |
| 7:30383851 : CT:C | 7 | 30383851 | NA | 5.12E-06 | NA | 0.01079 | 0.002234 |
| rs111250775 | 7 | 30384822 | ZNRF2 | 5.12E-06 | intron_variant | 0.01079 | 0.002234 |
| rs80107665 | 7 | 30387141 | ZNRF2 | 4.22E-05 | intron_variant | 0.01046 | 0.002437 |
| rs146758046 | 7 | 30387248 | ZNRF2 | 4.22E-05 | intron_variant | 0.01046 | 0.002437 |
| rs17158885 | 7 | 30389865 | ZNRF2 | 4.22E-05 | intron_variant | 0.01046 | 0.002437 |
| rs17158888 | 7 | 30391066 | ZNRF2 | 4.22E-05 | intron_variant | 0.01046 | 0.002437 |
| rs11531494 | 7 | 30392422 | ZNRF2 | 4.22E-05 | intron_variant | 0.01046 | 0.002437 |
| rs42580 | 7 | 30397028 | ZNRF2 | 5.12E-06 | intron_variant | 0.01079 | 0.002234 |
| rs42589 | 7 | 30402571 | AC006978. 2 | 2.22E-05 | intron_variant | 0.00981 7 | 0.002202 |
| rs73695251 | 7 | 39331345 | POU6F2 | 3.36E-05 | NMD_transcript_ variant | 0.02137 | 0.004912 |
| rs73695253 | 7 | 39333216 | POU6F2 | 3.36E-05 | NMD_transcript_ variant | 0.02137 | 0.004912 |
| rs117797113 | 7 | 42239771 | GLI3 | 8.84E-06 | intron_variant | 0.01851 | 0.003943 |
| rs1525251 | 7 | 46738745 | NA | 3.12E-05 | | 0.01668 | 0.003818 |
| 7:46949185 :A: ACCCCC | 7 | 46949185 | NA | 6.37E-06 | NA | 0.01094 | 0.002292 |
| rs12673320 | 7 | 46955408 | NA | 2.40E-05 | | 0.01026 | 0.002312 |
| rs58346043 | 7 | 46957249 | NA | 1.76E-05 | | 0.01015 | 0.002246 |
| rs55968856 | 7 | 46963997 | NA | 2.40E-05 | | 0.01026 | 0.002312 |
| 7:46964575 : GT:G | 7 | 46964575 | NA | 1.76E-05 | NA | 0.01015 | 0.002246 |
| rs115849624 | 7 | 46964743 | NA | 2.40E-05 | NA | 0.01026 | 0.002312 |
| rs13224225 | 7 | 46966997 | NA | 1.76E-05 | | 0.01015 | 0.002246 |
| rs10951882 | 7 | 46967335 | NA | 1.76E-05 | | 0.01015 | 0.002246 |
| rs57132170 | 7 | 46968669 | NA | 1.76E-05 | | 0.01015 | 0.002246 |
| rs16881424 | 7 | 46973649 | NA | 2.40E-05 | | 0.01026 | 0.002312 |
| rs117604188 | 7 | 52054590 | NA | 2.99E-05 | | 0.02149 | 0.004905 |
| rs146024011 | 7 | 52061279 | NA | 2.99E-05 | | 0.02149 | 0.004905 |
| rs114085512 | 7 | 52062875 | NA | 2.99E-05 | | 0.02149 | 0.004905 |
| rs117563572 | 7 | 52067629 | NA | 2.99E-05 | | 0.02149 | 0.004905 |
| rs138791169 | 7 | 52068597 | NA | 2.99E-05 | | 0.02149 | 0.004905 |
| rs139032318 | 7 | 52071591 | NA | 2.99E-05 | | 0.02149 | 0.004905 |
| rs78326066 | 7 | 52146162 | NA | 3.44E-06 | | 0.01574 | 0.003194 |

[Table 3-38]

| SNP ID | chr | position | gene locus | p-value | SNP location in gene locus | BETA | SE |
|---|---|---|---|---|---|---|---|
| rs77343772 | 7 | 73408830 | NA | 2.41E-05 | | 0.01764 | 0.003976 |
| rs6972672 | 7 | 73409861 | NA | 2.41E-05 | | 0.01764 | 0.003976 |
| rs6973358 | 7 | 73410290 | NA | 2.41E-05 | | 0.01764 | 0.003976 |
| rs6973541 | 7 | 73410408 | NA | 2.41E-05 | | 0.01764 | 0.003976 |
| 7:75438562 :T: TTTTTTA | 7 | 75438562 | NA | 3.95E-05 | NA | 0.008973 | 0.002083 |
| rs76364545 | 7 | 75440752 | NA | 3.04E-05 | | 0.01524 | 0.003482 |
| rs11465296 | 7 | 75442294 | CCL24 | 3.62E-06 | intron_variant | 0.02043 | 0.004156 |
| rs11465293 | 7 | 75442723 | CCL24 | 3.62E-06 | missense_ variant | 0.02043 | 0.004156 |
| rs11465286 | 7 | 75443961 | CCL24 | 3.62E-06 | intron_variant | 0.02043 | 0.004156 |
| rs79404066 | 7 | 75444215 | CCL24 | 3.62E-06 | intron_variant | 0.02043 | 0.004156 |
| rs117679282 | 7 | 75446052 | CCL24 | 3.62E-06 | intron_variant | 0.02043 | 0.004156 |
| rs11514980 | 7 | 75459923 | NA | 3.62E-06 | | 0.02043 | 0.004156 |
| rs11521067 | 7 | 75459954 | NA | 3.62E-06 | | 0.02043 | 0.004156 |
| rs147465974 | 7 | 75468367 | NA | 3.62E-06 | | 0.02043 | 0.004156 |
| 7:76910430 :C:CA | 7 | 76910430 | NA | 8.26E-06 | NA | 0.01788 | 0.003795 |
| 7:101289699 :G:A | 7 | 101289699 | NA | 1.04E-07 | NA | 0.03229 | 0.005617 |
| rs145263975 | 7 | 128449126 | CCDC136 | 1.72E-05 | intron_variant | 0.02707 | 0.005982 |
| rs189900162 | 7 | 128453455 | CCDC136 | 1.72E-05 | intron_variant | 0.02707 | 0.005982 |
| rs149344851 | 7 | 128455033 | CCDC136 | 1.72E-05 | intron_variant | 0.02707 | 0.005982 |
| rs143205471 | 7 | 128464165 | NA | 1.72E-05 | | 0.02707 | 0.005982 |
| rs147658468 | 7 | 128465079 | NA | 1.72E-05 | | 0.02707 | 0.005982 |

(continued)

| SNP ID | chr | position | gene locus | p-value | SNP location in gene locus | BETA | SE |
|---|---|---|---|---|---|---|---|
| rs141683491 | 7 | 128469854 | NA | 1.72E-05 | | 0.02707 | 0.005982 |
| rs199842696 | 7 | 128470680 | FLNC | 1.72E-05 | 5_prime_0. UTR_variant | 02707 | 0.005982 |
| rs151072488 | 7 | 128473938 | FLNC | 1.72E-05 | intron_variant | 0.02707 | 0.005982 |
| rs138755199 | 7 | 128475236 | FLNC | 1.72E-05 | intron_variant | 0.02707 | 0.005982 |
| rs144736830 | 7 | 128479080 | FLNC | 1.72E-05 | intron_variant | 0.02707 | 0.005982 |
| rs60691847 | 7 | 128499388 | NA | 1.72E-05 | | 0.02707 | 0.005982 |
| rs73459264 | 7 | 128505510 | KCP | 1.72E-05 | intron_variant | 0.02707 | 0.005982 |
| rs183994302 | 7 | 128513368 | KCP | 1.72E-05 | intron_variant | 0.02707 | 0.005982 |
| rs112749538 | 7 | 128513415 | KCP | 1.72E-05 | intron_variant | 0.02707 | 0.005982 |
| rs182514276 | 7 | 128514838 | KCP | 1.72E-05 | intron_variant | 0.02707 | 0.005982 |
| 7:128522809 :AAAAC: A | 7 | 128522809 | NA | NA | NA | NA | NA |
| 7:128522809 : AAAACAAACAAAC : AAAAC | 7 | 128522809 | NA | 1.72E-05 | NA | 0.02707 | 0.005982 |
| rs143202507 | 7 | 128523082 | KCP | 1.72E-05 | intronvariant | 0.02707 | 0.005982 |
| rs111838117 | 7 | 128524190 | KCP | 1.72E-05 | intron_variant | 0.02707 | 0.005982 |

[Table 3-39]

| SNP ID | chr | position | gene locus | p-value | SNP location in gene locus | BETA | SE |
|---|---|---|---|---|---|---|---|
| rs144312682 | 7 | 128526221 | KCP | 1.72E-05 | intron_variant | 0.02707 | 0.005982 |
| 7:131372591:A: G | 7 | 131372591 | NA | 8.84E-06 | NA | 0.02259 | 0.004813 |
| rs137956592 | 7 | 138063857 | NA | 2.50E-05 | | 0.02626 | 0.005931 |
| rs186944015 | 7 | 141792526 | MGAM | 2.19E-06 | intron_variant | 0.02908 | 0.005774 |
| 7:148274173:T: C | 7 | 148274173 | NA | 2.50E-05 | NA | 0.02626 | 0.005931 |
| rs140478856 | 7 | 148374761 | NA | 2.50E-05 | | 0.01067 | 0.00241 |
| rs77070865 | 7 | 148384615 | NA | 2.50E-05 | | 0.01067 | 0.00241 |

(continued)

| SNP ID | chr | position | gene locus | p-value | SNP location in gene locus | BETA | SE |
|---|---|---|---|---|---|---|---|
| rs35116518 | 7 | 148386436 | NA | 2.50E-05 | | 0.01067 | 0.00241 |
| rs114140823 | 7 | 148386467 | NA | 2.50E-05 | | 0.01067 | 0.00241 |
| rs34369394 | 7 | 148388377 | NA | 2.50E-05 | | 0.01067 | 0.00241 |
| rs10235843 | 7 | 148400466 | CUL1 | 2.34E-06 | intron_variant | 0.01112 | 0.002214 |
| 7:148422315:A:G | 7 | 148422315 | NA | 5.91E-07 | NA | 0.01284 | 0.0024 |
| rs11974639 | 7 | 148438804 | CUL1 | 1.26E-08 | intron_variant | 0.01571 | 0.002527 |
| rs147617254 | 7 | 148458807 | CUL1 | 1.39E-05 | intron_variant | 0.02248 | 0.00491 |
| rs17626232 | 7 | 148459675 | CUL1 | 1.26E-08 | intron_variant | 0.01571 | 0.002527 |
| rs10246773 | 7 | 148460536 | CUL1 | 1.26E-08 | intron_variant | 0.01571 | 0.002527 |
| rs3807446 | 7 | 148467447 | CUL1 | 1.26E-08 | intron_variant | 0.01571 | 0.002527 |
| rs758880 | 7 | 148477589 | CUL1 | 2.34E-05 | intron_variant | 0.00476 | 0.001071 |
| rs113817468 | 7 | 148478201 | CUL1 | 4.85E-07 | intron_variant | 0.01782 | 0.003303 |
| rs10245290 | 7 | 148530294 | EZH2 | 4.85E-07 | intron_variant | 0.01782 | 0.003303 |
| rs73158265 | 7 | 148537086 | EZH2 | 4.41E-06 | intron_variant | 0.01799 | 0.003696 |
| rs56248471 | 7 | 148553810 | EZH2 | 4.41E-06 | intron_variant | 0.01799 | 0.003696 |
| rs10952780 | 7 | 148554335 | EZH2 | 1.55E-05 | intron_variant | 0.006021 | 0.001323 |
| rs113910590 | 7 | 148554794 | EZH2 | 2.56E-06 | intron_variant | -0.007334 | 0.001467 |
| 7:148558320:CT:C | 7 | 148558320 | NA | 1.55E-05 | NA | D.006021 | 0.001323 |
| rs10952783 | 7 | 148559071 | EZH2 | 1.55E-05 | intron_variant | 0.006021 | 0.001323 |
| 7:148560015 :G:GTTTTGT | 7 | 148560015 | NA | 2.56E-06 | NA | -0.007334 | 0.001467 |
| rs28706086 | 7 | 148560068 | EZH2 | 2.56E-06 | intron_variant | -0.007334 | 0.001467 |
| rs10259102 | 7 | 148561029 | EZH2 | 2.56E-06 | intronvariant | -0.007334 | 0.001467 |
| rs10249392 | 7 | 148561324 | EZH2 | 2.56E-06 | intron_variant | -0.007334 | 0.001467 |
| rs11976154 | 7 | 148561586 | EZH2 | 2.56E-06 | intron_variant | -0.007334 | 0.001467 |
| rs28617605 | 7 | 148562161 | EZH2 | 2.56E-06 | intron_variant | -0.007334 | 0.001467 |
| 7:148562217 :CAAA:C | 7 | 148562217 | NA | 2.56E-06 | NA | -0.007334 | 0.001467 |
| rs1996996 | 7 | 148562543 | EZH2 | 2.56E-06 | intron_variant | -0.007334 | 0.001467 |
| rs73158280 | 7 | 148563393 | EZH2 | 2.56E-06 | intron_variant | -0.007334 | 0.001467 |

[Table 3-40]

| SNP ID | ch r | position | gene locus | p-value | SNP location in gene locus | BETA | SE |
|---|---|---|---|---|---|---|---|
| rs142648914 | 7 | 148563610 | EZH2 | 2.56E-06 | intron_variant | -0.007334 | 0.001467 |
| rs142248416 | 7 | 148563611 | EZH2 | 2.56E-06 | intron_variant | -0.007334 | 0.001467 |

(continued)

| SNP ID | ch r | position | gene locus | p-value | SNP location in gene locus | BETA | SE |
|---|---|---|---|---|---|---|---|
| rs111880657 | 7 | 148564058 | EZH2 | 2.56E-06 | intron_variant | -0.007334 | 0.001467 |
| rs113161209 | 7 | 148564367 | EZH2 | 2.56E-06 | intron_variant | -0.007334 | 0.001467 |
| rs73158281 | 7 | 148565649 | EZH2 | 2.56E-06 | intron_variant | -0.007334 | 0.001467 |
| rs149022366 | 7 | 148566024 | EZH2 | 2.56E-06 | intronvariant | -0.007334 | 0.001467 |
| rs1917057 | 7 | 148567957 | EZH2 | 2.56E-06 | intron_variant | -0.007334 | 0.001467 |
| rs2373415 | 7 | 148570280 | EZH2 | 2.56E-06 | intron_variant | -0.007334 | 0.001467 |
| rs2373416 | 7 | 148570344 | EZH2 | 2.56E-06 | intron_variant | -0.007334 | 0.001467 |
| rs2373417 | 7 | 148570382 | EZH2 | 2.56E-06 | intron_variant | -0.007334 | 0.001467 |
| rs2373418 | 7 | 148570447 | EZH2 | 2.56E-06 | intron_variant | -0.007334 | 0.001467 |
| rs7458365 | 7 | 148570551 | EZH2 | 2.56E-06 | intron_variant | -0.007334 | 0.001467 |
| rs28455679 | 7 | 148570741 | EZH2 | 2.56E-06 | intron_variant | -0.007334 | 0.001467 |
| rs6972079 | 7 | 148571196 | EZH2 | 2.56E-06 | intron_variant | -0.007334 | 0.001467 |
| rs6963116 | 7 | 148571393 | EZH2 | 2.56E-06 | intron_variant | -0.007334 | 0.001467 |
| rs28814083 | 7 | 148573027 | EZH2 | 2.56E-06 | intron_variant | -0.007334 | 0.001467 |
| rs28844638 | 7 | 148573061 | EZH2 | 2.56E-06 | intron_variant | -0.007334 | 0.001467 |
| rs12531383 | 7 | 148573311 | AC073140.1 | 1.55E-05 | non_coding_ transcript_ exon_ variant | 0.006021 | 0.001323 |
| 7:148573384 : G:GA | 7 | 148573384 | NA | 2.56E-06 | NA | -0.007334 | 0.001467 |
| rs149343167 | 7 | 148573692 | NA | 2.56E-06 | NA | -0.007334 | 0.001467 |
| rs7783459 | 7 | 148573898 | EZH2 | 2.56E-06 | intron_variant | -0.007334 | 0.001467 |
| rs7795158 | 7 | 148576193 | EZH2 | 2.56E-06 | intron_variant | -0.007334 | 0.001467 |
| rs1880357 | 7 | 148577610 | EZH2 | 2.56E-06 | intron_variant | -0.007334 | 0.001467 |
| rs2177567 | 7 | 148578753 | EZH2 | 1.55E-05 | intron_variant | 0.006021 | 0.001323 |
| rs80052686 | 7 | 148580809 | EZH2 | 2.56E-06 | intron_variant | -0.007334 | 0.001467 |
| rs11984309 | 7 | 148589869 | NA | 2.68E-05 | | -0.006743 | 0.001529 |
| rs67648693 | 7 | 148591911 | NA | 1.73E-06 | | -0.007768 | 0.001525 |
| 7:148595511 : C:CA | 7 | 148595511 | NA | 1.81E-05 | NA | -0.007034 | 0.001559 |
| 7:148596986 : C:CT | 7 | 148596986 | NA | 6.30E-06 | NA | -0.007239 | 0.001515 |
| rs202221022 | 7 | 148597557 | NA | 1.73E-06 | NA | -0.007768 | 0.001525 |
| rs7790642 | 7 | 148600310 | NA | 1.97E-05 | | -0.007169 | 0.001597 |
| rs7777746 | 7 | 148602583 | NA | 1.71E-05 | | 0.005419 | 0.001197 |
| rs62505409 | 7 | 148602689 | NA | 1.73E-06 | | -0.007768 | 0.001525 |
| rs58371016 | 7 | 148604818 | NA | 1.73E-06 | | -0.007768 | 0.001525 |
| rs6558814 | 8 | 3589281 | CSMD1 | 7.33E-06 | intron_variant | 0.007301 | 0.00154 |
| rs79572000 | 8 | 3594099 | CSMD1 | 7.86E-06 | intron_variant | 0.006245 | 0.001323 |

[Table 3-41]

| SNP ID | chr | position | gene locus | p-value | SNP location in gene locus | BETA | SE |
|---|---|---|---|---|---|---|---|
| rs79727403 | 8 | 3595476 | CSMD1 | 6.22E-06 | intron_variant | 0.0082 | 0.001715 |
| rs78364798 | 8 | 3596451 | CSMD1 | 6.72E-06 | intron_variant | 0.006477 | 0.00136 |
| rs140360037 | 8 | 3596652 | CSMD1 | 4.28E-06 | intronvariant | 0.01006 | 0.002064 |
| rs17067299 | 8 | 3599207 | CSMD1 | 9.96E-06 | intron_variant | 0.009011 | 0.001932 |
| rs74706909 | 8 | 3600132 | CSMD1 | 4.94E-06 | intron_variant | 0.01022 | 0.002113 |
| rs74985428 | 8 | 3600451 | CSMD1 | 4.94E-06 | intronvariant | 0.01022 | 0.002113 |
| rs12549613 | 8 | 3601137 | CSMD1 | 4.94E-06 | intron_variant | 0.01022 | 0.002113 |
| rs79210493 | 8 | 3602245 | CSMD1 | 2.12E-06 | intron_variant | 0.01006 | 0.001995 |
| rs4639541 | 8 | 3604375 | CSMD1 | 2.12E-06 | intron_variant | 0.01006 | 0.001995 |
| rs4424283 | 8 | 3605823 | CSMD1 | 4.28E-06 | intron_variant | 0.01006 | 0.002064 |
| rs78615686 | 8 | 4795722 | CSMD1 | 1.04E-07 | intron_variant | 0.03229 | 0.005617 |
| rs75778595 | 8 | 4801168 | CSMD1 | 3.45E-08 | intron_variant | 0.02728 | 0.00455 |
| rs6558944 | 8 | 4802163 | CSMD1 | 3.45E-08 | intron_variant | 0.02728 | 0.00455 |
| rs75096153 | 8 | 4807509 | CSMD1 | 2.04E-05 | intron_variant | 0.01563 | 0.003489 |
| rs80229469 | 8 | 4823427 | CSMD1 | 3.45E-08 | intron_variant | 0.02728 | 0.00455 |
| rs75338858 | 8 | 4825118 | CSMD1 | 3.45E-08 | intron_variant | 0.02728 | 0.00455 |
| rs76891224 | 8 | 4827394 | CSMD1 | 3.45E-08 | intron_variant | 0.02728 | 0.00455 |
| rs3911307 | 8 | 4839914 | CSMD1 | 3.45E-08 | intron_variant | 0.02728 | 0.00455 |
| rs77772200 | 8 | 4840914 | CSMD1 | 3.45E-08 | intron_variant | 0.02728 | 0.00455 |
| rs73497791 | 8 | 4842344 | CSMD1 | 3.45E-08 | intron_variant | 0.02728 | 0.00455 |
| rs74350143 | 8 | 4842467 | CSMD1 | 3.45E-08 | intron_variant | 0.02728 | 0.00455 |
| rs17071982 | 8 | 4842970 | CSMD1 | 3.45E-08 | intron_variant | 0.02728 | 0.00455 |
| rs6999675 | 8 | 4844827 | CSMD1 | 3.45E-08 | intron_variant | 0.02728 | 0.00455 |
| rs7013059 | 8 | 4845003 | CSMD1 | 3.45E-08 | intron_variant | 0.02728 | 0.00455 |
| rs145241498 | 8 | 4854469 | NA | 3.45E-08 | | 0.02728 | 0.00455 |
| rs78561947 | 8 | 4856358 | NA | 3.45E-08 | | 0.02728 | 0.00455 |
| rs79998369 | 8 | 4858392 | NA | 3.45E-08 | | 0.02728 | 0.00455 |
| rs75281977 | 8 | 4863064 | NA | 3.45E-08 | | 0.02728 | 0.00455 |
| rs74639771 | 8 | 4870975 | NA | 3.45E-08 | | 0.02728 | 0.00455 |
| rs77504238 | 8 | 5030143 | NA | 1.07E-05 | | 0.02722 | 0.005861 |
| rs73219702 | 8 | 13552293 | NA | 2.95E-05 | | 0.01874 | 0.004274 |
| rs79671431 | 8 | 15284873 | TUSC3 | 1.89E-05 | non_coding_ transcript_ variant | 0.01924 | 0.004276 |
| rs12155706 | 8 | 16684041 | NA | 3.26E-05 | | 0.02121 | 0.004868 |
| rs12155708 | 8 | 16684111 | NA | 3.26E-05 | | 0.02121 | 0.004868 |
| rs56340335 | 8 | 16685007 | NA | 3.26E-05 | | 0.02121 | 0.004868 |
| rs73201722 | 8 | 16687313 | NA | 3.26E-05 | | 0.02121 | 0.004868 |

[Table 3-42]

| SNP ID | chr | position | gene locus | p-value | SNP location in gene locus | BETA | SE |
|---|---|---|---|---|---|---|---|
| rs73201728 | 8 | 16688532 | NA | 3.26E-05 | | 0.02121 | 0.004868 |
| rs73201732 | 8 | 16689879 | NA | 3.26E-05 | | 0.02121 | 0.004868 |
| rs73201733 | 8 | 16689886 | NA | 3.26E-05 | | 0.02121 | 0.004868 |
| rs4922106 | 8 | 19691250 | INTS10 | 1.71E-05 | intron_variant | 0.005608 | 0.001239 |
| 8:19691517:C:CA | 8 | 19691517 | NA | 2.62E-05 | NA | 0.00604 | 0.001368 |
| rs4922107 | 8 | 19691603 | INTS10 | 1.71E-05 | intron_variant | 0.005608 | 0.001239 |
| rs140015982 | 8 | 26335752 | BNIP3L | 2.72E-07 | intron_variant | 0.03139 | 0.005677 |
| rs112411185 | 8 | 53110658 | ST18 | 2.03E-05 | intron_variant | 0.0152 | 0.003391 |
| rs62509183 | 8 | 64209694 | NA | 1.55E-05 | | 0.01054 | 0.002315 |
| rs147990281 | 8 | 65206514 | NA | 2.30E-05 | | 0.02171 | 0.004879 |
| 8:65811756:TC:T | 8 | 65811756 | NA | 2.58E-05 | NA | 0.02213 | 0.005008 |
| rs147627421 | 8 | 72783939 | NA | 7.90E-06 | | 0.014 | 0.002965 |
| rs147840558 | 8 | 72968161 | TRPA1 | 1.67E-06 | intron_variant | 0.01859 | 0.003644 |
| rs142352715 | 8 | 72968759 | TRPA1 | 1.67E-06 | intron_variant | 0.01859 | 0.003644 |
| rs141156682 | 8 | 72970760 | TRPA1 | 1.67E-06 | intron_variant | 0.01859 | 0.003644 |
| rs2587560 | 8 | 72990136 | NA | 3.42E-05 | | 0.0133 | 0.003059 |
| rs2587563 | 8 | 72991997 | NA | 3.42E-05 | | 0.0133 | 0.003059 |
| rs2587564 | 8 | 72992052 | NA | 3.42E-05 | | 0.0133 | 0.003059 |
| rs2587565 | 8 | 72992580 | NA | 3.42E-05 | | 0.0133 | 0.003059 |
| rs1443951 | 8 | 72993388 | NA | 3.42E-05 | | 0.0133 | 0.003059 |
| rs2587568 | 8 | 72994349 | NA | 3.42E-05 | | 0.0133 | 0.003059 |
| rs2587569 | 8 | 72994434 | NA | 3.42E-05 | | 0.0133 | 0.003059 |
| rs2701457 | 8 | 72995307 | NA | 3.42E-05 | | 0.0133 | 0.003059 |
| rs2587572 | 8 | 72995403 | NA | 3.42E-05 | | 0.0133 | 0.003059 |
| rs1838286 | 8 | 72995797 | NA | 3.42E-05 | | 0.0133 | 0.003059 |
| rs2587574 | 8 | 72996845 | NA | 3.42E-05 | | 0.0133 | 0.003059 |
| rs2587575 | 8 | 72997364 | NA | 3.42E-05 | | 0.0133 | 0.003059 |
| rs1899762 | 8 | 72997899 | NA | 3.42E-05 | | 0.0133 | 0.003059 |
| rs1899761 | 8 | 72998278 | NA | 3.42E-05 | | 0.0133 | 0.003059 |
| rs2701455 | 8 | 72998638 | NA | 3.42E-05 | | 0.0133 | 0.003059 |
| rs13274010 | 8 | 73000500 | NA | 3.42E-05 | | 0.0133 | 0.003059 |
| rs59359973 | 8 | 73001147 | NA | 3.42E-05 | | 0.0133 | 0.003059 |
| rs1443950 | 8 | 73001565 | NA | 3.42E-05 | | 0.0133 | 0.003059 |
| rs1443949 | 8 | 73001624 | NA | 3.42E-05 | | 0.0133 | 0.003059 |
| rs10504528 | 8 | 73001662 | NA | 3.42E-05 | | 0.0133 | 0.003059 |
| rs10504529 | 8 | 73001833 | NA | 3.42E-05 | | 0.0133 | 0.003059 |

[Table 3-43]

| SNP ID | chr | position | gene locus | p-value | SNP location in gene locus | BETA | SE |
|---|---|---|---|---|---|---|---|
| rs11995530 | 8 | 73002151 | NA | 3.42E-05 | | 0.0133 | 0.003059 |
| rs1443947 | 8 | 73002691 | NA | 3.42E-05 | | 0.0133 | 0.003059 |
| rs34722660 | 8 | 73003389 | NA | 3.42E-05 | | 0.0133 | 0.003059 |
| rs16938000 | 8 | 73003527 | NA | 3.42E-05 | | 0.0133 | 0.003059 |
| rs34257316 | 8 | 73003708 | NA | 3.42E-05 | | 0.0133 | 0.003059 |
| rs34109030 | 8 | 73003720 | NA | 3.42E-05 | | 0.0133 | 0.003059 |
| rs2383847 | 8 | 73004294 | NA | 3.42E-05 | | 0.0133 | 0.003059 |
| rs2383848 | 8 | 73004297 | NA | 3.42E-05 | | 0.0133 | 0.003059 |
| rs2383850 | 8 | 73004347 | NA | 3.42E-05 | | 0.0133 | 0.003059 |
| rs2383851 | 8 | 73004372 | NA | 3.42E-05 | | 0.0133 | 0.003059 |
| rs11444287 | 8 | 73004443 | NA | 3.42E-05 | | 0.0133 | 0.003059 |
| rs2120468 | 8 | 73005062 | NA | 3.42E-05 | | 0.0133 | 0.003059 |
| rs2028707 | 8 | 73005371 | NA | 3.42E-05 | | 0.0133 | 0.003059 |
| rs35398772 | 8 | 73016364 | NA | 8.38E-07 | | 0.01769 | 0.00336 |
| rs13276665 | 8 | 73016825 | NA | 8.38E-07 | | 0.01769 | 0.00336 |
| rs17815050 | 8 | 73017580 | NA | 8.38E-07 | | 0.01769 | 0.00336 |
| rs13278765 | 8 | 73018900 | NA | 8.38E-07 | | 0.01769 | 0.00336 |
| rs4601363 | 8 | 73024793 | NA | 8.38E-07 | | 0.01769 | 0.00336 |
| rs35873044 | 8 | 73025944 | NA | 8.38E-07 | | 0.01769 | 0.00336 |
| rs13267160 | 8 | 73030409 | NA | 8.38E-07 | | 0.01769 | 0.00336 |
| rs28429254 | 8 | 76012207 | NA | 4.74E-06 | | 0.00985 | 0.002032 |
| rs17370342 | 8 | 76012812 | NA | 4.74E-06 | | 0.00985 | 0.002032 |
| rs10504590 | 8 | 76013464 | NA | 5.41E-06 | | 0.009636 | 0.002001 |
| rs28450580 | 8 | 76014941 | NA | 1.80E-05 | | 0.007101 | 0.001574 |
| rs142904843 | 8 | 89944036 | NA | 5.59E-06 | | 0.02836 | 0.005899 |
| rs76655252 | 8 | 89952367 | NA | 5.59E-06 | | 0.02836 | 0.005899 |
| rs114639281 | 8 | 90009741 | NA | 7.75E-07 | | 0.02188 | 0.004141 |
| 8:101583921 : A:C | 8 | 101583921 | NA | 2.54E-05 | NA | 0.02167 | 0.004898 |
| rs184447468 | 8 | 101800848 | NA | 2.54E-05 | | 0.02167 | 0.004898 |
| rs143939515 | 8 | 103149542 | NA | 5.25E-06 | | 0.01227 | 0.002544 |
| rs6993195 | 8 | 103164396 | NA | 1.93E-05 | | 0.01491 | 0.003317 |
| rs142025872 | 8 | 103169327 | NA | 1.93E-05 | | 0.01491 | 0.003317 |
| rs56191596 | 8 | 103177900 | NA | 1.92E-05 | | 0.01904 | 0.004234 |
| rs33954588 | 8 | 106008738 | ZFPM2 | 1.45E-05 | non_coding_ transcript_ variant | 0.005002 | 0.001095 |

(continued)

| SNP ID | chr | position | gene locus | p-value | SNP location in gene locus | BETA | SE |
|---|---|---|---|---|---|---|---|
| rs1879688 | 8 | 106010200 | ZFPM2 | 1.45E-05 | non_coding_transcript _variant | 0.005002 | 0.001095 |
| 8:106025397 : TAAA:T | 8 | 106025397 | NA | 1.45E-05 | NA | 0.005002 | 0.001095 |

[Table 3-44]

| SNP ID | chr | position | gene locus | p-value | SNP location in gene locus | BETA | SE |
|---|---|---|---|---|---|---|---|
| rs6984262 | 8 | 106030373 | ZFPM2 | 1.45E-05 | non_coding_ transcript_ variant | 0.005002 | 0.001095 |
| rs73304184 | 8 | 106537180 | ZFPM2 | 1.49E-05 | intron_variant | 0.01946 | 0.004266 |
| rs76868435 | 8 | 106537986 | ZFPM2 | 1.49E-05 | intron_variant | 0.01946 | 0.004266 |
| rs79001083 | 8 | 106538183 | ZFPM2 | 1.49E-05 | intron_variant | 0.01946 | 0.004266 |
| rs73304192 | 8 | 106540300 | ZFPM2 | 1.49E-05 | intron_variant | 0.01946 | 0.004266 |
| rs74395542 | 8 | 106544183 | ZFPM2 | 1.49E-05 | intron_variant | 0.01946 | 0.004266 |
| 8:106545091 : GTT:GT | 8 | 106545091 | NA | NA | NA | NA | NA |
| 8:106545091 : GTT:G | 8 | 106545091 | NA | 1.49E-05 | NA | 0.01946 | 0.004266 |
| rs73306213 | 8 | 106550015 | ZFPM2 | 1.49E-05 | intron_variant | 0.01946 | 0.004266 |
| rs73306228 | 8 | 106556176 | ZFPM2 | 1.49E-05 | intron_variant | 0.01946 | 0.004266 |
| rs73306230 | 8 | 106558263 | ZFPM2 | 1.49E-05 | intron_variant | 0.01946 | 0.004266 |
| rs73306231 | 8 | 106559352 | ZFPM2 | 1.49E-05 | intron_variant | 0.01946 | 0.004266 |
| rs7844467 | 8 | 106562252 | ZFPM2 | 1.49E-05 | intron_variant | 0.01946 | 0.004266 |
| rs34823616 | 8 | 106566606 | ZFPM2 | 104E-07 | intron_variant | 0.03229 | 0.005617 |
| 8:108811752: T:C | 8 | 108811752 | NA | 322E-05 | NA | 0.02682 | 0.00615 |
| 8:108849027: T:A | 8 | 108849027 | NA | 3.22E-05 | NA | 0.02682 | 0.00615 |
| 8:108850008: G:A | 8 | 108850008 | NA | 3.22E-05 | NA | 0.02682 | 0.00615 |
| 8:108850585: G:A | 8 | 108850585 | NA | 3.22E-05 | NA | 0.02682 | 0.00615 |
| rs115268316 | 8 | 108863551 | NA | 3.56E-06 | | 0.02366 | 0.004811 |
| rs57284855 | 8 | 108865154 | NA | 3.56E-06 | | 0.02366 | 0.004811 |
| 8:108870725: G:A | 8 | 108870725 | NA | 3.56E-06 | NA | 0.02366 | 0.004811 |
| rs149805601 | 8 | 108872570 | NA | 356E-06 | | 0.02366 | 0.004811 |
| rs13269427 | 8 | 108873161 | NA | 3.56E-06 | | 0.02366 | 0.004811 |

(continued)

| SNP ID | chr | position | gene locus | p-value | SNP location in gene locus | BETA | SE |
|---|---|---|---|---|---|---|---|
| rs186061014 | 8 | 108889120 | NA | 3.56E-06 | | 0.02366 | 0.004811 |
| 8:108896855 : G:A | 8 | 108896855 | NA | 3.56E-06 | NA | 0.02366 | 0.004811 |
| rs146645406 | 8 | 108899766 | NA | 356E-06 | | 0.02366 | 0.004811 |
| rs150156788 | 8 | 108900989 | NA | 3.56E-Q6 | | 0.02366 | 0.004811 |
| 8:108901410 : A:G | 8 | 108901410 | NA | 356E-06 | NA | 0.02366 | 0.004811 |
| 8:108906538: C:T | 8 | 108906538 | NA | 3.56E-06 | NA | 0.02366 | 0.004811 |
| 8:108907254: G:T | 8 | 108907254 | NA | 3.56E-06 | NA | 0.02366 | 0.004811 |
| 8:108907257: C:G | 8 | 108907257 | NA | 3.56E-06 | NA | 0.02366 | 0.004811 |
| rs191118779 | 8 | 108907979 | NA | 3.56E-06 | NA | 0.02366 | 0.004811 |
| 8:108908022: G:A | 8 | 108908022 | NA | 3.56E-06 | NA | 0.02366 | 0.004811 |
| rs191097915 | 8 | 108915170 | RSP02 | 3.56E-06 | intron_variant | 0.02366 | 0.004811 |
| rs114143791 | 8 | 108916220 | RSP02 | 3.56E-06 | intron_variant | 0.02366 | 0.004811 |
| 8:108921884: GT:G | 8 | 108921884 | NA | 3.56E-06 | NA | 0.02366 | 0.004811 |

[Table 3-45]

| SNP ID | chr | position | gene locus | p-value | SNP location in gene locus | BETA | SE |
|---|---|---|---|---|---|---|---|
| rs138179792 | 8 | 108923715 | RSP02 | 3.56E-06 | intron_variant | 0.02366 | 0.004811 |
| rs140554138 | 8 | 108925826 | RSP02 | 3.56E-06 | intron_variant | 0.02366 | 0.004811 |
| rs144255075 | 8 | 108925827 | RSP02 | 3.56E-06 | intron_variant | 0.02366 | 0.004811 |
| 8:108927282 :A:C | 8 | 108927282 | NA | 3.22E-05 | NA | 0.02682 | 0.00615 |
| 8:108928843 :G:A | 8 | 108928843 | NA | 3.56E-06 | NA | 0.02366 | 0.004811 |
| rs140670079 | 8 | 108929408 | RSP02 | 3.56E-06 | intron_variant | 0.02366 | 0.004811 |
| rs190110401 | 8 | 108930742 | RSP02 | 3.56E-06 | intron_variant | 0.02366 | 0.004811 |
| rs192028485 | 8 | 116822572 | NA | 1.22E-05 | | 0.02236 | 0.004848 |
| 8:116832799 :T:C | 8 | 116832799 | NA | 1.22E-05 | NA | 0.02236 | 0.004848 |
| rs7836570 | 8 | 116840302 | NA | 1.22E-05 | | 0.02236 | 0.004848 |
| rs16887807 | 8 | 116843619 | NA | 2.50E-05 | | 0.01869 | 0.004222 |
| 8:116846927 :A:C | 8 | 116846927 | NA | 1.22E-05 | NA | 0.02236 | 0.004848 |
| rs28366597 | 8 | 116851543 | NA | 1.22E-05 | | 0.02236 | 0.004848 |
| 8:116855719 :T:A | 8 | 116855719 | NA | 1.22E-05 | NA | 0.02236 | 0.004848 |

(continued)

| SNP ID | chr | position | gene locus | p-value | SNP location in gene locus | BETA | SE |
|---|---|---|---|---|---|---|---|
| 8:126647803 :C:A | 8 | 126647803 | NA | 2.25E-05 | NA | 0.01711 | 0.00384 |
| 8:142514003 :AT:A | 8 | 142514003 | NA | 3.40E-05 | NA | -0.005301 | 0.001219 |
| rs34896467 | 9 | 2213511 | NA | 1.92E-05 | | 0.01136 | 0.002526 |
| rs12350147 | 9 | 2217178 | NA | 2.21E-07 | | 0.01299 | 0.002329 |
| rs7024532 | 9 | 2220348 | NA | 2.21E-07 | | 0.01299 | 0.002329 |
| rs71504697 | 9 | 2222951 | NA | 7.85E-06 | | 0.01228 | 0.0026 |
| rs71504698 | 9 | 2223409 | NA | 7.85E-06 | | 0.01228 | 0.0026 |
| rs17390013 | 9 | 2226756 | NA | 7.85E-06 | | 0.01228 | 0.0026 |
| rs10491702 | 9 | 2227837 | NA | 7.85E-06 | | 0.01228 | 0.0026 |
| rs10491704 | 9 | 2228755 | NA | 7.85E-06 | | 0.01228 | 0.0026 |
| rs12344786 | 9 | 2229313 | NA | 7.85E-06 | | 0.01228 | 0.0026 |
| rs74987881 | 9 | 2229455 | NA | 7.85E-06 | | 0.01228 | 0.0026 |
| rs1105379 | 9 | 16319287 | NA | 6.63E-06 | | 0.01017 | 0.002134 |
| 9:16325344 :G:A | 9 | 16325344 | NA | NA | NA | NA | NA |
| rs72714927 | 9 | 16325344 | NA | 2.19E-06 | | 0.02908 | 0.005774 |
| rs67783457 | 9 | 16325568 | NA | 2.19E-06 | | 0.02908 | 0.005774 |
| rs12553053 | 9 | 16330357 | NA | 2.19E-06 | | 0.02908 | 0.005774 |
| rs56190435 | 9 | 16331743 | NA | 2.19E-06 | | 0.02908 | 0.005774 |
| rs12686134 | 9 | 19605230 | SLC24A2 | 3.51E-05 | intron_variant | 0.02589 | 0.005965 |
| rs35444308 | 9 | 19605274 | SLC24A2 | 3.51E-05 | intron_variant | 0.02589 | 0.005965 |
| rs141589871 | 9 | 19609951 | SLC24A2 | 3.51E-05 | intron_variant | 0.02589 | 0.005965 |
| rs752539 | 9 | 19640344 | SLC24A2 | 2.28E-05 | intron_variant | 0.009444 | 0.002121 |

[Table 3-46]

| SNP ID | ch r | position | gene locus | p-value | SNP location in gene locus | BETA | SE |
|---|---|---|---|---|---|---|---|
| rs12683123 | 9 | 19642100 | SLC24A2 | 2.28E-05 | intron_variant | 0.009444 | 0.002121 |
| rs16937677 | 9 | 19642563 | SLC24A2 | 1.36E-05 | intron_variant | 0.0094 | 0.002051 |
| rs75209268 | 9 | 19653017 | SLC24A2 | 4.78E-05 | intron_variant | 0.009327 | 0.002191 |
| rs12686530 | 9 | 19655641 | SLC24A2 | 4.78E-05 | intron_variant | 0.009327 | 0.002191 |
| rs78907538 | 9 | 19655825 | SLC24A2 | 4.78E-05 | intron_variant | 0.009327 | 0.002191 |
| rs75642022 | 9 | 19657307 | SLC24A2 | 4.78E-05 | intronvariant | 0.009327 | 0.002191 |
| rs72501450 | 9 | 19658168 | SLC24A2 | 4.78E-05 | intron_variant | 0.009327 | 0.002191 |
| rs19412153 | 9 | 19658668 | SLC24A2 | 4.78E-05 | intron_variant | 0.009327 | 0.002191 |
| rs114378135 | 9 | 25102230 | NA | 9.83E-06 | | 0.02248 | 0.004817 |
| rs10966802 | 9 | 25226973 | NA | 3.68E-05 | | 0.00806 | 0.001863 |
| 9:25264525 :G:T | 9 | 25264525 | NA | 5.59E-06 | NA | 0.02836 | 0.005899 |

(continued)

| SNP ID | ch r | position | gene locus | p-value | SNP location in gene locus | BETA | SE |
|---|---|---|---|---|---|---|---|
| rs143890288 | 9 | 25267126 | NA | 5.59E-06 | | 0.02836 | 0.005899 |
| 9:25269360 :G: GAC | 9 | 25269360 | NA | 5.59E-06 | NA | 0.02836 | 0.005899 |
| rs138481346 | 9 | 27860943 | NA | 2.05E-05 | | 0.01887 | 0.004213 |
| rs117835027 | 9 | 28108997 | LING02 | 3.70E-05 | intron_variant | 0.01859 | 0.004298 |
| rs7034549 | 9 | 28109195 | LING02 | 3.70E-05 | intron_variant | 0.01859 | 0.004298 |
| rs7034769 | 9 | 28109318 | LING02 | 3.70E-05 | intron_variant | 0.01859 | 0.004298 |
| rs148234325 | 9 | 28109777 | NA | 3.70E-05 | NA | 0.01859 | 0.004298 |
| rs9722119 | 9 | 28111224 | LING02 | 3.70E-05 | intron_variant | 0.01859 | 0.004298 |
| rs189664295 | 9 | 28111978 | LING02 | 3.70E-05 | intron_variant | 0.01859 | 0.004298 |
| rs147490749 | 9 | 28112009 | LING02 | 370E-05 | intron_variant | 0.01859 | 0.004298 |
| 9:28117021 :T:G | 9 | 28117021 | NA | 3.70E-05 | NA | 0.01859 | 0.004298 |
| rs112776037 | 9 | 37038910 | NA | 356E-06 | | 0.02366 | 0.004811 |
| rs76697541 | 9 | 37039286 | NA | 356E-06 | | 0.02366 | 0.004811 |
| rs118107060 | 9 | 73107527 | NA | 1.72E-05 | | 0.02707 | 0.005982 |
| rs11137993 | 9 | 81625189 | NA | 3.22E-05 | | 0.02682 | 0.00615 |
| rs148742339 | 9 | 82896313 | NA | 1.07E-05 | | 0.02722 | 0.005861 |
| rs148593924 | 9 | 85082296 | AL162726 .3 | 3.41E-05 | non_coding_ tran script_ variant | 0.01657 | 0.003812 |
| rs111776923 | 9 | 87283234 | NA | 4.36E-05 | | 0.01505 | 0.003514 |
| rs111867627 | 9 | 87336518 | NTRK2 | 4.36E-05 | intron_variant | 0.01505 | 0.003514 |
| rs113711779 | 9 | 87352036 | NTRK2 | 4.36E-05 | intron_variant | 0.01505 | 0.003514 |
| rs1114192Q | 9 | 90262011 | DAPK1 | 366E-05 | intron_variant | 0.00712 | 0.001645 |
| rs2274607 | 9 | 90262393 | DAPK1 | 1.57E-05 | intron_variant | 0.007678 | 0.001688 |
| rs145382400 | 9 | 90796382 | NA | 4.07E-05 | | 0.009834 | 0.002287 |
| rs10991738 | 9 | 93757561 | NA | 1.97E-06 | | 0.01165 | 0.002301 |

[Table 3-47]

| SNP ID | chr | position | gene locus | p-value | SNP location in gene locus | BETA | SE |
|---|---|---|---|---|---|---|---|
| 9:93759544 :GGTGT: GGTGTGT | 9 | 93759544 | NA | 4.96E-06 | NA | 0.01079 | 0.002232 |
| rs71509677 | 9 | 93763449 | NA | 1.97E-06 | | 0.01165 | 0.002301 |
| rs35931712 | 9 | 93769334 | NA | 9.88E-06 | | 0.01056 | 0.002264 |
| rs35794334 | 9 | 93777994 | NA | 1.97E-06 | | 0.01165 | 0.002301 |

(continued)

| SNP ID | chr | position | gene locus | p-value | SNP location in gene locus | BETA | SE |
|---|---|---|---|---|---|---|---|
| rs35246326 | 9 | 93789668 | NA | 1.97E-06 | | 0.01165 | 0.002301 |
| rs72739474 | 9 | 93838589 | NA | 1.20E-05 | | 0.008878 | 0.001923 |
| rs296636 | 9 | 93843942 | NA | 8.45E-Q6 | | 0.006094 | 0.001295 |
| rs35748257 | 9 | 93848258 | NA | 3.03E-05 | | 0.005595 | 0.001278 |
| rs55687842 | 9 | 93856705 | NA | 1.47E-05 | | 0.00958 | 0.002099 |
| rs72739496 | 9 | 93857087 | NA | 1.47E-05 | | 0.00958 | 0.002099 |
| rs11312435 | 9 | 93858207 | NA | 1.47E-05 | | 0.00958 | 0.002099 |
| rs79287236 | 9 | 108886577 | NA | 5.59E-06 | | 0.02836 | 0.005899 |
| rs116925051 | 9 | 110829415 | NA | 4.21E-05 | | 0.02111 | 0.00492 |
| rs12337098 | 9 | 113513534 | MUSK | 1.72E-05 | intron_variant | 0.02707 | 0.005982 |
| rs7043625 | 9 | 113516179 | MUSK | 1.72E-05 | intron_variant | 0.02707 | 0.005982 |
| rs77542599 | 9 | 115702427 | NA | 5.59E-06 | | 0.02836 | 0.005899 |
| rs11413824 | 9 | 118344670 | NA | 2.74E-05 | | 0.01152 | 0.002615 |
| rs72765920 | 9 | 124702107 | TTLL1 1 | 2.67E-05 | NMD_transcript_variant | 0.02147 | 0.004867 |
| rs11999389 | 9 | 126872652 | NA | 6.18E-06 | | 0.01846 | 0.003859 |
| rs58848387 | 9 | 128211958 | MAPKAP1 | 2.19E-05 | intron_variant | 0.01024 | 0.002295 |
| 9:128723505 :T:C | 9 | 128723505 | NA | 1.84E-06 | NA | 0.02406 | 0.004738 |
| rs73582691 | 9 | 128752360 | NA | 4.40E-05 | | 0.01313 | 0.003069 |
| rs118085053 | 9 | 139614734 | DIPK1 B | 4.71E-07 | intron_variant | 0.03074 | 0.00569 |
| rs17158762 | 10 | 2701823 | NA | 2.72E-07 | | 0.03139 | 0.005677 |
| 10:5725303:C:T | 10 | 5725303 | NA | 2.72E-07 | NA | 0.03139 | 0.005677 |
| rs4749996 | 10 | 10878872 | CELF2 | 1.04E-07 | intron_variant | 0.03229 | 0.005617 |

(continued)

| SNP ID | chr | position | gene locus | p-value | SNP location in gene locus | BETA | SE |
|---|---|---|---|---|---|---|---|
| rs138294262 | 10 | 10891440 | CELF2 | 1.04E-07 | intron_variant | 0.03229 | 0.005617 |
| 10:13274942 :TA:T | 10 | 13274942 | NA | 3.89E-05 | NA | 0.005751 | 0.001334 |
| rs41291315 | 10 | 13275437 | UCMA | 1.84E-06 | intron_variant | 0.005557 | 0.001094 |
| rs116961903 | 10 | 14008637 | FRMD4A | 3.08E-05 | intron_variant | 0.01168 | 0.00267 |
| rs139902130 | 10 | 14009189 | FRMD4A | 3.08E-05 | intron_variant | 0.01168 | 0.00267 |
| 10:14017770:T:C | 10 | 14017770 | NA | NA | NA | NA | NA |
| 10:14017770 :T:TGC | 10 | 14017770 | NA | 1.64E-05 | NA | 0.009266 | 0.002042 |
| rs1609477 | 10 | 14021383 | FRMD4A | 1.64E-05 | intron_variant | 0.009266 | 0.002042 |
| rs11258773 | 10 | 14025006 | FRMD4A | 1.64E-05 | intron_variant | 0.009266 | 0.002042 |

[Table 3-48]

| SNP ID | chr | position | gene locus | p-value | SNP location in gene locus | BETA | SE |
|---|---|---|---|---|---|---|---|
| rs12572939 | 10 | 14030842 | FRMD4A | 6.94E-07 | intron_variant | 0.01694 | 0.003191 |
| rs7096979 | 10 | 17539678 | NA | 4.71E-07 | | 0.03074 | 0.00569 |
| 10:28017198:AT:A | 10 | 28077198 | NA | 1.07E-05 | NA | 0.02722 | 0.005861 |
| rs351803 | 10 | 55317094 | NA | 2.78E-05 | | 0.006062 | 0.001378 |
| rs351802 | 10 | 55317221 | NA | 2.78E-05 | | 0.006062 | 0.001378 |
| rs351801 | 10 | 55317903 | NA | 2.78E-05 | | 0.006062 | 0.001378 |
| rs351800 | 10 | 55317928 | NA | 2.78E-05 | | 0.006062 | 0.001378 |
| rs351799 | 10 | 55317976 | NA | 2.78E-05 | | 0.006062 | 0.001378 |
| rs2992011 | 10 | 55318012 | NA | 2.78E-05 | | 0.006062 | 0.001378 |
| rs2931565 | 10 | 55318016 | NA | 2.78E-05 | | 0.006062 | 0.001378 |
| rs6481015 | 10 | 55318082 | NA | 2.78E-05 | | 0.006062 | 0.001378 |
| rs6481016 | 10 | 55318158 | NA | 2.78E-05 | | 0.006062 | 0.001378 |
| rs6415918 | 10 | 55318166 | NA | 2.78E-05 | | 0.006062 | 0.001378 |
| rs145658941 | 10 | 55318826 | NA | 2.78E-05 | NA | 0.006062 | 0.001378 |
| rs689581 | 10 | 55318856 | NA | 2.78E-05 | | 0.006062 | 0.001378 |
| rs35010388 | 10 | 55319128 | NA | 2.78E-05 | | 0.006062 | 0.001378 |
| rs145725620 | 10 | 55319312 | NA | 2.78E-05 | NA | 0.006062 | 0.001378 |
| rs394770 | 10 | 55319395 | NA | 2.78E-05 | | 0.006062 | 0.001378 |
| rs149503551 | 10 | 55319419 | NA | 2.78E-05 | NA | 0.006062 | 0.001378 |

(continued)

| SNP ID | chr | position | gene locus | p-value | SNP location in gene locus | BETA | SE |
|---|---|---|---|---|---|---|---|
| rs413137 | 10 | 55319542 | NA | 2.78E-05 | | 0.006062 | 0.001378 |
| rs443931 | 10 | 55319592 | NA | 2.78E-05 | | 0.006062 | 0.001378 |
| rs420314 | 10 | 55319617 | NA | 2.78E-05 | | 0.006062 | 0.001378 |
| rs428563 | 10 | 55319634 | NA | 2.78E-05 | | 0.006062 | 0.001378 |
| rs450516 | 10 | 55319865 | NA | 2.78E-05 | | 0.006062 | 0.001378 |
| rs426290 | 10 | 55320087 | NA | 2.78E-05 | | 0.006062 | 0.001378 |
| rs444728 | 10 | 55320115 | NA | 2.78E-05 | | 0.006062 | 0.001378 |
| rs430495 | 10 | 55320281 | NA | 2.78E-05 | | 0.006062 | 0.001378 |
| rs423146 | 10 | 55320430 | NA | 2.78E-05 | | 0.006062 | 0.001378 |
| rs453158 | 10 | 55320437 | NA | 2.78E-05 | | 0.006062 | 0.001378 |
| rs2484209 | 10 | 55320471 | NA | 2.78E-05 | | 0.006062 | 0.001378 |
| rs668434 | 10 | 55320596 | NA | 2.78E-05 | | 0.006062 | 0.001378 |
| rs668014 | 10 | 55320664 | NA | 2.78E-05 | | 0.006062 | 0.001378 |
| rs451485 | 10 | 55320992 | NA | 2.78E-05 | | 0.006062 | 0.001378 |
| rs396374 | 10 | 55321338 | NA | 2.78E-05 | | 0.006062 | 0.001378 |
| rs423384 | 10 | 55321415 | NA | 2.78E-05 | | 0.006062 | 0.001378 |
| rs409001 | 10 | 55321543 | NA | 2.78E-05 | | 0.006062 | 0.001378 |

[Table 3-49]

| SNP ID | chr | position | gene locus | p-value | SNP location in gene locus | BETA | SE |
|---|---|---|---|---|---|---|---|
| rs399606 | 10 | 55321588 | NA | 2.78E-05 | | 0.006062 | 0.001378 |
| rs371543 | 10 | 55321684 | NA | 2.78E-05 | | 0.006062 | 0.001378 |
| rs382870 | 10 | 55321728 | NA | 2.78E-05 | | 0.006062 | 0.001378 |
| rs428467 | 10 | 55321781 | NA | 2.78E-05 | | 0.006062 | 0.001378 |
| rs446990 | 10 | 55321931 | NA | 2.78E-05 | | 0.006062 | 0.001378 |
| rs379026 | 10 | 55321948 | NA | 2.78E-05 | | 0.006062 | 0.001378 |
| rs379033 | 10 | 55321950 | NA | 2.78E-05 | | 0.006062 | 0.001378 |
| rs407646 | 10 | 55322061 | NA | 2.78E-05 | | 0.006062 | 0.001378 |
| rs407337 | 10 | 55322216 | NA | 2.78E-05 | | 0.006062 | 0.001378 |
| rs445488 | 10 | 55322557 | NA | 2.78E-05 | | 0.006062 | 0.001378 |
| rs394942 | 10 | 55322731 | NA | 2.78E-05 | | 0.006062 | 0.001378 |
| rs423806 | 10 | 55322780 | NA | 2.78E-05 | | 0.006062 | 0.001378 |
| rs389244 | 10 | 55322918 | NA | 2.78E-05 | | 0.006062 | 0.001378 |
| rs451607 | 10 | 55323157 | NA | 2.78E-05 | | 0.006062 | 0.001378 |
| rs425467 | 10 | 55323180 | NA | 2.78E-05 | | 0.006062 | 0.001378 |
| rs395825 | 10 | 55323468 | NA | 2.78E-05 | | 0.006062 | 0.001378 |

(continued)

| SNP ID | chr | position | gene locus | p-value | SNP location in gene locus | BETA | SE |
|---|---|---|---|---|---|---|---|
| rs437390 | 10 | 55323572 | NA | 2.78E-05 | | 0.006062 | 0.001378 |
| rs2484208 | 10 | 55323779 | NA | 2.02E-05 | | 0.006188 | 0.00138 |
| rs2450530 | 10 | 55323807 | NA | 2.78E-05 | | 0.006062 | 0.001378 |
| rs440743 | 10 | 55323832 | NA | 2.78E-05 | | 0.006062 | 0.001378 |
| rs427506 | 10 | 55324048 | NA | 2.78E-05 | | 0.006062 | 0.001378 |
| rs11527477 | 10 | 55324102 | NA | 2.78E-05 | | 0.006062 | 0.001378 |
| rs11527478 | 10 | 55324117 | NA | 2.78E-05 | | 0.006062 | 0.001378 |
| 10:55324128:A:G | 10 | 55324128 | NA | 2.78E-05 | NA | 0.006062 | 0.001378 |
| 10:55324134:T:G | 10 | 55324134 | NA | 2.78E-05 | NA | 0.006062 | 0.001378 |
| rs7075007 | 10 | 55324148 | NA | 2.78E-05 | | 0.006062 | 0.001378 |
| rs11527272 | 10 | 55324150 | NA | 2.78E-05 | | 0.006062 | 0.001378 |
| rs610251 | 10 | 55324303 | NA | 2.78E-05 | | 0.006062 | 0.001378 |
| rs61859905 | 10 | 55324317 | NA | 2.78E-05 | | 0.006062 | 0.001378 |
| rs609908 | 10 | 55324342 | NA | 2.78E-05 | | 0.006062 | 0.001378 |
| rs663624 | 10 | 55324413 | NA | 2.78E-05 | | 0.006062 | 0.001378 |
| rs455078 | 10 | 55324459 | NA | 2.78E-05 | | 0.006062 | 0.001378 |
| rs374891 | 10 | 55324475 | NA | 2.78E-05 | | 0.006062 | 0.001378 |
| 10:55324510:AC:A | 10 | 55324510 | NA | 2.78E-05 | NA | 0.006062 | 0.001378 |
| 10:55324513:C:T | 10 | 55324513 | NA | 2.78E-05 | NA | 0.006062 | 0.001378 |
| 10:55324514:A:G | 10 | 55324514 | NA | 2.78E-05 | NA | 0.006062 | 0.001378 |

[Table 3-50]

| SNP ID | chr | position | gene locus | p-value | SNP location in gene locus | BETA | SE |
|---|---|---|---|---|---|---|---|
| rs398169 | 10 | 55324751 | NA | 2.78E-05 | | 0.006062 | 0.001378 |
| rs416275 | 10 | 55324754 | NA | 2.78E-05 | | 0.006062 | 0.001378 |
| rs665850 | 10 | 55324915 | NA | 2.78E-05 | | 0.006062 | 0.001378 |
| rs665881 | 10 | 55324942 | NA | 2.78E-05 | | 0.006062 | 0.001378 |
| rs607276 | 10 | 55324943 | NA | 2.78E-05 | | 0.006062 | 0.001378 |

(continued)

| SNP ID | chr | position | gene locus | p-value | SNP location in gene locus | BETA | SE |
|---|---|---|---|---|---|---|---|
| rs607228 | 10 | 55324976 | NA | 2.78E-05 | | 0.006062 | 0.001378 |
| rs596456 | 10 | 55325084 | NA | 2.78E-05 | | 0.006062 | 0.001378 |
| rs4456174 | 10 | 55325288 | NA | 2.78E-05 | | 0.006062 | 0.001378 |
| rs678445 | 10 | 55325383 | NA | 6.84E-06 | | 0.00675 | 0.001419 |
| rs678459 | 10 | 55325386 | NA | 6.84E-06 | | 0.00675 | 0.001419 |
| rs399032 | 10 | 55325442 | NA | 6.84E-06 | | 0.00675 | 0.001419 |
| 10:55325458:G:C | 10 | 55325458 | NA | 6.84E-06 | NA | 0.00675 | 0.001419 |
| 10:55325459:G:T | 10 | 55325459 | NA | 6.84E-06 | NA | 0.00675 | 0.001419 |
| 10:55325460:A:G | 10 | 55325460 | NA | 6.84E-06 | NA | 0.00675 | 0.001419 |
| rs57606495 | 10 | 55325466 | NA | 6.84E-06 | NA | 0.00675 | 0.001419 |
| rs34789289 | 10 | 55325562 | NA | 6.84E-06 | | 0.00675 | 0.001419 |
| rs392668 | 10 | 55325629 | NA | 6.84E-06 | | 0.00675 | 0.001419 |
| 10:55325657:T: TAA | 10 | 55325657 | NA | 6.84E-06 | NA | 0.00675 | 0.001419 |
| rs451452 | 10 | 55325763 | NA | 6.84E-06 | | 0.00675 | 0.001419 |
| rs401333 | 10 | 55325803 | NA | 6.84E-06 | | 0.00675 | 0.001419 |
| rs381979 | 10 | 55325986 | NA | 6.84E-06 | | 0.00675 | 0.001419 |
| rs364694 | 10 | 55326079 | NA | 6.84E-06 | | 0.00675 | 0.001419 |
| rs428127 | 10 | 55326235 | NA | 6.84E-06 | | 0.00675 | 0.001419 |
| rs376278 | 10 | 55326251 | NA | 6.84E-06 | | 0.00675 | 0.001419 |
| rs417712 | 10 | 55326306 | NA | 6.84E-06 | | 0.00675 | 0.001419 |
| rs59540423 | 10 | 55326321 | NA | 6.84E-06 | NA | 0.00675 | 0.001419 |
| rs11003681 | 10 | 55326332 | NA | 6.84E-06 | | 0.00675 | 0.001419 |

(continued)

| SNP ID | chr | position | gene locus | p-value | SNP location in gene locus | BETA | SE |
|---|---|---|---|---|---|---|---|
| rs11003682 | 10 | 55326343 | NA | 6.84E-06 | | 0.00675 | 0.001419 |
| rs66668165 | 10 | 55326413 | NA | 6.84E-06 | | 0.00675 | 0.001419 |
| rs61859906 | 10 | 55326428 | NA | 6.84E-06 | | 0.00675 | 0.001419 |
| rs61859907 | 10 | 55326465 | NA | 6.84E-06 | | 0.00675 | 0.001419 |
| rs61859908 | 10 | 55326470 | NA | 6.84E-06 | | 0.00675 | 0.001419 |
| 10:55326554:T:TGA | 10 | 55326554 | NA | 6.84E-06 | NA | 0.00675 | 0.001419 |
| rs34095257 | 10 | 55326663 | NA | 6.84E-06 | | 0.00675 | 0.001419 |
| rs35891588 | 10 | 55326698 | NA | 6.84E-06 | | 0.00675 | 0.001419 |
| rs34632242 | 10 | 55326715 | NA | 6.84E-06 | | 0.00675 | 0.001419 |

[Table 3-51]

| SNP ID | chr | position | gene locus | p-value | SNP location in gene locus | BETA | SE |
|---|---|---|---|---|---|---|---|
| rs35424889 | 10 | 55326737 | NA | 6.84E-06 | | 0.00675 | 0.001419 |
| rs66678299 | 10 | 55326829 | NA | 6.84E-06 | | 0.00675 | 0.001419 |
| 10:55326858 : GCAAAGAGTTCATGAC:G | 10 | 55326858 | NA | 6.84E-06 | NA | 0.00675 | 0.001419 |
| 10:55326914:AGAG:A | 10 | 55326914 | NA | 6.84E-06 | NA | 0.00675 | 0.001419 |
| rs7898607 | 10 | 55326930 | NA | 6.84E-06 | | 0.00675 | 0.001419 |
| rs7898708 | 10 | 55326954 | NA | 6.84E-06 | | 0.00675 | 0.001419 |
| rs598529 | 10 | 55327033 | NA | 6.84E-06 | | 0.00675 | 0.001419 |
| rs598536 | 10 | 55327042 | NA | 6.84E-06 | | 0.00675 | 0.001419 |
| 10:55327064:A:G | 10 | 55327064 | NA | 6.84E-06 | NA | 0.00675 | 0.001419 |
| 10:55327072:GAA:G | 10 | 55327072 | NA | 6.84E-06 | NA | 0.00675 | 0.001419 |

(continued)

| SNP ID | chr | position | gene locus | p-value | SNP location in gene locus | BETA | SE |
|---|---|---|---|---|---|---|---|
| rs674159 | 10 | 55327158 | NA | 6.84E-06 | | 0.00675 | 0.001419 |
| rs409369 | 10 | 55327195 | NA | 6.84E-06 | | 0.00675 | 0.001419 |
| rs55653225 | 10 | 55327405 | NA | 6.84E-06 | | 0.00675 | 0.001419 |
| rs55712224 | 10 | 55327472 | NA | 6.84E-06 | | 0.00675 | 0.001419 |
| rs418047 | 10 | 55327526 | NA | 6.84E-06 | | 0.00675 | 0.001419 |
| 10:55327577:G:A | 10 | 55327577 | NA | 6.84E-06 | NA | 0.00675 | 0.001419 |
| rs401407 | 10 | 55327799 | NA | 6.84E-06 | | 0.00675 | 0.001419 |
| rs2632535 | 10 | 55327835 | NA | 6.84E-06 | | 0.00675 | 0.001419 |
| rs637714 | 10 | 55327902 | NA | 6.84E-06 | | 0.00675 | 0.001419 |
| rs1762109 | 10 | 55327985 | NA | 6.84E-06 | | 0.00675 | 0.001419 |
| rs142344752 | 10 | 55328020 | NA | 6.84E-06 | | 0.00675 | 0.001419 |
| rs659421 | 10 | 55328188 | NA | 6.84E-06 | | 0.00675 | 0.001419 |
| rs636340 | 10 | 55328236 | NA | 6.84E-06 | | 0.00675 | 0.001419 |
| rs659014 | 10 | 55328271 | NA | 6.84E-06 | | 0.00675 | 0.001419 |
| 10:55328288:T:A | 10 | 55328288 | NA | 6.84E-06 | NA | 0.00675 | 0.001419 |
| rs658983 | 10 | 55328294 | NA | 6.84E-06 | | 0.00675 | 0.001419 |
| rs4628595 | 10 | 55328376 | NA | 6.84E-06 | | 0.00675 | 0.001419 |
| rs392293 | 10 | 55328466 | NA | 6.84E-06 | | 0.00675 | 0.001419 |
| rs454270 | 10 | 55328737 | NA | 6.84E-06 | | 0.00675 | 0.001419 |
| rs414849 | 10 | 55328752 | NA | 6.84E-06 | | 0.00675 | 0.001419 |
| rs365966 | 10 | 55328774 | NA | 6.84E-06 | | 0.00675 | 0.001419 |
| rs404593 | 10 | 55328985 | NA | 6.84E-06 | | 0.00675 | 0.001419 |

(continued)

| SNP ID | chr | position | gene locus | p-value | SNP location in gene locus | BETA | SE |
|---|---|---|---|---|---|---|---|
| rs409814 | 10 | 55328990 | NA | 6.84E-06 | | 0.00675 | 0.001419 |
| 10:55329057:AC:A | 10 | 55329057 | NA | 6.84E-06 | NA | 0.00675 | 0.001419 |
| rs368703 | 10 | 55329112 | NA | 6.84E-06 | | 0.00675 | 0.001419 |

[Table 3-52]

| SNP ID | chr | position | gene locus | p-value | SNP location in gene locus | BETA | SE |
|---|---|---|---|---|---|---|---|
| rs447549 | 10 | 55329255 | NA | 6.84E-06 | | 0.00675 | 0.001419 |
| rs368110 | 10 | 55329416 | NA | 6.84E-06 | | 0.00675 | 0.001419 |
| rs374009 | 10 | 55329487 | NA | 6.84E-06 | | 0.00675 | 0.001419 |
| rs436403 | 10 | 55329513 | NA | 6.84E-06 | | 0.00675 | 0.001419 |
| rs452019 | 10 | 55329523 | NA | 6.84E-06 | | 0.00675 | 0.001419 |
| rs427201 | 10 | 55329632 | NA | 6.84E-06 | | 0.00675 | 0.001419 |
| rs433582 | 10 | 55329954 | NA | 6.84E-06 | | 0.00675 | 0.001419 |
| rs433574 | 10 | 55329961 | NA | 6.84E-06 | | 0.00675 | 0.001419 |
| rs441336 | 10 | 55329964 | NA | 6.84E-06 | | 0.00675 | 0.001419 |
| rs427372 | 10 | 55330380 | NA | 6.84E-06 | | 0.00675 | 0.001419 |
| rs380989 | 10 | 55330434 | NA | 6.84E-06 | | 0.00675 | 0.001419 |
| rs17695283 | 10 | 55332545 | NA | 5.32E-06 | | 0.007515 | 0.001559 |
| rs444186 | 10 | 55334166 | NA | 6.84E-06 | | 0.00675 | 0.001419 |
| rs10996833 | 10 | 67784976 | CTNNA3 | 8.74E-07 | intron_variant | 0.02473 | 0.004704 |
| rs34539937 | 10 | 67787534 | CTNNA3 | 8.74E-07 | intron_variant | 0.02473 | 0.004704 |
| rs35716649 | 10 | 67788443 | CTNNA3 | 8.74E-07 | intron_variant | 0.02473 | 0.004704 |
| rs12571522 | 10 | 67790052 | CTNNA3 | 8.74E-07 | intron_variant | 0.02473 | 0.004704 |
| rs35744226 | 10 | 67803006 | CTNNA3 | 8.74E-07 | intron_variant | 0.02473 | 0.004704 |
| rs12778483 | 10 | 67805050 | CTNNA3 | 8.74E-07 | intron_variant | 0.02473 | 0.004704 |
| rs17243463 | 10 | 67805413 | CTNNA3 | 8.74E-07 | intron_variant | 0.02473 | 0.004704 |
| rs75400168 | 10 | 67834004 | CTNNA3 | 8.74E-07 | intron_variant | 0.02473 | 0.004704 |
| rs71493960 | 10 | 67835457 | CTNNA3 | 8.74E-07 | intron_variant | 0.02473 | 0.004704 |
| rs35053158 | 10 | 67841299 | CTNNA3 | 1.02E-05 | intron_variant | 0.01964 | 0.004217 |
| rs35376201 | 10 | 67842582 | CTNNA3 | 1.02E-05 | intron_variant | 0.01964 | 0.004217 |
| rs67242129 | 10 | 67842799 | NA | 1.02E-05 | NA | 0.01964 | 0.004217 |
| rs1247787 | 10 | 78830256 | KCNMA1 | 1.65E-O5 | intron_variant | 0.008385 | 0.001849 |
| rs1247779 | 10 | 78839034 | KCNMA1 | 5.44E-06 | intron_variant | 0.008992 | 0.001868 |
| rs2258870 | 10 | 78840266 | KCNMA1 | 1.65E-05 | intron_variant | 0.008385 | 0.001849 |
| rs10762747 | 10 | 78842860 | KCNMA1 | 1.54E-05 | intron_variant | 0.006863 | 0.001508 |

(continued)

| SNP ID | chr | position | gene locus | p-value | SNP location in gene locus | BETA | SE |
|---|---|---|---|---|---|---|---|
| rs2574789 | 10 | 78851342 | KCNMA1 | 1.54E-05 | intron_variant | 0.006863 | 0.001508 |
| rs74462085 | 10 | 78856298 | KCNMA1 | 3.49E-05 | intron_variant | 0.01407 | 0.003241 |
| rs80058374 | 10 | 78859025 | KCNMA1 | 2.78E-06 | intron_variant | 0.007806 | 0.001568 |
| rs79411761 | 10 | 78861868 | KCNMA1 | 2.78E-06 | intron_variant | 0.007806 | 0.001568 |
| rs2574791 | 10 | 78864094 | KCNMA1 | 4.93E-05 | intron_variant | 0.006743 | 0.001587 |
| rs2574787 | 10 | 78883564 | KCNMA1 | 4.73E-05 | intron_variant | 0.006157 | 0.001445 |
| rs4980128 | 10 | 78886557 | KCNMA1 | 3.52E-05 | intron_variant | 0.006441 | 0.001485 |

[Table 3-53]

| SNP ID | chr | position | gene locus | p-value | SNP location in gene locus | BETA | SE |
|---|---|---|---|---|---|---|---|
| rs61574126 | 10 | 80740616 | NA | 2.92E-05 | | 0.01105 | 0.002518 |
| rs60394423 | 10 | 80742406 | NA | 2.92E-05 | | 0.01105 | 0.002518 |
| rs77451861 | 10 | 80743419 | NA | 2.92E-05 | | 0.01105 | 0.002518 |
| rs116906077 | 10 | 80755918 | NA | 1.79E-05 | | 0.01229 | 0.002723 |
| rs78685078 | 10 | 80755928 | NA | 1.79E-05 | | 0.01229 | 0.002723 |
| rs117213013 | 10 | 81685744 | NA | 3.97E-06 | | 0.02353 | Q.004811 |
| rs7089837 | 10 | 85433827 | NA | 5.79E-06 | | 0.02315 | 0.004824 |
| 10:85434294: T:A | 10 | 85434294 | NA | 5.79E-06 | NA | 0.02315 | 0.004824 |
| rs115787429 | 10 | 85436477 | NA | 5.79E-06 | | 0.02315 | 0.004824 |
| rs17102402 | 10 | 85436920 | NA | 5.79E-06 | | 0.02315 | 0.004824 |
| rs149846651 | 10 | 85437351 | NA | 5.79E-06 | | 0.02315 | 0.004824 |
| rs74773350 | 10 | 99321878 | UBTD1 | 2.19E-06 | intron_variant | 0.02908 | 0.005774 |
| 10:112855651 :T:C | 10 | 112855651 | NA | 1.04E-07 | NA | 0.03229 | 0.005617 |
| rs75665088 | 10 | 116038167 | VWA2 | 2.36E-05 | intron_variant | 0.0219 | 0.004928 |
| rs10736246 | 10 | 117982524 | GFRA1 | 4.15E-05 | intron_variant | 0.00707 | 0.001646 |
| rs7094346 | 10 | 117986207 | GFRA1 | 3.29E-05 | intronvariant | 0.007208 | 0.001655 |
| rs4628599 | 10 | 117986457 | GFRA1 | 4.15E-05 | intron_variant | 0.00707 | 0.001646 |
| rs141923679 | 10 | 118354939 | PNLIPRP1 | 7.03E-06 | intron_variant | 0.02301 | 0.004844 |
| rs59679890 | 10 | 118636461 | EN04 | 5.59E-06 | intron_variant | 0.02836 | 0.005899 |
| rs72631124 | 10 | 125679317 | CPXM2 | 1.09E-05 | intron_variant | 0.01353 | 0.002917 |
| rs72631125 | 10 | 125679694 | CPXM2 | 1.09E-05 | intron_variant | 0.01353 | 0.002917 |
| rs72631126 | 10 | 125679714 | CPXM2 | 1.09E-05 | intron_variant | 0.01353 | 0.002917 |
| rs72631127 | 10 | 125680048 | CPXM2 | 1.09E-05 | intron_variant | 0.01353 | 0.002917 |
| rs72631128 | 10 | 125680075 | CPXM2 | 1.09E-05 | intron_variant | 0.01353 | 0.002917 |
| rs182795662 | 10 | 125680804 | CPXM2 | 1.09E-05 | intron_variant | 0.01353 | 0.002917 |
| rs186918206 | 10 | 125680805 | CPXM2 | 1.09E-05 | intron_variant | 0.01353 | 0.002917 |
| rs72631129 | 10 | 125680924 | CPXM2 | 1.09E-05 | intron_variant | 0.01353 | 0.002917 |

(continued)

| SNP ID | chr | position | gene locus | p-value | SNP location in gene locus | BETA | SE |
|---|---|---|---|---|---|---|---|
| rs72631130 | 10 | 125681159 | CPXM2 | 1.09E-05 | intron_variant | 0.01353 | 0.002917 |
| 10:125681455 :T:CC | 10 | 125681455 | NA | 1.09E-05 | NA | 0.01353 | 0.002917 |
| rs115841965 | 10 | 125681633 | CPXM2 | 1.09E-05 | intron_variant | 0.01353 | 0.002917 |
| rs72631132 | 10 | 125682219 | CPXM2 | 1.09E-05 | intron_variant | 0.01353 | 0.002917 |
| rs72631134 | 10 | 125682486 | CPXM2 | 1.09E-05 | intron_variant | 0.01353 | 0.002917 |
| rs142639355 | 10 | 125682701 | CPXM2 | 1.09E-05 | intron_variant | 0.01353 | 0.002917 |
| rs115903046 | 10 | 125682823 | CPXM2 | 1.09E-05 | intron_variant | 0.01353 | 0.002917 |
| rs140301692 | 10 | 125682862 | CPXM2 | 1.09E-05 | intron_variant | 0.01353 | 0.002917 |
| rs149963239 | 10 | 125727519 | NA | 9.05E-06 | | 0.01432 | 0.003055 |

[Table 3-54]

| SNP ID | chr | position | gene locus | p-value | SNP location in gene locus | BETA | SE |
|---|---|---|---|---|---|---|---|
| rs143323962 | 10 | 125728098 | NA | 9.05E-06 | | 0.01432 | 0.003055 |
| rs11594441 | 10 | 130456079 | NA | 8.40E-07 | | 0.007871 | 0.001495 |
| rs3847456 | 10 | 130457648 | NA | 8.40E-07 | | 0.007871 | 0.001495 |
| rs3847457 | 10 | 130457732 | NA | 8.4DE-07 | | 0.007871 | 0.001495 |
| rs72847142 | 10 | 130458683 | NA | 4.97E-07 | | 0.008093 | 0.001502 |
| rs72280102 | 10 | 130460388 | NA | 1.38E-06 | | 0.00878 | 0.001106 |
| rs11598828 | 10 | 130463073 | NA | 4.97E-07 | | 0.008093 | 0.001502 |
| rs11598671 | 10 | 130466623 | NA | 9.54E-08 | | 0.008854 | 0.001535 |
| rs1475391 | 10 | 130474188 | NA | 7.18E-08 | | 0.008854 | 0.001518 |
| rs112036204 | 10 | 130475046 | NA | 1.05E-05 | | 0.007401 | 0.001592 |
| rs113908408 | 11 | 7845074 | NA | 4.71E-07 | | 0.03074 | 0.00569 |
| rs112933444 | 11 | 7845341 | NA | 4.71E-07 | | 0.03074 | 0.00569 |
| rs112043976 | 11 | 7845400 | NA | 4.71E-07 | | 0.03074 | 0.00569 |
| rs73408430 | 11 | 7855420 | NA | 4.71E-07 | | 0.03074 | 0.00569 |
| rs73408442 | 11 | 7857655 | NA | 4.71E-07 | | 0.03074 | 0.00569 |
| rs73408450 | 11 | 7858446 | NA | 4.71E-07 | | 0.03074 | 0.00569 |
| rs73408454 | 11 | 7859334 | NA | 4.71E-07 | | 0.03074 | 0.00569 |
| rs79725644 | 11 | 7861967 | NA | 4.71E-07 | | 0.03074 | 0.00569 |
| rs58454329 | 11 | 7864544 | NA | 4.71E-07 | | 0.03074 | 0.00569 |
| rs369143 | 11 | 7865713 | NA | 4.71E-07 | | 0.03074 | 0.00569 |
| rs57721290 | 11 | 7867024 | NA | 4.71E-07 | | 0.03074 | 0.00569 |
| 11:7868688:AT:A | 11 | 7868688 | NA | 4.71E-07 | NA | 0.03074 | 0.00569 |
| rs11606178 | 11 | 7872593 | NA | 4.71E-07 | | 0.03074 | 0.00569 |
| rs77771703 | 11 | 7879250 | NA | 4.71E-07 | | 0.03074 | 0.00569 |
| rs79195468 | 11 | 7897753 | NA | 4.71E-07 | | 0.03074 | 0.00569 |

(continued)

| SNP ID | chr | position | gene locus | p-value | SNP location in gene locus | BETA | SE |
|---|---|---|---|---|---|---|---|
| 11:8797713:A:G | 11 | 8797713 | NA | 1.10E-05 | NA | 0.01775 | 0.003826 |
| rs11042070 | 11 | 8819367 | DENND2B | 8.74E-07 | intron_ variant | 0.02473 | 0.004704 |
| rs142465660 | 11 | 8821504 | NA | 8.74E-07 | NA | 0.02473 | 0.004704 |
| rs4929915 | 11 | 8824418 | DENND2B | 8.74E-07 | intron_ variant | 0.02473 | 0.004704 |
| rs17337866 | 11 | 8827750 | DENND2B | 8.74E-07 | intron_ variant | 0.02473 | 0.004704 |
| rs3915868 | 11 | 8833800 | DENND2B | 8.74E-07 | intron_ variant | 0.02473 | 0.004704 |
| rs34023999 | 11 | 8839379 | DENND2B | 8.74E-07 | intron_ variant | 0.02473 | 0.004704 |
| rs36094920 | 11 | 8845266 | DENND2B | 8.74E-07 | intron_ variant | 0.02473 | 0.004704 |
| rs7127197 | 11 | 0845607 | DENND2B | 8.74E-07 | intron_ variant | 002473 | 0.004704 |
| rs35503692 | 11 | 8848785 | DENND2B | 8.74E-07 | intron_ variant | 0.02473 | 0.004704 |
| rs34033747 | 11 | 8853774 | DENND2B | 1.91E-09 | intron_ variant | 0.02547 | 0.003843 |

[Table 3-55]

| SNP ID | chr | position | gene locus | p-value | SNP location in gene locus | BETA | SE |
|---|---|---|---|---|---|---|---|
| 11:8854132:A : ACTGCG | 11 | 8854132 | NA | 1.91E-09 | NA | 0.02547 | 0.003843 |
| rs34418236 | 11 | 8859656 | DENND2B | 2.67E-05 | intron_variant _ | 0.01448 | 0.003283 |
| rs77784346 | 11 | 8998278 | NA | 6.68E-06 | | 0.00642 | 0.001348 |
| rs149624805 | 11 | 12188071 | MICAL2 | 1.04E-07 | intron_variant | 0.03229 | 0.005617 |
| rs118093648 | 11 | 18162065 | NA | 2.50E-05 | | 0.01885 | 0.004257 |
| rs7120135 | 11 | 24831117 | LUZP2 | 2.27E-05 | intron_variant | 0.01903 | 0.004275 |
| rs61877058 | 11 | 26600213 | AN03 | 2.14E-05 | intron_variant | 0.02201 | 0.004926 |
| rs61877059 | 11 | 26604143 | AN03 | 2.14E-05 | intron_variant | 0.02201 | 0.004926 |
| 11:26605036: C: T | 11 | 26605036 | NA | 2.14E-05 | NA | 0.02201 | 0.004926 |
| rs17243461 | 11 | 26607661 | AN03 | 2.14E-05 | intron_variant | 0.02201 | 0.004926 |
| rs192211136 | 11 | 26608276 | AN03 | 2.14E-05 | intron_variant | 0.02201 | 0.004926 |
| rs11243468 | 11 | 26608776 | AN03 | 2.14E-05 | intron_variant | 0.02201 | 0.004926 |
| 11:26610935: G: GA | 11 | 26610935 | NA | 2.14E-05 | NA | 0.02201 | 0.004926 |
| rs61878565 | 11 | 26611476 | AN03 | 214E-05 | intron_variant | 0.02201 | 0.004926 |
| rs6187856fi | 11 | 26612275 | AN03 | 2.14E-05 | intron_variant | 0.02201 | 0.004926 |
| rs61878567 | 11 | 26613230 | AN03 | 2.14E-05 | intron_variant | 0.02201 | 0.004926 |
| rs61878568 | 11 | 26613870 | AN03 | 2.14E-05 | intron_variant | 0.02201 | 0.004926 |
| rs61878572 | 11 | 26616967 | AN03 | 2.14E-05 | intron_variant | 0.02201 | 0.004926 |
| rs61878588 | 11 | 26618785 | AN03 | 2.14E-05 | intron_variant | 0.02201 | 0.004926 |
| rs61878589 | 11 | 26618960 | AN03 | 2.14E-05 | intron_variant | 0.02201 | 0.004926 |
| rs200761473 | 11 | 26625591 | AN03 | 2.14E-05 | intron_variant | 0.02201 | 0.004926 |

(continued)

| SNP ID | chr | position | gene locus | p-value | SNP location in gene locus | BETA | SE |
|---|---|---|---|---|---|---|---|
| rs61878592 | 11 | 26625601 | AN03 | 2.14E-05 | intron_variant | 0.02201 | 0.004926 |
| rs36063975 | 11 | 35574891 | NA | 2.18E-05 | | 0.006145 | 0.001377 |
| rs12798613 | 11 | 35574908 | NA | 2.18E-05 | | 0.006145 | 0.001377 |
| rs10836415 | 11 | 35575254 | NA | 4.21E-05 | | 0.005086 | 0.001185 |
| rs59100957 | 11 | 35575495 | NA | 3.49E-05 | | 0.00602 | 0.001387 |
| rs12420268 | 11 | 35576512 | NA | 4.21E-05 | | 0.005086 | 0.001185 |
| rs144733009 | 11 | 44566820 | NA | 4.57E-05 | | 0.02085 | 0.004883 |
| rs2045018 | 11 | 44843041 | TSPAN18 | 1.84E-05 | intron_variant | 0.02192 | 0.004864 |
| rs11230471 | 11 | 60539384 | MS4A15 | 3.95E-05 | intron_variant | 0.01877 | 0.004356 |
| rs142518783 | 11 | 62237951 | AHNAK | 1.55E-05 | intron_variant | 0.02217 | 0.004872 |
| rs12281345 | 11 | 62773213 | SLC22A8 | 1.04E-07 | intronvariant | 0.03229 | 0.005617 |
| rs12808517 | 11 | 72601276 | FCHSD2 | 8.46E-06 | intron_variant | 0.01984 | 0.004217 |
| rs150783164 | 11 | 74983240 | ARRB1 | 2.76E-05 | intron_variant | 0.02139 | 0.00486 |
| rs78089535 | 11 | 79215837 | NA | 1.72E-05 | | 0.02707 | 0.005982 |
| rs112798455 | 11 | 79216359 | NA | 1.72E-05 | | 0.02707 | 0.005982 |

[Table 3-56]

| SNP ID | ch r | position | gene locus | p-value | SNP location in gene locus | BETA | SE |
|---|---|---|---|---|---|---|---|
| 11:79243641 : AT:A | 11 | 79243641 | NA | 1.72E-05 | NA | 0.02707 | 0.005982 |
| rs74659625 | 11 | 79250136 | NA | 1.72E-05 | | 0.02707 | 0.005982 |
| rs7628009Q | 11 | 79250154 | NA | 1.72E-05 | | 0.02707 | 0.005982 |
| rs147247218 | 11 | 84483667 | DLG2 | 1.04E-07 | intron_variant | 0.03229 | 0.005617 |
| rs139305706 | 11 | 89858108 | NA | 3.74E-05 | | 0.02123 | 0.004913 |
| rs149012812 | 11 | 90175103 | NA | 3.74E-05 | | 0.02123 | 0.004913 |
| rs17860938 | 11 | 102652744 | WTAPP1 | 3.44E-06 | non_coding_ transcript_ variant | 0.02077 | 0.004217 |
| rs72977553 | 11 | 102653834 | WTAPP1 | 3.44E-06 | non_coding_ transcript_ variant | 0.02077 | 0.004217 |
| rs7112080 | 11 | 102655053 | WTAPP1 | 3.44E-06 | non_coding_ transcript_ variant | 0.02077 | 0.004217 |
| rs7933558 | 11 | 102655137 | WTAPP1 | 3.44E-06 | non_coding_ transcript_ variant | 0.02077 | 0.004217 |
| rs72977568 | 11 | 102657657 | WTAPP1 | 3.44E-06 | non_coding_ transcript_ variant | 0.02077 | 0.004217 |

(continued)

| SNP ID | ch r | position | gene locus | p-value | SNP location in gene locus | BETA | SE |
|---|---|---|---|---|---|---|---|
| rs187059006 | 11 | 102658035 | WTAPP1 | 3.44E-06 | non_coding_ transcriptvariant | 0.02077 | 0.004217 |
| rs11390663 | 11 | 102660054 | WTAPP1 | 3.44E-06 | non_coding _transcript_ variant | 0.02077 | 0.004217 |
| rs7945189 | 11 | 102660564 | WTAPP1 | 3.44E-06 | non_coding_ transcript_ variant | 0.02077 | 0.004217 |
| rs17878905 | 11 | 102661757 | MMP1 | 3.44E-06 | intron_variant | 0.02077 | 0.004217 |
| rs178$2844 | 11 | 102661813 | MMP1 | 3.44E-06 | intron_variant | 0.02077 | 0.004217 |
| rs146555295 | 11 | 105747176 | NA | 1.91E-05 | NA | 0.005895 | 0.001311 |
| rs12419614 | 11 | 105840184 | GRIA4 | 8.51E-06 | intronvariant | 0.006329 | 0.001346 |
| rs111988538 | 11 | 107103114 | NA | 1.90E-08 | | 0.01674 | 0.002731 |
| rs78496161 | 11 | 107148735 | NA | 2.73E-05 | | 0.009222 | 0.002094 |
| 11:10715103 1: A:AT | 11 | 107151031 | NA | 7.38E-07 | NA | 0.008734 | 0.001649 |
| rs79062526 | 11 | 107153318 | NA | 7.38E-07 | | 0.008734 | 0.001649 |
| rs76404176 | 11 | 107157966 | NA | 6.34E-06 | | 0.007565 | 0.001584 |
| rs17106711 | 11 | 107158740 | NA | 4.50E-05 | | 0.007238 | 0.001694 |
| rs7943234 | 11 | 107165710 | NA | 9.01E-07 | | 0.008734 | 0.001664 |
| rs78568149 | 11 | 107222059 | CWF19L2 | 3.85E-05 | intron_variant | 0.01079 | 0.002501 |
| rs74346769 | 11 | 107226643 | CWF19L2 | 3.85E-05 | intron_variant | 0.01079 | 0.002501 |
| rs118Q53355 | 11 | 107328818 | NA | 6.93E-06 | | 0.01768 | 0.003719 |
| rs17107015 | 11 | 107353313 | NA | 1.07E-05 | | 0.01306 | 0.002811 |
| rs7937106 | 11 | 107354312 | NA | 6.93E-06 | | 0.01768 | 0.003719 |
| rs80050705 | 11 | 107354858 | NA | 6.93E-06 | | 0.01768 | 0.003719 |
| rs77739772 | 11 | 107356982 | NA | 1.07E-05 | | 0.01306 | 0.002811 |
| rs201645325 | 11 | 107357860 | NA | 1.07E-05 | NA | 0.01306 | 0.002811 |
| rs78482726 | 11 | 107362733 | NA | 4.52E-05 | | 0.01474 | 0.003449 |
| rs149277905 | 11 | 107364050 | NA | 4.52E-05 | | 0.01474 | 0.003449 |
| rs12417562 | 11 | 107364413 | NA | 4.52E-05 | | 0.01474 | 0.003449 |

[Table 3-57]

| SNP ID | ch r | position | gene locus | p-value | SNP location in gene locus | BETA | SE |
|---|---|---|---|---|---|---|---|
| rs115051850 | 11 | 117280512 | CEP164 | 2.72E-07 | synonymous_ variant | 0.03139 | 0.005677 |
| rs61743854 | 11 | 117280517 | CEP164 | 2.72E-07 | missense_ variant | 0.03139 | 0.005677 |

(continued)

| SNP ID | ch r | position | gene locus | p-value | SNP location in gene locus | BETA | SE |
|---|---|---|---|---|---|---|---|
| rs111910740 | 11 | 119625360 | NA | 8.52E-06 | | 0.02781 | 0.005915 |
| rs140081101 | 11 | 119625819 | NA | 8.52E-06 | | 0.02781 | 0.005915 |
| rs148275870 | 11 | 119630952 | NA | 8.52E-06 | | 0.02781 | 0.005915 |
| rs115832414 | 11 | 119656930 | NA | 8.52E-06 | | 0.02781 | 0.005915 |
| rs146686876 | 11 | 119659763 | NA | 8.52E-06 | | 0.02781 | 0.005915 |
| rs117150747 | 11 | 126994608 | NA | 4.83E-05 | | 0.02078 | 0.004884 |
| rs78730731 | 11 | 127031263 | NA | 8.15E-06 | | 0.01638 | 0.003476 |
| rs1944828 | 11 | 127048359 | NA | 7.66E-06 | | 0.01112 | 0.002351 |
| rs2513442 | 11 | 127053597 | NA | 7.66E-06 | | 0.01112 | 0.002351 |
| 12:7859133:G:A | 12 | 7859133 | NA | 2.16E-06 | NA | 0.005248 | 0.001041 |
| rs17834986 | 12 | 15261437 | RERG | 2.15E-05 | 3_prime_ UTR_varian t | 0.01078 | 0.002413 |
| rs111641368 | 12 | 26800096 | 1TPR2 | 3.72E-05 | intron_variant | 0.02107 | 0.004874 |
| rs140725931 | 12 | 26802734 | 1TPR2 | 3.72E-05 | intron_variant | 0.02107 | 0.004874 |
| rs112815221 | 12 | 26829320 | 1TPR2 | 3.72E-05 | intron_variant | 0.02107 | 0.004874 |
| rs74849908 | 12 | 26836556 | 1TPR2 | 3.72E-05 | intron_variant | 0.02107 | 0.004874 |
| rs76630621 | 12 | 26841931 | 1TPR2 | 3.72E-05 | intron_variant | 0.02107 | 0.004874 |
| 12:28124654:A:G | 12 | 28124654 | NA | 4.71E-07 | NA | 0.03074 | 0.00569 |
| rs61918684 | 12 | 29635132 | 0VCH1 | 6.11E-06 | intron_variant | 0.02291 | 0.004788 |
| rs61922198 | 12 | 29779895 | TMTC1 | 1.24E-06 | non_coding_ transcript_ variant | 0.01086 | 0.002099 |
| rs1105Q326 | 12 | 29782241 | TMTC1 | 1.24E-06 | non_coding_ transcript_ variant | 0.01086 | 0.002099 |
| rs12300501 | 12 | 29782247 | TMTC1 | 1.24E-06 | non_coding_ transcript_ variant | 0.01086 | 0.002099 |
| rs11050329 | 12 | 29786413 | TMTC1 | 4.35E-07 | non_coding_ transcript_ variant | 0.01107 | 0.002042 |
| 12:29788125:C:CA | 12 | 29788125 | NA | 4.35E-07 | NA | 0.01107 | 0.002042 |
| rs10771557 | 12 | 29788245 | TMTC1 | 4.35E-07 | non_coding_ transcript_ variant | 0.01107 | 0.002042 |
| rs11050333 | 12 | 29789329 | TMTC1 | 4.35E-07 | non_coding_ transcript_ variant | 0.01107 | 0.002042 |
| rs10843455 | 12 | 29789750 | TMTC1 | 9.83E-08 | non_coding_ transcript_ variant | 0.0106 | 0.001839 |

(continued)

| SNP ID | ch r | position | gene locus | p-value | SNP location in gene locus | BETA | SE |
|---|---|---|---|---|---|---|---|
| rs10843457 | 12 | 29790399 | TMTC1 | 7.98E-08 | non_coding_ transcript_ variant | 0.009364 | 0.001612 |
| rs11050334 | 12 | 29790982 | TMTC1 | 7.98E-08 | non_coding_ transcript_ variant | 0.009364 | 0.001612 |
| rs12827042 | 12 | 29791219 | TMTC1 | 7.98E-08 | non_coding_ transcript_ variant | 0.009364 | 0.001612 |
| rs12826106 | 12 | 29791229 | TMTC1 | 798E-08 | non_coding_ transcript_ variant | 0.009364 | 0.001612 |
| rs1972681 | 12 | 29791455 | TMTC1 | 798E-08 | non_coding_ transcript_ variant | 0.009364 | 0.001612 |
| rs1972682 | 12 | 29791633 | TMTC1 | 798E-08 | non_coding_ transcript_ variant | 0.009364 | 0.001612 |
| rs1549408 | 12 | 29791646 | TMTC1 | 7.98E-08 | non_coding_ transcript_ variant | 0.009364 | 0.001612 |
| rs1549409 | 12 | 29791672 | TMTC1 | 7.98E-08 | non_coding_ transcript_ variant | 0.009364 | 0.001612 |

[Table 3-58]

| SNP ID | ch r | position | gene locus | p-value | SNP location in gene locus | BETA | SE |
|---|---|---|---|---|---|---|---|
| rs1549410 | 12 | 29791697 | TMTC1 | 7.98E-08 | non_coding_ transcript_variant | 0.009364 | 0.001612 |
| rs10843458 | 12 | 29791936 | TMTC1 | 3.18E-05 | non_coding_ transcript_variant | 0.006018 | 0.001379 |
| rs7955768 | 12 | 29792069 | TMTC1 | 4.71E-07 | non_coding_ transcript_variant | 0.03074 | 0.00569 |
| rs10771558 | 12 | 29792233 | TMTC1 | 3.18E-05 | non_coding_ transcript_variant | 0.006018 | 0.001379 |
| rs34265384 | 12 | 29792497 | TMTC1 | 7.98E-08 | non_coding_ transcript_variant | 0.009364 | 0.001612 |
| rs1549411 | 12 | 29793140 | TMTC1 | 5.82E-07 | non_coding_ transcript_variant | 0.009218 | 0.001722 |
| rs113386809 | 12 | 29794462 | TMTC1 | 4.71E-07 | non_coding_ transcript_variant | 0.03074 | 0.00569 |
| rs113094413 | 12 | 29795584 | TMTC1 | 4.71E-07 | non_coding_ transcript_variant | 0.03074 | 0.00569 |

(continued)

| SNP ID | chr | position | gene locus | p-value | SNP location in gene locus | BETA | SE |
|---|---|---|---|---|---|---|---|
| rs113767075 | 12 | 29795807 | TMTC1 | 4.71E-07 | non_coding_ transcript_variant | 0.03074 | 0.00569 |
| rs12371181 | 12 | 31193873 | NA | 7.48E-06 | | 0.009285 | 0.001961 |
| rs150392011 | 12 | 31198640 | NA | 7.48E-06 | | 0.009285 | 0.001961 |
| rs11051218 | 12 | 31201717 | NA | 7.48E-06 | | 0.009285 | 0.001961 |
| rs11051219 | 12 | 31201922 | NA | 7.48E-06 | | 0.009285 | 0.001961 |
| rs11051221 | 12 | 31203590 | NA | 7.48E-06 | | 0.009285 | 0.001961 |
| rs11051222 | 12 | 31203920 | NA | 7.48E-06 | | 0.009285 | 0.001961 |
| rs4244855 | 12 | 31213686 | NA | 6.90E-06 | | 0.009012 | 0.001895 |
| rs4931415 | 12 | 31216523 | NA | 6.90E-06 | | 0.009012 | 0.001895 |
| rs4931416 | 12 | 31219161 | NA | 6.90E-06 | | 0.009012 | 0.001895 |
| rs145929359 | 12 | 31222582 | NA | 2.23E-06 | | 0.009578 | 0.001903 |
| 12:31225561: G : GTTAGTGAGA | 12 | 31225561 | NA | 1.22E-06 | NA | 0.009967 | 0.001925 |
| rs2302846 | 12 | 31226930 | DDX11 | 1.22E-06 | 5_primeUTR_ variant | 0.009967 | 0.001925 |
| rs12579271 | 12 | 31230735 | DDX11 | 1.22E-06 | intron_variant | 0.009967 | 0.001925 |
| rs11832720 | 12 | 57257244 | AC121758.2 | 8.71E-06 | intron_variant | 0.009135 | 0.001945 |
| rs75465620 | 12 | 57259691 | AC121758.2 | 8.71E-06 | intron_variant | 0.009135 | 0.001945 |
| rs144935800 | 12 | 57263384 | NA | 8.71E-06 | NA | 0.009135 | 0.001945 |
| rs117991841 | 12 | 57263617 | AC121758.2 | 8.71E-06 | intron_variant | 0.009135 | 0.001945 |
| rs74995587 | 12 | 57264426 | AC121758.2 | 8.71E-06 | intron_variant | 0.009135 | 0.001945 |
| rs4403833 | 12 | 57265921 | AC121758.2 | 8.71E-06 | non_coding_transcr ipt_exon_variant | 0.009135 | 0.001945 |
| rs12810024 | 12 | 57331444 | NA | 3.71E-05 | | 0.008493 | 0.001964 |

(continued)

| SNP ID | ch r | position | gene locus | p-value | SNP location in gene locus | BETA | SE |
|---|---|---|---|---|---|---|---|
| rs200182685 | 12 | 57333170 | NA | 3.71E-05 | | 0.008493 | 0.001964 |
| 12:57335432: A :ATTTA | 12 | 57335432 | NA | 2.66E-05 | NA | 0.008792 | 0.001993 |
| 12:57335432: A :ATTTT | 12 | 57335432 | NA | NA | NA | NA | NA |
| rs143723418 | 12 | 57339182 | NA | 2.47E-05 | | 0.009394 | 0.00212 |
| rs1072670 | 12 | 57342100 | NA | 2.47E-05 | | 0.009394 | 0.00212 |
| rs11836517 | 12 | 57343892 | NA | 2.47E-05 | | 0.009394 | 0.00212 |

[Table 3-59]

| SNP ID | ch r | position | gene locus | p-value | SNP location in gene locus | BETA | SE |
|---|---|---|---|---|---|---|---|
| rs901068 | 12 | 57346805 | RDH16 | 2.47E-05 | intron_variant | 0.009394 | 0.00212 |
| rs746049 | 12 | 57349039 | RDH16 | 2.47E-05 | intron_variant | 0.009394 | 0.00212 |
| rs67771054 | 12 | 57349813 | RDH16 | 2.47E-05 | intron_variant | 0.009394 | 0.00212 |
| rs12815538 | 12 | 57352871 | RDH16 | 2.47E-05 | non_coding_ transcript_exon_ variant | 0.009394 | 0.00212 |
| rs144844809 | 12 | 57353192 | NA | 2.47E-05 | NA | 0.009394 | 0.00212 |
| rs138278919 | 12 | 57353274 | NA | 2.47E-05 | | 0.009394 | 0.00212 |
| rs148154364 | 12 | 57356239 | NA | 2.47E-05 | NA | 0.009394 | 0.00212 |
| rs143470873 | 12 | 57356240 | NA | 2.47E-05 | NA | 0.009394 | 0.00212 |
| rs34253098 | 12 | 57356325 | NA | 2.47E-05 | | 0.009394 | 0.00212 |
| 12:57356830:A: G | 12 | 57356830 | NA | 2.47E-05 | NA | 0.009394 | 0.00212 |
| rs12820005 | 12 | 57356885 | NA | 2.47E-05 | | 0.009394 | 0.00212 |
| rs11837093 | 12 | 57356893 | NA | 2.47E-05 | | 0.009394 | 0.00212 |
| rs79861271 | 12 | 57357106 | NA | 2.47E-05 | | 0.009394 | 0.00212 |
| 12:57357322:A: G | 12 | 57357322 | NA | 2.47E-05 | NA | 0.009394 | 0.00212 |
| rs181056553 | 12 | 57357512 | NA | 2.47E-05 | | 0.009394 | 0.00212 |
| rs77781354 | 12 | 57357721 | NA | 2.47E-05 | | 0.009394 | 0.00212 |
| 12:57357983:G: A | 12 | 57357983 | NA | 2.47E-05 | NA | 0.009394 | 0.00212 |
| 12:57358181:C: G | 12 | 57358181 | NA | 2.47E-05 | NA | 0.009394 | 0.00212 |
| rs703818 | 12 | 57359073 | NA | 2.47E-05 | | 0.009394 | 0.00212 |
| 12:57359261:C: CA | 12 | 57359261 | NA | 2.47E-05 | NA | 0.009394 | 0.00212 |
| rs2888139 | 12 | 57359436 | NA | 2.47E-05 | | 0.009394 | 0.00212 |

(continued)

| SNP ID | ch r | position | gene locus | p-value | SNP location in gene locus | BETA | SE |
|---|---|---|---|---|---|---|---|
| rs12812499 | 12 | 57359643 | NA | 2.47E-05 | | 0.009394 | 0.00212 |
| rs144223544 | 12 | 57360141 | NA | 2.47E-05 | | 0.009394 | 0.00212 |
| rs60380217 | 12 | 57360851 | NA | 2.47E-05 | | 0.009394 | 0.00212 |
| rs1843311 | 12 | 57361414 | NA | 2.47E-05 | | 0.009394 | 0.00212 |
| rs1843312 | 12 | 57361562 | NA | 2.47E-05 | | 0.009394 | 0.00212 |
| rs1843313 | 12 | 57361594 | NA | 2.47E-05 | | 0.009394 | 0.00212 |
| rs2167306 | 12 | 57362345 | NA | 2.47E-05 | | 0.009394 | 0.00212 |
| rs7969312 | 12 | 57362968 | NA | 2.47E-05 | | 0.009394 | 0.00212 |
| rs7956166 | 12 | 57363128 | NA | 2.47E-05 | | 0.009394 | 0.00212 |
| rs7487948 | 12 | 57363907 | NA | 2.47E-05 | | 0.009394 | 0.00212 |
| rs149233794 | 12 | 57364288 | NA | 2.47E-05 | NA | 0.009394 | 0.00212 |
| rs12827286 | 12 | 57365564 | NA | 2.47E-05 | | 0.009394 | 0.00212 |
| 12:57365771:A:AAC | 12 | 57365771 | NA | 1.16E-06 | NA | 0.01115 | 0.002149 |
| rs145602952 | 12 | 59622709 | NA | 1.07E-05 | | 0.02722 | 0.005861 |
| rs142940655 | 12 | 59629178 | NA | 1.07E-05 | | 0.02722 | 0.005861 |

[Table 3-60]

| SNP ID | chr | position | gene locus | p-value | SNP location in gene locus | BETA | SE |
|---|---|---|---|---|---|---|---|
| rs76598008 | 12 | 59709510 | NA | 1.07E-05 | | 0.02722 | 0.005861 |
| rs75811180 | 12 | 59825922 | NA | 1.07E-05 | | 0.02722 | 0.005861 |
| rs144524094 | 12 | 64412328 | SRGAP1 | 1.05E-07 | intron_variant | 0.0269 | 0.00468 |
| rs75779275 | 12 | 66668726 | NA | 4.19E-05 | | 0.02094 | 0.004878 |
| rs11176136 | 12 | 66700104 | HELB | 2.52E-05 | intron_variant | 0.01546 | 0.003494 |
| rs7976871 | 12 | 69125513 | NUP107 | 3.52E-05 | intron_variant | 0.007087 | 0.001633 |
| rs7976876 | 12 | 69125514 | NUP107 | 3.52E-05 | intron_variant | 0.007087 | 0.001633 |
| rs7976687 | 12 | 69125578 | NUP107 | 3.52E-05 | intron_variant | 0.007087 | 0.001633 |
| rs10878865 | 12 | 69149478 | SLC35E3 | 4.34E-06 | NMD transcript_variant | 0.007871 | 0.001616 |
| rs3730603 | 12 | 69222379 | MDM2 | 4.71E-07 | intron_variant | 0.03074 | 0.00569 |
| rs146370968 | 12 | 69223034 | MDM2 | 4.71E-07 | intron_variant | 0.03074 | 0.00569 |
| rs3730617 | 12 | 69224892 | MDM2 | 4.71E-07 | intron_variant | 0.03074 | 0.00569 |
| rs71454208 | 12 | 69226396 | MDM2 | 4.71E-07 | intron_variant | 0.03074 | 0.00569 |
| rs3730635 | 12 | 69229123 | MDM2 | 1.04E-05 | intron_variant | 0.02262 | 0.004864 |
| rs3730652 | 12 | 69232793 | MDM2 | 4.71E-07 | intron_variant | 0.03074 | 0.00569 |
| rs769412 | 12 | 69233215 | MDM2 | 4.71E-07 | synonymous_variant | 0.03074 | 0.00569 |
| rs11296638 | 12 | 69238437 | MDM2 | 4.71E-07 | 3_prime_UTR_variant | 0.03074 | 0.00569 |
| rs7956669 | 12 | 69238507 | MDM2 | 4.71E-07 | 3_prime_UTR_variant | 0.03074 | 0.00569 |
| rs78497497 | 12 | 94965816 | TMCC3 | 4.41E-05 | intron_variant | 0.02116 | 0.004945 |
| rs142944124 | 12 | 99162625 | ANKS1B | 1.04E-07 | intron_variant | 0.03229 | 0.005617 |
| rs143208630 | 12 | 99174638 | ANKS1B | 1.04E-07 | intron_variant | 0.03229 | 0.005617 |
| rs10507253 | 12 | 115218522 | NA | 2.50E-05 | | 0.02626 | 0.005931 |
| rs10850384 | 12 | 115218755 | NA | 2.50E-05 | | 0.02626 | 0.005931 |
| rs11608897 | 12 | 115219079 | NA | 2.50E-05 | | 0.02626 | 0.005931 |
| rs144088350 | 12 | 115219743 | NA | 2.50E-05 | | 0.02626 | 0.005931 |

(continued)

| SNP ID | ch r | position | gene locus | p-value | SNP location in gene locus | BETA | SE |
|---|---|---|---|---|---|---|---|
| rs118030982 | 12 | 118766151 | TA0K3 | 4.85E-05 | intron_variant | 0.01514 | 0.00356 |
| 12:118770780 :A:AAC | 12 | 118770780 | NA | 4.85E-05 | NA | 0.01514 | 0.00356 |
| 12:118770780:A :AACACACACACACAC | 12 | 118770780 | NA | NA | NA | NA | NA |
| 12:125161441 :T:C | 12 | 125161441 | NA | 2.44E-05 | NA | 0.009873 | 0.002227 |
| rs11058227 | 12 | 126018404 | TMEM132B | 3.20E-05 | intron_variant | -0.005877 | 0.001347 |
| rs146061449 | 13 | 31045629 | HMGB1 | 8.73E-06 | intron_variant | 0.02263 | 0.004819 |
| rs9541338 | 13 | 35177997 | NA | 9.53E-06 | | 0.02259 | 0.004833 |
| rs9599308 | 13 | 35178625 | NA | 9.53E-06 | | 0.02259 | 0.004833 |
| rs9592551 | 13 | 35179080 | NA | 9.53E-06 | | 0.02259 | 0.004833 |
| rs7400628 | 13 | 38475425 | NA | 1.62E-05 | | 0.01275 | 0.002809 |

[Table 3-61]

| SNP ID | chr | position | gene locus | p-value | SNP location in gene locus | BETA | SE |
|---|---|---|---|---|---|---|---|
| rs4941879 | 13 | 38482388 | NA | 1.62E-05 | | 0.01275 | 0.002809 |
| rs9603278 | 13 | 38484455 | NA | 1.62E-05 | | 0.01275 | 0.002809 |
| rs77525380 | 13 | 38486498 | NA | 1.62E-05 | | 0.01275 | 0.002809 |
| rs11620108 | 13 | 38523618 | NA | 1.62E-05 | | 0.01275 | 0.002809 |
| rs75300170 | 13 | 38527975 | NA | 1.62E-05 | | 0.01275 | 0.002809 |
| rs10161861 | 13 | 38533329 | NA | 1.62E-05 | | 0.01275 | 0.002809 |
| rs140158226 | 13 | 38545248 | NA | 1.62E-05 | | 0.01275 | 0.002809 |
| rs11619292 | 13 | 38545843 | NA | 1.62E-05 | | 0.01275 | 0.002809 |
| rs113982010 | 13 | 38582994 | NA | 1.62E-05 | | 0.01275 | 0.002809 |
| rs9603302 | 13 | 38585865 | NA | 1.62E-05 | | 0.01275 | 0.002809 |
| rs7999781 | 13 | 38593133 | NA | 1.62E-05 | | 0.01275 | 0.002809 |
| rs139272125 | 13 | 61294761 | NA | 3.51E-05 | | 0.02589 | 0.005965 |
| rs140760839 | 13 | 61350501 | NA | 3.51E-05 | | 0.02589 | 0.005965 |
| rs79016205 | 13 | 65322102 | NA | 3.75E-05 | | 0.02103 | 0.004866 |
| rs9317907 | 13 | 70792954 | NA | 1.04E-07 | | 0.03229 | 0.005617 |
| rs118058117 | 13 | 71328142 | NA | 5.59E-06 | | 0.02836 | 0.005899 |
| rs9599962 | 13 | 73002308 | NA | 2.72E-07 | | 0.03139 | 0.005677 |
| rs184865348 | 13 | 73049971 | NA | 2.51E-06 | | 0.024 | 0.004796 |
| rs117639698 | 13 | 73077947 | NA | 2.51E-06 | | 0.024 | 0.004796 |
| rs35985160 | 13 | 75124959 | NA | 3.88E-06 | | 0.01063 | 0.00217 |
| rs76467960 | 13 | 78194746 | SCEL | 4.35E-05 | intron_variant | 0.0112 | 0.002615 |
| rs75814545 | 13 | 78196291 | SCEL | 4.35E-05 | intron_variant | 0.0112 | 0.002615 |
| 13:78211558:T:C | 13 | 78211558 | NA | 4.35E-05 | NA | 0.0112 | 0.002615 |
| rs9593249 | 13 | 78229617 | NA | 4.67E-05 | | 0.01167 | 0.002738 |
| rs9544568 | 13 | 78242133 | NA | 2.83E-05 | | 0.006437 | 0.001464 |
| rs186924328 | 13 | 88085503 | NA | 2.19E-06 | | 0.02908 | 0.005774 |
| rs184461986 | 13 | 88122173 | NA | 2.19E-06 | | 0.02908 | 0.005774 |
| rs184036706 | 13 | 88124358 | NA | 2.19E-06 | | 0.02908 | 0.005774 |
| rs143514766 | 13 | 88150782 | NA | 2.19E-06 | | 0.02908 | 0.005774 |
| rs145616607 | 13 | 88175773 | NA | 2.19E-06 | | 0.02908 | 0.005774 |
| rs146077205 | 13 | 88183851 | NA | 2.19E-06 | | 0.02908 | 0.005774 |
| rs115011805 | 13 | 88183871 | NA | 2.19E-06 | | 0.02908 | 0.005774 |
| rs149371095 | 13 | 88186758 | NA | 2.19E-06 | | 0.02908 | 0.005774 |
| rs141046761 | 13 | 88188257 | NA | 2.19E-06 | | 0.02908 | 0.005774 |
| rs7990951 | 13 | 95592144 | NA | 4.29E-05 | | 0.005125 | 0.001196 |
| rs4773831 | 13 | 95595122 | NA | 4.27E-05 | | 0.005166 | 0.001205 |

[Table 3-62]

| SNP ID | chr | position | gene locus | p-value | SNP location in gene locus | BETA | SE |
|---|---|---|---|---|---|---|---|
| rs7991523 | 13 | 95602158 | NA | 5.25E-06 | | 0.005282 | 0.001095 |
| rs8002051 | 13 | 95606804 | NA | 3.49E-05 | | 0.004797 | 0.001105 |
| rs9524705 | 13 | 95609326 | NA | 4.47E-05 | | 0.004714 | 0.001103 |
| rs7998757 | 13 | 95610131 | NA | 2.97E-05 | | 0.004772 | 0.001089 |
| rs192331957 | 13 | 101175688 | PCCA | 2.50E-05 | intron_variant | 0.02626 | 0.005931 |
| rs1408046 | 13 | 103484653 | BIVM-ERCC5 | 3.13E-06 | intron_variant | 0.01694 | 0.003422 |
| rs76029744 | 13 | 103486018 | BIVM-ERCC5 | 2.63E-08 | intron_variant | 0.02164 | 0.003573 |
| rs7327832 | 13 | 103499900 | BIVM-ERCC5 | 3.13E-06 | intron_variant | 0.01694 | 0.003422 |
| rs6491714 | 13 | 103500458 | BIVM-ERCC5 | 3.13E-06 | intron_variant | 0.01694 | 0.003422 |
| rs75982504 | 13 | 103501928 | BIVM-ERCC5 | 3.13E-06 | intron_variant | 0.01694 | 0.003422 |
| rs7328951 | 13 | 103502204 | BIVM-ERCC5 | 3.13E-06 | intron_variant | 0.01694 | 0.003422 |
| rs7333684 | 13 | 103502332 | BIVM-ERCC5 | 3.13E-06 | intron_variant | 0.01694 | 0.003422 |
| rs2104301 | 13 | 103502553 | BIVM-ERCC5 | 3.13E-06 | intron_variant | 0.01694 | 0.003422 |
| rs4150263 | 13 | 103504344 | BIVM-ERCC5 | 3.13E-06 | intron_variant | 0.01694 | 0.003422 |
| rs4150278 | 13 | 103507753 | BIVM-ERCC5 | 3.13E-06 | intron_variant | 0.01694 | 0.003422 |
| rs4150281 | 13 | 103508095 | BIVM-ERCC5 | 3.13E-06 | intron_variant | 0.01694 | 0.003422 |
| rs4150287 | 13 | 103509132 | BIVM-ERCC5 | 3.43E-07 | intron_variant | 0.01561 | 0.002851 |
| rs12854564 | 13 | 103976233 | NA | 9.04E-06 | | 0.009568 | 0.002041 |
| rs75174938 | 13 | 108015726 | FAM155A | 1.65E-08 | intron_variant | 0.01241 | 0.002014 |
| rs9520931 | 13 | 109193709 | NA | 2.63E-05 | | 0.01282 | 0.002904 |
| rs3825469 | 13 | 110881627 | COL4A1 | 7.03E-06 | intron_variant | 0.01786 | 0.003759 |
| 13:113504421:A:G | 13 | 113504421 | NA | 2.1OE-05 | NA | 0.01886 | 0.004217 |
| 13:113505107:A:G | 13 | 113505107 | NA | 2.10E-05 | NA | 0.01886 | 0.004217 |
| rs12877285 | 13 | 113519561 | ATP11A | 3.66E-05 | intron_variant | 0.009453 | 0.002184 |
| 13:113679279:G:A | 13 | 113679279 | NA | 2.10E-05 | NA | 0.01886 | 0.004217 |

(continued)

| SNP ID | chr | position | gene locus | p-value | SNP location in gene locus | BETA | SE |
|---|---|---|---|---|---|---|---|
| 13:113687158:C:T | 13 | 113687158 | NA | 2.10E-05 | NA | 0.01886 | 0.004217 |
| rs117419951 | 13 | 114211421 | NA | 3.22E-05 | | 0.02682 | 0.00615 |
| rs182523160 | 13 | 114214047 | NA | 3.22E-05 | | 0.02682 | 0.00615 |
| rs78785829 | 14 | 29770720 | NA | 1.77E-06 | | 0.02136 | 0.004198 |
| rs79415577 | 14 | 29823747 | NA | 2.01E-05 | | 0.01736 | 0.003872 |
| rs9788541 | 14 | 29841230 | NA | 1.77E-06 | | 0.02136 | 0.004198 |
| rs10141779 | 14 | 46320098 | NA | 1.07E-05 | | 0.02722 | 0.005861 |
| rs10130975 | 14 | 46320102 | NA | 1.07E-05 | | D.02722 | 0.005861 |
| rs10142107 | 14 | 46320162 | NA | 1.07E-05 | | 0.02722 | 0.005861 |
| rs17116552 | 14 | 46322340 | NA | 2.10E-05 | | 0.01894 | 0.004234 |
| rs145926281 | 14 | 47920866 | MDGA2 | 1.04E-07 | intron_variant | 0.03229 | 0.005617 |

[Table 3-63]

| SNP ID | chr | position | gene locus | p-value | SNP location in gene locus | BETA | SE |
|---|---|---|---|---|---|---|---|
| rs74506457 | 14 | 48138790 | MDGA2 | 1. 67E-06 | intron_variant | 0.01859 | 0.003644 |
| rs140552479 | 14 | 48452990 | NA | 1.04E-07 | | 0.03229 | 0.005617 |
| rs4483778 | 14 | 62669733 | NA | 3.86E-05 | | 0.01719 | 0.003984 |
| rs74055659 | 14 | 62672299 | NA | 3.86E-05 | | 0.01719 | 0.003984 |
| rs74055661 | 14 | 62674106 | NA | 3.86E-05 | | 0.01719 | 0.003984 |
| rs76079973 | 14 | 62674412 | NA | 3.86E-05 | | 0.01719 | 0.003984 |
| rs60686625 | 14 | 62675734 | NA | 3.86E-05 | | 0.01719 | 0.003984 |
| rs4902111 | 14 | 62679573 | NA | 3.86E-05 | | 0.01719 | 0.003984 |
| rs72476707 | 14 | 62679925 | NA | 3.86E-05 | | 0.01719 | 0.003984 |
| rs72476708 | 14 | 62679941 | NA | 3.86E-05 | | 0.01719 | 0.003984 |
| rs740556fi5 | 14 | 62683062 | NA | 3.86E-05 | | 0.01719 | 0.003984 |
| rs74055666 | 14 | 62683158 | NA | 3.86E-05 | | 0.01719 | 0.003984 |
| rs11622218 | 14 | 62684550 | NA | 3.86E-05 | | 0.01719 | 0.003984 |
| rs74055670 | 14 | 62695028 | NA | 3.86E-05 | | 0.01719 | 0.003984 |
| rs200453336 | 14 | 62696350 | NA | 3.86E-05 | | 0.01719 | 0.003984 |
| rs28793052 | 14 | 62697293 | NA | 3.86E-05 | | 0.01719 | 0.003984 |
| rs2090184 | 14 | 62698275 | NA | 3.86E-05 | | 0.01719 | 0.003984 |
| rs2365677 | 14 | 78681121 | NRXN3 | 3.94E-05 | intron_variant | 0.01536 | 0.003565 |
| rs142079680 | 14 | 84269688 | NA | 1.15E-05 | | 0.01757 | 0.003797 |
| rs77476904 | 14 | 85191547 | NA | 1.69E-06 | | 0.01266 | 0.002482 |
| rs74344396 | 14 | 85195062 | NA | 1.48E-05 | | 0.01206 | 0.002642 |

(continued)

| SNP ID | chr | position | gene locus | p-value | SNP location in gene locus | BETA | SE |
|---|---|---|---|---|---|---|---|
| rs12435636 | 14 | 85195464 | NA | 1.48E-05 | | 0.01206 | 0.002642 |
| rs1076961 | 14 | 85203036 | NA | 1.06E-05 | | 0.01022 | 0.0022 |
| rs74890854 | 14 | 88800193 | NA | 1.85E-05 | | 0.01258 | 0.002792 |
| rs78906101 | 14 | 89689421 | F0XN3 | 2.36E-05 | intron_variant | 0.0219 | 0.004928 |
| rs113893721 | 14 | 92365125 | FBLN5 | 1.07E-05 | intron_variant | 0.02722 | 0.005861 |
| rs1861086 | 14 | 92371328 | FBLN5 | 1.07E-05 | intron_variant | 0.02722 | 0.005861 |
| rs72702992 | 14 | 101655820 | NA | 8.97E-06 | | 0.01428 | 0.003045 |
| rs61994508 | 14 | 101656254 | NA | 8.97E-06 | | 0.01428 | 0.003045 |
| rs72702993 | 14 | 101656819 | NA | 8.97E-06 | | 0.01428 | 0.003045 |
| rs72702996 | 14 | 101657080 | NA | 8.97E-06 | | 0.01428 | 0.003045 |
| rs56907901 | 14 | 101657647 | NA | 8.97E-06 | | 0.01428 | 0.003045 |
| rs72702997 | 14 | 101659182 | NA | 8.97E-06 | | 0.01428 | 0.003045 |
| rs7147137 | 14 | 101678763 | NA | 4.74E-05 | | 0.01403 | 0.003294 |
| rs35480234 | 15 | 31093529 | NA | 2.60E-06 | | 0.0189 | 0.003784 |
| rs7163291 | 15 | 31093881 | NA | 2. 60E-06 | | 0.0189 | 0.003784 |

[Table 3-64]

| SNP ID | chr | position | gene locus | p-value | SNP location in gene locus | BETA | SE |
|---|---|---|---|---|---|---|---|
| rs35579443 | 15 | 31095933 | NA | 2.60E-06 | | 0.0189 | 0.003784 |
| rs35622207 | 15 | 31097419 | NA | 2.60E-06 | | 0.0189 | 0.003784 |
| rs11853322 | 15 | 31097795 | NA | 2.60E-06 | | 0.0189 | 0.003784 |
| rs6493297 | 15 | 31099054 | NA | 2.60E-06 | | 0.0189 | 0.003784 |
| rs71399735 | 15 | 31099728 | NA | 2.60E-06 | | 0.0189 | 0.003784 |
| rs57386153 | 15 | 31101060 | NA | 2.60E-06 | | 0.0189 | 0.003784 |
| rs3933429 | 15 | 31101915 | NA | 2.60E-06 | | 0.0189 | 0.003784 |
| rs28584365 | 15 | 31103314 | NA | 2.6DE-O6 | | 0.0189 | 0.003784 |
| rs10557815 | 15 | 31244352 | MTMR10 | 2.19E-06 | intron_variant | 0.02908 | 0.005774 |
| rs141616718 | 15 | 31253523 | MTMR10 | 2.19E-06 | intron_variant | 0.02908 | 0.005774 |
| rs76467407 | 15 | 31255220 | MTMR10 | 2.19E-06 | intron_variant | 0.02908 | 0.005774 |
| rs12898290 | 15 | 31294343 | TRPM1 | 2.19E-06 | missense_variant | 0.02908 | 0.005774 |
| rs3784589 | 15 | 31294114 | TRPM1 | 2.19E-06 | stop_gained | 0.02908 | 0.005774 |
| rs10162919 | 15 | 31295788 | TRPM1 | 5.30E-06 | intron_variant | 0.0203 | 0.004212 |
| rs7182547 | 15 | 31297672 | TRPM1 | 5.30E-06 | intronvariant | 0.0203 | 0.004212 |
| rs1161796 | 15 | 31298145 | TRPM1 | 5.30E-06 | intron_variant | 0.0203 | 0.004212 |
| rs7167074 | 15 | 31298352 | TRPM1 | 5.30E-06 | intron_variant | 0.0203 | 0.004212 |
| rs7182571 | 15 | 31298442 | TRPM1 | 5.30E-06 | intron_variant | 0.0203 | 0.004212 |

(continued)

| SNP ID | chr | position | gene locus | p-value | SNP location in gene locus | BETA | SE |
|---|---|---|---|---|---|---|---|
| rs7166214 | 15 | 31298483 | TRPM1 | 5.30E-06 | intron_variant | 0.0203 | 0.004212 |
| rs35616506 | 15 | 31298737 | TRPM1 | 2.19E-06 | intron_variant | 0.02908 | 0.005774 |
| rs143969953 | 15 | 31299528 | TRPM1 | 2.19E-06 | intron_variant | 0.02908 | 0.005774 |
| 15:31300031:C:CATTT | 15 | 31300031 | NA | 2.19E-06 | NA | 0.02908 | 0.005774 |
| rs61997145 | 15 | 31301159 | TRPM1 | 5.30E-06 | intron_variant | 0.0203 | 0.004212 |
| rs12913087 | 15 | 31304551 | TRPM1 | 5.30E-06 | intron_variant | 0.0203 | 0.004212 |
| rs201396966 | 15 | 31305173 | TRPM1 | 2.19E-06 | intron_variant | 0.02908 | 0.005774 |
| rs35891405 | 15 | 31307063 | TRPM1 | 5.30E-06 | intron_variant | 0.0203 | 0.004212 |
| 15:31309197:A:C | 15 | 31309197 | NA | 5.30E-06 | NA | 0.0203 | 0.004212 |
| rs12911930 | 15 | 31309225 | TRPM1 | 5.30E-06 | intron_variant | 0.0203 | 0.004212 |
| rs12898815 | 15 | 31309468 | TRPM1 | 5.30E-06 | intron_variant | 0.0203 | 0.004212 |
| rs35208656 | 15 | 31310534 | TRPM1 | 7.45E-06 | intron_variant | 0.01539 | 0.00325 |
| rs71420543 | 15 | 31312193 | NA | 2.19E-06 | NA | 0.02908 | 0.005774 |
| rs12915504 | 15 | 31312619 | TRPM1 | 5.30E-06 | intron_variant | 0.0203 | 0.004212 |
| rs12902573 | 15 | 31314311 | TRPM1 | 5.30E-06 | intron_variant | 0.0203 | 0.004212 |
| rs151309116 | 15 | 33681236 | RYR3 | 3.22E-05 | intron_variant | 0.02682 | 0.00615 |
| rs148846874 | 15 | 33899434 | RYR3 | 2.45E-05 | intron_variant | 0.0167 | 0.003767 |
| rs12905712 | 15 | 37264445 | MEIS2 | 3.69E-06 | intron_variant | 0.006133 | 0.001249 |

[Table 3-65]

| SNP ID | chr | position | gene locus | p-value | SNP location in gene locus | BETA | SE |
|---|---|---|---|---|---|---|---|
| rs1194770 | 15 | 37268081 | MEIS2 | 3.99E-05 | intron_variant | -0.004826 | 0.001121 |
| rs80351865 | 15 | 40161155 | GPR176 | 3.17E-05 | intron_variant | 0.01254 | 0.002902 |
| rs138955007 | 15 | 55865286 | PYG01 | 1.95E-05 | intron_variant | 0.01041 | 0.002317 |
| rs77212634 | 15 | 55875096 | PYG01 | 9.13E-06 | intron_variant | 0.01618 | 0.003454 |
| rs72738039 | 15 | 57687411 | CGNL1 | 5.91E-06 | non_coding_transcript_variant | 0.02049 | 0.004276 |
| rs73414637 | 15 | 62288216 | VPS13C | 8.05E-08 | intron_variant | 0.027 | 0.004649 |
| rs78465010 | 15 | 62313069 | VPS13C | 8.05E-08 | intron_variant | 0.027 | 0.004649 |
| rs67566652 | 15 | 64125992 | HERC1 | 4.48E-05 | intron_variant | 0.01097 | 0.002567 |
| rs9972484 | 15 | 64127038 | NA | 4.48E-05 | | 0.01097 | 0.002567 |
| rs9972527 | 15 | 64127531 | NA | 4.48E-05 | | 0.01097 | 0.002567 |
| rs7169537 | 15 | 64129914 | NA | 4.48E-05 | | 0.01097 | 0.002567 |
| rs12148623 | 15 | 64131474 | NA | 4.48E-05 | | 0.01097 | 0.002567 |
| rs2120252 | 15 | 64136472 | NA | 4.48E-05 | | 0.01097 | 0.002567 |

(continued)

| SNP ID | chr | position | gene locus | p-value | SNP location in gene locus | BETA | SE |
|---|---|---|---|---|---|---|---|
| rs116914345 | 15 | 66971210 | NA | 4.07E-05 | | 0.02127 | 0.004946 |
| rs190172459 | 15 | 78702652 | NA | 9.68E-06 | | 0.01414 | 0.003029 |
| rs144928882 | 15 | 78702663 | NA | 9.68E-06 | | 0.01414 | 0.003029 |
| rs181427460 | 15 | 78702665 | NA | 9.68E-06 | | 0.01414 | 0.003029 |
| rs77156427 | 15 | 78703944 | NA | 9.68E-06 | | 0.01414 | 0.003029 |
| rs76061647 | 15 | 78704580 | NA | 9.68E-06 | | 0.01414 | 0.003029 |
| rs76765649 | 15 | 78705102 | NA | 9.68E-06 | | 0.01414 | 0.003029 |
| rs11637161 | 15 | 78706391 | NA | 9.68E-06 | | 0.01414 | 0.003029 |
| rs79961164 | 15 | 78707978 | NA | 9.68E-06 | | 0.01414 | 0.003029 |
| rs80225953 | 15 | 78708302 | NA | 9.68E-06 | | 0.01414 | 0.003029 |
| rs77826529 | 15 | 78708555 | NA | 9.68E-06 | | 0.01414 | 0.003029 |
| rs72738490 | 15 | 80424475 | ZFAND6 | 4.29E-06 | intron_variant | 0.02363 | 0.004848 |
| rs12905402 | 15 | 80516383 | CTXND1 | 4.29E-06 | intron_variant | 0.02363 | 0.004848 |
| rs35971717 | 15 | 80517610 | CTXND1 | 4.29E-06 | intron_variant | 0.02363 | 0.004848 |
| 15:80517776:T:TC | 15 | 80517776 | NA | 4.29E-06 | NA | 0.02363 | 0.004848 |
| rs71399403 | 15 | 80517919 | CTXND1 | 4.29E-06 | intron_variant | 0.02363 | 0.004848 |
| rs34765237 | 15 | 80520275 | CTXND1 | 4.29E-06 | intron_variant | 0.02363 | 0.004848 |
| rs80244168 | 15 | 80520961 | CTXND1 | 4.29E-06 | intron_variant | 0.02363 | 0.004848 |
| rs28621634 | 15 | 80521264 | CTXND1 | 4.29E-06 | intron_variant | 0.02363 | 0.004848 |
| rs142665219 | 15 | 82201101 | NA | 1.72E-05 | | 0.02707 | 0.005982 |
| rs138527956 | 15 | 82208029 | NA | 1.72E-05 | | 0.02707 | 0.005982 |
| rs146297278 | 15 | 82224258 | NA | 1.72E-05 | | 0.02707 | 0.005982 |
| rs74028454 | 15 | 82275400 | NA | 1.72E-05 | | 0.02707 | 0.005982 |

[Table 3-66]

| SNP ID | chr | position | gene locus | p-value | SNP location in gene locus | BETA | SE |
|---|---|---|---|---|---|---|---|
| rs141857089 | 15 | 82275840 | NA | 1.72E-05 | | 0.02707 | 0.005982 |
| 15:82290701:T:A | 15 | 82290701 | NA | 1.72E-05 | NA | 0.02707 | 0.005982 |
| rs74028477 | 15 | 82298644 | NA | 1.72E-05 | | 0.02707 | 0.005982 |
| rs72746755 | 15 | 85851875 | ADAMTS7P4 | 2.45E-05 | non_coding_ transcript_var iant | 0.0167 | 0.003767 |
| rs4284612 | 15 | 85880568 | NA | 2.45E-05 | | 0.0167 | 0.003767 |
| rs74039987 | 15 | 101405738 | NA | 178E-05 | | 0.01001 | 0.002218 |

(continued)

| SNP ID | chr | position | gene locus | p-value | SNP location in gene locus | BETA | SE |
|---|---|---|---|---|---|---|---|
| rs141389573 | 15 | 101715258 | NA | 1.04E-07 | | 0.03229 | 0.005617 |
| rs76076521 | 16 | 11530047 | AC099489.1 | 1.76E-05 | intron_variant | 0.01221 | 0.002702 |
| rs113344842 | 16 | 11697890 | L1TAF | 4.72E-05 | intron_variant | 0.02094 | 0.004915 |
| 16:29011862:C:T | 16 | 29011862 | NA | 2.17E-05 | NA | 0.02183 | 0.00489 |
| rs147914226 | 16 | 29114347 | AC009093.9 | 1.72E-05 | non_coding_ transcript variant | 0.02707 | 0.005982 |
| rs117889226 | 16 | 29115569 | NA | 1.72E-05 | NA | 0.02707 | 0.005982 |
| rs117272179 | 16 | 29222277 | AC009093.8 | 8.84E-06 | non_coding_ transcript_ variant | 0.02259 | 0.004813 |
| rs147323464 | 16 | 29294541 | NA | 1.72E-05 | | 0.02707 | 0.005982 |
| rs143769600 | 16 | 29332310 | AC025279.2 | 1.72E-05 | non_coding_ transcript_ exon_ variant | 0.02707 | 0.005982 |
| 16:55795832:C:T | 16 | 55795832 | NA | 1.04E-07 | NA | 0.03229 | 0.005617 |
| rs79304792 | 16 | 57532336 | NA | 2.42E-06 | | 0.02391 | 0.00477 |
| rs8052995 | 16 | 60372574 | NA | 2.42E-06 | | 0.02391 | Q.00477 |
| rs935754 | 16 | 60377491 | NA | 4.71E-07 | | 0.03074 | 0.00569 |
| 16:71153772:G:A | 16 | 71153772 | NA | 1.51E-05 | NA | 0.00546 | 0.001198 |
| rs4888922 | 16 | 79121475 | WWOX | 107E-05 | intron_variant | 0.02722 | 0.005861 |
| rs57113334 | 16 | 80150523 | NA | 3.51E-05 | | 0.02589 | 0.005965 |
| rs114992408 | 16 | 82564106 | NA | 496E-05 | | 0.02109 | 0.004965 |
| rs111336258 | 16 | 82565313 | NA | 4.96E-05 | | 0.02109 | 0.004965 |
| rs12448746 | 16 | 84495825 | ATP2C2 | 4.60E-05 | intronvariant | 0.01052 | 0.002465 |
| rs12149961 | 16 | 85133324 | FAM92B | 2.90E-05 | intron_variant | 0.007613 | 0.001734 |
| rs113597486 | 16 | 85135378 | FAM92B | 1.07E-05 | intron_variant | 0.01948 | 0.004194 |
| rs75760369 | 16 | 85136066 | FAM92B | 1.07E-05 | intron_variant | 0.01948 | 0.004194 |

(continued)

| SNP ID | chr | position | gene locus | p-value | SNP location in gene locus | BETA | SE |
|---|---|---|---|---|---|---|---|
| rs150351869 | 16 | 85136199 | FAM92B | 1.07E-05 | intronvariant | 0.01948 | 0.004194 |
| rs77654959 | 16 | 85136253 | FAM92B | 1.07E-05 | intron_variant | 0.01948 | 0.004194 |
| 16:85185004:G:A | 16 | 85185004 | NA | 8.52E-06 | NA | 0.02781 | 0.005915 |
| rs142376288 | 16 | 85194913 | NA | 8.52E-06 | | 0.02781 | 0.005915 |
| rs143768726 | 17 | 6245605 | NA | 3.22E-05 | | 0.02682 | 0.00615 |
| rs116884024 | 17 | 7444424 | NA | 2.83E-05 | | 0.01318 | 0.002999 |
| rs11655029 | 17 | 17649172 | RAI1 | 1.01E-05 | intron_variant | 0.007793 | 0.001672 |
| rs9900803 | 17 | 17650978 | RAI1 | 1.01E-05 | intron_variant | 0.007793 | 0.001672 |

[Table 3-67]

| SNP ID | chr | position | gene locus | p-value | SNP location in gene locus | BETA | SE |
|---|---|---|---|---|---|---|---|
| rs11656775 | 17 | 17654319 | RAI1 | 1.01E-05 | intron_variant | 0.007793 | 0.001672 |
| rs941448 | 17 | 17654542 | RAI1 | 1.01E-05 | intron_variant | 0.007793 | 0.001672 |
| rs4925109 | 17 | 17661802 | RAI1 | 1.01E-05 | intron_variant | 0.007793 | 0.001672 |
| 17:17666365:G:GA | 17 | 17666365 | NA | 1.32E-05 | NA | 0.00984 | 0.002143 |
| 17:17678848:T : TAACACACATTTTGTA | 17 | 17678848 | NA | 1.01E-05 | NA | 0.007793 | 0.001672 |
| rs8072510 | 17 | 33772658 | SLFN13 | 2.24E-05 | stop_gained | 0.01047 | 0.00235 |
| rs11652363 | 17 | 33774675 | SLFN13 | 2.22E-05 | intron_variant | 0.0102 | 0.002289 |
| rs145674942 | 17 | 33775604 | SLFN13 | 2.22E-05 | intron_variant | 0.0102 | 0.002289 |
| rs75361910 | 17 | 33793208 | SLFN12L | 9.10E-06 | intron_variant | 0.01098 | 0.002343 |
| rs78856173 | 17 | 33805956 | SLFN12L | 194E-05 | intron_variant | 0.01221 | 0.002717 |
| 17:64650539:C:CAG | 17 | 64650539 | NA | NA | NA | NA | NA |
| 17:64650539:C:CAT | 17 | 64650539 | NA | 2.89E-05 | NA | 0.007664 | 0.001744 |

(continued)

| SNP ID | chr | position | gene locus | p-value | SNP location in gene locus | BETA | SE |
|---|---|---|---|---|---|---|---|
| rs138440170 | 17 | 71704366 | NA | 3.22E-05 | | 0.02682 | 0.00615 |
| rs78274099 | 17 | 77275723 | RBF0X3 | 7.83E-06 | intron_variant | 0.009909 | 0.002098 |
| rs9675480 | 18 | 1444697 | NA | 5.59E-06 | | 0.02836 | 0.005899 |
| rs73365235 | 18 | 1450563 | NA | 5.59E-06 | | 0.02836 | 0.005899 |
| rs2160960 | 18 | 1453341 | NA | 5.59E-06 | | 0.02836 | 0.005899 |
| rs143955284 | 18 | 1453688 | NA | 5.59E-06 | NA | 0.02836 | 0.005899 |
| rs57847079 | 18 | 1453700 | NA | 5.59E-06 | | 0.02836 | 0.005899 |
| rs78812196 | 18 | 1459687 | NA | 5.59E-06 | | 0.02836 | 0.005899 |
| rs75905563 | 18 | 1460889 | NA | 5.59E-06 | | 0.02836 | 0.005899 |
| 18:1460930:C:G | 18 | 1460930 | NA | 5.59E-06 | NA | 0.02836 | 0.005899 |
| rs16940518 | 18 | 1460930 | NA | NA | | NA | NA |
| rs73365261 | 18 | 1463910 | NA | 5.59E-06 | | 0.02836 | 0.005899 |
| rs72634385 | 18 | 3225679 | NA | 2.87E-06 | | 0.01176 | 0.002365 |
| rs55762828 | 18 | 3242809 | NA | 124E-06 | | 0.01185 | 0.002291 |
| rs1662349 | 18 | 3246289 | NA | 2.59E-05 | | 0.009618 | 0.002177 |
| rs7235642 | 18 | 4454027 | DLGAP1 | 5.59E-06 | intron_variant | 0.02836 | 0.005899 |
| rs7236361 | 18 | 4454161 | DLGAP1 | 5.59E-06 | intron_variant | 0.02836 | 0.005899 |
| rs151109374 | 18 | 4575611 | NA | 1.61E-05 | | 0.01715 | 0.003777 |
| rs56830227 | 18 | 9699877 | NA | 1.47E-05 | | 0.01938 | 0.004247 |
| 18:11624174:G:C | 18 | 11624174 | NA | 167E-05 | NA | 0.01206 | 0.002662 |
| 18:19415747:T:A | 18 | 19415747 | NA | 2.14E-05 | NA | 0.02166 | 0.004848 |
| rs1944288 | 18 | 25749095 | CDH2 | 4.63E-07 | intron_variant | 0.01969 | 0.003642 |

(continued)

| SNP ID | chr | position | gene locus | p-value | SNP location in gene locus | BETA | SE |
|---|---|---|---|---|---|---|---|
| rs2191636 | 18 | 25749888 | CDH2 | 4.63E-07 | intron_variant | 0.01969 | 0.003642 |

[Table 3-68]

| SNP ID | chr | position | gene locus | p-value | SNP location in gene locus | BETA | SE |
|---|---|---|---|---|---|---|---|
| rs11661487 | 18 | 25750950 | CDH2 | 4. 63E-07 | i ntron_var i ant | 0 . 01969 | 0.003642 |
| rs11874478 | 18 | 25751637 | CDH2 | 4. 63E-07 | intron_variant | 0. 01969 | 0.003642 |
| rs75095140 | 18 | 28690670 | NA | 2. 29E-05 | | 0. 01218 | 0.002738 |
| rs77485878 | 18 | 28713843 | DSC1 | 1. 75E-06 | intron_variant | 0. 02485 | 0.004883 |
| rs117854132 | 18 | 32857590 | ZSCAN30 | 4. 26E-05 | intron_variant | 0.014 | 0.003265 |
| rs75504094 | 18 | 32926349 | NA | 4. 26E-05 | | 0.014 | 0.003265 |
| rs72893124 | 18 | 35136494 | CELF4 | 2. 19E-06 | intron_variant | 0. 02908 | 0.005774 |
| rs72893132 | 18 | 35138693 | CELF4 | 2. 19E-06 | intron_variant | 0. 02908 | 0.005774 |
| rs150664883 | 18 | 35146206 | NA | 2. 19E-06 | | 0. 02908 | 0.005774 |
| rs55996300 | 18 | 35158843 | NA | 2.19E-06 | | 0. 02908 | 0.005774 |
| rs72893187 | 18 | 35172187 | NA | 2. 19E-06 | | 0. 02908 | 0.005774 |
| rs72895006 | 18 | 35189638 | NA | 2.19E-06 | | 0. 02908 | 0.005774 |
| rs55778135 | 18 | 35192829 | NA | 2. 19E-06 | | 0. 02908 | 0.005774 |
| rs111869452 | 18 | 37930904 | NA | 3. 75E-05 | NA | 0. 006374 | 0.001475 |
| rs111697888 | 18 | 49777775 | NA | 4. 24E-05 | | 0. 02123 | 0. 004948 |
| rs113445640 | 18 | 49787377 | NA | 2. 72E-07 | | 0. 03139 | 0.005677 |
| rs112197694 | 18 | 49792926 | NA | 4. 24E-05 | | 0. 02123 | 0.004948 |
| rs62081521 | 18 | 49793572 | NA | 4. 24E-05 | | 0. 02123 | 0.004948 |
| rs73445368 | 18 | 49794194 | NA | 4. 24E-05 | | 0. 02123 | 0.004948 |
| rs62081522 | 18 | 49796696 | NA | 4. 24E-05 | | 0. 02123 | 0.004948 |
| rs73445370 | 18 | 49796944 | NA | 4. 24E-05 | | 0. 02123 | 0.004948 |
| rs62081545 | 18 | 49803581 | NA | 4. 24E-05 | | 0. 02123 | 0.004948 |
| rs62081546 | 18 | 49805925 | NA | 4. 24E-05 | | 0. 02123 | 0.004948 |
| rs145744015 | 18 | 49808004 | NA | 4. 24E-05 | NA | 0. 02123 | 0. 004948 |
| rs62081550 | 18 | 49810293 | NA | 4. 24E-05 | | 0. 02123 | 0.004948 |
| rs7240258 | 18 | 49812967 | NA | 4. 24E-05 | | 0. 02123 | 0.004948 |
| rs62081552 | 18 | 49813508 | NA | 4. 24E-05 | | 0. 02123 | 0.004948 |
| rs62081553 | 18 | 49816209 | NA | 4. 24E-05 | | 0. 02123 | 0.004948 |
| rs62081554 | 18 | 49816348 | NA | 4. 24E-05 | | 0. 02123 | 0.004948 |
| rs8097353 | 18 | 49816961 | NA | 4. 24E-05 | | 0. 02123 | 0. 004948 |
| rs62081555 | 18 | 49817639 | NA | 4. 24E-05 | | 0. 02123 | 0. 004948 |
| rs28592006 | 18 | 50295649 | DCC | 2. 12E-05 | i ntron_var i ant | 0.0117 | 0.002617 |

(continued)

| SNP ID | chr | position | gene locus | p-value | SNP location in gene locus | BETA | SE |
|---|---|---|---|---|---|---|---|
| rs149621133 | 18 | 50299840 | DCC | 2. 12E-05 | intron_variant | 0.0117 | 0.002617 |
| rs114489043 | 18 | 50301355 | DCC | 2. 12E-05 | intron_variant | 0.0117 | 0. 00217 |
| rs74671490 | 18 | 50301411 | DCC | 2. 12E-05 | intron_variant | 0.0117 | 0.002617 |
| rs74600925 | 18 | 50301425 | DCC | 2. 12E-05 | intron_variant | 0.0117 | 0.002617 |

[Table 3-69]

| SNP ID | chr | position | gene locus | p-value | SNP location in gene locus | BETA | SE |
|---|---|---|---|---|---|---|---|
| rs78939599 | 18 | 50301601 | DCC | 2. 12E-05 | intron_variant | 0.0117 | 0.002617 |
| rs75368394 | 18 | 50301737 | DCC | 2. 12E-05 | intron_variant | 0.0117 | 0. 002617 |
| rs113902371 | 18 | 50301765 | DCC | 2. 12E-05 | intron_variant | 0.0117 | 0.002617 |
| rs9954223 | 18 | 50301788 | DCC | 2. 12E-05 | intron_variant | 0.0117 | 0. 002617 |
| rs9954366 | 18 | 50301832 | DCC | 2. 12E-05 | intron_variant | 0.0117 | 0. 002617 |
| rs9954387 | 18 | 50301888 | DCC | 2. 12E-05 | intron_variant | 0.0117 | 0. 002617 |
| rs9956954 | 18 | 50302147 | DCC | 2. 12E-05 | intron_variant | 0. 0117 | 0.002617 |
| rs9946240 | 18 | 50302221 | DCC | 2. 12E-05 | intron_variant | 0.0117 | 0. 002617 |
| rs9957208 | 18 | 50302225 | DCC | 2. 12E-05 | intron_variant | 0.0117 | 0.002617 |
| rs9957066 | 18 | 50302280 | DCC | 2. 12E-05 | intron_variant | 0.0117 | 0. 002617 |
| rs28720976 | 18 | 50302448 | DCC | 2. 12E-05 | intron_variant | 0.0117 | 0. 002617 |
| rs28710315 | 18 | 50302476 | DCC | 2. 12E-05 | intron_variant | 0.0117 | 0. 002617 |
| rs199781562 | 18 | 50302657 | DCC | 2. 12E-05 | intron_variant | 0.0117 | 0.002617 |
| 18:50302661:AG:A | 18 | 50302661 | NA | 2. 12E-05 | NA | 0.0117 | 0. 002617 |
| rs75255207 | 18 | 50302726 | DCC | 2. 12E-05 | intron_variant | 0.0117 | 0. 002617 |
| rs146773466 | 18 | 50302743 | DCC | 2. 12E-05 | intron_variant | 0. 0117 | 0. 002617 |
| rs112096055 | 18 | 50302828 | DCC | 2. 12E-05 | intron_variant | 0.0117 | 0. 002617 |

(continued)

| SNP ID | chr | position | gene locus | p-value | SNP location in gene locus | BETA | SE |
|---|---|---|---|---|---|---|---|
| rs77911615 | 18 | 50302866 | DCC | 2. 12E-05 | intron_variant | 0.0117 | 0.002617 |
| rs77161102 | 18 | 50303025 | DCC | 2. 12E-05 | intron_variant | 0.0117 | 0. 002617 |
| rs9960305 | 18 | 50303080 | DCC | 2. 12E-05 | intron_variant | 0.0117 | 0. 002617 |
| rs76926243 | 18 | 50303305 | DCC | 2. 12E-05 | intron_variant | 0.0117 | 0.002617 |
| rs9949728 | 18 | 50303409 | DCC | 2. 12E-05 | intron variant | 0.0117 | 0.002617 |
| rs72079451 | 18 | 50303416 | DCC | 2. 12E-05 | intron_variant | 0.0117 | 0. 002617 |
| rs140753418 | 18 | 50303838 | NA | 2. 12E-05 | NA | 0. 0117 | 0.002617 |
| rs28418704 | 18 | 50303935 | DCC | 2. 12E-05 | intron_variant | 0.0117 | 0. 002617 |
| rs1984342 | 18 | 50303991 | DCC | 2. 12E-05 | intron_variant | 0.0117 | 0.002617 |
| 18:50304137 :A: AGGGGG | 18 | 50304137 | NA | 2. 12E-05 | NA | 0.0117 | 0. 002617 |
| rs75177472 | 18 | 50304283 | DCC | 2. 12E-05 | intron_variant | 0.0117 | 0.002617 |
| rs28662803 | 18 | 50304359 | DCC | 2. 12E-05 | intron_variant | 0.0117 | 0. 002617 |
| rs79241149 | 18 | 50304605 | DCC | 2. 12E-05 | intron_variant | 0.0117 | 0. 002617 |
| rs9963981 | 18 | 50304678 | DCC | 2. 12E-05 | intron_variant | 0.0117 | 0. 002617 |
| rs74601030 | 18 | 50304685 | DCC | 2. 12E-05 | intron_variant | 0.0117 | 0. 002617 |
| rs9966586 | 18 | 50304959 | DCC | 2. 12E-05 | intron_variant | 0.0117 | 0. 002617 |
| rs9955760 | 18 | 50305011 | DCC | 2. 12E-05 | intron_variant | 0.0117 | 0. 002617 |
| rs9955775 | 18 | 50305063 | DCC | 2. 12E-05 | intron_variant | 0.0117 | 0.002617 |
| rs76362387 | 18 | 50305186 | DCC | 2. 12E-05 | intron_variant | 0.0117 | 0. 002617 |

[Table 3-70]

| SNP ID | chr | position | gene locus | p-value | SNP location in gene locus | BETA | SE |
|---|---|---|---|---|---|---|---|
| rs76980476 | 18 | 50305329 | DCC | 2. 12E-05 | intron_variant | 0.0117 | 0. 002617 |
| rs140215942 | 18 | 50305430 | DCC | 2. 12E-05 | intron_variant | 0.0117 | 0.002617 |
| rs79518038 | 18 | 50305494 | DCC | 2. 12E-05 | intron_variant | 0.0117 | 0.002617 |
| rs201972311 | 18 | 50305531 | DCC | 2. 12E-05 | intron_variant | 0.0117 | 0.002617 |
| rs116940662 | 18 | 50305545 | DCC | 2. 12E-05 | intron_variant | 0.0117 | 0. 002617 |
| rs9967152 | 18 | 50305573 | DCC | 2. 12E-05 | intron_variant | 0.0117 | 0.002617 |
| rs9956376 | 18 | 50305628 | DCC | 2.12E-05 | intron_variant | 0.0117 | 0.002617 |
| rs9967408 | 18 | 50305708 | DCC | 2. 12E-05 | intron_variant | 0.0117 | 0. 002617 |
| rs9946762 | 18 | 50305989 | DCC | 2.12E-05 | intron_variant | 0.0117 | 0.002617 |
| rs4638688 | 18 | 50306239 | DCC | 2. 12E-05 | intron_variant | 0.0117 | 0. 002617 |
| rs9947241 | 18 | 50306289 | DCC | 2. 12E-05 | intron_variant | 0.0117 | 0.002617 |
| rs9959370 | 18 | 50306321 | DCC | 2. 12E-05 | intron_variant | 0.0117 | 0. 002617 |
| 18:50306418:C : CTTCATTCA | 18 | 50306418 | NA | 2.12E-05 | NA | 0.0117 | 0.002617 |
| rs78062210 | 18 | 50306489 | DCC | 2. 12E-05 | intron_variant | 0.0117 | 0.002617 |
| rs78835166 | 18 | 50306510 | DCC | 2. 12E-05 | intron_variant | 0.0117 | 0.002617 |
| rs183630099 | 18 | 50306529 | DCC | 2. 12E-05 | intron_variant | 0.0117 | 0.002617 |
| rs188676378 | 18 | 50306530 | DCC | 2. 12E-05 | intron_variant | 0.0117 | 0.002617 |
| rs74542236 | 18 | 50306601 | DCC | 2. 12E-05 | intron_variant | 0.0117 | 0. 002617 |
| rs74406804 | 18 | 50306611 | DCC | 2. 12E-05 | intron_variant | 0.0117 | 0. 002617 |
| rs76093543 | 18 | 50306638 | DCC | 2.12E-05 | intron_variant | 0.0117 | 0.002617 |
| rs74641489 | 18 | 50306652 | DCC | 2. 12E-05 | intron_variant | 0.0117 | 0. 002617 |
| rs79335626 | 18 | 50306756 | DCC | 2. 12E-05 | intron_variant | 0.0117 | 0. 002617 |

(continued)

| SNP ID | chr | position | gene locus | p-value | SNP location in gene locus | BETA | SE |
|---|---|---|---|---|---|---|---|
| rs79579873 | 18 | 50306952 | DCC | 2. 12E-05 | intron_variant | 0.0117 | 0.002617 |
| rs9962392 | 18 | 50307080 | DCC | 2. 12E-05 | intron_variant | 0.0117 | 0. 002617 |
| rs9950339 | 18 | 50307252 | DCC | 2. 12E-05 | intron_variant | 0.0117 | 0.002617 |
| rs9950448 | 18 | 50307351 | DCC | 2. 12E-05 | intron_variant | 0.0117 | 0.002617 |
| rs9950558 | 18 | 50307440 | DCC | 2. 12E-05 | intron_variant | 0.0117 | 0.002617 |
| rs9950856 | 18 | 50307670 | DCC | 2. 12E-05 | intron_variant | 0.0117 | 0.002617 |
| rs9951083 | 18 | 50307728 | DCC | 2. 12E-05 | intron_variant | 0.0117 | 0. 002617 |
| rs9950936 | 18 | 50307750 | DCC | 2. 12E-05 | intron_variant | 0.0117 | 0.002617 |
| rs143651192 | 18 | 50308170 | NA | 2. 12E-05 | NA | 0.0117 | 0.002617 |
| 18:50308240:G:GTT | 18 | 50308240 | NA | 2. 12E-05 | NA | 0.0117 | 0. 002617 |
| rs80118519 | 18 | 50308340 | DCC | 2. 12E-05 | intron_variant | 0.0117 | 0. 002617 |
| rs80289134 | 18 | 50308699 | DCC | 2.12E-05 | intron_variant | 0.0117 | 0.002617 |
| rs77481124 | 18 | 50308701 | DCC | 2. 12E-05 | intron_variant | 0.0117 | 0. 002617 |

[Table 3-71]

| SNP ID | chr | position | gene locus | p-value | SNP location in gene locus | BETA | SE |
|---|---|---|---|---|---|---|---|
| rs113720878 | 18 | 50308740 | DCC | 2. 12E-05 | intron_variant | 0.0117 | 0.002617 |
| rs112252157 | 18 | 50308746 | DCC | 2. 12E-05 | intron_variant | 0.0117 | 0.002617 |
| rs28407519 | 18 | 50308840 | DCC | 2. 12E-05 | intron_variant | 0.0117 | 0.002617 |
| rs28485029 | 18 | 50308845 | DCC | 2. 12E-05 | intron_variant | 0.0117 | 0.002617 |
| rs28610143 | 18 | 50308878 | DCC | 2. 12E-05 | intron_variant | 0.0117 | 0.002617 |
| rs142971261 | 18 | 50308896 | DCC | 2. 12E-05 | intron_variant | 0.0117 | 0.002617 |
| rs111627202 | 18 | 50308902 | DCC | 2. 12E-05 | intron_variant | 0.0117 | 0.002617 |
| rs28398323 | 18 | 50309063 | DCC | 2. 12E-05 | intron_variant | 0.0117 | 0.002617 |
| rs28659932 | 18 | 50309081 | DCC | 2. 12E-05 | intron_variant | 0.0117 | 0.002617 |
| rs28524721 | 18 | 50309291 | DCC | 2. 12E-05 | intron_variant | 0.0117 | 0.002617 |
| rs77721880 | 18 | 50309453 | DCC | 2. 12E-05 | intron_variant | 0.0117 | 0.002617 |

(continued)

| SNP ID | chr | position | gene locus | p-value | SNP location in gene locus | BETA | SE |
|---|---|---|---|---|---|---|---|
| rs78434669 | 18 | 50309461 | DCC | 2. 12E-05 | intron_variant | 0.0117 | 0.002617 |
| rs137865031 | 18 | 50309499 | DCC | 2. 12E-05 | intron_variant | 0.0117 | 0.002617 |
| rs114083506 | 18 | 50309615 | DCC | 2. 12E-05 | intron_variant | 0.0117 | 0.002617 |
| rs79875724 | 18 | 50309626 | DCC | 2. 12E-05 | intron_variant | 0.0117 | 0.002617 |
| rs150063286 | 18 | 50309652 | DCC | 2. 12E-05 | intron_variant | 0.0117 | 0.002617 |
| rs9959936 | 18 | 50309944 | DCC | 2. 12E-05 | intron_variant | 0.0117 | 0.002617 |
| rs111664411 | 18 | 50309956 | DCC | 2. 12E-05 | intron_variant | 0.0117 | 0.002617 |
| rs139769416 | 18 | 50310074 | DCC | 2. 12E-05 | intron_variant | 0.0117 | 0.002617 |
| rs75906728 | 18 | 50310166 | DCC | 2. 12E-05 | intron_variant | 0.0117 | 0.002617 |
| rs74650785 | 18 | 50310219 | DCC | 2. 12E-05 | intron_variant | 0.0117 | 0.002617 |
| rs9949295 | 18 | 50310263 | DCC | 2. 12E-05 | intron_variant | 0.0117 | 0.002617 |
| rs76434772 | 18 | 50310291 | DCC | 2. 12E-05 | intron_variant | 0.0117 | 0.002617 |
| rs75112069 | 18 | 50310392 | DCC | 2. 12E-05 | intron_variant | 0.0117 | 0.002617 |
| rs111554653 | 18 | 50310597 | DCC | 2. 12E-05 | intron_variant | 0.0117 | 0.002617 |
| 18:50310625:C : CTITCT | 18 | 50310625 | NA | 2. 12E-05 | NA | 0.0117 | 0.002617 |
| rs78714353 | 18 | 50310649 | DCC | 2. 12E-05 | intron_variant | 0.0117 | 0.002617 |
| rs75493591 | 18 | 50310680 | DCC | 2. 12E-05 | intron_variant | 0.0117 | 0.002617 |
| rs77393461 | 18 | 50310710 | DCC | 2. 12E-05 | intron_variant | 0.0117 | 0.002617 |
| rs9960581 | 18 | 50310772 | DCC | 2. 12E-05 | intron_variant | 0.0117 | 0.002617 |
| rs77105189 | 18 | 50310911 | DCC | 2. 12E-05 | intron_variant | 0.0117 | 0.002617 |
| rs115627213 | 18 | 50310920 | DCC | 2. 12E-05 | intron_variant | 0.0117 | 0.002617 |
| rs113795849 | 18 | 50310929 | DCC | 2. 12E-05 | intron_variant | 0.0117 | 0.002617 |
| rs76212234 | 18 | 50311037 | DCC | 2. 12E-05 | intron_variant | 0.0117 | 0.002617 |
| rs75762275 | 18 | 50311039 | DCC | 2. 12E-05 | intron_variant | 0.0117 | 0.002617 |
| rs78544169 | 18 | 50311097 | DCC | 2. 12E-05 | intron_variant | 0.0117 | 0.002617 |

[Table 3-72]

| SNP ID | chr | position | gene locus | p-value | SNP location in gene locus | BETA | SE |
|---|---|---|---|---|---|---|---|
| rs28762705 | 18 | 50311168 | DCC | 2. 12E-05 | intron_variant | 0.0117 | 0. 002617 |
| rs28820319 | 18 | 50311273 | DCC | 2.12E-05 | intron_variant | 0.0117 | 0. 002617 |
| 18:50311304:G : GGAGA | 18 | 50311304 | NA | 2.12E-05 | NA | 0.0117 | 0. 002617 |
| rs9953235 | 18 | 50311467 | DCC | 2. 12E-05 | intron_variant | 0.0117 | 0 . 002617 |

(continued)

| SNP ID | chr | position | gene locus | p-value | SNP location in gene locus | BETA | SE |
|---|---|---|---|---|---|---|---|
| rs9953246 | 18 | 50311486 | DCC | 2. 12E-05 | intron_variant | 0.0117 | 0 . 002617 |
| rs28624269 | 18 | 50311586 | DCC | 2. 12E-05 | intron_variant | 0.0117 | 0. 002617 |
| rs28416202 | 18 | 50311587 | DCC | 2. 12E-05 | intron_variant | 0.0117 | 0.002617 |
| rs28587032 | 18 | 50311625 | DCC | 2.12E-05 | intron_variant | 0.0117 | 0. 002617 |
| rs9953616 | 18 | 50311854 | DCC | 2.12E-05 | intron_variant | 0.0117 | 0. 002617 |
| rs9956105 | 18 | 50311914 | DCC | 2. 12E-05 | intron_variant | 0.0117 | 0. 002617 |
| rs9956125 | 18 | 50311962 | DCC | 2.12E-05 | intron_variant | 0.0117 | 0.002617 |
| rs9945323 | 18 | 50312029 | DCC | 2. 12E-05 | intron_variant | 0.0117 | 0.002617 |
| rs9956060 | 18 | 50312136 | DCC | 2.12E-05 | intron_variant | 0.0117 | 0. 002617 |
| rs9945509 | 18 | 50312217 | DCC | 2. 12E-05 | intron_variant | 0.0117 | 0. 002617 |
| rs9956657 | 18 | 50312399 | DCC | 2.12E-05 | intron_variant | 0.0117 | 0. 002617 |
| rs9956597 | 18 | 50312504 | DCC | 2. 12E-05 | intron_variant | 0.0117 | 0.002617 |
| rs111891224 | 18 | 50312516 | DCC | 2.12E-05 | intron_variant | 0.0117 | 0.002617 |
| rs9941424 | 18 | 50312580 | DCC | 2. 12E-05 | intron_variant | 0.0117 | 0. 002617 |
| rs143959738 | 18 | 50312634 | DCC | 2.12E-05 | intron_variant | 0.0117 | 0.002617 |
| rs9941425 | 18 | 50312809 | DCC | 2. 12E-05 | intron_variant | 0.0117 | 0 . 002617 |
| 18:50312832:T:TA | 18 | 50312832 | NA | 2.12E-05 | NA | 0.0117 | 0. 002617 |
| rs9941430 | 18 | 50313014 | DCC | 2. 12E-05 | intron_variant | 0.0117 | 0. 002617 |
| rs9941431 | 18 | 50313046 | DCC | 2. 12E-05 | intron_variant | 0.0117 | 0.002617 |
| rs78139846 | 18 | 50313451 | DCC | 2.12E-05 | intron variant | 0.0117 | 0.002617 |
| rs75846297 | 18 | 50313646 | DCC | 2.12E-05 | intron_variant | 0.0117 | 0.002617 |
| rs28748057 | 18 | 50313692 | DCC | 2. 12E-05 | intron_variant | 0.0117 | 0. 002617 |

(continued)

| SNP ID | chr | position | gene locus | p-value | SNP location in gene locus | BETA | SE |
|---|---|---|---|---|---|---|---|
| rs199624929 | 18 | 50313803 | DCC | 2.12E-05 | intron_variant | 0.0117 | 0. 002617 |
| 18:50313941 :T : TAAATA | 18 | 50313941 | NA | 2.12E-05 | NA | 0.0117 | 0. 002617 |
| rs28770857 | 18 | 50314106 | DCC | 2. 12E-05 | intron_variant | 0.0117 | 0. 002617 |
| rs28872917 | 18 | 50314112 | DCC | 2.12E-05 | intron_variant | 0.0117 | 0.002617 |
| rs9941438 | 18 | 50314590 | DCC | 2.12E-05 | intron_variant | 0.0117 | 0. 002617 |
| rs9941411 | 18 | 50314697 | DCC | 2.12E-05 | intron_variant | 0.0117 | 0. 002617 |
| rs9941419 | 18 | 50314952 | DCC | 2. 12E-05 | intron_variant | 0.0117 | 0. 002617 |
| rs9941444 | 18 | 50315007 | DCC | 2. 12E-05 | intron_variant | 0.0117 | 0. 002617 |
| rs138603450 | 18 | 50315409 | DCC | 2. 12E-05 | intron_variant | 0.0117 | 0.002617 |
| rs9955427 | 18 | 50315451 | DCC | 2.12E-05 | intron_variant | 0.0117 | 0. 002617 |
| rs9966590 | 18 | 50315572 | DCC | 2.12E-05 | intron_variant | 0.0117 | 0. 002617 |
| rs9955595 | 18 | 50315604 | DCC | 2. 12E-05 | intron_variant | 0.0117 | 0.002617 |
| rs9966461 | 18 | 50315677 | DCC | 2.12E-05 | intron_variant | 0.0117 | 0. 002617 |

[Table 3-73]

| SNP ID | chr | position | gene locus | p-value | SNP location in gene locus | BETA | SE |
|---|---|---|---|---|---|---|---|
| rs77259281 | 18 | 50316233 | DCC | 2. 12E-05 | intron_variant | 0. 0117 | 0. 002617 |
| rs79494668 | 18 | 50316355 | DCC | 2. 12E-05 | intron_variant | 0. 0117 | 0. 002617 |
| rs9946558 | 18 | 50316362 | DCC | 2. 12E-05 | intron_variant | 0. 0117 | 0. 002617 |
| rs9958738 | 18 | 50316480 | DCC | 2. 12E-05 | intron_variant | 0. 0117 | 0. 002617 |
| rs9946735 | 18 | 50316499 | DCC | 2. 12E-05 | intron_variant | 0. 0117 | 0. 002617 |
| rs9958825 | 18 | 50316550 | DCC | 2. 12E-05 | intron_variant | 0. 0117 | 0. 002617 |
| rs144978667 | 18 | 56289433 | ALPK2 | 2. 19E-06 | intron_variant | 0. 02908 | 0. 005774 |
| rs28757575 | 18 | 61639238 | HMSD | 9. 58E-06 | intron_variant | 0. 01028 | 0. 002199 |
| rs28374149 | 18 | 61639311 | HMSD | 9. 58E-06 | intron_variant | 0. 01028 | 0. 002199 |
| rs117230606 | 18 | 64368841 | NA | 1. 46E-05 | | 0. 01522 | 0. 003333 |
| rs77930714 | 18 | 64381429 | NA | 1. 46E-05 | | 0. 01522 | 0. 003333 |

(continued)

| SNP ID | chr | position | gene locus | p-value | SNP location in gene locus | BETA | SE |
|---|---|---|---|---|---|---|---|
| rs75773722 | 18 | 64426972 | NA | 1. 46E-05 | | 0. 01522 | 0. 003333 |
| rs73967432 | 18 | 65688400 | NA | 5. 23E-06 | | 0. 01261 | 0. 002614 |
| rs113960301 | 18 | 65707948 | NA | 1. 92E-05 | | 0. 01105 | 0. 002457 |
| rs183621604 | 18 | 74404956 | NA | 3. 51E-05 | | 0. 02589 | 0. 005965 |
| rs80055446 | 18 | 75608998 | NA | 4. 71E-07 | | 0. 03074 | 0. 00569 |
| rs74686099 | 18 | 75861241 | NA | 3. 81E-05 | | 0. 006701 | 0. 001552 |
| rs77331644 | 18 | 75861619 | NA | 3. 81E-05 | | 0. 006701 | 0. 001552 |
| rs12458593 | 18 | 75862469 | NA | 3. 81E-05 | | 0. 006701 | 0. 001552 |
| rs113119443 | 18 | 76384089 | NA | 3. 51E-05 | | 0. 02589 | 0. 005965 |
| 18:76393189 :G:A | 18 | 76393189 | NA | 3. 51E-05 | NA | 0. 02589 | 0. 005965 |
| rs11672916 | 19 | 1036631 | CNN2 | 4. 57E-05 | intron_variant | 0. 01033 | 0. 00242 |
| rs11667191 | 19 | 1036718 | CNN2 | 4. 57E-05 | intron_variant | 0. 01033 | 0. 00242 |
| rs11672975 | 19 | 1036793 | CNN2 | 4. 57E-05 | intron_variant | 0. 01033 | 0. 00242 |
| rs11670441 | 19 | 1036913 | CNN2 | 4. 57E-05 | intron_variant | 0. 01033 | 0. 00242 |
| rs11665845 | 19 | 1037170 | CNN2 | 4. 57E-05 | intron_variant | 0. 01033 | 0. 00242 |
| rs113353568 | 19 | 1037551 | CNN2 | 4. 57E-05 | intron_variant | 0. 01033 | 0. 00242 |
| rs111525696 | 19 | 1037563 | CNN2 | 4. 57E-05 | intron_variant | 0. 01033 | 0. 00242 |
| rs72973561 | 19 | 1037584 | CNN2 | 4. 57E-05 | intron_variant | 0. 01033 | 0. 00242 |
| rs2232775 | 19 | 8373152 | CD320 | 2. 29E-05 | missense_ variant | 0. 01297 | 0. 002914 |
| rs7249111 | 19 | 8378870 | AC010323. 1 | 2. 29E-05 | intron_variant | 0. 01297 | 0. 002914 |
| rs8109861 | 19 | 8379894 | AC010323. 1 | 2. 29E-05 | intron_variant | 0. 01297 | 0. 002914 |
| rs8110376 | 19 | 8380149 | AC010323. 1 | 2. 29E-05 | intron_variant | 0 .01297 | 0. 002914 |

[Table 3-74]

| SNP ID | chr | position | gene locus | p-value | SNP location in gene locus | BETA | SE |
|---|---|---|---|---|---|---|---|
| rs11669423 | 19 | 21091222 | NA | 4.93E-05 | | 0.005645 | 0.001329 |
| rs11667242 | 19 | 21092697 | NA | 493E-05 | | 0.005645 | 0.001329 |
| rs2288896 | 19 | 30067812 | NA | 2.91E-05 | | 0.02152 | 0.004905 |
| 19:44183972:G:C | 19 | 44183972 | NA | 1.07E-05 | NA | 0.02722 | 0.005861 |
| rs145903192 | 19 | 44185599 | NA | 1.07E-05 | | 0.02722 | 0.005861 |
| rs112639353 | 19 | 44187225 | NA | 1.07E-05 | | 0.02722 | 0.005861 |
| rs79273520 | 19 | 44187737 | NA | 1.07E-05 | | 0.02722 | 0.005861 |
| rs75691094 | 19 | 44195271 | AC005622.1 | 1.07E-05 | intronvariant | 0.02722 | 0.005861 |
| rs140415511 | 19 | 44195583 | AC005622.1 | 107E-05 | intron_variant | 0.02722 | 0.005861 |
| rs201608105 | 19 | 44198053 | AC005622.1 | 1.07E-05 | intron_variant | 0.02722 | 0.005861 |

(continued)

| SNP ID | chr | position | gene locus | p-value | SNP location in gene locus | BETA | SE |
|---|---|---|---|---|---|---|---|
| rs59447107 | 19 | 44199652 | AC005622.1 | 1.07E-05 | intron_variant | 0.02722 | 0.005861 |
| rs148383153 | 19 | 44200274 | AC005622.1 | 1.07E-05 | intron_variant | 0.02722 | 0.005861 |
| 19:56565924:A:G | 19 | 56565924 | NA | 5.59E-06 | NA | 0.02836 | 0.005899 |
| rs117472015 | 19 | 56566789 | NLRP5 | 5.59E-06 | intron_variant | 0.02836 | 0.005899 |
| rs199933435 | 19 | 56574059 | NA | 5.59E-06 | | 0.02836 | 0.005899 |
| rs79145402 | 19 | 56576351 | NA | 5.59E-06 | | 0.02836 | 0.005899 |
| rs56070738 | 20 | 6655570 | NA | 2.45E-05 | | 0.0167 | 0.003767 |
| rs62194864 | 20 | 6655626 | NA | 2.45E-05 | | 0.0167 | 0.003767 |
| rs62194867 | 20 | 6663199 | NA | 2.45E-05 | | 0.0167 | 0.003767 |
| rs8121041 | 20 | 6664256 | NA | 2.45E-05 | | 0.0167 | 0.003767 |
| rs7272805 | 20 | 6666442 | NA | 2.45E-05 | | 0.0167 | 0.003767 |
| rs118144766 | 20 | 11371362 | NA | 2.16E-05 | | 0.01674 | 0.003749 |
| rs147182367 | 20 | 11374504 | NA | 2.16E-05 | | 0.01674 | 0.003749 |
| rs3067207 | 20 | 11516851 | NA | 6.41E-06 | | 0.01627 | 0.00341 |
| rs6111325 | 20 | 16858631 | NA | 4.82E-05 | | 0.01001 | 0.002352 |
| rs73247070 | 20 | 16872792 | NA | 4.82E-05 | | 0.01001 | 0.002352 |
| rs6105671 | 20 | 16874257 | NA | 4.82E-05 | | 0.01001 | 0.002352 |
| rs6105672 | 20 | 16874319 | NA | 7.91E-06 | | 0.01126 | 0.002385 |
| rs75411817 | 20 | 17437518 | PCSK2 | 3.11E-05 | intron_variant | 0.009288 | 0.002125 |
| rs2284912 | 20 | 17438213 | PCSK2 | 108E-05 | intron_variant | 0.009993 | 0.002152 |
| rs199610440 | 20 | 17444153 | PCSK2 | 1.08E-05 | intron_variant | 0.009993 | 0.002152 |
| rs73900815 | 20 | 17445166 | PCSK2 | 2.67E-05 | intron_variant | 0.009313 | 0.002112 |
| rs2269028 | 20 | 17446328 | PCSK2 | 1.08E-05 | intron_variant | 0.009993 | 0.002152 |
| rs2269029 | 20 | 17446362 | PCSK2 | 2.67E-05 | intron_variant | 0.009313 | 0.002112 |
| rs2269035 | 20 | 17450530 | PCSK2 | 3.39E-05 | intron_variant | 0.009732 | 0.002238 |
| rs117870212 | 20 | 17796971 | NA | 3.41E-05 | | 0.01657 | 0.003812 |

[Table 3-75]

| SNP ID | chr | position | gene locus | p-value | SNP location in gene locus | BETA | SE |
|---|---|---|---|---|---|---|---|
| rs149160109 | 20 | 19787051 | RIN2 | 3.22E-05 | intron_variant | 0.02682 | 0.00615 |
| 20:19957482: G:GTC | 20 | 19957482 | NA | 4.71E-07 | NA | 0.03074 | 0.00569 |
| rs60381469 | 20 | 19958065 | RIN2 | 4.71E-07 | intron_variant | 0.03074 | 0.00569 |
| rs143672948 | 20 | 42432890 | NA | 1.74E-07 | | 0.01711 | 0.003039 |
| rs17728712 | 20 | 50064221 | NFATC2 | 3.71E-05 | intron_variant | 0.01681 | 0.003887 |
| rs6096436 | 20 | 50064556 | NFATC2 | 3.71E-05 | intron_variant | 0.01681 | 0.003887 |
| rs79332570 | 20 | 55465963 | NA | 4.71E-07 | | 0.03074 | 0.00569 |

(continued)

| SNP ID | chr | position | gene locus | p-value | SNP location in gene locus | BETA | SE |
|--------|-----|----------|------------|---------|----------------------------|------|-----|
| rs12624457 | 20 | 55642740 | NA | 2.19E-06 | | 0.02908 | 0.005774 |
| rs16980692 | 20 | 55643296 | NA | 2.19E-06 | | 0.02908 | 0.005774 |
| rs6014915 | 20 | 55643568 | NA | 2.19E-06 | | 0.02908 | 0.005774 |
| rs1923179 | 20 | 55644273 | NA | 2.19E-06 | | 0.02908 | 0.005774 |
| rs78441963 | 20 | 55657024 | NA | 2.51E-06 | | 0.024 | 0.004796 |
| rs201245644 | 20 | 55681308 | NA | 2.19E-06 | | 0.02908 | 0.005774 |
| rs8115658 | 20 | 55686994 | NA | 4.71E-05 | | 0.01061 | 0.00249 |
| rs149841438 | 20 | 55704642 | NA | 1.55E-05 | | 0.01452 | 0.003191 |
| rs142759888 | 20 | 55708318 | NA | 1.55E-05 | | 0.01452 | 0.003191 |
| rs76064436 | 20 | 55750436 | BMP7 | 2.19E-06 | intron_variant | 0.02908 | 0.005774 |
| rs6128207 | 20 | 56436532 | NA | 2.96E-05 | | 0.01249 | 0.00285 |
| rs117535594 | 20 | 57263529 | STX16-NPEPL1 | 2.19E-06 | intron_variant | 0.02908 | 0.005774 |
| rs242813 | 20 | 58649794 | NA | 2.31E-05 | | 0.01108 | 0.002491 |
| 20:61493128: C:T | 20 | 61493128 | NA | 2.50E-05 | NA | 0.02626 | 0.005931 |
| 20:61493129: GCC:G | 20 | 61493129 | NA | 2.50E-05 | NA | 0.02626 | 0.005931 |
| 20:61493132: G:T | 20 | 61493132 | NA | 2.50E-05 | NA | 0.02626 | 0.005931 |
| 20:61493133: GCCACA:G | 20 | 61493133 | NA | 2.50E-05 | NA | 0.02626 | 0.005931 |
| rs144044846 | 21 | 19485388 | CHODL | 4.67E-05 | intron_variant | 0.01122 | 0.00263 |
| rs139603158 | 21 | 32201681 | KRTAP7-1 | 3.51E-05 | 3_prime_UTR _variant | 0.02589 | 0.005965 |
| rs66481055 | 21 | 40382795 | NA | 4.45E-05 | NA | 0.005382 | 0.001258 |
| rs450808 | 21 | 43706944 | ABCG1 | 3.54E-05 | intron_variant | 0.005963 | 0.001375 |
| rs77905583 | 21 | 44278878 | WDR4 | 5.59E-06 | intron_variant | 0.02836 | 0.005899 |
| rs77931345 | 21 | 44282099 | WDR4 | 5.59E-06 | intron_variant | 0.02836 | 0.005899 |
| rs112237505 | 21 | 44283785 | WDR4 | 5.59E-06 | intron_variant | 0.02836 | 0.005899 |
| rs5748948 | 22 | 17694856 | ADA2 | 2.93E-05 | intron_variant | 0.006512 | 0.001485 |
| rs117100146 | 22 | 17700318 | ADA2 | 9.76E-07 | 5_prime_UTR _variant | 0.02476 | 0.004735 |
| rs200828580 | 22 | 19162510 | NA | 2.71E-06 | | 0.02374 | 0.004763 |
| rs5746673 | 22 | 19163047 | NA | 2.71E-06 | | 0.02374 | 0.004763 |
| rs190264011 | 22 | 19166705 | NA | 2.71E-06 | | 0.02374 | 0.004763 |

[Table 3-76]

| SNP ID | chr | position | gene locus | p-value | SNP location in gene locus | BETA | SE |
|---|---|---|---|---|---|---|---|
| rs5748024 | 22 | 19168288 | CLTCL1 | 2.71E-06 | missense_variant | 0.02374 | 0.004763 |
| rs193038051 | 22 | 19169259 | CLTCL1 | 2.71E-06 | intron_variant | 0.02374 | 0.004763 |
| rs5748025 | 22 | 19169793 | CLTCL1 | 2.71E-06 | intron_variant | 0.02374 | 0.004763 |
| rs139626 | 22 | 25224556 | SGSM1 | 2.63E-05 | intron_variant | -0.007253 | 0.001643 |
| rs139629 | 22 | 25225119 | SGSM1 | 2.07E-05 | intron_variant | -0.00751 | 0.001678 |
| rs139633 | 22 | 25225879 | SGSM1 | 2.07E-05 | intron_variant | -0.00751 | 0.001678 |
| rs139637 | 22 | 25228375 | SGSM1 | 2.07E-05 | intron_variant | -0.00751 | 0.001678 |
| rs139644 | 22 | 25232065 | SGSM1 | 2.07E-05 | intron_variant | -0.00751 | 0.001678 |
| rs139648 | 22 | 25233260 | SGSM1 | 2.07E-05 | intron_variant | -0.00751 | 0.001678 |
| rs139649 | 22 | 25233565 | SGSM1 | 2.07E-05 | intron_variant | -0.00751 | 0.001678 |
| rs132548 | 22 | 29317559 | ZNRF3 | 4.72E-05 | intron_variant | 0.005545 | 0.001301 |
| rs5997861 | 22 | 31464163 | SMTN | 1.96E-05 | intron_variant | 0.01161 | 0.002586 |
| 22:31466683:G:T | 22 | 31466683 | NA | 2.89E-05 | NA | 0.01429 | 0.003255 |
| rs7287041 | 22 | 31469691 | SMTN | 1.96E-05 | intron_variant | 0.01161 | 0.002586 |
| rs9621179 | 22 | 31472299 | SMTN | 1.96E-05 | intron_variant | 0.01161 | 0.002586 |
| rs35749963 | 22 | 39244118 | NA | 2.13E-05 |  | 0.01373 | 0.003073 |
| rs9611038 | 22 | 39247482 | NA | 1.81E-06 |  | 0.01627 | 0.003202 |
| rs9607583 | 22 | 39248667 | NA | 1.81E-06 |  | 0.01627 | 0.003202 |
| rs5764095 | 22 | 44530469 | PARVB | 1.87E-05 | intron_variant | 0.01942 | 0.004312 |
| rs118092933 | 22 | 44547847 | PARVB | 5.90E-06 | intron_variant | 0.02329 | 0.004858 |
| rs8137152 | 22 | 44750797 | NA | 4.54E-05 |  | 0.008016 | 0.001877 |
| 22:47168035:T:C | 22 | 47168035 | NA | 1.60E-05 | NA | 0.01372 | 0.00302 |

206

(continued)

| SNP ID | chr | position | gene locus | p-value | SNP location in gene locus | BETA | SE |
|---|---|---|---|---|---|---|---|
| 22:47168059:T:C | 22 | 47168059 | NA | 1.60E-05 | NA | 0.01372 | 0.00302 |
| rs184367871 | 22 | 47177009 | TBC1D22A | 1.04E-07 | intron_variant | 0.03229 | 0.005617 |
| rs112425324 | 22 | 48826561 | NA | 1.04E-07 | | 0.03229 | 0.005617 |
| rs140475504 | 22 | 49593633 | NA | 1.07E-05 | | 0.02722 | 0.005861 |
| 22:49780452:C:T | 22 | 49780452 | NA | 8.52E-06 | NA | 0.02781 | 0.005915 |
| rs5934808 | 23 | 10164106 | CLCN4 | 3.96E-05 | intron_variant | 0.00853 | 0.00198 |
| rs5979274 | 23 | 10164535 | CLCN4 | 4.67E-05 | intron_variant | 0.008274 | 0.001941 |
| rs2238891 | 23 | 10165403 | CLCN4 | 4.67E-05 | intron_variant | 0.008274 | 0.001941 |
| rs72626456 | 23 | 42378072 | NA | 4.28E-05 | | 0.004867 | 0.001135 |
| rs5909566 | 23 | 118006329 | NA | 2.99E-05 | | 0.008719 | 0.00199 |
| rs59778322 | 23 | 118053423 | NA | 2.99E-05 | | 0.008719 | 0.00199 |
| rs35026453 | 23 | 118058304 | NA | 2.99E-05 | | 0.008719 | 0.00199 |
| rs200499538 | 23 | 118062390 | NA | 3.24E-05 | NA | 0.008527 | 0.001956 |
| X:118069424 : CTCTT:C | 23 | 118069424 | NA | 1.90E-06 | NA | 0.01001 | 0.001974 |

[Table 3-77]

| SNP ID | chr | position | gene locus | p-value | SNP location in gene locus | BETA | SE |
|---|---|---|---|---|---|---|---|
| rs5957082 | 23 | 118072203 | NA | 8.56E-06 | | 0.009242 | 0.001966 |
| rs200504262 | 23 | 118073507 | NA | 8.56E-06 | | 0.009242 | 0.001966 |
| rs4825392 | 23 | 118076725 | NA | 8.56E-06 | | 0.009242 | 0.001966 |
| rs5910471 | 23 | 118120492 | LONRF3 | 1.10E-05 | intron_variant | 0.009309 | 0.002007 |
| rs112219900 | 23 | 118820044 | SEPTIN6 | 3.22E-05 | intron_variant | 0.02682 | 0.00615 |
| rs6634729 | 23 | 128444668 | NA | 3.13E-05 | | 0.006383 | 0.001461 |
| rs72614106 | 23 | 128449159 | NA | 3.13E-05 | | 0.006383 | 0.001461 |
| rs6637592 | 23 | 128543945 | NA | 2.72E-06 | | 0.01091 | 0.002189 |
| rs6637593 | 23 | 128551239 | NA | 8.99E-06 | | 0.009448 | 0.002015 |
| rs6637596 | 23 | 128559316 | NA | 8.99E-06 | | 0.009448 | 0.002015 |

(continued)

| SNP ID | chr | position | gene locus | p-value | SNP location in gene locus | BETA | SE |
|---|---|---|---|---|---|---|---|
| rs185542983 | 23 | 143920769 | NA | 1.99E-05 | | 0.009942 | 0.002216 |
| rs137885389 | 23 | 151775134 | NA | 4.05E-06 | NA | 0.01449 | 0.002965 |
| rs4400524 | 23 | 151777266 | NA | 4.05E-06 | | 0.01449 | 0.002965 |
| rs58154948 | 23 | 151793551 | NA | 4.05E-06 | | 0.01449 | 0.002965 |

rs34033747 on chromosome 11 which was an intronic SNP in the DENN domain (DENND2B) containing 2B was identified as the highest SNP in the genome-wide association study (p-value=$1.91 \times 10^{-9}$) (Table 4). In Table 4, in the item "Allele," the left side means a basic allele and the right side is a minor allele (mutant-type allele), and the item "Gene ID" means the ID assigned to each gene defined by NCBI. The same meaning applies to the subsequent tables.

[Table 4]

| chr | position (bp) | SNP ID | beta | SE | minimum p value | Allel 6 | Gene name | Gene ID |
|---|---|---|---|---|---|---|---|---|
| 11 | 8853774 | rs34033747 | $2.55 \times 10^{-2}$ | $3.84 \times 10^{-3}$ | $1.91 \times 10^{-9}$ | C/T | DENND2B (ST5) | 6764 |
| 3 | 65873820 | rs58687721 | $1.92 \times 10^{-2}$ | $2.89 \times 10^{-3}$ | $1.97 \times 10^{-9}$ | T/G | MAGI1 | 9223 |
| 7 | 148438804 | rsl1974639 | $1.57 \times 10^{-2}$ | $2.53 \times 10^{-3}$ | $1.26 \times 10^{-8}$ | C/T | CUL1 | 8454 |
| 13 | 108015726 | rs75174938 | $1.24 \times 10^{-2}$ | $2.01 \times 10^{-3}$ | $1.65 \times 10^{-8}$ | G; A | FAM155A | 728215 |
| 13 | 103486018 | rs76029744 | $2.16 \times 10^{-2}$ | $3.57 \times 10^{-3}$ | $2.63 \times 10^{-8}$ | A/T | BIVN -ERCC5 | 100533467 |
| 8 | 4801168 | rs75778595 | $2.73 \times 10^{-2}$ | $4.55 \times 10^{-3}$ | $3.45 \times 10^{-8}$ | G/A | CSMD1 | 64478 |
| 12 | 29790399 | rs10843457 | $9.36 \times 10^{-3}$ | $1.61 \times 10^{-3}$ | $7.98 \times 10^{-8}$ | T; C | TMTC1 | 83857 |
| 8 | 106566606 | rs34823616 | $3.23 \times 10^{-2}$ | $5.62 \times 10^{-3}$ | $1.04 \times 10^{-7}$ | T/C | ZFPM2 | 23414 |
| 5 | 41442044 | rs318065 | $1.66 \times 10^{-2}$ | $2.96 \times 10^{-3}$ | $1.80 \times 10^{-7}$ | T/C | PLCXD3 | 345557 |
| 4 | 122249973 | rs6821123 | $2.55 \times 10^{-2}$ | $4.68 \times 10^{-3}$ | $3.98 \times 10^{-7}$ | C/T | QRFPR | 84109 |
| 7 | 148530294 | rs10245290 | $1.78 \times 10^{-2}$ | $3.30 \times 10^{-3}$ | $4.85 \times 10^{-7}$ | T/C | EZH2 | 2146 |
| 10 | 67784976 | rs10996833 | $2.47 \times 10^{2}$ | $4.70 \times 10^{3}$ | $8.74 \times 10^{7}$ | A/G | CTNNA3 | 29119 |
| 6 | 105721926 | rs72938040 | $2.43 \times 10^{-2}$ | $4.75 \times 10^{-3}$ | $1.66 \times 10^{-6}$ | A/G | PREP | 5550 |
| 15 | 31294343 | rs12898290 | $2.91 \times 10^{-2}$ | $5.77 \times 10^{-3}$ | $2.19 \times 10^{-6}$ | A/T | TRPM1 | 4308 |
| 10 | 78859025 | rs80058374 | $7.81 \times 10^{-3}$ | $1.57 \times 10^{-3}$ | $2.78 \times 10^{-6}$ | T; C | KCNMA1 | 3778 |

(continued)

| chr | position (bp) | SNP ID | beta | SE | minimum p value | Allel 6 | Gene name | Gene ID |
|---|---|---|---|---|---|---|---|---|
| 1 | 24495722 | rs4649197 | $9.86 \times 10^{-3}$ | $2.03 \times 10^{-3}$ | $4.76 \times 10^{-6}$ | A/G | IFNLR1 | 163702 |
| 7 | 30370786 | rs11974360 | $1.08 \times 10^{-2}$ | $2.23 \times 10^{-3}$ | $5.12 \times 10^{-6}$ | A/G | ZNRF2 | 223082 |
| 1 | 58715824 | rs117567026 | $1.52 \times 10^{-2}$ | $3.20 \times 10^{-3}$ | $6.69 \times 10^{-6}$ | C/G | DAB1 | 1600 |
| 10 | 125679317 | rs72631124 | $1.35 \times 10^{-2}$ | $2.92 \times 10^{-3}$ | $1.09 \times 10^{-5}$ | G/A | CPXM2 | 119587 |
| 9 | 19642563 | rs16937677 | $9.40 \times 10^{-3}$ | $2.05 \times 10^{-3}$ | $1.36 \times 10^{-5}$ | G; A | SLC24A2 | 25769 |
| 3 | 77035984 | rs67172613 | $1.73 \times 10^{-2}$ | $3.80 \times 10^{-3}$ | $1.60 \times 10^{-5}$ | T/C | R0B02 | 6092 |
| 18 | 50295649 | rs28592006 | $1.17 \times 10^{-2}$ | $2.62 \times 10^{-3}$ | $2.12 \times 10^{-5}$ | C/G | DCC | 1630 |
| 11 | 26600213 | rs61877058 | $2.20 \times 10^{-2}$ | $4.93 \times 10^{-3}$ | $2.14 \times 10^{-5}$ | G/A | AN03 | 63982 |
| 4 | 147199809 | rs60367087 | $2.13 \times 10^{-2}$ | $4.87 \times 10^{-3}$ | $3.1 \times 10^{-5}$ | C/T | SLC10A7 | 84068 |

Five gene loci including CULI, QRFP, CTNNA3, DAB1, and DCC and associated genes were known to be associated with Aβ production. Furthermore, eight gene loci including MAGI1, TMTC1, TRPM1, KCNMA1, DAB1, CPXM2, ROBO2, and ANO3 and associated genes have been reported as clinical biomarkers or AD-associated gene loci for clinical GWAS. Twelve gene loci and associated genes were novel as Aβ- or AD-associated genes (Tables 5-1 and 5-2). In Tables 5-1 and 5-2, "EOAD" means early-onset Alzheimer's disease, "LOAD" means late-onset Alzheimer's disease, "CNV" means copy number variation, and "OR" means an odds ratio. In addition, "yes" in the item "Brain" means high expression in the brain, "low" means low expression in the brain, and "nd" means that there is no data in the GTEx Portal (https://gtexportal.org/home/). In addition, the item "Brain cell-type" describes the top three cell types that showed high gene expression on the Brain RNA-Seq portal (https://www.brainrnaseq.org/). The same meaning applies to the subsequent tables.

[Table 5-1]

| chr | Gene name | Gene ID | Brain | Brain cell-type | Relationship to Alzheimer's disease | Study type | Publication |
|---|---|---|---|---|---|---|---|
| 11 | DENND2B (ST5) | 6764 | yes | N/A | NA | NA | NA |
| 3 | MAGI1 | 9223 | yes | nd | Duplication in autosomal dominant EOAD | Array-based CNV analysis for AD patient cohort | Eur J Hum Genet. 2012 Jun; 20(6): 613-617. |
| 7 | CUL1 | 8454 | low | N/A | Regulation of Aβ production via CUL-related NRBP1 | Basic research by using neuronal cells | Cell Rep. 2020 Mar 10;30(10): 34 78-3491.e6. |
| 13 | FAM155A | 728215 | yes | N/A | NA | NA | NA |

(continued)

| chr | Gene name | Gene ID | Brain | Brain cell-type | Relationship to Alzheimer's disease | Study type | Publication |
|---|---|---|---|---|---|---|---|
| 13 | BIVM-ERCCS | 100533467 | nd | N | NA | NA | NA |
| 8 | CSMD1 | 64478 | yes | N | NA | NA | NA |
| 12 | TMTC1 | 83857 | yes | N/A | rs302318, OR = 0.59(0.48-0.74) for AD in APOE ε4 Adjusted Analysis | GWAS for American african cohort | Arch Neurol . 2011 Dcc;68 (12):1 569-79. |
| 8 | ZFPM2 | 23414 | yes | N/A/O | NA | NA | NA |
| 5 | PLCXD3 | 345557 | yes | N/M | NA | NA | NA |
| 4 | QRFPR | 84109 | yes | E/N | QRFP protect neurons from Aβ toxicity | Basic research by using neuronal cells | Sci Rep. 2015 Jul 30;5:12584. |
| 7 | EZH2 | 2146 | low | A | NA | NA | NA |
| 10 | CTNNA3 | 29119 | yes | O/A/N | D10S1225 is correlated to plasma Aβ42 | linkage analysis and genome-wide screen of LOAD | Science . 2000 Dec 22;290 (5500) :2303-4. |
| | | | | | Associated with LOAD, independent of the APOE-epsilon4 allele | GWAS for Japanese LOAD | Hum Mol Genet. 2007 Dec 1;16 (23):285 4-69. |
| 6 | PREP | 5550 | yes | N/A/M | Colocalizes with Aβ and Tau in the brain of AD patients | Neuropatholo gical study for AD patients | Neuroscience . 2013 Jul 9;242: 140-50. |

[Table 5-2]

| chr | Gene name | Gene ID | Brain | Brain cell-type | Relationship to Alzheimer's disease | Study type | Publication |
|---|---|---|---|---|---|---|---|
| 15 | TRPM1 | 4308 | yes | O | NA | NA | NA |
| 10 | KCNMA1 | 3778 | yes | N/A | rs16934131, OR = 1.2210 for LOAD | GWAS for the Collaborative Alzheimer Project | Am J Hum Genet. 2009 Jan;84(i): 35-43. |
| 1 | IFNLR1 | 163702 | yes | M | NA | NA | NA |
| 7 | ZNRF2 | 223082 | yes | M/A/N | NA | NA | NA |

(continued)

| chr | Gene name | Gene ID | Brain | Brain cell-type | Relationship to Alzheimer's disease | Study type | Publication |
|---|---|---|---|---|---|---|---|
| 1 | DAB1 | 1600 | yes | N/A | Phosphorylation of DAB1 modulates Aβ production | Basic research by using cell-lines and primary neurons | J Biol Chem . 2006 Nov 17; 281(46):351 76-85. |
| | | | | | DAB1 Interacts with AICD and is increased in the brain of AD patients | Neuropathologic al study for AD patients | Curr Alzheimer Res. 2011 Aug; 8(5):573-82. |
| | | | | | DAB1 phosphorylation decreases in the brain of AD patients | Neuropathologic al study for AD patients | Sci Rep. 2016 Aug 17;6: 31646. |
| 10 | CPXM2 | 119587 | no | - | rs2362967 is associated with LOAD | Case-control study for Taiwanese in validation set (N = 619) | Sci Rep . 2016 Nov 2;6:36155. |
| 9 | SLC24A2 | 25769 | yes | O/N | NA | NA | NA |
| 3 | R0B02 | 6092 | yes | A/N | ROBO2 was decreased in the CSF of Ap- MCI patients | Multiplex proteomics study for BioFINDER study | Acta Neuropathol Commun . 2019 Nov 6;7 (1):169. |
| 18 | DCC | 1630 | yes | N/A/O | DCC ligand, Netrin-1, interacts with amyloid precursor protein and regulates Aβ production | Basic research by using cell-lines and primary neurons | Cell Death Differ. 2009 May; 16(5): 655-663. |
| 11 | ANO3 | 63992 | yes | N/E | Related to APOEε4 carrier | Weighted gene co-expression network analysis for LOAD | Front Aging Neurosci. 2016; 8: 171. |
| 4 | SLC10A7 | 84068 | low | A/O/N | NA | NA | NA |

Furthermore, most of the identified genes are expressed in the brain (GTEx Portal, https://gtexportal.org/home/) and 19 genes are highly expressed in neurons (Brain RNA-Seq portal, http://www.brainmaseq.org/) (Tables 5-1 and 5-2). Unbiased pathway analysis identified the "calcium signaling pathway" as the top canonical pathway (p-value=$2.51 \times 10^{-5}$) (FIG. 5C). These networks are known to alter Aβ metabolism. These results proved that the SNPs and associated genes identified by the analysis of the polygenic architecture described above contribute to Alzheimer's disease and the Aβ42/40 ratio of cerebral cortical neurons as cell type-specific features of AD pathology.

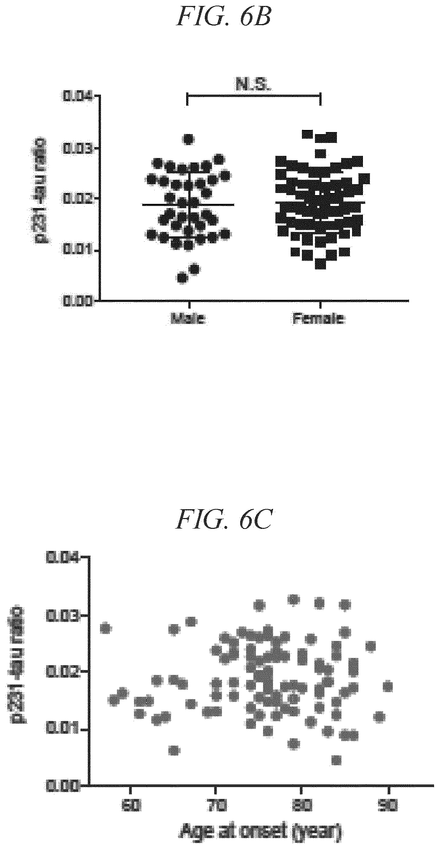

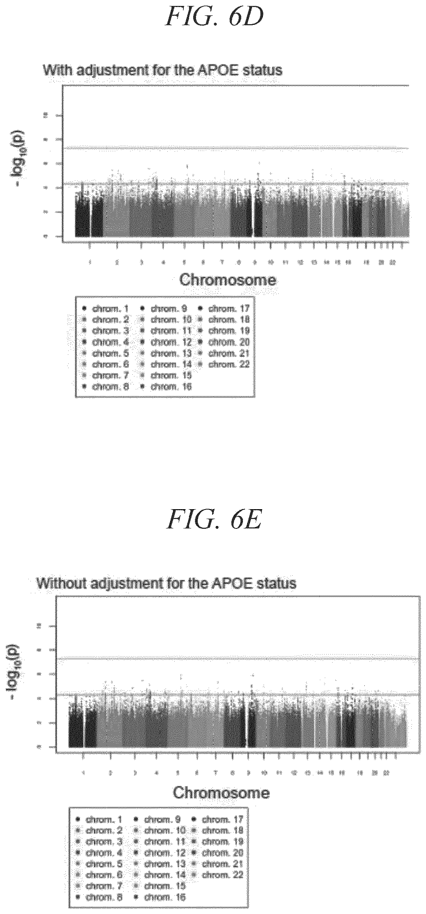

Furthermore, since p231-tau, which was tau phosphorylated at the 231st threonine from the N-terminus, was a high-sensitivity marker for diagnosis of AD or tracking the progression of AD, the p231-tau/total tau ratio (p23 I-tau ratio) was quantified to apply the p231-tau ratio to CDiP. The APOE-ε4 genotypes, sex, and the age at onset of AD did not correlate with the p231-tau ratio (FIGS. 6A, 6B, and 6C). CDiP was performed using the p231-tau ratio as a trait with or without APOE genotype adjustment (FIGS. 6D and 6E) to identify SNPs and associated gene loci (p-value<$5 \times 10^{-5}$) (Tables 6 and 7-1 to 7-9). The SNP with the lowest p-value was rs68888116 (p=$1.24 \times 10^{-6}$) in the TNFAIP8 gene locus which was an inflammation-associated molecule (Table 6). In Table 6, "insATCT" means that ATCT-CAG(A)$_{12}$TTCTCTATCT (SEQ ID NO: 3) is inserted.

[Table 6]

| chr | position (bp) | SNP ID | beta | SE | minimum p value | Allele | Gene name | Gene ID |
|---|---|---|---|---|---|---|---|---|
| 5 | 118659579 | rs6888116 | 4.19E-03 | 8.09E-04 | 1.24E-06 | A>C | P8P | 25816 |
| 9 | 112886940 | rs17806439 | -6.84E-03 | 1.33E-03 | 1.34E-06 | A>G | PALM2 AKAP2 | 445815 |
| 3 | 167162439 | rs98.99743 | 4.23E-03 | 8.58E-04 | 3.37E-06 | G>A.T | SERPIN 12 | 5276 |
| 2 | 131609539 | rs16856198 | -3.66E-03 | 7.49E-04 | 4.12E-06 | A>C | ARHGE F4 | 50649 |
| 7 | 100378274 | rs113223392 | 3.71E-03 | 7.64E-04 | 4.61E-06 | insATCT* | ZAN | 7455 |
| 10 | 68650441 | rs17231504 | 8.56E-03 | 1.84E-03 | 9.91E-06 | T>A,C | CTNNA 3 | 29119 |
| 16 | 15140211 | rs4500751 | 3.39E-03 | 7.41E-04 | 1.41E-05 | C>T | NTA\1 | 123803 |
| 16 | 6144047 | rs2034597 | 3.66E-03 | 8.03E-04 | 1.49E-05 | G>A | RBFOX 1 | 54715 |
| 11 | 95830567 | rs4753718 | 4.15E-03 | 9.11E-04 | 1.50E-05 | T>C | MAML2 | 84441 |
| 5 | 132139308 | rs2652696 | 3.76E-03 | 8.41E-04 | 2.18E-05 | T>A,C,G | SEPTIN 8 | 23176 |
| 2 | 50916658 | rs9309188 | 3.64E-03 | 8.16E-04 | 2.26E-05 | C>G | NRXN1 | 9378 |
| 8 | 37688733 | rs2070494 | -6.03E-03 | 1.38E-03 | 3.12E-05 | T>C | ADGRA 2 | 25960 |
| 17 | 10054889 | rs1024369 | -3.27E-03 | 7.50E-04 | 3.26E-05 | C>G,T | GAS7 | 8522 |
| 4 | 39428057 | rs200342845 | 3.93E-03 | 9.06E-04 | 3.49E-05 | delAG | KLB | 152831 |
| 7 | 17831018 | rs1404420 | -3.93E-03 | 9.06E-04 | 3.53E-05 | T>A,C,G | SNX13 | 23161 |
| 16 | 15125441 | rs4985124 | 3.32E-03 | 7.66E-04 | 3.55E-05 | T>A,G | PDXDC 1 | 23042 |
| 2 | 238791983 | rs74342940 | -4.01E-03 | 9.26E-04 | 3.67E-05 | delG | RAMP1 | 10267 |
| 14 | 78790792 | rs7153577 | 3.44E-03 | 7.96E-04 | 3.72E-05 | G>A,C,T | NRXN3 | 9369 |
| 5 | 132198287 | rs254285 | 3.46E-03 | 8.01E-04 | 3.73E-05 | C>G | GDF9 | 2661 |
| 4 | 169425778 | rs10517996 | 3.65E-03 | 8.46E-04 | 3.77E-05 | C>A,G,T | PALLD | 23022 |
| 9 | 101776487 | rs7035399 | -3.39E-03 | 7.86E-04 | 3.90E-05 | T>A,C,G | COL15 A1 | 1306 |

(continued)

| chr | position (bp) | SNP ID | beta | SE | minimum p value | Allele | Gene name | Gene ID |
|---|---|---|---|---|---|---|---|---|
| 20 | 42758834 | **rs761207** | -3.71E-03 | 8.65E-04 | 4.31E-05 | T>A,C,G | **JPH2** | 57158 |
| 5 | 35861068 | **rs1494558** | 3.95E-03 | 9.25E-04 | 4.59E-05 | T>A,C | **IL7R** | 3575 |
| 3 | 76104023 | **rs9812834** | 3.66E-03 | 8.60E-04 | 4.85E-05 | A>T | **ROBO2** | 6092 |

[Table 7-1]

| SNP ID | chr | position | gene locus | p-value | BETA | SE |
|---|---|---|---|---|---|---|
| rs1209461 | 1 | 58408470 | DAB1 | 4. 88E-05 | -0. 004235 | 0. 000996 |
| rs1202850 | 1 | 58408855 | DAB1 | 4. 88E-05 | -0. 004235 | 0. 000996 |
| rs1202851 | 1 | 58409755 | DAB1 | 4. 88E-05 | -0. 004235 | 0. 000996 |
| rs1202788 | 1 | 58414715 | DAB1 | 4. 88E-05 | -0. 004235 | 0. 000996 |
| rs1202790 | 1 | 58417348 | DAB1 | 4. 88E-05 | -0. 004235 | 0. 000996 |
| rs969719 | 2 | 50874681 | CTNNA3 | 4. 68E-05 | -0.006336 | 0.001486 |
| rs79725983 | 2 | 50876601 | CTNNA3 | 2. 76E-05 | -0. 006645 | 0.00151 |
| rs9309188 | 2 | 50916658 | NA | 2. 26E-05 | 0. 003635 | 0.0008162 |
| rs9309190 | 2 | 50918967 | NA | 3. 64E-05 | -0. 005043 | 0.001165 |
| rs7606024 | 2 | 56616595 | SYT9 | 4. 29E-05 | 0. 005784 | 0. 001349 |
| rs2566747 | 2 | 82304139 | MAML2 | 4. 55E-06 | 0. 005446 | 0. 001121 |
| rs2116045 | 2 | 82315179 | MAML2 | 4. 55E-06 | 0. 005446 | 0. 001121 |
| rs7601451 | 2 | 82322288 | NA | 1.48E-05 | 0.004155 | 0.0009108 |
| rs10202137 | 2 | 82322613 | NA | 1. 20E-05 | 0. 005371 | 0.001164 |
| rs12998884 | 2 | 82323175 | NA | 1. 20E-05 | 0. 005371 | 0.001164 |
| rs7605223 | 2 | 82323314 | NA | 3. 77E-05 | 0. 004022 | 0.000931 |
| rs5832525 | 2 | 82324709 | NA | 1.20E-05 | 0. 005371 | 0.001164 |
| rs1346859 | 2 | 82324973 | NA | 1. 20E-05 | 0. 005371 | 0.001164 |
| rs10173814 | 2 | 82326240 | NA | 1.20E-05 | 0. 005371 | 0.001164 |
| rs10197852 | 2 | 82326536 | NA | 1.20E-05 | 0. 005371 | 0.001164 |
| rs1346860 | 2 | 82327886 | NA | 1.20E-05 | 0. 005371 | 0.001164 |
| rs10658639 | 2 | 82335834 | NA | 1.20E-05 | 0. 005371 | 0. 001164 |
| rs113286166 | 2 | 82335943 | NA | 1.20E-05 | 0. 005371 | 0. 001164 |
| rs4852212 | 2 | 82337967 | NA | 1.20E-05 | 0. 005371 | 0. 001164 |
| rs1430708 | 2 | 82339149 | NA | 1.20E-05 | 0. 005371 | 0. 001164 |
| 2:82340763:C:T | 2 | 82340763 | NA | 1.20E-05 | 0. 005371 | 0. 001164 |
| 2:82340764:A:C | 2 | 82340764 | NA | 1.20E-05 | 0. 005371 | 0. 001164 |
| 2:82340765:C:A | 2 | 82340765 | NRXN3 | 1.20E-05 | 0. 005371 | 0. 001164 |
| rs10206958 | 2 | 82342060 | NA | 1.20E-05 | 0. 005371 | 0. 001164 |

(continued)

| SNP ID | chr | position | gene locus | p-value | BETA | SE |
|---|---|---|---|---|---|---|
| rs7583816 | 2 | 82344289 | PDXDC1 | 1.20E-05 | 0. 005371 | 0.001164 |
| rs1521665 | 2 | 82373290 | PDXDC1 | 3. 78E-05 | 0.00518 | 0.001199 |
| rs1367437 | 2 | 82379605 | PDXDC1 | 4. 14E-05 | 0. 005086 | 0. 001184 |
| rs1367438 | 2 | 82380031 | PDXDC1 | 3. 78E-05 | 0.00518 | 0. 001199 |
| rs10204952 | 2 | 82383092 | PDXDC1 | 3. 78E-05 | 0.00518 | 0. 001199 |
| 2:82383337:AT:A | 2 | 82383337 | PDXDC1 | 3. 78E-05 | 0.00518 | 0. 001199 |
| rs6704771 | 2 | 82384630 | PDXDC1 | 4. 14E-05 | 0. 005086 | 0.001184 |
| rs2052957 | 2 | 82388160 | PDXDC1 | 4. 14E-05 | 0. 005086 | 0. 001184 |
| rs934308 | 2 | 82391200 | NA | 3. 78E-05 | 0.00518 | 0. 001199 |
| rs10432699 | 2 | 82391773 | NTAN1 | 3. 78E-05 | 0.00518 | 0.001199 |
| rs934309 | 2 | 82393511 | NTAN1 | 3. 78E-05 | 0.00518 | 0.001199 |
| rs10190586 | 2 | 82394929 | NTAN1 | 3. 78E-05 | 0.00518 | 0. 001199 |
| rs4852215 | 2 | 82401288 | NA | 3. 78E-05 | 0.00518 | 0. 001199 |
| rs11676296 | 2 | 82402793 | NTAN1 | 4. 29E-05 | 0. 005045 | 0.001177 |

[Table 7-2]

| SNP ID | chr | position | gene locus | p-value | BETA | SE |
|---|---|---|---|---|---|---|
| rs1367442 | 2 | 82403420 | NTAN1 | 3.78E-05 | 0.00518 | 0.001199 |
| rs1430723 | 2 | 82405835 | NTAN1 | 3.78E-05 | 0.00518 | 0.001199 |
| rs4516453 | 2 | 82405937 | NTAN1 | 3.78E-05 | 0.00518 | 0.001199 |
| rs2902367 | 2 | 82407091 | RBF0X1 | 3.78E-05 | 0.00518 | 0.001199 |
| rs1430725 | 2 | 82410156 | NA | 3.78E-05 | 0.00518 | 0.001199 |
| rs7561758 | 2 | 82410827 | GAS7 | 3.78E-05 | 0.00518 | 0.001199 |
| rs11894492 | 2 | 82413470 | GAS7 | 3.78E-05 | 0.00518 | 0.001199 |
| rs12471807 | 2 | 82413980 | GAS7 | 4.29E-05 | 0.005045 | 0.001177 |
| rs4571075 | 2 | 82433870 | NA | 3.78E-05 | 0.00518 | 0.001199 |
| rs7599433 | 2 | 82436635 | NA | 3.78E-05 | 0.00518 | 0.001199 |
| rs4852682 | 2 | 82440268 | NA | 3.78E-05 | 0.00518 | 0.001199 |
| rs11690894 | 2 | 82452553 | NA | 3.78E-05 | 0.00518 | 0.001199 |
| rs16856198 | 2 | 131609539 | NA | 4.12E-06 | -0.003659 | 0.0007494 |
| rs12464519 | 2 | 131622215 | NA | 4.70E-05 | -0.003333 | 0.0007821 |
| rs7573895 | 2 | 131625295 | NA | 5.84E-06 | -0.003678 | 0.0007668 |
| rs34469492 | 2 | 131628354 | ARHGEF4 | 4.34E-05 | -0.003994 | 0.0009324 |
| 2:131629169:TAC:T | 2 | 131629169 | ARHGEF4 | 2.60E-05 | -0.003487 | 0.0007894 |
| rs3072355 | 2 | 131631093 | ARHGEF4 | 5.84E-06 | -0.003678 | 0.0007668 |
| rs12693750 | 2 | 154396396 | ARHGEF4 | 3.20E-05 | 0.00301 | 0.0006898 |
| rs1595456 | 2 | 154396518 | NA | 3.20E-05 | 0.00301 | 0.0006898 |

(continued)

| SNP ID | chr | position | gene locus | p-value | BETA | SE |
|---|---|---|---|---|---|---|
| rs6747679 | 2 | 154397389 | ARHGEF4 | 2.56E-05 | 0.003056 | 0.0006913 |
| rs6736497 | 2 | 154397886 | NA | 2.56E-05 | 0.003056 | 0.0006913 |
| rs7605995 | 2 | 154398711 | NA | 2.56E-05 | 0.003056 | 0.0006913 |
| rs7603189 | 2 | 154398824 | NA | 2.56E-05 | 0.003056 | 0.0006913 |
| 2:154398862:C:A | 2 | 154398862 | NA | 2.19E-05 | 0.00326 | 0.0007305 |
| 2:154398866:C:A | 2 | 154398866 | NA | 1.32E-05 | 0.003349 | 0.0007293 |
| 2:154398870:C:A | 2 | 154398870 | NA | 1.32E-05 | 0.003349 | 0.0007293 |
| 2:154411738:T:C | 2 | 154411738 | NA | 1.41E-05 | 0.003543 | 0.0007745 |
| 2:154411747:A:G | 2 | 154411747 | NA | 1.41E-05 | 0.003543 | 0.0007745 |
| rs77398198 | 2 | 173160555 | NA | 4.21E-05 | 0.008172 | 0.001905 |
| rs55687168 | 2 | 212203087 | NA | 4.60E-05 | -0.003447 | 0.0008076 |
| rs16845916 | 2 | 212203146 | NA | 4.60E-05 | -0.003447 | 0.0008076 |
| rs74342940 | 2 | 238791983 | NA | 3.67E-05 | -0.004006 | 0.0009257 |
| rs4685035 | 3 | 13847602 | NA | 3.51E-05 | 0.003475 | 0.0008009 |
| rs4685036 | 3 | 13848830 | NA | 3.51E-05 | 0.003475 | 0.0008009 |
| rs6777825 | 3 | 13850933 | RAMP1 | 4.94E-05 | 0.003188 | 0.0007504 |
| rs9812834 | 3 | 76104023 | NRXN1 | 4.85E-05 | 0.003659 | 0.0008602 |
| rs66550319 | 3 | 77725567 | NRXN1 | 1.37E-05 | -0.003683 | 0.0008037 |
| rs6444327 | 3 | 167158977 | NRXN1 | 4.49E-05 | 0.003547 | 0.0008298 |
| rs9874384 | 3 | 167161621 | NRXN1 | 4.49E-05 | 0.003547 | 0.0008298 |
| rs12487400 | 3 | 167162076 | NA | 4.49E-05 | 0.003547 | 0.0008298 |
| rs9859743 | 3 | 167162439 | NA | 3.37E-06 | 0.004233 | 0.0008583 |
| rsl3085856 | 3 | 167164661 | NA | 3.37E-06 | 0.004233 | 0.0008583 |

[Table 7-3]

| SNP ID | chr | position | gene locus | p-value | BETA | SE |
|---|---|---|---|---|---|---|
| rs13081388 | 3 | 167164968 | NA | 3.37E-06 | 0.004233 | 0.0008583 |
| rs9853197 | 3 | 167166760 | NA | 3.37E-06 | 0.004233 | 0.0008583 |
| rs11373865 | 3 | 167167493 | NA | 4.49E-05 | 0.003547 | 0.0008298 |
| rs1104570 | 3 | 167167997 | NA | 3.46E-06 | 0.004181 | 0.0008489 |
| rs9863185 | 3 | 167168148 | NA | 3.37E-06 | 0.004233 | 0.0008583 |
| 3:167200293:T:TA | 3 | 167200293 | NA | 2.67E-05 | 0.003413 | 0.0007738 |
| rs74715659 | 3 | 187690581 | NA | 4.57E-05 | -0.007549 | 0.001768 |
| rs78575164 | 3 | 187691168 | NA | 4.57E-05 | -0.007549 | 0.001768 |
| rs77292072 | 3 | 187692633 | NA | 4.57E-05 | -0.007549 | 0.001768 |
| rs78313080 | 3 | 187693634 | NA | 4.57E-05 | -0.007549 | 0.001768 |
| rs17745214 | 3 | 187695429 | NA | 4.57E-05 | -0.007549 | 0.001768 |
| rs17807098 | 3 | 187696291 | NA | 4.57E-05 | -0.007549 | 0.001768 |
| rs60889349 | 3 | 187696727 | NA | 4.57E-05 | -0.007549 | 0.001768 |

(continued)

| SNP ID | chr | position | gene locus | p-value | BETA | SE |
|---|---|---|---|---|---|---|
| rs56398386 | 4 | 4890108 | NA | 2.39E-05 | -0.00381 | 0.0008583 |
| rs62291849 | 4 | 4910704 | NA | 3.16E-05 | -0.00353 | 0.0008085 |
| rs6446700 | 4 | 4911642 | NA | 1.87E-05 | 0.003329 | 0.0007392 |
| rs10029683 | 4 | 4926097 | NA | 1.77E-05 | 0.004201 | 0.0009301 |
| rs10866392 | 4 | 32100576 | NA | 7.76E-06 | 0.004117 | 0.0008712 |
| rs7434982 | 4 | 32102920 | NA | 7.76E-06 | 0.004117 | 0.0008712 |
| rs7700063 | 4 | 32108077 | NA | 7.76E-06 | 0.004117 | 0.0008712 |
| rs4331815 | 4 | 32109975 | NA | 1.91E-05 | 0.003952 | 0.0008789 |
| rs4501264 | 4 | 32113715 | NA | 1.91E-05 | 0.003952 | 0.0008789 |
| 4:32135307:A:T | 4 | 32135307 | NA | 3.67E-05 | 0.003826 | 0.0008841 |
| rs12502887 | 4 | 36986503 | NA | 4.85E-05 | 0.003327 | 0.0007822 |
| rs10034215 | 4 | 37006612 | NA | 4.89E-05 | 0.003288 | 0.0007733 |
| rs10023279 | 4 | 37006976 | NA | 4.89E-05 | 0.003288 | 0.0007733 |
| rs113390631 | 4 | 37007446 | NA | 4.89E-05 | 0.003288 | 0.0007733 |
| rs7686039 | 4 | 37008671 | NA | 4.89E-05 | 0.003288 | 0.0007733 |
| rs36022714 | 4 | 37009144 | NA | 4.89E-05 | 0.003288 | 0.0007733 |
| rs13111377 | 4 | 37013371 | NA | 4.89E-05 | 0.003288 | 0.0007733 |
| rs9995577 | 4 | 37014190 | NA | 4.89E-05 | 0.003288 | 0.0007733 |
| rs10028774 | 4 | 37014223 | NA | 4.89E-05 | 0.003288 | 0.0007733 |
| rs13127134 | 4 | 37019361 | NA | 4.89E-05 | 0.003288 | 0.0007733 |
| rs13111292 | 4 | 37020558 | NA | 4.89E-05 | 0.003288 | 0.0007733 |
| rs9306943 | 4 | 37024228 | NA | 4.89E-05 | 0.003288 | 0.0007733 |
| rs1885879 | 4 | 37029967 | NA | 2.65E-05 | 0.003523 | 0.0007986 |
| rs13145678 | 4 | 37030696 | NA | 4.89E-05 | 0.003288 | 0.0007733 |
| rs34180164 | 4 | 37030722 | NA | 2.65E-05 | 0.003523 | 0.0007986 |
| rs77850243 | 4 | 37031126 | NA | 2.65E-05 | 0.003523 | 0.0007986 |
| rs145655984 | 4 | 37031740 | NA | 2.65E-05 | 0.003523 | 0.0007986 |
| rs11417369 | 4 | 37032249 | NA | 2.65E-05 | 0.003523 | 0.0007986 |
| rs1885880 | 4 | 37034278 | NA | 2.65E-05 | 0.003523 | 0.0007986 |
| rs17289939 | 4 | 37036317 | NA | 4.89E-05 | 0.003288 | 0.0007733 |

[Table 7-4]

| SNP ID | chr | position | gene locus | p-value | BETA | SE |
|---|---|---|---|---|---|---|
| rs34767825 | 4 | 37038306 | NA | 4.89E-05 | 0.003288 | 0.0007733 |
| rs61540608 | 4 | 37040276 | JPH2 | 3.15E-05 | 0.003563 | 0.0008159 |
| rs17289946 | 4 | 37042065 | NA | 4.89E-05 | 0.003288 | 0.0007733 |
| rs13127946 | 4 | 37042611 | NA | 4.89E-05 | 0.003288 | 0.0007733 |

(continued)

| SNP ID | chr | position | gene locus | p-value | BETA | SE |
|---|---|---|---|---|---|---|
| rs34800491 | 4 | 37044502 | NA | 3.15E-05 | 0.003563 | 0.0008159 |
| 4:37044634:AG:A | 4 | 37044634 | NA | 3.15E-05 | 0.003563 | 0.0008159 |
| rs7655238 | 4 | 37045620 | NA | 3.96E-05 | 0.003319 | 0.0007705 |
| rs11096857 | 4 | 37046140 | NA | 4.89E-05 | 0.003288 | 0.0007733 |
| rs2608817 | 4 | 39427201 | SERPINI2 | 4.28E-05 | 0.003767 | 0.0008788 |
| rs200342845 | 4 | 39428057 | SERPINI2 | 3.49E-05 | 0.003931 | 0.0009057 |
| rs1979283 | 4 | 39430969 | SERPINI2 | 3.49E-05 | 0.003931 | 0.0009057 |
| rs2687978 | 4 | 39431576 | SERPINI2 | 3.49E-05 | 0.003931 | 0.0009057 |
| 4:39432027:T:C | 4 | 39432027 | SERPINI2 | 3.49E-05 | 0.003931 | 0.0009057 |
| rs1542423 | 4 | 39432132 | SERPINI2 | 3.49E-05 | 0.003931 | 0.0009057 |
| rs2926042 | 4 | 39432747 | SERPINI2 | 3.49E-05 | 0.003931 | 0.0009057 |
| rs3107672 | 4 | 39432966 | SERPINI2 | 3.49E-05 | 0.003931 | 0.0009057 |
| rs12643330 | 4 | 74729606 | SERPINI2 | 3.45E-05 | 0.003579 | 0.000824 |
| rs28391070 | 4 | 166476773 | NA | 3.05E-05 | 0.00662 | 0.001513 |
| rs28487969 | 4 | 166478246 | NA | 3.05E-05 | 0.00662 | 0.001513 |
| rs7658683 | 4 | 166478405 | NA | 3.05E-05 | 0.00662 | 0.001513 |
| rs7659327 | 4 | 166478477 | NA | 1.84E-05 | 0.006554 | 0.001454 |
| rs1914836 | 4 | 166482946 | NA | 3.05E-05 | 0.00662 | 0.001513 |
| 4:166484748:CT:C | 4 | 166484748 | NA | 3.05E-05 | 0.00662 | 0.001513 |
| rs79544601 | 4 | 166484791 | NA | 3.05E-05 | 0.00662 | 0.001513 |
| rs17046745 | 4 | 166485142 | NA | 3.05E-05 | 0.00662 | 0.001513 |
| rs1914835 | 4 | 166485729 | R0B02 | 3.05E-05 | 0.00662 | 0.001513 |
| rs1914834 | 4 | 166485893 | NA | 3.05E-05 | 0.00662 | 0.001513 |
| rs6831798 | 4 | 166487806 | NA | 3.05E-05 | 0.00662 | 0.001513 |
| rs6855033 | 4 | 166488036 | NA | 3.05E-05 | 0.00662 | 0.001513 |
| rs10017297 | 4 | 166488615 | NA | 3.05E-05 | 0.00662 | 0.001513 |
| rs150507584 | 4 | 166488986 | NA | 3.05E-05 | 0.00662 | 0.001513 |
| rs6819473 | 4 | 166489595 | NA | 3.05E-05 | 0.00662 | 0.001513 |
| rs17046756 | 4 | 166491564 | NA | 3.05E-05 | 0.00662 | 0.001513 |
| rs7694281 | 4 | 166492825 | NA | 3.05E-05 | 0.00662 | 0.001513 |
| rs7694612 | 4 | 166492946 | NA | 3.05E-05 | 0.00662 | 0.001513 |
| rs28583482 | 4 | 166494345 | NA | 3.05E-05 | 0.00662 | 0.001513 |
| rs10517996 | 4 | 169425778 | NA | 3.77E-05 | 0.003653 | 0.0008456 |
| rs2218255 | 4 | 180035723 | NA | 4.26E-05 | 0.003454 | 0.0008055 |
| rs4410622 | 5 | 4672392 | NA | 2.40E-05 | 0.003959 | 0.000892 |
| rs1496366 | 5 | 7146388 | NA | 2.89E-05 | 0.005429 | 0.001237 |
| rs1006152 | 5 | 7151032 | NA | 4.65E-05 | 0.004994 | 0.001171 |
| rs1494558 | 5 | 35861068 | NA | 4.59E-05 | 0.003946 | 0.0009245 |

(continued)

| SNP ID | chr | position | gene locus | p-value | BETA | SE |
|---|---|---|---|---|---|---|
| rs6888116 | 5 | 118659579 | NA | 1.24E-06 | 0.004187 | 0.0008094 |

[Table 7-5]

| SNP ID | chr | position | gene locus | p-value | BETA | SE |
|---|---|---|---|---|---|---|
| rs1876736 | 5 | 118665393 | NA | 1.27E-05 | 0.003771 | 0.0008195 |
| rs250307 | 5 | 118667748 | NA | 2.03E-06 | 0.004078 | 0.0008067 |
| rs250306 | 5 | 118669094 | NA | 2.33E-06 | 0.004017 | 0.0008 |
| rs1895202 | 5 | 118669851 | PALLD | 1.29E-06 | 0.004141 | 0.000802 |
| rs250305 | 5 | 118670120 | NA | 2.49E-06 | 0.003929 | 0.000785 |
| rs1355123 | 5 | 118675585 | NA | 3.54E-05 | 0.003531 | 0.0008141 |
| rs2652696 | 5 | 132139308 | NA | 2.18E-05 | 0.003755 | 0.0008413 |
| rs708460 | 5 | 132144898 | NA | 2.06E-05 | 0.004009 | 0.0008954 |
| rs254289 | 5 | 132167542 | NA | 3.10E-05 | 0.004016 | 0.0009187 |
| rs254287 | 5 | 132172048 | NA | 4.27E-05 | 0.003922 | 0.0009148 |
| rs254293 | 5 | 132178571 | NA | 3.10E-05 | 0.004016 | 0.0009187 |
| rs809140 | 5 | 132193555 | NA | 2.11E-05 | 0.003748 | 0.0008381 |
| rs254285 | 5 | 132198287 | NA | 3.73E-05 | 0.003462 | 0.0008009 |
| rs2431138 | 5 | 174833410 | NA | 1.02E-05 | -0.00345 | 0.0007408 |
| rs265984 | 5 | 174836280 | NA | 9.66E-06 | -0.003603 | 0.0007715 |
| 6:32579277:C:T | 6 | 32579277 | NA | 4.11E-05 | 0.003062 | 0.0007126 |
| rs116953562 | 6 | 32676388 | NA | 4.34E-05 | 0.006323 | 0.001476 |
| rs114931041 | 6 | 32677158 | NA | 4.34E-05 | 0.006323 | 0.001476 |
| rs116823632 | 6 | 32677235 | NA | 4.34E-05 | 0.006323 | 0.001476 |
| rs112057165 | 6 | 32677822 | NA | 4.34E-05 | 0.006323 | 0.001476 |
| rs114912316 | 6 | 32677960 | NA | 4.34E-05 | 0.006323 | 0.001476 |
| rs114134393 | 6 | 32678064 | LINC02616 | 4.34E-05 | 0.006323 | 0.001476 |
| rs116915647 | 6 | 32679726 | NA | 4.34E-05 | 0.006323 | 0.001476 |
| rs114788646 | 6 | 32679762 | NA | 4.34E-05 | 0.006323 | 0.001476 |
| rs116115695 | 6 | 32680012 | NA | 4.34E-05 | 0.006323 | 0.001476 |
| rs114245356 | 6 | 32680153 | NA | 4.34E-05 | 0.006323 | 0.001476 |
| rs115025251 | 6 | 32680267 | NA | 4.34E-05 | 0.006323 | 0.001476 |
| rs115719910 | 6 | 32680352 | NA | 4.34E-05 | 0.006323 | 0.001476 |
| rs115942220 | 6 | 32680448 | NA | 4.34E-05 | 0.006323 | 0.001476 |
| rs114095721 | 6 | 32680867 | NA | 4.34E-05 | 0.006323 | 0.001476 |
| rs115938991 | 6 | 32681079 | NA | 4.34E-05 | 0.006323 | 0.001476 |
| rs115036360 | 6 | 32681308 | NA | 4.34E-05 | 0.006323 | 0.001476 |
| rs2254737 | 7 | 17811797 | NA | 9.85E-06 | -0.004268 | 0.0009147 |
| rs2110015 | 7 | 17812739 | NA | 4.12E-05 | -0.003981 | 0.0009265 |

(continued)

| SNP ID | chr | position | gene locus | p-value | BETA | SE |
|---|---|---|---|---|---|---|
| rs2254594 | 7 | 17813614 | NA | 4.12E-05 | -0.003981 | 0.0009265 |
| rs2723521 | 7 | 17813651 | NA | 4.12E-05 | -0.003981 | 0.0009265 |
| rs2723520 | 7 | 17813677 | NA | 4.12E-05 | -0.003981 | 0.0009265 |
| rs2723519 | 7 | 17814005 | NA | 4.12E-05 | -0.003981 | 0.0009265 |
| rs1404419 | 7 | 17814029 | NA | 4.12E-05 | -0.003981 | 0.0009265 |
| rs2723518 | 7 | 17814046 | KLB | 4.12E-05 | -0.003981 | 0.0009265 |
| rs2537592 | 7 | 17814051 | KLB | 4.12E-05 | -0.003981 | 0.0009265 |
| rs1404418 | 7 | 17814085 | KLB | 4.12E-05 | -0.003981 | 0.0009265 |
| rs1404417 | 7 | 17814165 | KLB | 4.12E-05 | -0.003981 | 0.0009265 |

[Table 7-6]

| SNP ID | chr | position | gene locus | p-value | BETA | SE |
|---|---|---|---|---|---|---|
| rs2723517 | 7 | 17814202 | NA | 4. 12E-05 | -0. 003981 | 0. 0009265 |
| rs1404416 | 7 | 17814272 | KLB | 4. 12E-05 | -0. 003981 | 0. 0009265 |
| rs2723516 | 7 | 17814284 | KLB | 4. 12E-05 | -0. 003981 | 0. 0009265 |
| rs2537593 | 7 | 17814531 | KLB | 4. 12E-05 | -0. 003981 | 0.0009265 |
| rs2537594 | 7 | 11814536 | NA | 4. 12E-05 | -0. 003981 | 0. 0009265 |
| rs2723514 | 7 | 17814548 | NA | 4. 12E-05 | -0. 003981 | 0. 0009265 |
| rs2068172 | 7 | 17814607 | NA | 4. 12E-05 | -0. 003981 | 0. 0009265 |
| 7:17814644:AT:A | 7 | 17814644 | NA | 4. 12E-05 | -0. 003981 | 0. 0009265 |
| rs2723513 | 7 | 17814888 | NA | 4. 12E-05 | -0. 003981 | 0. 0009265 |
| rs2254361 | 7 | 17815625 | TNFAIP8 | 4. 12E-05 | -0. 003981 | 0. 0009265 |
| rs2691617 | 7 | 17822328 | TNFAIP8 | 4. 28E-05 | -0. 004001 | 0.0009332 |
| rs2691621 | 7 | 17822450 | TNFAIP8 | 3. 69E-05 | -0. 003657 | 0. 0008453 |
| rs146211890 | 7 | 17823353 | TNFAIP8 | 3. 69E-05 | -0. 003657 | 0.0008453 |
| rs145940129 | 7 | 17823499 | TNFAIP8 | 3. 69E-05 | -0. 003657 | 0. 0008453 |
| rs2691625 | 7 | 17823624 | TNFAIP8 | 3. 69E-05 | -0. 003657 | 0. 0008453 |
| rs72079142 | 7 | 17824200 | TNFAIP8 | 3. 69E-05 | -0. 003657 | 0.0008453 |
| rs2691627 | 7 | 17824251 | SEPTIN8 | 3. 69E-05 | -0. 003657 | 0. 0008453 |
| rs2691628 | 7 | 17824356 | NA | 3. 69E-05 | -0. 003657 | 0. 0008453 |
| rs2691629 | 7 | 17824631 | NA | 3. 69E-05 | -0. 003657 | 0. 0008453 |
| rs2723500 | 7 | 17824735 | NA | 3. 69E-05 | -0. 003657 | 0. 0008453 |
| rs2691630 | 7 | 17824787 | NA | 3. 69E-05 | -0. 003657 | 0. 0008453 |
| rs2723499 | 7 | 17824885 | NA | 3. 69E-05 | -0. 003657 | 0.0008453 |
| rs2723498 | 7 | 17824986 | GDF9 | 3. 69E-05 | -0. 003657 | 0. 0008453 |
| rs2254407 | 7 | 17825281 | NA | 1. 47E-05 | -0. 003843 | 0.0008419 |
| rs998342 | 7 | 17825589 | NA | 3. 69E-05 | -0. 003657 | 0. 0008453 |

(continued)

| SNP ID | chr | position | gene locus | p-value | BETA | SE |
|---|---|---|---|---|---|---|
| rs2254176 | 7 | 17825605 | IL7R | 3. 69E-05 | -0. 003657 | 0.0008453 |
| rs144839998 | 7 | 17825719 | NA | 3. 69E-05 | -0. 003657 | 0. 0008453 |
| rs2723497 | 7 | 17825828 | NA | 3. 69E-05 | -0. 003657 | 0. 0008453 |
| rs2723496 | 7 | 17825991 | NA | 3. 69E-05 | -0. 003657 | 0. 0008453 |
| rs2691553 | 7 | 17826282 | NA | 3. 69E-05 | -0. 003657 | 0. 0008453 |
| rs1852010 | 7 | 17826368 | NA | 3. 69E-05 | -0. 003657 | 0. 0008453 |
| rs1852011 | 7 | 17826628 | NA | 3. 69E-05 | -0. 003657 | 0. 0008453 |
| rs1404420 | 7 | 17831018 | NA | 3. 53E-05 | -0. 00393 | 0. 0009059 |
| rs2691583 | 7 | 17831806 | NA | 3. 69E-05 | -0. 003657 | 0. 0008453 |
| rs2293768 | 7 | 100348384 | NA | 1. 42E-05 | 0. 003728 | 0. 0008154 |
| rs78389571 | 7 | 100368389 | NA | 3. 77E-05 | 0. 003504 | 0. 0008111 |
| rs2293766 | 7 | 100371358 | NA | 3. 77E-05 | 0. 003504 | 0. 0008111 |
| rs17147741 | 7 | 100371583 | NA | 3. 77E-05 | 0. 003504 | 0. 0008111 |
| 7:100372539:GTTTTTTGT:G | 7 | 100372539 | NA | 3. 77E-05 | 0. 003504 | 0. 0008111 |
| rs56872215 | 7 | 100376414 | NA | 3. 77E-05 | 0. 003504 | 0. 0008111 |
| rs75119362 | 7 | 100376679 | NA | 3. 77E-05 | 0. 003504 | 0. 0008111 |
| rs77017835 | 7 | 100377364 | NA | 3. 77E-05 | 0. 003504 | 0. 0008111 |
| rs113223392 | 7 | 100378274 | NA | 4.61E-06 | 0. 003707 | 0. 0007636 |

[Table 7-7]

| SNP ID | chr | position | gene locus | p-value | BETA | SE |
|---|---|---|---|---|---|---|
| 7:100382845:CTT:C | 7 | 100382845 | NA | 5.41E-06 | 0.003783 | 0.0007857 |
| rs2622261 | 7 | 153522341 | NA | 2. 86E-05 | -0.004194 | 0.0009549 |
| rs2622262 | 7 | 153522368 | NA | 2. 86E-05 | -0.004194 | 0.0009549 |
| rs2622263 | 7 | 153522433 | ZAN | 2. 86E-05 | -0.004194 | 0.0009549 |
| rs113278579 | 7 | 153522636 | ZAN | 3. 33E-05 | -0.00348 | 0.0007995 |
| rs2538419 | 7 | 153522987 | ZAN | 2. 86E-05 | -0. 004194 | 0. 0009549 |
| rs2538418 | 7 | 153523926 | ZAN | 2. 86E-05 | -0. 004194 | 0. 0009549 |
| rs10687210 | 7 | 153524089 | NA | 2. 86E-05 | -0.004194 | 0.0009549 |
| rs1544613 | 7 | 153524357 | ZAN | 2. 86E-05 | -0.004194 | 0.0009549 |
| rs1544612 | 7 | 153524514 | ZAN | 2. 86E-05 | -0. 004194 | 0. 0009549 |
| rs1544611 | 7 | 153525542 | ZAN | 1. 87E-05 | -0.003952 | 0.0008775 |
| rs2622241 | 7 | 153526331 | ZAN | 1. 87E-05 | -0. 003952 | 0. 0008775 |
| rs2622242 | 7 | 153526464 | NA | 1.87E-05 | -0.003952 | 0.0008775 |
| rs2070494 | 8 | 37688733 | NA | 3. 12E-05 | -0.006028 | 0.00138 |
| rs10957210 | 8 | 62969408 | NA | 2. 89E-05 | -0. 003742 | 0. 0008525 |
| rs4871749 | 8 | 127897460 | NA | 2. 66E-05 | 0.004427 | 0.001003 |
| rs1863563 | 8 | 129020784 | NA | 3.81E-05 | 0.003228 | 0.0007476 |

(continued)

| SNP ID | chr | position | gene locus | p-value | BETA | SE |
|---|---|---|---|---|---|---|
| rs12550891 | 9 | 26569055 | NA | 4. 29E-05 | 0.003526 | 0.0008227 |
| rs1033988 | 9 | 26571951 | NA | 3. 97E-05 | 0.003622 | 0.0008412 |
| rs10819940 | 9 | 98927435 | NA | 2. 60E-05 | 0.004089 | 0.0009257 |
| rs10819941 | 9 | 98927481 | NA | 4. 54E-05 | 0.003969 | 0.0009291 |
| rs4743609 | 9 | 98930736 | NA | 4. 54E-05 | 0.003969 | 0.0009291 |
| rs4743611 | 9 | 98932408 | NA | 2. 15E-05 | 0.004245 | 0.0009505 |
| rs10819949 | 9 | 98934331 | NA | 1.21E-05 | 0.004346 | 0.0009421 |
| rs4742873 | 9 | 98950164 | NA | 8. 78E-06 | 0.004305 | 0.0009169 |
| rs4742876 | 9 | 98955876 | NA | 8. 78E-06 | 0.004305 | 0.0009169 |
| rs7020106 | 9 | 98974141 | NA | 4. 16E-05 | 0.003553 | 0.0008273 |
| rs7047022 | 9 | 98974144 | NA | 4.16E-05 | 0.003553 | 0.0008273 |
| rs7035399 | 9 | 101776487 | NA | 3. 90E-05 | -0.003388 | 0.0007859 |
| rs77405629 | 9 | 112867809 | NA | 3. 49E-05 | -0.004905 | 0.00113 |
| rs1 7806439 | 9 | 112886940 | NA | 1. 34E-06 | -0.006842 | 0. 001327 |
| rs13297551 | 9 | 120961083 | NA | 4. 56E-05 | 0.004668 | 0. 001093 |
| rs71895773 | 9 | 120961500 | NA | 4. 56E-05 | 0.004668 | 0.001093 |
| rs79652695 | 10 | 6976748 | NA | 1.91E-05 | -0.01037 | 0. 002306 |
| rs75366909 | 10 | 68602734 | NA | 3. 35E-05 | 0.008866 | 0.002037 |
| rs17231504 | 10 | 68650441 | NA | 9.91E-06 | 0.008563 | 0.001836 |
| rs1320042 | 11 | 7444027 | NA | 4. 86E-05 | -0.003659 | 0.0008603 |
| rs4753718 | 11 | 95830567 | NA | 1. 50E-05 | 0.004151 | 0.0009105 |
| rs7944701 | 11 | 95831023 | NA | 1. 50E-05 | 0.004151 | 0.0009105 |
| rs7934906 | 11 | 119488133 | NA | 3.16E-05 | -0.003171 | 0.0007263 |
| rs1855286 | 13 | 47746652 | NA | 3.07E-05 | 0.003322 | 0.0007594 |
| rs1537457 | 13 | 47752877 | NA | 9. 37E-06 | 0.003435 | 0.0007343 |
| rs4142743 | 13 | 47756210 | NA | 4.80E-06 | 0.003634 | 0. 0007501 |

[Table 7-8]

| SNP ID | chr | position | gene locus | p-value | BETA | SE |
|---|---|---|---|---|---|---|
| rs4142742 | 13 | 47756308 | NA | 1. 13E-05 | 0.003519 | 0. 0007598 |
| rs2406095 | 13 | 47762205 | NA | 9. 50E-06 | 0.00341 | 0. 0007294 |
| rs1930826 | 13 | 47767039 | NA | 1. 82E-05 | 0. 003256 | 0. 000722 |
| rs7332153 | 13 | 47767339 | NA | 1. 83E-05 | 0.003187 | 0. 000707 |
| rs6561358 | 13 | 47770535 | NA | 1. 68E-05 | 0.003194 | 0.0007051 |
| rs7333999 | 13 | 47771258 | NA | 1. 68E-05 | 0. 003194 | 0.0007051 |
| rs10140617 | 14 | 51843558 | NA | 8. 50E-07 | -0.004466 | 0.0008485 |
| rs2356901 | 14 | 51845948 | NA | 2. 47E-06 | -0.004198 | 0. 0008384 |
| rs7153577 | 14 | 78790792 | NA | 3. 72E-05 | 0. 003444 | 0.0007964 |

(continued)

| SNP ID | chr | position | gene locus | p-value | BETA | SE |
|---|---|---|---|---|---|---|
| rs58546183 | 14 | 101716329 | NA | 2. 74E-05 | -0.004786 | 0.001087 |
| rs78489963 | 14 | 101719276 | NA | 7. 24E-06 | -0.004975 | 0.001049 |
| rs75949493 | 14 | 101719529 | NA | 5. 80E-06 | -0.004872 | 0.001015 |
| rs12587451 | 14 | 101719796 | NA | 1. 83E-06 | -0. 005025 | 0.0009891 |
| 15:87561879:AT:ATT | 15 | 87561879 | NA | 3. 56E-06 | 0.01073 | 0.002182 |
| rs2034597 | 16 | 6144047 | NA | 1. 49E-05 | 0. 003661 | 0. 0008026 |
| rs4985124 | 16 | 15125441 | NA | 3. 55E-05 | 0. 003322 | 0.0007661 |
| rs72789541 | 16 | 15127534 | NA | 3. 55E-05 | 0. 003322 | 0.0007661 |
| rs72789542 | 16 | 15127535 | NA | 3. 55E-05 | 0. 003322 | 0.0007661 |
| rs7200543 | 16 | 15129970 | NA | 3. 57E-05 | 0. 003348 | 0. 0007724 |
| rs1741 | 16 | 15130351 | NA | 3. 55E-05 | 0. 003322 | 0.0007661 |
| rs6498540 | 16 | 15130594 | NA | 3. 57E-05 | 0. 003348 | 0. 0007724 |
| rs1121 | 16 | 15131076 | NA | 3. 57E-05 | 0. 003348 | 0.0007724 |
| rs4985154 | 16 | 15131642 | NA | 3. 57E-05 | 0. 003348 | 0.0007724 |
| 16:15131654:C:CA | 16 | 15131654 | NA | 2. 05E-05 | 0. 003498 | 0.0007809 |
| rs1135999 | 16 | 15131962 | NA | 3. 57E-05 | 0. 003348 | 0.0007724 |
| rs1136001 | 16 | 15131974 | SNX13 | 3. 57E-05 | 0. 003348 | 0. 0007724 |
| rs2740 | 16 | 15132108 | SNX13 | 3. 57E-05 | 0. 003348 | 0. 0007724 |
| 16:15132211:A:AAAAGTTG | 16 | 15132211 | NA | 3. 57E-05 | 0. 003348 | 0.0007724 |
| rs34614532 | 16 | 15132908 | NA | 3. 57E-05 | 0. 003348 | 0.0007724 |
| rs14347 | 16 | 15133889 | ADGRA2 | 3. 57E-05 | 0. 003348 | 0.0007724 |
| rs4500751 | 16 | 15140211 | NA | 1. 41E-05 | 0. 003389 | 0.0007407 |
| rs6498541 | 16 | 15140657 | C0L15A1 | 4. 36E-05 | 0.00323 | 0.0007543 |
| rs7192753 | 16 | 80098249 | PALM2AKAP2 | 2. 03E-05 | 0. 003968 | 0.0008854 |
| rs1024370 | 17 | 10054798 | PALM2AKAP2 | 3. 29E-05 | -0.003259 | 0.0007481 |
| rs1024369 | 17 | 10054889 | NA | 3. 26E-05 | -0.003267 | 0. 0007496 |
| rs1468086 | 17 | 10055514 | NA | 3. 30E-05 | -0.003265 | 0. 0007496 |
| rs4794500 | 17 | 52577372 | NA | 1. 41E-05 | -0.003893 | 0. 0008508 |
| rs60809192 | 17 | 52578429 | NA | 1. 41E-05 | -0.003893 | 0. 0008508 |
| rs113532194 | 17 | 52581343 | NA | 1. 41E-05 | -0.003893 | 0. 0008508 |
| rs56347481 | 17 | 52582030 | NA | 4. 17E-05 | -0.003519 | 0. 0008194 |
| rs11871596 | 17 | 52582282 | NA | 4. 17E-05 | -0.003519 | 0. 0008194 |
| rs7226207 | 17 | 52583485 | NA | 4. 17E-05 | -0. 003519 | 0. 0008194 |
| rs753719 | 19 | 411849 | NA | 1.01E-05 | 0. 003477 | 0.0007462 |

[Table 7-9]

| SNP ID | chr | position | gene locus | p-value | BETA | SE |
|---|---|---|---|---|---|---|
| rs6028721 | 20 | 38550395 | NA | 2. 67E-05 | 0.002924 | 0. 000663 |

(continued)

| SNP ID | chr | position | gene locus | p-value | BETA | SE |
|---|---|---|---|---|---|---|
| rs761207 | 20 | 42758834 | NA | 4. 31E-05 | -0.003706 | 0. 0008649 |
| 20:60359090:G:A | 20 | 60359090 | NA | 2. 34E-05 | -0.006556 | 0. 001475 |
| rs6001258 | 22 | 39245583 | NA | 2. 48E-05 | 0.006034 | 0. 001362 |

3. Identification of therapeutic target genes

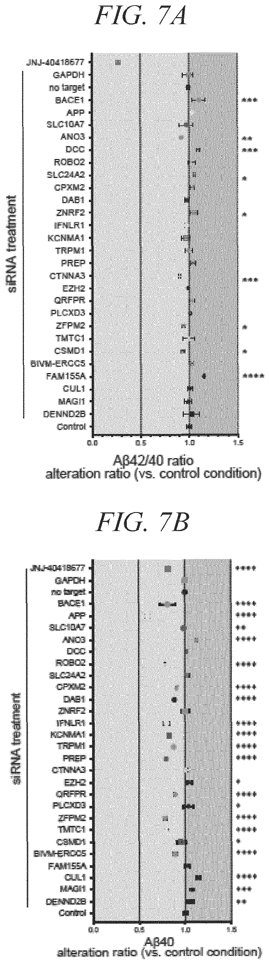

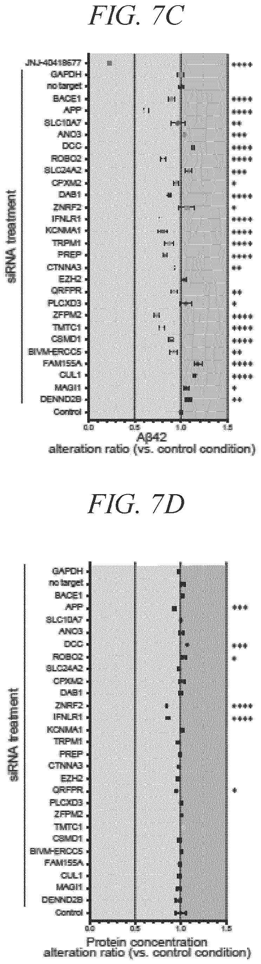

To prove a direct interaction between Aβ phenotypes and 24 genes identified through CDiP, Aβ species in a case where the identified genes were knocked down were quantified (FIGS. 7A, 7B, 7C, and FIG. 7D). Suppression of the expression of amyloid precursor protein (APP) or β-site APP-cleaving enzyme 1 (BACE1), a key component of Aβ production, reduced the amount of Aβ as expected (FIGS. 7B and 7C). Knockdown of 8 out of the 24 genes identified through CDiP significantly altered the Aβ42/40 ratio (FIG. 7A). In particular, focus was on the top three target genes, CTNNA3, ANO3, and CSMD1, with the greatest reduction in Aβ42/40 ratio. Regarding the amount of Aβ, knockdown of 23 out of 24 genes identified through CDiP altered the amount of Aβ42 or Aβ40 (FIGS. 7B and 7C). Since it is necessary for alteration in neuronal density affect the amount of Aβ42, the protein concentration was quantified after siRNA treatment before selecting genes to focus on (FIG. 7D). The results confirmed that knockdown of QRFPR, INFLRI, ZNRF2, ROBO2, DCC, and APP decreased the total protein concentration, as previously reported. Accordingly, ZNRF2, INFLR1, DCC, and APP were excluded from the candidate genes affecting the amount of Aβ42. Thereafter, focus was the top three target genes, ZFPM2, TMTC1, and KCNMA1, with the greatest reduction in amount of Aβ42.

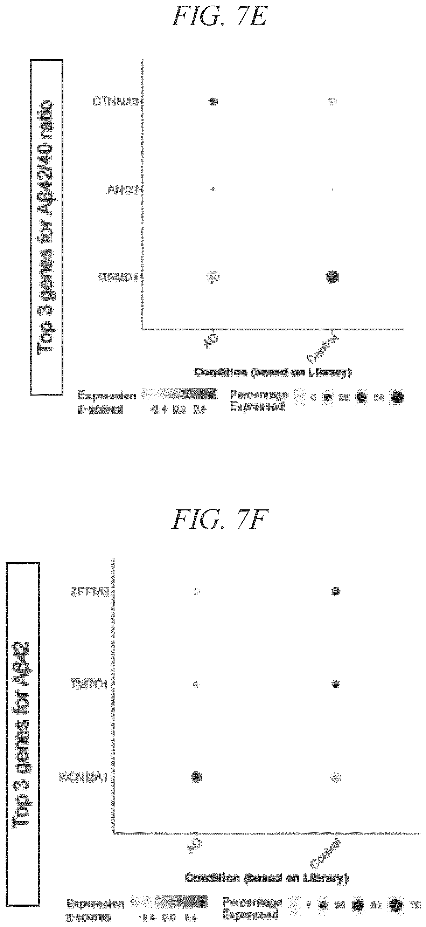

To narrow down potential targets for knockout therapy, it is necessary to select genes whose expression is elevated in brain neurons of AD patients. To examine the expression status of genes of interest in neurons of AD patients, single cell-based transcriptomic data from the cerebral cortex of six AD patient brains and six control brains was used. This provides transcriptomic data for individual cell types, including neurons, astrocytes, oligodendrocyte precursor cells, oligodendrocytes, microglia, and endothelial cells. Mean expression of focused genes such as CTNNA3, ANO3, and CSMD1 with respect to the Aβ42/40 ratio, ZFPM2, TMTC1, and KCNMA1 with respect to Aβ42, especially in neurons, was plotted (FIGS. 7E and 7F). The results revealed that CTNNA3, ANO3, and KCNMA1 were more highly expressed in the AD patient brains.

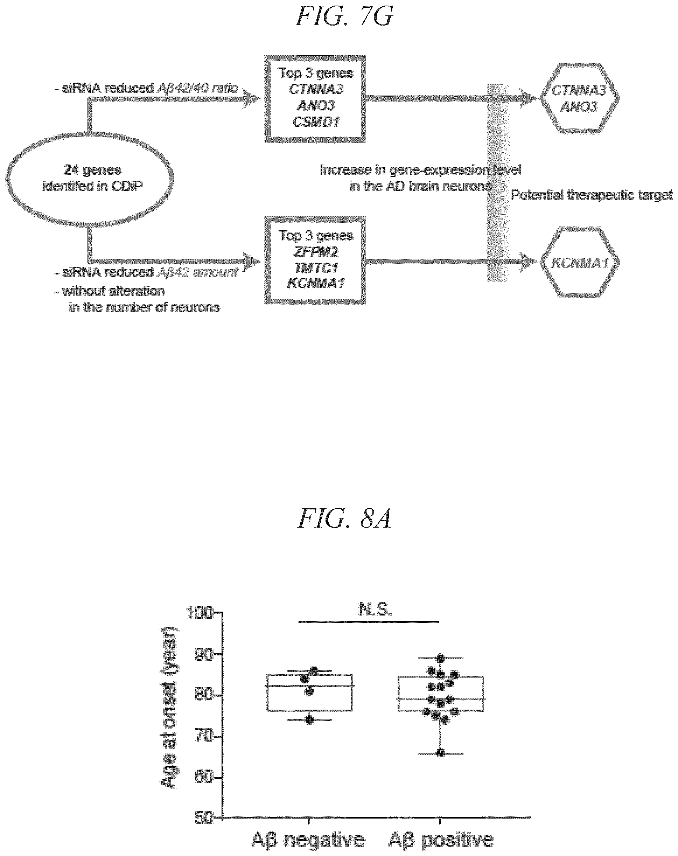

From these results, it was concluded that CTNNA3 and ANO3 with respect to the Aβ42/40 ratio and KCNMA1 with respect to the amount of Aβ42 may be potential therapeutic targets for AD (FIG. 7G).

A protein encoded by CTNNA3 plays a role in intercellular adhesion, and mutations in CTNNA3 cause familial arrhythmogenic right ventricular dysplasia caused by mishandling of electrolytes such as potassium and calcium.

A protein encoded by KCNMA1 is composed of a voltage- and calcium-sensitive potassium channel (KCa1.1) that regulates smooth muscle tone and neuronal excitability. KCa1.1 is known as a target of cromolyn, notably having been tested in phase III trials for AD.

A protein encoded by ANO3 has been reported to function in endoplasmic reticulum-dependent calcium signaling, and an ANO3 mutation causes familial dystonia type 24 via abnormal neuronal excitability.

These results suggest that the identified therapeutic targets may be involved in calcium handling, excitability, and a key pathway of Aβ regulation.

In summary, genome-wide analysis was performed by analyzing complex cell types of AD in cerebral cortical neurons and setting neuron-specific Aβ and tau phenotypes as pathological features of AD. As a result, CDiP revealed genotype sets that partially contributed to the polygenic architecture behind the disease pathomechanism of AD.

Of the 24 genes identified above, 11 genes are novel AD-associated genes whose relationship to AD has not been reported so far. The reason why these genes have not been found so far is probably because various confounding factors have become noise in clinical GWAS alone.

[Example 2]

(Prediction of onset of AD using polygene dataset obtained from cell GWAS)

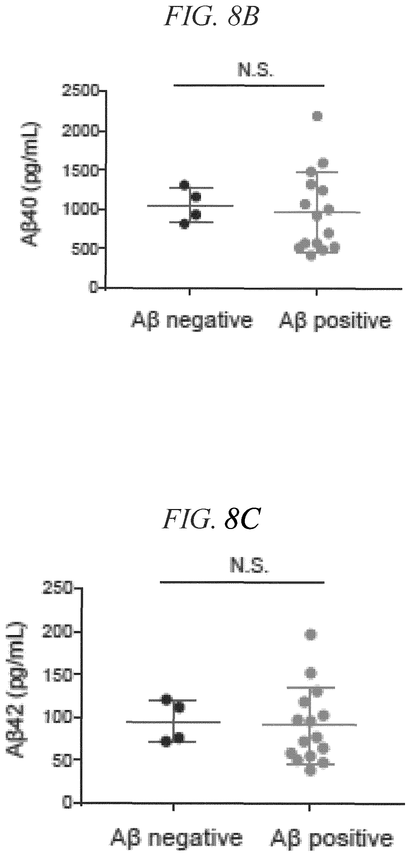

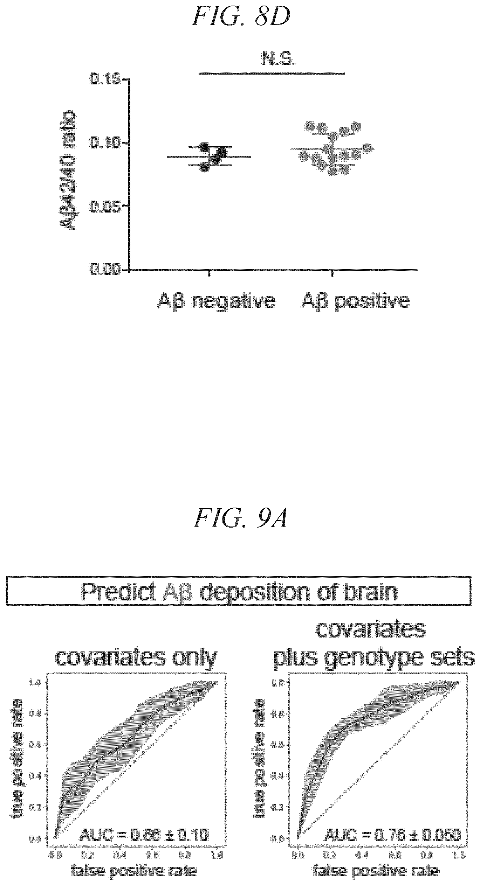

Next, the similarity between real-world data and an in vitro dataset consisting of PET imaging of Aβ deposition in brains of patients who provided PBMCs for iPS cell establishment in this study was evaluated. The correlation between the quantified Aβ phenotypes in cerebral cortical neurons and brain Aβ deposition measured by Pittsburgh Compound-B (PiB)-PET imaging was analyzed. However, neither age at onset nor Aβ phenotypes correlated with brain Aβ deposition (FIGS. 8A, 8B, 8C, and 8D).

These results confirmed that simple quantified disease phenotypes without genetic information could not reflect real-world data.

Accordingly, it was examined whether these genotype sets could be used to predict real-world big data from independent AD cohorts.

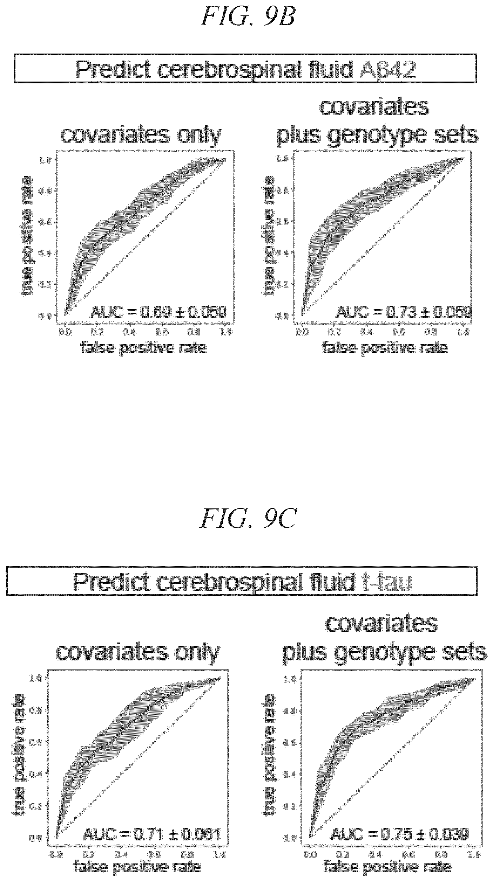

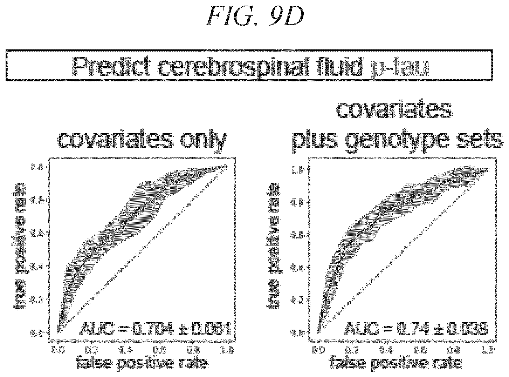

The Alzheimer's Disease Neuroimaging Initiative (ADNI) database was utilized. This includes genome-wide genotypes, brain Aβ deposition (AV45-PET), an amount of Aβ42 in cerebrospinal fluid (CSF), total tau (t-tau) levels in CSF, and phosphorylated tau (p-tau) levels in CSF. First, attempts have been made to predict positive brain Aβ deposition using only covariates consisting of age, sex, and APOE-ε4 allele genotypes, or using covariates and genotype sets. Machine learning models were established to predict positive brain Aβ deposition using only covariates consisting of age, sex, and APOE-ε4 allele genotypes, or using covariates and identified genotype sets. Using the trained models, attempts have been made to predict brain Aβ deposition, and the area under the curve (AUC) was compared between two different models. The AUC with the covariates plus genotype sets (AUC=0.76) was statistically higher than the AUC with the covariates alone (AUC=0.66) (FIG. 9A). Similarly, the covariates plus genotype sets could predict the reduction in amount of Aβ42 in CSF with significantly higher accuracy than the covariates alone (FIG. 9B). However, when predicting t-tau levels in CSF or p-tau levels in CSF, there was no significant difference in AUC between the covariates and the covariates plus genotype sets (FIGS. 9C and 9D).

Based on the above results, it was possible to predict real clinical data for AD using the genotype sets identified through CDiP. In addition, it was possible to predict whether intracerebral accumulation of Aβ would occur using the SNP information of the sporadic AD patients with an accuracy of AUC=0.76±0.050 (FIG. 9A). It was possible to predict whether abnormal test values for Aβ in cerebrospinal fluid would be generated using the SNP information of the sporadic AD patients with an accuracy of AUC=0.73±0.059 (FIG. 9B). Since these intracerebral accumulation of Aβ and abnormal test values for Aβ in cerebrospinal fluid are almost consistent with the clinical diagnosis of AD, the prediction using the information processing method of the present embodiment can also be generally extrapolated and regarded as accurate with an AUC of 0.73 to 0.76.

[Example 3]

(Discovery of rare variants by cell GWAS)

To confirm further applicability of the system to real clinical data, it was examined whether the identified gene sets formed SAD. The relevance of the genes identified in this test as rare variants was examined. These are known to be low frequency but minor factors in the onset of AD.

Genome-wide exome data from the Japanese Alzheimer's Disease Neuroimaging Initiative (J-ADNI) were used to examine rare variants at the identified gene loci. Rare variants at 24 gene loci associated with the Aβ42/40 ratio were investigated by investigating exome data from healthy donors (N=152) and SAD patients (N=255). A rare variant of KCNMA1 showed a relationship with AD (p=0.032, odds ratio=1.45) (Table 8). In Table 8, "p" means a p-value from a summation test, "se" means an approximate standard error associated with the genotype effect, "cmafTotal" means cumulative minor allele frequency of a gene, "cmafUsed" means cumulative minor allele frequency of SNPs used for analysis, "nsnpsTotal" means the number of SNPs in a gene, "nsnpsUsed" means the number of SNPs used for analysis, and "nmiss" means the number of missing SNPs. In a case of genes with a single SNP, "nmiss" is the number of individuals who did not contribute to analysis because the test did not report results for that SNP. In a case of genes with multiple SNPs, these are summed across genes. The same meaning applies to the subsequent tables.

[Table 8]

| chr | gene | p | beta | se | Cmaf Total | Cmaf Used | Nsnps Total | Nsnps Used | nmiss | OR | SE |
|---|---|---|---|---|---|---|---|---|---|---|---|
| 1 | DAB1 | 0.885 | 0.17 | 1.20 | 0.004 | 0.004 | 3 | 3 | 0 | 1.19 | 3.31 |
| 1 | IFNLR1 | 0.743 | 0.18 | 0.54 | 0.018 | 0.018 | 8 | 8 | 0 | 1.19 | 1.72 |
| 3 | MAGI1 | 0.810 | 0.07 | 0.30 | 0.055 | 0.055 | 11 | 11 | 0 | 1.07 | 1.35 |
| 3 | ROBO2 | 0.600 | -0.55 | 1.04 | 0.005 | 0.005 | 4 | 4 | 0 | 0.58 | 2.83 |
| 4 | QRFPR | 0.822 | 0.10 | 0.47 | 0.015 | 0.015 | 9 | 9 | 0 | 1.11 | 1.59 |
| 4 | SLC10A7 | 0 519 | -0.55 | 0.85 | 0.007 | 0.007 | 3 | 3 | 0 | 0.58 | 2.34 |
| 5 | PLCXD3 | NA | NA | NA | 0.000 | 0.000 | 2 | 2 | 0 | NA | NA |

(continued)

| chr | gene | p | beta | se | Cmaf Total | Cmaf Used | Nsnps Total | Nsnps Used | nmiss | OR | SE |
|---|---|---|---|---|---|---|---|---|---|---|---|
| 6 | PREP | 0.753 | -019 | 0.61 | 0.015 | 0.015 | 8 | 8 | 0 | 0.83 | 1.83 |
| 7 | CUL1 | 0.424 | -0.12 | 0.15 | 0.038 | 0.038 | 7 | 7 | 0 | 0.89 | 1.16 |
| 7 | EZH2 | 0.082 | 1.62 | 0.93 | 0.006 | 0.006 | 2 | 2 | 0 | 5.04 | 2.54 |
| 7 | ZNRF2 | 0.277 | 1.59 | 1.47 | 0.002 | 0.002 | 1 | 1 | 0 | 4.91 | 4.33 |
| 8 | CSMD1 | 0.598 | 0.09 | 0.18 | 0.141 | 0.141 | 43 | 43 | 0 | 1.10 | 1.20 |
| 8 | ZFPM2 | 0.265 | 0.50 | 0.45 | 0.025 | 0.025 | 8 | 8 | 0 | 1.65 | 1.57 |
| 9 | SLC24A2 | 0.643 | 0.25 | 0.54 | 0.016 | 0.016 | 7 | 7 | 0 | 1.28 | 1.72 |
| 10 | CPXM2 | 0266 | -047 | 0.42 | 0.021 | 0.021 | 9 | 9 | 0 | 0.63 | 1.52 |
| 10 | CTNNA3 | 0 188 | 061 | 0.46 | 0026 | 0.026 | 12 | 12 | 0 | 1.84 | 1.59 |
| 10 | KCNMA1 | 0.032 | 0.37 | 0.17 | 0.123 | 0.123 | 13 | 13 | 0 | 1.45 | 1.19 |
| 11 | ANO3 | 0.898 | 0.07 | 0.56 | 0.017 | 0.017 | 11 | 11 | 0 | 1.07 | 1.75 |
| 11 | DENND2B | 0.312 | -0.29 | 0.28 | 0.064 | 0.064 | 14 | 14 | 0 | 0.75 | 1.33 |
| 12 | TMTC1 | 0.661 | -0.21 | 0.49 | 0.023 | 0.023 | 9 | 9 | 0 | 0.81 | 1.63 |
| 13 | BIVM-ERCC5 | 0.741 | -0.17 | 0.51 | 0.021 | 0.021 | 12 | 12 | 0 | 0.84 | 1.67 |
| 13 | FAM155A | 0711 | -0.54 | 1.47 | 0.002 | 0.002 | 3 | 3 | 0 | 0.59 | 4.33 |
| 15 | TRPM1 | 0.261 | 0.40 | 0.35 | 0.047 | 0.047 | 16 | 16 | 0 | 1.49 | 1.42 |
| 18 | DCC | 0.650 | -0.10 | 0.21 | 0.118 | 0.118 | 24 | 24 | 0 | 0.91 | 1.24 |

To confirm reproducibility of rare variants in different cohorts and different ethnicities, meta-analysis was performed to investigate rare variants at 24 gene loci, and meta-analysis of J-ADNI and US-ADNI re-identified the rare variant in the KCNMA1 gene locus (p=0.010, odds ratio=1.49) (Table 9).

[Table 9]

| chr | gene | p | beta | se | Cmaf Total | Cmaf Used | Nsnps Total | Nsnps Used | nmiss | OR | SE |
|---|---|---|---|---|---|---|---|---|---|---|---|
| 1 | DAB1 | 0.223 | -0.75 | 0.61 | 0.012 | 0.012 | 9 | 9 | 3,879 | 0.47 | 1.85 |
| 1 | IFNLR1 | 0.344 | 0.44 | 0.47 | 0.024 | 0.024 | 13 | 13 | 5,867 | 1.55 | 1.59 |
| 3 | MACI1 | 0.668 | -0.08 | 0.19 | 0.078 | 0.078 | 36 | 36 | 14,007 | 0.92 | 1.21 |
| 3 | ROBO2 | 0.649 | -0.13 | 0.28 | 0.052 | 0.052 | 25 | 25 | 10,463 | 0.88 | 1.33 |
| 4 | QRFPR | 0.389 | -0.18 | 0.21 | 0.059 | 0.059 | 21 | 21 | 9,195 | 0.83 | 1.24 |
| 4 | SLC10A7 | 0.840 | -0.11 | 0.53 | 0.017 | 0.017 | 13 | 13 | 5,507 | 0.90 | 1.70 |
| 5 | PLCXD3 | 0.145 | -2.06 | 1.42 | 0.002 | 0.002 | 4 | 4 | 1,772 | 0.13 | 4.13 |
| 6 | PREP | 0.800 | 0.14 | 0.56 | 0.017 | 0.017 | 13 | 13 | 5,867 | 1.15 | 1.75 |
| 7 | CUL1 | 0.513 | -0.09 | 0.14 | 0.046 | 0.046 | 11 | 11 | 4,981 | 0.91 | 1.16 |
| 7 | EZH2 | 0.215 | 0.85 | 0.68 | 0.010 | 0.010 | 6 | 6 | 2,586 | 2.33 | 1.98 |
| 7 | ZNRF2 | 0.233 | 0.51 | 0.43 | 0.025 | 0.025 | 6 | 6 | 2,514 | 1.66 | 1.53 |
| 8 | CSMD1 | 0.187 | 0.18 | 0.14 | 0.224 | 0.224 | 111 | 111 | 46,836 | 1.20 | 1.15 |
| 8 | ZFPM2 | 0.242 | 0.31 | 0.26 | 0.064 | 0.064 | 29 | 29 | 11,900 | 1.36 | 1.30 |

(continued)

| chr | gene | p | beta | se | Cmaf Total | Cmaf Used | Nsnps Total | Nsnps Used | nmiss | OR | SE |
|---|---|---|---|---|---|---|---|---|---|---|---|
| 9 | SLC24A2 | 0917 | 0.03 | 0.29 | 0.049 | 0.049 | 21 | 21 | 8,572 | 1.03 | 1.33 |
| 10 | CPXM2 | 0.218 | -0.39 | 0.32 | 0.040 | 0.040 | 25 | 2 5 | 10,823 | 0.68 | 1.37 |
| 10 | CTNNA3 | 0.932 | 0.02 | 0.26 | 0.065 | 0.065 | 33 | 33 | 14,295 | 1.02 | 1.30 |
| 10 | KCNMA1 | 0.010 | 0.40 | 0.16 | 0.093 | 0.093 | 38 | 38 | 14,007 | 1.49 | 1.17 |
| 11 | ANO3 | 0.327 | 0.29 | 0.30 | 0.050 | 0.050 | 23 | 23 | 10,153 | 1.34 | 1.34 |
| 11 | DENND2B | 0.098 | -0.40 | 0.24 | 0.081 | 0.081 | 22 | 22 | 9,962 | 0.67 | 1.28 |
| 12 | TMTC1 | 0.954 | 0.02 | 0.28 | 0.054 | 0.054 | 23 | 23 | 10,009 | 1.02 | 1.33 |
| 13 | BIVM-ERCC5 | 0.207 | -0.34 | 0.27 | 0.053 | 0.053 | 41 | 41 | 16,114 | 0.71 | 1.31 |
| 13 | FAM155A | 0.481 | 0.26 | 0.37 | 0.031 | 0.031 | 9 | 9 | 3,879 | 1.30 | 1.45 |
| 15 | TRPM1 | 0.070 | 0.30 | 0.17 | 0.143 | 0.143 | 55 | 55 | 23,537 | 1.35 | 1.18 |
| 18 | DCC | 0.460 | -0.11 | 0.15 | 0.207 | 0.207 | 53 | 53 | 23,299 | 0.89 | 1.17 |

These results indicated that the identified gene sets are applicable for elucidating predisposing factors for the onset of SAD. It was also shown that the understanding of the genetic background of AD obtained through cell GWAS could lead to more detailed classification (that is, stratification) of AD, as specific populations could be extracted based on genetic information from a clinically homogeneous group of AD patients.

[Consideration]

This test identified risk SNPs, genes in which SNPs are located, and molecular pathways that affect $A\beta$ production in cerebral cortical neurons. Indeed, 5 out of the 24 genes identified through CDiP, namely TMTC1, CTNNA3, KCNMA1, CPXM2, and ANO3, were consistent with reported results from clinical genome-wide studies based on clinical data involving the onset of diseases or brain $A\beta$ deposition (refer to Tables 5-1 and 5-2 above). This advantage may be due to the fact that homogenous populations of cerebral cortical neurons which are major cell types functioning as resources for $A\beta$ production were used.

The newly identified genes in this test (Table 4) may not only play very important roles in the pathogenesis of AD, but may also represent potential biomarker and therapeutic target candidates. To extend the system presented here, other types of neuronal phenotypes in AD pathology can be applied to CDiP to identify the genetic background specific to each trait, such as synaptic loss, neuronal cell death, drug response, and vulnerability to environmental stress. Furthermore, new combinations of variable cell types of cell type-specific pathologies or the like and glial cells will reveal new genetic structures of hidden molecular pathologies through clinical GWAS.

Recent studies have emphasized the concept that AD is the sum of multiple cell-type pathologies. Based on a similar idea to this test, single-nucleus transcriptome from autopsy AD brains provided information on gene expression in various cell types. However, such an approach based on autopsied brain samples can take a late-stage snapshot of AD pathology that has been changing for decades. In contrast, CDiP can investigate isolated AD pathology with cell-type specificity, and can also model baseline conditions without confounding factors which may become potential noise for genome-wide studies. A limitation of CDiP is that it is based on 2D monolayer culture consisting of a single cell type. To understand the cellular interactions between various cell types, the combination of CDiP and single-nucleus transcriptome from autopsy brains of AD patients may be two of the most important tools to investigate the polygenicity of AD presented in this test (FIGS. 8B and 8C).

Furthermore, CDiP using neurons identified rare variants and potential therapeutic targets associated with $A\beta$ phenotypes. On the other hand, SNPs associated with the tau phenotypes showed more moderate and statistically significant correlations. This difference between $A\beta$ and tau showed that $A\beta$ pathology may be primarily based on neuronal polygenicity, whereas tau pathology may be composed exclusively of a plurality of non-neuronal cell types.

Indeed, previous reports have shown that inflammatory conditions and brain networks with microglia and astrocytes accelerate tau pathology. Furthermore, there is clinical evidence to suggest that APOE regulates tau pathology independently of $A\beta$ pathology. CDiP with neurons may suggest one aspect of discontinuity between $A\beta$ and tau pathol-

226

ogies.

In the future, it is expected that the integrated and comprehensive understanding of the genetic background obtained by these cell type-specific analytical approaches will lead to a better understanding of the complex pathogenesis of AD.

In this test, CDiP predicted AD real-world data, stratified rare variant-associated AD, and identified CTNNA3, ANO3, and KCNMA1 as potential therapeutic targets. CDiP serves as a screening tool to associate pathological phenotypes with hidden genotypes. On the other hand, it is also important to accumulate evidence using various modalities, such as mouse models and patient specimens, to accommodate the actual AD pathology, which is composed of various cell types and matures over decades. CDiP provides clues for understanding complex pathology, consisting of the sum of polygenicity and traits in disease target cells, paving the way for precision medicine.

[Industrial Applicability]

According to the information processing method, information processing device, and program of the present embodiment, it is possible to predict a risk of developing AD in subjects.

[Reference Signs List]

100 Information processing device
110 Detection unit
120 Processing unit
121 Acquisition unit
122 Feature amount conversion unit
123 Determination unit
124 Output control unit
125 Machine Learning unit
130 Storage unit
131 Model information

**Claims**

1.  An information processing method comprising:

    Step 1 of detecting a first SNP which is a mutation of an Alzheimer's disease-associated gene in a genomic DNA sample derived from a subject; and
    Step 2 of determining whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

2.  The information processing method according to claim 1,

    wherein the machine learning model is Random Forest including a plurality of classifiers, and
    wherein each classifier trains using a specific training dataset selected from among the plurality of training datasets based on principal component information in attribute information and genetic information of the patient who has developed Alzheimer's disease.

3.  The information processing method according to claim 1 or 2,
    wherein the plurality of training datasets contain a dataset labeled with information on the onset of Alzheimer's disease for a genotype belonging to the second SNP inferred from the second SNP through genotype imputation.

4.  The information processing method according to claim 1 or 2,
    wherein the mutations are one or more mutations shown in Tables 1-1 to 1-77.

[Table 1-1]

| SNP ID | chr | position | gene locus | SNP location in gene locus |
|---|---|---|---|---|
| rs11260631 | 1 | 5565535 | NA | |
| rs12027231 | 1 | 5566294 | NA | |
| rs140532989 | 1 | 11054703 | NA | |
| rs145588311 | 1 | 11170657 | MTOR | intron_variant |
| rs17875920 | 1 | 11801023 | AGTRAP | intron_variant |
| rs7418059 | 1 | 11803080 | AGTRAP | intron_variant |
| rs34791043 | 1 | 11803485 | NA | NA |
| rs17875934 | 1 | 11804288 | AGTRAP | intron_variant |
| rs17875948 | 1 | 11807970 | AGTRAP | intron_variant |
| rs17875954 | 1 | 11809161 | AGTRAP | intron_variant |
| rs17875967 | 1 | 11811273 | AGTRAP | intron_variant |
| rs11875970 | 1 | 11811886 | AGTRAP | intron variant |
| rs116001018 | 1 | 11816860 | NA | |
| rs147306013 | 1 | 11817560 | NA | |
| rs72641053 | 1 | 12128435 | TNFRSF8 | intron_variant |
| rs79459531 | 1 | 17605062 | PADI3 | intron_variant |
| rs79724306 | 1 | 17607570 | PADI3 | intron_variant |
| rs187218036 | 1 | 17627131 | NA | |
| rs147239745 | 1 | 17631035 | NA | |
| rs10917055 | 1 | 19495467 | UBR4 | intron_variant |
| rs57482167 | 1 | 24495578 | NA | NA |
| rs4649197 | 1 | 24495722 | IFNLR1 | intron variant |
| rs11249017 | 1 | 24496662 | IFNLR1 | intron_variant |
| rs12128905 | 1 | 24497000 | IFNLR1 | intron_variant |
| rs11249018 | 1 | 24498130 | IFNLR1 | intron_variant |
| rs3932667 | 1 | 24498696 | IFNLR1 | intron_variant |
| 1:24499617:C:CA | 1 | 24499617 | NA | NA |
| rs34212240 | 1 | 24500325 | IFNLR1 | intron_variant |
| rs3897438 | 1 | 24506247 | IFNLR1 | intron_variant |
| rs3897439 | 1 | 24506510 | IFNLR1 | intron_variant |
| rs4081342 | 1 | 24506522 | IFNLR1 | intron_variant |
| 1:2450678fi:CA:C | 1 | 24506786 | NA | NA |
| rs3897440 | 1 | 24506861 | IFNLR1 | intron_variant |
| rs6680101 | 1 | 24507433 | IFNLR1 | intron_variant |
| rs6665649 | 1 | 24507437 | IFNLR1 | intron_variant |
| rs6683433 | 1 | 24507774 | IFNLR1 | intron_variant |

[Table 1-2]

| SNP ID | chr | position | gene locus | SNP location in gene locus |
|---|---|---|---|---|
| rs10903046 | 1 | 24507855 | IFNLR1 | intron_variant |
| rs79806176 | 1 | 34231799 | CSMD2 | NMD_transcript_variant |
| rs74658700 | 1 | 34234656 | CSMD2 | NMD transcript_variant |
| rs76405888 | 1 | 34238146 | CSMD2 | NMD_transcript_variant |
| rs74224650 | 1 | 34244450 | CSMD2 | NMD_transcript_variant |
| rs58862022 | 1 | 34244587 | CSMD2 | NMD_transcript_variant |
| rs34278452 | 1 | 40588662 | NA | |
| rs12760525 | 1 | 40589754 | NA | |
| rs11800679 | 1 | 40603234 | NA | |
| rs35156697 | 1 | 40617030 | NA | |
| rs10493090 | 1 | 40626918 | NA | |
| rs11584362 | 1 | 40631460 | RLF | intron_variant |
| rs5028951 | 1 | 40632581 | RLF | intron_variant |
| rs12724048 | 1 | 40632954 | RLF | intron_variant |
| rs34795058 | 1 | 40636231 | RLF | intron_variant |
| rs12744808 | 1 | 40644934 | RLF | intron_variant |
| rs61780442 | 1 | 40649468 | RLF | intron_variant |
| rs11808248 | 1 | 40662090 | RLF | intron_variant |
| rs17878476 | 1 | 40668727 | RLF | intron_variant |
| 1:40682172:AAGAG:A | 1 | 40682172 | NA | NA |
| rs61778546 | 1 | 40687368 | RLF | intron_variant |
| rs16827064 | 1 | 40688132 | RLF | intron_variant |
| rs16827106 | 1 | 40735472 | ZMPSTE24 | intron_variant |
| rs16827109 | 1 | 40735817 | ZMPSTE24 | intron_variant |
| rs12757549 | 1 | 40742366 | ZMPSTE24 | intron_variant |
| rs71060364 | 1 | 40753372 | NA | NA |
| rs12725815 | 1 | 40753766 | ZMPSTE24 | intron_variant |
| rs76609695 | 1 | 41315101 | NA | |
| rs61773507 | 1 | 48411428 | TRABD2B | intron_variant |
| rs115906838 | 1 | 58708368 | DAB1 | intron_variant |
| rs117567026 | 1 | 58715824 | DAB1 | intron_variant |
| rs12039506 | 1 | 58721695 | DAB1 | intron_variant |
| rs12035886 | 1 | 58721704 | DAB1 | intron variant |
| rs140969406 | 1 | 58721802 | NA | NA |
| rs17117229 | 1 | 58733538 | DAB1 | intron_variant |
| rs1510fi4393 | 1 | 58734680 | DAB1 | intron_variant |

[Table 1-3]

| SNP ID | chr | position | gene locus | SNP location in gene locus |
|---|---|---|---|---|
| rs117388414 | 1 | 58745146 | DAB1 | intron_variant |
| rs 17117259 | 1 | 58753770 | DAB1 | intron_variant |
| rs17117277 | 1 | 58763802 | DAB1 | intron_variant |
| rs17117283 | 1 | 58764638 | DAB1 | intron_variant |
| rs142567959 | 1 | 58766083 | DAB1 | intron_variant |
| rs115426189 | 1 | 64539974 | R0R1 | intron_variant |
| rs138138409 | 1 | 64554364 | R0R1 | intron_variant |
| rs78457326 | 1 | 76867147 | ST6GALNAC3 | intron_variant |
| rs140199001 | 1 | 81198118 | NA | |
| rs192853907 | 1 | 81226954 | NA | |
| rs114618747 | 1 | 81240468 | NA | |
| rs117721292 | 1 | 81251534 | NA | |
| rs144408429 | 1 | 81252438 | NA | |
| rs145068891 | 1 | 81303947 | NA | |
| rs77325472 | 1 | 81308364 | NA | |
| rs142985266 | 1 | 81326827 | NA | |
| rs75819420 | 1 | 81328646 | NA | |
| rs76876118 | 1 | 81329832 | NA | |
| rs80094910 | 1 | 81330079 | NA | |
| rs10493678 | 1 | 81333502 | NA | |
| rs10493679 | 1 | 81334027 | NA | |
| rs61767086 | 1 | 89066582 | NA | |
| rs1988189 | 1 | 89082365 | NA | |
| rs4596856 | 1 | 89082375 | NA | |
| rs4259618 | 1 | 89082380 | NA | |
| rs12033271 | 1 | 89084247 | NA | |
| 1:89085206:TAAA:T | 1 | 89085206 | NA | NA |
| 1:89085227:A:G | 1 | 89085227 | NA | NA |
| rs75316566 | 1 | 91967640 | CDC7 | intron_variant |
| 1:92031771:C:CA | 1 | 92031771 | NA | NA |
| rs61209856 | 1 | 94589551 | NA | |
| rs56931023 | 1 | 94590379 | NA | |
| rs78142006 | 1 | 94590533 | NA | |
| rs72962326 | 1 | 94595330 | NA | |
| rs117428698 | 1 | 95399858 | NA | |
| rs141570401 | 1 | 95424962 | NA | |

[Table 1-4]

| SNP ID | chr | position | gene locus | SNP location in gene locus |
|---|---|---|---|---|
| 1:102574438:C:T | 1 | 102574438 | NA | NA |
| rs117439108 | 1 | 104894981 | NA | |
| rs189348849 | 1 | 104978328 | NA | |
| rs11799443 | 1 | 107989087 | NTNG1 | intron_variant |
| rs142243476 | 1 | 107991761 | NTNG1 | intron_variant |
| rs111451024 | 1 | 114832660 | NA | |
| rs76185230 | 1 | 114833460 | NA | |
| rs79125386 | 1 | 114834247 | NA | |
| rs6660468 | 1 | 114835204 | NA | |
| rs116493739 | 1 | 114836116 | NA | |
| rs77470545 | 1 | 114836560 | NA | |
| rs7522214 | 1 | 114840898 | NA | |
| rs75376490 | 1 | 114841671 | NA | |
| rs7534361 | 1 | 114844072 | NA | |
| rs148371870 | 1 | 114845387 | NA | |
| rs142305684 | 1 | 114848595 | NA | NA |
| rs199724897 | 1 | 114849666 | NA | |
| 1:114850653:T:C | 1 | 114850653 | NA | NA |
| rs142658681 | 1 | 114850730 | NA | |
| rs7540256 | 1 | 114851811 | NA | |
| rs116039729 | 1 | 114852370 | NA | |
| rs145900544 | 1 | 114852385 | NA | |
| rs142737501 | 1 | 114852517 | NA | |
| 1:114855828:T:A | 1 | 114855828 | NA | NA |
| rs41470845 | 1 | 114856929 | NA | |
| rs74580704 | 1 | 114862402 | NA | |
| rs76169339 | 1 | 114865513 | NA | |
| rs6669141 | 1 | 114865863 | NA | |
| rs12132759 | 1 | 115628467 | TSPAN2 | intron_variant |
| rs6701221 | 1 | 152778558 | LCE1C | intron_variant |
| 1:158992906:AT:A | 1 | 158992906 | NA | NA |
| rs4646881 | 1 | 165667980 | ALDH9A1 | 5_prime_UTR_variant |
| rs79887819 | 1 | 167633592 | RCSD1 | intron_variant |
| rs78645780 | 1 | 170132537 | METTL11B | intron_variant |
| rs115223117 | 1 | 170139597 | NA | |
| rs115433886 | 1 | 170140312 | NA | NA |

[Table 1-5]

| SNP ID | ch r | position | gene locus | SNP location in gene locus |
|---|---|---|---|---|
| rs7537272 | 1 | 170142133 | NA | |
| rs10564453 | 1 | 170142729 | NA | |
| rs143134448 | 1 | 170144306 | NA | |
| rs77373691 | 1 | 170146623 | NA | |
| rs6689201 | 1 | 170146938 | NA | |
| rs6671761 | 1 | 170158174 | NA | |
| rs74822235 | 1 | 170159553 | NA | |
| rs77849060 | 1 | 170161011 | NA | |
| rs17345810 | 1 | 170166066 | NA | |
| rs17349942 | 1 | 170167702 | NA | |
| rs78968813 | 1 | 170167937 | NA | |
| rs140761847 | 1 | 170169900 | NA | |
| 1:170171514:T:A | 1 | 170171514 | NA | NA |
| rs10919327 | 1 | 170171514 | NA | |
| rs17345893 | 1 | 170171874 | NA | |
| rs6662495 | 1 | 170172742 | NA | |
| rs6687139 | 1 | 170173262 | NA | |
| rs17345907 | 1 | 170174046 | NA | |
| rs12144655 | 1 | 170174298 | NA | |
| rs12142060 | 1 | 170176678 | NA | |
| rs77199503 | 1 | 170179140 | NA | |
| rs17345935 | 1 | 170179341 | NA | |
| rs17350032 | 1 | 170181363 | NA | |
| rs17350053 | 1 | 170184112 | NA | |
| rs74981093 | 1 | 170193063 | NA | |
| rs12562203 | 1 | 175145938 | KIAA0040 | intron_variant |
| rs139718675 | 1 | 177096274 | ASTN1 | intron_variant |
| rs57530364 | 1 | 177096284 | ASTN1 | intron_variant |
| rs10798498 | 1 | 177096980 | ASTN1 | intron_variant |
| rs10753143 | 1 | 177097241 | ASTN1 | intron_variant |
| rs10798499 | 1 | 177097442 | ASTN1 | intron_variant |
| 1:177126430:TTTG:T | 1 | 177126430 | NA | NA |
| rs60826838 | 1 | 183545685 | NCF2 | intron_variant |
| rs117250867 | 1 | 184582654 | C1orf21 | intron_variant |
| rs12096795 | 1 | 184590346 | C1orf21 | 3_prime_UTR_variant |
| rs12023933 | 1 | 184621712 | NA | |

[Table 1-6]

| SNP ID | chr | position | gene locus | SNP location in gene locus |
|---|---|---|---|---|
| rs16823401 | 1 | 184666214 | EDEM3 | intron_variant |
| rs116897291 | 1 | 184814667 | NIBAN1 | intron_variant |
| rs148184379 | 1 | 184886968 | NIBAN1 | intron_variant |
| rs117517283 | 1 | 184921127 | NIBAN1 | intron_variant |
| rs74607511 | 1 | 192513026 | NA | |
| rs77505887 | 1 | 192515584 | NA | |
| rs74508649 | 1 | 192526317 | NA | |
| rs74419088 | 1 | 192529469 | NA | |
| rs77258347 | 1 | 192534699 | NA | |
| rs74367555 | 1 | 192538724 | NA | |
| rs76623154 | 1 | 192538746 | NA | |
| rs74130685 | 1 | 192538948 | NA | |
| rs115743395 | 1 | 192538965 | NA | |
| rs74130686 | 1 | 192539013 | NA | |
| rs147211012 | 1 | 199309033 | NA | |
| rs4915476 | 1 | 201047062 | CACNA1S | synonymous_variant |
| rs4915213 | 1 | 201047774 | CACNA1S | intron_variant |
| rs6427877 | 1 | 201048265 | CACNA1S | intron_variant |
| rs3767511 | 1 | 201052199 | CACNA1S | intron_variant |
| rs75488105 | 1 | 204065966 | S0X13 | intron_variant |
| rs113144751 | 1 | 204066542 | S0X13 | intron_variant |
| rs145279852 | 1 | 204066674 | S0X13 | intron_variant |
| rs147225273 | 1 | 204066680 | S0X13 | intron_variant |
| rs17188797 | 1 | 211187783 | AL590132.1 | intron_variant |
| rs2378009 | 1 | 218947264 | NA | |
| rs6681600 | 1 | 218948258 | NA | |
| rs6673437 | 1 | 218948649 | NA | |
| rs6541208 | 1 | 218952920 | NA | |
| rs11452039 | 1 | 218954472 | NA | |
| rs6541210 | 1 | 218955436 | NA | |
| rs12032085 | 1 | 223641019 | NA | |
| rs117913581 | 1 | 232777842 | NA | |
| rs138567986 | 1 | 241325817 | RGS7 | intron_variant |
| 1:244577487:T:TAATA | 1 | 244577487 | NA | NA |
| rs3127454 | 1 | 244578852 | ADSS2 | intron_variant |
| rs3127462 | 1 | 244597892 | ADSS2 | intron_variant |

[Table 1-7]

| SNP ID | chr | position | gene locus | SNP location in gene locus |
|---|---|---|---|---|
| rs3127463 | 1 | 244598196 | ADSS2 | intron_variant |
| rs3127464 | 1 | 244599858 | ADSS2 | intron_variant |
| 1:244601900:C:CAT | 1 | 244601900 | NA | NA |
| rs3003213 | 1 | 244608181 | ADSS2 | intron_variant |
| 1:244608950:T:TA | 1 | 244608950 | NA | NA |
| 1:244608950:T:TAA | 1 | 244608950 | NA | NA |
| rs3006004 | 1 | 244609092 | ADSS2 | intron_variant |
| rs3003212 | 1 | 244612252 | ADSS2 | intron_variant |
| rs3127466 | 1 | 244614497 | ADSS2 | intron_variant |
| rs3003210 | 1 | 244617509 | NA | |
| rs141944966 | 1 | 245746729 | KIF26B | intron_variant |
| rs145014144 | 1 | 245759684 | KIF26B | intron_variant |
| rs78194184 | 1 | 247365152 | AL390728.4 | intron_variant |
| rs61272272 | 2 | 6215734 | NA | |
| rs12621265 | 2 | 6220141 | NA | |
| rs2609122 | 2 | 6220654 | NA | |
| rs76756243 | 2 | 6224392 | NA | |
| rs12052388 | 2 | 6224960 | NA | |
| rs60904524 | 2 | 6225735 | NA | |
| rs73150377 | 2 | 6227222 | NA | |
| rs73150378 | 2 | 6227726 | NA | |
| rs77944704 | 2 | 6228902 | NA | |
| rs76144832 | 2 | 6231193 | NA | |
| rs74706208 | 2 | 6233421 | NA | |
| rs16864697 | 2 | 6233460 | NA | |
| rs12052438 | 2 | 6236379 | NA | |
| rs77918648 | 2 | 6270044 | NA | |
| rs76480233 | 2 | 6270267 | NA | |
| rs16864772 | 2 | 6271097 | NA | |
| rs16864773 | 2 | 6271288 | NA | |
| rs16864776 | 2 | 6271533 | NA | |
| rs16864778 | 2 | 6271646 | NA | |
| rs78933180 | 2 | 6274257 | NA | |
| rs75637712 | 2 | 6278287 | NA | |
| rs7557935 | 2 | 6281053 | NA | |
| rs7567221 | 2 | 6281198 | NA | |

[Table 1-8]

| SNP ID | chr | position | gene locus | SNP location in gene locus |
|---|---|---|---|---|
| rs11897984 | 2 | 7538773 | NA | |
| rs1346810 | 2 | 7539421 | NA | |
| rs10179128 | 2 | 7539984 | NA | |
| rs6709430 | 2 | 7544818 | NA | |
| rs1546026 | 2 | 10832167 | NA | |
| rs77715032 | 2 | 14959674 | NA | |
| 2:16523989:AT:A | 2 | 16523989 | NA | NA |
| rs201150095 | 2 | 16523991 | NA | NA |
| rs55641030 | 2 | 16982622 | NA | |
| rs74671599 | 2 | 16983140 | NA | |
| rs114875341 | 2 | 18374830 | KCNS3 | intron_variant |
| rs115436106 | 2 | 20365483 | NA | NA |
| rs2090033 | 2 | 20372497 | NA | |
| rs4233758 | 2 | 20376022 | NA | |
| rs75019476 | 2 | 31155554 | GALNT14 | intron_variant |
| rs75182639 | 2 | 31244662 | GALNT14 | intron_variant |
| rs59717368 | 2 | 34269641 | NA | |
| rs117140225 | 2 | 41848143 | NA | |
| rs117076730 | 2 | 41855067 | NA | |
| rs75983203 | 2 | 41860583 | NA | |
| 2:47055014:A:T | 2 | 47055014 | NA | NA |
| rs72806356 | 2 | 52982063 | NA | |
| rs199881113 | 2 | 52987966 | NA | |
| rs115366500 | 2 | 52996352 | NA | |
| rs62127009 | 2 | 52998723 | NA | |
| rs72908943 | 2 | 53689606 | NA | |
| rs6745622 | 2 | 53704053 | NA | |
| rs145963514 | 2 | 66470046 | NA | |
| rs76686125 | 2 | 111908163 | BCL2L11 | intron_variant |
| rs113351584 | 2 | 114643053 | NA | |
| rs142890162 | 2 | 114728805 | NA | |
| rs111252481 | 2 | 114734142 | NA | |
| rs143348587 | 2 | 114753287 | NA | |
| rs10207532 | 2 | 138096983 | THSD7B | intron_variant |
| rs10196367 | 2 | 138210725 | THSD7B | intron_variant |
| rs28415678 | 2 | 138297618 | THSD7B | intron_variant |

[Table 1-9]

| SNP ID | chr | position | gene locus | SNP location in gene locus |
|---|---|---|---|---|
| rs72048484 | 2 | 142051977 | LRP1B | intron_variant |
| rs2679451 | 2 | 142053453 | LRP1B | intron_variant |
| rs2679455 | 2 | 142060374 | LRP1B | intron_variant |
| rs2714170 | 2 | 142068173 | LRP1B | intron_variant |
| rs118164915 | 2 | 142489626 | LRP1B | intron_variant |
| rs80027625 | 2 | 142563101 | LRP1B | intron_variant |
| rs7563677 | 2 | 161356717 | NA | |
| 2:161357267:AAAAG:A | 2 | 161357267 | NA | NA |
| rs141879065 | 2 | 161358374 | NA | |
| rs201036025 | 2 | 161358504 | NA | |
| rs72970060 | 2 | 161360952 | NA | |
| rs72970066 | 2 | 161362938 | NA | |
| rs72970074 | 2 | 161365114 | NA | |
| rs6736111 | 2 | 161368211 | NA | |
| rs187697067 | 2 | 161384015 | NA | NA |
| rs192262735 | 2 | 172895083 | METAP1D | intron_variant |
| rs79029608 | 2 | 172907025 | METAP1D | intron_variant |
| rs60153685 | 2 | 172920131 | METAP1D | intron_variant |
| rs148393698 | 2 | 173138493 | NA | |
| 2:173790761:CGGAG:C | 2 | 173790761 | NA | NA |
| rs11695398 | 2 | 173791196 | RAPGEF4 | intron_variant |
| 2:173792363:T:TA | 2 | 173792363 | NA | NA |
| rs143907126 | 2 | 193371023 | NA | |
| rs12185569 | 2 | 202875552 | NA | |
| rs12185583 | 2 | 202875768 | NA | |
| rs10931984 | 2 | 202876281 | NA | |
| rs10931985 | 2 | 202876761 | NA | |
| rs13420220 | 2 | 202876981 | NA | |
| rs10490083 | 2 | 202877944 | NA | |
| rs10182797 | 2 | 202878867 | NA | |
| rs11682513 | 2 | 202879218 | NA | |
| rs17386240 | 2 | 202879624 | NA | |
| rs12328354 | 2 | 202882815 | NA | |
| rs12328774 | 2 | 202882897 | NA | |
| rs62184862 | 2 | 204683751 | NA | |
| rs62184863 | 2 | 204683797 | NA | |

[Table 1-10]

| SNP ID | chr | position | gene locus | SNP location in gene locus |
|---|---|---|---|---|
| 2:204695698:T:TA | 2 | 204695698 | NA | NA |
| rs58738696 | 2 | 204712127 | NA | |
| rs57248923 | 2 | 204712398 | NA | |
| rs62182593 | 2 | 204713735 | NA | |
| rs75622501 | 2 | 204714054 | NA | |
| rs6741283 | 2 | 204714810 | NA | |
| rs61602779 | 2 | 204716370 | NA | |
| rs13016652 | 2 | 211927188 | NA | |
| rs10932366 | 2 | 211954880 | NA | |
| rs188502110 | 2 | 216164766 | NA | |
| rs140760330 | 2 | 216165186 | NA | |
| rs111485414 | 2 | 228498025 | C2orf83 | non_coding_transcript_exon_variant |
| rs2048812 | 2 | 228518634 | NA | |
| rs2048813 | 2 | 228518649 | NA | |
| rs2048814 | 2 | 228518704 | NA | |
| rs7424668 | 2 | 228521539 | NA | |
| rs62189839 | 2 | 228521691 | NA | |
| rs140104950 | 2 | 228523377 | NA | |
| rs112530470 | 2 | 228523536 | NA | |
| rs146239501 | 2 | 235171239 | NA | NA |
| rs1878963 | 2 | 235171335 | NA | |
| 2:235172453:T:C | 2 | 235172453 | NA | NA |
| rs6711496 | 2 | 235172514 | NA | |
| 2:235172825: ATATAGTTGGAG:A | 2 | 235172825 | NA | NA |
| rs4663367 | 2 | 235172880 | NA | |
| rs10865081 | 2 | 239876256 | NA | |
| rs149564461 | 2 | 239881016 | NA | |
| rs4643560 | 2 | 239881730 | NA | |
| rs4544463 | 2 | 239883557 | NA | |
| rs4852058 | 2 | 239884849 | NA | |
| rs4852059 | 2 | 239884850 | NA | |
| rs13000373 | 2 | 239886775 | NA | |
| rs6543539 | 2 | 239888052 | NA | |
| rs7579583 | 2 | 239888571 | NA | |
| rs7586010 | 2 | 239888597 | NA | |

[Table 1-11]

| SNP ID | chr | position | gene locus | SNP location in gene locus |
|---|---|---|---|---|
| rs6721090 | 2 | 239888880 | NA | |
| rs11387730 | 2 | 239888961 | NA | |
| rs11124201 | 2 | 239889428 | NA | |
| rs11124202 | 2 | 239889603 | NA | |
| rs4452172 | 2 | 239890163 | NA | |
| rs4852064 | 2 | 239890370 | NA | |
| rs4852065 | 2 | 239890581 | NA | |
| rs4852066 | 2 | 239890696 | NA | |
| rs4852067 | 2 | 239890850 | NA | |
| 2:239890901:G:GCCA | 2 | 239890901 | NA | NA |
| rs6754831 | 2 | 239890949 | NA | |
| rs4851977 | 2 | 239891296 | NA | |
| rs6543542 | 2 | 239891644 | NA | |
| rs7580803 | 2 | 239892335 | NA | |
| rs4241232 | 2 | 239893010 | NA | |
| rs145427397 | 2 | 239902414 | NA | |
| rs181670597 | 2 | 242151208 | AN07 | intron_variant |
| 3:1749235:A:ATAT | 3 | 1749235 | NA | NA |
| rs139850749 | 3 | 1917654 | NA | |
| rs138485991 | 3 | 1934976 | NA | |
| rs7617928 | 3 | 5679782 | NA | |
| rs73808617 | 3 | 7086525 | GRM7 | NMD transcript variant |
| rs17046693 | 3 | 7088276 | GRM7 | NMD_transcript_variant |
| rs17046715 | 3 | 7090793 | GRM7 | NMD_transcript_variant |
| rs74285835 | 3 | 7100399 | GRM7 | NMD_transcript_variant |
| rs74285838 | 3 | 7111851 | GRM7 | NMD_transcript_variant |
| rs6767094 | 3 | 7118997 | GRM7 | NMD_transcript_variant |
| rs75442424 | 3 | 7121831 | GRM7 | NMD_transcript_variant |
| rs76240495 | 3 | 7122393 | GRM7 | NMD_transcript_variant |
| 3:7122411:CA:GA | 3 | 7122411 | NA | NA |
| 3:7122411:CA:C | 3 | 7122411 | NA | NA |
| rs77578494 | 3 | 11980791 | NA | |
| rs75863593 | 3 | 12814936 | NA | |
| rs73028847 | 3 | 14272393 | NA | |
| rs28547889 | 3 | 14273762 | NA | |
| rs55961609 | 3 | 14274855 | NA | |

[Table 1-12]

| SNP ID | chr | position | gene locus | SNP location in gene locus |
|---|---|---|---|---|
| rs6762079 | 3 | 14275457 | NA | |
| rs7615075 | 3 | 14282434 | NA | |
| rs9867815 | 3 | 14282631 | NA | |
| rs17305345 | 3 | 14285488 | NA | |
| rs9855896 | 3 | 14287150 | NA | |
| rs9839148 | 3 | 14287726 | NA | |
| rs6764810 | 3 | 14289062 | NA | |
| rs9882488 | 3 | 14290607 | NA | |
| rs7640375 | 3 | 14290987 | NA | |
| rs7626720 | 3 | 14291503 | NA | |
| rs9817301 | 3 | 14292389 | NA | |
| rs9823520 | 3 | 14293832 | NA | |
| rs9860771 | 3 | 14293838 | NA | |
| 3:14296073:G: GCCTTGGGTGCACCTGGCCCT | 3 | 14296073 | NA | NA |
| rs11920518 | 3 | 14296545 | NA | |
| rs2341654 | 3 | 14298116 | NA | |
| rs2128162 | 3 | 14298264 | NA | |
| rs1038298 | 3 | 14302455 | NA | |
| rs74710259 | 3 | 21874157 | ZNF385D | NMD_transcript_variant |
| rs79680055 | 3 | 21874582 | ZNF385D | NMD_transcript_variant |
| rs75670791 | 3 | 21914171 | ZNF385D | NMD_transcript_variant |
| rs74903313 | 3 | 28653345 | AC098650. 1 | NMD_transcript_variant |
| rs143877686 | 3 | 28653565 | AC098650. 1 | NMD_transcript_variant |
| rs78649247 | 3 | 28660023 | AC098650. 1 | NMD_transcript_variant |
| rs75624012 | 3 | 28762528 | AC098650. 1 | NMD_transcript_variant |
| rs12491791 | 3 | 28769252 | AC098650. 1 | NMD_transcript_variant |
| rs144410553 | 3 | 28796544 | AC098650. 1 | NMD_transcript_variant |
| rs114769913 | 3 | 28799686 | AC098650.1 | NMD_transcript_variant |
| rs62241678 | 3 | 29333361 | AC098650. 1 | NMD_transcript_variant |
| rs150022738 | 3 | 32753524 | CN0T10 | intron_variant |
| rs62250842 | 3 | 32758483 | CN0T10 | intron_variant |
| rs62250843 | 3 | 32760921 | CN0T10 | intron_variant |
| rs6793550 | 3 | 32765663 | CN0T10 | intron_variant |
| 3:32774098:G:GA | 3 | 32774098 | NA | NA |
| rs145862784 | 3 | 32780082 | CN0T10 | intron_variant |

[Table 1-13]

| SNP ID | chr | position | gene locus | SNP location in gene locus |
|---|---|---|---|---|
| rs7612883 | 3 | 32889638 | TRIM71 | intron_variant |
| rs13060342 | 3 | 32891423 | TRIM71 | intron_variant |
| rs6800867 | 3 | 32981043 | NA | |
| rs6803409 | 3 | 32981316 | NA | |
| rs113716980 | 3 | 32982985 | NA | |
| 3:32983158:CT:C | 3 | 32983158 | NA | NA |
| 3:32987068:C:CT | 3 | 32987068 | NA | NA |
| rs4074331 | 3 | 32989009 | NA | |
| rs4074330 | 3 | 32989010 | NA | |
| rs73055186 | 3 | 32991856 | NA | |
| rs62250867 | 3 | 32991963 | NA | |
| rs7632357 | 3 | 32992203 | NA | |
| rs3774527 | 3 | 53705003 | CACNA1D | intron_variant |
| rs74426676 | 3 | 54509829 | CACNA2D3 | intron variant |
| 3:60608100:A:AT | 3 | 60608100 | NA | NA |
| rs142745963 | 3 | 65616074 | MAGI1 | intron_variant |
| rs61555164 | 3 | 65741871 | MAGI1 | intron_variant |
| rs9841082 | 3 | 65743597 | MAGI1 | intron_variant |
| rs35219449 | 3 | 65872186 | MAGI1 | intron_variant |
| rs71306785 | 3 | 65872578 | MAGI1 | intron_variant |
| rs71306786 | 3 | 65873461 | MAGI1 | intron variant |
| rs58687721 | 3 | 65873820 | MAGI1 | intron_variant |
| rs35168985 | 3 | 65874420 | MAGI1 | intron_variant |
| rs2372397 | 3 | 65875834 | MAGI1 | intron_variant |
| rs117501871 | 3 | 65876113 | MAGI1 | intron_variant |
| rs181795375 | 3 | 65877601 | MAGI1 | intron_variant |
| rs71306788 | 3 | 65877703 | MAGI1 | intron_variant |
| rs7130fi789 | 3 | 65877793 | MAGI1 | intron_variant |
| rs34548503 | 3 | 65878656 | MAGI1 | intron_variant |
| 3:65878951:G:GA | 3 | 65878951 | NA | NA |
| rs924021 | 3 | 65879837 | MAGI1 | intron_variant |
| rs35482956 | 3 | 65880339 | MAGI1 | intron_variant |
| rs74795753 | 3 | 65926053 | MAGI1 | intron_variant |
| rs117740205 | 3 | 65931724 | MAGI1 | intron_variant |
| rs114019667 | 3 | 66710033 | NA | |
| rs13319937 | 3 | 68599941 | NA | |

[Table 1-14]

| SNP ID | chr | position | gene locus | SNP location in gene locus |
|---|---|---|---|---|
| rs1858373 | 3 | 68601038 | NA | |
| rs1858372 | 3 | 68601193 | NA | |
| rs55744776 | 3 | 68601734 | NA | |
| rs7623705 | 3 | 68601948 | NA | |
| rs72106943 | 3 | 68605001 | NA | |
| rs10576290 | 3 | 68605535 | NA | |
| rs7616164 | 3 | 68606548 | NA | |
| rs9837106 | 3 | 68606690 | NA | |
| rs9866302 | 3 | 68609673 | NA | |
| rs13321458 | 3 | 68610030 | NA | |
| rs9835386 | 3 | 68610847 | NA | |
| rs67172613 | 3 | 77035984 | ROB02 | intron_variant |
| rs72902045 | 3 | 77036238 | R0B02 | intron_variant |
| rs7427534 | 3 | 77041173 | ROB02 | intron_variant |
| rs6795635 | 3 | 77045304 | ROB02 | intron_variant |
| rs6806644 | 3 | 77045353 | ROB02 | intron_variant |
| rs6548472 | 3 | 77045731 | ROB02 | intron_variant |
| rsl1374746 | 3 | 77045771 | R0B02 | intron_variant |
| rs6786589 | 3 | 77046740 | R0B02 | intron_variant |
| rs9875709 | 3 | 77047311 | R0B02 | intron_variant |
| rs6774797 | 3 | 77048488 | R0B02 | intron_variant |
| rs11918007 | 3 | 77051881 | ROB02 | intron_variant |
| 3:84969212:T:A | 3 | 84969212 | NA | NA |
| rs144035609 | 3 | 84972857 | NA | |
| rs150062308 | 3 | 85019042 | CADM2 | intron_variant |
| rs148678484 | 3 | 85042238 | CADM2 | intron_variant |
| rs143096570 | 3 | 85051020 | CADM2 | intron_variant |
| rs2875508 | 3 | 86021016 | CADM2 | intron_variant |
| rs76958889 | 3 | 86022860 | CADM2 | intron_variant |
| 3:107339790:T:G | 3 | 107339790 | NA | NA |
| 3:107502792:C:CA | 3 | 107502792 | NA | NA |
| 3:112698726:AATGG:AATGGATGG | 3 | 112698726 | NA | NA |
| 3:112698726:AATGG:A | 3 | 112698726 | NA | NA |
| rs2613959 | 3 | 112808299 | NA | |
| rs73229010 | 3 | 112899616 | NA | |
| rs73229016 | 3 | 112901722 | NA | |

[Table 1-15]

| SNP ID | chr | position | gene locus | SNP location in gene locus |
|---|---|---|---|---|
| rs79608790 | 3 | 112958412 | B0C | intron_variant |
| 3:112958412:T:G | 3 | 112958412 | NA | NA |
| rs58481194 | 3 | 172947355 | NA | |
| rs6443469 | 3 | 177286455 | NA | |
| rs7627379 | 3 | 177286946 | NA | |
| rs937509 | 3 | 177287701 | NA | |
| rs6768291 | 3 | 177290183 | NA | |
| rs2055366 | 3 | 177290345 | NA | |
| rs1970860 | 3 | 177290953 | NA | |
| rs1970861 | 3 | 177291213 | NA | |
| rs4241483 | 3 | 177291318 | NA | |
| rs5854759 | 3 | 177292429 | NA | |
| rs6806363 | 3 | 177294081 | NA | |
| rs13083115 | 3 | 177294781 | NA | |
| rs1973469 | 3 | 177295093 | NA | |
| rs2863011 | 3 | 177295520 | NA | |
| rs2133596 | 3 | 177295608 | NA | |
| rs11713121 | 3 | 177295661 | NA | |
| rs60882310 | 3 | 177295932 | NA | |
| rs35403806 | 3 | 177296263 | NA | |
| rs36018104 | 3 | 177296267 | NA | |
| rs57592389 | 3 | 177296362 | NA | |
| rs58786527 | 3 | 177296363 | NA | |
| rs13062339 | 3 | 177296394 | NA | |
| rs13084960 | 3 | 177296448 | NA | |
| rs34858531 | 3 | 177296554 | NA | |
| rs4857705 | 3 | 177296885 | NA | |
| rs4857706 | 3 | 177296956 | NA | |
| rs4857707 | 3 | 177297074 | NA | |
| rs4857708 | 3 | 177297173 | NA | |
| 3:177297644:G:GTT | 3 | 177297644 | NA | NA |
| rs6801659 | 3 | 177297792 | NA | |
| rs6801849 | 3 | 177297824 | NA | |
| rs1463859 | 3 | 177298071 | NA | |
| rs1463858 | 3 | 177298127 | NA | |
| rs1463857 | 3 | 177298327 | NA | |

[Table 1-16]

| SNP ID | chr | position | gene locus | SNP location in gene locus |
|---|---|---|---|---|
| rs4555562 | 3 | 177298563 | NA | |
| rs4257602 | 3 | 177298695 | NA | |
| rs4273409 | 3 | 177298785 | NA | |
| rs4290846 | 3 | 177298934 | NA | |
| rs35168340 | 3 | 177299052 | NA | |
| rs34332266 | 3 | 177299061 | NA | |
| rs34316712 | 3 | 177299065 | NA | |
| rs36102700 | 3 | 177299109 | NA | |
| rs35921520 | 3 | 177299232 | NA | |
| rs6805429 | 3 | 177299269 | NA | |
| rs6781232 | 3 | 177299379 | NA | |
| 3:177299739:A:ATTT | 3 | 177299739 | NA | NA |
| rs6808260 | 3 | 177299832 | NA | |
| rs7630147 | 3 | 177300391 | NA | |
| rs7610690 | 3 | 177300717 | NA | |
| rs7610926 | 3 | 177300753 | NA | |
| rs4857709 | 3 | 177300955 | NA | |
| rs4857710 | 3 | 177300965 | NA | |
| rs4857711 | 3 | 177300980 | NA | |
| rs4857712 | 3 | 177301016 | NA | |
| rs6763782 | 3 | 177301280 | NA | |
| 3:177301483: AGGACTTAAGCAGAT:A | 3 | 177301483 | NA | NA |
| rs2863019 | 3 | 177301836 | NA | |
| rs2863018 | 3 | 177301911 | NA | |
| rs2133592 | 3 | 177301939 | NA | |
| rs2133593 | 3 | 177301992 | NA | |
| rs2863017 | 3 | 177302033 | NA | |
| rs2173608 | 3 | 177302338 | NA | |
| rs6783033 | 3 | 177302751 | NA | |
| rs6770216 | 3 | 177302927 | NA | |
| rs2863016 | 3 | 177302980 | NA | |
| rs2863015 | 3 | 177303179 | NA | |
| rs2863014 | 3 | 177303192 | NA | |
| rs2133595 | 3 | 177303610 | NA | |
| rs1875095 | 3 | 177303723 | NA | |

[Table 1-17]

| SNP ID | chr | position | gene locus | SNP location in gene locus |
|---|---|---|---|---|
| rs1875096 | 3 | 177303864 | NA | |
| rs1875097 | 3 | 177304032 | NA | |
| rs7620560 | 3 | 177304176 | NA | |
| rs7620503 | 3 | 177304298 | NA | |
| rs7634809 | 3 | 177304668 | NA | |
| rs7645281 | 3 | 177304836 | NA | |
| rs7623309 | 3 | 177304851 | NA | |
| rs6443474 | 3 | 177305124 | NA | |
| rs6777561 | 3 | 177305198 | NA | |
| rs13077994 | 3 | 177305705 | NA | |
| rs58453015 | 3 | 177305847 | NA | NA |
| rs4857713 | 3 | 177306442 | NA | |
| rs4857612 | 3 | 177306621 | NA | |
| rs11350527 | 3 | 177306757 | NA | |
| rs7637965 | 3 | 177307642 | NA | |
| rs6443476 | 3 | 177307695 | NA | |
| rs6443477 | 3 | 177307933 | NA | |
| rs6769466 | 3 | 177308014 | NA | |
| rs7616679 | 3 | 177308486 | NA | |
| rs11708469 | 3 | 177308718 | NA | |
| rs6797636 | 3 | 177309442 | NA | |
| rs13079175 | 3 | 177309533 | NA | |
| 3:186444809:A:G | 3 | 186444809 | NA | NA |
| rs117064584 | 3 | 189164430 | NA | |
| rs117987791 | 3 | 189523891 | TP63 | intron variant |
| rs72613184 | 4 | 5235040 | STK32B | intron_variant |
| rs149515956 | 4 | 6341633 | PPP2R2C | intron_variant |
| rs147293851 | 4 | 13966004 | NA | |
| rs142792029 | 4 | 14017337 | NA | |
| rs62294496 | 4 | 17260883 | NA | |
| rs118004921 | 4 | 17304792 | NA | |
| rs11933789 | 4 | 17825159 | NCAPG | intron_variant |
| rs62295154 | 4 | 19769915 | NA | |
| rs13435165 | 4 | 19772721 | NA | |
| rs79235820 | 4 | 22171802 | NA | |
| rs116460686 | 4 | 31892334 | NA | |

[Table 1-18]

| SNP ID | chr | position | gene locus | SNP location in gene locus |
|---|---|---|---|---|
| rs115446883 | 4 | 31896317 | NA | |
| rs146966940 | 4 | 41153294 | APBB2 | intron_variant |
| 4:41784275:A:C | 4 | 41784275 | NA | NA |
| rs11942428 | 4 | 44580439 | NA | |
| rs148742662 | 4 | 54567050 | NA | NA |
| rs144162078 | 4 | 54580834 | AC058822. 1 | intron_variant |
| 4:54582351:CA:C | 4 | 54582351 | NA | NA |
| rs143711414 | 4 | 54604589 | AC058822.1 | intron_variant |
| 4:61494990:A:AT | 4 | 61494990 | NA | NA |
| rs117340277 | 4 | 63017478 | NA | |
| rs76314468 | 4 | 64039941 | NA | |
| rs79892130 | 4 | 64040056 | NA | |
| rs75655453 | 4 | 64040607 | NA | |
| rs77849378 | 4 | 64041936 | NA | |
| rs74461070 | 4 | 64042807 | NA | |
| rs77802168 | 4 | 64042829 | NA | |
| rs6816831 | 4 | 64044660 | NA | |
| rs6848438 | 4 | 64044675 | NA | |
| rs143395876 | 4 | 64046194 | NA | |
| rs4502710 | 4 | 64047829 | NA | |
| rs143437614 | 4 | 64048178 | NA | |
| rs4035516 | 4 | 64049319 | NA | |
| rs138747488 | 4 | 64049566 | NA | |
| rs79890012 | 4 | 64050856 | NA | |
| rs139008026 | 4 | 64051485 | NA | |
| rs116239045 | 4 | 64051678 | NA | |
| rs139446359 | 4 | 64052143 | NA | |
| rs144091716 | 4 | 64052292 | NA | |
| rs114269773 | 4 | 64053241 | NA | |
| rs148230700 | 4 | 64054216 | NA | |
| rs114403067 | 4 | 64054570 | NA | |
| rs115936064 | 4 | 64055253 | NA | |
| rs142760460 | 4 | 64055706 | NA | |
| rs147936120 | 4 | 64057031 | NA | |
| rs115409842 | 4 | 64057046 | NA | |
| rs115633947 | 4 | 64058695 | NA | |

[Table 1-19]

| SNP ID | chr | position | gene locus | SNP location in gene locus |
|---|---|---|---|---|
| rs148720007 | 4 | 64058783 | NA | |
| rs140223857 | 4 | 64058820 | NA | |
| rs7680092 | 4 | 64061134 | NA | |
| 4:64061283:AT:ATT | 4 | 64061283 | NA | NA |
| 4:64061283:AT:A | 4 | 64061283 | NA | NA |
| rs80287150 | 4 | 64061543 | NA | |
| rs115458156 | 4 | 64061728 | NA | |
| 4:64062006:C:CA | 4 | 64062006 | NA | NA |
| 4: 64062006:C:CAA | 4 | 64062006 | NA | NA |
| rs74340891 | 4 | 64062350 | NA | |
| rs116187324 | 4 | 64064808 | NA | |
| rs74796023 | 4 | 64066685 | NA | |
| rs74864007 | 4 | 64066878 | NA | |
| rs115897647 | 4 | 64066892 | NA | |
| rs201614722 | 4 | 64067415 | NA | |
| rs201701309 | 4 | 64067892 | NA | |
| rs201649360 | 4 | 64068147 | NA | |
| rs184640169 | 4 | 64069492 | NA | |
| rs77460761 | 4 | 64069972 | NA | |
| rs182226229 | 4 | 64070081 | NA | |
| rs183837605 | 4 | 64071489 | NA | |
| rs146847342 | 4 | 64072931 | NA | |
| rs76764827 | 4 | 64072957 | NA | |
| rs74392895 | 4 | 64074311 | NA | |
| rs79387056 | 4 | 64074978 | NA | |
| rs75876899 | 4 | 64076116 | NA | |
| rs116177742 | 4 | 64076886 | NA | |
| rs114946744 | 4 | 64076904 | NA | |
| rs80315791 | 4 | 64078203 | NA | |
| rs80199562 | 4 | 64079037 | NA | |
| rs76630438 | 4 | 64079127 | NA | |
| rs78126082 | 4 | 64079408 | NA | |
| rs2881626 | 4 | 64080016 | NA | |
| rs75900830 | 4 | 64080483 | NA | |
| rs77305257 | 4 | 64080642 | NA | |
| rs77019672 | 4 | 64081403 | NA | |

[Table 1-20]

| SNP ID | chr | position | gene locus | SNP location in gene locus |
|---|---|---|---|---|
| rs115778258 | 4 | 64082381 | NA | |
| rs79113257 | 4 | 64086632 | NA | |
| rs147278503 | 4 | 93215159 | NA | |
| rs141054368 | 4 | 93217241 | NA | |
| rs7658734 | 4 | 96509012 | NA | |
| 4:96513678:C:T | 4 | 96513678 | NA | NA |
| rs62306715 | 4 | 96513884 | NA | |
| 4:96514526:T:C | 4 | 96514526 | NA | NA |
| rs113843935 | 4 | 96514739 | NA | |
| rs67930114 | 4 | 96515437 | NA | |
| rs57799366 | 4 | 96517154 | NA | |
| rs62306720 | 4 | 96517458 | NA | |
| 4:96518109:G:GA | 4 | 96518109 | NA | NA |
| rs2123367 | 4 | 109711528 | NA | |
| rs77922013 | 4 | 109712306 | NA | |
| rs76550713 | 4 | 109712541 | NA | |
| rs150990204 | 4 | 109712817 | NA | |
| rs17039415 | 4 | 109722415 | NA | |
| rs146480894 | 4 | 113229907 | ALPK1 | non_coding_transcript_variant |
| rs150718823 | 4 | 113232846 | ALPK1 | non_coding_transcript_variant |
| rs115122068 | 4 | 117719540 | NA | |
| rs150939893 | 4 | 117744934 | NA | |
| rs190696080 | 4 | 117750582 | NA | |
| rs113295558 | 4 | 117755974 | NA | |
| rs191714458 | 4 | 117758243 | NA | |
| rs186618613 | 4 | 117762073 | NA | |
| rs190966932 | 4 | 117762377 | NA | |
| rs192343018 | 4 | 117767150 | NA | |
| rs181001803 | 4 | 117773288 | NA | |
| rs7684310 | 4 | 117773712 | NA | |
| rs189192694 | 4 | 117774888 | NA | |
| rs191512269 | 4 | 117777305 | NA | |
| 4:117780739:G:T | 4 | 117780739 | NA | NA |
| rs182361185 | 4 | 117787075 | NA | |
| 4:117793406:A:T | 4 | 117793406 | NA | NA |
| rs74657487 | 4 | 122236579 | NA | |

[Table 1-21]

| SNP ID | chr | position | gene locus | SNP location in gene locus |
|---|---|---|---|---|
| rs80300492 | 4 | 122239822 | NA | |
| rs75622168 | 4 | 122240327 | NA | |
| rs79585077 | 4 | 122241796 | NA | |
| rs76926525 | 4 | 122242129 | NA | |
| rs77958862 | 4 | 122244530 | NA | |
| rs77294448 | 4 | 122246900 | NA | |
| rs6821123 | 4 | 122249973 | QRFPR | 3_prime_UTR_variant |
| rs113334103 | 4 | 122252388 | QRFPR | intron_variant |
| rs55975435 | 4 | 122254014 | ORFPR | 3_prime_UTR_variant |
| rs55776058 | 4 | 122254284 | QRFPR | intron_variant |
| rs2302308 | 4 | 122258149 | QRFPR | intron_variant |
| rs74398478 | 4 | 122259584 | QRFPR | intron_variant |
| rs17051338 | 4 | 122260042 | QRFPR | intron_variant |
| rs77438622 | 4 | 122264692 | QRFPR | intron_variant |
| rs76536576 | 4 | 122268524 | QRFPR | intron_variant |
| rs182992688 | 4 | 122270068 | QRFPR | intron_variant |
| rs187168138 | 4 | 122270076 | QRFPR | intron_variant |
| rs77006299 | 4 | 122270900 | QRFPR | intron_variant |
| rs10518388 | 4 | 122280335 | QRFPR | intron_variant |
| rs17051344 | 4 | 122285263 | QRFPR | intron_variant |
| rs66511907 | 4 | 122286927 | QRFPR | intron_variant |
| rs76503879 | 4 | 122287794 | QRFPR | intron_variant |
| 4: 133557517:A:T | 4 | 133557517 | NA | NA |
| rs201392439 | 4 | 134152521 | NA | |
| 4: 136278810:T:G | 4 | 136278810 | NA | NA |
| rs79757000 | 4 | 136309269 | NA | |
| rs10005391 | 4 | 136318357 | NA | |
| rs10017268 | 4 | 136318429 | NA | |
| rs10005518 | 4 | 136318641 | NA | |
| rs10008741 | 4 | 136319479 | NA | |
| rs10008854 | 4 | 136319621 | NA | |
| 4: 136320586:G:GTT | 4 | 136320586 | NA | NA |
| 4: 136320587:C:A | 4 | 136320587 | NA | NA |
| rs150556358 | 4 | 136320899 | NA | NA |
| rs28701471 | 4 | 136321575 | NA | |
| 4:136321714:CAA:CA | 4 | 136321714 | NA | NA |

[Table 1-22]

| SNP ID | chr | position | gene locus | SNP location in gene locus |
|---|---|---|---|---|
| 4:136321714:CAA:C | 4 | 136321714 | NA | NA |
| rs33960846 | 4 | 136321824 | NA | |
| rs28481887 | 4 | 136322868 | NA | |
| rs28673025 | 4 | 136322980 | NA | |
| rs28709698 | 4 | 136323895 | NA | |
| rs76261505 | 4 | 136324955 | NA | |
| rs10028807 | 4 | 136327412 | NA | |
| rs145137955 | 4 | 136327637 | NA | |
| 4:136327754:CT:C | 4 | 136327754 | NA | NA |
| rs28872626 | 4 | 136327960 | NA | |
| rs28790884 | 4 | 136327980 | NA | |
| rs28838886 | 4 | 136327990 | NA | |
| rs28857199 | 4 | 136328206 | NA | |
| 4:136328885:T:A | 4 | 136328885 | NA | NA |
| 4:136328886:G:T | 4 | 136328886 | NA | NA |
| rs6810998 | 4 | 140693268 | MAML3 | intron_variant |
| rs78180498 | 4 | 141207904 | SC0C | intron_variant |
| rs78619534 | 4 | 141219174 | SCOC | intron_variant |
| rs17021272 | 4 | 147156085 | NA | |
| rs72948673 | 4 | 147174693 | NA | |
| rs60367087 | 4 | 147199809 | SLC10A7 | intron_variant |
| rs72950642 | 4 | 147238329 | SLC10A7 | intron variant |
| rs72950644 | 4 | 147239235 | SLC10A7 | intron_variant |
| 4:147245033:T:C | 4 | 147245033 | NA | NA |
| rs112713961 | 4 | 147245957 | SLC10A7 | intron_variant |
| rs72950652 | 4 | 147246540 | SLC10A7 | intron_variant |
| rs72950654 | 4 | 147248032 | SLC10A7 | intron_variant |
| rsl13969284 | 4 | 147248604 | SLC10A7 | intron_variant |
| rs72950658 | 4 | 147253902 | SLC10A7 | intron_variant |
| rs72950664 | 4 | 147255021 | SLC10A7 | intron_variant |
| rs72950667 | 4 | 147255582 | SLC10A7 | intron_variant |
| rs142662018 | 4 | 147255649 | SLC10A7 | intron_variant |
| rs72950672 | 4 | 147260169 | SLC10A7 | intron variant |
| rs72950677 | 4 | 147261151 | SLC10A7 | intron_variant |
| rs72950679 | 4 | 147262990 | SLC10A7 | intron_variant |
| rs72950681 | 4 | 147264589 | SLC10A7 | intron_variant |

[Table 1-23]

| SNP ID | chr | position | gene locus | SNP location in gene locus |
|---|---|---|---|---|
| rs72950682 | 4 | 147264764 | SLC10A7 | intron_variant |
| rs78199829 | 4 | 147267044 | SLC10A7 | intron_variant |
| rs72950684 | 4 | 147267419 | SLC10A7 | intron_variant |
| rs72950690 | 4 | 147269478 | SLC10A7 | intron variant |
| rs58672301 | 4 | 147269836 | SLC10A7 | intron variant |
| rs111963516 | 4 | 147271022 | SLC10A7 | intron_variant |
| rs76206578 | 4 | 147271027 | SLC10A7 | intron_variant |
| 4:147271544:A:G | 4 | 147271544 | NA | NA |
| 4:147272211:A:T | 4 | 147272211 | NA | NA |
| 4:147272243:A:G | 4 | 147272243 | NA | NA |
| 4:147272464:C:T | 4 | 147272464 | NA | NA |
| rs72950697 | 4 | 147277445 | SLC10A7 | intron_variant |
| rs58494546 | 4 | 147278079 | SLC10A7 | intron_variant |
| rs72952504 | 4 | 147278240 | SLC10A7 | intron_variant |
| rs72952506 | 4 | 147278447 | SLC10A7 | intron_variant |
| rs72952508 | 4 | 147278472 | SLC10A7 | intron variant |
| rs11455281 | 4 | 147279083 | SLC10A7 | intron_variant |
| rs111635640 | 4 | 147279100 | SLC10A7 | intron_variant |
| rs6816058 | 4 | 147279394 | SLC10A7 | intron_variant |
| rs72952510 | 4 | 147280039 | SLC10A7 | intron_variant |
| rs111476662 | 4 | 147280504 | SLC10A7 | intron_variant |
| rs72952511 | 4 | 147280820 | SLC10A7 | intron variant |
| rs72952513 | 4 | 147281127 | SLC10A7 | intron_variant |
| rs72952515 | 4 | 147281316 | SLC10A7 | intron_variant |
| 4:147281756:C:CT | 4 | 147281756 | NA | NA |
| rs60404214 | 4 | 147282897 | SLC10A7 | intron_variant |
| rs2630284 | 4 | 147284129 | SLC10A7 | intron_variant |
| rs4027057 | 4 | 147284502 | SLC10A7 | intron_variant |
| rs2679154 | 4 | 147285031 | SLC10A7 | intron_variant |
| rs2679153 | 4 | 147286796 | SLC10A7 | intron_variant |
| rs2255242 | 4 | 147286963 | SLC10A7 | intron_variant |
| rs2679151 | 4 | 147287007 | SLC10A7 | intron_variant |
| rs2244316 | 4 | 147289414 | SLC10A7 | intron variant |
| rs2244317 | 4 | 147289424 | SLC10A7 | intron_variant |
| rs2244318 | 4 | 147289431 | SLC10A7 | intron_variant |
| rs2244321 | 4 | 147289522 | SLC10A7 | intron_variant |

[Table 1-24]

| SNP ID | chr | position | gene locus | SNP location in gene locus |
|---|---|---|---|---|
| rs2679149 | 4 | 147290788 | SLC10A7 | intron_variant |
| rs2630278 | 4 | 147292164 | SLC10A7 | intron_variant |
| rs2630277 | 4 | 147292832 | SLC10A7 | intron_variant |
| rs2244840 | 4 | 147293347 | SLC10A7 | intron_variant |
| rs2244850 | 4 | 147293653 | SLC10A7 | intron_variant |
| rs2679145 | 4 | 147294299 | SLC10A7 | intron_variant |
| 4:147295793:C:G | 4 | 147295793 | NA | NA |
| rs2679121 | 4 | 147296826 | SLC10A7 | intron_variant |
| rs2245274 | 4 | 147297184 | SLC10A7 | intron_variant |
| rs2245282 | 4 | 147297453 | SLC10A7 | intron_variant |
| rs2262884 | 4 | 147297927 | SLC10A7 | intron_variant |
| rs111925113 | 4 | 147298186 | SLC10A7 | intron_variant |
| 4:147300051:A :AAAGCAGATAATCTGCT | 4 | 147300051 | NA | NA |
| rs2248347 | 4 | 147300898 | SLC10A7 | intron_variant |
| rs2248348 | 4 | 147300931 | SLC10A7 | intron_variant |
| rs2248826 | 4 | 147305421 | SLC10A7 | intron_variant |
| rs2630297 | 4 | 147306646 | SLC10A7 | intron_variant |
| rs2460033 | 4 | 147307479 | SLC10A7 | intron_variant |
| rs138801798 | 4 | 157046897 | NA | |
| rs6834759 | 4 | 158779650 | NA | |
| rs150752209 | 4 | 158783439 | NA | |
| rs17036774 | 4 | 158783852 | NA | |
| rs150456849 | 4 | 158784767 | NA | |
| rs6836401 | 4 | 158786983 | NA | |
| rs6813626 | 4 | 158787102 | NA | |
| rs55817402 | 4 | 174427221 | NA | |
| rs116894613 | 4 | 185063146 | ENPP6 | intron_variant |
| rs140512564 | 4 | 185116348 | ENPP6 | intron_variant |
| rs76233088 | 4 | 185808870 | NA | |
| rs35834666 | 4 | 187138175 | KLKB1 | intron_variant |
| 4:187138442:T:TTTG | 4 | 187138442 | NA | NA |
| rs34852777 | 4 | 187139062 | KLKB1 | intron_variant |
| rs13102931 | 4 | 187145626 | KLKB1 | intron_variant |
| rs13102788 | 4 | 187145738 | KLKB1 | intron_variant |
| rs138695518 | 4 | 187146418 | KLKB1 | intron_variant |

[Table 1-25]

| SNP ID | chr | position | gene locus | SNP location in gene locus |
|---|---|---|---|---|
| rs35669195 | 4 | 187146559 | KLKB1 | intron_variant |
| rs4253241 | 4 | 187149233 | KLKB1 | intron_variant |
| rs4253246 | 4 | 187154424 | KLKB1 | intron_variant |
| rs184644242 | 4 | 187157181 | KLKB1 | intron_variant |
| rs145549717 | 4 | 187157232 | KLKB1 | intron_variant |
| 5:2020945:T:A | 5 | 2020945 | NA | NA |
| rs142127390 | 5 | 11734734 | CTNND2 | intron_variant |
| rs10076098 | 5 | 11737241 | CTNND2 | intron_variant |
| rs57612673 | 5 | 13733453 | DNAH5 | intron_variant |
| 5: 14710594:C:G | 5 | 14710594 | NA | NA |
| 5: 14722584:T:C | 5 | 14722584 | NA | NA |
| 5: 14740327:C:T | 5 | 14740327 | NA | NA |
| rs77818967 | 5 | 19736762 | CDH18 | intron_variant |
| rs12657194 | 5 | 19762895 | CDH18 | intron_variant |
| rs141488363 | 5 | 19785302 | CDH18 | intron_variant |
| rs140741351 | 5 | 19808374 | CDH18 | intron_variant |
| rs34847415 | 5 | 19909533 | CDH18 | intron_variant |
| rs72740870 | 5 | 19910167 | CDH18 | intron_variant |
| rs79509881 | 5 | 24693838 | NA | |
| rs62357273 | 5 | 25683630 | NA | |
| rs111941500 | 5 | 25688951 | NA | |
| rsl13538604 | 5 | 25690607 | NA | |
| rs13159231 | 5 | 25691118 | NA | |
| rs148804803 | 5 | 25696038 | NA | |
| rs138213578 | 5 | 25705703 | NA | |
| rs200325656 | 5 | 25706012 | NA | |
| rs35750564 | 5 | 28029472 | NA | |
| rs139552645 | 5 | 28069123 | NA | |
| rs147157201 | 5 | 37104905 | NA | |
| rs301904 | 5 | 37111565 | CPLANE1 | intron_variant |
| 5:37111995:T:TA | 5 | 37111995 | NA | NA |
| rs301894 | 5 | 37127778 | CPLANE1 | intron_variant |
| rs301895 | 5 | 37128315 | CPLANE1 | intron_variant |
| rs12110069 | 5 | 37160914 | CPLANE1 | intron_variant |
| rs10056250 | 5 | 37171884 | CPLANE1 | intron_variant |
| rs75589774 | 5 | 37182902 | CPLANE1 | missense_variant |

[Table 1-26]

| SNP ID | chr | position | gene locus | SNP location in gene locus |
|--------|-----|----------|------------|----------------------------|
| rs79298055 | 5 | 37202084 | CPLANE1 | intron_variant |
| rs76607706 | 5 | 37892303 | NA | |
| rs111622158 | 5 | 37928055 | NA | |
| rs75661047 | 5 | 41337919 | PLCXD3 | intron_variant |
| rs189501062 | 5 | 41421703 | PLCXD3 | intron_variant |
| rs318065 | 5 | 41442044 | PLCXD3 | intron_variant |
| rs78362058 | 5 | 41448308 | PLCXD3 | intron_variant |
| rs78872736 | 5 | 41449972 | PLCXD3 | intron_variant |
| rs79993711 | 5 | 41451447 | PLCXD3 | intron_variant |
| rs3883955 | 5 | 41455054 | PLCXD3 | intron_variant |
| rs191995881 | 5 | 41456607 | PLCXD3 | intron_variant |
| rs77129467 | 5 | 41459002 | PLCXD3 | intron_variant |
| rs117577517 | 5 | 41460797 | PLCXD3 | intron_variant |
| rs149176711 | 5 | 41460944 | PLCXD3 | intron_variant |
| rs145608729 | 5 | 41462819 | PLCXD3 | intron_variant |
| rs142790831 | 5 | 41462961 | PLCXD3 | intron_variant |
| rs80187604 | 5 | 41464479 | PLCXD3 | intron_variant |
| rs118037167 | 5 | 41465566 | PLCXD3 | intron_variant |
| rs116809573 | 5 | 41469306 | PLCXD3 | intron_variant |
| rs117880348 | 5 | 41471050 | PLCXD3 | intron_variant |
| rs74850462 | 5 | 41473868 | PLCXD3 | intron variant |
| rs75235442 | 5 | 41474166 | PLCXD3 | intron_variant |
| rs117907903 | 5 | 41474535 | PLCXD3 | intron_variant |
| rs117365372 | 5 | 41474536 | PLCXD3 | intron_variant |
| rs318043 | 5 | 41474553 | PLCXD3 | intron_variant |
| rs318042 | 5 | 41474575 | PLCXD3 | intron_variant |
| rs139821901 | 5 | 41475221 | PLCXD3 | intron_variant |
| rs185789756 | 5 | 41475660 | PLCXD3 | intron_variant |
| rs189078681 | 5 | 41475661 | PLCXD3 | intron_variant |
| rs149824778 | 5 | 41475846 | PLCXD3 | intron_variant |
| rs6451577 | 5 | 41476751 | PLCXD3 | intron_variant |
| rs148818052 | 5 | 41476865 | PLCXD3 | intron_variant |
| rs117592865 | 5 | 41479099 | PLCXD3 | intron_variant |
| rs78415819 | 5 | 41480451 | PLCXD3 | intron_variant |
| rs185514305 | 5 | 41480794 | PLCXD3 | intron_variant |
| rs149877177 | 5 | 41480973 | PLCXD3 | intron_variant |

[Table 1-27]

| SNP ID | chr | position | gene locus | SNP location in gene locus |
|---|---|---|---|---|
| rs144898661 | 5 | 41481008 | PLCXD3 | intron_variant |
| 5:41481231:A:G | 5 | 41481231 | NA | NA |
| rs592607 | 5 | 41482461 | PLCXD3 | intron_variant |
| rs76025981 | 5 | 41484106 | PLCXD3 | intron_variant |
| rs147796140 | 5 | 41485734 | PLCXD3 | intron_variant |
| rs115209137 | 5 | 41485860 | PLCXD3 | intron_variant |
| rs150658476 | 5 | 41487649 | PLCXD3 | intron_variant |
| 5:41492084:G:A | 5 | 41492084 | NA | NA |
| rs318047 | 5 | 41492737 | PLCXD3 | intron_variant |
| rs193139535 | 5 | 41494163 | PLCXD3 | intron_variant |
| 5:41496974:CTT:C | 5 | 41496974 | NA | NA |
| 5:41499344:T:TA | 5 | 41499344 | NA | NA |
| rs79200111 | 5 | 41503974 | PLCXD3 | intron_variant |
| rs143005632 | 5 | 41505112 | PLCXD3 | intron_variant |
| rs80129576 | 5 | 41507151 | PLCXD3 | intron_variant |
| rs149750323 | 5 | 41508080 | PLCXD3 | intron variant |
| rs145378048 | 5 | 41508511 | PLCXD3 | intron_variant |
| rs142480844 | 5 | 41509735 | PLCXD3 | intron_variant |
| 5:41510809:C:T | 5 | 41510809 | NA | NA |
| rs74732406 | 5 | 41511954 | NA | |
| rs77991880 | 5 | 41515898 | NA | |
| rs3863151 | 5 | 41518789 | NA | |
| rs140198935 | 5 | 41521169 | NA | |
| rs140097421 | 5 | 41525867 | NA | |
| rs78523327 | 5 | 41525908 | NA | |
| rs78310877 | 5 | 41528894 | NA | |
| rs79027993 | 5 | 41529340 | NA | |
| rs149621750 | 5 | 41530150 | NA | |
| rs148809865 | 5 | 41530798 | NA | |
| rs142486112 | 5 | 41530854 | NA | |
| 5:41535859:G:A | 5 | 41535859 | NA | NA |
| rs79544594 | 5 | 41538064 | NA | |
| rs78787781 | 5 | 41538772 | NA | |
| rs76663349 | 5 | 41539637 | NA | |
| rs111541941 | 5 | 41541437 | NA | |
| rs77020012 | 5 | 41541725 | NA | |

[Table 1-28]

| SNP ID | chr | position | gene locus | SNP location in gene locus |
|---|---|---|---|---|
| rs79117365 | 5 | 41542625 | NA | |
| 5:41548979:G:A | 5 | 41548979 | NA | NA |
| 5:41566271:G:A | 5 | 41566271 | NA | NA |
| rs142949110 | 5 | 41572513 | NA | |
| 5:41597151:T:C | 5 | 41597151 | NA | NA |
| rs74919030 | 5 | 41611365 | NA | |
| rs137930663 | 5 | 41692962 | NA | |
| 5:41715811:C:T | 5 | 41715811 | NA | NA |
| rs139531587 | 5 | 42148442 | NA | |
| rs79035719 | 5 | 42217448 | NA | |
| rs185613690 | 5 | 42243590 | NA | |
| rs184276051 | 5 | 42257845 | NA | |
| 5:42354014:C:T | 5 | 42354014 | NA | NA |
| rs142622848 | 5 | 42379421 | NA | |
| rs12522729 | 5 | 60541282 | NA | |
| rs74948200 | 5 | 60601182 | NA | |
| rs12522321 | 5 | 60723105 | ZSWIM6 | intron_variant |
| rs79053743 | 5 | 60829488 | ZSWIM6 | intron_variant |
| rs1827387 | 5 | 81930239 | NA | |
| rs74366119 | 5 | 88156454 | MEF2C | intron_variant |
| 5:88795078:G:GAA | 5 | 88795078 | NA | NA |
| rs80106148 | 5 | 92325356 | NA | |
| rs75089614 | 5 | 100358025 | NA | |
| rs28641111 | 5 | 100366992 | NA | |
| rs10072145 | 5 | 100395409 | NA | |
| rs10057008 | 5 | 100395973 | NA | |
| rs10055504 | 5 | 100397154 | NA | |
| rs115735320 | 5 | 100399760 | NA | |
| rs1423681 | 5 | 100404890 | NA | |
| 5:100432544:C:CAT | 5 | 100432544 | NA | NA |
| rs13356491 | 5 | 112378789 | MCC | intron_variant |
| rs6865320 | 5 | 112388791 | MCC | intron_variant |
| 5:124492243:T:C | 5 | 124492243 | NA | NA |
| rs78855508 | 5 | 124557431 | NA | |
| rs73326798 | 5 | 125390012 | NA | |
| rs4373275 | 5 | 125393103 | NA | |

[Table 1-29]

| SNP ID | chr | position | gene locus | SNP location in gene locus |
|---|---|---|---|---|
| 5:125393103:T:G | 5 | 125393103 | NA | NA |
| rs138975964 | 5 | 125393851 | NA | |
| rs60924028 | 5 | 125399302 | NA | |
| rs57885244 | 5 | 125399376 | NA | |
| rs60069077 | 5 | 125399447 | NA | |
| rs57317805 | 5 | 125399676 | NA | |
| rs57908529 | 5 | 125399679 | NA | |
| rs60950409 | 5 | 125400706 | NA | |
| rs73328735 | 5 | 125401914 | NA | |
| rs111944066 | 5 | 125402054 | NA | |
| rs73328738 | 5 | 125402365 | NA | |
| rs58002426 | 5 | 125402709 | NA | |
| rs61572014 | 5 | 125402753 | NA | |
| rs61376002 | 5 | 125402760 | NA | |
| rs57762059 | 5 | 125403219 | NA | |
| rs57282782 | 5 | 125403419 | NA | |
| rs60443581 | 5 | 125403795 | NA | |
| rs73328747 | 5 | 125404393 | NA | |
| rs73328752 | 5 | 125404704 | NA | |
| rs73328754 | 5 | 125405005 | NA | |
| rs73328756 | 5 | 125405014 | NA | |
| rs73328762 | 5 | 125406249 | NA | |
| rs28867125 | 5 | 125408077 | NA | |
| rs141339888 | 5 | 125408665 | NA | |
| rs73328767 | 5 | 125408969 | NA | |
| rs74968976 | 5 | 125410229 | NA | |
| rs6869320 | 5 | 125410380 | NA | |
| rs6870176 | 5 | 125410856 | NA | |
| rs77250321 | 5 | 125412256 | NA | |
| rs73328774 | 5 | 125412457 | NA | |
| 5:143430342:GTTGT:G | 5 | 143430342 | NA | NA |
| rs76612072 | 5 | 147183847 | NA | |
| rs11954595 | 5 | 147194146 | NA | |
| rs78681108 | 5 | 147232262 | NA | |
| rs116969221 | 5 | 151269893 | GLRA1 | intron_variant |
| 5:153395900:GA:G | 5 | 153395900 | NA | NA |

[Table 1-30]

| SNP ID | chr | position | gene locus | SNP location in gene locus |
|---|---|---|---|---|
| 5:153395903:A:G | 5 | 153395903 | NA | NA |
| rs141072734 | 5 | 163003513 | NA | |
| rs79560759 | 5 | 163021709 | NA | |
| rs60283376 | 5 | 166904236 | TENM2 | intron_variant |
| rs77246061 | 5 | 166906243 | TENM2 | intron variant |
| rs79999983 | 5 | 166910600 | TENM2 | intron_variant |
| rs79072342 | 5 | 166910635 | TENM2 | intron_variant |
| rs117190309 | 5 | 166910664 | TENM2 | intron_variant |
| rs140266055 | 5 | 166910826 | TENM2 | intron_variant |
| rs79374298 | 5 | 166911354 | TENM2 | intron_variant |
| rs117911019 | 5 | 166913316 | TENM2 | intron_variant |
| rs59932109 | 5 | 166919170 | TENM2 | intron_variant |
| rs1445795 | 5 | 172839988 | NA | |
| rs10463029 | 5 | 172842872 | NA | |
| rs11134795 | 5 | 172843707 | NA | |
| rs11134796 | 5 | 172844177 | NA | |
| rs13357412 | 5 | 172844784 | NA | |
| rs4867711 | 5 | 172847035 | NA | |
| rs115721274 | 5 | 172858622 | NA | |
| rs190979351 | 5 | 173034050 | NA | |
| rs75505838 | 5 | 173111885 | NA | |
| rs117041609 | 5 | 173234139 | NA | |
| rs75159910 | 5 | 173282893 | NA | |
| rs149874863 | 5 | 173855117 | NA | |
| rs72811273 | 5 | 176225113 | NA | |
| rs7720553 | 5 | 179623637 | RASGEF1C | intron_variant |
| rs60316563 | 6 | 6368046 | NA | |
| rs57686849 | 6 | 6368154 | NA | |
| rs141864440 | 6 | 8237754 | NA | |
| rs9349929 | 6 | 14046145 | NA | |
| rs6915882 | 6 | 14046411 | NA | |
| rs2840257 | 6 | 14048680 | NA | |
| rs909709 | 6 | 14049882 | NA | |
| 6:14051468:TAC:T | 6 | 14051468 | NA | NA |
| 6:14051468:TACAC:T | 6 | 14051468 | NA | NA |
| rs1928638 | 6 | 14057157 | NA | |

[Table 1-31]

| SNP ID | chr | position | gene locus | SNP location in gene locus |
|---|---|---|---|---|
| rs1928639 | 6 | 14057161 | NA | |
| rs57463781 | 6 | 14057622 | NA | |
| rs12208529 | 6 | 14063790 | NA | |
| rs9476476 | 6 | 14064273 | NA | |
| rs72836088 | 6 | 19842560 | NA | |
| rs115079901 | 6 | 29558364 | NA | NA |
| rs200911534 | 6 | 30741569 | NA | |
| 6:30744985:G:T | 6 | 30744985 | NA | NA |
| rs191987001 | 6 | 30775579 | NA | NA |
| rs2499716 | 6 | 34073338 | GRM4 | intron_variant |
| rs1873248 | 6 | 34073344 | GRM4 | intron_variant |
| rs139096142 | 6 | 44147011 | CAPN11 | intron_variant |
| rs78401606 | 6 | 70051582 | ADGRB3 | intron_variant |
| rs144698880 | 6 | 70207289 | NA | |
| 6:70210202:G:A | 6 | 70210202 | NA | NA |
| rs11757257 | 6 | 70217452 | NA | |
| rs13205898 | 6 | 70271095 | NA | |
| rs79873641 | 6 | 71389721 | SMAP1 | intron_variant |
| rs145666404 | 6 | 71513933 | SMAP1 | intron_variant |
| rs78049725 | 6 | 75836785 | C0L12A1 | intron_variant |
| rs77854307 | 6 | 82472695 | NA | |
| rs79069596 | 6 | 82806731 | NA | |
| rs77125903 | 6 | 82807806 | NA | |
| rsl1962648 | 6 | 82824374 | NA | |
| rs6914737 | 6 | 82824575 | NA | |
| rs201137924 | 6 | 82824635 | NA | |
| rs77793849 | 6 | 82825638 | NA | |
| rs75421360 | 6 | 82826545 | NA | |
| rs74572694 | 6 | 82831444 | NA | |
| rs11965072 | 6 | 82832842 | NA | |
| rs11755112 | 6 | 82833728 | NA | |
| rs36156078 | 6 | 82838594 | NA | |
| rs2152842 | 6 | 82841134 | NA | |
| rs11967918 | 6 | 82842822 | NA | |
| rs10943858 | 6 | 82845197 | NA | |
| rs79009267 | 6 | 82846023 | NA | |

[Table 1-32]

| SNP ID | chr | position | gene locus | SNP location in gene locus |
|---|---|---|---|---|
| rs11961436 | 6 | 82847140 | NA | |
| rsl1752161 | 6 | 82848130 | NA | |
| rs11752130 | 6 | 82848159 | NA | |
| rs77937699 | 6 | 82880897 | IBTK | 3 prime UTR variant |
| rs201520516 | 6 | 82965363 | NA | |
| rs76325080 | 6 | 84248518 | NA | |
| rs78628697 | 6 | 86590415 | NA | |
| rs58725789 | 6 | 86600143 | NA | |
| 6:91762389:C:A | 6 | 91762389 | NA | NA |
| rs112761794 | 6 | 105688813 | NA | |
| rs141339608 | 6 | 105688876 | NA | NA |
| rs72936201 | 6 | 105691598 | NA | |
| rs111427560 | 6 | 105692447 | NA | |
| rs183560286 | 6 | 105692807 | NA | NA |
| rs142938303 | 6 | 105695489 | NA | |
| 6:105697055:G:T | 6 | 105697055 | NA | NA |
| rs35695447 | 6 | 105699862 | NA | |
| rs111612241 | 6 | 105703217 | NA | |
| rs17065682 | 6 | 105704149 | NA | |
| rs17065694 | 6 | 105704658 | NA | |
| rs10499042 | 6 | 105706581 | NA | |
| rs111857724 | 6 | 105707260 | NA | |
| rs113487540 | 6 | 105707498 | NA | |
| rs72938014 | 6 | 105707927 | NA | |
| rs 17054938 | 6 | 105709113 | NA | |
| rs72938025 | 6 | 105713764 | NA | |
| rs75718226 | 6 | 105715441 | NA | |
| rs72938030 | 6 | 105716807 | NA | |
| rs72938031 | 6 | 105716902 | NA | |
| rs72938040 | 6 | 105721926 | PREP | 3_prime_UTR_variant |
| rs17054949 | 6 | 105721969 | PREP | 3_prime UTR variant |
| rs111772455 | 6 | 105722560 | PREP | 3_prime_UTR_variant |
| rs17467544 | 6 | 105722943 | PREP | 3_prime_UTR_variant |
| rs41285466 | 6 | 105726874 | PREP | intron_variant |
| rs72944139 | 6 | 105727873 | PREP | intron_variant |
| rs72944142 | 6 | 105728191 | PREP | intron_variant |

EP 4 324 922 A1

[Table 1-33]

| SNP ID | chr | position | gene locus | SNP location in gene locus |
|---|---|---|---|---|
| rs77676511 | 6 | 105729054 | PREP | intron_variant |
| rs2236285 | 6 | 105730619 | PREP | intron_variant |
| rs3800001 | 6 | 105735808 | PREP | intron_variant |
| rs17546638 | 6 | 105738261 | PREP | intron_variant |
| rs17546735 | 6 | 105741586 | PREP | intron_variant |
| rs17468237 | 6 | 105742660 | PREP | intron_variant |
| rs17068024 | 6 | 107484367 | PDSS2 | intron_variant |
| rs145412027 | 6 | 107493416 | PDSS2 | intron_variant |
| rs75692260 | 6 | 107498607 | PDSS2 | intron_variant |
| rs147212820 | 6 | 107504762 | PDSS2 | intron_variant |
| rs79713062 | 6 | 110875473 | NA | |
| rs12195889 | 6 | 129623880 | LAMA2 | intron variant |
| rs2451682 | 6 | 129625432 | LAMA2 | intron_variant |
| rs12200836 | 6 | 129640745 | LAMA2 | intron variant |
| rs12200538 | 6 | 129646004 | LAMA2 | intron_variant |
| rs2451679 | 6 | 129660222 | LAMA2 | intron_variant |
| rs77026545 | 6 | 137559310 | NA | |
| rs117451378 | 6 | 137570062 | NA | |
| rs57516822 | 6 | 137739295 | NA | |
| rs59391960 | 6 | 137739389 | NA | |
| rs77091814 | 6 | 137742839 | NA | |
| rs73566001 | 6 | 137744363 | NA | |
| rs7769446 | 6 | 137745965 | NA | |
| rs71811814 | 6 | 137746238 | NA | NA |
| rs6917588 | 6 | 137746860 | NA | |
| rs1335302 | 6 | 141637219 | NA | |
| rs1335308 | 6 | 141645736 | NA | |
| rs4270804 | 6 | 141652711 | NA | |
| rs9376607 | 6 | 141661460 | NA | |
| rs9403288 | 6 | 141672901 | NA | |
| rs9399363 | 6 | 141682404 | NA | |
| rs9373305 | 6 | 141683547 | NA | |
| rs11760135 | 6 | 141685630 | NA | |
| 6:141689730:AT:A | 6 | 141689730 | NA | NA |
| rs9376611 | 6 | 141692633 | NA | |
| rs11754010 | 6 | 141700708 | NA | |

260

[Table 1-34]

| SNP ID | chr | position | gene locus | SNP location in gene locus |
|---|---|---|---|---|
| rs55968168 | 6 | 141703453 | NA | |
| rs9403300 | 6 | 141706902 | NA | |
| 6:141712104:G:A | 6 | 141712104 | NA | NA |
| rs73779399 | 6 | 145196065 | NA | |
| rs55645496 | 6 | 145197134 | NA | |
| rs73779401 | 6 | 145199490 | NA | |
| rs73781203 | 6 | 145200275 | NA | |
| rs73781205 | 6 | 145201088 | NA | |
| rs73781206 | 6 | 145201247 | NA | |
| rs6940222 | 6 | 145203608 | NA | |
| rs73781208 | 6 | 145205435 | NA | |
| rs57049867 | 6 | 145205680 | NA | |
| rs73781214 | 6 | 145210203 | NA | |
| rs62436288 | 6 | 147952729 | SAMD5 | intron_variant |
| rs2064333 | 6 | 147953574 | SAMD5 | intron_variant |
| rs17388532 | 6 | 147956771 | SAMD5 | intron_variant |
| rs7753842 | 6 | 148553550 | NA | |
| rs1871921 | 6 | 149221294 | UST | intron_variant |
| rs17665064 | 6 | 149226781 | UST | intron_variant |
| rs7760563 | 6 | 149232496 | UST | intron_variant |
| rs72994230 | 6 | 149236366 | UST | intron_variant |
| rs112183061 | 6 | 149236666 | UST | intron_variant |
| 6:149269058:G:GA | 6 | 149269058 | NA | NA |
| 6:149269058:G:GAA | 6 | 149269058 | NA | NA |
| rs117816679 | 6 | 150790648 | NA | |
| rs147175280 | 6 | 150793218 | NA | |
| rs146914832 | 6 | 150795155 | NA | |
| 6:150796988:G:T | 6 | 150796988 | NA | NA |
| rs148080540 | 6 | 150806519 | NA | |
| rs117959775 | 6 | 150809802 | NA | |
| rs111474682 | 6 | 150811773 | NA | |
| rs77886049 | 6 | 156030889 | NA | |
| rs9397273 | 6 | 156248029 | NA | |
| rs4370375 | 6 | 156250340 | NA | |
| rs7756499 | 6 | 156250986 | NA | |
| rs9384398 | 6 | 156252277 | NA | |

[Table 1-35]

| SNP ID | chr | position | gene locus | SNP location in gene locus |
|---|---|---|---|---|
| rs9397274 | 6 | 156253640 | NA | |
| rs7740419 | 6 | 156255416 | NA | |
| rs17086886 | 6 | 156261635 | NA | |
| rs74781715 | 6 | 156271293 | NA | |
| rs9397864 | 6 | 156271539 | NA | |
| rs4259259 | 6 | 156272383 | NA | |
| rs9397865 | 6 | 156272671 | NA | |
| rs9397866 | 6 | 156272681 | NA | |
| rs12111075 | 6 | 162304205 | PRKN | intron_variant |
| rs12110765 | 6 | 162304245 | PRKN | intron_variant |
| rs58436306 | 6 | 170758245 | NA | |
| rs7750857 | 6 | 170760133 | NA | |
| rs140838906 | 6 | 170761583 | NA | |
| 7:4230856:G:A | 7 | 4230856 | NA | NA |
| rs201457651 | 7 | 9092066 | NA | |
| rs148328240 | 7 | 9093559 | NA | |
| rs10265621 | 7 | 9099399 | NA | |
| rs76355504 | 7 | 9313494 | NA | |
| rs58749480 | 7 | 13292034 | NA | |
| rs139024590 | 7 | 17010996 | NA | NA |
| rs11980801 | 7 | 22290160 | RAPGEF5 | intron_variant |
| rs2686469 | 7 | 22299059 | RAPGEF5 | intron_variant |
| 7:22888700:CAA:CTTAA | 7 | 22888700 | NA | NA |
| rs182887339 | 7 | 22895543 | NA | |
| rs80009912 | 7 | 22920084 | NA | |
| rs147497510 | 7 | 28699881 | CREB5 | intron_variant |
| rs73685962 | 7 | 30292368 | NA | |
| rs6958640 | 7 | 30293612 | NA | |
| rs114684317 | 7 | 30294944 | NA | |
| rs149057499 | 7 | 30296625 | NA | |
| rs6970146 | 7 | 30297969 | NA | |
| rs77413551 | 7 | 30301817 | NA | |
| rs201581869 | 7 | 30303728 | NA | |
| 7:30303731:GCACTCCTCCC:G | 7 | 30303731 | NA | NA |
| 7:30303742:GC:G | 7 | 30303742 | NA | NA |

[Table 1-36]

| SNP ID | chr | position | gene locus | SNP location in gene locus |
|---|---|---|---|---|
| rs149622189 | 7 | 30307465 | NA | |
| rs200550125 | 7 | 30311765 | NA | |
| 7:30319553:TA:T | 7 | 30319553 | NA | NA |
| rs114645450 | 7 | 30321650 | NA | |
| rs79699598 | 7 | 30322482 | NA | |
| 7:30324192:C:CG | 7 | 30324192 | NA | NA |
| rs143478611 | 7 | 30327096 | ZNRF2 | intron_variant |
| rs6970041 | 7 | 30332914 | ZNRF2 | intron_variant |
| rs 17158839 | 7 | 30335403 | ZNRF2 | intron_variant |
| rs148707872 | 7 | 30336156 | ZNRF2 | intron_variant |
| rs115884980 | 7 | 30336503 | ZNRF2 | intron_variant |
| rs140607084 | 7 | 30336572 | ZNRF2 | intron_variant |
| rs147812016 | 7 | 30336672 | ZNRF2 | intron_variant |
| rs116610215 | 7 | 30338896 | ZNRF2 | intron variant |
| rs74501594 | 7 | 30339220 | ZNRF2 | intron_variant |
| rs6968097 | 7 | 30344956 | ZNRF2 | intron_variant |
| 7:30346948:T:G | 7 | 30346948 | NA | NA |
| rs8180762 | 7 | 30348387 | ZNRF2 | intron_variant |
| rs73685995 | 7 | 30349511 | ZNRF2 | intron_variant |
| rs9639614 | 7 | 30350328 | ZNRF2 | intron_variant |
| rs7804225 | 7 | 30350755 | ZNRF2 | intronvariant |
| rs34458739 | 7 | 30351379 | ZNRF2 | intronvariant |
| 7:30355351:G:C | 7 | 30355351 | NA | NA |
| rs112527213 | 7 | 30358471 | ZNRF2 | intron_variant |
| rs6959139 | 7 | 30360832 | ZNRF2 | intron variant |
| rs78301492 | 7 | 30362276 | ZNRF2 | intron_variant |
| rs140099650 | 7 | 30362853 | ZNRF2 | intron_variant |
| rs114690145 | 7 | 30364225 | ZNRF2 | intron_variant |
| rs6943601 | 7 | 30365310 | ZNRF2 | intron_variant |
| rs77946931 | 7 | 30365824 | ZNRF2 | intron_variant |
| rs115112447 | 7 | 30365833 | ZNRF2 | intron_variant |
| rs12056300 | 7 | 30367221 | ZNRF2 | intron_variant |
| rs17158851 | 7 | 30368265 | ZNRF2 | intron_variant |
| rs56694867 | 7 | 30368384 | ZNRF2 | intron_variant |
| rs12056131 | 7 | 30369299 | ZNRF2 | intron_variant |
| rs6979671 | 7 | 30370051 | ZNRF2 | intron_variant |

[Table 1-37]

| SNP ID | chr | position | gene locus | SNP location in gene locus |
|---|---|---|---|---|
| rs11974360 | 7 | 30370786 | ZNRF2 | intron_variant |
| rs76302490 | 7 | 30373774 | ZNRF2 | intron_variant |
| rs80280885 | 7 | 30373940 | ZNRF2 | intron_variant |
| rs145282100 | 7 | 30375239 | ZNRF2 | intron variant |
| rs9691527 | 7 | 30376086 | ZNRF2 | intron variant |
| rs11979006 | 7 | 30381303 | ZNRF2 | intron_variant |
| 1:30383851:CT:G | 7 | 30383851 | NA | NA |
| rs111250775 | 7 | 30384822 | ZNRF2 | intron_variant |
| rs80107665 | 7 | 30387141 | ZNRF2 | intron variant |
| rs146758046 | 7 | 30387248 | ZNRF2 | intron_variant |
| rs17158885 | 7 | 30389865 | ZNRF2 | intron_variant |
| rs17158888 | 7 | 30391066 | ZNRF2 | intron_variant |
| rs11531494 | 7 | 30392422 | ZNRF2 | intron_variant |
| rs42580 | 7 | 30397028 | ZNRF2 | intron_variant |
| rs42589 | 7 | 30402571 | AC006978.2 | intron_variant |
| rs73695251 | 7 | 39331345 | P0U6F2 | NMD_transcript_variant |
| rs73695253 | 7 | 39333216 | P0U6F2 | NMD_transcript_variant |
| rs117797113 | 7 | 42239771 | GL13 | intron_variant |
| rs1525251 | 7 | 46738745 | NA | |
| 7:46949185:A:ACCCCC | 7 | 46949185 | NA | NA |
| rs12673320 | 7 | 46955408 | NA | |
| rs58346043 | 7 | 46957249 | NA | |
| rs55968856 | 7 | 46963997 | NA | |
| 7:46964575:GT:G | 7 | 46964575 | NA | NA |
| rs115849624 | 7 | 46964743 | NA | NA |
| rs13224225 | 7 | 46966997 | NA | |
| rs10951882 | 7 | 46967335 | NA | |
| rs57132170 | 7 | 46968669 | NA | |
| rs16881424 | 7 | 46973649 | NA | |
| rs117604188 | 7 | 52054590 | NA | |
| rs146024011 | 7 | 52061279 | NA | |
| rs114085512 | 7 | 52062875 | NA | |
| rs117563572 | 7 | 52067629 | NA | |
| rs138791169 | 7 | 52068597 | NA | |
| rs139032318 | 7 | 52071591 | NA | |
| rs78326066 | 7 | 52146162 | NA | |

[Table 1-38]

| SNP ID | chr | position | gene locus | SNP location in gene locus |
|---|---|---|---|---|
| rs77343772 | 7 | 73408830 | NA | |
| rs6972672 | 7 | 73409861 | NA | |
| rs6973358 | 7 | 73410290 | NA | |
| rs6973541 | 7 | 73410408 | NA | |
| 7:75438562:T:TTTTTTA | 7 | 75438562 | NA | NA |
| rs76364545 | 7 | 75440752 | NA | |
| rs11465296 | 7 | 75442294 | CCL24 | intron_variant |
| rs11465293 | 7 | 75442723 | CCL24 | missense_variant |
| rsl1465286 | 7 | 75443961 | CCL24 | intron_variant |
| rs79404066 | 7 | 75444215 | CCL24 | intron_variant |
| rs117679282 | 7 | 75446052 | CCL24 | intron_variant |
| rs11514980 | 7 | 75459923 | NA | |
| rs11521067 | 7 | 75459954 | NA | |
| rs147465974 | 7 | 75468367 | NA | |
| 7:76910430:C:CA | 7 | 76910430 | NA | NA |
| 7:101289699:G:A | 7 | 101289699 | NA | NA |
| rs145263975 | 7 | 128449126 | CCDC136 | intron variant |
| rs189900162 | 7 | 128453455 | CCDC136 | intron_variant |
| rs149344851 | 7 | 128455033 | CCDC136 | intron_variant |
| rs143205471 | 7 | 128464165 | NA | |
| rs147658468 | 7 | 128465079 | NA | |
| rs141683491 | 7 | 128469854 | NA | |
| rs199842696 | 7 | 128470680 | FLNC | 5_prime_UTR_variant |
| rs151072488 | 7 | 128473938 | FLNC | intron_variant |
| rs138755199 | 7 | 128475236 | FLNC | intron_variant |
| rs144736830 | 7 | 128479080 | FLNC | intron_variant |
| rs60691847 | 7 | 128499388 | NA | |
| rs73459264 | 7 | 128505510 | KCP | intron_variant |
| rs183994302 | 7 | 128513368 | KCP | intron_variant |
| rs112749538 | 7 | 128513415 | KCP | intron_variant |
| rs182514276 | 7 | 128514838 | KCP | intron_variant |
| 7:128522809:AAAAC:A | 7 | 128522809 | NA | NA |
| 7:128522809:AAAACAAACAAAC =AAAAC | 7 | 128522809 | NA | NA |
| rs143202507 | 7 | 128523082 | KCP | intron_variant |
| rs111838117 | 7 | 128524190 | KCP | intron_variant |

[Table 1-39]

| SNP ID | chr | position | gene locus | SNP location in gene locus |
|---|---|---|---|---|
| rs144312682 | 7 | 128526221 | KCP | intron_variant |
| 7:131372591:A:G | 7 | 131372591 | NA | NA |
| rs137956592 | 7 | 138063857 | NA | |
| rs186944015 | 7 | 141792526 | MGAM | intron_variant |
| 7:148274173:T:C | 7 | 148274173 | NA | NA |
| rs140478856 | 7 | 148374761 | NA | |
| rs77070865 | 7 | 148384615 | NA | |
| rs35116518 | 7 | 148386436 | NA | |
| rs114140823 | 7 | 148386467 | NA | |
| rs34369394 | 7 | 148388377 | NA | |
| rs10235843 | 7 | 148400466 | CUL1 | intron_variant |
| 7:148422315:A:G | 7 | 148422315 | NA | NA |
| rs11974639 | 7 | 148438804 | CUL1 | intron_variant |
| rs147617254 | 7 | 148458807 | CUL1 | intron_variant |
| rs 17626232 | 7 | 148459675 | CUL1 | intron_variant |
| rs10246773 | 7 | 148460536 | CUL1 | intron_variant |
| rs3807446 | 7 | 148467447 | CUL1 | intron_variant |
| rs758880 | 7 | 148477589 | CUL1 | intron_variant |
| rs113817468 | 7 | 148478201 | CUL1 | intron_variant |
| rs10245290 | 7 | 148530294 | EZH2 | intron_variant |
| rs73158265 | 7 | 148537086 | EZH2 | intron_variant |
| rs56248471 | 7 | 148553810 | EZH2 | intron_variant |
| rs10952780 | 7 | 148554335 | EZH2 | intron_variant |
| rs113910590 | 7 | 148554794 | EZH2 | intron_variant |
| 7:148558320:CT:C | 7 | 148558320 | NA | NA |
| rs10952783 | 7 | 148559071 | EZH2 | intron_variant |
| 7:148560015:G:GTTTTGT | 7 | 148560015 | NA | NA |
| rs28706086 | 7 | 148560068 | EZH2 | intron_variant |
| rs10259102 | 7 | 148561029 | EZH2 | intron_variant |
| rs10249392 | 7 | 148561324 | EZH2 | intron_variant |
| rs11976154 | 7 | 148561586 | EZH2 | intron_variant |
| rs28617605 | 7 | 148562161 | EZH2 | intron_variant |
| 7:148562217:CAAA:C | 7 | 148562217 | NA | NA |
| rs1996996 | 7 | 148562543 | EZH2 | intron_variant |
| rs73158280 | 7 | 148563393 | EZH2 | intron_variant |

[Table 1-40]

| SNP ID | chr | position | gene locus | SNP location in gene locus |
|---|---|---|---|---|
| rs142648914 | 7 | 148563610 | EZH2 | intron_variant |
| rs142248416 | 7 | 148563611 | EZH2 | intron_variant |
| rs111880657 | 7 | 148564058 | EZH2 | intron_variant |
| rs113161209 | 7 | 148564367 | EZH2 | intron_variant |
| rs73158281 | 7 | 148565649 | EZH2 | intron variant |
| rs149022366 | 7 | 148566024 | EZH2 | intron_variant |
| rs1917057 | 7 | 148567957 | EZH2 | intron_variant |
| rs2373415 | 7 | 148570280 | EZH2 | intron_variant |
| rs2373416 | 7 | 148570344 | EZH2 | intron variant |
| rs2373417 | 7 | 148570382 | EZH2 | intron_variant |
| rs2373418 | 7 | 148570447 | EZH2 | intron_variant |
| rs7458365 | 7 | 148570551 | EZH2 | intron_variant |
| rs28455679 | 7 | 148570741 | EZH2 | intron_variant |
| rs6972079 | 7 | 148571196 | EZH2 | intron variant |
| rs6963116 | 7 | 148571393 | EZH2 | intron_variant |
| rs28814083 | 7 | 148573027 | EZH2 | intron_variant |
| rs28844638 | 7 | 148573061 | EZH2 | intron_variant |
| rs12531383 | 7 | 148573311 | AC073140. 1 | non_coding_transcript_exon_variant |
| 7:148573384:G:GA | 7 | 148573384 | NA | NA |
| rs149343167 | 7 | 148573692 | NA | NA |
| rs7783459 | 7 | 148573898 | EZH2 | intron_variant |
| rs7795158 | 7 | 148576193 | EZH2 | intron variant |
| rs1880357 | 7 | 148577610 | EZH2 | intron_variant |
| rs2177567 | 7 | 148578753 | EZH2 | intron_variant |
| rs80052686 | 7 | 148580809 | EZH2 | intron_variant |
| rs11984309 | 7 | 148589869 | NA | |
| rs67648693 | 7 | 148591911 | NA | |
| 7:148595511:C:CA | 7 | 148595511 | NA | NA |
| 7:148596986:C:CT | 7 | 148596986 | NA | NA |
| rs202221022 | 7 | 148597557 | NA | NA |
| rs7790642 | 7 | 148600310 | NA | |
| rs7777746 | 7 | 148602583 | NA | |
| rs62505409 | 7 | 148602689 | NA | |
| rs58371016 | 7 | 148604818 | NA | |
| rs6558814 | 8 | 3589281 | CSMD1 | intron_variant |
| rs79572000 | 8 | 3594099 | CSMD1 | intron_variant |

[Table 1-41]

| SNP ID | chr | position | gene locus | SNP location in gene locus |
|---|---|---|---|---|
| rs79727403 | 8 | 3595476 | CSMD1 | intron_variant |
| rs78364798 | 8 | 3596451 | CSMD1 | intron_variant |
| rs140360037 | 8 | 3596652 | CSMD1 | intron_variant |
| rs17067299 | 8 | 3599207 | CSMD1 | intron_variant |
| rs74706909 | 8 | 3600132 | CSMD1 | intron_variant |
| rs74985428 | 8 | 3600451 | CSMD1 | intron_variant |
| rs12549613 | 8 | 3601137 | CSMD1 | intron_variant |
| rs79210493 | 8 | 3602245 | CSMD1 | intron variant |
| rs4639541 | 8 | 3604375 | CSMD1 | intron_variant |
| rs4424283 | 8 | 3605823 | CSMD1 | intron_variant |
| rs78615686 | 8 | 4795722 | CSMD1 | intron_variant |
| rs75778595 | 8 | 4801168 | CSMD1 | intron_variant |
| rs6558944 | 8 | 4802163 | CSMD1 | intron_variant |
| rs75096153 | 8 | 4807509 | CSMD1 | intron_variant |
| rs80229469 | 8 | 4823427 | CSMD1 | intron_variant |
| rs75338858 | 8 | 4825118 | CSMD1 | intron_variant |
| rs76891224 | 8 | 4827394 | CSMD1 | intron_variant |
| rs3911307 | 8 | 4839914 | CSMD1 | intron_variant |
| rs77772200 | 8 | 4840914 | CSMD1 | intron_variant |
| rs73497791 | 8 | 4842344 | CSMD1 | intron_variant |
| rs74350143 | 8 | 4842467 | CSMD1 | intron_variant |
| rs17071982 | 8 | 4842970 | CSMD1 | intron_variant |
| rs6999675 | 8 | 4844827 | CSMD1 | intron_variant |
| rs7013059 | 8 | 4845003 | CSMD1 | intron_variant |
| rs145241498 | 8 | 4854469 | NA | |
| rs78561947 | 8 | 4856358 | NA | |
| rs79998369 | 8 | 4858392 | NA | |
| rs75281977 | 8 | 4863064 | NA | |
| rs74639771 | 8 | 4870975 | NA | |
| rs77504238 | 8 | 5030143 | NA | |
| rs73219702 | 8 | 13552293 | NA | |
| rs79671431 | 8 | 15284873 | TUSC3 | non_coding_transcript_variant |
| rs12155706 | 8 | 16684041 | NA | |
| rs12155708 | 8 | 16684111 | NA | |
| rs56340335 | 8 | 16685007 | NA | |
| rs73201722 | 8 | 16687313 | NA | |

[Table 1-42]

| SNP ID | chr | position | gene locus | SNP location in gene locus |
|---|---|---|---|---|
| rs73201728 | 8 | 16688532 | NA | |
| rs73201732 | 8 | 16689879 | NA | |
| rs73201733 | 8 | 16689886 | NA | |
| rs4922106 | 8 | 19691250 | INTS10 | intron_variant |
| 8:19691517:C:CA | 8 | 19691517 | NA | NA |
| rs4922107 | 8 | 19691603 | INTS10 | intron_variant |
| rs140015982 | 8 | 26335752 | BNIP3L | intron_variant |
| rs112411185 | 8 | 53110658 | ST18 | intron_variant |
| rs62509183 | 8 | 64209694 | NA | |
| rs147990281 | 8 | 65206514 | NA | |
| 8:65811756:TC:T | 8 | 65811756 | NA | NA |
| rs147627421 | 8 | 72783939 | NA | |
| rs147840558 | 8 | 72968161 | TRPA1 | intron_variant |
| rs142352715 | 8 | 72968759 | TRPA1 | intron_variant |
| rs141156682 | 8 | 72970760 | TRPA1 | intron_variant |
| rs2587560 | 8 | 72990136 | NA | |
| rs2587563 | 8 | 72991997 | NA | |
| rs2587564 | 8 | 72992052 | NA | |
| rs2587565 | 8 | 72992580 | NA | |
| rs1443951 | 8 | 72993388 | NA | |
| rs2587568 | 8 | 72994349 | NA | |
| rs2587569 | 8 | 72994434 | NA | |
| rs2701457 | 8 | 72995307 | NA | |
| rs2587572 | 8 | 72995403 | NA | |
| rs1838286 | 8 | 72995797 | NA | |
| rs2587574 | 8 | 72996845 | NA | |
| rs2587575 | 8 | 72997364 | NA | |
| rs1899762 | 8 | 72997899 | NA | |
| rs1899761 | 8 | 72998278 | NA | |
| rs2701455 | 8 | 72998638 | NA | |
| rs13274010 | 8 | 73000500 | NA | |
| rs59359973 | 8 | 73001147 | NA | |
| rs1443950 | 8 | 73001565 | NA | |
| rs1443949 | 8 | 73001624 | NA | |
| rs10504528 | 8 | 73001662 | NA | |
| rs10504529 | 8 | 73001833 | NA | |

[Table 1-43]

| SNP ID | chr | position | gene locus | SNP location in gene locus |
|---|---|---|---|---|
| rs11995530 | 8 | 73002151 | NA | |
| rsl443947 | 8 | 73002691 | NA | |
| rs34722660 | 8 | 73003389 | NA | |
| rs16938000 | 8 | 73003527 | NA | |
| rs34257316 | 8 | 73003708 | NA | |
| rs34109030 | 8 | 73003720 | NA | |
| rs2383847 | 8 | 73004294 | NA | |
| rs2383848 | 8 | 73004297 | NA | |
| rs2383850 | 8 | 73004347 | NA | |
| rs2383851 | 8 | 73004372 | NA | |
| rs11444287 | 8 | 73004443 | NA | |
| rs2120468 | 8 | 73005062 | NA | |
| rs2028707 | 8 | 73005371 | NA | |
| rs35398772 | 8 | 73016364 | NA | |
| rsl3276665 | 8 | 73016825 | NA | |
| rs17815050 | 8 | 73017580 | NA | |
| rsl3278765 | 8 | 73018900 | NA | |
| rs4601363 | 8 | 73024793 | NA | |
| rs35873044 | 8 | 73025944 | NA | |
| rs13267160 | 8 | 73030409 | NA | |
| rs28429254 | 8 | 76012207 | NA | |
| rs17370342 | 8 | 76012812 | NA | |
| rs10504590 | 8 | 76013464 | NA | |
| rs28450580 | 8 | 76014941 | NA | |
| rs142904843 | 8 | 89944036 | NA | |
| rs76655252 | 8 | 89952367 | NA | |
| rs114639281 | 8 | 90009741 | NA | |
| 8:101583921:A:C | 8 | 101583921 | NA | NA |
| rs184447468 | 8 | 101800848 | NA | |
| rs143939515 | 8 | 103149542 | NA | |
| rs6993195 | 8 | 103164396 | NA | |
| rs142025872 | 8 | 103169327 | NA | |
| rs56191596 | 8 | 103177900 | NA | |
| rs33954588 | 8 | 106008738 | ZFPM2 | non_coding_transcript_variant |
| rs1879688 | 8 | 106010200 | ZFPM2 | non_coding_transcript_variant |
| 8:106025397:TAAA:T | 8 | 106025397 | NA | NA |

[Table 1-44]

| SNP ID | chr | position | gene locus | SNP location in gene locus |
|---|---|---|---|---|
| rs6984262 | 8 | 106030373 | ZFPM2 | non_coding_transcript_variant |
| rs73304184 | 8 | 106537180 | ZFPM2 | intron_variant |
| rs76868435 | 8 | 106537986 | ZFPM2 | intron_variant |
| rs79001083 | 8 | 106538183 | ZFPM2 | intron variant |
| rs73304192 | 8 | 106540300 | ZFPM2 | intron variant |
| rs74395542 | 8 | 106544183 | ZFPM2 | intron_variant |
| 8:106545091:GTT:GT | 8 | 106545091 | NA | NA |
| 8:106545091:GTT: G | 8 | 106545091 | NA | NA |
| rs73306213 | 8 | 106550015 | ZFPM2 | intron_variant |
| rs73306228 | 8 | 106556176 | ZFPM2 | intron_variant |
| rs73306230 | 8 | 106558263 | ZFPM2 | intron_variant |
| rs73306231 | 8 | 106559352 | ZFPM2 | intron_variant |
| rs7844467 | 8 | 106562252 | ZFPM2 | intron_variant |
| rs34823616 | 8 | 106566606 | ZFPM2 | intron variant |
| 8:108811752:T:C | 8 | 108811752 | NA | NA |
| 8:108849027:T: A | 8 | 108849027 | NA | NA |
| 8:108850008:G:A | 8 | 108850008 | NA | NA |
| 8:108850585:G:A | 8 | 108850585 | NA | NA |
| rs115268316 | 8 | 108863551 | NA | |
| rs57284855 | 8 | 108865154 | NA | |
| 8:108870725:G:A | 8 | 108870725 | NA | NA |
| rs149805601 | 8 | 108872570 | NA | |
| rs13269427 | 8 | 108873161 | NA | |
| rs186061014 | 8 | 108889120 | NA | |
| 8:108896855:G:A | 8 | 108896855 | NA | NA |
| rs146645406 | 8 | 108899766 | NA | |
| rs150156788 | 8 | 108900989 | NA | |
| 8:108901410:A:G | 8 | 108901410 | NA | NA |
| 8:108906538:C:T | 8 | 108906538 | NA | NA |
| 8:108907254:G:T | 8 | 108907254 | NA | NA |
| 8:108907257:C:G | 8 | 108907257 | NA | NA |
| rs191118779 | 8 | 108907979 | NA | NA |
| 8:108908022:G:A | 8 | 108908022 | NA | NA |
| rs191097915 | 8 | 108915170 | RSP02 | intron_variant |
| rs114143791 | 8 | 108916220 | RSP02 | intron_variant |
| 8:108921884:GT:G | 8 | 108921884 | NA | NA |

[Table 1-45]

| SNP ID | chr | position | gene locus | SNP location in gene locus |
|---|---|---|---|---|
| rs138179792 | 8 | 108923715 | RSP02 | intron_variant |
| rs140554138 | 8 | 108925826 | RSP02 | intron_variant |
| rs144255075 | 8 | 108925827 | RSP02 | intron_variant |
| 8:108927282:A:C | 8 | 108927282 | NA | NA |
| 8:108928843:G:A | 8 | 108928843 | NA | NA |
| rs140670079 | 8 | 108929408 | RSP02 | intron_variant |
| rs190110401 | 8 | 108930742 | RSP02 | intron_variant |
| rs192028485 | 8 | 116822572 | NA | |
| 8:116832799:T:C | 8 | 116832799 | NA | NA |
| rs7836570 | 8 | 116840302 | NA | |
| rs16887807 | 8 | 116843619 | NA | |
| 8:116846927:A:C | 8 | 116846927 | NA | NA |
| rs28366597 | 8 | 116851543 | NA | |
| 8:116855719:T:A | 8 | 116855719 | NA | NA |
| 8:126647803:C:A | 8 | 126647803 | NA | NA |
| 8:142514003:AT:A | 8 | 142514003 | NA | NA |
| rs34896467 | 9 | 2213511 | NA | |
| rs12350147 | 9 | 2217178 | NA | |
| rs7024532 | 9 | 2220348 | NA | |
| rs71504697 | 9 | 2222951 | NA | |
| rs71504698 | 9 | 2223409 | NA | |
| rs17390013 | 9 | 2226756 | NA | |
| rs10491702 | 9 | 2227837 | NA | |
| rs10491704 | 9 | 2228755 | NA | |
| rs12344786 | 9 | 2229313 | NA | |
| rs74987881 | 9 | 2229455 | NA | |
| rs1105379 | 9 | 16319287 | NA | |
| 9:16325344:G:A | 9 | 16325344 | NA | NA |
| rs72714927 | 9 | 16325344 | NA | |
| rs67783457 | 9 | 16325568 | NA | |
| rs12553053 | 9 | 16330357 | NA | |
| rs56190435 | 9 | 16331743 | NA | |
| rs12686134 | 9 | 19605230 | SLC24A2 | intron_variant |
| rs35444308 | 9 | 19605274 | SLC24A2 | intron variant |
| rs141589871 | 9 | 19609951 | SLC24A2 | intron_variant |
| rs752539 | 9 | 19640344 | SLC24A2 | intron_variant |

[Table 1-46]

| SNP ID | chr | position | gene locus | SNP location in gene locus |
|---|---|---|---|---|
| rs12683123 | 9 | 19642100 | SLC24A2 | intron_variant |
| rs16937677 | 9 | 19642563 | SLC24A2 | intron_variant |
| rs75209268 | 9 | 19653017 | SLC24A2 | intron_variant |
| rs12686530 | 9 | 19655641 | SLC24A2 | intron_variant |
| rs78907538 | 9 | 19655825 | SLC24A2 | intron variant |
| rs75642022 | 9 | 19657307 | SLC24A2 | intron_variant |
| rs72501450 | 9 | 19658168 | SLC24A2 | intron_variant |
| rs79412153 | 9 | 19658668 | SLC24A2 | intron_variant |
| rsl14378135 | 9 | 25102230 | NA | |
| rs10966802 | 9 | 25226973 | NA | |
| 9:25264525:G:T | 9 | 25264525 | NA | NA |
| rs143890288 | 9 | 25267126 | NA | |
| 9:25269360:G:GAG | 9 | 25269360 | NA | NA |
| rs138481346 | 9 | 27860943 | NA | |
| rs117835027 | 9 | 28108997 | LING02 | intron_variant |
| rs7034549 | 9 | 28109195 | LING02 | intron_variant |
| rs7034769 | 9 | 28109318 | LING02 | intron_variant |
| rs148234325 | 9 | 28109777 | NA | NA |
| rs9722119 | 9 | 28111224 | LING02 | intron_variant |
| rs189664295 | 9 | 28111978 | LING02 | intron_variant |
| rs147490749 | 9 | 28112009 | LING02 | intron_variant |
| 9:28117021:T:G | 9 | 28117021 | NA | NA |
| rs112776037 | 9 | 37038910 | NA | |
| rs76697541 | 9 | 37039286 | NA | |
| rs118107060 | 9 | 73107527 | NA | |
| rs11137993 | 9 | 81625189 | NA | |
| rs148742339 | 9 | 82896313 | NA | |
| rs148593924 | 9 | 85082296 | AL162726.3 | non_coding_transcript_variant |
| rs111776923 | 9 | 87283234 | NA | |
| rs111867627 | 9 | 87336518 | NTRK2 | intron_variant |
| rs113711779 | 9 | 87352036 | NTRK2 | intron_variant |
| rs11141920 | 9 | 90262011 | DAPK1 | intron_variant |
| rs2274607 | 9 | 90262393 | DAPK1 | intron_variant |
| rs145382400 | 9 | 90796382 | NA | |
| rs10991738 | 9 | 93757561 | NA | |

[Table 1-47]

| SNP ID | chr | position | gene locus | SNP location in gene locus |
|---|---|---|---|---|
| 9:93759544:GGTGT:GGTGTGT | 9 | 93759544 | NA | NA |
| rs71509677 | 9 | 93763449 | NA | |
| rs35931712 | 9 | 93769334 | NA | |
| rs35794334 | 9 | 93777994 | NA | |
| rs35246326 | 9 | 93789668 | NA | |
| rs72739474 | 9 | 93838589 | NA | |
| rs296636 | 9 | 93843942 | NA | |
| rs35748257 | 9 | 93848258 | NA | |
| rs55687842 | 9 | 93856705 | NA | |
| rs72739496 | 9 | 93857087 | NA | |
| rs11312435 | 9 | 93858207 | NA | |
| rs79287236 | 9 | 108886577 | NA | |
| rs116925051 | 9 | 110829415 | NA | |
| rs12337098 | 9 | 113513534 | MUSK | intron_variant |
| rs7043625 | 9 | 113516179 | MUSK | intron_variant |
| rs77542599 | 9 | 115702427 | NA | |
| rs11413824 | 9 | 118344670 | NA | |
| rs 72765920 | 9 | 124702107 | TTLL11 | NMD_transcript_variant |
| rs11999389 | 9 | 126872652 | NA | |
| rs58848387 | 9 | 128211958 | MAPKAP1 | intron_variant |
| 9:128723505:T:C | 9 | 128723505 | NA | NA |
| rs73582691 | 9 | 128752360 | NA | |
| rs118085053 | 9 | 139614734 | DIPK1B | intron_variant |
| rs17158762 | 10 | 2701823 | NA | |
| 10:5725303:C:T | 10 | 5725303 | NA | NA |
| rs4749996 | 10 | 10878872 | CELF2 | intron_variant |
| rs138294262 | 10 | 10891440 | CELF2 | intron_variant |
| 10:13274942:TA:T | 10 | 13274942 | NA | NA |
| rs41291315 | 10 | 13275437 | UCMA | intron_variant |
| rs116961903 | 10 | 14008637 | FRMD4A | intron_variant |
| rs139902130 | 10 | 14009189 | FRMD4A | intron_variant |
| 10:14017770:T:C | 10 | 14017770 | NA | NA |
| 10:14017770:T:TGC | 10 | 14017770 | NA | NA |
| rs1609477 | 10 | 14021383 | FRMD4A | intron_variant |
| rs11258773 | 10 | 14025006 | FRMD4A | intron_variant |

[Table 1-48]

| SNP ID | chr | position | gene locus | SNP location in gene locus |
|---|---|---|---|---|
| rs12572939 | 10 | 14030842 | FRMD4A | intron_variant |
| rs7096979 | 10 | 17539678 | NA | |
| 10:28077198:AT:A | 10 | 28077198 | NA | NA |
| rs351803 | 10 | 55317094 | NA | |
| rs351802 | 10 | 55317221 | NA | |
| rs351801 | 10 | 55317903 | NA | |
| rs351800 | 10 | 55317928 | NA | |
| rs351799 | 10 | 55317976 | NA | |
| rs2992011 | 10 | 55318012 | NA | |
| rs2931565 | 10 | 55318016 | NA | |
| rs6481015 | 10 | 55318082 | NA | |
| rs6481016 | 10 | 55318158 | NA | |
| rs6415918 | 10 | 55318166 | NA | |
| rs145658941 | 10 | 55318826 | NA | NA |
| rs689581 | 10 | 55318856 | NA | |
| rs35010388 | 10 | 55319128 | NA | |
| rs145725620 | 10 | 55319312 | NA | NA |
| rs394770 | 10 | 55319395 | NA | |
| rs149503551 | 10 | 55319419 | NA | NA |
| rs413137 | 10 | 55319542 | NA | |
| rs443931 | 10 | 55319592 | NA | |
| rs420314 | 10 | 55319617 | NA | |
| rs428563 | 10 | 55319634 | NA | |
| rs450516 | 10 | 55319865 | NA | |
| rs426290 | 10 | 55320087 | NA | |
| rs444728 | 10 | 55320115 | NA | |
| rs430495 | 10 | 55320281 | NA | |
| rs423146 | 10 | 55320430 | NA | |
| rs453158 | 10 | 55320437 | NA | |
| rs2484209 | 10 | 55320471 | NA | |
| rs668434 | 10 | 55320596 | NA | |
| rs668014 | 10 | 55320664 | NA | |
| rs451485 | 10 | 55320992 | NA | |
| rs396374 | 10 | 55321338 | NA | |
| rs423384 | 10 | 55321415 | NA | |
| rs409001 | 10 | 55321543 | NA | |

[Table 1-49]

| SNP ID | chr | position | gene locus | SNP location in gene locus |
|---|---|---|---|---|
| rs399606 | 10 | 55321588 | NA | |
| rs371543 | 10 | 55321684 | NA | |
| rs382870 | 10 | 55321728 | NA | |
| rs428467 | 10 | 55321781 | NA | |
| rs446990 | 10 | 55321931 | NA | |
| rs379026 | 10 | 55321948 | NA | |
| rs379033 | 10 | 55321950 | NA | |
| rs407646 | 10 | 55322061 | NA | |
| rs407337 | 10 | 55322216 | NA | |
| rs445488 | 10 | 55322557 | NA | |
| rs394942 | 10 | 55322731 | NA | |
| rs423806 | 10 | 55322780 | NA | |
| rs389244 | 10 | 55322918 | NA | |
| rs451607 | 10 | 55323157 | NA | |
| rs425467 | 10 | 55323180 | NA | |
| rs395825 | 10 | 55323468 | NA | |
| rs437390 | 10 | 55323572 | NA | |
| rs2484208 | 10 | 55323779 | NA | |
| rs2450530 | 10 | 55323807 | NA | |
| rs440743 | 10 | 55323832 | NA | |
| rs427506 | 10 | 55324048 | NA | |
| rs11527477 | 10 | 55324102 | NA | |
| rs11527478 | 10 | 55324117 | NA | |
| 10:55324128:A:G | 10 | 55324128 | NA | NA |
| 10:55324134:T:G | 10 | 55324134 | NA | NA |
| rs7075007 | 10 | 55324148 | NA | |
| rs11527272 | 10 | 55324150 | NA | |
| rs610251 | 10 | 55324303 | NA | |
| rs61859905 | 10 | 55324317 | NA | |
| rs609908 | 10 | 55324342 | NA | |
| rs663624 | 10 | 55324413 | NA | |
| rs455078 | 10 | 55324459 | NA | |
| rs374891 | 10 | 55324475 | NA | |
| 10:55324510:AC:A | 10 | 55324510 | NA | NA |
| 10:55324513:C:T | 10 | 55324513 | NA | NA |
| 10:55324514:A:G | 10 | 55324514 | NA | NA |

[Table 1-50]

| SNP ID | chr | position | gene locus | SNP location in gene locus |
|---|---|---|---|---|
| rs398169 | 10 | 55324751 | NA | |
| rs416275 | 10 | 55324754 | NA | |
| rs665850 | 10 | 55324915 | NA | |
| rs665881 | 10 | 55324942 | NA | |
| rs607276 | 10 | 55324943 | NA | |
| rs607228 | 10 | 55324976 | NA | |
| rs596456 | 10 | 55325084 | NA | |
| rs4456174 | 10 | 55325288 | NA | |
| rs678445 | 10 | 55325383 | NA | |
| rs678459 | 10 | 55325386 | NA | |
| rs399032 | 10 | 55325442 | NA | |
| 10:55325458:G:C | 10 | 55325458 | NA | NA |
| 10:55325459:G:T | 10 | 55325459 | NA | NA |
| 10:55325460:A:G | 10 | 55325460 | NA | NA |
| rs57606495 | 10 | 55325466 | NA | NA |
| rs34789289 | 10 | 55325562 | NA | |
| rs392668 | 10 | 55325629 | NA | |
| 10:55325657:T:TAA | 10 | 55325657 | NA | NA |
| rs451452 | 10 | 55325763 | NA | |
| rs401333 | 10 | 55325803 | NA | |
| rs381979 | 10 | 55325986 | NA | |
| rs364694 | 10 | 55326079 | NA | |
| rs428127 | 10 | 55326235 | NA | |
| rs376278 | 10 | 55326251 | NA | |
| rs417712 | 10 | 55326306 | NA | |
| rs59540423 | 10 | 55326321 | NA | NA |
| rs11003681 | 10 | 55326332 | NA | |
| rs11003682 | 10 | 55326343 | NA | |
| rs66668165 | 10 | 55326413 | NA | |
| rs61859906 | 10 | 55326428 | NA | |
| rs61859907 | 10 | 55326465 | NA | |
| rs61859908 | 10 | 55326470 | NA | |
| 10:55326554:T:TGA | 10 | 55326554 | NA | NA |
| rs34095257 | 10 | 55326663 | NA | |
| rs35891588 | 10 | 55326698 | NA | |
| rs34632242 | 10 | 55326715 | NA | |

[Table 1-51]

| SNP ID | chr | position | gene locus | SNP location in gene locus |
|---|---|---|---|---|
| rs35424889 | 10 | 55326737 | NA | |
| rs66678299 | 10 | 55326829 | NA | |
| 10:55326858 :GCAAAGAGTTCATGAC:G | 10 | 55326858 | NA | NA |
| 10:55326914:AGAG:A | 10 | 55326914 | NA | NA |
| rs7898607 | 10 | 55326930 | NA | |
| rs7898708 | 10 | 55326954 | NA | |
| rs598529 | 10 | 55327033 | NA | |
| rs598536 | 10 | 55327042 | NA | |
| 10:55327064:A:G | 10 | 55327064 | NA | NA |
| 10:55327072:GAA:G | 10 | 55327072 | NA | NA |
| rs674159 | 10 | 55327158 | NA | |
| rs409369 | 10 | 55327195 | NA | |
| rs55653225 | 10 | 55327405 | NA | |
| rs55712224 | 10 | 55327472 | NA | |
| rs418047 | 10 | 55327526 | NA | |
| 10:55327577:G:A | 10 | 55327577 | NA | NA |
| rs401407 | 10 | 55327799 | NA | |
| rs2632535 | 10 | 55327835 | NA | |
| rs637714 | 10 | 55327902 | NA | |
| rs1762109 | 10 | 55327985 | NA | |
| rs142344752 | 10 | 55328020 | NA | |
| rs659421 | 10 | 55328188 | NA | |
| rs636340 | 10 | 55328236 | NA | |
| rs659Q14 | 10 | 55328271 | NA | |
| 10:55328288:T:A | 10 | 55328288 | NA | NA |
| rs658983 | 10 | 55328294 | NA | |
| rs4628595 | 10 | 55328376 | NA | |
| rs392293 | 10 | 55328466 | NA | |
| rs454270 | 10 | 55328737 | NA | |
| rs414849 | 10 | 55328752 | NA | |
| rs365966 | 10 | 55328774 | NA | |
| rs404593 | 10 | 55328985 | NA | |
| rs409814 | 10 | 55328990 | NA | |
| 10:55329057:AC:A | 10 | 55329057 | NA | NA |
| rs368703 | 10 | 55329112 | NA | |

[Table 1-52]

| SNP ID | chr | position | gene locus | SNP location in gene locus |
|---|---|---|---|---|
| rs447549 | 10 | 55329255 | NA | |
| rs368110 | 10 | 55329416 | NA | |
| rs374009 | 10 | 55329487 | NA | |
| rs436403 | 10 | 55329513 | NA | |
| rs452019 | 10 | 55329523 | NA | |
| rs427201 | 10 | 55329632 | NA | |
| rs433582 | 10 | 55329954 | NA | |
| rs433574 | 10 | 55329961 | NA | |
| rs441336 | 10 | 55329964 | NA | |
| rs427372 | 10 | 55330380 | NA | |
| rs380989 | 10 | 55330434 | NA | |
| rs17695283 | 10 | 55332545 | NA | |
| rs444186 | 10 | 55334166 | NA | |
| rs10996833 | 10 | 67784976 | CTNNA3 | intron_variant |
| rs34539937 | 10 | 67787534 | CTNNA3 | intron_variant |
| rs35716649 | 10 | 67788443 | CTNNA3 | intron_variant |
| rs12571522 | 10 | 67790052 | CTNNA3 | intron_variant |
| rs35744226 | 10 | 67803006 | CTNNA3 | intron_variant |
| rs12778483 | 10 | 67805050 | CTNNA3 | intron_variant |
| rsl7243463 | 10 | 67805413 | CTNNA3 | intron_variant |
| rs75400168 | 10 | 67834004 | CTNNA3 | intron_variant |
| rs71493960 | 10 | 67835457 | CTNNA3 | intron_variant |
| rs35053158 | 10 | 67841299 | CTNNA3 | intron_variant |
| rs35376201 | 10 | 67842582 | CTNNA3 | intron_variant |
| rs67242129 | 10 | 67842799 | NA | NA |
| rs1247787 | 10 | 78830256 | KCNMA1 | intron_variant |
| rs1247779 | 10 | 78839034 | KCNMA1 | intron_variant |
| rs2258870 | 10 | 78840266 | KCNMA1 | intron_variant |
| rs10762747 | 10 | 78842860 | KCNMA1 | intron_variant |
| rs2574789 | 10 | 78851342 | KCNMA1 | intron_variant |
| rs74462085 | 10 | 78856298 | KCNMA1 | intron_variant |
| rs80058374 | 10 | 78859025 | KCNMA1 | intron_variant |
| rs79411761 | 10 | 78861868 | KCNMA1 | intron variant |
| rs2574791 | 10 | 78864094 | KCNMA1 | intron variant |
| rs2574787 | 10 | 78883564 | KCNMA1 | intron_variant |
| rs4980128 | 10 | 78886557 | KCNMA1 | intron_variant |

[Table 1-53]

| SNP ID | chr | position | gene locus | SNP location in gene locus |
|---|---|---|---|---|
| rs61574126 | 10 | 80740616 | NA | |
| rs60394423 | 10 | 80742406 | NA | |
| rs77451861 | 10 | 80743419 | NA | |
| rs116906077 | 10 | 80755918 | NA | |
| rs78685078 | 10 | 80755928 | NA | |
| rs117213013 | 10 | 81685744 | NA | |
| rs7089837 | 10 | 85433827 | NA | |
| 10:85434294:T:A | 10 | 85434294 | NA | NA |
| rs115787429 | 10 | 85436477 | NA | |
| rs17102402 | 10 | 85436920 | NA | |
| rs149846651 | 10 | 85437351 | NA | |
| rs74773350 | 10 | 99321878 | UBTD1 | intron_variant |
| 10:112855651:T:C | 10 | 112855651 | NA | NA |
| rs75665088 | 10 | 116038167 | VWA2 | intron_variant |
| rsl0736246 | 10 | 117982524 | GFRA1 | intron_variant |
| rs7094346 | 10 | 117986207 | GFRA1 | intron_variant |
| rs4628599 | 10 | 117986457 | GFRA1 | intron_variant |
| rs141923fi79 | 10 | 118354939 | PNLIPRP1 | intron_variant |
| rs59679890 | 10 | 118636461 | EN04 | intron_variant |
| rs72631124 | 10 | 125679317 | CPXM2 | intron_variant |
| rs72631125 | 10 | 125679694 | CPXM2 | intron_variant |
| rs72631126 | 10 | 125679714 | CPXM2 | intron_variant |
| rs72631127 | 10 | 125680048 | CPXM2 | intron_variant |
| rs72631128 | 10 | 125680075 | CPXM2 | intron_variant |
| rs182795662 | 10 | 125680804 | CPXM2 | intron_variant |
| rs186918206 | 10 | 125680805 | CPXM2 | intron_variant |
| rs72631129 | 10 | 125680924 | CPXM2 | intron_variant |
| rs72631130 | 10 | 125681159 | CPXM2 | intron_variant |
| 10:125681455:T:CC | 10 | 125681455 | NA | NA |
| rs1158419fi5 | 10 | 125681633 | CPXM2 | intron_variant |
| rs72631132 | 10 | 125682219 | CPXM2 | intron_variant |
| rs72631134 | 10 | 125682486 | CPXM2 | intron_variant |
| rs142639355 | 10 | 125682701 | CPXM2 | intron_variant |
| rs115903046 | 10 | 125682823 | CPXM2 | intron_variant |
| rs140301692 | 10 | 125682862 | CPXM2 | intron_variant |
| rs149963239 | 10 | 125727519 | NA | |

[Table 1-54]

| SNP ID | chr | position | gene locus | SNP location in gene locus |
|---|---|---|---|---|
| rs143323962 | 10 | 125728098 | NA | |
| rs11594441 | 10 | 130456079 | NA | |
| rs3847456 | 10 | 130457648 | NA | |
| rs3847457 | 10 | 130457732 | NA | |
| rs72847142 | 10 | 130458683 | NA | |
| rs72280102 | 10 | 130460388 | NA | |
| rs11598828 | 10 | 130463073 | NA | |
| rs11598671 | 10 | 130466623 | NA | |
| rs1475391 | 10 | 130474188 | NA | |
| rs112036204 | 10 | 130475046 | NA | |
| rs113908408 | 11 | 7845074 | NA | |
| rs112933444 | 11 | 7845341 | NA | |
| rs112043976 | 11 | 7845400 | NA | |
| rs73408430 | 11 | 7855420 | NA | |
| rs73408442 | 11 | 7857655 | NA | |
| rs73408450 | 11 | 7858446 | NA | |
| rs73408454 | 11 | 7859334 | NA | |
| rs79725644 | 11 | 7861967 | NA | |
| rs58454329 | 11 | 7864544 | NA | |
| rs369143 | 11 | 7865713 | NA | |
| rs57721290 | 11 | 7867024 | NA | |
| 11:7868688:AT:A | 11 | 7868688 | NA | NA |
| rs11606178 | 11 | 7872593 | NA | |
| rs77771703 | 11 | 7879250 | NA | |
| rs79195468 | 11 | 7897753 | NA | |
| 11:8797713:A:G | 11 | 8797713 | NA | NA |
| rs11042070 | 11 | 8819367 | DENND2B | intron_variant |
| rs142465660 | 11 | 8821504 | NA | NA |
| rs4929915 | 11 | 8824418 | DENND2B | intron_variant |
| rs17337866 | 11 | 8827750 | DENND2B | intron_variant |
| rs3915868 | 11 | 8833800 | DENND2B | intron_variant |
| rs34023999 | 11 | 8839379 | DENND2B | intron_variant |
| rs36094920 | 11 | 8845266 | DENND2B | intron_variant |
| rs7127197 | 11 | 8845607 | DENND2B | intron_variant |
| rs35503692 | 11 | 8848785 | DENND2B | intron_variant |
| rs34033747 | 11 | 8853774 | DENND2B | intron_variant |

[Table 1-55]

| SNP ID | chr position | gene locus | SNP location in gene locus |
|---|---|---|---|
| 11:8854132:A:ACTGCG | 11 8854132 | NA | NA |
| rs34418236 | 11 8859656 | DENND2B | intron_variant |
| rs77784346 | 11 8998278 | NA | |
| rs149624805 | 11 12188071 | MICAL2 | intron_variant |
| rs118093648 | 11 18162065 | NA | |
| rs7120135 | 11 24831117 | LUZP2 | intron_variant |
| rs61877058 | 11 26600213 | AN03 | intron_variant |
| rs61877059 | 11 26604143 | AN03 | intron_variant |
| 11:26605036:C:T | 11 26605036 | NA | NA |
| rsl7243461 | 11 26607661 | AN03 | intron_variant |
| rs192211136 | 11 26608276 | AN03 | intron_variant |
| rs17243468 | 11 26608776 | AN03 | intron_variant |
| 11:26610935:G:GA | 11 26610935 | NA | NA |
| rs61878565 | 11 26611476 | AN03 | intron_variant |
| rs61878566 | 11 26612275 | AN03 | intron_variant |
| rs61878567 | 11 26613230 | AN03 | intron_variant |
| rs61878568 | 11 26613870 | AN03 | intron_variant |
| rs61878572 | 11 26616967 | AN03 | intron_variant |
| rs61878588 | 11 26618785 | AN03 | intron_variant |
| rs61878589 | 11 26618960 | AN03 | intron_variant |
| rs200761473 | 11 26625591 | AN03 | intron variant |
| rs61878592 | 11 26625601 | AN03 | intron_variant |
| rs36063975 | 11 35574891 | NA | |
| rs12798613 | 11 35574908 | NA | |
| rs10836415 | 11 35575254 | NA | |
| rs59100957 | 11 35575495 | NA | |
| rs12420268 | 11 35576512 | NA | |
| rs144733009 | 11 44566820 | NA | |
| rs2045018 | 11 44843041 | TSPAN18 | intron_variant |
| rs11230471 | 11 60539384 | MS4A15 | intron_variant |
| rs142518783 | 11 62237951 | AHNAK | intron_variant |
| rs12281345 | 11 62773213 | SLC22A8 | intron_variant |
| rs12808517 | 11 72601276 | FCHSD2 | intron_variant |
| rs150783164 | 11 74983240 | ARRB1 | intron_variant |
| rs78089535 | 11 79215837 | NA | |
| rs112798455 | 11 79216359 | NA | |

[Table 1-56]

| SNP ID | chr | position | gene locus | SNP location in gene locus |
|---|---|---|---|---|
| 11:79243641:AT:A | 11 | 79243641 | NA | NA |
| rs74659625 | 11 | 79250136 | NA | |
| rs76280090 | 11 | 79250154 | NA | |
| rs147247218 | 11 | 84483667 | DLG2 | intron_variant |
| rs139305706 | 11 | 89858108 | NA | |
| rs149012812 | 11 | 90175103 | NA | |
| rs17860938 | 11 | 102652744 | WTAPP1 | non_coding_transcript_variant |
| rs72977553 | 11 | 102653834 | WTAPP1 | non_coding_transcript_variant |
| rs7112080 | 11 | 102655053 | WTAPP1 | non_coding_transcript_variant |
| rs7933558 | 11 | 102655137 | WTAPP1 | non_coding_transcript_variant |
| rs72977568 | 11 | 102657657 | WTAPP1 | non_coding_transcript_variant |
| rs187059006 | 11 | 102658035 | WTAPP1 | non_coding_transcript_variant |
| rs11390663 | 11 | 102660054 | WTAPP1 | non_coding_transcript_variant |
| rs7945189 | 11 | 102660564 | WTAPP1 | non_coding_transcript_variant |
| rs17878905 | 11 | 102661757 | MMP1 | intron_variant |
| rs17882844 | 11 | 102661813 | MMP1 | intron variant |
| rs146555295 | 11 | 105747176 | NA | NA |
| rs12419614 | 11 | 105840184 | GRIA4 | intron_variant |
| rs111988538 | 11 | 107103114 | NA | |
| rs78496161 | 11 | 107148735 | NA | |
| 11:107151031:A:AT | 11 | 107151031 | NA | NA |
| rs79062526 | 11 | 107153318 | NA | |
| rs76404176 | 11 | 107157966 | NA | |
| rs17106711 | 11 | 107158740 | NA | |
| rs7943234 | 11 | 107165710 | NA | |
| rs78568149 | 11 | 107222059 | CWF19L2 | intron_variant |
| rs74346769 | 11 | 107226643 | CWF19L2 | intron_variant |
| rs118053355 | 11 | 107328818 | NA | |
| rs17107015 | 11 | 107353313 | NA | |
| rs7937106 | 11 | 107354312 | NA | |
| rs80050705 | 11 | 107354858 | NA | |
| rs77739772 | 11 | 107356982 | NA | |
| rs201645325 | 11 | 107357860 | NA | NA |
| rs78482726 | 11 | 107362733 | NA | |
| rs149277905 | 11 | 107364050 | NA | |
| rs12417562 | 11 | 107364413 | NA | |

[Table 1-57]

| SNP ID | chr | position | gene locus | SNP location in gene locus |
|---|---|---|---|---|
| rs115051850 | 11 | 117280512 | CEP164 | synonymous_variant |
| rs61743854 | 11 | 117280517 | CEP164 | missense_variant |
| rs111910740 | 11 | 119625360 | NA | |
| rs140081101 | 11 | 119625819 | NA | |
| rs148275870 | 11 | 119630952 | NA | |
| rs115832414 | 11 | 119656930 | NA | |
| rs146686876 | 11 | 119659763 | NA | |
| rs117150747 | 11 | 126994608 | NA | |
| rs78730731 | 11 | 127031263 | NA | |
| rs1944828 | 11 | 127048359 | NA | |
| rs2513442 | 11 | 127053597 | NA | |
| 12:7859133:G:A | 12 | 7859133 | NA | NA |
| rs17834986 | 12 | 15261437 | RERG | 3_prime_UTR_variant |
| rs1116413fi8 | 12 | 26800096 | ITPR2 | intron variant |
| rs140725931 | 12 | 26802734 | ITPR2 | intron_variant |
| rs112815221 | 12 | 26829320 | ITPR2 | intron_variant |
| rs74849908 | 12 | 26836556 | ITPR2 | intron_variant |
| rs76630621 | 12 | 26841931 | ITPR2 | intron_variant |
| 12:28124654:A:G | 12 | 28124654 | NA | NA |
| rs61918684 | 12 | 29635132 | OVCH1 | intron_variant |
| rs61922198 | 12 | 29779895 | TMTC1 | non_coding_transcript_variant |
| rs11050326 | 12 | 29782241 | TMTC1 | non_coding_transcript_variant |
| rs12300501 | 12 | 29782247 | TMTC1 | non_coding_transcript_variant |
| rs11050329 | 12 | 29786413 | TMTC1 | non_coding_transcript_variant |
| 12:29788125:C:CA | 12 | 29788125 | NA | NA |
| rs10771557 | 12 | 29788245 | TMTC1 | non_coding_transcript_variant |
| rs11050333 | 12 | 29789329 | TMTC1 | non_coding_transcript_variant |
| rs10843455 | 12 | 29789750 | TMTC1 | non_coding_transcript_variant |
| rs10843457 | 12 | 29790399 | TMTC1 | non_coding_transcript_variant |
| rs11050334 | 12 | 29790982 | TMTC1 | non_coding_transcript_variant |
| rs12827042 | 12 | 29791219 | TMTC1 | non_coding_transcript_variant |
| rs12826106 | 12 | 29791229 | TMTC1 | non_coding_transcript_variant |
| rs1972681 | 12 | 29791455 | TMTC1 | non_coding_transcript_variant |
| rs1972682 | 12 | 29791633 | TMTC1 | non_coding_transcript_variant |
| rs1549408 | 12 | 29791646 | TMTC1 | non_coding_transcript_variant |
| rs1549409 | 12 | 29791672 | TMTC1 | non_coding_transcript_variant |

[Table 1-58]

| SNP ID | ch r | position | gene locus | SNP location in gene locus |
|---|---|---|---|---|
| rs1549410 | 12 | 29791697 | TMTC1 | non_coding_transcript_variant |
| rs10843458 | 12 | 29791936 | TMTC1 | non_coding_transcript_variant |
| rs7955768 | 12 | 29792069 | TMTC1 | non_coding_transcript_variant |
| rs10771558 | 12 | 29792233 | TMTC1 | non_coding_transcript_variant |
| rs34265384 | 12 | 29792497 | TMTC1 | non_coding_transcript_variant |
| rs1549411 | 12 | 29793140 | TMTC1 | non_coding_transcript_variant |
| rs113386809 | 12 | 29794462 | TMTC1 | non_coding_transcript_variant |
| rs113094413 | 12 | 29795584 | TMTC1 | non_coding_transcript_variant |
| rs113767075 | 12 | 29795807 | TMTC1 | non_coding_transcript_variant |
| rs12371181 | 12 | 31193873 | NA | |
| rs150392011 | 12 | 31198640 | NA | |
| rs11051218 | 12 | 31201717 | NA | |
| rs11051219 | 12 | 31201922 | NA | |
| rs11051221 | 12 | 31203590 | NA | |
| rs11051222 | 12 | 31203920 | NA | |
| rs4244855 | 12 | 31213686 | NA | |
| rs4931415 | 12 | 31216523 | NA | |
| rs4931416 | 12 | 31219161 | NA | |
| rs145929359 | 12 | 31222582 | NA | |
| 12:31225561:G :GTTAGTGAGA | 12 | 31225561 | NA | NA |
| rs2302846 | 12 | 31226930 | DDX11 | 5_prime_UTR_variant |
| rs12579271 | 12 | 31230735 | DDX11 | intron_variant |
| rs11832720 | 12 | 57257244 | AC121758. 2 | intron variant |
| rs75465620 | 12 | 57259691 | AC121758. 2 | intron_variant |
| rs144935800 | 12 | 57263384 | NA | NA |
| rs117991841 | 12 | 57263617 | AC121758. 2 | intron_variant |
| rs74995587 | 12 | 57264426 | AC121758.2 | intron_variant |
| rs4403833 | 12 | 57265921 | AC121758.2 | non_cod i ng_transcr i pt_exon_var i ant |
| rs12810024 | 12 | 57331444 | NA | |
| rs200182685 | 12 | 57333170 | NA | |
| 12:57335432:A:ATTTA | 12 | 57335432 | NA | NA |
| 12:57335432:A:ATTTT | 12 | 57335432 | NA | NA |
| rs143723418 | 12 | 57339182 | NA | |
| rs1072670 | 12 | 57342100 | NA | |
| rs11836517 | 12 | 57343892 | NA | |

[Table 1-59]

| SNP ID | chr | position | gene locus | SNP location in gene locus |
|---|---|---|---|---|
| rs901068 | 12 | 57346805 | RDH16 | intron_variant |
| rs746049 | 12 | 57349039 | RDH16 | intron_variant |
| rs67771054 | 12 | 57349813 | RDH16 | intron_variant |
| rs12815538 | 12 | 57352871 | RDH16 | non_coding_transcriptexon_variant |
| rs144844809 | 12 | 57353192 | NA | NA |
| rs138278919 | 12 | 57353274 | NA | |
| rs148154364 | 12 | 57356239 | NA | NA |
| rs143470873 | 12 | 57356240 | NA | NA |
| rs34253098 | 12 | 57356325 | NA | |
| 12:57356830:A:G | 12 | 57356830 | NA | NA |
| rs12820005 | 12 | 57356885 | NA | |
| rs11837093 | 12 | 57356893 | NA | |
| rs79861271 | 12 | 57357106 | NA | |
| 12:57357322:A:G | 12 | 57357322 | NA | NA |
| rs181056553 | 12 | 57357512 | NA | |
| rs77781354 | 12 | 57357721 | NA | |
| 12:57357983:G:A | 12 | 57357983 | NA | NA |
| 12:57358181:C:G | 12 | 57358181 | NA | NA |
| rs703818 | 12 | 57359073 | NA | |
| 12:57359261:C:CA | 12 | 57359261 | NA | NA |
| rs2888139 | 12 | 57359436 | NA | |
| rs12812499 | 12 | 57359643 | NA | |
| rs144223544 | 12 | 57360141 | NA | |
| rs60380217 | 12 | 57360851 | NA | |
| rs1843311 | 12 | 57361414 | NA | |
| rs1843312 | 12 | 57361562 | NA | |
| rs1843313 | 12 | 57361594 | NA | |
| rs2167306 | 12 | 57362345 | NA | |
| rs7969312 | 12 | 57362968 | NA | |
| rs7956166 | 12 | 57363128 | NA | |
| rs7487948 | 12 | 57363907 | NA | |
| rs149233794 | 12 | 57364288 | NA | NA |
| rs12827286 | 12 | 57365564 | NA | |
| 12:57365771:A:AAC | 12 | 57365771 | NA | NA |
| rs145602952 | 12 | 59622709 | NA | |
| rs142940655 | 12 | 59629178 | NA | |

[Table 1-60]

| SNP ID | chr | position | gene locus | SNP location in gene locus |
|---|---|---|---|---|
| rs76598008 | 12 | 59709510 | NA | |
| rs75811180 | 12 | 59825922 | NA | |
| rs144524094 | 12 | 64412328 | SRGAP1 | intron_variant |
| rs75779275 | 12 | 66668726 | NA | |
| rs11176136 | 12 | 66700104 | HELB | intron_variant |
| rs7976871 | 12 | 69125513 | NUP107 | intron_variant |
| rs7976876 | 12 | 69125514 | NUP107 | intron_variant |
| rs7976687 | 12 | 69125578 | NUP107 | intron_variant |
| rs10878865 | 12 | 69149478 | SLC35E3 | NMD_transcript_variant |
| rs3730603 | 12 | 69222379 | MDM2 | intron_variant |
| rs146370968 | 12 | 69223034 | MDM2 | intron_variant |
| rs3730617 | 12 | 69224892 | MDM2 | intron variant |
| rs71454208 | 12 | 69226396 | MDM2 | intron_variant |
| rs3730635 | 12 | 69229123 | MDM2 | intron_variant |
| rs3730652 | 12 | 69232793 | MDM2 | intron_variant |
| rs769412 | 12 | 69233215 | MDM2 | synonymous_variant |
| rs11296638 | 12 | 69238437 | MDM2 | 3_prime_UTR_variant |
| rs7956669 | 12 | 69238507 | MDM2 | 3_prime_UTR_variant |
| rs78497497 | 12 | 94965816 | TMCC3 | intron_variant |
| rs142944124 | 12 | 99162625 | ANKS1B | intron_variant |
| rs143208630 | 12 | 99174638 | ANKS1B | intron_variant |
| rs10507253 | 12 | 115218522 | NA | |
| rs10850384 | 12 | 115218755 | NA | |
| rs11608897 | 12 | 115219079 | NA | |
| rs144088350 | 12 | 115219743 | NA | |
| rs118030982 | 12 | 118166151 | TAOK3 | intron_variant |
| 12:118170780:A:AAC | 12 | 118770780 | NA | NA |
| 12:118770780:A :AACACACACACACAC | 12 | 118770780 | NA | NA |
| 12:125161441:T:C | 12 | 125161441 | NA | NA |
| rs11058227 | 12 | 126018404 | TMEM132B | intron_variant |
| rs146061449 | 13 | 31045629 | HMGB1 | intron_variant |
| rs9541338 | 13 | 35177997 | NA | |
| rs9599308 | 13 | 35178625 | NA | |
| rs9592551 | 13 | 35179080 | NA | |
| rs7400628 | 13 | 38475425 | NA | |

[Table 1-61]

| SNP ID | chr | position | gene locus | SNP location in gene locus |
|---|---|---|---|---|
| rs4941879 | 13 | 38482388 | NA | |
| rs9603278 | 13 | 38484455 | NA | |
| rs77525380 | 13 | 38486498 | NA | |
| rs11620108 | 13 | 38523618 | NA | |
| rs75300170 | 13 | 38527975 | NA | |
| rs10161861 | 13 | 38533329 | NA | |
| rs140158226 | 13 | 38545248 | NA | |
| rs11619292 | 13 | 38545843 | NA | |
| rs113982010 | 13 | 38582994 | NA | |
| rs9603302 | 13 | 38585865 | NA | |
| rs7999781 | 13 | 38593133 | NA | |
| rs139272125 | 13 | 61294761 | NA | |
| rs140760839 | 13 | 61350501 | NA | |
| rs79016205 | 13 | 65322102 | NA | |
| rs9317907 | 13 | 70792954 | NA | |
| rs118058117 | 13 | 71328142 | NA | |
| rs9599962 | 13 | 73002308 | NA | |
| rs184865348 | 13 | 73049971 | NA | |
| rs117639698 | 13 | 73077947 | NA | |
| rs35985160 | 13 | 75124959 | NA | |
| rs76467960 | 13 | 78194746 | SCEL | intron_variant |
| rs75814545 | 13 | 78196291 | SCEL | intron variant |
| 13:78211558:T:C | 13 | 78211558 | NA | NA |
| rs9593249 | 13 | 78229617 | NA | |
| rs9544568 | 13 | 78242133 | NA | |
| rs186924328 | 13 | 88085503 | NA | |
| rs184461986 | 13 | 88122173 | NA | |
| rs184036706 | 13 | 88124358 | NA | |
| rs143514766 | 13 | 88150782 | NA | |
| rs145616607 | 13 | 88175773 | NA | |
| rs146077205 | 13 | 88183851 | NA | |
| rs115011805 | 13 | 88183871 | NA | |
| rs149371095 | 13 | 88186758 | NA | |
| rs141046761 | 13 | 88188257 | NA | |
| rs7990951 | 13 | 95592144 | NA | |
| rs4773831 | 13 | 95595122 | NA | |

[Table 1-62]

| SNP ID | chr | position | gene locus | SNP location in gene locus |
|---|---|---|---|---|
| rs7991523 | 13 | 95602158 | NA | |
| rs8002051 | 13 | 95606804 | NA | |
| rs9524705 | 13 | 95609326 | NA | |
| rs7998757 | 13 | 95610131 | NA | |
| rs192331957 | 13 | 101175688 | PCCA | intron_variant |
| rs1408046 | 13 | 103484653 | BIVM-ERCC5 | intron_variant |
| rs76029744 | 13 | 103486018 | BIVM-ERCC5 | intron_variant |
| rs7327832 | 13 | 103499900 | BIVM-ERCC5 | intron_variant |
| rs6491714 | 13 | 103500458 | BIVM-ERCC5 | intron_variant |
| rs75982504 | 13 | 103501928 | BIVM-ERCC5 | intron_variant |
| rs7328951 | 13 | 103502204 | BIVM-ERCC5 | intron_variant |
| rs7333684 | 13 | 103502332 | BIVM-ERCC5 | intron_variant |
| rs2104301 | 13 | 103502553 | BIVM-ERCC5 | intron_variant |
| rs4150263 | 13 | 103504344 | BIVM-ERCC5 | intron_variant |
| rs4150278 | 13 | 103507753 | BIVM-ERCC5 | intron_variant |
| rs4150281 | 13 | 103508095 | BIVM-ERCC5 | intron_variant |
| rs4150287 | 13 | 103509132 | BIVM-ERCC5 | intron_variant |
| rs12854564 | 13 | 103976233 | NA | |
| rs75174938 | 13 | 108015726 | FAM155A | intron_variant |
| rs9520931 | 13 | 109193709 | NA | |
| rs3825469 | 13 | 110881627 | COL4A1 | intron_variant |
| 13:113504421:A:G | 13 | 113504421 | NA | NA |
| 13:113505107:A:G | 13 | 113505107 | NA | NA |
| rs12877285 | 13 | 113519561 | ATP11A | intron_variant |
| 13:113679279:G:A | 13 | 113679279 | NA | NA |
| 13:113687158:C:T | 13 | 113687158 | NA | NA |
| rs117419951 | 13 | 114211421 | NA | |
| rs182523160 | 13 | 114214047 | NA | |
| rs78785829 | 14 | 29770720 | NA | |
| rs79415577 | 14 | 29823747 | NA | |
| rs9788541 | 14 | 29841230 | NA | |
| rs10141779 | 14 | 46320098 | NA | |
| rs10130975 | 14 | 46320102 | NA | |
| rs10142107 | 14 | 46320162 | NA | |
| rs17116552 | 14 | 46322340 | NA | |
| rs145926281 | 14 | 47920866 | MDGA2 | intron_variant |

[Table 1-63]

| SNP ID | chr | position | gene locus | SNP location in gene locus |
|---|---|---|---|---|
| rs74506457 | 14 | 48138790 | MDGA2 | intron_variant |
| rs140552479 | 14 | 48452990 | NA | |
| rs4483778 | 14 | 62669733 | NA | |
| rs74055659 | 14 | 62672299 | NA | |
| rs74055661 | 14 | 62674106 | NA | |
| rs76079973 | 14 | 62674412 | NA | |
| rs60686625 | 14 | 62675734 | NA | |
| rs4902111 | 14 | 62679573 | NA | |
| rs72476707 | 14 | 62679925 | NA | |
| rs72476708 | 14 | 62679941 | NA | |
| rs74055665 | 14 | 62683062 | NA | |
| rs74055666 | 14 | 62683158 | NA | |
| rs11622218 | 14 | 62684550 | NA | |
| rs74055670 | 14 | 62695028 | NA | |
| rs200453336 | 14 | 62696350 | NA | |
| rs28793052 | 14 | 62697293 | NA | |
| rs2090184 | 14 | 62698275 | NA | |
| rs2365677 | 14 | 78681121 | NRXN3 | intron_variant |
| rs142079680 | 14 | 84269688 | NA | |
| rs77476904 | 14 | 85191547 | NA | |
| rs74344396 | 14 | 85195062 | NA | |
| rs12435636 | 14 | 85195464 | NA | |
| rs1076961 | 14 | 85203036 | NA | |
| rs74890854 | 14 | 88800193 | NA | |
| rs78906101 | 14 | 89689421 | FOXN3 | intron_variant |
| rs113893721 | 14 | 92365125 | FBLN5 | intron_variant |
| rs1861086 | 14 | 92371328 | FBLN5 | intron_variant |
| rs72702992 | 14 | 101655820 | NA | |
| rs61994508 | 14 | 101656254 | NA | |
| rs72702993 | 14 | 101656819 | NA | |
| rs72702996 | 14 | 101657080 | NA | |
| rs56907901 | 14 | 101657647 | NA | |
| rs72702997 | 14 | 101659182 | NA | |
| rs7147137 | 14 | 101678763 | NA | |
| rs35480234 | 15 | 31093529 | NA | |
| rs7163291 | 15 | 31093881 | NA | |

[Table 1-64]

| SNP ID | chr | position | gene locus | SNP location in gene locus |
|---|---|---|---|---|
| rs35579443 | 15 | 31095933 | NA | |
| rs35622207 | 15 | 31097419 | NA | |
| rs11853322 | 15 | 31097795 | NA | |
| rs6493297 | 15 | 31099054 | NA | |
| rs71399735 | 15 | 31099728 | NA | |
| rs57386153 | 15 | 31101060 | NA | |
| rs3933429 | 15 | 31101915 | NA | |
| rs28584365 | 15 | 31103814 | NA | |
| rs10557815 | 15 | 31244352 | MTMR10 | intron_variant |
| rs147616718 | 15 | 31253523 | MTMR10 | intron_variant |
| rs76467407 | 15 | 31255220 | MTMR10 | intron_variant |
| rs12898290 | 15 | 31294343 | TRPM1 | missense_variant |
| rs3784589 | 15 | 31294714 | TRPM1 | stop_gained |
| rs10162919 | 15 | 31295788 | TRPM1 | intron_variant |
| rs7182547 | 15 | 31297672 | TRPM1 | intron_variant |
| rs7161796 | 15 | 31298145 | TRPM1 | intron_variant |
| rs7167074 | 15 | 31298352 | TRPM1 | intron_variant |
| rs7182571 | 15 | 31298442 | TRPM1 | intron_variant |
| rs7166214 | 15 | 31298483 | TRPM1 | intron_variant |
| rs35616506 | 15 | 31298737 | TRPM1 | intron_variant |
| rs143969953 | 15 | 31299528 | TRPM1 | intron_variant |
| 15:31300031:C:CATTT | 15 | 31300031 | NA | NA |
| rs61997145 | 15 | 31301159 | TRPM1 | intron_variant |
| rs12913087 | 15 | 31304551 | TRPM1 | intron_variant |
| rs201396966 | 15 | 31305173 | TRPM1 | intron_variant |
| rs35891405 | 15 | 31307063 | TRPM1 | intron_variant |
| 15:31309197:A:C | 15 | 31309197 | NA | NA |
| rs12911930 | 15 | 31309225 | TRPM1 | intron_variant |
| rs12898815 | 15 | 31309468 | TRPM1 | intron_variant |
| rs35208656 | 15 | 31310534 | TRPM1 | intron_variant |
| rs71420543 | 15 | 31312193 | NA | NA |
| rs12915504 | 15 | 31312619 | TRPM1 | intron_variant |
| rs12902573 | 15 | 31314317 | TRPM1 | intron_variant |
| rs151309116 | 15 | 33681236 | RYR3 | intron_variant |
| rs148846874 | 15 | 33899434 | RYR3 | intron_variant |
| rs12905712 | 15 | 37264445 | MEIS2 | intron_variant |

[Table 1-65]

| SNP ID | chr | position | gene locus | SNP location in gene locus |
|---|---|---|---|---|
| rs1194770 | 15 | 37268081 | MEIS2 | intron_variant |
| rs80351865 | 15 | 40161155 | GPR176 | intron_variant |
| rs138955007 | 15 | 55865286 | PYG01 | intron_variant |
| rs77212634 | 15 | 55875096 | PYG01 | intron_variant |
| rs72738039 | 15 | 57687411 | CGNL1 | non_coding_transcript_variant |
| rs73414637 | 15 | 62288216 | VPS13C | intron_variant |
| rs78465010 | 15 | 62313069 | VPS13C | intron_variant |
| rs67566652 | 15 | 64125992 | HERC1 | intron variant |
| rs9972484 | 15 | 64127038 | NA | |
| rs9972527 | 15 | 64127531 | NA | |
| rs7169537 | 15 | 64129914 | NA | |
| rs12148623 | 15 | 64131474 | NA | |
| rs2120252 | 15 | 64136472 | NA | |
| rs116914345 | 15 | 66971210 | NA | |
| rs190172459 | 15 | 78702652 | NA | |
| rs144928882 | 15 | 78702663 | NA | |
| rs181427460 | 15 | 78702665 | NA | |
| rs77156427 | 15 | 78703944 | NA | |
| rs76061647 | 15 | 78704580 | NA | |
| rs76765649 | 15 | 78705102 | NA | |
| rs11637161 | 15 | 78706391 | NA | |
| rs79961164 | 15 | 78707978 | NA | |
| rs80225953 | 15 | 78708302 | NA | |
| rs77826529 | 15 | 78708555 | NA | |
| rs72738490 | 15 | 80424475 | ZFAND6 | intron_variant |
| rs12905402 | 15 | 80516383 | CTXND1 | intron_variant |
| rs35971717 | 15 | 80517610 | CTXND1 | intron_variant |
| 15:80517776:T:TC | 15 | 80517776 | NA | NA |
| rs71399403 | 15 | 80517919 | CTXND1 | intron_variant |
| rs34765237 | 15 | 80520275 | CTXND1 | intron_variant |
| rs80244168 | 15 | 80520961 | CTXND1 | intron_variant |
| rs28621634 | 15 | 80521264 | CTXND1 | intron_variant |
| rs142665219 | 15 | 82201101 | NA | |
| rs138527956 | 15 | 82208029 | NA | |
| rs146297278 | 15 | 82224258 | NA | |
| rs74028454 | 15 | 82275400 | NA | |

[Table 1-66]

| SNP ID | chr | position | gene locus | SNP location in gene locus |
|---|---|---|---|---|
| rs141857089 | 15 | 82275840 | NA | |
| 15:82290701:T:A | 15 | 82290701 | NA | NA |
| rs74028477 | 15 | 82298644 | NA | |
| rs72746755 | 15 | 85851875 | ADAMTS7P4 | non_coding_transcript_variant |
| rs4284612 | 15 | 85880568 | NA | |
| rs74039987 | 15 | 101405738 | NA | |
| rs141389573 | 15 | 101715258 | NA | |
| rs76076521 | 16 | 11530047 | AC099489.1 | intron_variant |
| rs113344842 | 16 | 11697890 | LITAF | intron_variant |
| 16:29011862:C:T | 16 | 29011862 | NA | NA |
| rs147914226 | 16 | 29114347 | AC009093.9 | non_coding_transcript_variant |
| rs117889226 | 16 | 29115569 | NA | NA |
| rs117272179 | 16 | 29222277 | AC009093.8 | non_coding_transcript_variant |
| rs147323464 | 16 | 29294541 | NA | |
| rs143769600 | 16 | 29332310 | AC025279.2 | non_coding_transcript_exon_variant |
| 16:55795832:G:T | 16 | 55795832 | NA | NA |
| rs79304792 | 16 | 57532336 | NA | |
| rs8052995 | 16 | 60372574 | NA | |
| rs935754 | 16 | 60377491 | NA | |
| 16:71153772:G:A | 16 | 71153772 | NA | NA |
| rs4888922 | 16 | 79121475 | WWOX | intron_variant |
| rs57113334 | 16 | 80150523 | NA | |
| rs114992408 | 16 | 82564106 | NA | |
| rs111336258 | 16 | 82565313 | NA | |
| rs12448746 | 16 | 84495825 | ATP2C2 | intron_variant |
| rs12149961 | 16 | 85133324 | FAM92B | intron_variant |
| rs113597486 | 16 | 85135378 | FAM92B | intron_variant |
| rs75760369 | 16 | 85136066 | FAM92B | intron_variant |
| rs150351869 | 16 | 85136199 | FAM92B | intron_variant |
| rs77654959 | 16 | 85136253 | FAM92B | intron_variant |
| 16:85185004:G:A | 16 | 85185004 | NA | NA |
| rs142376288 | 16 | 85194913 | NA | |
| rs143768726 | 17 | 6245605 | NA | |
| rs116884024 | 17 | 7444424 | NA | |
| rs11655029 | 17 | 17649172 | RAI1 | intron_variant |
| rs9900803 | 17 | 17650978 | RAI1 | intron_variant |

[Table 1-67]

| SNP ID | chr | position | gene locus | SNP location in gene locus |
|---|---|---|---|---|
| rs11656775 | 17 | 17654319 | RAI1 | intron_variant |
| rs941448 | 17 | 17654542 | RAI1 | intron_variant |
| rs4925109 | 17 | 17661802 | RAI1 | intron_variant |
| 17:17666365:G:GA | 17 | 17666365 | NA | NA |
| 17:17678848:T :TAACAGACATTTTGTA | 17 | 17678848 | NA | NA |
| rs8072510 | 17 | 33772658 | SLFN13 | stop_gained |
| rs11652363 | 17 | 33774675 | SLFN13 | intron_variant |
| rs145674942 | 17 | 33775604 | SLFN13 | intron_variant |
| rs75361910 | 17 | 33793208 | SLFN12L | intron_variant |
| rs78856173 | 17 | 33805956 | SLFN12L | intron_variant |
| 17:64650539:C:GAG | 17 | 64650539 | NA | NA |
| 17:64650539:C:CAT | 17 | 64650539 | NA | NA |
| rs138440170 | 17 | 71704366 | NA | |
| rs78274099 | 17 | 77275723 | RBFOX3 | intron_variant |
| rs9675480 | 18 | 1444697 | NA | |
| rs73365235 | 18 | 1450563 | NA | |
| rs2160960 | 18 | 1453341 | NA | |
| rs143955284 | 18 | 1453688 | NA | NA |
| rs57847079 | 18 | 1453700 | NA | |
| rs78812196 | 18 | 1459687 | NA | |
| rs75905563 | 18 | 1460889 | NA | |
| 18:1460930:C:G | 18 | 1460930 | NA | NA |
| rs16940518 | 18 | 1460930 | NA | |
| rs73365261 | 18 | 1463910 | NA | |
| rs72634385 | 18 | 3225679 | NA | |
| rs55762828 | 18 | 3242809 | NA | |
| rs1662349 | 18 | 3246289 | NA | |
| rs7235642 | 18 | 4454027 | DLGAP1 | intron_variant |
| rs7236361 | 18 | 4454161 | DLGAP1 | intron_variant |
| rs151109374 | 18 | 4575611 | NA | |
| rs56830227 | 18 | 9699877 | NA | |
| 18:11624174:G:C | 18 | 11624174 | NA | NA |
| 18:19415747:T:A | 18 | 19415747 | NA | NA |
| rs1944288 | 18 | 25749095 | CDH2 | intron_variant |
| rs2191636 | 18 | 25749888 | CDH2 | intron_variant |

[Table 1-68]

| SNP ID | chr | position | gene locus | SNP location in gene locus |
|---|---|---|---|---|
| rs11661487 | 18 | 25750950 | CDH2 | intron_variant |
| rs11874478 | 18 | 25751637 | CDH2 | intron_variant |
| rs75095140 | 18 | 28690670 | NA | |
| rs77485878 | 18 | 28713843 | DSC1 | intron_variant |
| rs117854132 | 18 | 32857590 | ZSCAN30 | intron_variant |
| rs75504094 | 18 | 32926349 | NA | |
| rs72893124 | 18 | 35136494 | CELF4 | intron_variant |
| rs72893132 | 18 | 35138693 | CELF4 | intron_variant |
| rs150664883 | 18 | 35146206 | NA | |
| rs55996300 | 18 | 35158843 | NA | |
| rs72893187 | 18 | 35172187 | NA | |
| rs72895006 | 18 | 35189638 | NA | |
| rs55778135 | 18 | 35192829 | NA | |
| rs111869452 | 18 | 37930904 | NA | NA |
| rs111697888 | 18 | 49777775 | NA | |
| rs113445640 | 18 | 49787377 | NA | |
| rs112197694 | 18 | 49792926 | NA | |
| rs62081521 | 18 | 49793572 | NA | |
| rs73445368 | 18 | 49794194 | NA | |
| rs62081522 | 18 | 49796696 | NA | |
| rs73445370 | 18 | 49796944 | NA | |
| rs62081545 | 18 | 49803581 | NA | |
| rs62081546 | 18 | 49805925 | NA | |
| rs145744015 | 18 | 49808004 | NA | NA |
| rs62081550 | 18 | 49810293 | NA | |
| rs7240258 | 18 | 49812967 | NA | |
| rs62081552 | 18 | 49813508 | NA | |
| rs62081553 | 18 | 49816209 | NA | |
| rs62081554 | 18 | 49816348 | NA | |
| rs8097353 | 18 | 49816961 | NA | |
| rs62081555 | 18 | 49817639 | NA | |
| rs28592006 | 18 | 50295649 | DCC | intron_variant |
| rs149621133 | 18 | 50299840 | DCC | intron_variant |
| rs114489043 | 18 | 50301355 | DCC | intron_variant |
| rs74671490 | 18 | 50301411 | DCC | intron_variant |
| rs74600925 | 18 | 50301425 | DCC | intron_variant |

[Table 1-69]

| SNP ID | chr | position | gene locus | SNP location in gene locus |
|---|---|---|---|---|
| rs78939599 | 18 | 50301601 | DCC | intron_variant |
| rs75368394 | 18 | 50301737 | DCC | intron_variant |
| rs113902371 | 18 | 50301765 | DCC | intron_variant |
| rs9954223 | 18 | 50301788 | DCC | intron_variant |
| rs9954366 | 18 | 50301832 | DCC | intron_variant |
| rs9954387 | 18 | 50301888 | DCC | intron_variant |
| rs9956954 | 18 | 50302147 | DCC | intron_variant |
| rs9946240 | 18 | 50302221 | DCC | intron_variant |
| rs9957208 | 18 | 50302225 | DCC | intron_variant |
| rs9957066 | 18 | 50302280 | DCC | intron_variant |
| rs28720976 | 18 | 50302448 | DCC | intron_variant |
| rs28710315 | 18 | 50302476 | DCC | intron variant |
| rs199781562 | 18 | 50302657 | DCC | intron_variant |
| 18:50302661:AG:A | 18 | 50302661 | NA | NA |
| rs75255207 | 18 | 50302726 | DCC | intron_variant |
| rs146773466 | 18 | 50302743 | DCC | intron_variant |
| rs112096055 | 18 | 50302828 | DCC | intron_variant |
| rs77911615 | 18 | 50302866 | DCC | intron_variant |
| rs77161102 | 18 | 50303025 | DCC | intron_variant |
| rs9960305 | 18 | 50303080 | DCC | intron_variant |
| rs76926243 | 18 | 50303305 | DCC | intron_variant |
| rs9949728 | 18 | 50303409 | DCC | intron_variant |
| rs72079451 | 18 | 50303416 | DCC | intron_variant |
| rs140753418 | 18 | 50303838 | NA | NA |
| rs28418704 | 18 | 50303935 | DCC | intron_variant |
| rs1984342 | 18 | 50303991 | DCC | intron_variant |
| 18:50304137:A:AGGGGG | 18 | 50304137 | NA | NA |
| rs75177472 | 18 | 50304283 | DCC | intron_variant |
| rs28662803 | 18 | 50304359 | DCC | intron_variant |
| rs79241149 | 18 | 50304605 | DCC | intron_variant |
| rs9963981 | 18 | 50304678 | DCC | intron_variant |
| rs74601030 | 18 | 50304685 | DCC | intron_variant |
| rs9966586 | 18 | 50304959 | DCC | intron_variant |
| rs9955760 | 18 | 50305011 | DCC | intron_variant |
| rs9955775 | 18 | 50305063 | DCC | intron_variant |
| rs76362387 | 18 | 50305186 | DCC | intron_variant |

[Table 1-70]

| SNP ID | chr | position | gene locus | SNP location in gene locus |
|---|---|---|---|---|
| rs76980476 | 18 | 50305329 | DCC | intron_variant |
| rs140215942 | 18 | 50305430 | DCC | intron_variant |
| rs79518038 | 18 | 50305494 | DCC | intron_variant |
| rs201972311 | 18 | 50305531 | DCC | intron_variant |
| rs116940662 | 18 | 50305545 | DCC | intron_variant |
| rs9967152 | 18 | 50305573 | DCC | intron_variant |
| rs9956376 | 18 | 50305628 | DCC | intron_variant |
| rs9967408 | 18 | 50305708 | DCC | intron_variant |
| rs9946762 | 18 | 50305989 | DCC | intron_variant |
| rs4638688 | 18 | 50306239 | DCC | intron_variant |
| rs9947241 | 18 | 50306289 | DCC | intron_variant |
| rs9959370 | 18 | 50306321 | DCC | intron_variant |
| 18:50306418:C:CTTCATTCA | 18 | 50306418 | NA | NA |
| rs78062210 | 18 | 50306489 | DCC | intron_variant |
| rs78835166 | 18 | 50306510 | DCC | intron_variant |
| rs183630099 | 18 | 50306529 | DCC | intron_variant |
| rs188676378 | 18 | 50306530 | DCC | intron_variant |
| rs74542236 | 18 | 50306601 | DCC | intron_variant |
| rs74406804 | 18 | 50306611 | DCC | intron_variant |
| rs76093543 | 18 | 50306638 | DCC | intron_variant |
| rs74641489 | 18 | 50306652 | DCC | intron_variant |
| rs79335626 | 18 | 50306756 | DCC | intron_variant |
| rs79579873 | 18 | 50306952 | DCC | intron_variant |
| rs9962392 | 18 | 50307080 | DCC | intron_variant |
| rs9950339 | 18 | 50307252 | DCC | intron_variant |
| rs9950448 | 18 | 50307351 | DCC | intron_variant |
| rs9950558 | 18 | 50307440 | DCC | intron_variant |
| rs9950856 | 18 | 50307670 | DCC | intron_variant |
| rs9951083 | 18 | 50307728 | DCC | intron_variant |
| rs9950936 | 18 | 50307750 | DCC | intron_variant |
| rs143651192 | 18 | 50308170 | NA | NA |
| 18:50308240:G:GTT | 18 | 50308240 | NA | NA |
| rs80118519 | 18 | 50308340 | DCC | intron_variant |
| rs80289134 | 18 | 50308699 | DCC | intron_variant |
| rs77481124 | 18 | 50308701 | DCC | intron_variant |

[Table 1-71]

| SNP ID | chr | position | gene locus | SNP location in gene locus |
|---|---|---|---|---|
| rs113720878 | 18 | 50308740 | DCC | intron_variant |
| rs112252157 | 18 | 50308746 | DCC | intron_variant |
| rs28407519 | 18 | 50308840 | DCC | intron_variant |
| rs28485029 | 18 | 50308845 | DCC | intron_variant |
| rs28610143 | 18 | 50308878 | DCC | intron_variant |
| rs142971261 | 18 | 50308896 | DCC | intron_variant |
| rs111627202 | 18 | 50308902 | DCC | intron_variant |
| rs28398323 | 18 | 50309063 | DCC | intron_variant |
| rs28659932 | 18 | 50309081 | DCC | intron variant |
| rs28524721 | 18 | 50309291 | DCC | intron_variant |
| rs77721880 | 18 | 50309453 | DCC | intron_variant |
| rs78434669 | 18 | 50309461 | DCC | intron_variant |
| rs137865031 | 18 | 50309499 | DCC | intron_variant |
| rs114083506 | 18 | 50309615 | DCC | intron_variant |
| rs79875724 | 18 | 50309626 | DCC | intron_variant |
| rs150063286 | 18 | 50309652 | DCC | intron_variant |
| rs9959936 | 18 | 50309944 | DCC | intron_variant |
| rs111664411 | 18 | 50309956 | DCC | intron_variant |
| rs139769416 | 18 | 50310074 | DCC | intron_variant |
| rs75906728 | 18 | 50310166 | DCC | intron_variant |
| rs74650785 | 18 | 50310219 | DCC | intron_variant |
| rs9949295 | 18 | 50310263 | DCC | intron_variant |
| rs76434772 | 18 | 50310291 | DCC | intron_variant |
| rs75112069 | 18 | 50310392 | DCC | intron_variant |
| rs111554653 | 18 | 50310597 | DCC | intron_variant |
| 18:50310625:C:CTTTCT | 18 | 50310625 | NA | NA |
| rs78714353 | 18 | 50310649 | DCC | intron_variant |
| rs75493591 | 18 | 50310680 | DCC | intron_variant |
| rs77393461 | 18 | 50310710 | DCC | intron_variant |
| rs9960581 | 18 | 50310772 | DCC | intron_variant |
| rs77105189 | 18 | 50310911 | DCC | intron_variant |
| rs115627213 | 18 | 50310920 | DCC | intron_variant |
| rs113795849 | 18 | 50310929 | DCC | intron_variant |
| rs76212234 | 18 | 50311037 | DCC | intron_variant |
| rs75762275 | 18 | 50311039 | DCC | intron_variant |
| rs78544169 | 18 | 50311097 | DCC | intron_variant |

[Table 1-72]

| SNP ID | chr | position | gene locus | SNP location in gene locus |
|---|---|---|---|---|
| rs28762705 | 18 | 50311168 | DCC | intron_variant |
| rs28820319 | 18 | 50311273 | DCC | intron_variant |
| 18:50311304:G:GGAGA | 18 | 50311304 | NA | NA |
| rs9953235 | 18 | 50311467 | DCC | intron_variant |
| rs9953246 | 18 | 50311486 | DCC | intron_variant |
| rs28624269 | 18 | 50311586 | DCC | intron_variant |
| rs28416202 | 18 | 50311587 | DCC | intron_variant |
| rs28587032 | 18 | 50311625 | DCC | intron_variant |
| rs9953616 | 18 | 50311854 | DCC | intron_variant |
| rs9956105 | 18 | 50311914 | DCC | intron_variant |
| rs9956125 | 18 | 50311962 | DCC | intron_variant |
| rs9945323 | 18 | 50312029 | DCC | intron_variant |
| rs9956060 | 18 | 50312136 | DCC | intron_variant |
| rs9945509 | 18 | 50312217 | DCC | intron_variant |
| rs9956657 | 18 | 50312399 | DCC | intron_variant |
| rs9956597 | 18 | 50312504 | DCC | intron_variant |
| rs111891224 | 18 | 50312516 | DCC | intron_variant |
| rs9941424 | 18 | 50312580 | DCC | intron_variant |
| rs143959738 | 18 | 50312634 | DCC | intron variant |
| rs9941425 | 18 | 50312809 | DCC | intron_variant |
| 18:50312832:T:TA | 18 | 50312832 | NA | NA |
| rs9941430 | 18 | 50313014 | DCC | intron_variant |
| rs9941431 | 18 | 50313046 | DCC | intron_variant |
| rs78139846 | 18 | 50313451 | DCC | intron_variant |
| rs75846297 | 18 | 50313646 | DCC | intron_variant |
| rs28748057 | 18 | 50313692 | DCC | intron_variant |
| rs199624929 | 18 | 50313803 | DCC | intron_variant |
| 18:50313941:T:TAAATA | 18 | 50313941 | NA | NA |
| rs28770857 | 18 | 50314106 | DCC | intron_variant |
| rs28872917 | 18 | 50314112 | DCC | intron_variant |
| rs9941438 | 18 | 50314590 | DCC | intron_variant |
| rs9941411 | 18 | 50314697 | DCC | intron_variant |
| rs9941419 | 18 | 50314952 | DCC | intron_variant |
| rs9941444 | 18 | 50315007 | DCC | intron_variant |
| rs138603450 | 18 | 50315409 | DCC | intron_variant |
| rs9955427 | 18 | 50315451 | DCC | intron_variant |
| rs9966590 | 18 | 50315572 | DCC | intron_variant |
| rs9955595 | 18 | 50315604 | DCC | intron_variant |

(continued)

| SNP ID | chr | position | gene locus | SNP location in gene locus |
|---|---|---|---|---|
| rs9966461 | 18 | 50315677 | DCC | intron_variant |

[Table 1-73]

| SNP ID | chr | position | gene locus | SNP location in gene locus |
|---|---|---|---|---|
| rs77259281 | 18 | 50316233 | DCC | intron_variant |
| rs79494668 | 18 | 50316355 | DCC | intron_variant |
| rs9946558 | 18 | 50316362 | DCC | intron_variant |
| rs9958738 | 18 | 50316480 | DCC | intron_variant |
| rs9946735 | 18 | 50316499 | DCC | intron_variant |
| rs9958825 | 18 | 50316550 | DCC | intron_variant |
| rs144978667 | 18 | 56289433 | ALPK2 | intron_variant |
| rs28757575 | 18 | 61639238 | HMSD | intron_variant |
| rs28374149 | 18 | 61639311 | HMSD | intron_variant |
| rs117230606 | 18 | 64368841 | NA | |
| rs77930714 | 18 | 64381429 | NA | |
| rs75773722 | 18 | 64426972 | NA | |
| rs73967432 | 18 | 65688400 | NA | |
| rs113960301 | 18 | 65707948 | NA | |
| rs183621604 | 18 | 74404956 | NA | |
| rs80055446 | 18 | 75608998 | NA | |
| rs74686099 | 18 | 75861241 | NA | |
| rs77331644 | 18 | 75861619 | NA | |
| rs12458593 | 18 | 75862469 | NA | |
| rs113119443 | 18 | 76384089 | NA | |
| 18:76393189:G:A | 18 | 76393189 | NA | NA |
| rs11672916 | 19 | 1036631 | CNN2 | intron_variant |
| rs11667191 | 19 | 1036718 | CNN2 | intron_variant |
| rs11fi72975 | 19 | 1036793 | CNN2 | intron_variant |
| rs11670441 | 19 | 1036913 | CNN2 | intron_variant |
| rs11665845 | 19 | 1037170 | CNN2 | intron_variant |
| rs113353568 | 19 | 1037551 | CNN2 | intron_variant |
| rs111525696 | 19 | 1037563 | CNN2 | intron_variant |
| rs72973561 | 19 | 1037584 | CNN2 | intron_variant |
| rs2232775 | 19 | 8373152 | CD320 | missense_variant |
| rs7249111 | 19 | 8378870 | AC010323.1 | intron_variant |
| rs8109861 | 19 | 8379894 | AC010323.1 | intron_variant |
| rs8110376 | 19 | 8380149 | AC010323.1 | intron_variant |

[Table 1-74]

| SNP ID | chr | position | gene locus | SNP location in gene locus |
|---|---|---|---|---|
| rs11669423 | 19 | 21091222 | NA | |
| rs11667242 | 19 | 21092697 | NA | |
| rs2288896 | 19 | 30067812 | NA | |
| 19:44183972:G:C | 19 | 44183972 | NA | NA |
| rs145903192 | 19 | 44185599 | NA | |
| rs112639353 | 19 | 44187225 | NA | |
| rs79273520 | 19 | 44187737 | NA | |
| rs75691094 | 19 | 44195271 | AC005622.1 | intron variant |
| rs140415511 | 19 | 44195583 | AC005622.1 | intron_variant |
| rs201608105 | 19 | 44198053 | AC005622.1 | intron_variant |
| rs59447107 | 19 | 44199652 | AC005622.1 | intron_variant |
| rs148383153 | 19 | 44200274 | AC005622.1 | intron_variant |
| 19:56565924:A:G | 19 | 56565924 | NA | NA |
| rs117472015 | 19 | 56566789 | NLRP5 | intron_variant |
| rs199933435 | 19 | 56574059 | NA | |
| rs79145402 | 19 | 56576351 | NA | |
| rs56070738 | 20 | 6655570 | NA | |
| rs62194864 | 20 | 6655626 | NA | |
| rs62194867 | 20 | 6663199 | NA | |
| rs8121041 | 20 | 6664256 | NA | |
| rs7272805 | 20 | 6666442 | NA | |
| rs118144766 | 20 | 11371362 | NA | |
| rs147182367 | 20 | 11374504 | NA | |
| rs3067207 | 20 | 11516851 | NA | |
| rs6111325 | 20 | 16858631 | NA | |
| rs73247070 | 20 | 16872792 | NA | |
| rs6105671 | 20 | 16874257 | NA | |
| rs6105672 | 20 | 16874319 | NA | |
| rs75411817 | 20 | 17437518 | PCSK2 | intron_variant |
| rs2284912 | 20 | 17438213 | PCSK2 | intron_variant |
| rs199610440 | 20 | 17444153 | PCSK2 | intron_variant |
| rs73900815 | 20 | 17445166 | PCSK2 | intron_variant |
| rs2269028 | 20 | 17446328 | PCSK2 | intron_variant |
| rs2269029 | 20 | 17446362 | PCSK2 | intron_variant |
| rs2269035 | 20 | 17450530 | PCSK2 | intron_variant |
| rs117870212 | 20 | 17796971 | NA | |

[Table 1-75]

| SNP ID | chr | position | gene locus | SNP location in gene locus |
|---|---|---|---|---|
| rs149160109 | 20 | 19787051 | RIN2 | intron_variant |
| 20:19957482:G:GTC | 20 | 19957482 | NA | NA |
| rs60381469 | 20 | 19958065 | RIN2 | intron_variant |
| rs143672948 | 20 | 42432890 | NA | |
| rs17728712 | 20 | 50064221 | NFATC2 | intron_variant |
| rs6096436 | 20 | 50064556 | NFATC2 | intron_variant |
| rs79332570 | 20 | 55465963 | NA | |
| rs12624457 | 20 | 55642740 | NA | |
| rs16980692 | 20 | 55643296 | NA | |
| rs6014915 | 20 | 55643568 | NA | |
| rs1923179 | 20 | 55644273 | NA | |
| rs78441963 | 20 | 55657024 | NA | |
| rs201245644 | 20 | 55681308 | NA | |
| rs8115658 | 20 | 55686994 | NA | |
| rs149841438 | 20 | 55704642 | NA | |
| rs142759888 | 20 | 55708318 | NA | |
| rs76064436 | 20 | 55750436 | BMP7 | intron_variant |
| rs6128207 | 20 | 56436532 | NA | |
| rs117535594 | 20 | 57263529 | STX16-NPEPL1 | intron_variant |
| rs242813 | 20 | 58649794 | NA | |
| 20:61493128:C:T | 20 | 61493128 | NA | NA |
| 20:61493129:GCC:G | 20 | 61493129 | NA | NA |
| 20:61493132:G:T | 20 | 61493132 | NA | NA |
| 20:61493133:GCCACA:G | 20 | 61493133 | NA | NA |
| rs144044846 | 21 | 19485388 | CHODL | intron_variant |
| rs139603158 | 21 | 32201681 | KRTAP7-1 | 3_prime_UTR_variant |
| rs66481055 | 21 | 40382795 | NA | NA |
| rs450808 | 21 | 43706944 | ABCG1 | intron_variant |
| rs77905583 | 21 | 44278878 | WDR4 | intron_variant |
| rs77931345 | 21 | 44282099 | WDR4 | intron_variant |
| rs112237505 | 21 | 44283785 | WDR4 | intron_variant |
| rs5748948 | 22 | 17694856 | ADA2 | intron_variant |
| rs117100146 | 22 | 17700318 | ADA2 | 5_prime_UTR_variant |
| rs200828580 | 22 | 19162510 | NA | |
| rs5746673 | 22 | 19163047 | NA | |
| rs190264011 | 22 | 19166705 | NA | |

[Table 1-76]

| SNP ID | chr | position | gene locus | SNP location in gene locus |
|---|---|---|---|---|
| rs5748024 | 22 | 19168288 | CLTCL1 | missense_variant |
| rs193038051 | 22 | 19169259 | CLTCL1 | intron_variant |
| rs5748025 | 22 | 19169793 | CLTCL1 | intron_variant |
| rs139626 | 22 | 25224556 | SGSM1 | intron_variant |
| rs139629 | 22 | 25225119 | SGSM1 | intron_variant |
| rs139633 | 22 | 25225879 | SGSM1 | intron_variant |
| rs139637 | 22 | 25228375 | SGSM1 | intron_variant |
| rs139644 | 22 | 25232065 | SGSM1 | intron_variant |
| rs139648 | 22 | 25233260 | SGSM1 | intron_variant |
| rs139649 | 22 | 25233565 | SGSM1 | intron_variant |
| rs132548 | 22 | 29317559 | ZNRF3 | intron_variant |
| rs5997861 | 22 | 31464163 | SMTN | intron_variant |
| 22:31466683:G:T | 22 | 31466683 | NA | NA |
| rs7287041 | 22 | 31469691 | SMTN | intron_variant |
| rs9621179 | 22 | 31472299 | SMTN | intron_variant |
| rs35749963 | 22 | 39244118 | NA | |
| rs9611038 | 22 | 39247482 | NA | |
| rs9607583 | 22 | 39248667 | NA | |
| rs5764095 | 22 | 44530469 | PARVB | intron_variant |
| rs118092933 | 22 | 44547847 | PARVB | intron_variant |
| rs8137152 | 22 | 44750797 | NA | |
| 22:47168035:T:C | 22 | 47168035 | NA | NA |
| 22:47168059:T:C | 22 | 47168059 | NA | NA |
| rs184367871 | 22 | 47177009 | TBC1D22A | intron_variant |
| rs112425324 | 22 | 48826561 | NA | |
| rs140475504 | 22 | 49593633 | NA | |
| 22:49780452C:T | 22 | 49780452 | NA | NA |
| rs5934808 | 23 | 10164106 | CLCN4 | intron_variant |
| rs5979274 | 23 | 10164535 | CLCN4 | intron_variant |
| rs2238891 | 23 | 10165403 | CLCN4 | intron_variant |
| rs72626456 | 23 | 42378072 | NA | |
| rs5909566 | 23 | 118006329 | NA | |
| rs59778322 | 23 | 118053423 | NA | |
| rs35026453 | 23 | 118058304 | NA | |
| rs200499538 | 23 | 118062390 | NA | NA |
| X:118069424:CTCTT:C | 23 | 118069424 | NA | NA |

[Table 1-77]

| SNP ID | chr | position | gene locus | SNP location in gene locus |
|---|---|---|---|---|
| rs5957082 | 23 | 118072203 | NA | |
| rs200504262 | 23 | 118073507 | NA | |
| rs4825392 | 23 | 118076725 | NA | |
| rs5910471 | 23 | 118120492 | LONRF3 | intron_variant |
| rs112219900 | 23 | 118820044 | SEPTIN6 | intron_variant |
| rs6634729 | 23 | 128444668 | NA | |
| rs72614106 | 23 | 128449159 | NA | |
| rs6637592 | 23 | 128543945 | NA | |
| rs6637593 | 23 | 128551239 | NA | |
| rs6637596 | 23 | 128559316 | NA | |
| rs185542983 | 23 | 143920769 | NA | |
| rs137885389 | 23 | 151775134 | NA | NA |
| rs4400524 | 23 | 151777266 | NA | |
| rs58154948 | 23 | 151793551 | NA | |

5. The information processing method according to claim 4,
   wherein the mutations further include one or more mutations shown in Tables 2-1 to 2-9.

[Table 2-1]

| SNP ID | chr | position | gene locus |
|---|---|---|---|
| rs1209461 | 1 | 58408470 | DAB1 |
| rs1202850 | 1 | 58408855 | DAB1 |
| rs1202851 | 1 | 58409755 | DAB1 |
| rs1202788 | 1 | 58414715 | DAB1 |
| rs1202790 | 1 | 58417348 | DAB1 |
| rs969719 | 2 | 50874681 | CTNNA3 |
| rs79725983 | 2 | 50876601 | CTNNA3 |
| rs9309188 | 2 | 50916658 | NA |
| rs9309190 | 2 | 50918967 | NA |
| rs7606024 | 2 | 56616595 | SYT9 |
| rs2566747 | 2 | 82304139 | MAML2 |
| rs2116045 | 2 | 82315179 | MAML2 |
| rs7601451 | 2 | 82322288 | NA |
| rs10202137 | 2 | 82322613 | NA |
| rs12998884 | 2 | 82323175 | NA |
| rs7605223 | 2 | 82323314 | NA |
| rs5832525 | 2 | 82324709 | NA |
| rs1346859 | 2 | 82324973 | NA |

(continued)

| SNP ID | chr | position | gene locus |
|---|---|---|---|
| rs10173814 | 2 | 82326240 | NA |
| rs10197852 | 2 | 82326536 | NA |
| rs1346860 | 2 | 82327886 | NA |
| rs10658639 | 2 | 82335834 | NA |
| rs113286166 | 2 | 82335943 | NA |
| rs4852212 | 2 | 82337967 | NA |
| rs1430708 | 2 | 82339149 | NA |
| 2:82340763:C:T | 2 | 82340763 | NA |
| 2:82340764:A:C | 2 | 82340764 | NA |
| 2:82340765:C:A | 2 | 82340765 | NRXN3 |
| rs10206958 | 2 | 82342060 | NA |
| rs7583816 | 2 | 82344289 | PDXDC1 |
| rs1521665 | 2 | 82373290 | PDXDC1 |
| rs1367437 | 2 | 82379605 | PDXDC1 |
| rs1367438 | 2 | 82380031 | PDXDC1 |
| rs10204952 | 2 | 82383092 | PDXDC1 |
| 2:82383337:AT:A | 2 | 82383337 | PDXDC1 |
| rs6704771 | 2 | 82384630 | PDXDC1 |
| rs2052957 | 2 | 82388160 | PDXDC1 |
| rs934308 | 2 | 82391200 | NA |
| rs10432699 | 2 | 82391773 | NTAN1 |
| rs934309 | 2 | 82393511 | NTAN1 |
| rs10190586 | 2 | 82394929 | NTAN1 |
| rs4852215 | 2 | 82401288 | NA |
| rs11676296 | 2 | 82402793 | NTAN1 |

[Table 2-2]

| SNP ID | chr | position | gene locus |
|---|---|---|---|
| rs1367442 | 2 | 82403420 | NTAN1 |
| rs1430723 | 2 | 82405835 | NTAN1 |
| rs4516453 | 2 | 82405937 | NTAN1 |
| rs2902367 | 2 | 82407091 | RBFOX1 |
| rs1430725 | 2 | 82410156 | NA |
| rs7561758 | 2 | 82410827 | GAS7 |
| rs11894492 | 2 | 82413470 | GAS7 |
| rs12471807 | 2 | 82413980 | GAS7 |
| rs4571075 | 2 | 82433870 | NA |
| rs7599433 | 2 | 82436635 | NA |

(continued)

| SNP ID | chr | position | gene locus |
|---|---|---|---|
| rs4852682 | 2 | 82440268 | NA |
| rs11690894 | 2 | 82452553 | NA |
| rs16856198 | 2 | 131609539 | NA |
| rs12464519 | 2 | 131622215 | NA |
| rs7573895 | 2 | 131625295 | NA |
| rs34469492 | 2 | 131628354 | ARHGEF4 |
| 2:131629169:TAC:T | 2 | 131629169 | ARHGEF4 |
| rs3072355 | 2 | 131631093 | ARHGEF4 |
| rs12693750 | 2 | 154396396 | ARHGEF4 |
| rs1595456 | 2 | 154396518 | NA |
| rs6747679 | 2 | 154397389 | ARHGEF4 |
| rs6736497 | 2 | 154397886 | NA |
| rs7605995 | 2 | 154398711 | NA |
| rs7603189 | 2 | 154398824 | NA |
| 2:154398862:C:A | 2 | 154398862 | NA |
| 2:154398866:C:A | 2 | 154398866 | NA |
| 2:154398870:C:A | 2 | 154398870 | NA |
| 2:154411738:T:C | 2 | 154411738 | NA |
| 2:154411747:A:G | 2 | 154411747 | NA |
| rs77398198 | 2 | 173160555 | NA |
| rs55687168 | 2 | 212203087 | NA |
| rs16845916 | 2 | 212203146 | NA |
| rs74342940 | 2 | 238791983 | NA |
| rs4685035 | 3 | 13847602 | NA |
| rs4685036 | 3 | 13848830 | NA |
| rs6777825 | 3 | 13850933 | RAMP1 |
| rs9812834 | 3 | 76104023 | NRXN1 |
| rs66550319 | 3 | 77725567 | NRXN1 |
| rs6444327 | 3 | 167158977 | NRXN1 |
| rs9874384 | 3 | 167161621 | NRXN1 |
| rs12487400 | 3 | 167162076 | NA |
| rs9859743 | 3 | 167162439 | NA |
| rs13085856 | 3 | 167164661 | NA |

[Table 2-3]

| SNP ID | chr | position | gene locus |
|---|---|---|---|
| rs13081388 | 3 | 167164968 | NA |

(continued)

| SNP ID | chr | position | gene locus |
|---|---|---|---|
| rs9853197 | 3 | 167166760 | NA |
| rs11373865 | 3 | 167167493 | NA |
| rs1104570 | 3 | 167167997 | NA |
| rs9863185 | 3 | 167168148 | NA |
| 3:167200293:T:TA | 3 | 167200293 | NA |
| rs74715659 | 3 | 187690581 | NA |
| rs78575164 | 3 | 187691168 | NA |
| rs77292072 | 3 | 187692633 | NA |
| rs78313080 | 3 | 187693634 | NA |
| rs17745214 | 3 | 187695429 | NA |
| rs17807098 | 3 | 187696291 | NA |
| rs60889349 | 3 | 187696727 | NA |
| rs56398386 | 4 | 4890108 | NA |
| rs62291849 | 4 | 4910704 | NA |
| rs6446700 | 4 | 4911642 | NA |
| rs10029683 | 4 | 4926097 | NA |
| rs10866392 | 4 | 32100576 | NA |
| rs7434982 | 4 | 32102920 | NA |
| rs7700063 | 4 | 32108077 | NA |
| rs4331815 | 4 | 32109975 | NA |
| rs4501264 | 4 | 32113715 | NA |
| 4:32135307:A:T | 4 | 32135307 | NA |
| rs12502887 | 4 | 36986503 | NA |
| rs10034215 | 4 | 37006612 | NA |
| rs10023279 | 4 | 37006976 | NA |
| rs113390631 | 4 | 37007446 | NA |
| rs7686039 | 4 | 37008671 | NA |
| rs36022714 | 4 | 37009144 | NA |
| rs13111377 | 4 | 37013371 | NA |
| rs9995577 | 4 | 37014190 | NA |
| rs10028774 | 4 | 37014223 | NA |
| rs13127134 | 4 | 37019361 | NA |
| rs13111292 | 4 | 37020558 | NA |
| rs9306943 | 4 | 37024228 | NA |
| rs1885879 | 4 | 37029967 | NA |
| rs13145678 | 4 | 37030696 | NA |
| rs34180164 | 4 | 37030722 | NA |
| rs77850243 | 4 | 37031126 | NA |

(continued)

| SNP ID | chr | position | gene locus |
|---|---|---|---|
| rs145655984 | 4 | 37031740 | NA |
| rs11417369 | 4 | 37032249 | NA |
| rs1885880 | 4 | 37034278 | NA |
| rs17289939 | 4 | 37036317 | NA |

[Table 2-4]

| SNP ID | chr | position | gene locus |
|---|---|---|---|
| rs34767825 | 4 | 37038306 | NA |
| rs61540608 | 4 | 37040276 | JPH2 |
| rs17289946 | 4 | 37042065 | NA |
| rs13127946 | 4 | 37042611 | NA |
| rs34800491 | 4 | 37044502 | NA |
| 4:37044634:AG:A | 4 | 37044634 | NA |
| rs7655238 | 4 | 37045620 | NA |
| rs11096857 | 4 | 37046140 | NA |
| rs2608817 | 4 | 39427201 | SERPINI2 |
| rs200342845 | 4 | 39428057 | SERPINI2 |
| rs1979283 | 4 | 39430969 | SERPINI2 |
| rs2687978 | 4 | 39431576 | SERPINI2 |
| 4:39432027:T:C | 4 | 39432027 | SERPINI2 |
| rs1542423 | 4 | 39432132 | SERPINI2 |
| rs2926042 | 4 | 39432747 | SERPINI2 |
| rs3107672 | 4 | 39432966 | SERPINI2 |
| rs12643330 | 4 | 74729606 | SERPINI2 |
| rs28391070 | 4 | 166476773 | NA |
| rs28487969 | 4 | 166478246 | NA |
| rs7658683 | 4 | 166478405 | NA |
| rs7659327 | 4 | 166478477 | NA |
| rs1914836 | 4 | 166482946 | NA |
| 4:166484748:CT:C | 4 | 166484748 | NA |
| rs79544601 | 4 | 166484791 | NA |
| rs17046745 | 4 | 166485142 | NA |
| rs1914835 | 4 | 166485729 | R0B02 |
| rs1914834 | 4 | 166485893 | NA |
| rs6831798 | 4 | 166487806 | NA |
| rs6855033 | 4 | 166488036 | NA |
| rs10017297 | 4 | 166488615 | NA |

(continued)

| SNP ID | chr | position | gene locus |
|---|---|---|---|
| rs150507584 | 4 | 166488986 | NA |
| rs6819473 | 4 | 166489595 | NA |
| rs17046756 | 4 | 166491564 | NA |
| rs7694281 | 4 | 166492825 | NA |
| rs7694612 | 4 | 166492946 | NA |
| rs28583482 | 4 | 166494345 | NA |
| rs10517996 | 4 | 169425778 | NA |
| rs2218255 | 4 | 180035723 | NA |
| rs4410622 | 5 | 4672392 | NA |
| rs1496366 | 5 | 7146388 | NA |
| rs1006152 | 5 | 7151032 | NA |
| rs1494558 | 5 | 35861068 | NA |
| rs6888116 | 5 | 118659579 | NA |

[Table 2-5]

| SNP ID | chr | position | gene locus |
|---|---|---|---|
| rs1876736 | 5 | 118665393 | NA |
| rs250307 | 5 | 118667748 | NA |
| rs250306 | 5 | 118669094 | NA |
| rs1895202 | 5 | 118669851 | PALLD |
| rs250305 | 5 | 118670120 | NA |
| rs1355123 | 5 | 118675585 | NA |
| rs2652696 | 5 | 132139308 | NA |
| rs708460 | 5 | 132144898 | NA |
| rs254289 | 5 | 132167542 | NA |
| rs254287 | 5 | 132172048 | NA |
| rs254293 | 5 | 132178571 | NA |
| rs809140 | 5 | 132193555 | NA |
| rs254285 | 5 | 132198287 | NA |
| rs2431138 | 5 | 174833410 | NA |
| rs265984 | 5 | 174836280 | NA |
| 6:32579277:C:T | 6 | 32579277 | NA |
| rs1 16953562 | 6 | 32676388 | NA |
| rs114931041 | 6 | 32677158 | NA |
| rs116823632 | 6 | 32677235 | NA |
| rs112057165 | 6 | 32677822 | NA |
| rs114912316 | 6 | 32677960 | NA |
| rs114134393 | 6 | 32678064 | LINC02616 |

(continued)

| SNP ID | chr | position | gene locus |
|---|---|---|---|
| rs116915647 | 6 | 32679726 | NA |
| rs114788646 | 6 | 32679762 | NA |
| rs116115695 | 6 | 32680012 | NA |
| rs114245356 | 6 | 32680153 | NA |
| rs115025251 | 6 | 32680267 | NA |
| rs115719910 | 6 | 32680352 | NA |
| rs115942220 | 6 | 32680448 | NA |
| rs114095721 | 6 | 32680867 | NA |
| rs115938991 | 6 | 32681079 | NA |
| rs115036360 | 6 | 32681308 | NA |
| rs2254737 | 7 | 17811797 | NA |
| rs2110015 | 7 | 17812739 | NA |
| rs2254594 | 7 | 17813614 | NA |
| rs2723521 | 7 | 17813651 | NA |
| rs2723520 | 7 | 17813677 | NA |
| rs2723519 | 7 | 17814005 | NA |
| rs1404419 | 7 | 17814029 | NA |
| rs2723518 | 7 | 17814046 | KLB |
| rs2537592 | 7 | 17814051 | KLB |
| rs1404418 | 7 | 17814085 | KLB |
| rs1404417 | 7 | 17814165 | KLB |

[Table 2-6]

| SNP ID | chr | position | gene locus |
|---|---|---|---|
| rs2723517 | 7 | 17814202 | NA |
| rs1404416 | 7 | 17814272 | KLB |
| rs2723516 | 7 | 17814284 | KLB |
| rs2537593 | 7 | 17814531 | KLB |
| rs2537594 | 7 | 17814536 | NA |
| rs2723514 | 7 | 17814548 | NA |
| rs2068172 | 7 | 17814607 | NA |
| 7:17814644:AT:A | 7 | 17814644 | NA |
| rs2723513 | 7 | 17814888 | NA |
| rs2254361 | 7 | 17815625 | TNFAIP8 |
| rs2691617 | 7 | 17822328 | TNFAIP8 |
| rs2691621 | 7 | 17822450 | TNFAIP8 |
| rs146211890 | 7 | 17823353 | TNFAIP8 |

(continued)

| SNP ID | chr | position | gene locus |
|---|---|---|---|
| rs145940129 | 7 | 17823499 | TNFAIP8 |
| rs2691625 | 7 | 17823624 | TNFAIP8 |
| rs72079142 | 7 | 17824200 | TNFAIP8 |
| rs2691627 | 7 | 17824251 | SEPTIN8 |
| rs2691628 | 7 | 17824356 | NA |
| rs2691629 | 7 | 17824631 | NA |
| rs2723500 | 7 | 17824735 | NA |
| rs2691630 | 7 | 17824787 | NA |
| rs2723499 | 7 | 17824885 | NA |
| rs2723498 | 7 | 17824986 | GDF9 |
| rs2254407 | 7 | 17825281 | NA |
| rs998342 | 7 | 17825589 | NA |
| rs2254176 | 7 | 17825605 | IL7R |
| rs144839998 | 7 | 17825719 | NA |
| rs2723497 | 7 | 17825828 | NA |
| rs2723496 | 7 | 17825991 | NA |
| rs2691553 | 7 | 17826282 | NA |
| rs1852010 | 7 | 17826368 | NA |
| rs1852011 | 7 | 17826628 | NA |
| rs1404420 | 7 | 17831018 | NA |
| rs2691583 | 7 | 17831806 | NA |
| rs2293768 | 7 | 100348384 | NA |
| rs78389571 | 7 | 100368389 | NA |
| rs2293766 | 7 | 100371358 | NA |
| rs17147741 | 7 | 100371583 | NA |
| 7:100372539:GTTTTTTGT:G | 7 | 100372539 | NA |
| rs56872215 | 7 | 100376414 | NA |
| rs75119362 | 7 | 100376679 | NA |
| rs77017835 | 7 | 100377364 | NA |
| rs113223392 | 7 | 100378274 | NA |

[Table 2-7]

| SNP ID | chr | position | gene locus |
|---|---|---|---|
| 7:100382845:CTT:C | 7 | 100382845 | NA |
| rs2622261 | 7 | 153522341 | NA |
| rs2622262 | 7 | 153522368 | NA |
| rs2622263 | 7 | 153522433 | ZAN |

(continued)

| SNP ID | chr | position | gene locus |
|---|---|---|---|
| rs113278579 | 7 | 153522636 | ZAN |
| rs2538419 | 7 | 153522987 | ZAN |
| rs2538418 | 7 | 153523926 | ZAN |
| rs10687210 | 7 | 153524089 | NA |
| rs1544613 | 7 | 153524357 | ZAN |
| rs1544612 | 7 | 153524514 | ZAN |
| rs1544611 | 7 | 153525542 | ZAN |
| rs2622241 | 7 | 153526331 | ZAN |
| rs2622242 | 7 | 153526464 | NA |
| rs2070494 | 8 | 37688733 | NA |
| rs10957210 | 8 | 62969408 | NA |
| rs4871749 | 8 | 127897460 | NA |
| rs1863563 | 8 | 129020784 | NA |
| rs12550891 | 9 | 26569055 | NA |
| rs1033988 | 9 | 26571951 | NA |
| rs10819940 | 9 | 98927435 | NA |
| rs10819941 | 9 | 98927481 | NA |
| rs4743609 | 9 | 98930736 | NA |
| rs4743611 | 9 | 98932408 | NA |
| rs10819949 | 9 | 98934331 | NA |
| rs4742873 | 9 | 98950164 | NA |
| rs4742876 | 9 | 98955876 | NA |
| rs7020106 | 9 | 98974141 | NA |
| rs7047022 | 9 | 98974144 | NA |
| rs7035399 | 9 | 101776487 | NA |
| rs77405629 | 9 | 112867809 | NA |
| rs17806439 | 9 | 112886940 | NA |
| rs13297551 | 9 | 120961083 | NA |
| rs71895773 | 9 | 120961500 | NA |
| rs79652695 | 10 | 6976748 | NA |
| rs75366909 | 10 | 68602734 | NA |
| rs17231504 | 10 | 68650441 | NA |
| rs1320042 | 11 | 7444027 | NA |
| rs4753718 | 11 | 95830567 | NA |
| rs7944701 | 11 | 95831023 | NA |
| rs7934906 | 11 | 119488133 | NA |
| rs1855286 | 13 | 47746652 | NA |
| rs1537457 | 13 | 47752877 | NA |

(continued)

| SNP ID | chr | position | gene locus |
|---|---|---|---|
| rs4142743 | 13 | 47756210 | NA |

[Table 2-8]

| SNP ID | chr | position | gene locus |
|---|---|---|---|
| rs4142742 | 13 | 47756308 | NA |
| rs2406095 | 13 | 47762205 | NA |
| rs1930826 | 13 | 47767039 | NA |
| rs7332153 | 13 | 47767339 | NA |
| rs6561358 | 13 | 47770535 | NA |
| rs7333999 | 13 | 47771258 | NA |
| rs10140617 | 14 | 51843558 | NA |
| rs2356901 | 14 | 51845948 | NA |
| rs7153577 | 14 | 78790792 | NA |
| rs58546183 | 14 | 101716329 | NA |
| rs78489963 | 14 | 101719276 | NA |
| rs75949493 | 14 | 101719529 | NA |
| rs12587451 | 14 | 101719796 | NA |
| 15:87561879:AT:ATT | 15 | 87561879 | NA |
| rs2034597 | 16 | 6144047 | NA |
| rs4985124 | 16 | 15125441 | NA |
| rs72789541 | 16 | 15127534 | NA |
| rs72789542 | 16 | 15127535 | NA |
| rs7200543 | 16 | 15129970 | NA |
| rs1741 | 16 | 15130351 | NA |
| rs6498540 | 16 | 15130594 | NA |
| rs1121 | 16 | 15131076 | NA |
| rs4985154 | 16 | 15131642 | NA |
| 16:15131654:C:CA | 16 | 15131654 | NA |
| rs1135999 | 16 | 15131962 | NA |
| rs1136001 | 16 | 15131974 | SNX13 |
| rs2740 | 16 | 15132108 | SNX13 |
| 16:15132211:A:AAAAGTTG | 16 | 15132211 | NA |
| rs34614532 | 16 | 15132908 | NA |
| rs14347 | 16 | 15133889 | ADGRA2 |
| rs4500751 | 16 | 15140211 | NA |
| rs6498541 | 16 | 15140657 | C0L15A1 |
| rs7192753 | 16 | 80098249 | PALM2AKAP2 |
| rs1024370 | 17 | 10054798 | PALM2AKAP2 |

(continued)

| SNP ID | chr | position | gene locus |
|---|---|---|---|
| rs1024369 | 17 | 10054889 | NA |
| rs1468086 | 17 | 10055514 | NA |
| rs4794500 | 17 | 52577372 | NA |
| rs60809192 | 17 | 52578429 | NA |
| rs113532194 | 17 | 52581343 | NA |
| rs56347481 | 17 | 52582030 | NA |
| rs11871596 | 17 | 52582282 | NA |
| rs7226207 | 17 | 52583485 | NA |
| rs753719 | 19 | 411849 | NA |

[Table 2-9]

| SNP ID | chr | position | gene locus |
|---|---|---|---|
| rs6028721 | 20 | 38550395 | NA |
| rs761207 | 20 | 42758834 | NA |
| 20:60359090:G:A | 20 | 60359090 | NA |
| rs6001258 | 22 | 39245583 | NA |

6. An information processing method comprising:

a detection unit configured to detect a first SNP which is a mutation of an Alzheimer's disease-associated gene in a genomic DNA sample derived from a subject; and
a determination unit configured to determine whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

7. A program causing a computer to execute:

Step 1 of detecting a first SNP which is a mutation of an Alzheimer's disease-associated gene in a genomic DNA sample derived from a subject; and
Step 2 of determining whether or not the subject will develop Alzheimer's disease from the first SNP using a machine learning model trained on a plurality of training datasets labeled with information on the onset of Alzheimer's disease for a second SNP, which is a mutation of the Alzheimer's disease-associated gene detected in a genomic DNA sample derived from a patient who has developed Alzheimer's disease.

8. A method for predicting a risk of developing Alzheimer's disease using the information processing method according to claim 1.

## FIG. 1

INFORMATION PROCESSING DEVICE ~100

PROCESSING UNIT ~120

DETECTION UNIT ~110

ACQUISITION UNIT ~121

FEATURE AMOUNT CONVERSION UNIT ~122

DETERMINATION UNIT ~123

STORAGE UNIT ~130

MODEL INFORMATION ~131

OUTPUT CONTROL UNIT ~124

LEARNING UNIT ~125

## FIG. 2A

START

ACQUIRE DETECTION DATA OF FIRST SNP ~S100

CONVERT DETECTION DATA OF FIRST SNP INTO FEATURE AMOUNT ~S101

INPUT FEATURE AMOUNT TO PREDICTION MODEL ~S102

IS SCORE GREATER THAN OR EQUAL TO THRESHOLD VALUE? ~S103

NO

YES

AD IS DEVELOPED ~S104

AD IS NOT DEVELOPED ~S105

OUTPUT DETERMINATION RESULTS ~S106

RETURN

*FIG. 2B*

MDL

WL-1

CLASSIFIER 1

WL-2

CLASSIFIER 2

FEATURE
AMOUNT

SCORE

SCORE

SCORE

WL-N

CLASSIFIER N

$\frac{1}{N}\Sigma$

NORMALIZED
SCORE

## FIG. 2C

START

GENERATE TRAINING DATASET — S200

CONVERT DETECTION DATA OF DETECTION DATA OF SNP INTO FEATURE AMOUNT — S201

INPUT TRAINING FEATURE AMOUNT TO CLASSIFIER WL-i — S202

ACQUIRE OUTPUT RESULTS FROM CLASSIFIER WL-i — S203

CALCULATE ERRORS — S204

TRAIN CLASSIFIER WL-I BASED ON ERRORS — S205

HAS LEARNING BEEN REPEATED PREDETERMINED NUMBER OF TIMES? — S206

NO

YES

INPUTS VERIFICATION FEATURE AMOUNTS TO TRAINED CLASSIFIERS WL-i — S207

SELECT TRAINED CLASSIFIER WL-i WITH HIGHEST PREDICTION ACCURACY — S208

HAVE N CLASSIFIERS BEEN TRAINED? — S209

NO
i = i+1

YES

RETURN

317

## FIG. 3A

## FIG. 3B

*FIG. 3C*

*FIG. 3D*

FIG. 4A

FIG. 4B

## FIG. 4C

## FIG. 4D

*FIG. 4E*

*FIG. 4F*

## FIG. 5A

## FIG. 5B

## FIG. 5C

White Adipose Tissue Browning Pathway
Synaptic Long Term Potentiation
GPCR-Mediated Nutrient Sensing in Enteroendocrine Cells
Neuropathic Pain Signaling In Dorsal Horn Neurons
Insulin Secretion Signaling Pathway
Senescence Pathway
CREB Signaling in Neurons
Synaptogenesis Signaling Pathway
nNOS Signaling in Skeletal Muscle Cells
Opioid Signaling Pathway
Corticotropin Releasing Hormone Signaling
Synaptic Long Term Depression
Netrin Signaling
Calcium Signaling

-log (p-value)

## FIG. 6A

N.S.

p-231 tau ratio

APOE genotype

## FIG. 6B

## FIG. 6C

## FIG. 6D

## FIG. 6E

## FIG. 7A

## FIG. 7B

## FIG. 7C

## FIG. 7D

*FIG. 7E*

*FIG. 7F*

*FIG. 7G*

*FIG. 8A*

## FIG. 8B

## FIG. 8C

FIG. 8D

FIG. 9A

## FIG. 9B

Predict cerebrospinal fluid Aβ42

covariates only — AUC = 0.69 ± 0.059

covariates plus genotype sets — AUC = 0.73 ± 0.059

## FIG. 9C

Predict cerebrospinal fluid t-tau

covariates only — AUC = 0.71 ± 0.061

covariates plus genotype sets — AUC = 0.75 ± 0.039

## FIG. 9D

Predict cerebrospinal fluid p-tau

covariates only

covariates plus genotype sets

AUC = 0.704 ± 0.061

AUC = 0.74 ± 0.038

<div align="center">

**INTERNATIONAL SEARCH REPORT**

</div>

| International application No. |
|---|
| **PCT/JP2022/017576** |

| | |
|---|---|
| **A.** | **CLASSIFICATION OF SUBJECT MATTER** |
| | ***C12N 15/00***(2006.01)i |
| | FI: C12N15/00 |
| According to International Patent Classification (IPC) or to both national classification and IPC |

| | |
|---|---|
| **B.** | **FIELDS SEARCHED** |
| Minimum documentation searched (classification system followed by classification symbols) |
| | C12N15/00 |

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | WO 2019/178167 A1 (I2DX, INC.) 19 September 2019 (2019-09-19) claims, pp. 12-13, 27, 37-43, tables 1-3 | 1, 3, 6-8 |
| Y | | 1-8 |
| Y | 井上治久, 近藤孝之, アルツハイマー病リスク予測技術開発による超高齢社会における QOLの向上, 医科学応用研究財団研究堯告 2018, 2020, vol. 37, pp. 61-64 p. 61, left column to right column, 1st paragraph, pp. 62-64, column "3. Results", (INOUE, Haruhisa. KONDO, Takayuki. Research Papers of the Suzuken Memorial Foundation.), non-official translation (Improving QOL in a super-aging society by developing Alzheimer's disease risk prediction technology) | 1-8 |
| Y | NGUYEN, T. et al. Genome-wide association data classification and SNPs selection using two-stage quality-based Random Forests. BMC Genomics. 2015, vol. 16, suppl. 2, article no. S5 (pp. 1-11) abstract | 1-8 |
| Y | ARAUJO, G. S. et al. Random Forest and Gene Networks for Association of SNPs to Alzheimer's Disease. Brazilian Symposium on Bioinformatics. 2013, LNBI 8213, pp. 104-115 abstract | 1-8 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 June 2022** | **05 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2022/017576** |

### C.    DOCUMENTS CONSIDERED TO BE RELEVANT

| Category* | Citation of document, with indication, where appropriate, of the relevant passages | Relevant to claim No. |
|---|---|---|
| P, X | KONDO, T. et al. Dissection of the polygenic architecture of neuronal Aβ production using a large sample of individual iPSC lines derived from Alzheimer's disease patients. Nature Aging. 17 February 2022, vol. 2, pp. 125-139 (suppl. pp. 1-19) <br> abstract, tables 1, 2 | 1-8 |
| A | WO 2020/067386 A1 (AJINOMOTO KK) 02 April 2020 (2020-04-02) <br> claims, paragraph [0044] | 1-8 |
| A | WO 2019/199105 A1 (SAMSUNG LIFE PUBLIC WELFARE FOUNDATION) 17 October 2019 (2019-10-17) <br> abstract, claims | 1-8 |
| A | 池内健, 4 アルツハイマー病のプレシジョン メディシンへの展望, Precision Medicine. 2018, vol. 1, no. 3, pp. 255-259 <br> abstract, columns 4., 5., (IKEUCHI, Takeshi. 4 Perspective on precision medicine in Alzheimer's disease.) | 1-8 |
| A | 樋口陽, 池内健, ゲノム情堯を活用した認知症予防, 老年内科, 2020, vol. 2, no. 4, pp. 441-446 <br> p. 443, (HIGUCHI, Yo. IKEUCHI, Takeshi. The role of genetics in dementia prevention. Geriatrics.) | 1-8 |

Form PCT/ISA/210 (second sheet) (January 2015)

**INTERNATIONAL SEARCH REPORT**

| International application No. |
| --- |
| **PCT/JP2022/017576** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

a.  ☑ forming part of the international application as filed:

☑ in the form of an Annex C/ST.25 text file.

☐ on paper or in the form of an image file.

b.  ☐ furnished together with the international application under PCT Rule 13*ter*.1(a) for the purposes of international search only in the form of an Annex C/ST.25 text file.

c.  ☐ furnished subsequent to the international filing date for the purposes of international search only:

☐ in the form of an Annex C/ST.25 text file (Rule 13*ter*.1(a)).

☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

### INTERNATIONAL SEARCH REPORT
#### Information on patent family members

International application No.

**PCT/JP2022/017576**

| Patent document cited in search report | | | Publication date (day/month/year) | Patent family member(s) | | | Publication date (day/month/year) |
|---|---|---|---|---|---|---|---|
| WO | 2019/178167 | A1 | 19 September 2019 | JP | 2021-515940 | A | |
| | | | | claims, paragraphs [0034], [0080], [0123]-[0137], tables 1-3 | | | |
| | | | | EP | 3764902 | A1 | |
| WO | 2020/067386 | A1 | 02 April 2020 | US | 2021/0278421 | A1 | |
| | | | | claims, paragraph [0098] | | | |
| | | | | EP | 3859339 | A1 | |
| WO | 2019/199105 | A1 | 17 October 2019 | KR | 10-2019-0119859 | A | |
| | | | | claims | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63174500

**Non-patent literature cited in the description**

- **SIMS R et al.** The multiplex model of the genetics of Alzheimer's disease. *Nature Neuroscience,* 2020, vol. 23, 311-322
- Molecular Cloning, A Laboratory Manual. Cold Spring Harbor Press, 2001
- Current Protocols in Molecular Biology. John Wiley&Sons, 1987
- DNA Cloning 1: Core Techniques, A Practical Approach. Oxford University, 1995
- *Science .,* 22 December 2000, vol. 290 (5500), 2303-4